# EUROPEAN PATENT APPLICATION

(11) **EP 4 650 343 A1**
(43) Date of publication of application: **19.11.2025**
(21) Application number: 24741327.1
(22) Date of filing: 11.01.2024
(51) Int. Cl.: C07C 237/10, C07C 229/12, A61K 47/22, A61K 48/00

(54) **AMINO LIPID COMPOUND, AND PREPARATION METHOD THEREFOR AND USE THEREOF**

(30) Priority: 11.01.2023 WO PCT/CN2023/071822; 20.01.2023 WO PCT/CN2023/073301; 07.02.2023 WO PCT/CN2023/074783; 21.04.2023 WO PCT/CN2023/089685; 13.06.2023 WO PCT/CN2023/099815; 28.09.2023 WO PCT/CN2023/122540
(71) Applicant: Shenzhen Shenxin Biotechnology Co., Ltd., Shenzhen, Guangdong 518000 (CN)
(72) Inventor: LI, Linxian, Shenzhen, Guangdong 518000 (CN); CHEN, Yonghao, Shenzhen, Guangdong 518000 (CN); HUANG, Xing, Shenzhen, Guangdong 518000 (CN)
(74) Representative: Nederlandsch Octrooibureau
(86) International application number: PCT/CN2024/071826
(87) International publication number: WO 2024/149331

(57) **Abstract**

An amino lipid compound, and a preparation method therefor and the use thereof. The present invention further relates to a lipid nanoparticle and a pharmaceutical composition containing the amino lipid compound, and the use thereof.

## Description

### Technical Field

The present disclosure relates to an amino lipid compound, and a preparation method therefor and a use thereof. The present disclosure also relates to a lipid nanoparticle and a pharmaceutical composition, containing the amino lipid compound, and use thereof.

### Background Art

Gene-based drugs are intended to introduce exogenous normal genes into target cells for the purpose of therapy or induction of immune response. However, the gene-based drugs are facing several challenges. Particularly, for nucleic acid drugs, it is very difficult to directly introduce the nucleic acids into cells, and they are extremely vulnerable to degradation by nucleic acid degrading enzymes in the cytoplasm. The delivery of nucleic acid drugs by lipid nanoparticles has been widely used. However, the targeting, safety, and delivery efficiency of nucleic acid drug delivery remain to be improved. Different nucleic acid drugs, cells (or tissues, organs), and application scenarios need to be matched with lipid nanoparticles of different properties.

Thus, it is of great research significance to develop, and there is a great realistic demand for developing different lipid nanoparticles, especially amino lipid compounds for preparing lipid nanoparticles, and related preparation methods and applications, to accommodate the need of delivering nucleic acid drugs in different application scenarios.

### Summary of the Invention

One aspect of the present disclosure provides an amino lipid compound represented by formula (I): 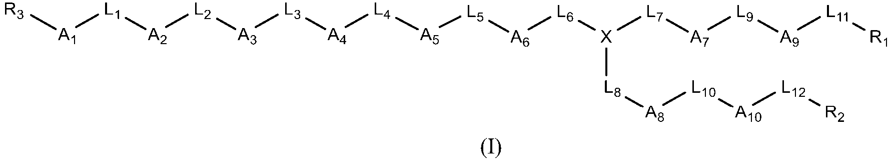 wherein L₁, L₂, L₃, L₄, L₅, L₆, L₇, L₈, L₉, L₁₀, L₁₁, L₁₂, A₁, A₂, A₃, A₄, A₅, A₆, A₇, A₈, A₉, A₁₀, X, R₁, R₂, and R₃ are each as defined below.

Another aspect of the present disclosure provides a method for preparing the amino lipid compound.

Another aspect of the present disclosure provides a use of the amino lipid compound in the manufacture of a vehicle for an active ingredient.

Another aspect of the present disclosure provides a lipid nanoparticle comprising the amino lipid compound.

Another aspect of the present disclosure provides a pharmaceutical composition comprising the lipid nanoparticle.

Another aspect of the present disclosure provides a method for delivering a biologically active ingredient into cells, tissues, or organs by the lipid nanoparticle or pharmaceutical composition.

Another aspect of the present disclosure provides a method for producing a polypeptide and/or protein of interest in mammalian cells by the lipid nanoparticle or pharmaceutical composition.

Another aspect of the present disclosure provides a use of the amino lipid compound, lipid nanoparticle, or pharmaceutical composition in the manufacture of a medicament.

Another aspect of the present disclosure provides a method for treating a disease or disorder in a mammal in need thereof by the lipid nanoparticle or pharmaceutical composition.

Another aspect of the present disclosure provides a use of the amino lipid compound, lipid nanoparticle, or pharmaceutical composition in the manufacture of a medicament for nucleic acid transfer.

### Brief Description of the Drawings

FIG. 1A ~ FIG. 1B show the results of *in vivo* delivery of lipid nanoparticles comprising different amino lipid compounds;
FIG. 2A ~ FIG. 2B show the results of *in vivo* safety (ALT enzymatic activity) test of lipid nanoparticles comprising different amino lipid compounds;
FIG. 3A ~ FIG. 3B show the results of *in vivo* safety (AST enzymatic activity) test of lipid nanoparticles comprising different amino lipid compounds; and
FIG. 4 ~ FIG. 11 show the delivery efficiency of lipid nanoparticles comprising different amino lipid compounds in different immune cell populations in the spleen.

### Detailed Description of the Invention

### Definitions

Unless otherwise defined below, all technical and scientific terms used herein are intended to have the same meaning as commonly understood by one of ordinary skill in the art. Reference to a technique as used herein is intended to mean a technique as commonly understood in the art, including those variations that are apparent to those skilled in the art, or substitutions of equivalence technique. While it is believed that the following terms are well understood by those skilled in the art, the following definitions are set forth to better explain the present invention.

As used herein, the terms "comprising", "including", "having", "containing", or "involving" as well as other variations thereof, are inclusive or open-ended and do not exclude other non-recited elements or method steps.

As used herein, the term "hydrocarbyl" refers to the group remaining after the loss of one hydrogen atom from an aliphatic hydrocarbon, including straight or branched, saturated or unsaturated hydrocarbyl groups. A hydrocarbyl group includes, but is not limited to, alkyl, alkenyl, and alkynyl groups. Preferably, the hydrocarbyl group has from 1 to 24 carbon atoms (C₁-C₂₄ hydrocarbyl), for example, 1, 2, 3, 4, 5, 6, 7, 8, 9, 10, 11, 12, 13, 14, 15, 16, 17, 18, 19, 20, 21, 22, 23 or 24 carbon atoms (C₁, C₂, C₃, ... C₂₁, C₂₂, C₂₃, or C₂₄ hydrocarbyl). Examples of the hydrocarbyl group include, but are not limited to, C₁-C₂₄ hydrocarbyl, C₁-C₂₂ hydrocarbyl, C₁-C₂₀ hydrocarbyl, C₁-C₁₈ hydrocarbyl, C₁-C₁₆ hydrocarbyl, C₁-C₁₂ hydrocarbyl, C₁-C₁₀ hydrocarbyl, C₁-C₈ hydrocarbyl, C₁-C₇ hydrocarbyl, C₁-C₆ hydrocarbyl, C₁-C₄ hydrocarbyl, C₁-C₃ hydrocarbyl, C₁-C₂ hydrocarbyl, C₂-C₈ hydrocarbyl, C₂-C₄ hydrocarbyl, C₄-C₈ hydrocarbyl, C₄-C₉ hydrocarbyl, C₅-C₈ hydrocarbyl, C₁-C₄ hydrocarbyl, C₂-C₈ hydrocarbyl, C₃ hydrocarbyl, C₄ hydrocarbyl, C₅ hydrocarbyl, C₆ hydrocarbyl, C₇ hydrocarbyl, and C₈ hydrocarbyl. Unless explicitly stated otherwise in this specification, the hydrocarbyl group is optionally substituted, and for the substituents, reference is made to the definition of "optionally substituted" below. In certain embodiments, the hydrocarbyl group has no branches (i.e., is a straight chain), one branch, two branches, or multiple branches.

As used herein, the term "hydrocarbylene" refers to a divalent group remaining after further loss of one hydrogen atom from the hydrocarbyl as defined above. Unless expressly stated otherwise in this specification, the hydrocarbylene group is also optionally substituted.

As used herein, the term "alkyl" is a straight or branched saturated monovalent hydrocarbyl. Preferably, an alkyl group has from 1 to 24 carbon atoms (C₁-C₂₄ alkyl), for example, 1, 2, 3, 4, 5, 6, 7, 8, 9, 10, 11, 12, 13, 14, 15, 16, 17, 18, 19, 20, 21, 22, 23 or 24 carbon atoms (C₁, C₂, C₃, ...C₂₁, C₂₂, C₂₃, or C₂₄ alkyl). Examples of the alkyl group include, but are not limited to, C₁-C₂₄ alkyl, C₁-C₂₂ alkyl, C₁-C₂₀ alkyl, C₁-C₁₈ alkyl, C₁-C₁₆ alkyl, C₁-C₁₂ alkyl, C₁-C₁₀ alkyl, C₁-C₈ alkyl, C₁-C₇ alkyl, C₁-C₆ alkyl, C₁-C₄ alkyl, C₁-C₃ alkyl, C₁-C₂ alkyl, C₂-C₈ alkyl, C₂-C₄ alkyl, C₄-C₈ alkyl, C₄-C₉ alkyl, C₅-C₈ alkyl, C₁-C₄ alkyl, C₂-C₈ alkyl, methyl, ethyl, n-propyl, isopropyl, n-butyl, sec-butyl, isobutyl, tert-butyl, n-pentyl, n-hexyl, n-heptyl, n-octyl, n-nonyl, n-decyl, n-undecyl, n-dodecyl, n-tridecyl, and tridecan-7-yl. Unless explicitly stated otherwise in this specification, the alkyl group is optionally substituted.

As used herein, the term "alkylene" refers to a divalent group remaining after further loss of one hydrogen atom from the alkyl as defined above. Unless expressly stated otherwise in this specification, the alkylene is also optionally substituted.

As used herein, the term "alkenyl" is a straight or branched monovalent hydrocarbyl containing one or more double bonds (C=C). Preferably, an alkenyl group has from 2 to 24 carbon atoms (C₂-C₂₄ alkenyl), for example, 2, 3, 4, 5, 6, 7, 8, 9, 10, 11, 12, 13, 14, 15, 16, 17, 18, 19, 20, 21, 22, 23 or 24 carbon atoms (C₂, C₃, C₄, ... C₂₁, C₂₂, C₂₃, or C₂₄ alkenyl), and has 1, 2, 3, 4, or more double bonds. The alkenyl group includes, but is not limited to, C₂-C₂₄ alkenyl, C₂-C₂₂ alkenyl, C₂-C₂₀ alkenyl, C₂-C₁₈ alkenyl, C₂-C₁₆ alkenyl, C₂-C₁₂ alkenyl, C₂-C₁₀ alkenyl, C₂-C₈ alkenyl, C₂-C₇ alkenyl, C₂-C₆ alkenyl, C₂-C₄ alkenyl, C₂-C₃ alkenyl, C₄-C₈ alkenyl, C₄-C₉ alkenyl, C₅-C₈ alkenyl having 1, 2, 3, 4 or more double bonds. Some more specific examples include, but are not limited to, ethenyl, propenyl, but-1-enyl, but-2-enyl, pent-1-enyl, pent-2-enyl, hex-1-enyl, hex-2-enyl, hex-3-enyl, hept-1-enyl, hept-2-enyl, hept-3-enyl, oct-1-enyl, oct-2-enyl, oct-3-enyl, non-1-enyl, non-2-enyl, and non-3-enyl. In some preferred embodiments, the alkenyl group has one double bond. Unless expressly stated otherwise in this specification, the alkenyl group is optionally substituted.

As used herein, the term "alkenylene" refers to a divalent group remaining after further loss of one hydrogen atom from the alkenyl as defined above. Unless expressly stated otherwise in the specification, the alkenylene group is also optionally substituted.

As used herein, the term "alkynyl" is a straight or branched monovalent hydrocarbyl group containing one or more triple bonds (C=C). Preferably, an alkynyl group has from 2 to 24 carbon atoms (C₂-C₂₄ alkynyl), for example, 2, 3, 4, 5, 6, 7, 8, 9, 10, 11, 12, 13, 14, 15, 16, 17, 18, 19, 20, 21, 22, 23 or 24 carbon atoms (C₂, C₃, C₄, ... C₂₁, C₂₂, C₂₃, or C₂₄ alkynyl), and having 1, 2, 3, 4, or more triple bonds. The alkynyl group includes, but is not limited to, C₂-C₂₄ alkynyl, C₂-C₂₂ alkynyl, C₂-C₂₀ alkynyl, C₂-C₁₈ alkynyl, C₂-C₁₆ alkynyl, C₂-C₁₂ alkynyl, C₂-C₁₀ alkynyl, C₂-C₈ alkynyl, C₂-C₇ alkynyl, C₂-C₆ alkynyl, C₂-C₄ alkynyl, C₂-C₃ alkynyl, C₄-C₈ alkynyl, C₄-C₉ alkynyl, C₅-C₈ alkynyl having 1, 2, 3, 4 or more triple bonds. Some more specific examples include, but are not limited to, ethynyl, propynyl, but-1-ynyl, but-2-ynyl, pent-1-ynyl, pent-2-ynyl, hex-1-ynyl, hex-2-ynyl, hex-3-ynyl, hept-1-ynyl, hept-2-ynyl, hept-3-ynyl, oct-1-ynyl, oct-2-ynyl, oct-3-ynyl, non-1-ynyl, non-2-ynyl, and non-3-ynyl. In some preferred embodiments, the alkynyl group has one triple bond. Unless explicitly stated otherwise in this specification, the alkynyl group is optionally substituted.

As used herein, the term "alkynylene" refers to a divalent group remaining after further loss of one hydrogen atom from the alkynyl as defined above. Unless explicitly stated otherwise in the specification, the alkynylene group is also optionally substituted.

As used herein, the terms "cyclohydrocarbyl", "cyclohydrocarbylene", and "hydrocarbon ring" refer to a saturated (i.e., "cycloalkyl" and "cycloalkylene") or unsaturated (i.e., having one or more double bonds (cycloalkenyl) and/or triple bonds (cycloalkynyl) in the ring) monocyclic or polycyclic system comprising one or more rings composed of ring carbon atoms. In certain embodiments, "cyclohydrocarbyl", "cyclohydrocarbylene", and "hydrocarbon ring" have, for example, from 3 to 10, suitably from 3 to 8, more suitably from 3 to 6, such as from 5 to 6 or from 5 to 7, ring carbon atoms. "Cyclohydrocarbyl", "cyclohydrocarbylene", and "hydrocarbon ring" include, but are not limited to, cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), etc. Unless expressly stated otherwise in this specification, the cyclohydrocarbyl, cyclohydrocarbylene, and hydrocarbon ring are optionally substituted.

As used herein, the term "cycloalkyl" refers to a saturated monocyclic or polycyclic (such as bicyclic) hydrocarbon ring (e.g., monocyclic, such as cyclopropyl, cyclobutyl, cyclopentyl, cyclohexyl, cycloheptyl, cyclooctyl, cyclononyl, or bicyclic, including spirocyclic, fused, or bridged systems, such as bicyclo[1.1.1] pentyl, bicyclo[2.2.1]heptyl, bicyclo[3.2.1]octyl or bicyclo[5.2.0]nonyl, decalin, etc.). In certain embodiments, the cycloalkyl group has, for example, from 3 to 10, such as from 3 to 7, from 5 to 6, or from 5 to 7 carbon atoms. Unless expressly stated otherwise in this specification, the cycloalkyl group is optionally substituted.

As used herein, the term "heterohydrocarbyl" or its subordinate concepts (e.g., heteroalkyl, heteroalkenyl, heteroalkynyl, heteroaryl, etc.) refer to a stable straight, branched, or cyclic hydrocarbon radical or combination thereof, consisting of a specified number of carbon atoms and at least one heteroatom. The heteroatom refers to an atom other than carbon and hydrogen. In certain embodiments, the heterohydrocarbyl group contains one, two, three, or more heteroatoms. In certain embodiments, the heterohydrocarbyl group contains one or more (e.g., two or three) identical heteroatoms, or contains multiple (e.g., two or three) different heteroatoms. Preferably, the heteroatom is selected from O, N and S. Examples of the heterohydrocarbyl group include, but are not limited to, -CH₂-CH₂-O-CH₃, -CH₂-CH₂-CH₂-O-CH₂-CH₃, -CH₂-(CH₂)₃-O-(CH₂)₅-CH₃, -CH₂-CH₂-NH-CH₃, -CH₂-CH₂-N(CH₃)-CH₃, -CH₂-S-CH₂-CH₃, -CH₂-CH₂, -CH=CH-O-CH₃, -CH₂-CH=N-OCH₃, -CH=CH-N(CH₃)-CH₃, and -CH₂-NH-OCH₃. Unless explicitly stated otherwise in this specification, the heterohydrocarbyl group or its subordinate concepts (e.g., heteroalkyl, heteroalkenyl, heteroalkynyl, heteroaryl, etc.) are optionally substituted.

As used herein, the term "heterohydrocarbylene" or its subordinate concepts (such as heteroalkylene, heteroalkenylene, heteroalkynylene, heteroarylene, etc.) refer to a divalent group remaining after further loss of one hydrogen atom from the heterohydrocarbyl as defined above. Unless explicitly stated otherwise in this specification, the heterohydrocarbylene group or its subordinate concepts (such as heteroalkylene, heteroalkenylene, heteroalkynylene, heteroarylene, etc.) are also optionally substituted.

As used herein, the term "carbocyclic ring" or "carbocyclic ring group" means an optionally substituted monocyclic or polycyclic system comprising one or more rings composed of carbon atoms. The ring may be a three-, four-, five-, six-, seven-, eight-, nine-, ten-, eleven-, twelve-, thirteen-, fourteen-, fifteen-, sixteen-, seventeen-, eighteen-, nineteen-, or twenty-membered ring. The symbol "C₃₋₆ carbocyclic ring" means a carbocyclic ring including a monocyclic ring having from 3 to 6 carbon atoms. The carbocyclic ring may include one or more carbon-carbon double bonds or triple bonds and may be a non-aromatic or aromatic ring (e.g., cycloalkyl or aryl). Examples of the carbocyclic ring include cyclopropyl, cyclopentyl, cyclohexyl, phenyl, naphthyl, and 1,2-dihydronaphthyl. The "carbocyclic ring" or "carbocyclic ring group" may be optionally substituted with one or more substituents, and for the substituents, reference is made to the definition of "optionally substituted" below. Unless explicitly stated otherwise in the specification, the carbocyclic ring or carbocyclic ring group is optionally substituted.

As used herein, the term "heterocycle", "heterocyclyl", or "heterocyclylene" means a cyclic group having a cyclic structure and containing one or more heteroatoms in the ring-forming atoms. In certain embodiments, the ring-forming atoms include one or more heteroatoms which are the same or different. In certain embodiments, the one or more heteroatoms included in the ring-forming atoms are selected from N, O, and S. The "heterocycle", "heterocyclyl" or "heterocyclylene" as disclosed herein is saturated or unsaturated. In certain embodiments, the "heterocycle", "heterocyclyl", or "heterocyclylene" comprises a monocyclic ring, a bicyclic ring, or a polycyclic ring. In certain embodiments, the "heterocycle", "heterocyclyl", or "heterocyclylene" is a 4- to 10-membered heterocycle, e.g., 4- to 7-membered heterocycle, 5- to 7-membered heterocycle. Preferably, in certain embodiments, the heterocycle group is a 4- to 10-membered heterocycle which may be optionally substituted, wherein the ring-forming atoms contain 1, 2, 3, 4, 5, or 6 heteroatoms selected from N, O, and S. More preferably, the heterocycle group is a 4- to 7-membered saturated heterocycle which may be optionally substituted, wherein the ring-forming atoms contain 1, 2, 3 or 4 heteroatoms selected from N, O and S; more preferably, the heterocycle group is a 5- to 7-membered (e.g., 5- to 6-membered) saturated heterocycle which may be optionally substituted, wherein the ring-forming atoms contain 1, 2 or 3 heteroatoms selected from N, O and S. The heterocycle may include one or more double bonds or triple bonds, and may be a non-aromatic or aromatic ring (e.g., heterocycloalkyl or heteroaryl). Examples of heterocycle include, but are not limited to, azetidine, oxetanyl, tetrahydrofuran, pyrrolidine, imidazolidine, pyrazolidine, tetrahydropyran, piperidine, morpholine, thiomorpholine, piperazine, and preferably pyrrolidine, piperidine, piperazine, and morpholine. The "heterocycle", "heterocyclyl", or "heterocyclylene" may be optionally substituted with one or more substituents, and for the substituents, reference is made to the definition for "optionally substituted" below. Unless expressly stated otherwise in this specification, the heterocycle, heterocyclyl, or heterocyclylene is optionally substituted.

As used herein, the term "aryl" refers to an all-carbon monocyclic or fused polycyclic aromatic group having a conjugated π-electron system. For example, as used herein, the term "C₆₋₁₄ aryl" means an aromatic group containing from 6 to 14 (e.g., from 6 to 12) carbon atoms, such as phenyl or naphthyl. Unless explicitly stated otherwise in this specification, the aryl group is optionally substituted.

As used herein, the term "heteroaryl" refers to a monocyclic or polycyclic (e.g., bicyclic or tricyclic) aromatic group having a conjugated π-electron system, of which ring atoms consist of carbon atoms and at least one heteroatom, for example, it has from 5 to 14 (e.g., 5, 6, 7, 8, 9, 10, 11, 12, 13, or 14) ring atoms, including 1, 2, 3, 4, 5, 6, 7, 8, 9, 10, 11, 12, or 13 carbon atoms and 1, 2, 3, 4, or 5 identical or different heteroatoms independently selected from N, O, S, and S(O)₂. One or more ring carbon atoms in the heteroaryl group may be substituted with C(O). The heteroaryl group may be benzo-fused. Unless explicitly stated otherwise in this specification, the heteroaryl group is optionally substituted.

As used herein, the term "optionally substituted" means that one or more hydrogen atoms attached to an atom or group are independently unsubstituted, or independently substituted with one or more (e.g., 1, 2, 3, or 4) substituents. The substituents are independently selected from, but are not limit to, deuterium (D), tritium (T), halogen, -OH, mercapto, -CN, -CD₃, C₁-C₆ alkyl (preferably C₁-C₃ alkyl), C₂-C₆ alkenyl, C₂-C₆ alkynyl, cycloalkyl (preferably C₃-C₈ cycloalkyl), aryl, heterocyclyl (preferably 3- to 8-membered heterocyclyl), heteroaryl, arylC₁-C₆ alkyl-, heteroarylC₁-C₆ alkyl, C₁-C₆ haloalkyl, -OC₁-C₆ alkyl (preferably -OC₁-C₃ alkyl), -OC₂-C₆ alkenyl, OC₁-C₆ alkylphenyl, C₁-C₆ alkyl-OH (preferably C₁-C₄ alkyl-OH), C₁-C₆ alkyl-SH, C₁-C₆ alkyl-O-C₁-C₆ alkyl, OC₁-C₆ haloalkyl, -NH₂, C₁-C₆ alkyl-NH₂ (preferably C₁-C₃ alkyl-NH₂), -N(C₁-C₆alkyl)₂ (preferably -N(C₁-C₃ alkyl)₂), -NH(C₁-C₆ alkyl) (preferably -NH(C₁-C₃ alkyl)), -N(C₁-C₆ alkyl)(C₁-C₆ alkylphenyl), -NH(C₁-C₆ alkylphenyl), nitro, -C(O)-OH, -C(O)OC₁-C₆ alkyl (preferably - C(O)OC₁-C₃ alkyl), -CONRiRii (wherein Ri and Rii is H, D and C₁-C₆ alkyl, preferably C₁-C₃ alkyl), - NHC(O)(C₁-C₆alkyl), -NHC(O)(phenyl), -N(C₁-C₆alkyl)C(O)(C₁-C₆alkyl), -N(C₁-C₆alkyl)C(O)(phenyl), - C(O)C₁-C₆ alkyl, -C(O)heteroaryl (preferably -C(O)-5- to 7-membered heteroaryl), -C(O)C₁-C₆ alkylphenyl, -C(O)C₁-C₆ haloalkyl, -OC(O)C₁-C₆ alkyl (preferably -OC(O)C₁-C₃ alkyl), -S(O)₂-C₁-C₆ alkyl, -S(O)-C₁-C₆ alkyl, -S(O)₂-phenyl, -S(O)₂-C₁-C₆ haloalkyl, -S(O)₂NH₂, -S(O)₂NH (C₁-C₆ alkyl), -S(O)₂NH(phenyl), - NHS(O)₂(C₁-C₆ alkyl), -NHS(O)₂(phenyl) and -NHS(O)₂(C₁-C₆ haloalkyl), wherein each of the alkyl, cycloalkyl, phenyl, aryl, heterocyclyl, and heteroaryl is optionally further substituted with one or more substituents selected from, but not limitated to, halogen, -OH, -NH₂, cycloalkyl, 3- to 8-membered heterocyclyl, C₁-C₄ alkyl, C₁-C₄ haloalkyl-, -OC₁-C₄ alkyl, -C₁-C₄ alkyl-OH, -C₁-C₄ alkyl-O-C₁-C₄ alkyl, - OC₁-C₄ haloalkyl, -CN, nitro, -C(O)-OH, -C(O)OC₁-C₆ alkyl, -CON(C₁-C₆ alkyl)₂, -CONH(C₁ -C₆ alkyl), - CONH₂ , -NHC(O)(C₁-C₆ alkyl), -NH(C₁-C₆ alkyl)C(O)(C₁-C₆ alkyl), -SO₂(C₁-C₆ alkyl), -SO₂(phenyl), - SO₂(C₁-C₆ haloalkyl), -SO₂NH₂, -SO₂NH(C₁-C₆ alkyl), -SO₂NH(phenyl), -NHSO₂(C₁-C₆ alkyl), - NHSO₂(phenyl), and -NHSO₂(C₁-C₆ haloalkyl). When an atom or group is substituted with a plurality of substituents, the plurality of substituents may be the same or different.

In certain embodiments, the substituents may be independently selected from, but are not limit to, a halogen (such as a chlorine, bromine, fluorine, or iodine), a carboxylic acid (such as -C(=O)-OH), an oxygen (such as =O), a sulfur (such as =S), a hydroxyl (such as -OH), an ester group (such as -C(=O)ORiii or - OC(=O)Riii), an aldehyde group (such as -C(=O)H), a carbonyl (such as -C(=O)Riii, or represented by C=O), an acyl halide (such as -C(=O)Xi, wherein Xi is selected from bromine, fluorine, chlorine, or iodine), a carbonic ester group (such as -OC(=O)ORiii), an alkoxy (such as -ORiii), an acetal (such as -C(ORiii)₂Riii, wherein each ORiii is the same or different and is an alkoxy group), a phosphate (such as P(=O)₄³⁻), a thiol (such as -SH), a sulfoxide (such as -S(=O)Riii), a sulfinic acid (such as -S(=O)OH), a sulfonic acid (such as -S(=O)₂OH), a thioaldehyde (such as -C(=S)H), a sulfate (such as S(=O)₄²⁻), a sulfonyl (such as -S(=O)₂Riii), a sulfinyl (such as -S(=O)Riii), an amide group (such as -C(=O)N(Riii)₂ or -N(Riii)C(=O)Riii), an azido (such as -N₃), a nitro (such as -NO₂), a cyano (such as -CN), an isocyano (such as -NC), an acyloxy (such as -OC(=O)Riii), an amino (such as -NRiii₂, -N(Riii)H or -NH₂), a carbamoyl (such as -OC(=O)NRiii₂, - OC(=O)N(Riii)H or -OC(=O)NH₂), a sulfonamide group (such as -S(=O)₂NRiii₂, -S(=O)₂NRiiiH, - S(=O)₂NH₂, -N(Riii)S(=O)₂Riii, -N(H)S(=O)₂Riii, -N(Riii)S(=O)₂H or -N(H)S(=O)₂H), an alkyl, an alkenyl, an alkynyl, a cyclohydrocarbyl (such as cycloalkyl, cycloalkenyl or cycloalkynyl), a heterocyclohydrocarbyl (such as heterocycloalkyl containing one or more heteroatoms selected from S, N, and O, or heterocycloalkenyl containing one or more heteroatoms selected from S, N, and O), an aryl (such as phenyl, or a fused ring group), a heteroaryl (such as an 8- to 10-membered bicyclic heteroaryl containing from 1 to 4 heteroatoms independently selected from nitrogen, oxygen, or sulfur), -C(=O)SRiii, -C(=N-CN)N(Riii)₂, - C(=N-O-CH₃)N(Riii)₁, -C(=N-SO₂-NH₂)N(Riii)₂, -C(=CH-NO₂)N(Riii)₂, -OC(=O)N(Riii)₂, - CH(NRiii)N(Riii)₂, -C(=O)N(Riii)ORiii, -N(Riii)₂C(=O)ORiii, -OP(=O)(ORiii)₂, -P(=O)(ORiii)₂, - N(ORiii)C(=O)Riii, -N(ORIII)S(=O)₂Riii, -N(ORiii)C(=O)ORiii, -N(ORiii)C(=O)N(Riii)₂, - N(OR111)C(=S)N(RIII)₂, -N(ORiii)C(NRiii)(NRiii)₂, -N(ORiii)C(CHRiii)N(Riii)₂. In any of the foregoing, Riii is a hydrogen, or alkyl, or alkenyl, or alkynyl, or heteroalkyl, or heteroalkenyl, or heteroalkynyl, as defined herein. In some embodiments, Riii is a hydrogen, or C₁-C₁₂ alkyl, or C₁-C₁₂ alkenyl, or C₁-C₁₂ alkynyl, or C₁-C₁₂ heteroalkyl, or C₁-C₁₂ heteroalkenyl, or C₁-C₁₂ heteroalkynyl, as defined herein.

In certain embodiments, the substituents itself may be further substituted with, for example, one or more substituents as defined herein. For example, the C₁-C₆ alkyl as a substituent may be further substituted with one or more substituents as define herein.

As use herein, that term "halo" or "halogen" group is defined to include F, Cl, Br, or I.

As used herein, "pharmaceutically acceptable salt" refers to an acid-addition salt or a base-addition salt of a compound of the present disclosure which retains the biological effectiveness and properties of the compound of the present disclosure and is not typically biologically or otherwise undesirable. In many cases, the compound of the present invention can form an acid and/or base salt due to the presence of an amino and/or carboxyl group or a similar group.

Pharmaceutically acceptable acid addition salts can be formed from the compound of the present disclosure and inorganic and/or organic acids, the inorganic acids being such as, but not limited to, hydrochloric acid, hydrobromic acid, sulfuric acid, nitric acid, and phosphoric acid; and the organic acids being such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphanic acid, camphor-10-sulfonic acid, capric acid, hexanoic acid, octanoic acid, carbonic acid, cinnamic acid, citric acid, cyclamic acid, dodecyl sulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxoglutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, and undecylenic acid.

Pharmaceutically acceptable base addition salts can be formed from the compound of the present disclosure and inorganic and/or organic bases. Salts derived from inorganic bases include, but are not limited to, sodium, potassium, lithium, ammonium, calcium, magnesium, iron, zinc, copper, manganese, aluminum salts, etc. Preferred inorganic salts are ammonium, sodium, potassium, calcium, and magnesium salts. Salts derived from organic bases include, but are not limited to, salts of primary amines, secondary amines, tertiary amines, substituted amines (including naturally occurring substituted amines), cyclic amines, and basic ion exchange resins as following: such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucosamine, theobromine, triethanolamine, tromethamine, purine, piperazine, piperidine, N-ethylpiperidine, and polyamine resins. Particularly preferred organic bases are isopropylamine, diethylamine, ethanolamine, trimethylamine, dicyclohexylamine, choline, and caffeine.

A numerical range stated herein should be understood to encompass the boundary values and any and all subranges contained therein. For example, a range of "from 1 to 10" should be understood to include not only the explicitly recited values of 1 and 10, but also any individual values in the range of from 1 to 10 (e.g., 2, 3, 4, 5, 6, 7, 8, and 9) and subranges (e.g., from 1 to 2, 1.5 to 2.5, 1 to 3, 1.5 to 3.5, 2.5 to 4, 3 to 4.5, etc.). This principle also applies to ranges that use only one value as a minimum or maximum.

As use herein, that term "isomer" means different compounds having the same molecular formula. "Stereoisomers" are isomers that differ only in the way the atoms are arranged in space. "Atropisomers" are stereoisomers resulting from hindered rotation about a single bond. "Enantiomers" are a pair of stereoisomers that are non-overlapping mirror images of each other. A mixture of any ratio of a pair of enantiomers may be referred to as a "racemic" mixture. "Diastereoisomers" are stereoisomers that have at least two asymmetric atoms and are not mirror-images of one another. "Tautomers" refer to isomeric forms of a compound that are in equilibrium with each other. The concentration of the isomeric form will depend on the environment in which the compound is found and may vary, for example, depending on whether the compound is a solid or in an organic or aqueous solution.

In certain embodiments, "stereoisomers" may also include the E and Z isomers, or mixtures thereof, as well as the *cis* and *trans* isomers, or mixtures thereof.

### Amino lipid compound

In one aspect, the present disclosure provides an amino lipid compound represented by the following formula (I): 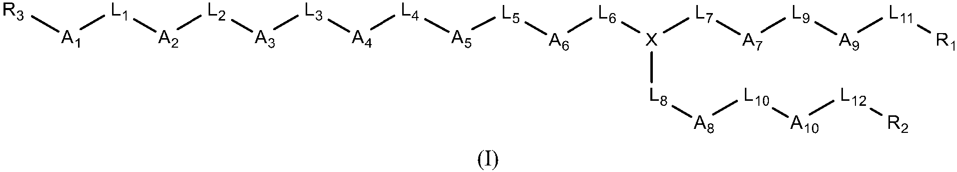 or a pharmaceutically acceptable salt thereof, or a stereoisomer thereof,
wherein:
X is C or N;
L₁, L₂, L₃, L₄, L₅, and L₆ are each independently C₁-C₆ hydrocarbylene or a bond;
L₇, L₈, L₉, L₁₀, L₁₁, and L₁₂ are each independently C₁-C₁₈ hydrocarbylene or a bond;
A₁, A₂, A₃, A₄, A₅, A₆, A₇, A₈, A₉, and A₁₀ are each independently -N(R₄)-, -C(=O)O-, -OC(=O)-, - OC(=O)O-, -O-, -C(=O)-, -S-, -C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, -N(R₄)C(=O)O-, -N(R₄)C(=O)N(R₄)-, -SC(=O)N(R₄)-, -N(R₄)C(=O)S-, - N(C(=O)L₁₃OR4)-, -N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, -OP(=O)(OH)O-, -OP(=O)(H)O-, -OS(=O)₂NH-, -NHS(=O)₂O-, or a bond;
each L₁₃ is independently C₁-C₈ hydrocarbylene or a bond;
R₁ and R₂ are each independently H, C₁-C₂₄ hydrocarbyl, C₁-C₂₄ heterohydrocarbyl, A₁₁R₅, or - C(R₆)(OL₁₄A₁₂R₇)₂;
each A₁₁ is independently -C(=O)O-, -OC(=O)-, -OC(=O)O-, -O-, -C(=O)-, -S-, -C(=O)S-, -SC(=O)-, - C(=O)N(R₄)-, -N(R₄)C(=O)-, -OC(=O)N(R₄)-, -N(R₄)C(=O)O-, -N(R₄)C(=O)N(R₄)-, -OC(=O)S-, - SC(=O)O-, -SC(=O)S-, -SC(=O)N(R₄)-, -N(R₄)C(=O)S-, -N(C(=O)L₁₃OR₄)-, -N(C(=O)L₁₃SR₄)-, - N(C(=O)R₄), -N(L₁₃OR₄)-, -N(L₁₃SR₄)-, -N(R₄)-, -OS(=O)O-, -OS(=O)₂O-, -OP(=O)O-, -OP(=O)(OH)O-, - OP(=O)(H)O-, -OS(=O)₂NH-, -NHS(=O)₂O-, or a benzene ring;
each A₁₂ is independently -C(=O)O-, -OC(=O)-, -OC(=O)O-, -O-, -C(=O)-, -S-, -C(=O)S-, -SC(=O)-, - C(=O)N(R₄)-, -N(R₄)C(=O)-, -OC(=O)N(R₄)-, -N(R₄)C(=O)O-, -N(R₄)C(=O)N(R₄)-, -OC(=O)S-, - SC(=O)O-, -SC(=O)S-, -SC(=O)N(R₄)-, -N(R₄)C(=O)S-, -N(C(=O)L₁₃OR₄)-, -N(C(=O)L₁₃SR₄)-, - N(C(=O)R₄), -N(L₁₃OR₄)-, -N(L₁₃SR₄)-, -N(R₄)-, -OS(=O)O-, -OS(=O)₂O-, -OP(=O)O-, -OP(=O)(OH)O-, - OP(=O)(H)O-, -OS(=O)₂NH-, -NHS(=O)₂O-, a benzene ring, or a bond;
each R₄ is independently H, C₁-C₆ hydrocarbyl, or C₁-C₆ heterohydrocarbyl containing O or S;
each R₅ is independently H, C₁-C₂₄ hydrocarbyl, or C₁-C₂₄ heterohydrocarbyl containing O or S;
each R₆ is independently H or C₁-C₆ hydrocarbyl;
each L₁₄ is independently C₁-C₁₈ hydrocarbylene or a bond;
each R₇ is independently C₁-C₂₄ hydrocarbyl or C₁-C₁₄ heterohydrocarbyl containing O or S;
R3 is -L₁₅-Z, 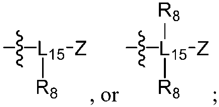
L₁₅ is C₁-C₁₂ hydrocarbylene or a bond;
Z is H, a carbocyclic ring, a heterocyclic ring, -CN, -OR₈, -OL₁₆N(R₈)₂, -C(=O)R₈, -C(=O)SR₈, - OC(=O)R₈, -OC(=O)OR₈, -OL₁₆OR₈, -N(R₈)₂, -C(=O)N(R₈)₂, -C(=S)N(R₈)₂, -S(=O)₂R₈, -S(=O)₂N(R₈)₂, - OC(=O)N(R₈)₂, -C(NR₈)N(R₈)₂, -C(NR₈)R₈, -C(=O)N(R₈)OR₈, -CH(R₈)N(R₈)C(=O)OR₈, -C(R₈)₃, - N(R₈)C(=O)R₈, -N(R₈)C(=O)OR₈, -N(R₈)S(=O)₂R₈, -N(R₈)C(=O)N(R₈)₂, -N(R₈)C(=S)N(R₈)₂, - N(R₈)C(NR₈)N(R₈)₂, -N(R₈)C(CHR₈)N(R₈)₂, -N(OR₈)C(=O)R₈, -N(OR₈)S(=O)₂R₈, -N(OR₈)C(=O)OR₈, - N(OR₈)C(=O)N(R₈)₂, -N(ORs)C(=S)N(R₈)₂, -N(OR₈)C(NR₈)N(R₈)₂, -N(OR₈)C(CHR₈)N(R₈)₂, - OP(=O)(OR₈)₂, -P(=O)(OR₈)₂, -C(=N-CN)N(R₈)₂, -C(=N-O-CH₃)N(R₈)₂, -C(=N-SO₂-NH₂)N(R₈)₂, or - C(=CH-NO₂)N(R₈)₂;
each R₈ is independently hydrogen, C₁-C₁₂ alkyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds; and
each L₁₆ is independently C₁-C₁₈ hydrocarbylene or a bond.

In some embodiments, the present disclosure provides the amino lipid compound of formula (I), or a pharmaceutically acceptable salt thereof, as described above, wherein Z is 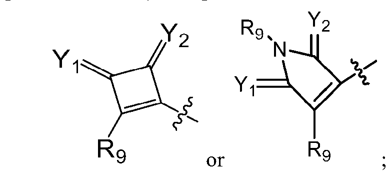 wherein:
Y₁ and Y₂ are each independently O or S;
each R₉ is independently H, halogen, -R_{b}, -N(R_{b})₂, cyano, -N₃, -C(=O)OR_{b}, -OC(=O)R_{b}, -OR_{b}, -SR_{b}, - S(-O)R_{b}, -S(=O)OR_{b}, -S(=O)₂OR_{b}, -N(R_{b})₂, -N(R_{b})S(=O)₂R_{b}, -NHRₐN(R_{b})₂, -NHRₐORₐN(R_{b})₂, -NHRₐOR_{b}, or -N(RₐOR_{b})₂;
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In some embodiments, the present disclosure provides the amino lipid compound of formula (I), or a pharmaceutically acceptable salt thereof, as described above, wherein Z is

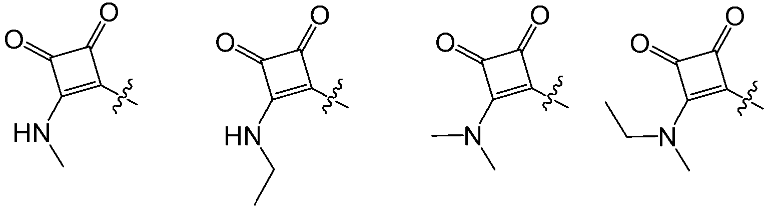

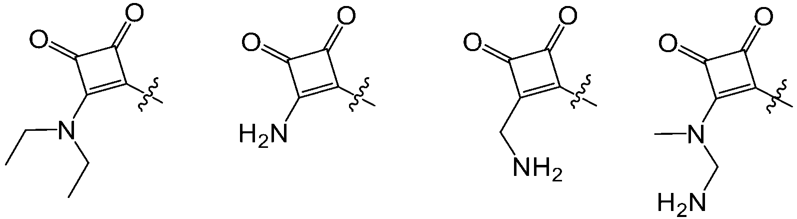

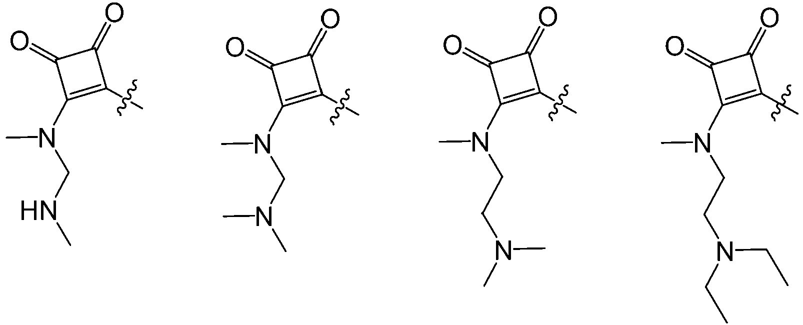

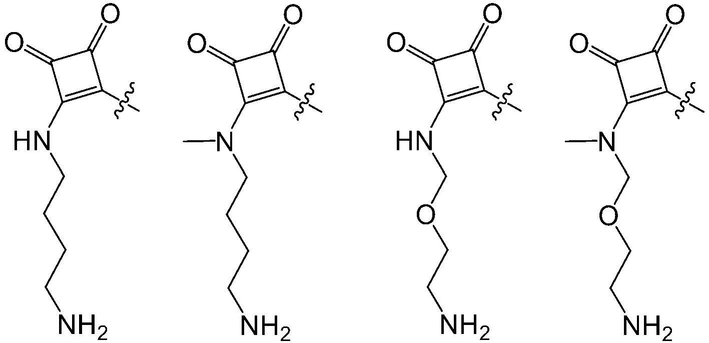

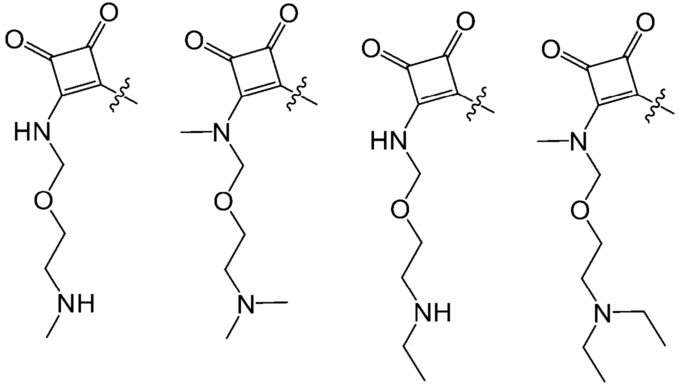

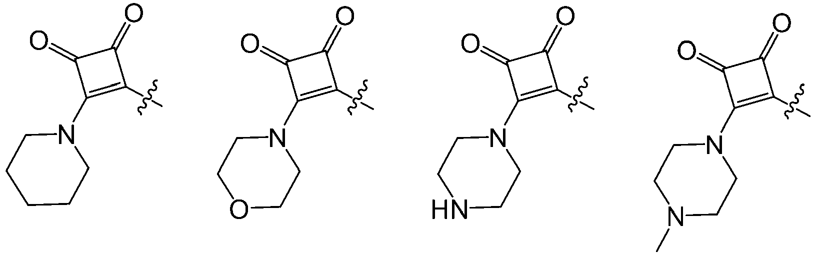

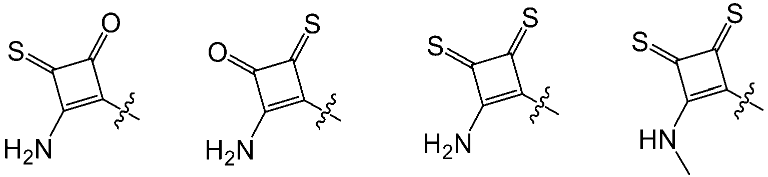

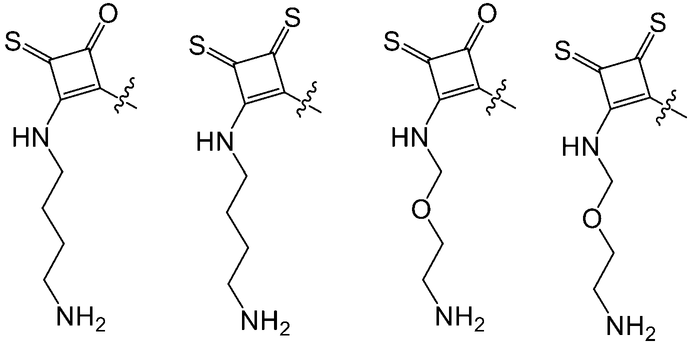

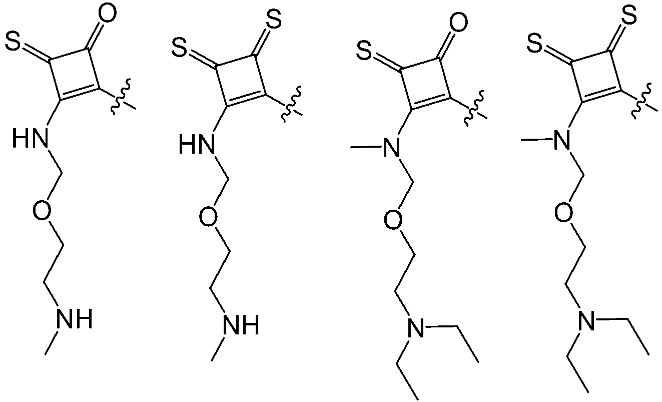

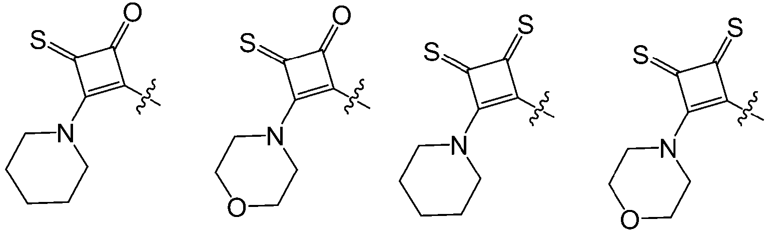

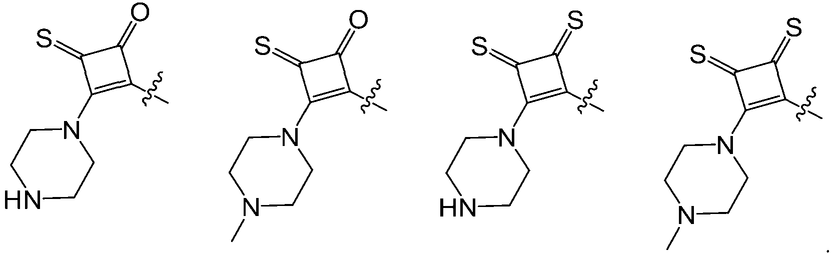

In some embodiments, the present disclosure provides the amino lipid compound of formula (I), or a pharmaceutically acceptable salt thereof, as described above, wherein R₃ is 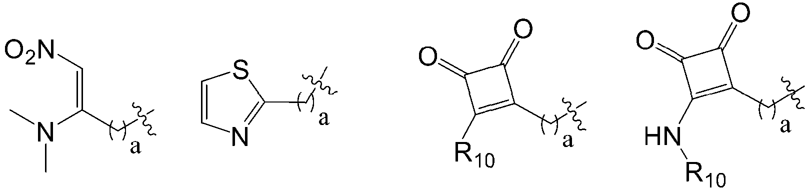 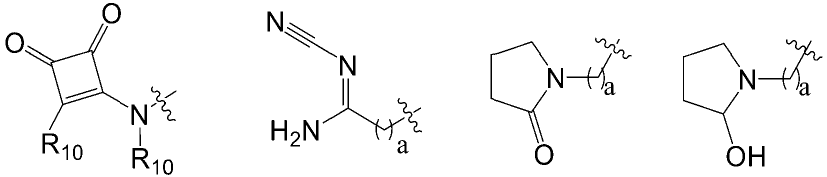 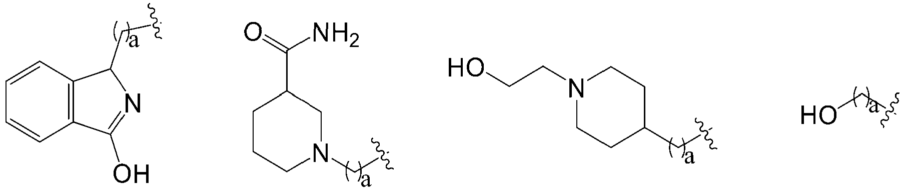 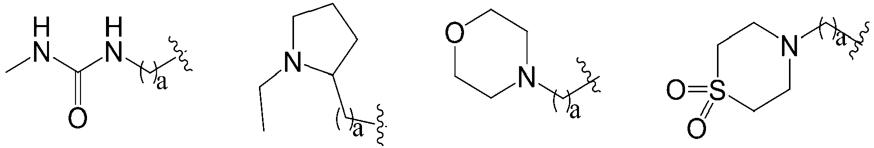 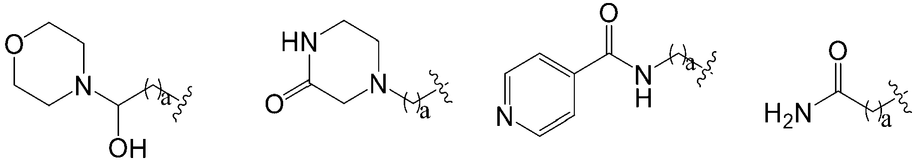 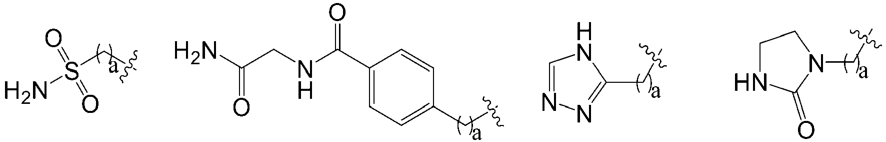 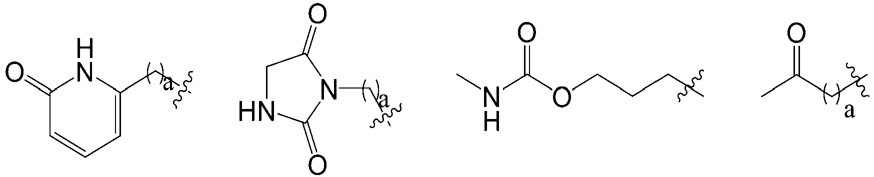 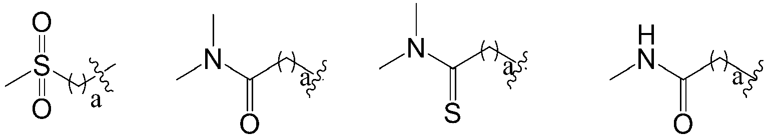 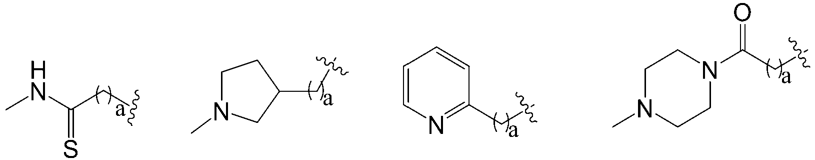 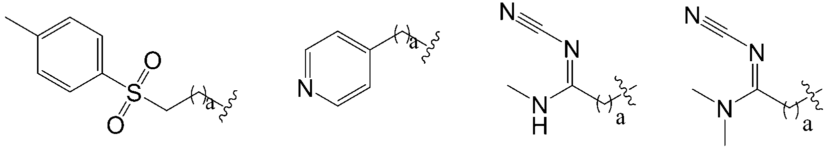 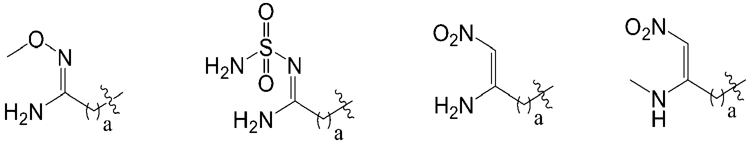 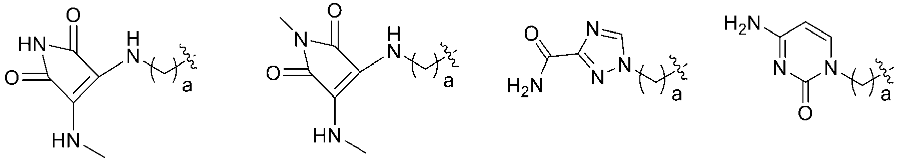 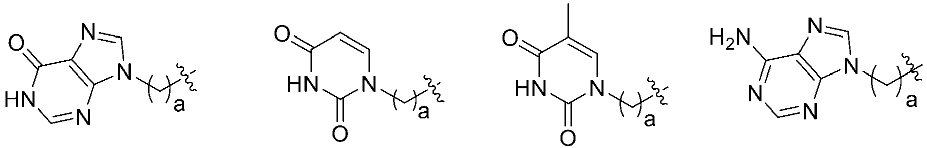
wherein: a is 0, 1, 2, 3, 4, or 5; and
each R₁₀ is independently H or C₁-C₃ hydrocarbyl, preferably is C₁-C₃ hydrocarbyl. In some embodiments, each is independently C₁, C₂, or C₃ alkyl, or C₂ or C₃ alkenyl, or C₂ or C₃ alkynyl.

In some embodiments, the present disclosure provides the amino lipid compound of formula (I), or a pharmaceutically acceptable salt thereof, as described above, wherein L₁, L₂, L₃, L₄, L₅, L₆, L₇, L₈, L₉, L₁₀, L₁₁, L₁₂, L₁₃, L₁₄, L₁₅, and L₁₆ are each independently substituted with hydroxyl, halogen, alkyl, alkenyl, alkynyl, cyano, oxygen, sulfur, nitrogen, an ester group, aryl, cycloalkyl, cycloalkenyl, amido, alkoxyl, or alkylthio.

In some embodiments, L₁, L₂, L₃, L₄, L₅, L₆, L₇, L₈, L₉, L₁₀, L₁₁, L₁₂, L₁₃, L₁₄, L₁₅, and L₁₆ are each independently substituted with hydroxyl, halogen, O, S, N, cyano, cyclohydrocarbyl, aryl, heterocyclyl, -R_{b}, -RₐOR_{b}> -RₐSR_{b}, -RₐOC(=O)OR_{b}, -RₐOC(=O)SR_{b}, -RₐC(=O)N(R_{b})₂, -RₐOC(=O)R_{b}, or -RₐSR_{b};
when L₁, L₂, L₃, L₄, L₅, L₆, L₇, L₈, L₉, L₁₀, L₁₁, L₁₂, L₁₃, L₁₄, L₁₅, and L₁₆ are each independently substituted with -R_{b}, -RₐOR_{b}, -RₐSR_{b}, -RₐOC(=O)OR_{b}, -RₐOC(=O)SR_{b}, -RₐC(=O)N(R_{b})₂, -RₐOC(=O)R_{b}, or - RₐSR_{b}, Rₐ and/or R_{b} may be attached to a C atom on L₁, L₂, L₃, L₄, L₅, L₆, L₇, L₈, L₉, L₁₀, L₁₁, L₁₂, L₁₃, L₁₄, L₁₅, or L₁₆ to form a three-, four-, five-, or six-membered ring;
wherein:
   each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing S or O, C₂-C₁₂ heteroalkenylene containing S or O with 1, 2, 3, or more double bonds, or C₂-C₁₂ heteroalkynylene containing S or O with 1, 2, 3, or more triple bonds; and
   each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing S or O, C₂-C₁₂ heteroalkenyl containing S or O with 1, 2, 3, or more double bonds, or C₂-C₁₂ heteroalkynyl containing S or O with 1, 2, 3, or more triple bonds.

In another aspect, the present disclosure provides an amino lipid compound represented by the following formula (IA): 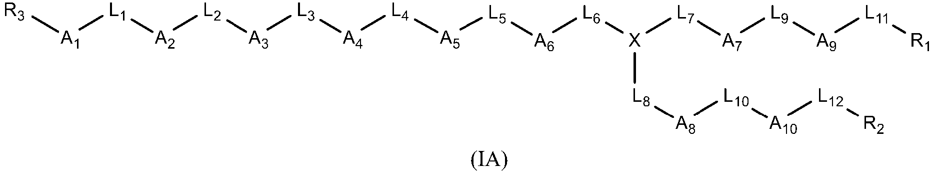 or a pharmaceutically acceptable salt thereof, or a stereoisomer thereof,
wherein:
X is C, N, or CR₁₃;
L₁, L₂, L₃, L₄, L₅, and L₆ are each independently C₁-C₆ hydrocarbylene, C₃-C₈ carbocyclic ring, heterocyclic ring, C₁-C₆ heterohydrocarbylene, or a bond;
L₇, L₈, L₉, L₁₀, L₁₁, and L₁₂ are each independently C₁-C₁₈ hydrocarbylene, C₁-C₁₈ heterohydrocarbylene, or a bond;
A₁, A₂, A₃, A₄, A₅, A₆, A₇, A₈, A₉, and A₁₀ are each independently -N(R₄)-, -C(=O)O-, -OC(=O)-, - OC(=O)O-, -O-, -C(=O)-, -S-, -C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, -N(R₄)C(=O)O-, -N(R₄)C(=O)N(R₄)-, -SC(=O)N(R₄)-, -N(R₄)C(=O)S-, - N(C(=O)L₁₃OR₄)-, -N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, -OP(=O)(OH)O-, -OP(=O)(H)O-, -OS(=O)₂NH-, -NHS(=O)₂O-, -OC(=O)C(=O)O-, - N(R₄)C(=O)C(=O)O-, -OC(=O)C(=O)N(R₄)-, -N(R₄)C(=O)C(=O)N(R₄)-, 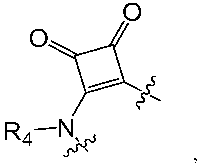 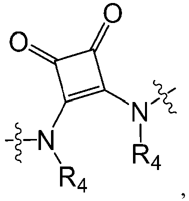 or a bond;
each L₁₃ is independently C₁-C₈ hydrocarbylene, or C₁-C₈ heterohydrocarbylene, or a bond;
R₁ and R₂ are each independently H, C₁-C₂₄ hydrocarbyl, C₁-C₂₄ heterohydrocarbyl, A₁₁R₅, - C(R₆)(OL₁₄A₁₂R₇)₂, -C(R₆)(SL₁₄A₁₂R₇)₂, or -C(R₆)(SL₁₄A₁₂R₇)(OL₁₄A₁₂R₇);
each A₁₁ is independently -C(=O)O-, -OC(=O)-, -OC(=O)O-, -O-, -C(=O)-, -S-, -C(=O)S-, -SC(=O)-, - C(=O)N(R₄)-, -N(R₄)C(=O)-, -OC(=O)N(R₄)-, -N(R₄)C(=O)O-, -N(R₄)C(=O)N(R₄)-, -OC(=O)S-, - SC(=O)O-, -SC(=O)S-, -SC(=O)N(R₄)-, -N(R₄)C(=O)S-, -N(C(=O)L₁₃OR₄)-, -N(C(=O)L₁₃SR₄)-, - N(C(=O)R₄), -N(L₁₃OR₄)-, -N(L₁₃SR₄)-, -N(R₄)-, -OS(=O)O-, -OS(=O)₂O-, -OP(=O)O-, -OP(=O)(OH)O-, - OP(=O)(H)O-, -OS(=O)₂NH-, -NHS(=O)₂O-, or a benzene ring;
each A₁₂ is independently -C(=O)O-, -OC(=O)-, -OC(=O)O-, -O-, -C(=O)-, -S-, -C(=O)S-, -SC(=O)-, - C(=O)N(R₄)-, -N(R₄)C(=O)-, -OC(=O)N(R₄)-, -N(R₄)C(=O)O-, -N(R₄)C(=O)N(R₄)-, -OC(=O)S-, - SC(=O)O-, -SC(=O)S-, -SC(=O)N(R₄)-, -N(R₄)C(=O)S-, -N(C(=O)L₁₃OR₄)-, -N(C(=O)L₁₃SR₄)-, - N(C(=O)R₄), -N(L₁₃OR₄)-, -N(L₁₃SR₄)-, -N(R₄)-, -OS(=O)O-, -OS(=O)₂O-, -OP(=O)O-, -OP(=O)(OH)O-, - OP(=O)(H)O-, -OS(=O)₂NH-, -NHS(=O)₂O-, a benzene ring, or a bond;
each R₄ is independently H, C₁-C₆ hydrocarbyl, or C₁-C₆ heterohydrocarbyl;
each R₅ is independently H, C₁-C₂₄ hydrocarbyl, or C₁-C₂₄ heterohydrocarbyl;
each R₆ is independently H, C₁-C₆ hydrocarbyl, or C₁-C₆ heterohydrocarbyl;
each L₁₄ is independently C₁-C₁₈ hydrocarbylene, C₁-C₁₈ heterohydrocarbylene, or a bond;
each R₇ is independently C₁-C₂₄ hydrocarbyl or C₁-C₂₄ heterohydrocarbyl;
R₃ is -L₁₅-Z;
L₁₅ is C₁-C₁₂ hydrocarbylene, C₁-C₁₂ heterohydrocarbylene, or a bond;
Z is H, a carbocyclic ring, a heterocyclic ring, -CN, -OR₈, -OL₁₆N(R₈)₂, -C(=O)OR₈, -C(=O)R₈, - C(=O)SR₈, -OC(=O)R₈, -OC(=O)OR₈, -OL₁₆OR₈, -N(R₈)₂, -C(=O)N(R₈)₂, -C(=S)N(R₈)₂, -S(=O)₂R₈, - S(=O)₂N(R₈)₂, -OC(=O)N(R₈)₂, -C(=NRs)N(R₈)₂, -C(=NR₈)R₈, -C(=O)N(R₈)OR₈, -CH(R₈)N(R₈)C(=O)OR₈, -C(R₈)₃, -N(R₈)C(=O)R₈, -N(R₈)C(=O)OR₈, -N(R₈)S(=O)₂R₈, -N(R₈)C(=O)N(R₈)₂, -N(R₈)C(=S)N(R₈)₂, - N(R₈)C(=NR₈)N(R₈)₂, -N(R₈)C(=CHR₈)N(R₈)₂, -N(OR₈)C(=O)R₈, -N(OR₈)S(=O)₂R₈, -N(OR₈)C(=O)OR₈, -N(OR₈)C(=O)N(R₈)₂, -N(OR₈)C(=S)N(R₈)₂, -N(OR₈)C(NR₈)N(Rg)₂, -N(OR₈)C(=CHR₈)N(R₈)₂, - OP(=O)(OR₈)₂, -P(=O)(OR₈)₂, -C(=N-CN)N(R₈)₂, -C(=N-O-CH₃)N(R₈)₂, -C(=N-SO₂-NH₂)N(R₈)₂, - C(=CH-NO₂)N(R₈)₂, -C(=O)OR₁₁, -N(R₈)R₁₁, -N(R₈)S(=O)₂R₁₁, -N(R₈)C(=NR₁₂)N(R₈)₂, - N(R₈)C(=CHR₁₂)N(R₈)₂, -N(OR₈)C(=NR₁₂)N(R₈)₂, -N(OR₈)C(=CHR₁₂)N(R₈)₂, -C(=NR₁₂)N(R₈)₂, - C(=NR₁₂)R₈, or -C(R₈)N(R₈)₂C(=O)OR₈;
each R₈ is independently hydrogen, or C₁-C₁₂ alkyl, or C₂-C₁₂ alkenyl with 1, 2, 3, or more double bonds, or C₂-C₁₂ alkynyl with 1, 2, 3, or more triple bonds, or C₁-C₁₂ heteroalkyl, or C₂-C₁₂ heteroalkenyl with 1, 2, 3, or more double bonds, or C₂-C₁₂ heteroalkynyl with 1, 2, 3, or more triple bonds;
each R₁₁ is independently C₃₋₆ carbocyclic ring or heterocyclic ring;
each R₁₂ is independently H, CN, NO₂, C₁₋₆ alkyl, -OR, -S(O)₂R, -S(O)₂N(R)₂, C₂₋₆ alkenyl, C₃₋₆ carbocyclic ring or heterocyclic ring;
each L₁₆ is independently C₁-C₁₈ hydrocarbylene, C₁-C₁₈ heterohydrocarbylene, or a bond; and
R₁₃ is H, C₁-C₆ hydrocarbyl, or C₁-C₆ heterohydrocarbyl.

The present disclosure provides the amino lipid compound of formula (IA), or a pharmaceutically acceptable salt thereof, or a stereoisomer thereof, as described above, including one or more of the following features, where applicable.

In some embodiments, the present disclosure provides the amino lipid compound of formula (IA), or a pharmaceutically acceptable salt thereof, or a stereoisomer thereof, as described above, wherein L₁, L₂, L₃, L₄, L₅, and L₆ are each independently C₁, C₂, C₃, C₄, C₅, or C₆ hydrocarbylene, or C₁, C₂, C₃, C₄, C₅, or C₆ heterohydrocarbylene.

In some embodiments, the present disclosure provides the amino lipid compound of formula (IA), or a pharmaceutically acceptable salt thereof, or a stereoisomer thereof, as described above, wherein L₁ is C₁-C₆ alkylene, or C₁-C₆ heteroalkylene containing O, N, or S, or a bond.

In some embodiments, L₁ is C₄, C₅, or C₆ alkylene.

In some embodiments, L₁ is C₁, C₂, C₃, or C₄ alkylene.

In some embodiments, L₁ is C₁-C₃ alkylene. In some other embodiments, L₁ is C₄ alkylene.

In some embodiments, L₁ is a bond.

In some embodiments, the present disclosure provides the amino lipid compound of formula (IA), or a pharmaceutically acceptable salt thereof, or a stereoisomer thereof, as described above, wherein L₂ is C₁-C₆ alkylene, or C₁-C₆ heteroalkylene containing O, N, or S, or a bond.

In some embodiments, L₂ is C₅ or C₆ alkylene.

In some embodiments, L₂ is C₁, C₂, C₃, or C₄ alkylene.

In some embodiments, L₂ is a bond.

In some embodiments, the present disclosure provides the amino lipid compound of formula (IA), or a pharmaceutically acceptable salt thereof, or a stereoisomer thereof, as described above, wherein L₃ is C₁-C₆ alkylene, or C₁-C₆ heteroalkylene containing O, N, or S, or a bond.

In some embodiments, L₃ is C₄, C₅, or C₆ alkylene.

In some embodiments, L₃ is C₂ or C₃ alkylene.

In some embodiments, L₃ is C₁ alkylene or a bond.

In some embodiments, the present disclosure provides the amino lipid compound of formula (IA), or a pharmaceutically acceptable salt thereof, or a stereoisomer thereof, as described above, wherein L₄ is a bond.

In some embodiments, L₄ is C₄, C₅, or C₆ alkylene, or C₄, C₅, or C₆ heteroalkylene containing O, N, or S.

In some embodiments, L₄ is C₂ or C₃ alkylene, or C₂ or C₃ heteroalkylene containing O, N, or S.

In some embodiments, L₄ is C₁ alkylene, or C₁ heteroalkylene containing O, N, or S, or a bond.

In some embodiments, the present disclosure provides the amino lipid compound of formula (IA), or a pharmaceutically acceptable salt thereof, or a stereoisomer thereof, as described above, wherein L₅ is a bond.

In some embodiments, L₅ is C₄, C₅, or C₆ alkylene, or C₄, C₅, or C₆ heteroalkylene containing O, N, or S.

In some embodiments, L₅ is C₂ or C₃ alkylene, or C₂ or C₃ heteroalkylene containing O, N, or S.

In some embodiments, L₅ is C₁ alkylene, or C₁ heteroalkylene containing O, N, or S, or a bond.

In some embodiments, the present disclosure provides the amino lipid compound of formula (IA), or a pharmaceutically acceptable salt thereof, or a stereoisomer thereof, as described above, wherein L₆ is a bond.

In some embodiments, L₆ is C₄, C₅, or C₆ alkylene, or C₄, C₅, or C₆ heteroalkylene containing O, N, or S.

In some embodiments, L₆ is C₂ or C₃ alkylene, or C₂ or C₃ heteroalkylene containing O, N, or S.

In some embodiments, L₆ is C₁ alkylene, or C₁ heteroalkylene containing O, N, or S, or a bond.

In some embodiments, the present disclosure provides the amino lipid compound of formula (IA), or a pharmaceutically acceptable salt thereof, or a stereoisomer thereof, as described above, wherein L₇, L₈, L₉, L₁₀, L₁₁, and L₁₂ are each independently C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ hydrocarbylene, or C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heterohydrocarbylene.

In some embodiments, the present disclosure provides the amino lipid compound of formula (IA), or a pharmaceutically acceptable salt thereof, or a stereoisomer thereof, as described above, wherein L₇ is C₁-C₁₈ alkylene, or C₁-C₁₈ heteroalkylene, or a bond.

In some embodiments, L₇ is C₁, C₂, C₃, C₄, C₅, C₆, C₇, C₈, C₉, C₁₀ C₁₁, C₁₂, C₁₃, C₁₄, C₁₅, or C₁₆ alkylene, or C₁, C₂, C₃, C₄, C₅, C₆, C₇, C₈, C₉, C₁₀, C₁₁, C₁₂, C₁₃, C₁₄, C₁₅, or C₁₆ heteroalkylene containing O, N, or S, or a bond.

In some embodiments, L₇ is C₁-C₁₄ alkylene.

In some embodiments, L₇ is C₄-C₁₁ alkylene.

In some embodiments, L₇ is C₂ or C₃ alkylene.

In some embodiments, L₇ is C₆-C₉ alkylene.

In some embodiments, L₇ is C₇ or C₈ alkylene.

In some embodiments, L₇ is C₁-C₄ alkylene.

In some embodiments, L₇ is a bond.

In some embodiments, the present disclosure provides the amino lipid compound of formula (IA), or a pharmaceutically acceptable salt thereof, or a stereoisomer thereof, as described above, wherein L₈ is C₁-C₁₈ alkylene, or C₁-C₁₈ heteroalkylene, or a bond.

In some embodiments, L₈ is C₁, C₂, C₃, C₄, C₅, C₆, C₇, C₈, C₉, C₁₀ C₁₁, C₁₂, C₁₃, C₁₄, C₁₅, or C₁₆ alkylene, or C₁, C₂, C₃, C₄, C₅, C₆, C₇, C₈, C₉, C₁₀, C₁₁, C₁₂, C₁₃, C₁₄, C₁₅, or C₁₆ heteroalkylene containing O, N or S, or a bond.

In some embodiments, L₈ is C₁-C₁₄ alkylene.

In some embodiments, L₈ is C₄-C₁₁ alkylene.

In some embodiments, L₈ is C₂ or C₃ alkylene.

In some embodiments, L₈ is C₆-C₉ alkylene.

In some embodiments, L₈ is C₇ or C₈ alkylene.

In some embodiments, L₈ is C₁-C₄ alkylene.

In some embodiments, L₈ is a bond.

In some embodiments, the present disclosure provides the amino lipid compound of formula (IA), or a pharmaceutically acceptable salt thereof, or a stereoisomer thereof, as described above, wherein L₉ is C₁-C₁₈ alkylene, or C₁-C₁₈ heteroalkylene, or a bond.

In some embodiments, L₉ is C₁, C₂, C₃, C₄, C₅, C₆, C₇, C₈, C₉, C₁₀ C₁₁, C₁₂, C₁₃, C₁₄, C₁₅, or C₁₆ alkylene, or C₁, C₂, C₃, C₄, C₅, C₆, C₇, C₈, C₉, C₁₀, C₁₁, C₁₂, C₁₃, C₁₄, C₁₅, or C₁₆ heteroalkylene containing O, N or S, or a bond.

In some embodiments, L₉ is C₁-C₁₄ alkylene.

In some embodiments, L₉ is C₁-C₈ alkylene.

In some embodiments, L₉ is C₃-C₆ alkylene.

In some embodiments, L₉ is C₂ alkylene.

In some embodiments, L₉ is C₃ alkylene.

In some embodiments, L₉ is C₅ or C₆ alkylene.

In some embodiments, L₉ is a bond.

In some embodiments, the present disclosure provides the amino lipid compound of formula (IA), or a pharmaceutically acceptable salt thereof, or a stereoisomer thereof, as described above, wherein L₁₀ is C₁-C₁₈ alkylene, or C₁-C₁₈ heteroalkylene, or a bond.

In some embodiments, L₁₀ is C₁, C₂, C₃, C₄, C₅, C₆, C₇, C₈, C₉, C₁₀, C₁₁, C₁₂, C₁₃, C₁₄, C₁₅, or C₁₆ alkylene, or C₁, C₂, C₃, C₄, C₅, C₆, C₇, C₈, C₉, C₁₀, C₁₁, C₁₂, C₁₃, C₁₄, C₁₅, or C₁₆ heteroalkylene containing O, N or S, or a bond.

In some embodiments, L₁₀ is C₁-C₁₄ alkylene.

In some embodiments, L₁₀ is C₁-C₈ alkylene.

In some embodiments, L₁₀ is C₃-C₆ alkylene.

In some embodiments, L₁₀ is C₂ alkylene.

In some embodiments, L₁₀ is C₃ alkylene.

In some embodiments, L₁₀ is C₅ or C₆ alkylene.

In some embodiments, L₁₀ is a bond.

In some embodiments, the present disclosure provides the amino lipid compound of formula (IA), or a pharmaceutically acceptable salt thereof, or a stereoisomer thereof, as described above, wherein L₁₁ is C₁-C₁₈ alkylene, or C₁-C₁₈ heteroalkylene, or a bond.

In some embodiments, L₁₁ is C₁, C₂, C₃, C₄, C₅, C₆, C₇, C₈, C₉, C₁₀, C₁₁, C₁₂, C₁₃, C₁₄, C₁₅, or C₁₆ alkylene, or C₁, C₂, C₃, C₄, C₅, C₆, C₇, C₈, C₉, C₁₀, C₁₁, C₁₂, C₁₃, C₁₄, C₁₅, or C₁₆ heteroalkylene containing O, N or S, or a bond.

In some embodiments, L₁₁ is C₁-C₁₄ alkylene.

In some embodiments, L₁₁ is C₁-C₈ alkylene.

In some embodiments, L ₁₁ is C₂ alkylene.

In some embodiments, L₁₁ is C₃-C₆ alkylene.

In some embodiments, L₁₁ is C₃ alkylene.

In some embodiments, L₁₁ is C₅ or C₆ alkylene.

In some embodiments, L₁₁ is a bond.

In some embodiments, the present disclosure provides the amino lipid compound of formula (IA), or a pharmaceutically acceptable salt thereof, or a stereoisomer thereof, as described above, wherein L₁₂ is C₁-C₁₈ alkylene, or C₁-C₁₈ heteroalkylene, or a bond.

In some embodiments, L₁₂ is C₁, C₂, C₃, C₄, C₅, C₆, C₇, C₈, C₉, C₁₀, C₁₁, C₁₂, C₁₃, C₁₄, C₁₅, or C₁₆ alkylene, or C₁, C₂, C₃, C₄, C₅, C₆, C₇, C₈, C₉, C₁₀, C₁₁, C₁₂, C₁₃, C₁₄, C₁₅, or C₁₆ heteroalkylene containing O, N or S, or a bond.

In some embodiments, L₁₂ is C₁-C₁₄ alkylene.

In some embodiments, L₁₂ is C₁-C₈ alkylene.

In some embodiments, L₁₂ is C₂ alkylene.

In some embodiments, L₁₂ is C₃-C₆ alkylene.

In some embodiments, L₁₂ is C₃ alkylene.

In some embodiments, L₁₂ is C₅ or C₆ alkylene.

In some embodiments, L₁₂ is a bond.

In some embodiments, the present disclosure provides the amino lipid compound of formula (IA), or a pharmaceutically acceptable salt thereof, or a stereoisomer thereof, as described above, wherein A₁, A₂, A₃, A₄, A₅, or A₆ are each independently -N(R₄)-.

In some embodiments, the present disclosure provides the amino lipid compound of formula (IA), or a pharmaceutically acceptable salt thereof, or a stereoisomer thereof, as described above, wherein each R₄ is independently C₁, C₂, C₃, C₄, C₅, or C₆ hydrocarbyl, or C₁, C₂, C₃, C₄, C₅, or C₆ heterohydrocarbyl.

In some embodiments, the present disclosure provides the amino lipid compound of formula (IA), or a pharmaceutically acceptable salt thereof, or a stereoisomer thereof, as described above, wherein each R₄ is independently C₁, C₂, C₃, C₄, C₅, or C₆ alkyl, or C₁, C₂, C₃, C₄, C₅, or C₆ heteroalkyl containing O, N, or S.

In some embodiments, each R₄ is independently a straight C₁, C₂, C₃, C₄, C₅, or C₆ alkyl, or a straight C₁, C₂, C₃, C₄, C₅, or C₆ heteroalkyl containing O, N, or S.

In some embodiments, each R₄ is independently a branched C₃, C₄, C₅, or C₆ alkyl, or a branched C₃, C₄, C₅, or C₆ heteroalkyl containing O, N, or S.

In some embodiments, the present disclosure provides the amino lipid compound of formula (IA), or a pharmaceutically acceptable salt thereof, or a stereoisomer thereof, as described above, wherein each R₄ is independently C₂, C₃, C₄, C₅, or C₆ alkenyl with 1, 2, or more double bonds, or C₂, C₃, C₄, C₅, or C₆ heteroalkenyl containing O, N, or S with 1, 2, or more double bonds.

In some embodiments, each R₄ is independently a straight C₂, C₃, C₄, C₅, or C₆ alkenyl with 1, 2, or more double bonds, or a straight C₂, C₃, C₄, C₅, or C₆ heteroalkenyl containing O, N, or S with 1, 2, or more double bonds.

In some embodiments, each R₄ is independently a branched C₃, C₄, C₅, or C₆ alkenyl with 1, 2, or more double bonds, or a branched C₃, C₄, C₅, or C₆ heteroalkenyl containing O, N, or S with 1, 2, or more double bonds.

In some embodiments, the present disclosure provides the amino lipid compound of formula (IA), or a pharmaceutically acceptable salt thereof, or a stereoisomer thereof, as described above, wherein each R₄ is independently C₂, C₃, C₄, C₅, or C₆ alkynyl with 1, 2, or more triple bonds, or C₂, C₃, C₄, C₅, or C₆ heteroalkynyl containing O, N, or S with 1, 2, or more double bonds.

In some embodiments, the present disclosure provides the amino lipid compound of formula (IA), or a pharmaceutically acceptable salt thereof, or a stereoisomer thereof, as described above, wherein each R₄ may be independently attached to a C atom on L₁, L₂, L₃, L₄, L₅, or L₆ to form a three-, four-, five-, six-, seven-, eight-, nine-, or ten-membered monocyclic or polycyclic N atom-containing heterocyclic ring.

In some embodiments, the present disclosure provides the amino lipid compound of formula (IA), or a pharmaceutically acceptable salt thereof, or a stereoisomer thereof, as described above, wherein each R₄ is independently H, or C₁, C₂, C₃, C₄, C₅, or C₆ alkyl.

In some embodiments, each R₄ is independently H.

In some embodiments, each R₄ is independently C₁ or C₂ alkyl.

In some embodiments, each R₄ is independently C₃ or C₄ alkyl.

In some embodiments, each R₄ is independently C₅ or C₆ alkyl.

In some embodiments, the present disclosure provides the amino lipid compound of formula (IA), or a pharmaceutically acceptable salt thereof, or a stereoisomer thereof, as described above, wherein A₁, A₂, A₃, A₄, A₅, A₆, A₇, A₈, A₉, and A₁₀ are each independently -N(R₄)-, -C(=O)O-, -OC(=O)-, -OC(=O)O-, -O-, - C(=O)-, -S-, -C(=O)S-, -SC(=O)-, -C(=O)N(R₄)-, -N(R₄)C(=O)-, -OC(=O)S-, -SC(=O)O-, -SC(=O)S-, - OC(=O)N(R₄)-, -N(R₄)C(=O)O-, -N(R₄)C(=O)N(R₄)-, -SC(=O)N(R₄)-, -N(R₄)C(=O)S-, -N(C(=O)L₁₃OR₄)-, -N(C(=O)L₁₃SR₄)-, -N(C(=O)R₄), -N(L₁₃OR₄)-, -N(L₁₃SR₄)-, -OS(=O)O-, -OS(=O)₂O-, -OC(=O)C(=O)O-, -N(R₄)C(=O)C(=O)O-, -OC(=O)C(=O)N(R₄)-, -N(R₄)C(=O)C(=O)N(R₄)-, 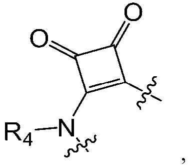 or

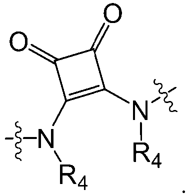

In some embodiments, A₁, A₂, A₃, A₄, A₅, A₆, A₇, A₈, A₉, and A₁₀ are each independently -N(R₄)-, - C(=O)O-, -OC(=O)-, -OC(=O)O-, -C(=O)S-, -SC(=O)-, -C(=O)N(R₄)-, -N(R₄)C(=O)-, -OC(=O)N(R₄)-, - N(R₄)C(=O)O-, -N(R₄)C(=O)N(R₄)-, -N(C(=O)R₄), -OC(=O)C(=O)O-, -N(R₄)C(=O)C(=O)O-, - OC(=O)C(=O)N(R₄)-, -N(R₄)C(=O)C(=O)N(R₄)-,

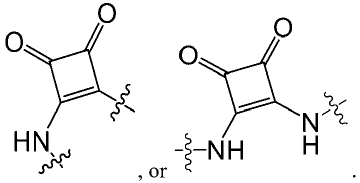

In some embodiments, the present disclosure provides the amino lipid compound of formula (IA), or a pharmaceutically acceptable salt thereof, or a stereoisomer thereof, as described above, wherein at least 3, e.g., 3, 4, 5, or 6, of A₁, A₂, A₃, A₄, A₅, and A₆ are each independently -N(R₄)-, -C(=O)O-, -OC(=O)-, - OC(=O)O-, -O-, -C(=O)-, -S-, -C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, -N(R₄)C(=O)O-, -N(R₄)C(=O)N(R₄)-, -SC(=O)N(R₄)-, -N(R₄)C(=O)S-, - N(C(=O)L₁₃OR₄)-, -N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, -OP(=O)(OH)O-, -OP(=O)(H)O-, -OS(=O)₂NH-, -NHS(=O)₂O-, -OC(=O)C(=O)O-, - N(R₄)C(=O)C(=O)O-, -OC(=O)C(=O)N(R₄)-, -N(R₄)C(=O)C(=O)N(R₄)-, 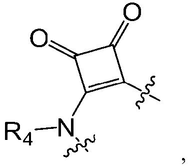 or

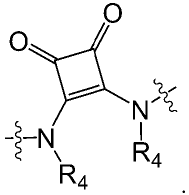

In some embodiments, the present disclosure provides the amino lipid compound of formula (IA), or a pharmaceutically acceptable salt thereof, or a stereoisomer thereof, as described above, wherein each L₁₃ is independently C₁, C₂, C₃, C₄, C₅, C₆, C₇, or C₈ hydrocarbylene, or C₁, C₂, C₃, C₄, C₅, C₆, C₇, or C₈ heterohydrocarbylene.

In some embodiments, the present disclosure provides the amino lipid compound of formula (IA), or a pharmaceutically acceptable salt thereof, or a stereoisomer thereof, as described above, wherein each L₁₃ is independently C₁, C₂, C₃, C₄, C₅, C₆, C₇, or C₈ alkylene, or C₁, C₂, C₃, C₄, C₅, C₆, C₇, or C₈ heteroalkylene containing O, N, or S.

In some embodiments, each L₁₃ is independently a straight C₁, C₂, C₃, C₄, C₅, C₆, C₇, or C₈ alkylene, or a straight C₁, C₂, C₃, C₄, C₅, C₆, C₇, or C₈ heteroalkylene containing O, N, or S.

In some embodiments, each L₁₃ is independently a branched C₃, C₄, C₅, C₆, C₇, or C₈ alkylene, or a branched C₃, C₄, C₅, C₆, C₇, or C₈ heteroalkylene containing O, N, or S.

In some embodiments, the present disclosure provides the amino lipid compound of formula (IA), or a pharmaceutically acceptable salt thereof, or a stereoisomer thereof, as described above, wherein each L₁₃ is independently C₂, C₃, C₄, C₅, C₆, C₇, or C₈ alkenylene with 1, 2, or more double bonds, or C₂, C₃, C₄, C₅, C₆, C₇, or C₈ heteroalkenylene containing O, N, or S with 1, 2, or more double bonds.

In some embodiments, each L₁₃ is independently a straight C₂, C₃, C₄, C₅, C₆, C₇, or C₈ alkenylene with 1, 2, or more double bonds, or a straight C₂, C₃, C₄, C₅, C₆, C₇, or C₈ heteroalkenylene containing O, N, or S with 1, 2, or more double bonds.

In some embodiments, each L₁₃ is independently a branched C₃, C₄, C₅, C₆, C₇, or C₈ alkenylene with 1, 2, or more double bonds, or a branched C₃, C₄, C₅, C₆, C₇, or C₈ heteroalkenylene containing O, N, or S with 1, 2, or more double bonds.

In some embodiments, the present disclosure provides the amino lipid compound of formula (IA), or a pharmaceutically acceptable salt thereof, or a stereoisomer thereof, as described above, wherein each L₁₃ is independently C₂, C₃, C₄, C₅, C₆, C₇, or C₈ alkynylene with 1, 2, or more triple bonds, or C₂, C₃, C₄, C₅, C₆, C₇, or C₈ heteroalkynylene containing O, N, or S with 1, 2, or more triple bonds.

In some embodiments, each L₁₃ is independently a straight C₂, C₃, C₄, C₅, C₆, C₇, or C₈ alkynylene with 1, 2, or more triple bonds, or a straight C₂, C₃, C₄, C₅, C₆, C₇, or C₈ heteroalkynylene containing O, N, or S with 1, 2, or more triple bonds.

In some embodiments, each L₁₃ is independently a branched C₄, C₅, C₆, C₇, or C₈ alkynylene with 1, 2, or more triple bonds, or a branched C₄, C₅, C₆, C₇, or C₈ heteroalkynylene containing O, N, or S with 1, 2, or more triple bonds.

In some embodiments, the present disclosure provides the amino lipid compound of formula (IA), or a pharmaceutically acceptable salt thereof, or a stereoisomer thereof, as described above, wherein:
R₁ and R₂ are each independently C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₁₄ hydrocarbyl, or C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heterohydrocarbyl.

In some embodiments, the present disclosure provides the amino lipid compound of formula (IA), or a pharmaceutically acceptable salt thereof, or a stereoisomer thereof, as described above, wherein:
R₁ and R₂ are each independently a straight C₁-C₂₄ alkyl, or a straight C₂-C₂₄ alkenyl with 1, 2, 3, 4, or more double bonds, or a straight C₂-C₂₄ alkynyl with 1, 2, 3, 4, or more triple bonds, or a straight C₁-C₂₄ heteroalkyl containing O, N, or S, or a straight C₂-C₂₄ heteroalkenyl containing O, N, or S with 1, 2, 3, 4, or more double bonds, or a straight C₂-C₂₄ heteroalkynyl containing O, N, or S with 1, 2, 3, 4, or more triple bonds.

In some embodiments, the present disclosure provides the amino lipid compound of formula (IA), or a pharmaceutically acceptable salt thereof, or a stereoisomer thereof, as described above, wherein:
R₁ and R₂ are each independently a straight C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, or a straight C₂, C₃, C₄, C₅, C₆, C₇, C₅, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkenyl with 1, 2, 3, 4, or more double bonds, a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₁₄ alkynyl with 1, 2, 3, 4, or more triple bonds, a straight C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₁₄ heteroalkyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkenyl with 1, 2, 3, 4, or more double bonds, or a straight C₂, C₃, C₄, C₅, C₆, C₇, C₈, C₉, C_{10,} C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkynyl with 1, 2, 3, 4, or more triple bonds.

In some embodiments, the present disclosure provides the amino lipid compound of formula (IA), or a pharmaceutically acceptable salt thereof, or a stereoisomer thereof, as described above, wherein:
R₁ and R₂ are each independently a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₁₄ alkyl, a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkenyl with 1, 2, 3, 4, or more double bonds, a branched C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkynyl with 1, 2, 3, 4, or more triple bonds, a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₁₄ heteroalkyl containing O, N, or S, a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₁₄ heteroalkenyl containing O, N, or S with 1, 2, 3, 4, or more double bonds, a branched C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkynyl containing O, N, or S with 1, 2, 3, 4, or more triple bonds.

In some embodiments, the present disclosure provides the amino lipid compound of formula (IA), or a pharmaceutically acceptable salt thereof, or a stereoisomer thereof, as described above, wherein:
R₁ and R₂ are each independently a straight or branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₁₀, C₂₁, C₂₂, C₂₃, or C₁₄ alkyl.

In some embodiments, R₁ and R₂ are each independently a straight or branched C₈-C₁₀ alkyl.

In some embodiments, R₁ and R₂ are each independently a straight or branched C₉-C₁₁ alkyl.

In some embodiments, R₁ and R₂ are each independently a straight or branched C₁₅-C₁₉ alkyl.

In some embodiments, the present disclosure provides the amino lipid compound of formula (IA), or a pharmaceutically acceptable salt thereof, or a stereoisomer thereof, as described above, wherein:
R₁ and R₂ are each independently A₁₁R₅.

In some embodiments, the present disclosure provides the amino lipid compound of formula (IA), or a pharmaceutically acceptable salt thereof, or a stereoisomer thereof, as described above, wherein:
each R₅ is independently C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₁₄ hydrocarbyl, or C₁, C₂, C₃, C₄, C₅, C₆, C₇, C₈, C₉, C₁₉, C₁₁, C₁₂, C₁₃, C₁₄, C₁₅, C₁₆, C₁₇, C₁₈, C₁₉, C₂₀, C₂₁, C₂₂, C₂₃, or C₁₄ heterohydrocarbyl.

In some embodiments, the present disclosure provides the amino lipid compound of formula (IA), or a pharmaceutically acceptable salt thereof, or a stereoisomer thereof, as described above, wherein:
each R₅ is independently a straight C₁-C₂₄ alkyl, or a straight C₂-C₂₄ alkenyl with 1, 2, 3, 4, or more double bonds, or a straight C₂-C₂₄ alkynyl with 1, 2, 3, 4, or more triple bonds, or a straight C₁-C₁₄ heteroalkyl containing O, N, or S, or a straight C₂-C₂₄ heteroalkenyl containing O, N, or S with 1, 2, 3, 4, or more double bonds, or a straight C₂-C₂₄ heteroalkynyl containing O, N, or S with 1, 2, 3, 4, or more triple bonds.

In some embodiments, the present disclosure provides the amino lipid compound of formula (IA), or a pharmaceutically acceptable salt thereof, or a stereoisomer thereof, as described above, wherein:
each R₅ is independently a branched C₃-C₂₄ alkyl, or a branched C₃-C₂₄ alkenyl with 1, 2, 3, 4, or more double bonds, or a branched C₄-C₂₄ alkynyl with 1, 2, 3, 4, or more triple bonds, or a branched C₃-C₂₄ heteroalkyl containing O, N, or S, or a branched C₃-C₂₄ heteroalkenyl containing O, N, or S with 1, 2, 3, 4, or more double bonds, or a branched C₄-C₂₄ heteroalkynyl containing O, N, or S with 1, 2, 3, 4, or more triple bonds.

In some embodiments, the present disclosure provides the amino lipid compound of formula (IA), or a pharmaceutically acceptable salt thereof, or a stereoisomer thereof, as described above, wherein:
R₁ and R₂ are each independently -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇).

In some embodiments, the present disclosure provides the amino lipid compound of formula (IA), or a pharmaceutically acceptable salt thereof, or a stereoisomer thereof, as described above, wherein:
R₁ and R₂ may further be each independently -C(R₆)(C(=O)OL₁₄A₁₂R₇)₂, -C(R₆)(OC(=O)L₁₄A₁₂R₇)₂, - C(R₆)(C(=O)OL₁₄A₁₂R₇)R₇, or -C(R₆)(OC(=O)L₁₄A₁₂R₇)R₇.

In some embodiments, the present disclosure provides the amino lipid compound of formula (IA), or a pharmaceutically acceptable salt thereof, or a stereoisomer thereof, as described above, wherein:
R₁ and R₂ are each independently -C(R₆)(OR₇)₂, -C(R₆)(SR₇)₂, -C(R₆)(SR₇)(OR₇), -C(R₆)(C(=O)OR₇)₂, -C(R₆)(OC(=O)R₇)₂, -C(R₆)(C(=O)OR₇)R₇, or -C(R₆)(OC(=O)R₇)R₇.

In some embodiments, the present disclosure provides the amino lipid compound of formula (IA), or a pharmaceutically acceptable salt thereof, or a stereoisomer thereof, as described above, wherein:
each R₆ is independently C₁, C₂, C₃, C₄, C₅, or C₆ hydrocarbyl, or C₁, C₂, C₃, C₄, C₅, or C₆ heterohydrocarbyl.

In some embodiments, the present disclosure provides the amino lipid compound of formula (IA), or a pharmaceutically acceptable salt thereof, or a stereoisomer thereof, as described above, wherein:
each R₆ is independently C₁, C₂, C₃, C₄, C₅, or C₆ alkyl, or H.

In some embodiments, each R₆ is independently C₁-C₃ alkyl.

In some embodiments, each R₆ is H.

In some embodiments, the present disclosure provides the amino lipid compound of formula (IA), or a pharmaceutically acceptable salt thereof, or a stereoisomer thereof, as described above, wherein each L₁₄ is independently C₁, C₂, C₃, C₄, C₅, C₆, C₇, C₅, C₉, C₁₀, C₁₁, C₁₂, C₁₃, C₁₄, C₁₅, C₁₆, C₁₇, or C₁₈ hydrocarbylene, or C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heterohydrocarbylene containing O, N, or S.

In some embodiments, the present disclosure provides the amino lipid compound of formula (IA), or a pharmaceutically acceptable salt thereof, or a stereoisomer thereof, as described above, wherein each L₁₄ is independently C₁, C₂, C₃, C₄, C₅, C₆, C₇, C₈, C₉, C₁₀, C₁₁, C₁₂, C₁₃, C₁₄, C₁₅, C₁₆, C₁₇, or C_{I8} alkylene, or a bond.

In some embodiments, each L₁₄ is independently C₁-C₁₂; alkylene, or a bond.

In some embodiments, each L₁₄ is independently C₁-C₈ alkylene, or a bond.

In some embodiments, each L₁₄ is independently C₁-C₄ alkylene, or a bond.

In some embodiments, each L₁₄ is independently C₁-C₃ alkylene, or a bond.

In some embodiments, the present disclosure provides the amino lipid compound of formula (IA), or a pharmaceutically acceptable salt thereof, or a stereoisomer thereof, as described above, wherein:
each R₇ is independently C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₁₄ hydrocarbyl, or C₁, C₂, C₃, C₄, C₅, C₆, C₇, C₈, C₉, C₁₉, C₁₁, C₁₂, C₁₃, C₁₄, C₁₅, C₁₆, C₁₇, C₁₈, C₁₉, C₂₀, C₂₁, C₂₂, C₂₃, or C₁₄ heterohydrocarbyl.

In some embodiments, the present disclosure provides the amino lipid compound of formula (IA), or a pharmaceutically acceptable salt thereof, or a stereoisomer thereof, as described above, wherein:
each R₇ is independently a straight C₁-C₂₄ alkyl, or a straight C₂-C₂₄ alkenyl with 1, 2, 3, 4, or more double bonds, or a straight C₂-C₂₄ alkynyl with 1, 2, 3, 4, or more triple bonds, or a straight C₁-C₂₄ heteroalkyl containing O, N, or S, or a straight C₂-C₂₄ heteroalkenyl containing O, N, or S with 1, 2, 3, 4, or more double bonds, or a straight C₂-C₂₄ heteroalkynyl containing O, N, or S with 1, 2, 3, 4, or more triple bonds.

In some embodiments, the present disclosure provides the amino lipid compound of formula (IA), or a pharmaceutically acceptable salt thereof, or a stereoisomer thereof, as described above, wherein:
each R₇ is independently a straight C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkenyl with 1, 2, 3, 4, or more double bonds, a straight C₂, C₃, C₄, C₅, C₆, C₇, C₈, C₉, C₁₀, C_{11,} C₁₂, C₁₃, C₁₄, C₁₅, C₁₆, C₁₇, C₁₈, C₁₉, C₂₀, C₂₁, C₂₂, C₂₃, or C₂₄ alkynyl with 1, 2, 3, 4, or more triple bonds, a straight C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkyl, or a straight C₂, C₃, C₄, C₅, C₆, C₇, C₈, C₉, C_{10,} C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkenyl with 1, 2, 3, 4, or more double bonds, or a straight C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkynyl with 1, 2, 3, 4, or more triple bonds.

In some embodiments, the present disclosure provides the amino lipid compound of formula (IA), or a pharmaceutically acceptable salt thereof, or a stereoisomer thereof, as described above, wherein:
each R₇ is independently a branched C₃-C₂₄ alkyl, or a branched C₃-C₂₄ alkenyl with 1, 2, 3, 4, or more double bonds, or a branched C₄-C₂₄ alkynyl with 1, 2, 3, 4, or more triple bonds, or a branched C₃-C₂₄ heteroalkyl containing O, N, or S, or a branched C₃-C₂₄ heteroalkenyl containing O, N, or S with 1, 2, 3, 4, or more double bonds, or a branched C₄-C₂₄ heteroalkynyl containing O, N, or S with 1, 2, 3, 4, or more triple bonds.

In some embodiments, the present disclosure provides the amino lipid compound of formula (IA), or a pharmaceutically acceptable salt thereof, or a stereoisomer thereof, as described above, wherein:
each R₇ is independently a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, a branched C₃, C₄, C₅, C₆, C₇, C₈, C₉, C_{10,} C₁₁, C₁₂, C₁₃, C₁₄, C₁₅, C₁₆, C₁₇, C₁₈, C₁₉, C₂₀, C₂₁, C₂₂, C₂₃, or C₂₄ alkenyl with 1, 2, 3, 4, or more double bonds, a branched C₄, C₅, C₆, C₇, C₈, C₉, C_{10,} C₁₁, C₁₂, C₁₃, C₁₄, C₁₅, C₁₆, C₁₇, C₁₈, C₁₉, C₂₀, C₂₁, C₂₂, C₂₃, or C₂₄ alkynyl with 1, 2, 3, 4, or more triple bonds, a branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkyl containing O, N, or S, a branched C₃, C₄, C₅, C₆, C₇, C₈, C₉, C_{10,} C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkenyl containing O, N, or S with 1, 2, 3, 4, or more double bonds, a branched C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ heteroalkynyl containing O, N, or S with 1, 2, 3, 4, or more triple bonds.

In some embodiments, each R₇ is independently C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, alkenyl, or alkynyl, or H.

In some embodiments, each R₇ is independently C₁-C₄ alkyl, alkenyl, or alkynyl, or H.

In some embodiments, each R₇ is independently C₂ or C₃ alkyl, alkenyl, or alkynyl.

In some embodiments, each R₇ is independently C₅-C₁₀ alkyl, alkenyl, or alkynyl.

In some embodiments, each R₇ is independently C₁₁-C₂₁ alkyl, alkenyl, or alkynyl.

In some embodiments, each R₇ is independently C₁₆-C₁₈ alkyl, alkenyl, or alkynyl.

In some embodiments, each R₅ or R₇ is independently selected from:

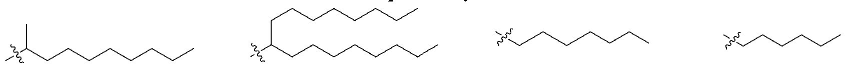

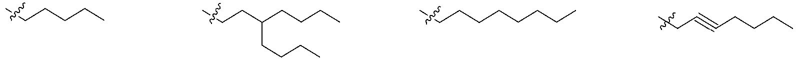

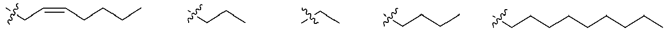

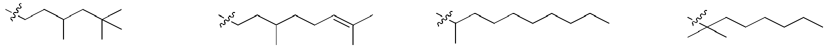

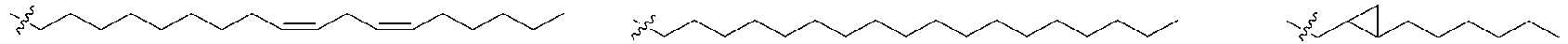

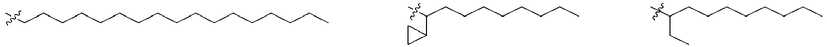

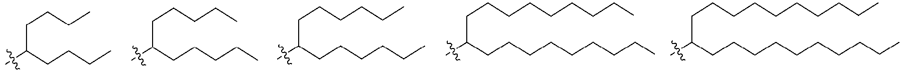

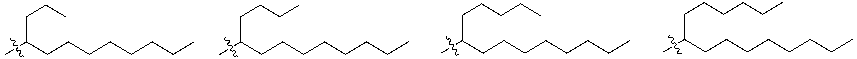

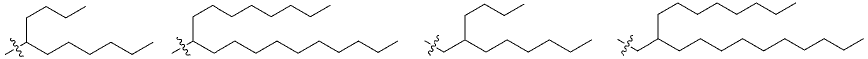

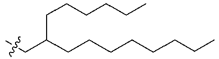

In some embodiments, the present disclosure provides the amino lipid compound of formula (IA), or a pharmaceutically acceptable salt thereof, or a stereoisomer thereof, as described above, wherein:
R₁ and R₂ are each independently selected from:

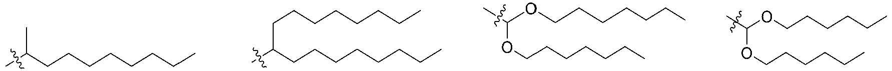

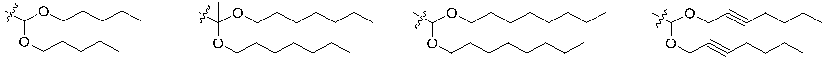

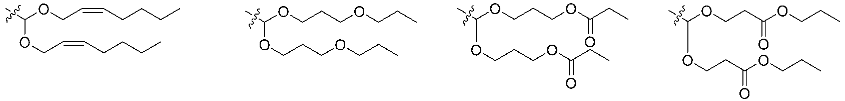

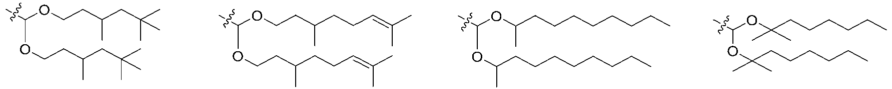

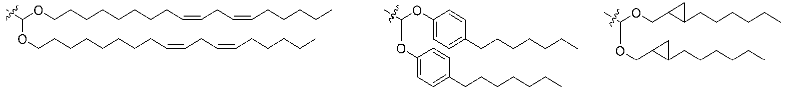

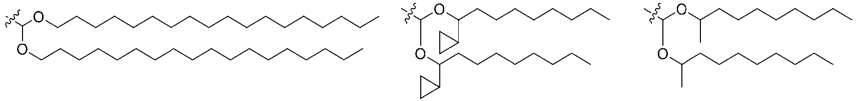

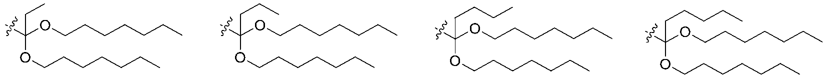

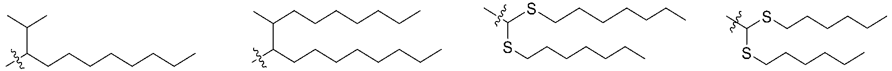

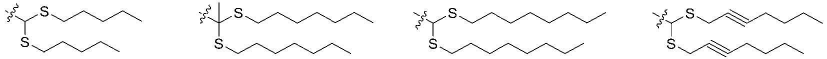

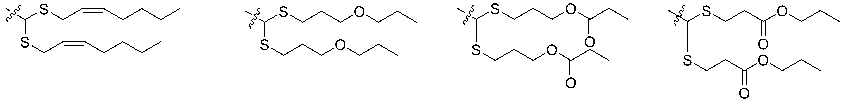

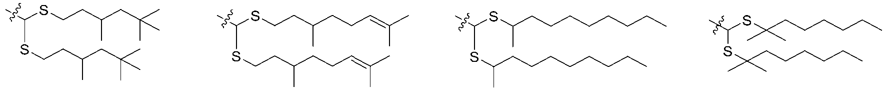

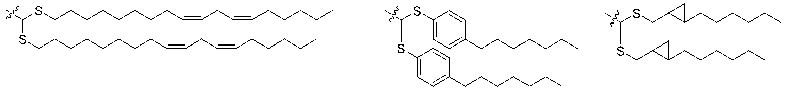

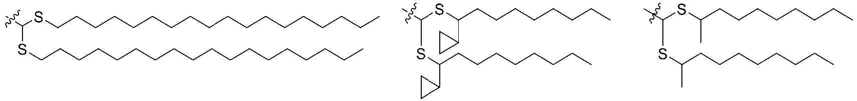

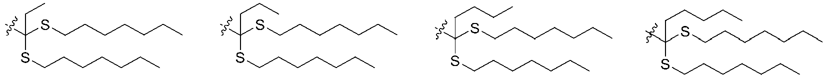

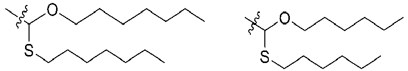

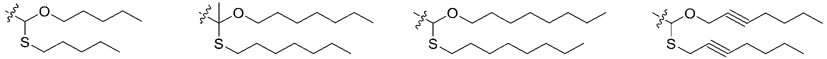

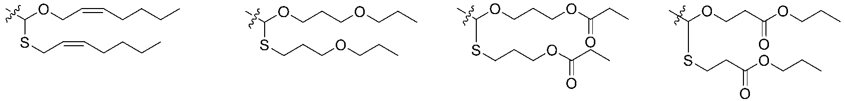

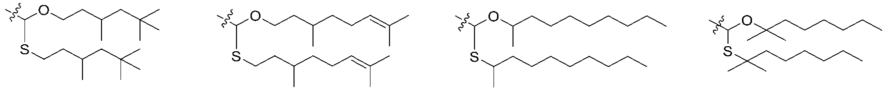

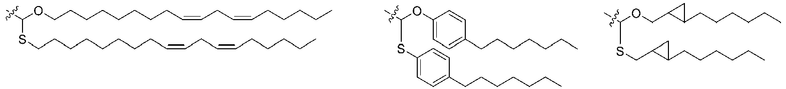

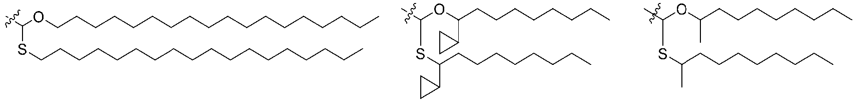

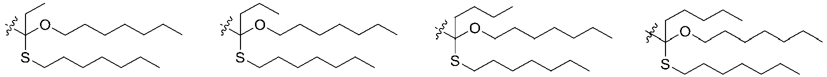

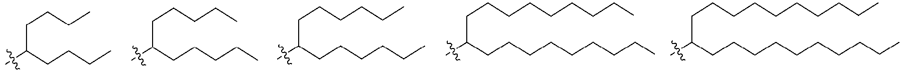

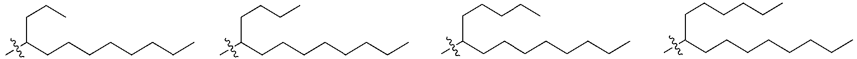

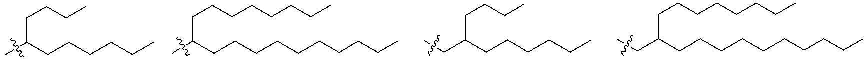

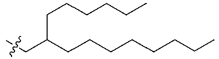

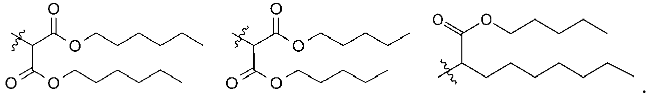

In some embodiments, the present disclosure provides the amino lipid compound of formula (IA), or a pharmaceutically acceptable salt thereof, or a stereoisomer thereof, as described above, wherein L₁₅ is C₁, C₂, C₃, C₄, C₅, C₆, C₇, C₈, C₉, C₁₀, C₁₁, or C₁₂ hydrocarbylene, or C₁, C₂, C₃, C₄, C₅, C₆, C₇, C₈, C₉, C₁₀, C₁₁, or C₁₂ heterohydrocarbylene containing O, N, or S.

In some embodiments, the present disclosure provides the amino lipid compound of formula (IA), or a pharmaceutically acceptable salt thereof, or a stereoisomer thereof, as described above, wherein L₁₅ is a bond.

In some embodiments, the present disclosure provides the amino lipid compound of formula (IA), or a pharmaceutically acceptable salt thereof, or a stereoisomer thereof, as described above, wherein L₁₅ is C₁, C₂, C₃, C₄, C₅, C₆, C₇, C₈, C₉, C₁₀, C₂₁,or C₁₂ alkylene, or C₁, C₂, C₃, C₄, C₅, C₆, C₇, C₈, C₉, C₁₀, C₂₁,or C₁₂ heteroalkylene containing O, N, or S.

In some embodiments, the present disclosure provides the amino lipid compound of formula (IA), or a pharmaceutically acceptable salt thereof, or a stereoisomer thereof, as described above, wherein:
each R₈ is independently a straight C₁, C₂, C₃, C₄, C₅, C₆, C₇, C₈, C₉, C₁₀, C₁₁, or C₁₂ alkyl, or a straight C₂, C₃, C₄, C₅, C₆, C₇, C₈, C₉, C₁₀, C₁₁, or C₁₂ alkenyl with 1, 2, 3, or more double bonds, a straight C₂, C₃, C₄, C₅, C₆, C₇, C₈, C₉, C₁₀, C₁₁, or C₁₂ alkynyl with 1, 2, 3, or more triple bonds, or a straight C₁, C₂, C₃, C₄, C₅, C₆, C₇, C₈, C₉, C₁₀, C₁₁, or C₁₂ heteroalkyl containing O, N, or S, or a straight C₂, C₃, C₄, C₅, C₆, C₇, C₈, C₉, C₁₀, C₁₁, or C₁₂ heteroalkenyl containing O, N, or S with 1, 2, 3, or more double bonds, or a straight C₂, C₃, C₄, C₅, C₆, C₇, C₈, C₉, C₁₀, C₁₁, or C₁₂ heteroalkynyl containing O, N, or S with 1, 2, 3, or more triple bonds.

In some embodiments, the present disclosure provides the amino lipid compound of formula (IA), or a pharmaceutically acceptable salt thereof, or a stereoisomer thereof, as described above, wherein:
each R₈ is independently a branched C₃, C₄, C₅, C₆, C₇, C₈, C₉, C₁₀, C₁₁, or C₁₂ alkyl, or a branched C₃, C₄, C₅, C₆, C₇, C₈, C₉, C₁₀, C₁₁, or C₁₂ alkenyl with 1, 2, 3, or more double bonds, or a branched C₄, C₅, C₆, C₇, C₈, C₉, C₁₀, C₁₁, or C₁₂ alkynyl with 1, 2, 3, or more triple bonds, or a branched C₃, C₄, C₅, C₆, C₇, C₈, C₉, C₁₀, C₂₁,or C₁₂ heteroalkyl containing O, N, or S, a branched C₃, C₄, C₅, C₆, C₇, C₈, C₉, C₁₀, C₁₁, or C₁₂ heteroalkenyl containing O, N, or S with 1, 2, 3, or more double bonds, or a branched C₄, C₅, C₆, C₇, C₈, C₉, C₁₀, C₁₁, or C₁₂ heteroalkynyl containing O, N, or S with 1, 2, 3, or more triple bonds.

In some embodiments, the present disclosure provides the amino lipid compound of formula (IA), or a pharmaceutically acceptable salt thereof, or a stereoisomer thereof, as described above, wherein each R₁₁ is independently C₃₋₆ carbocyclic ring.

In some embodiments, each R₁₁ is independently C₃, C₄, C₅, or C₆ cycloalkyl, or C₃, C₄, C₅, or C₆ cycloalkenyl.

In some embodiments, each R₁₁ is independently C₃-C₆ cycloalkyl, e.g., cyclopropyl, cyclobutyl, cyclopentyl, or cyclohexyl.

In some embodiments, each R₁₁ is independently C₃₋₆ cycloalkyl optionally substituted with a substituent such as -OH, halogen, C₁₋₆ alkyl, etc.; for example, R₁₁ is cyclohexyl substituted with -OH, e.g., 2-hydroxycyclohexyl.

In some embodiments, the present disclosure provides the amino lipid compound of formula (IA), or a pharmaceutically acceptable salt thereof, or a stereoisomer thereof, as described above, wherein each R₁₁ is independently a heterocyclic ring.

In some embodiments, each R₁₁ is independently a five-, six-, seven-, or eight-membered heterocyclic ring.

In some embodiments, each R₁₁ is independently a five-, six-, seven-, or eight-membered heterocyclic ring containing N, O, or S.

In some embodiments, each R₁₁ is independently five- or six-membered heterocyclic ring containing N, O, or S.

In some embodiments, the present disclosure provides the amino lipid compound of formula (IA), or a pharmaceutically acceptable salt thereof, or a stereoisomer thereof, as described above, wherein each R₁₂ is independently C₁, C₂, C₃, C₄, C₅, or C₆ alkyl.

In some embodiments, the present disclosure provides the amino lipid compound of formula (IA), or a pharmaceutically acceptable salt thereof, or a stereoisomer thereof, as described above, wherein each R₁₂ is independently C₂, C₃, C₄, C₅, or C₆ alkenyl.

In some embodiments, the present disclosure provides the amino lipid compound of formula (IA), or a pharmaceutically acceptable salt thereof, or a stereoisomer thereof, as described above, wherein each R₁₂ is independently C₃₋₆ carbocyclic ring.

In some embodiments, each R₁₂ is independently C₃, C₄, C₅, or C₆ cycloalkyl, or C₃, C₄, C₅, or C₆ cycloalkenyl.

In some embodiments, each R₁₂ is independently C₃-C₆ cycloalkyl, e.g., cyclopropyl, cyclobutyl, cyclopentyl, or cyclohexyl.

In some embodiments, each R₁₂ is independently C₃₋₆ cycloalkyl optionally substituted with a substituent such as -OH, halogen, C₁₋₆ alkyl, etc.; for example, R₁₁ is cyclohexyl substituted with -OH, e.g., 2-hydroxycyclohexyl.

In some embodiments, the present disclosure provides the amino lipid compound of formula (IA), or a pharmaceutically acceptable salt thereof, or a stereoisomer thereof, as described above, wherein each R₁₂ is independently a heterocyclic ring.

In some embodiments, each R₁₂ is independently a five-, six-, seven-, or eight-membered heterocyclic ring.

In some embodiments, each R₁₂ is independently a five-, six-, seven-, or eight-membered heterocyclic ring containing N, O, or S.

In some embodiments, each R₁₂ is independently five- or six-membered heterocyclic ring containing N, O, or S.

In some embodiments, the present disclosure provides the amino lipid compound of formula (IA), or a pharmaceutically acceptable salt thereof, or a stereoisomer thereof, as described above, wherein each L₁₆ is independently C₁, C₂, C₃, C₄, C₅, C₆, C₇, C₅, C₉, C₁₀, C₁₁, C₁₂, C₁₃, C₁₄, C₁₅, C₁₆, C₁₇, or C₁₈ hydrocarbylene, or C₁, C₂, C₃, C₄, C₅, C₆, C₇, C₈, C₉, C₁₀, C₁₁, C₁₂, C₁₃, C₁₄, C₁₅, C₁₆, C₁₇, or C₁₈ heterohydrocarbylene containing O, N, or S.

In some embodiments, the present disclosure provides the amino lipid compound of formula (IA), or a pharmaceutically acceptable salt thereof, or a stereoisomer thereof, as described above, wherein each L₁₆ is independently C₁, C₂, C₃, C₄, or C₅ alkylene. In some embodiments, each L₁₆ is independently C₂-C₄ alkylene.

In some embodiments, the present disclosure provides the amino lipid compound of formula (IA), or a pharmaceutically acceptable salt thereof, or a stereoisomer thereof, as described above, wherein Z is C₃₋₁₀ carbocyclic ring. In some embodiments, Z is C₃₋₈ carbocyclic ring, e.g., C₃ carbocyclic ring, C₄ carbocyclic ring, C₅ carbocyclic ring, C₆ carbocyclic ring, C₇ carbocyclic ring, or C₈ carbocyclic ring.

In some embodiments, the present disclosure provides the amino lipid compound of formula (IA), or a pharmaceutically acceptable salt thereof, or a stereoisomer thereof, as described above, wherein Z is C₃₋₈ cycloalkyl optionally substituted with one or more substituents, such as cyclopropyl optionally substituted with one or more substituents, cyclobutyl optionally substituted with one or more substituents, cyclopentyl optionally substituted with one or more substituents, cyclohexyl optionally substituted with one or more substituents, cycloheptyl optionally substituted with one or more substituents, and cyclooctyl optionally substituted with one or more substituents, wherein the substituents may be independently selected from, but not limited to, hydroxyl, oxygen, amino (-NH₂), monoalkyl or dialkyl amino, C₁-C₁₂ alkyl, C₂-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, halogen, sulfur (=S), C₃-C₈ heterocyclic ring, or aryl, wherein the amino, monoalkyl or dialkyl amino, C₁-C₁₂ alkyl, C₂-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, C₃-C₈ heterocyclic ring, or aryl may be further substituted.

In some embodiments, the present disclosure provides the amino lipid compound of formula (IA), or a pharmaceutically acceptable salt thereof, or a stereoisomer thereof, as described above, wherein Z is C₃₋₈ cycloalkenyl optionally substituted with one or more substituents, such as cyclopropenyl optionally substituted with one or more substituents, cyclobutenyl optionally substituted with one or more substituents, cyclopentenyl optionally substituted with one or more substituents, cyclohexenyl optionally substituted with one or more substituents, cycloheptenyl optionally substituted with one or more substituents, and cyclooctenyl optionally substituted with one or more substituents, wherein the substituents may be independently selected from, but not limited to, hydroxyl, oxygen, amino (-NH₂), monoalkyl or dialkyl amino, C₁-C₁₂ alkyl, C₂-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, halogen, sulfur (=S), C₃-C₈ heterocyclic ring, or aryl, wherein the amino, monoalkyl or dialkyl amino, C₁-C₁₂ alkyl, C₂-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, C₃-C₈ heterocyclic ring, or aryl may be further substituted.

In some embodiments, Z is cyclobutenyl substituted with one or more of oxygen, amino, and alkylamino, wherein the alkylamino is further substituted with, e.g., one or more of C₁-C₁₂ alkoxyl, amino, monoalkyl or dialkyl amino, and halogen. For example, Z is 3-(((methylamino)ethyl)amino)cyclobuty-3-enyl-1,2-dione.

In some embodiments, Z is cyclobutenyl substituted with one or more of oxygen, sulfur, and monoalkyl or dialkyl amino. For example, Z is 2-(diethylamino)-4-thiooxocyclobut-2-ene-1-one or 3-(diethylamino)-4-thiooxocyclobut-2-ene-1-one.

In some embodiments, Z is cyclobutenyl substituted with one or more of oxygen, and piperidyl, piperazinyl, or morpholinyl.

In some embodiments, Z is cyclobutenyl substituted with one or more of oxygen and a heterocyclic ring, wherein the heterocyclic ring is further substituted with, e.g., one or more C₁-C₁₂ alkyl groups.

In some embodiments, Z is cyclobutenyl substituted with one or more of oxygen and a heterocyclic ring, wherein the heterocyclic ring (such as piperidyl, piperazinyl, or morpholinyl) is further substituted with methyl.

In some embodiments, Z is cyclobutenyl substituted with one or more of oxygen and monoalkyl or dialkyl amino. For example, Z is 3-(diethylamino)cyclobut-3-ene-1,2-dione.

In some embodiments, Z is cyclobutenyl substituted with one or more of sulfur and monoalkyl or dialkyl amino. For example, Z is 3-(diethylamino)cyclobut-3-ene-1,2-dithione.

In some embodiments, Z is cyclobutenyl substituted with one or more of oxygen, sulfur, and monoalkyl or dialkyl amino. For example, Z is 3-(ethylamino)-4-thiooxocyclobut-2-ene-1-one or 2-(ethylamino)-4-thiooxocyclobut-2-ene-1-one.

In some embodiments, Z is cyclobutenyl substituted with one or more of oxygen and monoalkyl or dialkyl amino. For example, Z is 3-(ethylamino)cyclobut-3-ene-1,2-dione.

In some embodiments, Z is cyclobutenyl substituted with one or more of oxygen and monoalkyl or dialkyl amino, wherein the monoalkyl or dialkyl amino is further substituted with, e.g., one or more alkoxyl groups. For example, Z is 3-(bis(2-methoxyethyl)amino)cyclobut-3-ene-1,2-dione.

In some embodiments, Z is cyclobutenyl substituted with one or more of sulfur and monoalkyl or dialkyl amino. For example, Z is 3-(ethylamino)cyclobut-3-ene-1,2-dithione.

In some embodiments, the present disclosure provides the amino lipid compound of formula (IA), or a pharmaceutically acceptable salt thereof, or a stereoisomer thereof, as described above, wherein Z is a 3- to 20-membered heterocyclic ring. In some embodiments, Z is a 3- to 10-membered heterocyclic ring, such as a 3-membered heterocyclic ring, a 4-membered heterocyclic ring, a 5-membered heterocyclic ring, a 6-membered heterocyclic ring, a 7-membered heterocyclic ring, an 8-membered heterocyclic ring, a 9-membered heterocyclic ring, or a 10-membered heterocyclic ring. In some embodiments, Z is a 3- to 10-membered heterocyclic ring containing 1-3 heteroatoms independently selected from nitrogen, oxygen, or sulfur. In some embodiments, Z is a heteroaryl containing 1-4 heteroatoms independently selected from nitrogen, oxygen, and sulfur. In some embodiments, Z is an 8- to 10-membered bicyclic heteroaryl containing 1-4 heteroatoms independently selected from nitrogen, oxygen, or sulfur.

In some embodiments, the present disclosure provides the amino lipid compound of formula (IA), or a pharmaceutically acceptable salt thereof, or a stereoisomer thereof, as described above, wherein Z is a 5- to 10-membered monocyclic or polycyclic heterocyclic ring optionally substituted with one or more substituents, and the substituents may be independently selected from, but not limited to, hydroxyl, oxygen, amino (-NH₂), monoalkyl or dialkyl amino, C₁-C₁₂ alkyl, C₂-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, halogen, sulfur (=S), C₃-C₈ heterocyclic ring, or aryl, wherein the amino, monoalkyl or dialkyl amino, C₁-C₁₂ alkyl, C₂-C₁₂ alkenyl, C₁-C₁₂ alkynyl, C₃-C₈ cycloalkyl, C₃-C₈ cycloalkenyl, C₃-C₈ cycloalkynyl, C₁-C₁₂ alkoxyl, C₃-C₈ heterocyclic ring, or aryl may be further substituted.

In some embodiments, the present disclosure provides the amino lipid compound of formula (IA), or a pharmaceutically acceptable salt thereof, or a stereoisomer thereof, as described above, wherein Z is C₆₋₁₀ aryl (e.g., phenyl) or C₃₋₆ cycloalkyl optionally substituted with one or more substituents, and the substituents may be independently selected from, but not limited to, hydroxyl, oxygen, amino (-NH₂), monoalkyl or dialkyl amino, C₁-C₁₂ alkyl, C₂-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, halogen, sulfur (=S), C₃-C₈ heterocyclic ring, or aryl, wherein the amino, monoalkyl or dialkyl amino, C₁-C₁₂ alkyl, C₂-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, C₃-C₈ heterocyclic ring, or aryl may be further substituted.

In some embodiments, the present disclosure provides the amino lipid compound of formula (IA), or a pharmaceutically acceptable salt thereof, or a stereoisomer thereof, as described above, wherein Z is a 5- to 10-membered heteroaryl optionally substituted with one or more substituents, for example, Z is triazolyl, imidazolyl, pyrimidinyl, purinyl, 2-amino-1,9-dihydro-6H-purin-6-one-9-yl (or guanin-9-yl), adenin-9-yl, cytosin-1-yl, or uracil-1-yl, each of which is independently optionally substituted with one or more selected from, but not limited to, hydroxyl, oxygen, amino (-NH₂), monoalkyl or dialkyl amino, C₁-C₁₂ alkyl, C₂-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, halogen, sulfur (=S), C₃-C₈ heterocyclic ring, or aryl, wherein the amino, monoalkyl or dialkyl amino, C₁-C₁₂ alkyl, C₂-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, C₃-C₈ heterocyclic ring, or aryl may be further substituted.

In some embodiments, the present disclosure provides the amino lipid compound of formula (IA), or a pharmaceutically acceptable salt thereof, or a stereoisomer thereof, as described above, wherein Z is a 5- to 14-membered heterocycloalkyl optionally substituted with one or more substituents, and the substituents are selected from, but not limited to, hydroxyl, oxygen, amino (-NH₂), monoalkyl or dialkyl amino, C₁-C₁₂ alkyl, C₂-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, halogen, sulfur (=S), C₃-C₈ heterocyclic ring, or aryl, wherein the amino, monoalkyl or dialkyl amino, C₁-C₁₂ alkyl, C₁-C₁₂ alkenyl, C₂-C₁₂ alkynyl, C₃-C₈ cycloalkyl, C₃-C₈ cycloalkenyl, C₃-C₈ cycloalkynyl, C₁-C₁₂ alkoxyl, C₃-C₈ heterocyclic ring, or aryl may be further substituted.

In some embodiments, the present disclosure provides the amino lipid compound of formula (IA), or a pharmaceutically acceptable salt thereof, or a stereoisomer thereof, as described above, wherein Z is 4-methylpiperazinyl, 4-(4-methoxyphenylmethyl)piperazinyl, isoindolin-2-yl-1,3-dione, pyrrolidin-1-yl-2,5-dione, or imidazolidin-3-yl-2,4-dione.

In some embodiments, the present disclosure provides the amino lipid compound of formula (IA), or a pharmaceutically acceptable salt thereof, or a stereoisomer thereof, as described above, wherein Z is 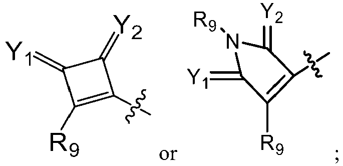 wherein:
Y₁ and Y₂ are each independently O or S;
each R₉ is independently H, halogen, -R_{b}, -N(R_{b})₂, -CN, -N₃, -C(=O)OR_{b}, -OC(=O)R_{b}, -OR_{b}, -SR_{b}, -S(-O)R_{b}, -S(=O)OR_{b}, -S(=O)₂OR_{b}, -N(R_{b})₂, -N(R_{S})S(=O)₂R_{b} -NHRₐN(R_{b})₂, -NHRₐORₐN(R_{b})₂ -NHRₐOR_{b}, or -N(RₐOR_{b})₂
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁₋C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds.

In some embodiments, the present disclosure provides the amino lipid compound of formula (IA), or a pharmaceutically acceptable salt thereof, or a stereoisomer thereof, as described above, wherein Z is 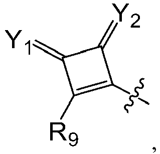 being selected from:

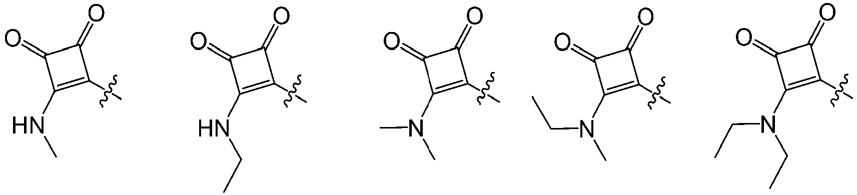

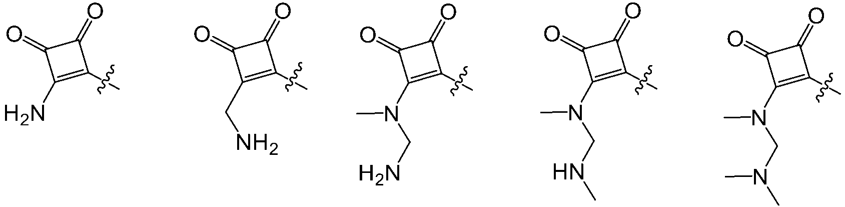

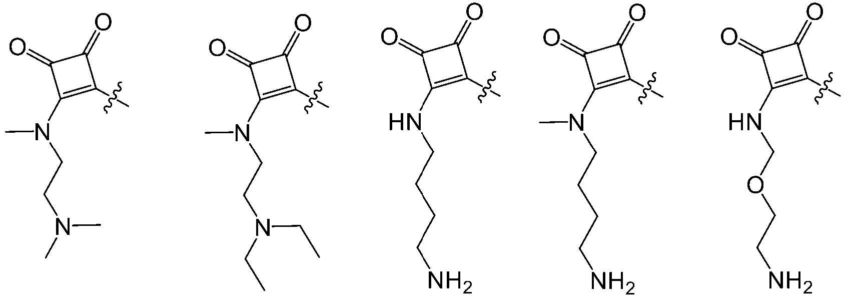

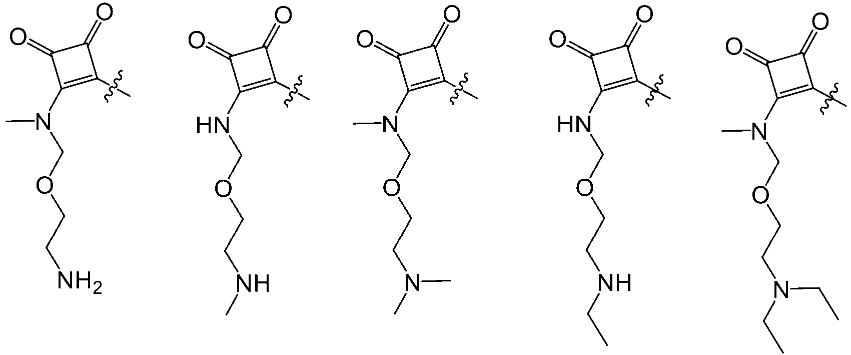

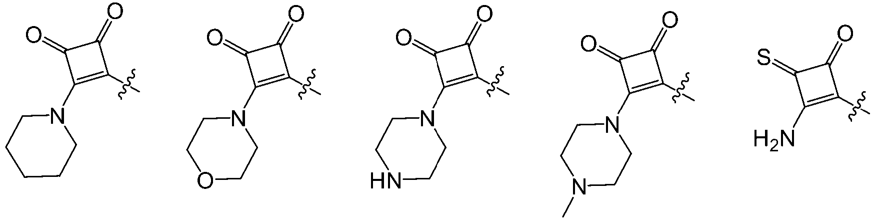

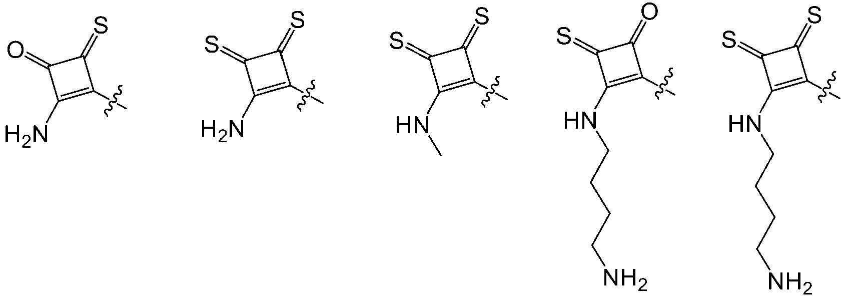

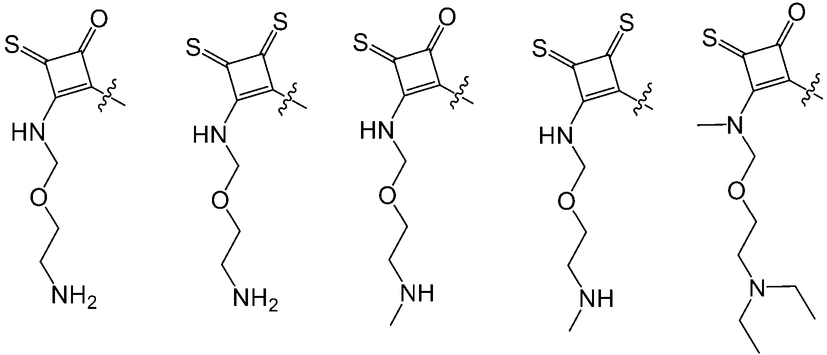

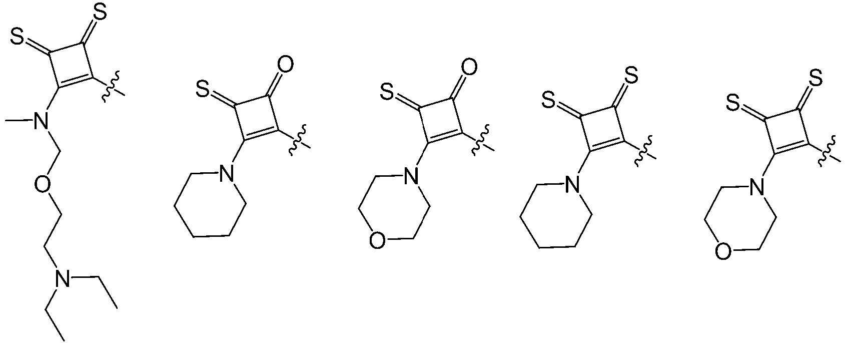

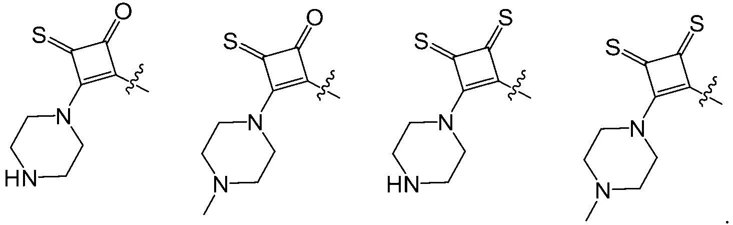

In some embodiments, the present disclosure provides the amino lipid compound of formula (IA), or a pharmaceutically acceptable salt thereof, or a stereoisomer thereof, as described above, wherein Z is 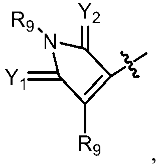 being selected from:

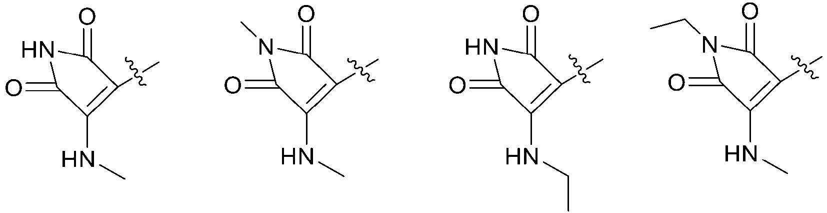

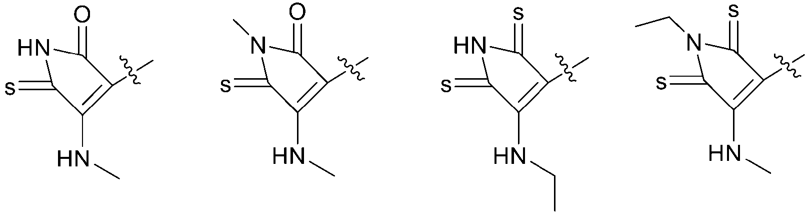

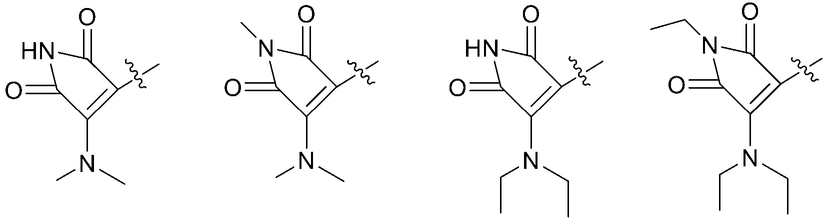

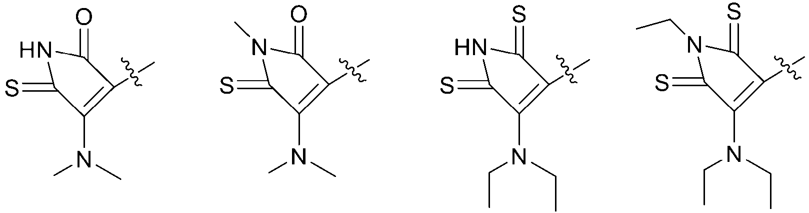

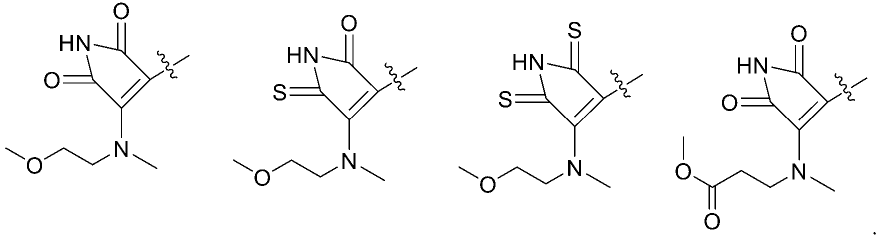

In some embodiments, the present disclosure provides the amino lipid compound of formula (IA), or a pharmaceutically acceptable salt thereof, or a stereoisomer thereof, as described above, wherein R₃ is selected from: 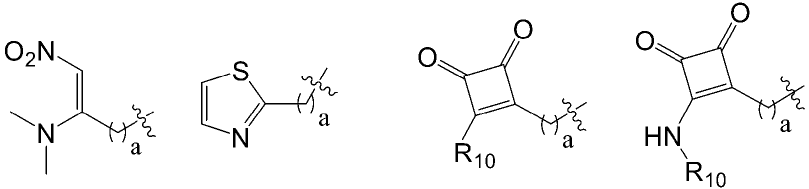 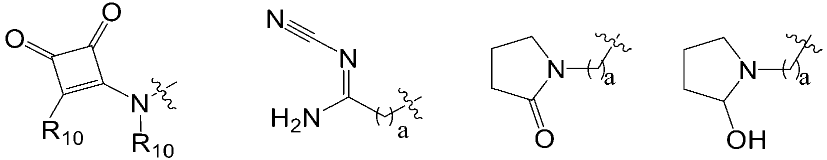 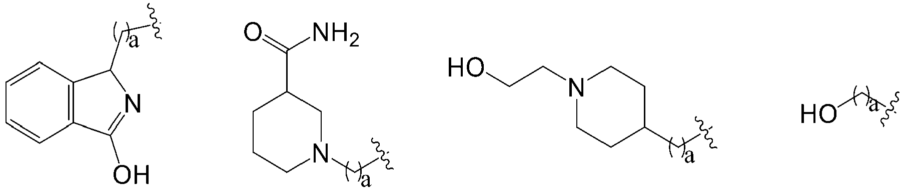 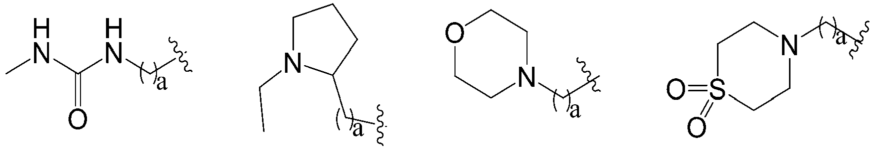 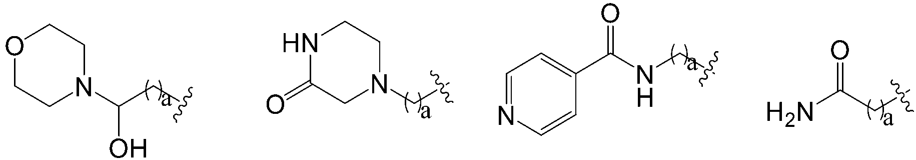 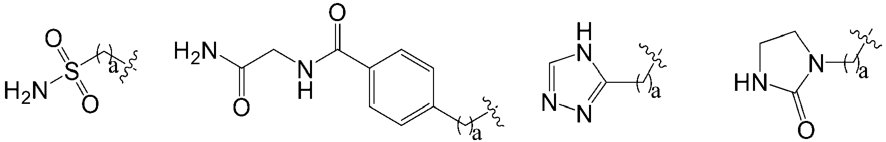 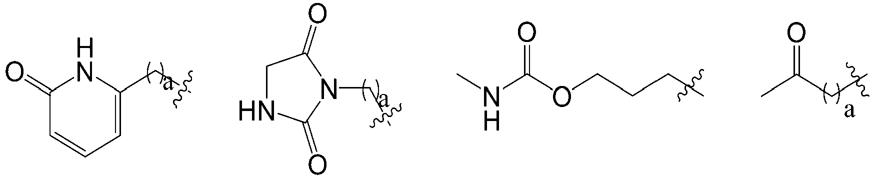 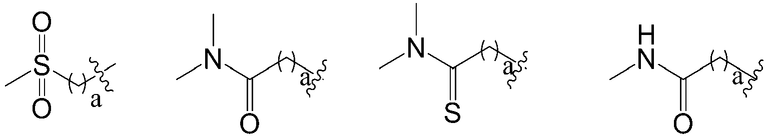 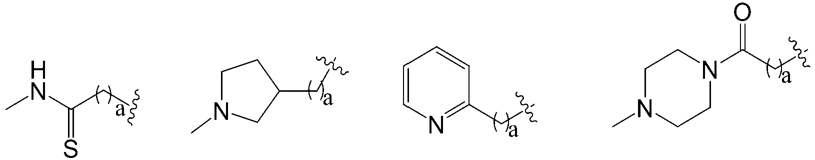 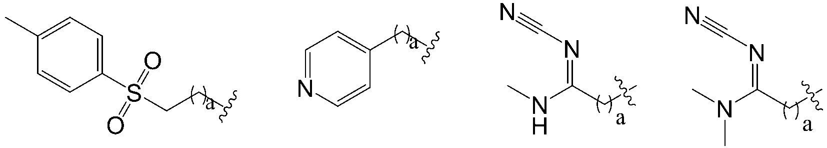 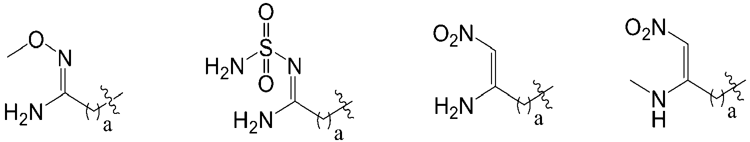 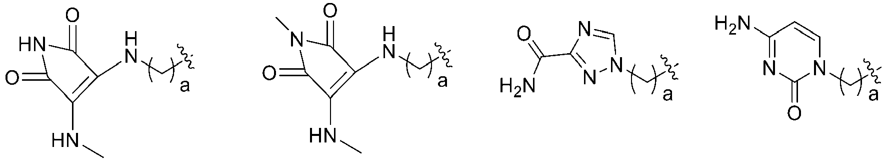 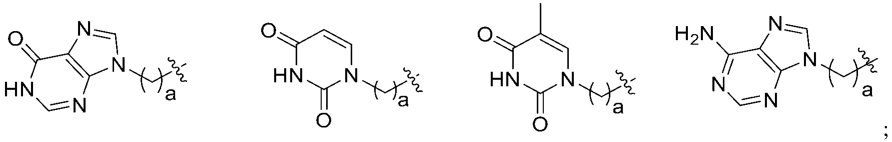
wherein a is 0, 1, 2, 3, 4, or 5; and
each R₁₀ is independently H or C₁-C₃ hydrocarbyl. In some embodiments, each R₁₀ is independently C₁-C₃ hydrocarbyl. In some embodiments, each R₁₀ is independently C₁, C₂, or C₃ alkyl, or C₂ or C₃ alkenyl, or C₂ or C₃ alkynyl.

In some embodiments, the present disclosure provides the amino lipid compound of formula (IA), or a pharmaceutically acceptable salt thereof, or a stereoisomer thereof, as described above, wherein R₁₃ is H.

**In** some embodiments, the present disclosure provides the amino lipid compound of formula (IA), or a pharmaceutically acceptable salt thereof, or a stereoisomer thereof, as described above, wherein R₁₃ is C₁, C₂, C₃, C₄, C₅, or C₆ hydrocarbyl, or C₁, C₂, C₃, C₄, C₅, or C₆ heterohydrocarbyl.

In some embodiments, the present disclosure provides the amino lipid compound of formula (IA), or a pharmaceutically acceptable salt thereof, or a stereoisomer thereof, as described above, wherein R₁₃ is C₁, C₂, C₃, C₄, C₅, or C₆ alkyl.

In some embodiments, R₁₃ is C₁ or C₂ alkyl.

In some embodiments, R₁₃ is C₃ or C₄ alkyl.

In some embodiments, R₁₃ is C₅ or C₆ alkyl.

In some embodiments, the present disclosure provides the amino lipid compound of formula (IA), or a pharmaceutically acceptable salt thereof, or a stereoisomer thereof, as described above, wherein A₁ is -N(R₄)-, -C(=O)O-, -OC(=O)-, -OC(=O)O-, -O-, -C(=O)-, -S-, -C(=O)S-, -SC(=O)-, -C(=O)N(R₄)-, -N(R₄)C(=O)-, - OC(=O)S-, -SC(=O)O-, -SC(=O)S-, -OC(=O)N(R₄)-, -N(R₄)C(=O)O-, -N(R₄)C(=O)N(R₄)-, -SC(=O)N(R₄)-, -N(R₄)C(=O)S-, -N(C(=O)L₁₃OR₄)-, -N(C(=O)L₁₃SR₄)-, -N(C(=O)R₄), -N(L₁₃OR₄)-, -N(L₁₃SR₄)-, - OS(=O)O-, -OS(=O)₂O-, -OP(=O)O-, -OP(=O)(OH)O-, -OP(=O)(H)O-, -OS(=O)₂NH-, -NHS(=O)₂O, - OC(=O)C(=O)O-, -N(R₄)C(=O)C(=O)O-, -OC(=O)C(=O)N(R₄)-, -N(R₄)C(=O)C(=O)N(R₄)-,

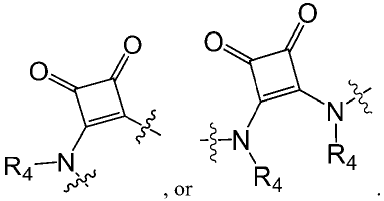

In some embodiments, one or more of L₁, L₂, L₃, L₄, L₅, and L₆ are substituted with one or more substituents (e.g., L₁ is substituted with one substituent, or L₁ is substituted with a plurality of substituents; for example, L₁ and L₂ are substituted with one or more substituents, when L₁ and L₂ are substituted with one substituent, it is included that L₁ is substituted with one substituent, or L₂ is substituted with one substituent; when L₁ and L₂ are substituted with a plurality of substituents, the plurality of substitutions occur only on L₁, or only on L₂, or on both L₁ and L₂), and the substituents include, but are not limited to, -OH, - SH, halogen, O, S, N, -CN, cyclohydrocarbyl, aryl, heterocyclyl, -R_{b}, -RₐN(R_{b})₂, -RₐOR_{b}, -RₐSR_{b}, - RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RₐC(=O)OR_{b}, -OC(=O)R_{b}, -C(=O)OR_{b}, -RₐSR_{b}, or 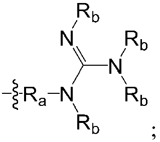 when L₁, L₂, L₃, L₄, L₅, or L₆ is substituted with a substituent including -R_{b}, -RₐOR_{b}, -RₐSR_{b}, - RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RₐC(=O)OR_{b}, -OC(=O)R_{b}, -C(=O)OR_{b}, -RₐSR_{b}, or 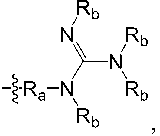 Rₐ and/or R_{b} may be attached to a C atom on L₁, L₂, L₃, L₄, L₅, or L₆ to form a three-, four-, five-, or six-membered ring.

In some embodiments, A₂ is -N(R₄)-, -C(=O)O-, -OC(=O)-, -OC(=O)O-, -0-, -C(=O)-, -S-, -C(=O)S-, - SC(=O)-, -C(=O)N(R₄)-, -N(R₄)C(=O)-, -OC(=O)S-, -SC(=O)O-, -SC(=O)S-, -OC(=O)N(R₄)-, - N(R₄)C(=O)O-, -N(R₄)C(=O)N(R₄)-, -SC(=O)N(R₄)-, -N(R₄)C(=O)S-, -N(C(=O)L₁₃OR₄)-, - N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, - OP(=O)(OH)O-, -OP(=O)(H)O-, -OS(=O)₂NH-, -NHS(=O)₂O-, -OC(=O)C(=O)O-, -N(R₄)C(=O)C(=O)O-, -OC(=O)C(=O)N(R₄)-, -N(R₄)C(=O)C(=O)N(R₄)-,

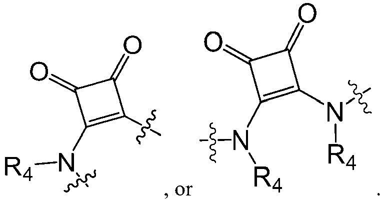

In some embodiments, Z is a carbocyclic ring, a heterocyclic ring, -CN, -OR₈, -OL₁₆N(R₈)₂, -C(=O)R₈, -C(=O)SR₈, -OC(=O)R₈, -OC(=O)OR₈, -OL₁₆OR₈, -N(R₈)₂, -C(=O)N(R₈)₂, -C(=S)N(R₈)₂, -S(=O)₂R₈, - S(=O)₂N(R₈)₂, -OC(=O)N(R₈)₂, -C(NR₈)N(R₈)₂, -C(NR₈)R₈, -C(=O)N(R₈)OR₈, -CH(R₈)N(R₈)C(=O)OR₈, - C(R₈)₃, -N(R₈)C(=O)R₈, -N(R₈)C(=O)OR₈, -N(R₈)S(=O)₂R₈, -N(R₈)C(=O)N(R₈)₂, -N(R₈)C(=S)N(R₈)₂, - N(R₈)C(NR₈)N(R₈)₁, -N(R₈)C(CHR₈)N(R₈)₂, -N(OR₈)C(=O)R₈, -N(OR₈)S(=O)₂R₈, -N(OR₈)C(=O)OR₈, - N(OR₈)C(=O)N(R₈)₂, -N(OR₈)C(=S)N(R₈)₂, -N(OR₈)C(NR₈)N(R₈)₁, -N(OR₈)C(CHR₈)N(R₈)₁, - OP(=O)(OR₈)₂, -P(=O)(OR₈)₂, -C(=N-CN)N(R₈)₂, -C(=N-O-CH₃)N(R₈)₂, -C(=N-SO₂-NH₂)N(R₈)₂, or - C(=CH-NO₂)N(R₈)₂.

In some embodiments, Z is

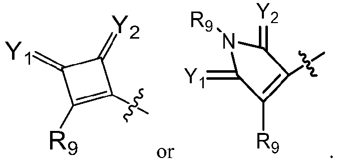

In some embodiments, Z is selected from:

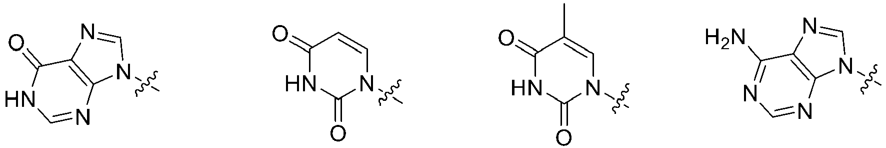

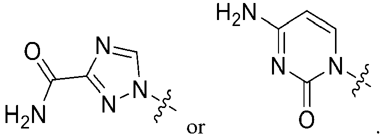

In some embodiments, L₁₅ is a bond.

In some embodiments, A₁ is -N(R₄)-.

In some embodiments, A₁ is -NH-.

In some embodiments, at least 2, e.g., 2, 3, 4, 5, or 6, of A₁, A₂, A₃, A₄, A₅, and A₆ are each independently -N(R₄)-, -C(=O)O-, -OC(=O)-, -OC(=O)O-, -O-, -C(=O)-, -S-, -C(=O)S-, -SC(=O)-, -C(=O)N(R₄)-, - N(R₄)C(=O)-, -OC(=O)S-, -SC(=O)O-, -SC(=O)S-, -OC(=O)N(R₄)-, -N(R₄)C(=O)O-, -N(R₄)C(=O)N(R₄)-, -SC(=O)N(R₄)-, -N(R₄)C(=O)S-, -N(C(=O)L₁₃OR₄)-, -N(C(=O)L₁₃OR₄)-, -N(C(=O)R₄), -N(L₁₃OR₄)-, - N(L₁₃SR₄)-, -OS(=O)O-, -OS(=O)₂O-, -OP(=O)O-, -OP(=O)(OH)O-, -OP(=O)(H)O-, -OS(=O)₂NH-, - NHS(=O)₂O-, -OC(=O)C(=O)O-, -N(R₄)C(=O)C(=O)O-, -OC(=O)C(=O)N(R₄)-, -N(R₄)C(=O)C(=O)N(R₄)-,

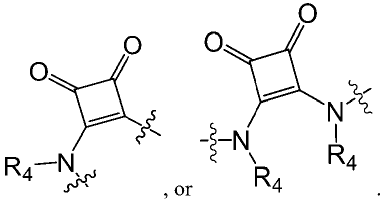

In some embodiments, the present disclosure provides the amino lipid compound of formula (IA), or a pharmaceutically acceptable salt thereof, or a stereoisomer thereof, as described above, wherein L₁, L₂, L₇, L₈, L₁₁, L₁₂, R₁, R₂, R₄, R₇, or R₉ is each independently substituted with one or more substituents which include, but are not limited to, hydroxyl, halogen, alkyl, alkenyl, alkynyl, -CN, oxygen, sulfur, nitrogen, an ester group, aryl, cycloalkyl, cycloalkenyl, amido, alkoxyl, or alkylthio.

In some embodiments, L₁, L₂, L₇, L₈, L₁₁, L₁₂, R₁, R₂, R₄, R₇, or R₉ is each independently substituted with one or more substituents which include, but are not limited to, -OH, -SH, halogen, O, S, N, -CN, cyclohydrocarbyl, aryl, heterocyclyl, -R_{b}, -RₐN(R_{b})₂, -RₐOR_{b}, -RₐSR_{b}, -RₐOC(=O)OR_{b}, -RₐOC(=O)SR_{b}, - RₐC(=O)N(R_{b})₂, -OC(=O)OR_{b}, -OC(=O)SR_{b}, -C(=O)N(R_{b})₂, -RₐOC(=O)R_{b}, -RₐC(=O)OR_{b}, -OC(=O)R_{b}, - C(=O)OR_{b}, -RₐSR_{b}, or 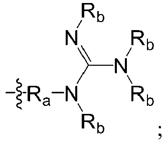 when L₁, L₂, L₇, L₈, L₁₁, L₁₂, R₁, R₂, R₄, R₇, or R₉ is each independently substituted with a substituent including -R_{b}, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, - OC(=O)SR_{b}, -C(=O)N(R_{b})₂, -RₐOC(=O)R_{b}, -RₐC(=O)OR_{b}, -OC(=O)R_{b}, -C(=O)OR_{b}, -RₐSR_{b}, or 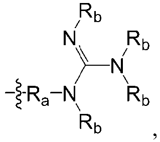 Rₐ and/or R_{b} may be attached to a C atom on L₁, L₂, L₇, L₈, L₁₁, L₁₂, R₁, R₂, R₄, R₇, or R₉ to form a three-, four-, five-, or six-membered ring;
wherein:
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds.

In some embodiments, in the amino lipid compound represented by formula (IA), or a pharmaceutically acceptable salt thereof, or a stereoisomer thereof, none of L₁, L₂, L₃, L₄, L₅, A₁, A₂, A₃, A₄, A₅, and A₆ is a bond.

In some embodiments, in the amino lipid compound represented by formula (IA), or a pharmaceutically acceptable salt thereof, or a stereoisomer thereof, none of L₁, L₂, L₃, L₄, A₁, A₂, A₃, A₄, and A₅ is a bond, and L₆ and A₆ is a bond.

In some embodiments, in the amino lipid compound represented by formula (IA), or a pharmaceutically acceptable salt thereof, or a stereoisomer thereof, none of L₁, L₂, L₃, A₁, A₂, A₃, and A₄ is a bond, and L₅, L₆, A₅, and A₆ are a bond.

In some embodiments, in the amino lipid compound represented by formula (IA), or a pharmaceutically acceptable salt thereof, or a stereoisomer thereof, the amino lipid compound has a structure represented by formula (I-1): 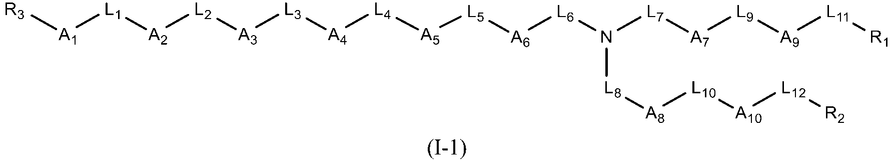 wherein the definitions of L₁, L₂, L₃, L₄, L₅, L₆, L₇, L₈, L₉, L₁₀, L₁₁, L₁₂, A₁, A₂, A₃, A₄, A₅, A₆, A₇, A₈, A₉, A₁₀, R₁, R₂, and R₃ are as defined in formula (IA).

In some embodiments, in the amino lipid compound represented by formula (I-1), or a pharmaceutically acceptable salt thereof, or a stereoisomer thereof, the definitions of L₁, L₂, L₃, L₄, L₅, L₆, L₇, L₈, L₉, L₁₀, L₁₁, L₁₂, A₁, A₂, A₃, A₄, A₅, A₆, A₇, A₈, A₉, A₁₀, and R₃ are as defined in formula (IA), R₁ and/or R₂ are/is - C(R₆)(OL₁₄A₁₂R₇)₂, -C(R₆)(SL₁₄A₁₂R₇)₂, or -C(R₆)(SL₁₄A₁₂R₇)(OL₁₄A₁₂R₇), and the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or - C(R₆)(OC(=O)L₁₄A₁₂R₇)R₇. In some embodiments, each R₆ is independently C₁, C₂, C₃, C₄, C₅, or C₆ alkyl. In some embodiments, each R₆ is independently C₁, C₂, or C₃ alkyl.

In some embodiments, in the amino lipid compound represented by formula (I-1), or a pharmaceutically acceptable salt thereof, or a stereoisomer thereof, the definitions of L₁, L₂, L₃, L₄, L₅, L₆, L₇, L₈, L₉, L₁₀, L₁₁, L₁₂, A₁, A₂, A₃, A₄, A₅, A₆, A₇, A₈, A₉, A₁₀, R₁, R₂, and R₃ are as defined in formula (IA), and none of A₁, A₂, A₃, A₄, A₅, A₆, L₁, L₂, L₃, L₄, and L₅ is a bond.

In some embodiments, in the amino lipid compound represented by formula (I-1), or a pharmaceutically acceptable salt thereof, or a stereoisomer thereof, the definitions of L₁, L₂, L₃, L₄, L₅, L₆, L₇, L₈, L₉, L₁₀, L₁₁, L₁₂, A₇, A₈, A₉, A₁₀, R₁, R₂, and R₃ are as defined in formula (IA), at least 3, e.g., 3, 4, 5, or 6, of A₁, A₂, A₃, A₄, A₅, and A₆ are each independently -N(R₄)-, -C(=O)O-, -OC(=O)-, -OC(=O)O-, -O-, -C(=O)-, -S-, - C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, - N(R₄)C(=O)O-, -N(R₄)C(=O)N(R₄)-, -SC(=O)N(R₄)-, -N(R₄)C(=O)S-, -N(C(=O)L₁₃OR₄)-, - N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, - OP(=O)(OH)O-, -OP(=O)(H)O-, -OS(=O)₂NH-, -NHS(=O)₂O-, -OC(=O)C(=O)O-, -N(R₄)C(=O)C(=O)O-, -OC(=O)C(=O)N(R₄)-, -N(R₄)C(=O)C(=O)N(R₄)-, 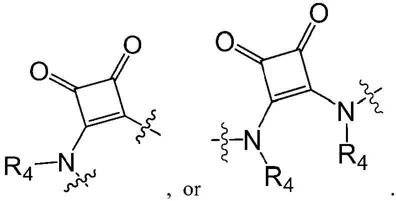 In some embodiments, none of L₁, L₂, L₃, L₄, or L₅ is a bond.

In some embodiments, in the amino lipid compound represented by formula (IA), or a pharmaceutically acceptable salt thereof, or a stereoisomer thereof, the amino lipid compound has a structure represented by formula (I-2): 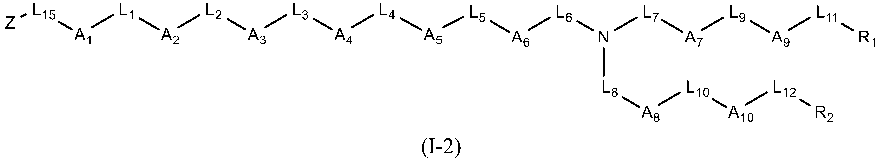 wherein the definitions of L₁, L₂, L₃, L₄, L₅, L₆, L₇, L₈, L₉, L₁₀, L₁₁, L₁₂, A₁, A₂, A₃, A₄, A₅, A₆, A₇, A₈, A₉, A₁₀, R₁, R₂, and L₁₅ are as defined in formula (IA), Z is 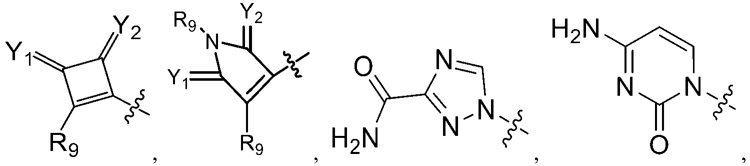 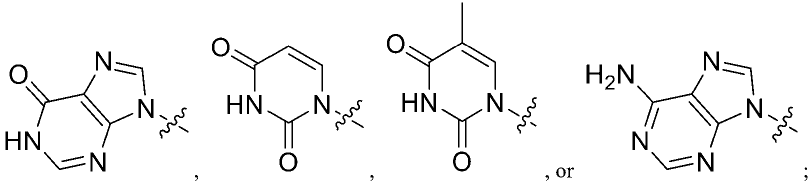 and
at least 2, e.g., 2, 3, 4, 5, or 6, of A₁, A₂, A₃, A₄, A₅, and A₆ are each independently -N(R₄)-, -C(=O)O-, -OC(=O)-, -OC(=O)O-, -O-, -C(=O)-, -S-, -C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, -N(R₄)C(=O)O-, -N(R₄)C(=O)N(R₄)-, -SC(=O)N(R₄)-, - N(R₄)C(=O)S-, -N(C(=O)Li₃OR₄)-, -N(C(=O)L₁₃SR₄)-, -N(C(=O)R₄), -N(L₁₃OR₄)-, -N(L₁₃SR₄)-, - OS(=O)O-, -OS(=O)₂O-, -OP(=O)O-, -OP(=O)(OH)O-, -OP(=O)(H)O-, -OS(=O)₂NH-, -NHS(=O)₂O-, - OC(=O)C(=O)O-, -N(R₄)C(=O)C(=O)O-, -OC(=O)C(=O)N(R₄)-, -N(R₄)C(=O)C(=O)N(R₄)-, 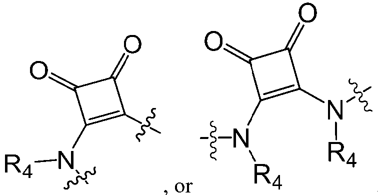 In some embodiments, R₁ and/or R₂ are/is -C(R₆)(OL₁₄A₁₂R₇)₂, -C(R₆)(SL₁₄A₁₂R₇)₂, or -C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently - C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or - C(R₆)(OC(=O)L₁₄A₁₂R₇)R₇.

In some embodiments, in the amino lipid compound represented by formula (I-2), or a pharmaceutically acceptable salt thereof, or a stereoisomer thereof, the definitions of L₁, L₂, L₃, L₄, L₅, L₆, L₇, L₈, L₉, L₁₀, L₁₁, L₁₂, A₁, A₂, A₃, A₄, A₅, A₆, A₇, A₈, A₉, A₁₀, R₁, R₂, and L₁₅ are as defined in formula (IA),
Z is 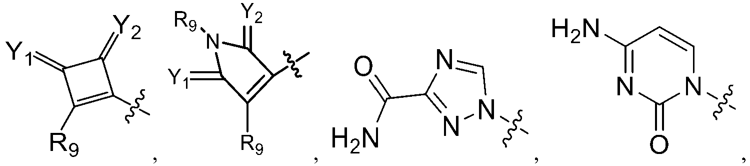 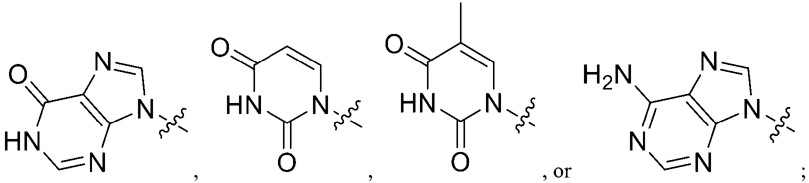 one or more of L₁, L₂, L₃, L₄, L₅, and L₆ are substituted with one or more substituents which include, but are not limited to, -OH, -SH, halogen, O, S, N, -CN, cyclohydrocarbyl, aryl, heterocyclyl, -R_{b}, -RₐN(R_{b})₂, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, -OC(=O)SR_{b}, - C(=O)N(R_{b})₂, -RₐOC(=O)R_{b}, -RₐC(=O)OR_{b}, -OC(=O)R_{b}, -C(=O)OR_{b}, -RₐSR_{b}, or 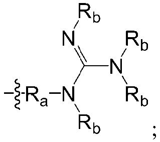 when one or more of L₁, L₂, L₃, L₄, L₅, and L₆ are substituted with a substituent including -R_{b}, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RₐC(=O)OR_{b}, -OC(=O)R_{b}, -C(=O)OR_{b}, -RₐSR_{b}, or 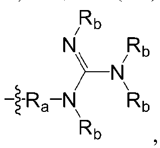 Rₐ and/or R_{b} may be attached to a C atom on L₁, L₂, L₃, L₄, L₅, or L₆ to form a three-, four-, five-, or six-membered ring; wherein:
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds. In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, in the amino lipid compound represented by formula (IA), or a pharmaceutically acceptable salt thereof, or a stereoisomer thereof, the amino lipid compound has a structure represented by formula (I-3): 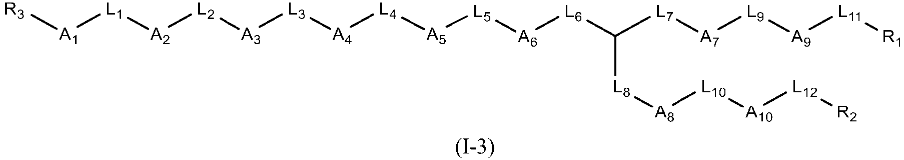 wherein the definitions of A₁, A₂, A₃, A₄, A₅, A₆, A₇, A₈, A₉, A₁₀, L₁, L₂, L₃, L₄, L₅, L₆, L₇, L₈, L₉, L₁₀, L₁₁, L₁₂, R₁, R₂, and R₃ are as defined in formula (IA), and at least one of A₁, A₂, A₃, A₄, A₅, and A₆ is -N(R₄)-, and R₄ is as defined in formula (IA).

In some embodiments, in the amino lipid compound represented by formula (I-3), or a pharmaceutically acceptable salt thereof, or a stereoisomer thereof, the definitions of A₁, A₂, A₃, A₄, A₅, A₆, A₇, A₈, A₉, A₁₀, L₁, L₂, L₃, L₄, L₅, L₆, L₇, L₈, L₉, L₁₀, L₁₁, L₁₂, and R₃ are as defined in formula (IA), and at least one of A₁, A₂, A₃, A₄, A₅, and A₆ is -N(R₄)-, R₄ is as defined in formula (IA), R₁ and/or R₂ are/is -C(R₆)(OL₁₄A₁₂R₇)₂, - C(R₆)(SL₁₄A₁₂R₇)₂, or -C(R₆)(SL₁₄A₁₂R₇)(OL₁₄A₁₂R₇), and the definitions of R₆, L₁₄, A₁₂, and R₇ are as defined in formula (IA). In some embodiments, R₁ and R₂ may further be each independently - C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or - C(R₆)(OC(=O)L₁₄A₁₂R₇)R₇. In some embodiments, each R₆ is independently C₁, C₂, C₃, C₄, C₅, or C₆ alkyl. In some embodiments, each R₆ is independently C₁, C₂, or C₃ alkyl.

In some embodiments, in the amino lipid compound represented by formula (I-3), or a pharmaceutically acceptable salt thereof, or a stereoisomer thereof, the definitions of L₁, L₂, L₃, L₄, L₅, L₆, L₇, L₈, L₉, L₁₀, L₁₁, L₁₂, A₁, A₂, A₃, A₄, A₅, A₆, A₇, As, A₉, A₁₀, R₁, R₂, and R₃ are as defined in formula (IA), wherein at least one of A₁, A₂, A₃, A₄, A₅, and A₆ is -N(R₄)-, R₄ is as defined in formula (IA), and none of A₁, A₂, A₃, A₄, A₅, A₆, L₁, L₂, L₃, L₄, and L₅ is a bond.

In some embodiments, in an amino lipid compound represented by formula (I-3), or a pharmaceutically acceptable salt thereof, or a stereoisomer thereof, L₁, L₂, L₃, L₄, L₅, L₆, L₇, L₈, L₉, L₁₀, L₁₁, L₁₂, A₇, A₈, A₉, A₁₀, R₁, R₂, and R₃ are as defined in formula (IA), at least 3, e.g., 3, 4, 5, or 6, of A₁, A₂, A₃, A₄, A₅, and A₆ are each independently -N(R₄)-, -C(=O)O-, -OC(=O)-, -OC(=O)O-, -O-, -C(=O)-, -S-, -C(=O)S-, -SC(=O)-, -C(=O)N(R₄)-, -N(R₄)C(=O)-, -OC(=O)S-, -SC(=O)O -, -SC(=O)S-, -OC(=O)N(R₄)-, -N(R₄)C(=O)O-, - N(R₄)C(=O)N(R₄)-, -SC(=O)N(R₄)-, -N(R₄)C(=O)S-, -N(C(=O)L₁₃OR₄)-, -N(C(=O)L₁₃SR₄)-, -N(C(=O)R₄), -N(L₁₃OR₄)-, -N(Li₃SR₄)-, -OS(=O)O-, -OS(=O)₂O-, -OP(=O)O-, -OP(=O)(OH)O-, -OP(=O)(H)O-, - OS(=O)₂NH-, -NHS(=O)₂O-, -OC(=O)C(=O)O-, -N(R₄)C(=O)C(=O)O-, -OC(=O)C(=O)N(R₄)-, - N(R₄)C(=O)C(=O)N(R₄)-, 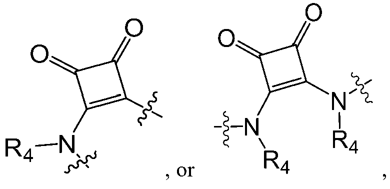 and at least one of the "at least 3" is -N(R₄)-, and R₄ is as defined in formula (IA). In some embodiments, none of L₁, L₂, L₃, L₄, or L₅ is a bond.

In some embodiments, in the amino lipid compound represented by formula (IA), or a pharmaceutically acceptable salt thereof, or a stereoisomer thereof, the amino lipid compound has a structure represented by formula (I-4): 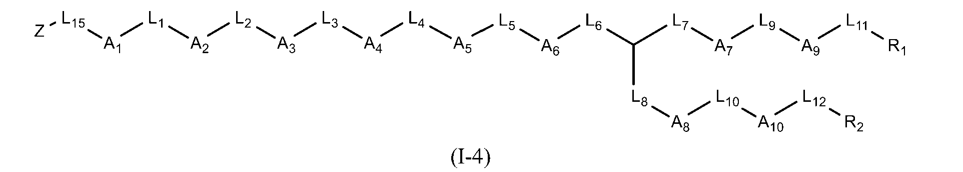 wherein the definitions of L₁, L₂, L₃, L₄, L₅, L₆, L₇, L₈, L₉, L₁₀, L₁₁, L₁₂, A₁, A₂, A₃, A₄, A₅, A₆, A₇, A₈, A₉, A₁₀, R₁, R₂, and L₁₅ are as defined in formula (IA),
Z is 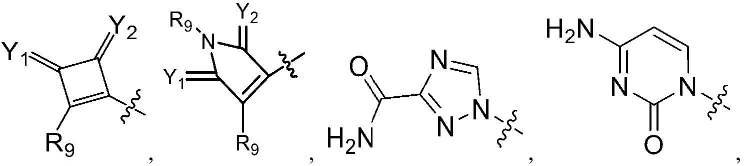 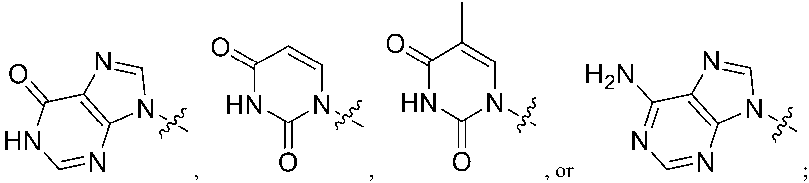 and at least 2, e.g., 2, 3, 4, 5, or 6, of A₁, A₂, A₃, A₄, A₅, and A₆ are each independently -N(R₄)-, -C(=O)O-, -OC(=O)-, -OC(=O)O-, -O-, -C(=O)-, -S-, -C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, -N(R₄)C(=O)O-, -N(R₄)C(=O)N(R₄)-, -SC(=O)N(R₄)-, - N(R₄)C(=O)S-, -N(C(=O)L₁₃OR₄)-, -N(C(=O)L₁₃SR₄)-, -N(C(=O)R₄), -N(L₁₃OR₄)-, -N(L₁₃SR₄)-, - OS(=O)O-, -OS(=O)₂O-, -OP(=O)O-, -OP(=O)(OH)O-, -OP(=O)(H)O-, -OS(=O)₂NH-, -NHS(=O)₂O-, - OC(=O)C(=O)O-, -N(R₄)C(=O)C(=O)O-, -OC(=O)C(=O)N(R₄)-, -N(R₄)C(=O)C(=O)N(R₄)-, 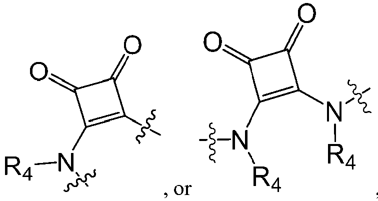 and at least one of the "at least 2" is -N(R₄)-, and R₄ is as defined in formula (IA). In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, the amino lipid compound represented by formula (IA), or a pharmaceutically acceptable salt thereof, or a stereoisomer thereof, has a structure represented by formula (I-4), wherein the definitions of L₁, L₂, L₃, L₄, L₅, L₆, L₇, L₈, L₉, L₁₀, L₁₁, L₁₂, A₁, A₂, A₃, A₄, A₅, A₆, A₇, A₈, A₉, A₁₀, R₁, R₂, and L₁₅ are as defined in formula (IA), and at least one of A₁, A₂, A₃, A₄, A₅, and A₆ is -N(R₄)-, R₄ is as defined in formula (IA),
Z is 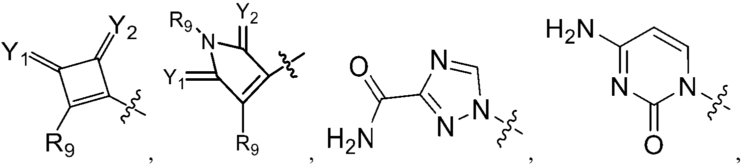 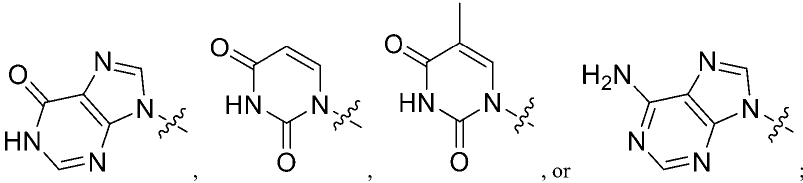 and one or more of L₁, L₂, L₃, L₄, L₅, and L₆ are substituted with one or more substituents which include, but are not limited to, -OH, -SH, halogen, O, S, N, -CN, cyclohydrocarbyl, aryl, heterocyclyl, -R_{b}, -RₐN(R_{b})₂, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, -OC(=O)SR_{b}, - C(=O)N(R_{b})₂, -RₐOC(=O)R_{b}, -RₐC(=O)OR_{b}, -OC(=O)R_{b}, -C(=O)OR_{b}, -RₐSR_{b}, or 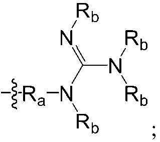 when one or more of L₁, L₂, L₃, L₄, L₅, and L₆ are substituted with a substituent including -R_{b}, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RₐC(=O)OR_{b}, -OC(=O)R_{b}, -C(=O)OR_{b}, -RₐSR_{b}, or 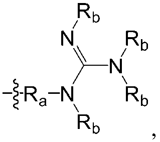 Rₐ and/or R_{b} may be attached to a C atom on L₁, L₂, L₃, L₄, L₅, or L₆ to form a three-, four-, five-, or six-membered ring;
wherein:
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds. In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, in the amino lipid compound represented by formula (IA), or a pharmaceutically acceptable salt thereof, or a stereoisomer thereof, the amino lipid compound has a structure represented by formula (IV): 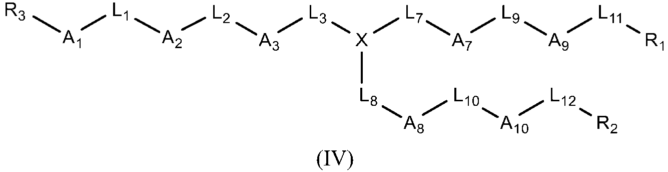 wherein the definitions of L₁, L₂, L₃, L₇, L₈, L₉, L₁₀, L₁₁, L₁₂, A₁, A₂, A₃, A₇, A₈, A₉, A₁₀, R₁, R₂, and R₃ are as defined in formula (IA).

In some embodiments, in the amino lipid compound represented by formula (IA), or a pharmaceutically acceptable salt thereof, or a stereoisomer thereof, the amino lipid compound has a structure represented by formula (IV-1): 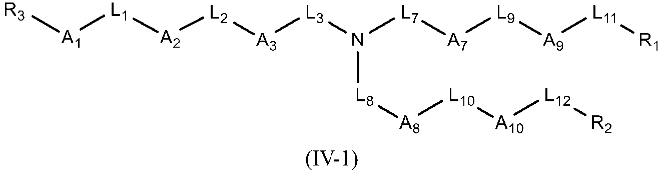 wherein the definitions of L₁, L₂, L₃, L₇, L₈, L₉, L₁₀, L₁₁, L₁₂, A₁, A₂, A₃, A₇, A₈, A₉, A₁₀, R₁, R₂, and R₃ are as defined in formula (IA).

In some embodiments, in the amino lipid compound represented by formula (IV-1), or a pharmaceutically acceptable salt thereof, or a stereoisomer thereof, the definitions of L₁, L₂, L₃, L₇, L₈, L₉, L₁₀, L₁₁, L₁₂, A₁, A₂, A₃, A₇, A₈, A₉, A₁₀, and R₃ are as defined in formula (IA), R₁ and/or R₂ are/is - C(R₆)(OL₁₄A₁₂R₇)₂, -C(R₆)(SL₁₄A₁₂R₇)₂, or -C(R₆)(SL₁₄A₁₂R₇)(OL₁₄A₁₂R₇), and the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or - C(R₆)(OC(=O)L₁₄A₁₂R₇)R₇. In some embodiments, each R₆ is independently C₁, C₂, C₃, C₄, C₅, or C₆ alkyl. In some embodiments, each R₆ is independently C₁, C₂, or C₃ alkyl.

In some embodiments, in the amino lipid compound represented by formula (IV-1), or a pharmaceutically acceptable salt thereof, or a stereoisomer thereof, the definitions of L₁, L₂, L₃, L₇, L₈, L₉, L₁₀, L₁₁, L₁₂, A₁, A₂, A₃, A₇, A₈, A₉, A₁₀, R₁, R₂, and R₃ are as defined in formula (IA), and none of A₁, A₂, A₃, L₁, and L₂ is a bond.

In some embodiments, in the amino lipid compound represented by formula (IV-1), or a pharmaceutically acceptable salt thereof, or a stereoisomer thereof, the definitions of L₁, L₂, L₃, L₇, L₈, L₉, L₁₀, L₁₁, L₁₂, A₇, A₈, A₉, A₁₀, R₁, R₂, and R₃ are as defined in formula (IA), and A₁, A₂, and A₃ are each independently -N(R₄)-, -C(=O)O-, -OC(=O)-, -OC(=O)O-, -O-, -C(=O)-, -S-, -C(=O)S-, -SC(=O)-, - C(=O')N(R₄)-, -N(R₄)C(=O)-, -OC(=O)S-, -SC(=O)O-, -SC(=O)S-, -OC(=O)N(R₄)-, -N(R₄)C(=O)O-, - N(R₄)C(=O)N(R₄)-, -SC(=O)N(R₄)-, -N(R₄)C(=O)S-, -N(C(=O)L₁₃OR₄)-, -N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, -OP(=O)(OH)O-, -OP(=O)(H)O-, - OS(=O)₂NH-, -NHS(=O)₂O-, -OC(=O)C(=O)O-, -N(R₄)C(=O)C(=O)O-, -OC(=O)C(=O)N(R₄)-, - N(R₄)C(=O)C(=O)N(R₄)-, 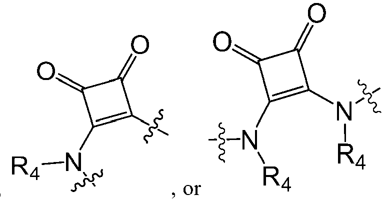 . In some embodiments, neither of L₁ and L₂ is a bond.

In some embodiments, in the amino lipid compound represented by formula (IA), or a pharmaceutically acceptable salt thereof, or a stereoisomer thereof, the amino lipid compound has a structure represented by formula (IV-2): 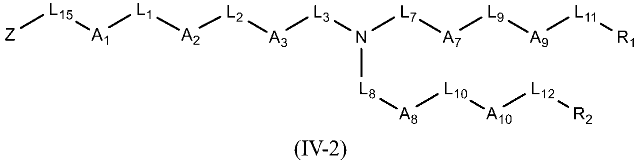 wherein the definitions of L₁, L₂, L₃, L₇, L₈, L₉, L₁₀, L₁₁, L₁₂, A₁, A₂, A₃, A₇, A₈, A₉, A₁₀, R₁, R₂, and L₁₅ are as defined in formula (IA),
Z is 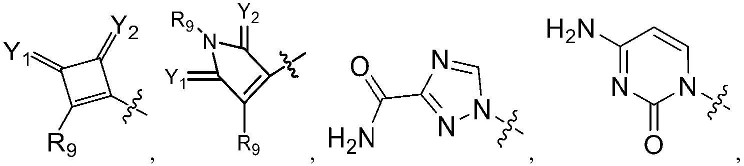 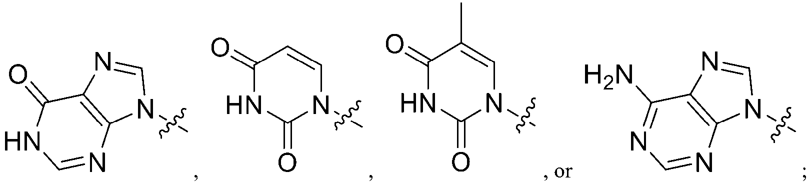 and at least 2 of A₁, A₂, and A₃ are each independently -N(R₄)-, -C(=O)O-, -OC(=O)-, -OC(=O)O-, -O-, - C(=O)-, -S-, -C(=O)S-, -SC(=O)-, -C(=O)N(R₄)-, -N(R₄)C(=O)-, -OC(=O)S-, -SC(=O)O-, -SC(=O)S-, - OC(=O)N(R₄)-, -N(R₄)C(=O)O-, -N(R₄)C(=O)N(R₄)-, -SC(=O)N(R₄)-, -N(R₄)C(=O)S-, -N(C(=O)L₁₃OR₄)-, -N(C(=O)Li₃SR₄)-, -N(C(=O)R₄), -N(L₁₃OR₄)-, -N(L₁₃SR₄)-, -OS(=O)O-, -OS(=O)₂O-, -OP(=O)O-, - OP(=O)(OH)O-, -OP(=O)(H)O-, -OS(=O)zNH-, -NHS(=O)₂O-, -OC(=O)C(=O)O-, -N(R₄)C(=O)C(=O)O-, -OC(=O)C(=O)N(R₄)-, -N(R₄)C(=O)C(=O)N(R₄)-, 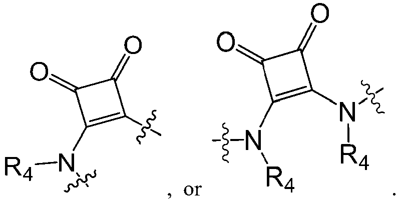 In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, in the amino lipid compound represented by formula (IV-2), or a pharmaceutically acceptable salt thereof, or a stereoisomer thereof, the definitions of L₁, L₂, L₃, L₇, L₈, L₉, L₁₀, L₁₁, L₁₂, A₁, A₂, A₃, A₇, A₈, A₉, A₁₀, R₁, R₂, and L₁₅ are as defined in formula (IA),
Z is 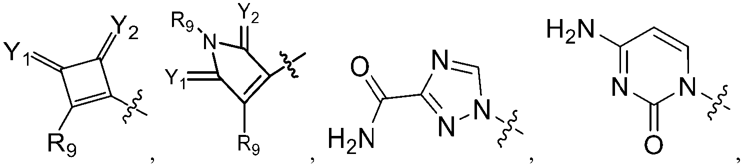 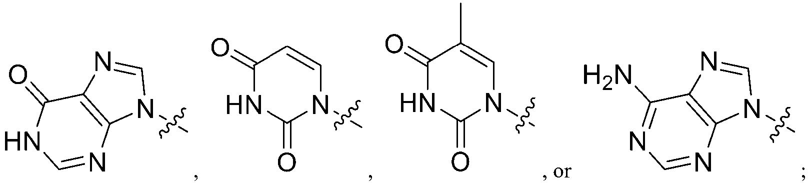 one or more of L₁, L₂, L₃ are substituted with one or more substituents which include, but are not limited to, -OH, -SH, halogen, O, S, N, -CN, cyclohydrocarbyl, aryl, heterocyclyl, -R_{b}, -RₐN(R_{b})₂, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RₐC(=O)OR_{b}, -OC(=O)R_{b}, -C(=O)OR_{b}, -RₐSR_{b}, or 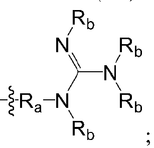 when one or more of L₁, L₂, L₃ are substituted with a substituent including -R_{b}, -RₐOR_{b}, -RₐSR_{b}, - RₐOC(=O)OR_{b}, -RₐOC(=O)SR_{b}, -RₐC(=O)N(R_{S})₂, -OC(=O)OR_{b}, -OC(=O)SR_{b}, -C(=O)N(R_{b})₂,-RₐOC(=O)R_{b}, -RₐC(=O)OR_{b}, -OC(=O)R_{b}, -C(=O)OR_{b}, -RₐSR_{b}, or 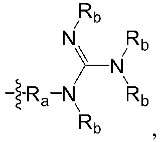 Rₐ and/or R_{b} may be attached to a C atom on L₁, L₂, or L₃ to form a three-, four-, five-, or six-membered ring;
wherein:
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds. In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, in the amino lipid compound represented by formula (IA), or a pharmaceutically acceptable salt thereof, or a stereoisomer thereof, the amino lipid compound has a structure represented by formula (IV-3): 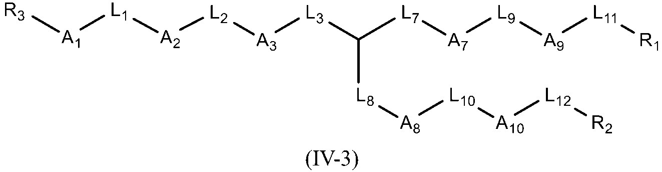 wherein A₁, A₂, A₃, A₇, A₈, A₉, A₁₀, L₁, L₂, L₃, L₇, L₈, L₉, L₁₀, L₁₁, L₁₂, R₁, R₂, and R₃ are as defined in formula (IA), and at least one of A₁, A₂, and A₃ is -N(R₄)-, and R₄ is as defined in formula (IA).

In some embodiments, in the amino lipid compound represented by formula (IV-3), or a pharmaceutically acceptable salt thereof, or a stereoisomer thereof, the definitions of A₁, A₂, A₃, A₇, A₈, A₉, A₁₀, L₁, L₂, L₃, L₇, L₈, L₉, L₁₀, L₁₁, L₁₂, and R₃ are as defined in formula (IA), and at least one of A₁, A₂, and A₃ is -N(R₄)-, R₄ is as defined in formula (IA), R₁ and/or R₂ are/is -C(R₆)(OL₁₄A₁₂R₇)₂, -C(R₆)(SL₁₄A₁₂R₇)₂, or -C(R₆)(SL₁₄A₁₂R₇)(OL₁₄A₁₂R₇), and the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇. In some embodiments, each R₆ is independently C₁, C₂, C₃, C₄, C₅, or C₆ alkyl. In some embodiments, each R₆ is independently C₁, C₂, or C₃ alkyl.

In some embodiments, in the amino lipid compound represented by formula (IV-3), or a pharmaceutically acceptable salt thereof, or a stereoisomer thereof, the definitions of L₁, L₂, L₃, L₇, L₈, L₉, L₁₀, L₁₁, L₁₂, A₁, A₂, A₃, A₇, A₈, A₉, A₁₀, R₁, R₂, and R₃ are as defined in formula (IA), wherein at least one of A₁, A₂, A₃, and A₄ is -N(R₄)-, R₄ is as defined in formula (IA), and none of A₁, A₂, A₃, L₁, and L₂ is a bond.

In some embodiments, in the amino lipid compound represented by formula (IV-3), or a pharmaceutically acceptable salt thereof, or a stereoisomer thereof, the definitions of L₁, L₂, L₃, L₇, L₈, L₉, L₁₀, L₁₁, L₁₂, A₇, A₈, A₉, A₁₀, R₁, R₂, and R₃ are as defined in formula (IA), A₁, A₂, and A₃ are each independently -N(R₄)-, -C(=O)O-, -OC(=O)-, -OC(=O)O-, -O-, -C(=O)-, -S-, -C(=O)S-, -SC(=O)-, - C(=O)N(R₄)-, -N(R₄)C(=O)-, -OC(=O)S-, -SC(=O)O-, -SC(=O)S-, -OC(=O)N(R₄)-, -N(R₄)C(=O)O-, - N(R₄)C(=O)N(R₄)-, -SC(=O)N(R₄)-, -N(R₄)C(=O)S-, -N(C(=O)L₁₃OR₄)-, -N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, -OP(=O)(OH)O-, -OP(=O)(H)O-, - OS(=O)₂NH-, -NHS(=O)₂O-, -OC(=O)C(=O)O-, -N(R₄)C(=O)C(=O)O-, -OC(=O)C(=O)N(R₄)-, - N(R₄)C(=O)C(=O)N(R₄)-, 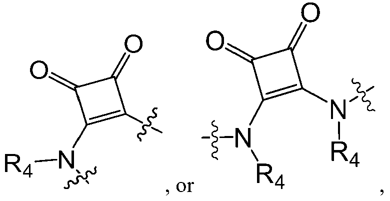 and at least one of A₁, A₂, and A₃ is - N(R₄)-, and R₄ is as defined in formula (IA). In some embodiments, neither of L₁ and L₂ is a bond.

In some embodiments, in the amino lipid compound represented by formula (IA), or a pharmaceutically acceptable salt thereof, or a stereoisomer thereof, the amino lipid compound has a structure represented by formula (IV-4): 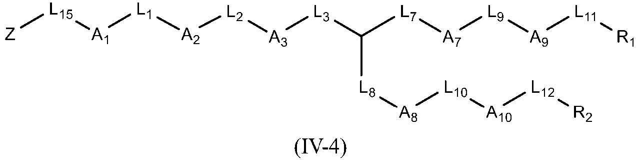 wherein the definitions of L₁, L₂, L₃, L₇, L₈, L₉, L₁₀, L₁₁, L₁₂, A₁, A₂, A₃, A₇, A₈, A₉, A₁₀, R₁, R₂, and L₁₅ are as defined in formula (IA), Z is 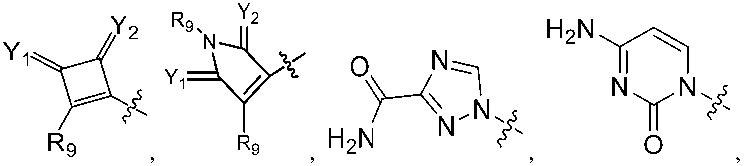 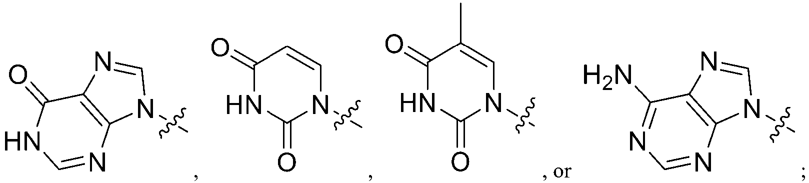 and at least 2, e.g., 2, 3, or 4, of A₁, A₂, and A₃ are each independently -N(R₄)-, -C(=O)O-, -OC(=O)-, - OC(=O)O-, -O-, -C(=O)-, -S-, -C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, -N(R₄)C(=O)O-, -N(R₄)C(=O)N(R₄)-, -SC(=O)N(R₄)-, -N(R₄)C(=O)S-, - N(C(=O)L₁₃OR₄)-, -N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, -OP(=O)(OH)O-, -OP(=O)(H)O-, -OS(=O)₂NH-, -NHS(=O)₂O-, -OC(=O)C(=O)O-, - N(R₄)C(=O)C(=O)O-, -OC(=O)C(=O)N(R₄)-, -N(R₄)C(=O)C(=O)N(R₄)-, 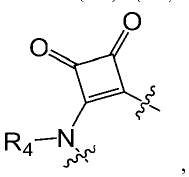 or 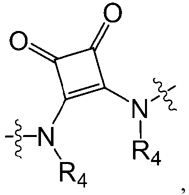 and at least one of the "at least 2" is -N(R₄)-, and R₄ is as defined in formula (IA). In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, the amino lipid compound represented by formula (IA), or a pharmaceutically acceptable salt thereof, or a stereoisomer thereof, has a structure represented by formula (IV-4), wherein the definitions of L₁, L₂, L₃, L₇, L₈, L₉, L₁₀, L₁₁, L₁₂, A₁, A₂, A₃, A₇, A₈, A₉, A₁₀, R₁, R₂, and L₁₅ are as defined in formula (IA), and at least one of A₁, A₂, and A₃ is -N(R₄)-, R₄ is as defined in formula (IA),
Z is 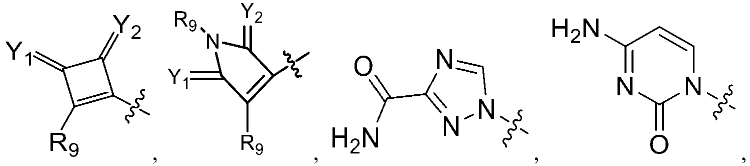 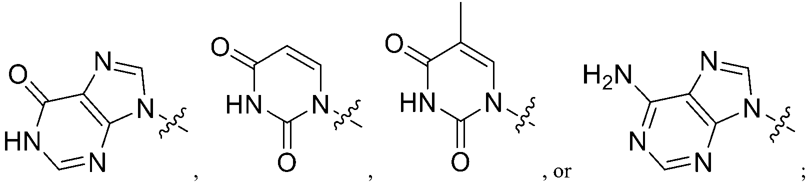 one or more of L₁, L₂, L₃ are substituted with one or more substituents which include, but are not limited to, -OH, -SH, halogen, O, S, N, -CN, cyclohydrocarbyl, aryl, heterocyclyl, -R_{b}, -RₐN(R_{b})₂, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RaC(=O)OR_{b}, -OC(=O)R_{b}, -C(=O)OR_{b}, -RₐSR_{b}, or 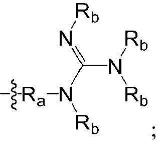 when one or more of L₁, L₂, L₃ are substituted with a substituent including -R_{b}, -RₐOR_{b}, -RₐSR_{b}, - RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RₐC(=O)OR_{b}, -OC(=O)R_{b}, -C(=O)OR_{b}, -RₐSR_{b}, or 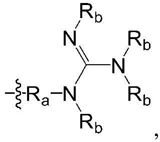 Rₐ and/or R_{b} may be attached to a C atom on L₁, L₂, or L₃ to form a three-, four-, five-, or six-membered ring;
wherein:
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds. In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, in the amino lipid compound represented by formula (IA), or a pharmaceutically acceptable salt thereof, or a stereoisomer thereof, the amino lipid compound has a structure represented by formula (V): 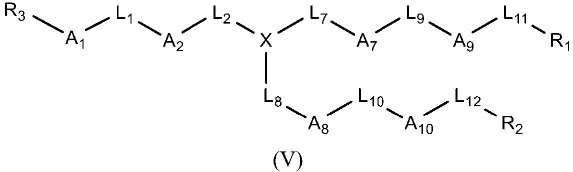 wherein the definitions of L₁, L₂, L₇, L₈, L₉, L₁₀, L₁₁, L₁₂, A₁, A₂, A₇, A₈, A₉, A₁₀, R₁, R₂, and R₃ are as defined in formula (IA).

In some embodiments, in the amino lipid compound represented by formula (IA), or a pharmaceutically acceptable salt thereof, or a stereoisomer thereof, the amino lipid compound has a structure represented by formula (V-1): 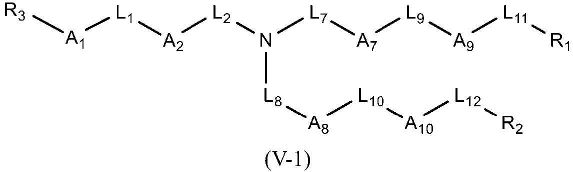 wherein the definitions of L₁, L₂, L₃, L₇, L₈, L₉, L₁₀, L₁₁, L₁₂, A₁, A₂, A₃, A₇, A₈, A₉, A₁₀, R₁, R₂, and R₃ are as defined in formula (IA).

In some embodiments, in the amino lipid compound represented by formula (V- 1), or a pharmaceutically acceptable salt thereof, or a stereoisomer thereof, the definitions of L₁, L₂, L₇, L₈, L₉, L₁₀, L₁₁, L₁₂, A₁, A₂, A₇, A₈, A₉, A_{10,} and R₃ are as defined in formula (IA), R₁ and/or R₂ are/is -C(R₆)(OL₁₄A₁₂R₇)₂, - C(R₆)(SL₁₄A₁₂R₇)₂, or -C(R₆)(SL₁₄A₁₂R₇)(OL₁₄A₁₂R₇), and the definitions of R₆, L₁₄, A₁₂, and R₇ are as defined in formula (IA). In some embodiments, R₁ and R₂ may further be each independently - C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or - C(R₆)(OC(=O)L₁₄A₁₂R₇)R₇. In some embodiments, each R₆ is independently C₁, C₂, C₃, C₄, C₅, or C₆ alkyl. In some embodiments, each R₆ is independently C₁, C₂, or C₃ alkyl.

In some embodiments, in the amino lipid compound represented by formula (V- 1), or a pharmaceutically acceptable salt thereof, or a stereoisomer thereof, the definitions of L₁, L₂, L₇, L₈, L₉, L₁₀, L₁₁, L₁₂, A₁, A₂, A₇, A₈, A₉, A₁₀, R₁, R₂, and R₃ are as defined in formula (IA), and none of A₁, A₂, A₃, and L₁ is a bond.

In some embodiments, in the amino lipid compound represented by formula (IA), or a pharmaceutically acceptable salt thereof, or a stereoisomer thereof, the amino lipid compound has a structure represented by formula (V-2): 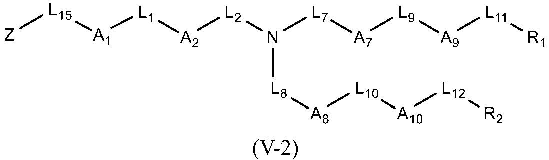 wherein the definitions of L₁, L₂, L₇, L₈, L₉, L₁₀, L₁₁, L₁₂, A₁, A₂, A₇, A₈, A₉, A₁₀, R₁, R₂, and L₁₅ are as defined in formula (IA), Z is 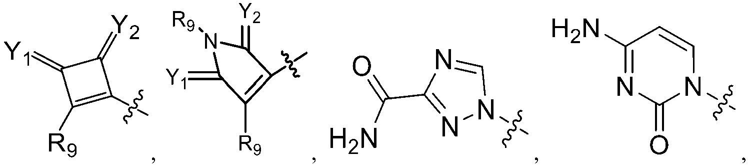 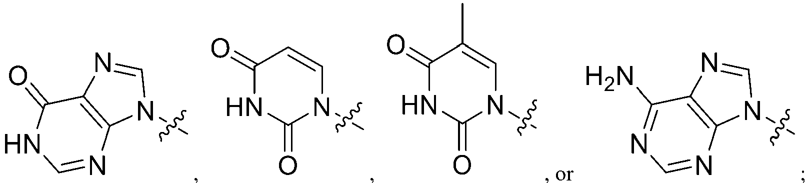 and
A₁ and A₂ are each independently -N(R₄)-, -C(=O)O-, -OC(=O)-, -OC(=O)O-, -O-, -C(=O)-, -S-, - C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, - N(R₄)C(=O)O-, -N(R₄)C(=O)N(R₄)-, -SC(=O)N(R₄)-, -N(R₄)C(=O)S-, -N(C(=O)L₁₃OR₄)-, - N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, - OP(=O)(OH)O-, -OP(=O)(H)O-, -OS(=O)₂NH-, -NHS(=O)₂O-, -OC(=O)C(=O)O-, -N(R₄)C(=O)C(=O)O-, -OC(=O)C(=O)N(R₄)-, -N(R₄)C(=O)C(=O)N(R₄)-, 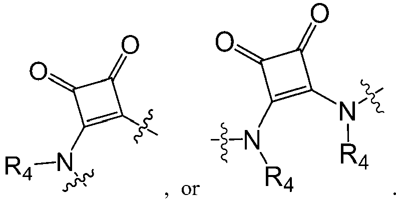 In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, in the amino lipid compound represented by formula (V-2), or a pharmaceutically acceptable salt thereof, or a stereoisomer thereof, the definitions of L₁, L₂, L₇, L₈, L₉, L₁₀, L₁₁, L₁₂, A₁, A₂, A₇, A₈, A₉, A₁₀, R₁, R₂, and L₁₅ are as defined in formula (IA),
Z is 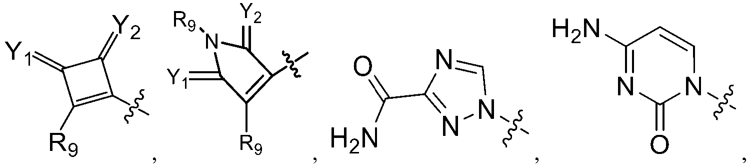 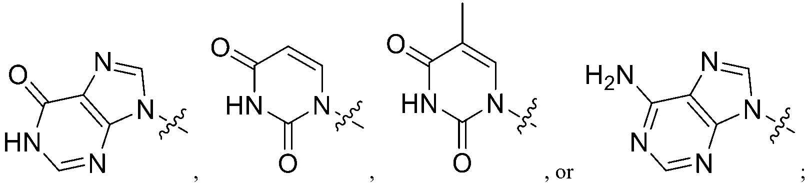
L₁ and/or L₂ are/is substituted with one or more substituents which include, but are not limited to, -OH, -SH, halogen, O, S, N, -CN, cyclohydrocarbyl, aryl, heterocyclyl, -R_{b}, -RₐN(R_{b})₂, -RₐOR_{b}, -RₐSR_{b}, - RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RₐC(=O)OR_{b}, -OC(=O)R_{b}, -C(=O)OR_{b}, -RₐSR_{b}, or 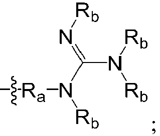
when one or more of L₁ and/or L₂ are substituted with a substituent including -R_{b}, -RₐOR_{b}, -RₐSR_{b}, - RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RₐC(=O)OR_{b}, -OC(=O)R_{b}, -C(=O)OR_{b}, -RₐSR_{b}, or 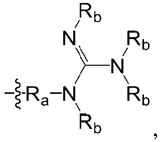 Rₐ and/or R_{b} may be attached to a C atom on L₁ or L₂ to form a three-, four-, five-, or six-membered ring;
wherein:
   each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
   each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds. In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, in the amino lipid compound represented by formula (IA), or a pharmaceutically acceptable salt thereof, or a stereoisomer thereof, the amino lipid compound has a structure represented by formula (V-3): 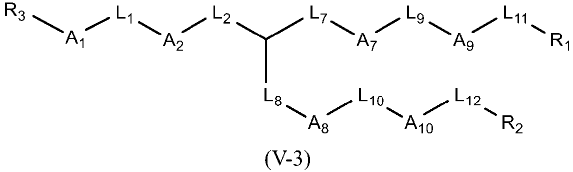 wherein A₁, A₂, A₇, A₈, A₉, A₁₀, L₁, L₂, L₇, L₈, L₉, L₁₀, L₁₁, L₁₂, R₁, R₂, and R₃ are as defined in formula (IA), and at least one of A₁ and A₂ is -N(R₄)-, and R₄ is as defined in formula (IA).

In some embodiments, in the amino lipid compound represented by formula (IV-3), or a pharmaceutically acceptable salt thereof, or a stereoisomer thereof, the definitions of A₁, A₂, A₇, A₈, A₉, A₁₀, L₁, L₂, L₇, L₈, L₉, L₁₀, L₁₁, L₁₂, and R₃ are as defined in formula (IA), and at least one of A₁ and A₂ is -N(R₄)-, R₄ is as defined in formula (IA), R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), and the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇. In some embodiments, each R₆ is independently C₁, C₂, C₃, C₄, C₅, or C₆ alkyl. In some embodiments, each R₆ is independently C₁, C₂, or C₃ alkyl.

In some embodiments, in the amino lipid compound represented by formula (V-3), or a pharmaceutically acceptable salt thereof, or a stereoisomer thereof, the definitions of L₁, L₂, L₇, L₈, L₉, L₁₀, L₁₁, L₁₂, A₁, A₂, A₇, A₈, A₉, A₁₀, R₁, R₂, and R₃ are as defined in formula (IA), wherein at least one of A₁ and A₂ is -N(R₄)-, R₄ is as defined in formula (IA), and none of A₁, A₂, and L₁ is a bond.

In some embodiments, in the amino lipid compound represented by formula (IA), or a pharmaceutically acceptable salt thereof, or a stereoisomer thereof, the amino lipid compound has a structure represented by formula (V-4): 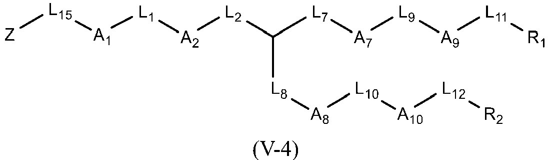 wherein the definitions of L₁, L₂, L₇, L₈, L₉, L₁₀, L₁₁, L₁₂, A₁, A₂, A₇, A₈, A₉, A₁₀, R₁, R₂, and L₁₅ are as defined in formula (IA), Z is 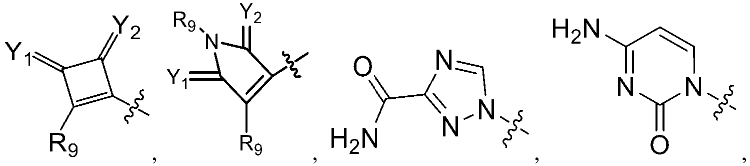 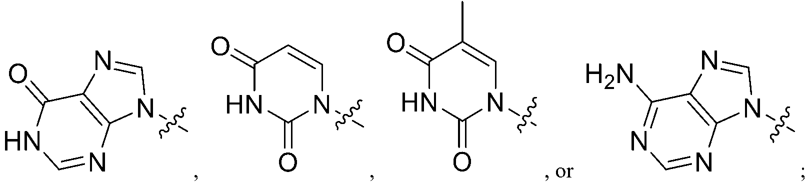 and A₁ and/or A₂ are/is each independently -N(R₄)-, -C(=O)O-, -OC(=O)-, -OC(=O)O-, -O-, -C(=O)-, -S-, - C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, - N(R₄)C(=O)O-, -N(R₄)C(=O)N(R₄)-, -SC(=O)N(R₄)-, -N(R₄)C(=O)S-, -N(C(=O)L₁₃OR₄)-, - N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, - OP(=O)(OH)O-, -OP(=O)(H)O-, -OS(=O)₂NH-, -NHS(=O)₂O-, -OC(=O)C(=O)O-, -N(R₄)C(=O)C(=O)O-, -OC(=O)C(=O)N(R₄)-, -N(R₄)C(=O)C(=O)N(R₄)-, 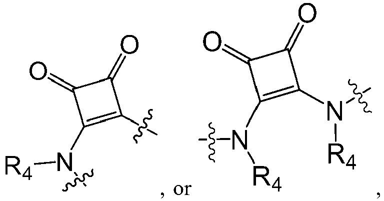 and at least one of A₁ and A₂ is -N(R₄)-, and R₄ is as defined in formula (IA). In some embodiments, R₁ and/or R₂ are/is -C(R₆)(OL₁₄A₁₂R₇)₂, -C(R₆)(SL₁₄A₁₂R₇)₂, or -C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or - C(R₆)(OC(=O)L₁₄A₁₂R₇)R₇.

In some embodiments, the amino lipid compound represented by formula (IA), or a pharmaceutically acceptable salt thereof, or a stereoisomer thereof, has a structure represented by formula (V-4), wherein the definitions of L₁, L₂, L₇, L₈, L₉, L₁₀ L₁₁, L₁₂, A₁, A₂, A₇, A₈, A₉, A₁₀, R₁, R₂, and L₁₅ are as defined in formula (IA), and at least one of A₁ and A₂ is -N(R₄)-, R₄ is as defined in formula (IA),
Z is 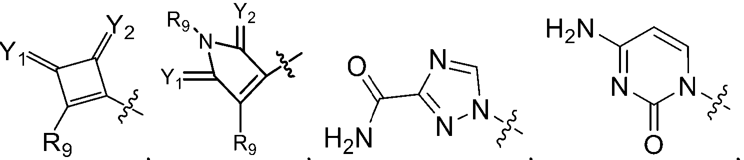 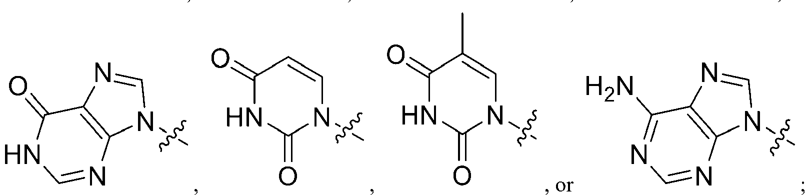
L₁ and/or L₂ are/is substituted with one or more substituents which include, but are not limited to, -OH, -SH, halogen, O, S, N, -CN, cyclohydrocarbyl, aryl, heterocyclyl, -R_{b}, -RₐN(R_{b})₂, -RₐOR_{b}, -RₐSR_{b}, - RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RₐC(=O)OR_{b}, -OC(=O)R_{b}, -C(=O)OR_{b}, -RₐSR_{b}, or 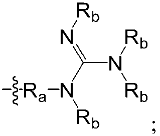
when L₁ and/or L₂ are/is substituted with a substituent including -R_{b}, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, -RₐOC(=O)R_{b}, -RₐC(=O)OR_{b}, -OC(=O)R_{b}, -C(=O)OR_{b}, -RₐSR_{b}, or 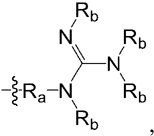 Rₐ and/or R_{b} may be attached to a C atom on L₁ or L₂ to form a three-, four-, five-, or six-membered ring;
wherein:
   each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
   each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds. In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OLi₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, in the amino lipid compound represented by formula (IA), or a pharmaceutically acceptable salt thereof, or a stereoisomer thereof, the amino lipid compound has a structure represented by formula (V-5): 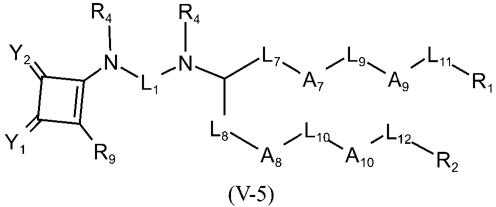 wherein R₁ and R₂ are each independently C₁-C₂₄ hydrocarbyl, -C(R₆)(OL₁₄A₁₂R₇)₂, - C(R₆)(SL₁₄A₁₂R₇)₂, -C(R₆)(SL₁₄A₁₂R₇)(OL₁₄A₁₂R₇), -C(R₆)(C(=O)OL₁₄A₁₂R₇)₂, -C(R₆)(OC(=O)L₁₄A₁₂R₇)₂, -C(R₆)(C(=O)OL₁₄Aₗ₂R₇)R₇, or -C(R₆)(OC(=O)L₁₄A₁₂R₇)R₇, and the definitions of L₁, L₇, L₈, L₉, L₁₀, L₁₁, L₁₂, A₇, A₈, A₉, A₁₀, R₄, R₉, Y₁, Y₂, R₆, L₁₄, A₁₂, and R₇ are as defined in formula (IA).

In some embodiments, in the amino lipid compound represented by formula (V-5), or a pharmaceutically acceptable salt thereof, or a stereoisomer thereof, the amino lipid compound has a structure represented by formula (V-5-1):

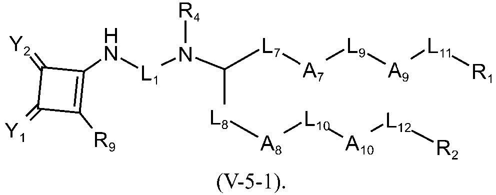

In some embodiments, in the amino lipid compound represented by formula (V-5), or a pharmaceutically acceptable salt thereof, or a stereoisomer thereof, the amino lipid compound has a structure represented by formula (V-5-2): 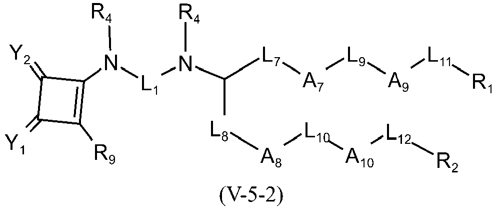
Y₁ and Y₂ are each independently O or S;
R₉ is H, halogen, -R_{b}, -N(R_{b})₂, -CN, -N₃, -C(=O)OR_{b}, -OC(=O)R_{b}, -OR_{b}, -SR_{b}, -S(-O)R_{b}, -S(=O)OR_{b}, - S(=O)₂OR_{b}, -N(R_{b})S(=O)₂R_{b}, -NHRₐN(R_{b})₂, -NHRₐORₐN(R_{b})₂, -NHRₐOR_{b}, or -N(RₐOR_{b})₂;
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds;
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds;
L₁ is C₁-C₆ hydrocarbylene, C₃-C₈ carbocyclic ring, or C₁-C₆ heterohydrocarbylene;
L₇ and L₈ are each independently C₁-C₁₀ hydrocarbylene;
L₁₁ and L₁₂ are each independently C₁-C₅ hydrocarbylene or a bond;
A₇ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 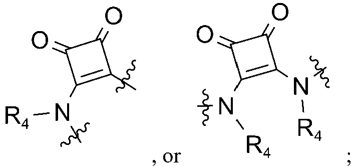
A₈ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 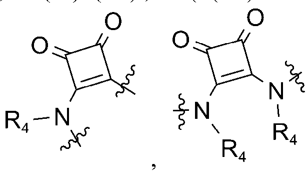 , or a bond;
R₁ and R₂ are each independently C₁-C₂₄ alkyl, -C(R₆)(OR₇)₂, -C(R₆)(SR₇)₂, or -C(R₆)(SR₇)(OR₇);
each R₇ is independently C₁-C₁₂ hydrocarbyl;
L₉, L₁₀, A₉, and A₁₀ are a bond; and
the definitions of R₄, L₁₃, and R₆ are as defined in formula (V-5).

The amino lipid compound of formula (V-5), (V-5-1), or (V-5-2), or a pharmaceutically acceptable salt thereof, or a stereoisomer thereof includes one or more of the following features, where applicable.

In some embodiments, Y₁ is O.

In some embodiments, Y₂ is O.

In some embodiments, Y₁ and Y₂ are O.

In some embodiments, R₉ is -N(R_{b})₂, -NHRₐN(R_{b})₂, -NHRₐORₐN(R_{b})₂, -NHRₐOR_{b}, or -N(RₐOR_{b})₂.

In some embodiments, R₉ is -N(R_{b})₂ or -NHRₐN(R_{b})₂.

In some embodiments, R₉ is -N(R_{b})₂.

In some embodiments, R₉ is -N(H)CH₃.

In some embodiments, Y₁ and Y₂ are each independently O or S, and R₉ is -N(R_{b})₂ or -NHRₐN(R_{b})₂.

In some embodiments, Y₁ and Y₂ are O, and R₉ is -N(R_{b})₂.

In some embodiments, Y₁ and Y₂ are O, and R₉ is -N(H)CH₃.

In some embodiments, each Rₐ is independently C₁-C₁₂ alkylene.

In some embodiments, each Rₐ is independently C₁-C₅ alkylene.

In some embodiments, each R_{b} is independently H, C₁-C₁₂ alkyl, or C₂-C₁₂ alkenyl.

In some embodiments, each R_{b} is independently H, C₁-C₆ alkyl, or C₂-C₃ alkenyl.

In some embodiments, each R_{b} is independently H, or C₁-C₃ alkyl.

In some embodiments, each R_{b} is independently H, or C₁-C₂ alkyl, e.g., C₁ alkyl.

In some embodiments, A₇ and A₈ are each independently -C(=O)O-, -OC(=O)-, -OC(=O)O-, - C(=O)N(R₄)-, -N(R₄)C(=O)-, -N(C(=O)L₁₃OR₄)-, -N(C(=O)L₁₃SR₄)-, -N(C(=O)R₄), -N(L₁₃OR₄)-,-OC(=O)C(=O)O-,

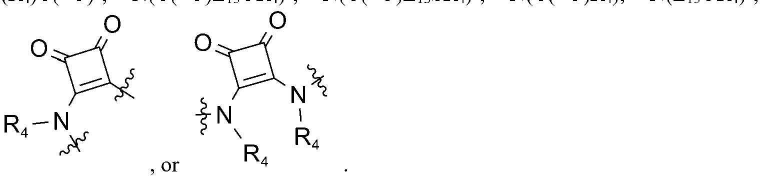

In some embodiments, A₇ and A₈ are each independently -C(=O)O-, -OC(=O)-, -OC(=O)O-, - C(=O)N(R₄)-, -N(R₄)C(=O)-, or -OC(=O)C(=O)O-.

In some embodiments, A₇ and A₈ are each independently -C(=O)O- or -OC(=O)-.

In some embodiments, A₇, A₈, A₉, and A₁₀ are each independently -C(=O)O-, -OC(=O)-, or a bond. In some embodiments, A₇ is -C(=O)O-.

In some embodiments, A₇ is -OC(=O)-.

In some embodiments, A₈ is -C(=O)O-.

In some embodiments, A₈ is -OC(=O)-.

In some embodiments, A₇ and A₈ are -C(=O)O-.

In some embodiments, A₇ and A₈ are -OC(=O)-.

In some embodiments, A₈ is a bond.

In some embodiments, A₉ is -C(=O)O-.

In some embodiments, A₉ is -OC(=O)-.

In some embodiments, A₁₀ is -C(=O)O-.

In some embodiments, A₁₀ is -OC(=O)-.

In some embodiments, A₉ is a bond.

In some embodiments, A₁₀ is a bond.

In some embodiments, A₉ and A₁₀ are -C(=O)O-.

In some embodiments, A₉ and A₁₀ are a bond.

In some embodiments, R₁ and R₂ are each independently C₁-C₂₄ alkyl, -C(R₆)(OR₇)₂, - C(R₆)(C(=O)OR₇)₂, -C(R₆)(OC(=O)R₇)₂, -C(R₆)(C(=O)OR₇)R₇, or -C(R₆)(OC(=O)R₇)R₇.

In some embodiments, R₁ and R₂ are each independently C₁-C₂₄ alkyl.

In some embodiments, R₁ is a straight C₁-C₁₈ alkyl.

In some embodiments, R₁ is a straight C₁-C₁₅ alkyl.

In some embodiments, R₁ is a straight C₄-C₁₂ alkyl.

In some embodiments, R₁ is a straight C₅-C₁₀ alkyl, such as C₅ alkyl, C₆ alkyl, or C_{g} alkyl.

In some embodiments, R₁ is a straight C₇-C₁₀ alkyl, such as C₇ alkyl, C₈ alkyl, or C_{g} alkyl.

In some embodiments, R₁ is a branched C₉-C₂₀ alkyl, such as a branched C₉-C₁₈ alkyl, a branched C₉-C₁₇ alkyl, a branched C₁₀-C₁₈ alkyl, a branched C₁₁-C₁₇ alkyl, a branched C₁₂-C₁₇ alkyl, a branched C₁₂ alkyl, a branched C₁₄ alkyl, a branched C₁₆ alkyl, or a branched C₁₇ alkyl.

In some embodiments, R₂ is a straight C₁-C₁₈ alkyl.

In some embodiments, R₂ is a straight C₁-C₁₅ alkyl.

In some embodiments, R₂ is a straight C₄-C₁₂ alkyl.

In some embodiments, R₂ is a straight C₅-C₁₀ alkyl, such as C₅ alkyl, C₆ alkyl, or C_{g} alkyl.

In some embodiments, R₂ is a straight C₇-C₁₀ alkyl, such as C₇ alkyl, C₈ alkyl, or C_{g} alkyl.

In some embodiments, R₂ is a branched C₉-C₂₀ alkyl, such as a branched C₉-C₁₈ alkyl, a branched C₉-C₁₇ alkyl, a branched C₁₀-C₁₈ alkyl, a branched C₁₁-C₁₇ alkyl, a branched C₁₂-C₁₇ alkyl, a branched C₁₂ alkyl, a branched C₁₄ alkyl, a branched C₁₆ alkyl, or a branched C₁₇ alkyl.

In some embodiments, R₁ is a straight C₅-C₁₀ alkyl, and R₂ is a branched C₉-C₂₀ alkyl.

In some embodiments, R₁ is a straight C₄-C₁₂ alkyl, and R₂ is a branched C₉-C₁₈ alkyl.

In some embodiments, R₁ is a straight C₇-C₁₀ alkyl, and R₂ is a branched C₉-C₁₇ alkyl.

In some embodiments, R₁ and R₂ are a branched C₉-C₂₀ alkyl.

In some embodiments, R₁ is a branched C₉-C₁₈ alkyl, and R₂ is a branched C₉-C₁₈ alkyl.

In some embodiments, R₁ is a branched C₉-C₁₇ alkyl, and R₂ is a branched C₉-C₁₇ alkyl.

In some embodiments, L₁₁ is a bond, and the branching of R₁ occurs at the α, β, or γ position of A₉, for example, the branching of R₁ occurs at the α position of A₉: 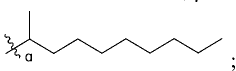 the branching of R₁ occurs at the β position of A₉:

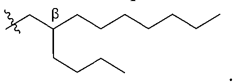

In some embodiments, L₉, A₉, and L₁₁ are a bond, and the branching of R₁ occurs at the α, β, or γ position of A₇. In some embodiments, the branching of R₁ occurs at the α or β position of A₇.

In some embodiments, L₁₂ is a bond, and the branching of R₂ occurs at the α, β, or γ position of A₁₀, for example, the branching of R₂ occurs at the α position of A₁₀: 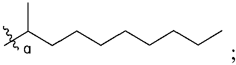 the branching of R₂ occurs at the β position of A₁₀:

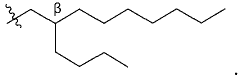

In some embodiments, L₁₀, A₁₀, and L₁₂ are a bond, and the branching of R₂ occurs at the α, β, or γ position of A₈. In some embodiments, the branching of R2 occurs at the α or β position of As.

In some embodiments, R₁ or R₂ is one of the following structures: 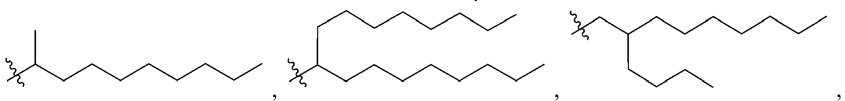 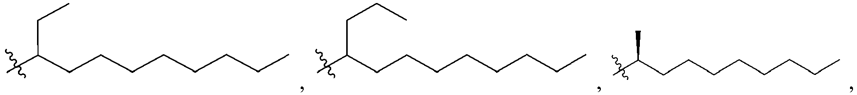 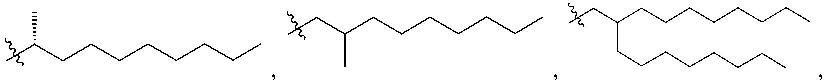 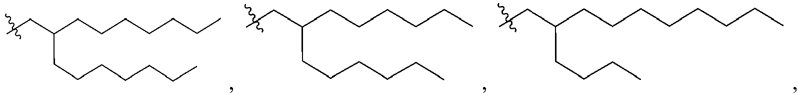 and

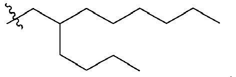

In some embodiments, R₁ is C(H)(OR₇)₂.

In some embodiments, R₁ is -C(H)(C(=O)OR₇)₂.

In some embodiments, R₁ is -C(H)(OC(=O)R₇)₂.

In some embodiments, R₂ is -C(H)(OR₇)₂.

In some embodiments, R₂ is -C(H)(C(=O)OR₇)₂.

In some embodiments, R₂ is -C(H)(OC(=O)R₇)₂.

In some embodiments, R₁ is -C(H)(OR₇)₂, and R₂ is a straight C₅-C₁₀ alkyl.

In some embodiments, R₁ and R₂ are -C(H)(OR₇)₂.

In some embodiments, R₁ and R₂ are -C(H)(C(=O)OR₇)₂.

In some embodiments, each R₇ is independently C₁-C₁₂ alkyl.

In some embodiments, each R₇ is independently C₃-C₁₀ alkyl, such as C₅-C₇ alkyl or C₆ alkyl.

In some embodiments, each R₇ is independently a substituted C₃-C₁₀ alkyl, e.g., substituted with methyl.

In some embodiments, L₁ is C₁-C₆ alkylene.

In some embodiments, L₁ is C₂-C₅ alkylene.

In some embodiments, L₁ is C₂-C₄ alkylene.

In some embodiments, L₁ is C₃-C₄ alkylene.

In some embodiments, L₁ is C₃ alkylene.

In some embodiments, L₁ is a substituted C₂-C₄ alkylene, for example, L₁ is C₃ alkylene substituted hydroxyl, e.g.,

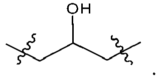

In some embodiments, each R₄ is independently H or C₁-C₆ alkyl.

In some embodiments, R₄ is C₁-C₆ alkyl.

In some embodiments, R₄ is C₂-C₅ alkyl, such as C₂ alkylene, C₃ alkylene, or C₅ alkylene.

In some embodiments, each R₄ is independently H or C₁-C₂ alkyl.

In some embodiments, R₄ is C₁-C₂ alkyl.

In some embodiments, R₄ is methyl.

In some embodiments, R₄ is a branched C₃-C₆ alkyl, such as

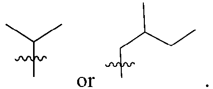

In some embodiments, R₄ is a substituted C₁-C₆ alkyl, e.g., substituted with -OH, -OC(=O)R_{b}, or - C(=O)OR_{b}, wherein each R_{b} is independently C₁-C₁₂ alkyl, C₂-C₁₂ alkenylene, or C₂-C₁₂ alkynylene.

In some embodiments, R₄ is C₁-C₆ alkyl substituted with -OH for example, R₄ is C₂ alkyl substituted with -OH, e.g.,

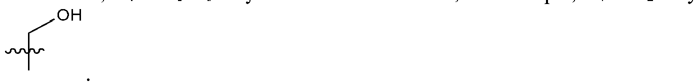

In some embodiments, R₄ is C₁-C₆ alkyl substituted with -OC(=O)R_{b} or -C(=O)OR_{b}, wherein each R_{b} is independently C₁-C₇ alkyl, such as 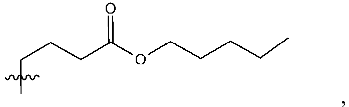 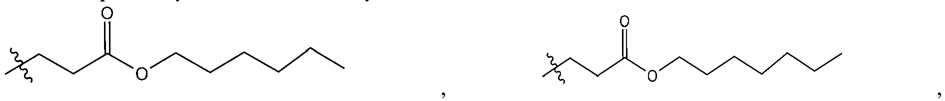 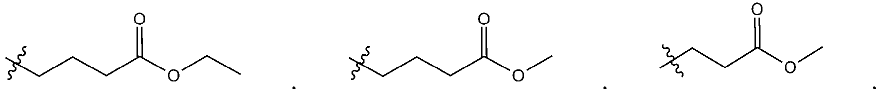 or

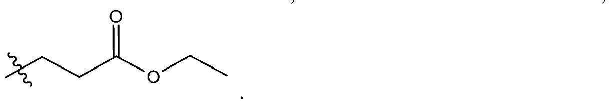

**In** some embodiments, L₇ and L₈ are each independently C₁-C₁₄ alkylene or a bond.

**In** some embodiments, L₇ is C₁-C₁₀ alkylene.

In some embodiments, L₇ is C₄-C₈ alkylene, such as Cs alkylene, C₆ alkylene, or C₇ alkylene.

In some embodiments, L₈ is C₁-C₁₀ alkylene.

In some embodiments, L₈ is C₄-C₈ alkylene, such as Cs alkylene, C₆ alkylene, or C₇ alkylene.

In some embodiments, L₇ is a bond.

In some embodiments, L₈ is a bond.

In some embodiments, L₇ and L₈ are each independently C₁-C₁₀ alkylene.

In some embodiments, L₇ and L₈ are each independently C₃-C₉ alkylene.

In some embodiments, L₇ and L₈ are each independently C₄-C₈ alkylene, such as C₅ alkylene, C₆ alkylene, or C₇ alkylene.

In some embodiments, L₇ and L₈ are a bond.

In some embodiments, L₇ and L₈ are each independently C₁-C₃ alkylene or a bond.

In some embodiments, L₇ and L₈ are each independently a branched C₂-C₃ alkylene, e.g., a branched C₂ alkylene( 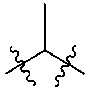 ).

In some embodiments, L₇ is C₁-C₃ alkylene, and L₈ is a branched C₂-C₃ alkylene.

In some embodiments, L₇ is a bond, and L₈ is a branched C₂-C₃ alkylene.

In some embodiments, L_{g} and L₁₀ are each independently C₄-C₇ alkylene.

In some embodiments, L₉ and L₁₀ are each independently a branched C₄-C₇ alkylene, e.g.,

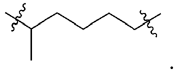

In some embodiments, L₉ is a bond.

In some embodiments, L₁₀ is a bond.

In some embodiments, L₉ and L₁₀ are a bond.

In some embodiments, L₁₁ is C₁-C₆ alkylene.

In some embodiments, L₁₁ is C₂-C₄ alkylene, e.g., C₃ alkylene.

In some embodiments, L₁₁ is a bond.

In some embodiments, L₁₁ is C₂-C₄ alkylene or a bond.

In some embodiments, L₁₂ is C₁-C₆ alkylene.

In some embodiments, L₁₂ is C₂-C₄ alkylene, e.g., C₃ alkylene.

In some embodiments, L₁₂ is a bond.

In some embodiments, L₁₂ is C₂-C₄ alkylene or a bond.

In some embodiments, L₁₁ is a bond, and L₁₂ is C₂-C₄ alkylene, e.g., C₃ alkylene.

In some embodiments, L₉, L₁₀, L₁₁, and L₁₂ are a bond, A₇ is -C(=O)O- or -OC(=O)-, A₈ is -C(=O)O- or -OC(=O)-, A₉ and A₁₀ are a bond, and R₁ and R₂ are each independently C₁-C₂₄ alkyl. In some embodiments, R₁ and R₂ are each independently a branched C₉-C₂₀ alkyl, such as a branched C₁₀-C₁₈ alkyl, a branched C₁₁-C₁₇ alkyl, a branched C₁₂-C₁₇ alkyl, a branched C₁₂ alkyl, a branched C₁₄ alkyl, a branched C₁₆ alkyl, or a branched C₁₇.

In some embodiments, A₇, A₈, A₉, and A₁₀ are each independently -C(=O)O- or -OC(=O)-, L₉ and L₁₀ are each independently C₄-C₇ alkylene, and L₁₁ and L₁₂ are a bond, and R₁ and R₂ are each C₉-C₂₀ alkyl. In some embodiments, L₉ and L₁₀ are branched C₄-C₇ alkylene. In some embodiments, L₉ and L₁₀ are branched C₆ alkyl, e.g., 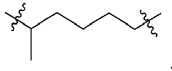 . In some embodiments, R₁ and R₂ are branched C₉-C₂₀ alkyl. In some embodiments, R₁ an d R₂ are each a branched C₁₂ alkyl, e.g.,

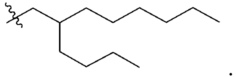

In some embodiments, L₉, L₁₀, A₉, and A₁₀ are a bond, A₇ and A₈ are each independently -C(=O)O- or - OC(=O)-, and R₁ and R₂ are -C(H)(OR₇)₂. In some embodiments, each R₇ is independently C₁-C₁₂ alkyl. In some embodiments, each R₇ is independently C₃-C₁₀ alkyl, such as C₅-C₇ alkyl or C₆ alkyl.

In some embodiments, L₉, L₁₀, A₉, and A₁₀ are a bond, A₇ and A₈ are each independently -C(=O)O- or - OC(=O)-, R₁ is -C(H)(OR₇)₂, and R₂ is C₆-C₁₂ alkyl.

In some embodiments, L₉, L₁₀, A₉, and A₁₀ are a bond, A₇ and A₈ are each independently -C(=O)O- or - OC(=O)-, and R₁ and R₂ are -C(R₆)(C(=O)OR₇)₂ or -C(R₆)(OC(=O)R₇)₂. In some embodiments, R₁ and R₂ are -C(H)(C(=O)OR₇)₂.

In some embodiments, L₇, L₈, L₉, L₁₀, A₉, and A₁₀ are a bond, A₇ and A₈ are each independently - C(=O)O- or -OC(=O)-, and R₁ and R₂ are -C(H)(OR₇)₂.

In some embodiments, in the amino lipid compound represented by formula (IA), or a pharmaceutically acceptable salt thereof, or a stereoisomer thereof, the amino lipid compound having a structure represented by formula (V-6): 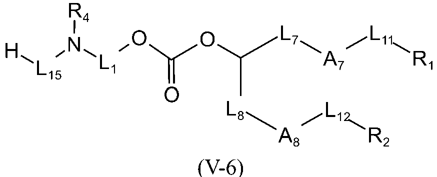 wherein R₁ and R₂ are each independently -C(R₆)(SL₁₄A₁₂R₇)₂, -C(R₆)(SL₁₄A₁₂R₇)(OL₁₄A₁₂R₇), - C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or - C(R₆)(OC(=O)L₁₄A₁₂R₇)R₇, and the definitions of L₁, L₇, L₈, L₁₁, L₁₂, L₁₅, A₇, A₈, R₄, R₆, L₁₄, A₁₂, and R₇ are as defined in formula (IA).

In some embodiments, in the amino lipid compound represented by formula (V-6), or a pharmaceutically acceptable salt thereof, or a stereoisomer thereof, the amino lipid compound has a structure represented by formula (V-6-1): 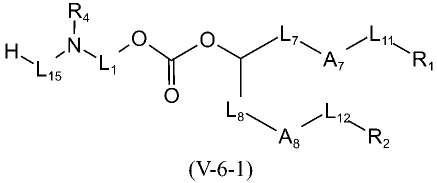 wherein:
L₁ is C₁-C₆ hydrocarbylene or C₁-C₆ heterohydrocarbylene;
L₁₅ is C₁-C₂ hydrocarbylene;
L₇ and L₈ are each independently C₁-C₁₀ hydrocarbylene;
L₁₁ and L₁₂ are each independently C₁-C₆ hydrocarbylene;
A₇ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 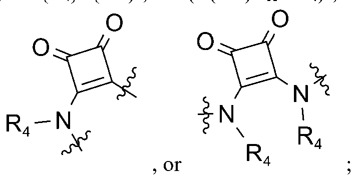
A₈ is -C(=O)O-, -OC=O-, -OC(=O)O-, -C(=O)N(R₄)-, -N(R₄)C(=O)-, -N(C(=O)L₃OR₄)-, - N(C(=O)L₁₃SR₄)-, -N(C(=O)R₄), -N(L₁₃OR₄)-, -OC(=O)C(=O)O-, 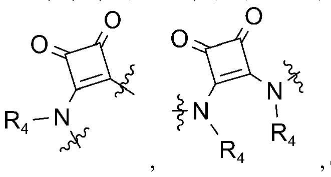 or a bond;
R₁ and R₂ are each independently -C(R₆)(C(=O)OR₇)₂, -C(R₆)(OC(=O)R₇)₂, -C(R₆)(C(=O)OR₇)R₇, or - C(R₆)(OC(=O)R₇)R₇;
each R₇ is independently C₁-C₁₂ hydrocarbyl; and
the definitions of R₄, L₁₃, and R₆ are as defined in formula (V-6).

The amino lipid compound of formula (V-6) or (V-6-1), or a pharmaceutically acceptable salt thereof, or a stereoisomer thereof, includes one or more of the following features, where applicable.

In some embodiments, A₇ and A₉ are each independently -C(=O)O-, -OC(=O)-, -OC(=O)O-, - C(=O)N(R₄)-, -N(R₄)C(=O)-, -N(C(=O)L₁₃OR₄)-, -N(C(=O)L₁₃SR₄)-, -N(C(=O)R₄), -N(L₁₃OR₄)-, - OC(=O)C(=O)O-,

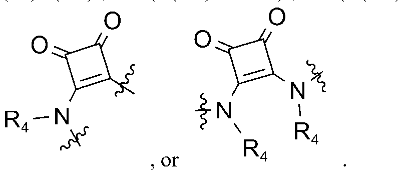

In some embodiments, A₇ and A₈ are each independently -C(=O)O-, -OC(=O)-, -OC(=O)O-, - C(=O)N(R₄)-, -N(R₄)C(=O)-, or -OC(=O)C(=O)O-.

In some embodiments, A₇ and A₈ are each independently -C(=O)O- or -OC(=O)-.

In some embodiments, A₇ is -C(=O)O.

In some embodiments, A₇ is -OC(=O)-.

In some embodiments, A₈ is -C(=O)O-.

In some embodiments, A₈ is -OC(=O)-.

In some embodiments, A₇ and A₈ are each -C(=O)O-.

In some embodiments, A₈ is a bond.

In some embodiments, R₁ and R₂ are each independently -C(H)(C(=O)OR₇)₂, -C(H)(OC(=O)R₇)₂-, C(H)(C(=O)OR₇)R₇, or -C(H)(OC(=O)R₇)R₇.

In some embodiments, R₁ is -C(H)(C(=O)OR₇)₂.

In some embodiments, R₁ is -C(H)(OC(=O)R₇)₂.

In some embodiments, R₁ is -C(H)(C(=O)OR₇)R₇.

In some embodiments, R₁ is -C(H)(OC(=O)R₇)R₇.

In some embodiments, R₂ is -C(H)(C(=O)OR₇)₂.

In some embodiments, R₂ is -C(H)(OC(=O)R₇)₂.

In some embodiments, R₂ is -C(H)(C(=O)OR₇)R₇.

In some embodiments, R₂ is -C(H)(OC(=O)R₇)R₇.

In some embodiments, R₁ and R₂ are each -C(H)(OC(=O)R₇)₂.

In some embodiments, R₁ and R₂ are each -C(H)(C(=O)OR₇)R₇.

In some embodiments, L₁₅ is C₁-C₂ alkylene.

In some embodiments, L₁₅ is C₁ alkylene.

In some embodiments, R₄ is C₁-C₃ alkyl.

In some embodiments, R₄ is C₁ alkyl.

In some embodiments, L₁ is C₂-C₄ alkylene.

In some embodiments, L₁ is C₃ alkylene.

In some embodiments, L₇ and L₈ are each independently C₁-C₁₀ alkylene.

In some embodiments, L₇ and L₈ are each independently C₃-C₉ alkylene.

In some embodiments, L₇ and L₈ are each independently C₃-C₈ alkylene.

In some embodiments, L₇ and L₈ are each independently C₄-C₇ alkylene, such as C₅-C₇ alkylene, C₅ alkylene, or C₆ alkylene.

In some embodiments, L₁₁ and L₁₂ are each independently C₂-C₄ alkylene, such as C₂ alkylene or C₃ alkylene.

In some embodiments, each R₇ is independently C₃-C₉ alkyl.

In some embodiments, each R₇ is independently C₅-C₇ alkyl.

In some embodiments, each R₇ is independently C₆ alkyl.

In some embodiments, A₇ and A₈ are -C(=O)O-, R₁ and R₂ are -C(H)(C(=O)OR₇)₂, and each R₇ is independently C₃-C₉ alkyl. In some embodiments, each R₇ is independently C₅-C₇ alkyl. In some embodiments, R₇ is C₅ alkyl. In some embodiments, R₇ is C₆ alkyl.

In some embodiments, A₇ and A₈ are -C(=O)O-, R₁ and R₂ are -C(H)(OC(=O)R₇)R₇, and each R₇ is independently C₃-C₉ alkyl. In some embodiments, each R₇ is independently C₅-C₇ alkyl, such as C₅ alkyl or C₆ alkyl. In some embodiments, each R₇ is C₅ alkyl or C₆ alkyl, for example, R₁ and R₂ are - C(H)(OC(=O)C₅H₁₁)C₆H₁₃.

In some embodiments, in the amino lipid compound represented by formula (IA), or a pharmaceutically acceptable salt thereof, or a stereoisomer thereof, the amino lipid compound has a structure represented by formula (VI): 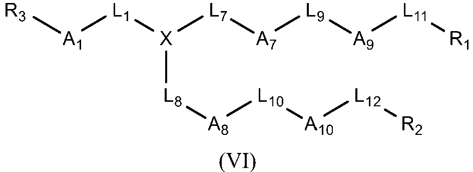 wherein the definitions of L₁, L₇, L₈, L₉, L₁₀, L₁₁, L₁₂, A₁, A₇, A₈, A₉, A₁₀, R₁, R₂, and R₃ are as defined in formula (IA).

In some embodiments, in the amino lipid compound represented by formula (IA), or a pharmaceutically acceptable salt thereof, or a stereoisomer thereof, the amino lipid compound has a structure represented by formula (VI-1): 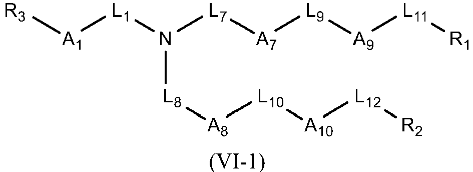 wherein the definitions of L₁, L₇, L₈, L₉, L₁₀, L₁₁, L₁₂, A₁, A₇, A₈, A₈, A₁₀, R₁, R₂, and R₃ are as defined in formula (IA).

In some embodiments, in the amino lipid compound represented by formula (VI-1), or a pharmaceutically acceptable salt thereof, or a stereoisomer thereof, the definitions of L₁, L₇, L₉, L₉, L₁₀, L₁₁, L₁₂, A₁, A₇, A₈, A₉, A₁₀, and R₃ are as defined in formula (IA), R₁ and/or R₂ are/is -C(R₆)(OL₁₄A₁₂R₇)₂, - C(R₆)(SL₁₄A₁₂R₇)₂, or -C(R₆)(SL₁₄A₁₂R₇)(OL₁₄A₁₂R₇), and the definitions of R₆, L₁₄, A₁₂, and R₇ are as defined in formula (IA). In some embodiments, R₁ and R₂ may further be each independently - C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or - C(R₆)(OC(=O)L₁₄A₁₂R₇)R₇. In some embodiments, each R₆ is independently C₁, C₂, C₃, C₄, C₅, or C₆ alkyl. In some embodiments, each R₆ is independently C₁, C₂, or C₃ alkyl.

In some embodiments, in the amino lipid compound represented by formula (VI-1), or a pharmaceutically acceptable salt thereof, or a stereoisomer thereof, the definitions of L₁, L₇, L₉, L₉, L₁₀, L₁₁, L₁₂, A₁, A₇, A₈, A₉, A₁₀, R₁, R₂, and R₃ are as defined in formula (IA), and neither of A₁ and L₁ is a bond.

In some embodiments, in the amino lipid compound represented by formula (IA), or a pharmaceutically acceptable salt thereof, or a stereoisomer thereof, the amino lipid compound has a structure represented by formula (VI-2): 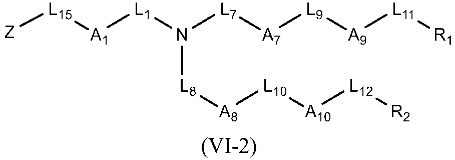 wherein the definitions of L₁, L₇, L₈, L₉, L₁₀, L₁₁, L₁₂, A₁, A₇, A₈, A₈, A₁₀, R₁, R₂, and L₁₅ are as defined in formula (IA), Z is 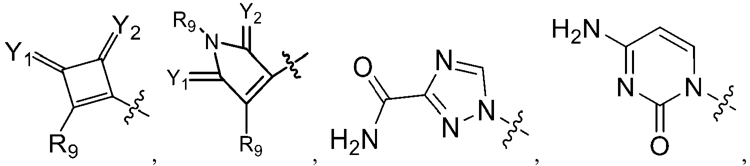 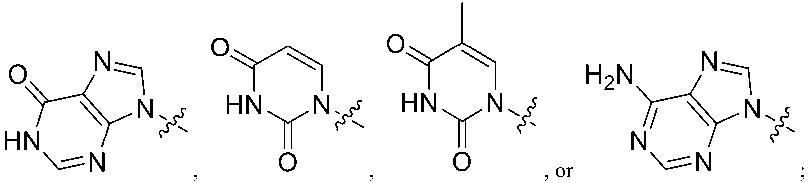 L₁ is substituted with one or more substituents which include, but are not limited to, -OH, -SH, halogen, O, S, N, -CN, cyclohydrocarbyl, aryl, heterocyclyl, -R_{b}, -RₐN(R_{b})₂, -RₐOR_{b}, -RₐSR_{b}, -RₐOC(=O)OR_{b}, - RₐOC(=O)SR_{b}, -RₐC(=O)N(R_{b})₂, -OC(=O)OR_{b}, -OC(=O)SR_{b}, -C(=O)N(R_{b})₂, -RₐOC(=O)R_{b}, -RₐC(=O)OR_{b}, -OC(=O)R_{b}, -C(=O)OR_{b}, -RₐSR_{b}, or 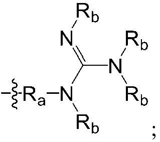 when one or more of L₁ are substituted with a substituent including -R_{b}, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, -RₐOC(=O)R_{b}, -RₐC(=O)OR_{b}, -OC(=O)R_{b}, -C(=O)OR_{b}, -RₐSR_{b}, or 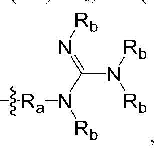 Rₐ and/or R_{b} may be attached to a C atom on L₁ to form a three-, four-, five-, or six-membered ring;
wherein:
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds. In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, A₁ is -N(R₄)-. In some embodiments, R₄ is H.

In some embodiments, in the amino lipid compound represented by formula (IA), or a pharmaceutically acceptable salt thereof, or a stereoisomer thereof, the amino lipid compound has a structure represented by formula (VI-3): 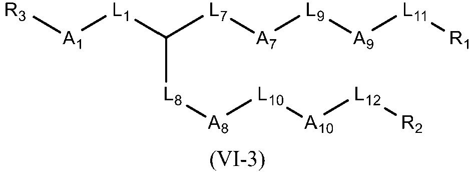 wherein A₁, A₇, A₈, A₉, A₁₀, L₁, L₇, L₈, L₉, L₁₀, L₁₁, L₁₂, R₁, R₂, and R₃ are as defined in formula (IA), and A₁ is -N(R₄)-, and R₄ is as defined in formula (IA).

In some embodiments, in the amino lipid compound represented by formula (VI-3), or a pharmaceutically acceptable salt thereof, or a stereoisomer thereof, the definitions of A₁, A₇, A₈, A₉, A₁₀, L₁, L₇, L₈, L₉, L₁₀, L₁₁, L₁₂, and R₃ are as defined in formula (IA), and A₁ is -N(R₄)-, R₄ is as defined in formula (IA), R₁ and/or R₂ are/is -C(R₆)(OL₁₄A₁₂R₇)₂, -C(R₆)(SL₁₄A₁₂R₇)₂, or -C(R₆)(SL₁₄A₁₂R₇)(OL₁₄A₁₂R₇), and the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, -C(R₆)(OC(=O)L₁₄A₁₂R₇)₂, - C(R₆)(C(=O)OL₁₄A₁₂R₇)R₇, or -C(R₆)(OC(=O)L₁₄A₁₂R₇)R₇. In some embodiments, each R₆ is independently C₁, C₂, C₃, C₄, C₅, or C₆ alkyl. In some embodiments, each R₆ is independently C₁, C₂, or C₃ alkyl.

In some embodiments, in the amino lipid compound represented by formula (VI-3), or a pharmaceutically acceptable salt thereof, or a stereoisomer thereof, the definitions of L₁, L₇, L₉, L₉, L₁₀, L₁₁, L₁₂, A₁, A₇, A₈, A₉, A₁₀, R₁, R₂, and R₃ are as defined in formula (IA), wherein A₁ is -N(R₄)-, R₄ is as defined in formula (IA), and neither of A₁ and L₁ is a bond.

In some embodiments, in the amino lipid compound represented by formula (IA), or a pharmaceutically acceptable salt thereof, or a stereoisomer thereof, the amino lipid compound has a structure represented by formula (VI-4): 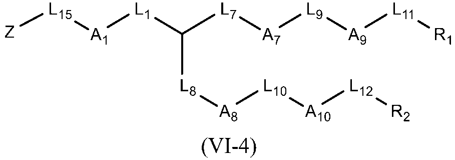 wherein the definitions of L₁, L₇, L₈, L₉, L₁₀, L₁₁, L₁₂, A₁, A₇, A₈, A₉, A₁₀, R₁, R₂, and L₁₅ are as defined in formula (IA), Z is 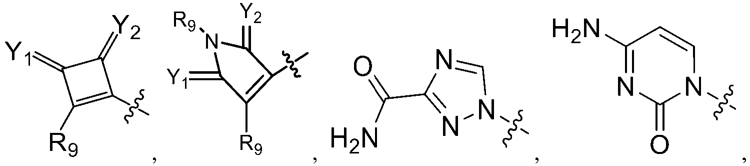 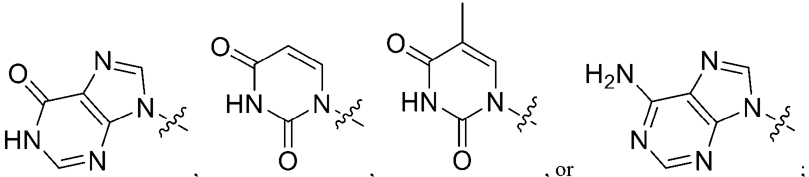 A₁ is -N(R₄)-, R₄ is as defined in formula (IA), and L₁ is substituted with one or more substituents which include, but are not limited to, -OH, -SH, halogen, O, S, N, -CN, cyclohydrocarbyl, aryl, heterocyclyl, -R_{b}, - RₐN(R_{b})₂, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, -RₐOC(=O)R_{b}, -RₐC(=O)OR_{b}, -OC(=O)R_{b}, -C(=O)OR_{b}, -RₐSR_{b}, or 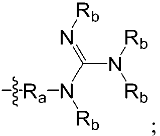 when L₁ is substituted with a substituent including -R_{b}, -RₐOR_{b}, -RₐSR_{b}, -RₐOC(=O)OR_{b}, - RₐOC(=O)SR_{b}, -RₐC(=O)N(R_{b})₂, -OC(=O)OR_{b}, -OC(=O)SR_{b}, -C(=O)N(R_{b})₂, -RₐOC(=O)R_{b}, -RₐC(=O)OR_{b}, -OC(=O)R_{b}, -C(=O)OR_{b}, -RₐSR_{b}, or 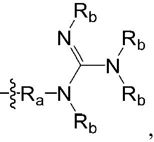 Rₐ and/or R_{b} may be attached to a C atom on L₁ to form a three-, four-, five-, or six-membered ring;
wherein:
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds. In some embodiments, R₁ and/or R₂ are/is -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇), wherein the definitions of R₆, L₁₄, A₁₂, and R₇ are as defined in formula (IA). In some embodiments, R₁ and R₂ may further be each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, in the amino lipid compound represented by formula (IA), or a pharmaceutically acceptable salt thereof, or a stereoisomer thereof, the amino lipid compound has a structure represented by formula (VI-5): 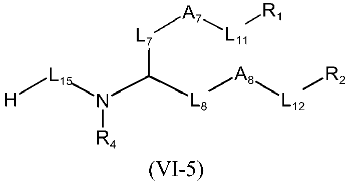 wherein R₁ and R₂ are each independently C₁-C₂₄ hydrocarbyl, -C(R₆)(OL₁₄A₁₂R₇)₂, - C(R₆)(SL₁₄A₁₂R₇)₂, -C(R₆)(SL₁₄A₁₂R₇)(OL₁₄A₁₂R₇), -C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or -C(R₆)(OC(=O)Li₄A₁₂R₇)R₇, and the definitions of L₇, L₉, L₁₁, L₁₂, L₁₅, A₇, A₈, R₄, R₆, L₁₄, A₁₂, and R₇ are as defined in formula (IA).

In some embodiments, in the amino lipid compound represented by formula (VI-5), or a pharmaceutically acceptable salt thereof, or a stereoisomer thereof, the amino lipid compound has a structure represented by formula (VI-5-1): 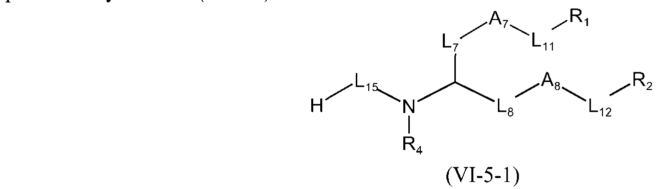 wherein:
L₁₅ is C₁-C₂ hydrocarbylene;
R₄ is C₁-C₆ hydrocarbyl or C₁-C₆ heterohydrocarbyl;
L₇ and L₈ are each independently C₁-C₁₀ hydrocarbylene;
L₁₁ and L₁₂ are each independently C₁-C₆ hydrocarbylene;
A₇ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC=OC=OO-, 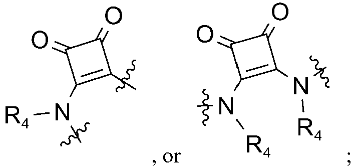
A₈ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, bond; 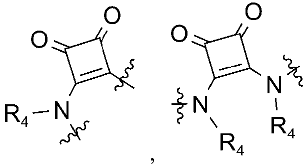 or a
R₁ and R₂ are each independently -C(R₆)(OR₇)₂, -C(R₆)(SR₇)₂, or -C(R₆)(SR₇)(OR₇);
each R₇ is independently C₁-C₁₂ hydrocarbyl; and
L₁₃ and R₆ are as defined in formula (VI-5).

The amino lipid compound of formula (VI-5) or (VI-5-1), or a pharmaceutically acceptable salt thereof, or a stereoisomer thereof, includes one or more of the following features, where applicable.

In some embodiments, A₇ and A₈ are each C(=O)O-, -OC(=O)-, -OC(=O)O-, -C(=O)N(R₄)-, - N(R₄)C(=O)-, -N(C(=O)L₁₃OR₄)-, -N(C(=O)L₁₃SR₄)-, -N(C(=O)R₄), -N(Li₃OR₄)-, -OC(=O)C(=O)O-,

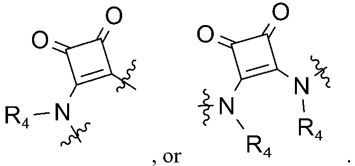

In some embodiments, A₇ and A₈ are each independently -C(=O)O-, -OC(=O)-, -OC(=O)O-, - C(=O)N(R₄)-, -N(R₄)C(=O)-, or -OC(=O)C(=O)O-.

In some embodiments, A₇ and A₈ are each independently -C(=O)O- or -OC(=O)-.

In some embodiments, A₇ is -C(=O)O-.

In some embodiments, A₇ is -OC(=O)-.

In some embodiments, A₈ is -C(=O)O-.

In some embodiments, A₈ is -OC(=O)-.

In some embodiments, A₇ and A₈ are -C(=O)O-.

In some embodiments, A₈ is a bond.

In some embodiments, R₁ and R₂ are each independently -C(H)(OR₇)₂, -C(H)(C(=O)OR₇)₂, - C(H)(OC(=O)R₇)₂-, C(H)(C(=O)OR₇)R₇, or -C(H)(OC(=O)R₇)R₇.

In some embodiments, R₁ is C(H)(OR₇)₂.

In some embodiments, R₂ is -C(H)(OR₇)₂.

In some embodiments, R₁ and R₂ are each -C(H)(OR₇)₂.

In some embodiments, L₁₅ is C₁-C₂ alkylene.

In some embodiments, L₁₅ is C₁ alkylene.

In some embodiments, R₄ is C₁-C₃ alkyl.

In some embodiments, R₄ is C₁ alkyl.

In some embodiments, L₇ and L₈ are each independently C₁-C₁₀ alkylene.

In some embodiments, L₇ and L₈ are each independently C₃-C₉ alkylene.

In some embodiments, L₇ and L₈ are each independently C₄-C₈ alkylene.

In some embodiments, L₇ and L₈ are each independently C₄-C₇ alkylene.

In some embodiments, L₇ and L₈ are each independently C₅-C₇ alkylene, such as C₅ alkylene or C₆ alkylene.

In some embodiments, L₁₁ and L₁₂ are each independently C₂-C₄ alkylene, such as C₂ alkylene or C₃ alkylene.

In some embodiments, each R₇ is independently C₃-C₉ alkyl.

In some embodiments, each R₇ is independently C₅-C₇ alkyl, such as C₆ alkyl.

In some embodiments, A₇ and A₈ are each -C(=O)O-, L₇ and L₈ are each independently C₄-C₈ alkylene, and R₁ and R₂ are -C(H)(OR₇)₂. In some embodiments, each R₇ is independently C₅-C₇ alkyl, such as C₆ alkyl.

In some embodiments, in the amino lipid compound represented by formula (IA), or a pharmaceutically acceptable salt thereof, or a stereoisomer thereof, the amino lipid compound has a structure represented by formula (V-7): 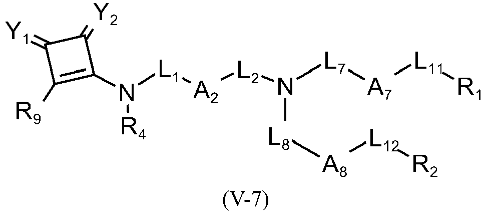 wherein:
Y₁ and Y₂ are each independently O or S;
each R₉ is independently H, halogen, -R_{b}, -N(R_{b})₂, -CN, -N₃, -C(=O)OR_{b}, -OC(=O)R_{b}, -OR_{b}, -SR_{b}, -S(-O)R_{b}, -S(=O)OR_{b}, -S(=O)₂OR_{b}, -N(R_{b})S(=O)₂R_{b}, -NHRₐN(R_{b})₂, -NHRₐORₐN(R_{b})₂, -NHRₐOR_{b}, or - N(RₐOR_{b})₂;
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds;
each R_{b} is independently H, C₁-C₁₂ alkyl, , C₂-C₁₂ alkenyl, C₂-C₁₂ alkynyl, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds;
L₁ and L₂ are each independently C₁-C₆ hydrocarbylene, C₃-C₈ carbocyclic ring, or C₁-C₆ heterohydrocarbylene;
L₇ and L₈ are each independently C₁-C₁₀ hydrocarbylene;
L₁₁ and L₁₂ are each independently C₁-C₅ hydrocarbylene or a bond;
A₂ and A₇ are each independently -N(R₄)-, -C(=O)O-, -OC(=O)-, -OC(=O)O-, -O-, -C(=O)-, -S-, - C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, - N(R₄)C(=O)O-, -N(R₄)C(=O)N(R₄)-, -SC(=O)N(R₄)-, -N(R₄)C(=O)S-, -N(C(=O)L₁₃OR₄)-, - N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, - OP(=O)(OH)O-, -OP(=O)(H)O-, -OS(=O)₂NH-, -NHS(=O)₂O-, -OC(=O)C(=O)O-, -N(R₄)C(=O)C(=O)O-, -OC(=O)C(=O)N(R₄)-, -N(R₄)C(=O)C(=O)N(R₄)-, 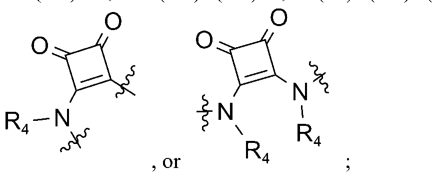
R₁ and R₂ are each independently C₁-C₂₄ hydrocarbyl, -C(R₆)(OL₁₄A₁₂R₇)₂, -C(R₆)(SL₁₄A₁₂R₇)₂, - C(R₆)(SL₁₄A₁₂R₇)(OL₁₄A₁₂R₇), -C(R₆)(C(=O)OL₁₄A₁₂R₇)₂, -C(R₆)(OC(=O)L₁₄A₁₂R₇)₂, - C(R₆)(C(=O)OL₁₄A₁₂R₇)R₇, or -C(R₆)(OC(=O)L₁₄A₁₂R₇)R₇;
each R₇ is independently C₁-C₁₂ hydrocarbyl; and
the definitions of A₈, R₄, R₆, L₁₃, L₁₄, and A₁₂ are as defined in formula (IA).

In some embodiments, in the amino lipid compound represented by formula (V-7), or a pharmaceutically acceptable salt thereof, or a stereoisomer thereof, the amino lipid compound has a structure represented by formula (V-7-1) or (V-7-2): 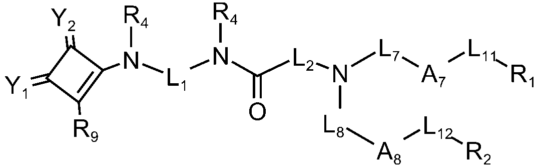 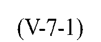 or 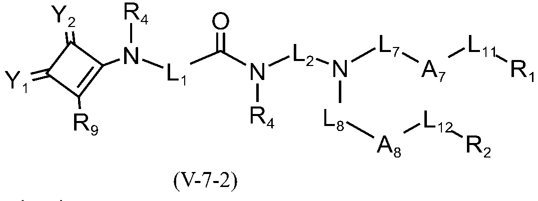 wherein:
A₇ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 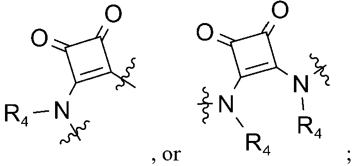
A₈ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 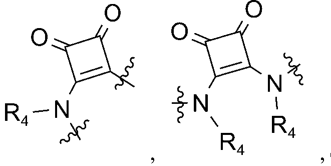 or a bond;
R₁ and R₂ are each independently C₁-C₂₄ alkyl, -C(R₆)(OR₇)₂, -C(R₆)(SR₇)₂, or -C(R₆)(SR₇)(OR₇); and
the definitions of L₁, L₂, L₇, L₈, L₁₁, L₁₂, L₁₃, R₄, R₆, R₇, R₉, Y₁, and Y₂ are as defined in formula (V-7).

In some embodiments, the present disclosure provides an amino lipid compound represented by formula (V-7-1) or (V-7-2), or a pharmaceutically acceptable salt thereof, or a stereoisomer thereof, wherein A₇ and A₈ are each independently -C(=O)O- or -OC(=O)-, L₁₁ is a bond, L₁₂ is a bond, and R₁ and R₂ are each independently a straight C₄-C₁₂ alkyl or a branched C₉-C₁₈ alkyl.

In some embodiments, the present disclosure provides an amino lipid compound represented by formula (V-7-1) or (V-7-2), or a pharmaceutically acceptable salt thereof, or a stereoisomer thereof, wherein A₇ and A₈ are each independently -C(=O)O- or -OC(=O)-, and R₁ and R₂ are each independently -C(H)(OR₇)₂.

In some embodiments, in the amino lipid compound represented by formula (IA), or a pharmaceutically acceptable salt thereof, or a stereoisomer thereof, the amino lipid compound has a structure represented by formula (VI-6): 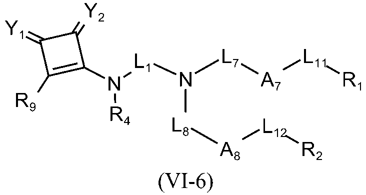 wherein:
Y₁ and Y₂ are each independently O or S;
each R₉ is independently H, halogen, -R_{b}, -N(R_{b})₂, -CN, -N₃, -C(=O)OR_{b}, -OC(=O)R_{b}, -OR_{b}, -SR_{b}, -S(-O)R_{b}, -S(=O)OR_{b}, -S(=O)₂OR_{b}, -N(R_{b})S(=O)₂R_{b}, -NHRₐN(R_{b})₂, -NHRₐORₐN(R_{b})₂, -NHRₐOR_{b}, or - N(RₐOR_{b})₂;
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds;
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds;
L₁ is C₁-C₆ hydrocarbylene, C₃-C₈ carbocyclic ring, or C₁-C₆ heterohydrocarbylene;
L₇ and L₈ are each independently C₁-C₁₀ hydrocarbylene;
L₁₁ and L₁₂ are each independently C₁-C₅ hydrocarbylene or a bond; and
A₇, A₈, R₄, R₁, and R₂ are one selected from the following:
   (1) R₁ and R₂ are each independently -C(R₆)(OR₇)₂, -C(R₆)(SR₇)₂, or -C(R₆)(SR₇)(OR₇);
      A₇ and A₈ are each independently -C(=O)O-, -OC(=O)-, -OC(=O)O-, -OC(=O)C(=O)O-, -O-, - SC(=O)O-, -OC(=O)S-, -C(=O)N(R₄)-, or -N(R₄)C(=O)-; and the definitions of R₄, R₆, and R₇ are as defined in formula (IA);
   (2) R₁ and R₂ are each independently C₁-C₂₄ hydrocarbyl; A₇ is -OC(=O)S- or -SC(=O)O-; A₈ is - C(=O)O-, -OC(=O)-, -OC(=O)O-, -O-, -SC(=O)O-, -OC(=O)S-, -C(=O)N(R₄)-, -N(R₄)C(=O)-, or - OC(=O)C(=O)O-; and the definition of R₄ is as defined in formula (IA); and
   (3) R₁ and R₂ are each independently C₁-C₂₄ hydrocarbyl; A₇ and A₈ are -OC(=S)O-; and the definition of R₄ is as defined in formula (IA).

In some embodiments, in the amino lipid compound represented by formula (IA), or a pharmaceutically acceptable salt thereof, or a stereoisomer thereof, the amino lipid compound has a structure represented by formula (IV-5): 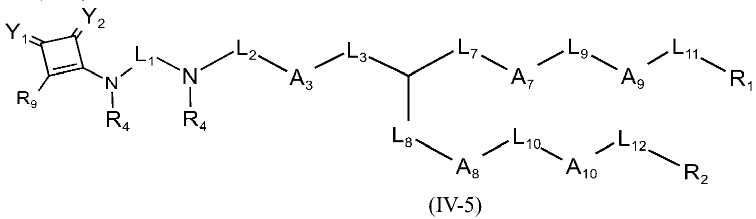 wherein:
Y₁ and Y₂ are each independently O or S;
each R₉ is independently H, halogen, -R_{b}, -N(R_{b})₂, -CN, -N₃, -C(=O)OR_{b}, -OC(=O)R_{b}, -OR_{b}, -SR_{b}, -S(-O)R_{b}, -S(=O)OR_{b}, -S(=O)₂OR_{b}, -N(R_{b})S(=O)₂R_{b}, -NHRₐN(R_{b})₂, -NHRₐORₐN(R_{b})₂, -NHRₐOR_{b}, or - N(RₐOR_{b})₂;
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds;
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds;
L₁ and L₂ are each independently C₁-C₆ hydrocarbylene;
L₃ is C₁-C₆ hydrocarbylene or a bond;
L₇ and L₈ are each independently C₁-C₁₀ hydrocarbylene or a bond;
L₁₁ and L₁₂ are each independently C₁-C₅ hydrocarbylene or a bond;
A₃ is -C(=O)O-, -OC(=O)-, -OC(=O)O-, -C(=O)N(R₄)-, -N(R₄)C(=O)-, -OC(=O)N(R₄)-, - N(R₄)C(=O)O-, -N(C(=O)L₁₃OR₄)-, -N(C(=O)L₁₃SR₄)-, -N(C(=O)R₄), -N(L₁₃OR₄)-, -OC(=O)C(=O)O-, - N(R₄)C(=O)C(=O)O-, -OC(=O)C(=O)N(R₄)-, -N(R₄)C(=O)C(=O)N(R₄)-, 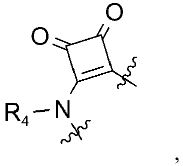 or 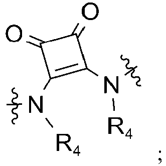
A₇ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC=OC=OO-, 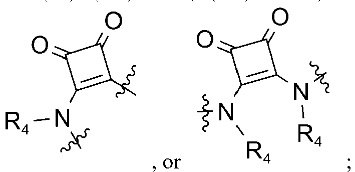
A₈ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 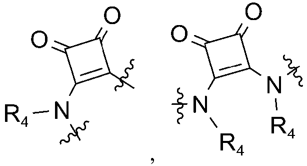 or a bond;
R₁ and R₂ are each independently C₁-C₂₄ alkyl, -C(R₆)(OR₇)₂, -C(R₆)(SR₇)₂, or -C(R₆)(SR₇)(OR₇); and
the definitions of L₉, L₁₀, L₁₃, A₉, A₁₀, R₄, R₆, or R₇ are as defined in formula (IA).

In some embodiments, in the amino lipid compound represented by formula (IV-5), or a pharmaceutically acceptable salt thereof, or a stereoisomer thereof, the amino lipid compound has a structure represented by formula (IV-5-1): 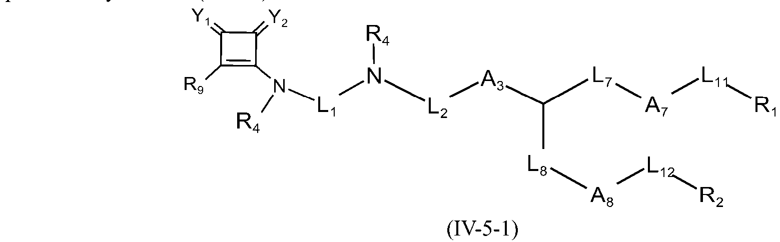 wherein:
R₁ and R₂ are each independently -C(R₆)(OR₇)₂, and the definitions of L₁, L₂, L₇, L₈, L₁₁, L₁₂, A₃, A₇, A₈, R₄, R₆, R₇, R₉, Y₁, and Y₂ are as defined in formula (IA-5).

The amino lipid compound represented by formula (V-7), (V-7-1), (V-7-2), (VI-6), (IV-5), or (IV-5-1), or a pharmaceutically acceptable salt thereof, or a stereoisomer thereof, includes one or more of the following features, where applicable.

In some embodiments, Y₁ and Y₂ are each independently O or S.

In some embodiments, Y₁ is O.

In some embodiments, Y₂ is O.

In some embodiments, Y₁ and Y₂ are O.

In some embodiments, R₉ is -N(R_{b})₂, -NHRₐN(R_{b})₂, -NHRₐORₐN(R_{b})₂, -NHRₐOR_{b}, or -N(RₐOR_{b})₂.

In some embodiments, R₉ is -N(R_{b})₂ or -NHRₐN(R_{b})₂.

In some embodiments, R₉ is -N(R_{b})₂.

In some embodiments, R₉ is -N(H)CH₃.

In some embodiments, Y₁ and Y₂ are each independently O or S, and R₉ is -N(R_{b})₂ or -NHRₐN(R_{b})₂.

In some embodiments, Y₁ and Y₂ are O, and R₉ is -N(R_{b})₂.

In some embodiments, Y₁ and Y₂ are O, and R₉ is -N(H)CH₃.

In some embodiments, each Rₐ is independently C₁-C₁₂ alkylene.

In some embodiments, each Rₐ is independently C₁-C₅ alkylene.

In some embodiments, each R_{b} is independently H, C₁-C₁₂ alkyl, or C₂-C₁₂ alkenyl.

In some embodiments, each R_{b} is independently H, C₁-C₆ alkyl, or C₂-C₃ alkenyl.

In some embodiments, each R_{b} is independently H, or C₁-C₃ alkyl.

In some embodiments, each R_{b} is independently H, or C₁-C₂ alkyl, e.g., C₁ alkyl.

In some embodiments, each R₄ is independently H or C₁-C₆ alkyl.

In some embodiments, A₂ is -C(=O)O-, -OC(=O)-, -OC(=O)O-, -C(=O)N(R₄)-, -N(R₄)C(=O)-, - N(C(=O)L₁₃OR₄)-, -N(C(=O)L₁₃SR₄)-, -N(C(=O)R₄), -N(L₁₃OR₄)-, -OC(=O)C(=O)O-, - N(R₄)C(=O)C(=O)O-, -OC(=O)C(=O)N(R₄)-, -N(R₄)C(=O)C(=O)N(R₄)-, 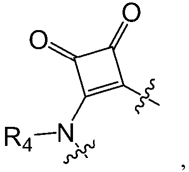 or

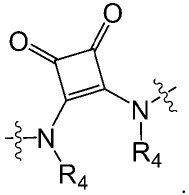

**In** some embodiments, A₂ is -C(=O)O-, -OC(=O)-, -OC(=O)O-, -C(=O)N(R₄)-, -N(R₄)C(=O)-, - OC(=O)C(=O)O-, -N(R₄)C(=O)C(=O)O-, -OC(=O)C(=O)N(R₄)-, or -N(R₄)C(=O)C(=O)N(R₄)-.

In some embodiments, A₂ is -C(=O)O-, -OC(=O)-, -OC(=O)O-, -C(=O)N(R₄)-, -N(R₄)C(=O)-, - OC(=O)C(=O)O-, or -N(R₄)C(=O)C(=O)O-.

In some embodiments, A₂ is -C(=O)O-, -OC(=O)O-, -OC(=O)-, -C(=O)N(R₄)-, or -N(R₄)C(=O)-.

In some embodiments, A₂ is -C(=O)N(R₄)- or -N(R₄)C(=O)-.

In some embodiments, A₂ is -C(=O)NH-.

In some embodiments, A₂ is -NHC(=O)-.

In some embodiments, A₃ is -C(=O)O-, -OC(=O)-, -OC(=O)O-, -C(=O)N(R₄)-, -N(R₄)C(=O)-, - OC(=O)N(R₄)-, -N(R₄)C(=O)O-, -N(C(=O)L₁₃OR₄)-, -N(C(=O)L₁₃SR₄)-, -N(C(=O)R₄), -N(L₁₃OR₄)-, - OC(=O)C(=O)O-, -N(R₄)C(=O)C(=O)O-, -OC(=O)C(=O)N(R₄)-, -N(R₄)C(=O)C(=O)N(R₄)-,

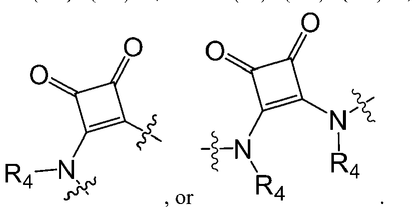

In some embodiments, A₃ is -C(=O)O-, -OC(=O)-, -OC(=O)O-, -C(=O)N(R₄)-, -N(R₄)C(=O)-, - OC(=O)N(R₄)-, -N(R₄)C(=O)O-, -OC(=O)C(=O)O-, -N(R₄)C(=O)C(=O)O-, -OC(=O)C(=O)N(R₄)-, or - N(R₄)C(=O)C(=O)N(R₄)-.

**In** some embodiments, A₃ is -OC(=O)-, -C(=O)O-, -OC(=O)O-, -C(=O)N(R₄)-, -N(R₄)C(=O)-, - OC(=O)N(R₄)-, or -N(R₄)C(=O)O-.

In some embodiments, A₃ is -C(=O)O-, -OC(=O)-, -OC(=O)O-, -C(=O)N(H)-, -N(H)C(=O)-, - OC(=O)N(H)-, or -N(H)C(=O)O-.

In some embodiments, A₃ is -OC(=O)O-, -N(H)C(=O)-, or -C(=O)N(H)-.

In some embodiments, A₃ is -OC(=O)O-, -N(H)C(=O)O-, or -OC(=O)N(H)-.

In some embodiments, A₇ and A₈ are each independently -C(=O)O-, -OC(=O)-, -O-, -SC(=O)O-, - OC(=O)S-, -C(=O)N(R₄)-, -N(R₄)C(=O)-, -N(C(=O)L₁₃OR₄)-, -N(C(=O)L₁₃SR₄)-, -N(C(=O)R₄), - N(L₁₃OR₄)-, -OC(=O)C(=O)O-,

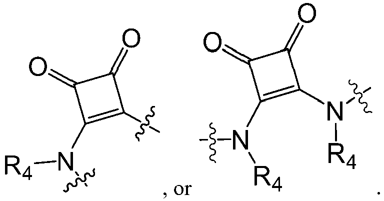

In some embodiments, A₇ and A₈ are each independently -C(=O)O-, -OC(=O)-, -OC(=O)O-, - C(=O)N(R₄)-, -N(R₄)C(=O)-, -N(C(=O)L₁₃OR₄)-, -N(C(=O)L₁₃SR₄)-, -N(C(=O)R₄), -N(L₁₃OR₄)-, - OCE=EOYC(=O)O-,

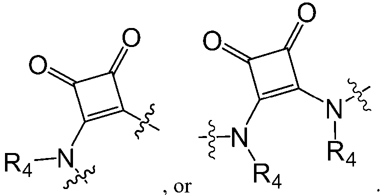

In some embodiments, A₇ and A₈ are each independently -C(=O)O-, -OC(=O)-, -OC(=O)O-, - C(=O)N(R₄)-, -N(R₄)C(=O)-, or -OC(=O)C(=O)O-.

In some embodiments, A₇ and A₈ are each independently -C(=O)O-, -OC(=O)-, or -OC(=O)O-.

In some embodiments, A₇ and A₈ are each independently -C(=O)O-, -OC(=O)-, -O-, -SC(=O)O-, - OC(=O)S-, -C(=O)N(R₄)-, or -N(R₄)C(=O)-.

In some embodiments, A₇ is -C(=O)O-, -OC(=O)-, -O-, -SC(=O)O-, -OC(=O)S-, -C(=O)N(R₄)-, or - N(R₄)C(=O)-.

In some embodiments, A₇ is -C(=O)O-, -OC(=O)-, -O-, -C(=O)N(R₄)-, or -N(R₄)C(=O)-.

In some embodiments, A₇ is -C(=O)N(H)- or -N(H)C(=O)-.

In some embodiments, A₈ is -C(=O)O-, -OC(=O)-, -O-, -SC(=O)O-, -OC(=O)S-, -C(=O)N(R₄)-, or - N(R₄)C(=O)-.

In some embodiments, A₈ is -O-, -C(=O)N(R₄)-, or -N(R₄)C(=O)-.

In some embodiments, A₈ is -SC(=O)O- or -OC(=O)S-.

In some embodiments, A₇ and A₈ are each independently -C(=O)O- or -OC(=O)-.

In some embodiments, A₇ is -C(=O)O-.

In some embodiments, A₇ is -OC(=O)-.

In some embodiments, A₈ is -C(=O)O-.

In some embodiments, A₈ is -OC(=O)-.

In some embodiments, A₇ and A₈ are -C(=O)O-.

In some embodiments, A₇ and A₈ are -OC(=O)-.

In some embodiments, A₇ is -C(=O)O-, and A₈ is -OC(=O)-.

In some embodiments, A₇ is -OC(=O)-, and A₈ is -C(=O)O-.

In some embodiments, A₇ is -O-, and A₈ is -C(=O)O-.

In some embodiments, A₇ and A₈ are -O-.

In some embodiments, A₇ is -C(=O)N(H)-, and A₈ is -C(=O)O-.

In some embodiments, A₈ is -C(=O)N(H)- or -N(H)C(=O)-.

In some embodiments, A₇ and A₈ are -SC(=O)O-.

In some embodiments, A₇ and A₈ are -OC(=O)S-.

In some embodiments, A₇ is -C(=O)O-, and A₈ is -OC(=O)S-.

In some embodiments, A₇ is -C(O)O-, -OC(O)-, -O-, -C(=O)N(R₄)-, or -N(R₄)C(=O)-, and A₈ is -O-, - C(=O)N(R₄)-, or -N(R₄)C(=O)-.

In some embodiments, A₇ is -C(=O)O-, -OC(=O)-, -SC(=O)O-, or -OC(=O)S-, and A₈ is -SC(=O)O- or -OC(=O)S-.

In some embodiments, A₇ is -C(=O)O-, -OC(=O)-, -OC(=S)O-, -SC(=O)O-, or -OC(=O)S-, and A₈ is - SC(=O)O-, -OC(=O)S-, or -OC(=S)O-.

In some embodiments, A₈ is a bond.

In some embodiments, A₈ is -C(=O)O-, -OC(=O)-, -O-, -SC(=O)O-, -OC(=O)S-, -C(=O)N(R₄)-, - N(R₄)C(=O)-, -N(C(=O)L₁₃OR₄)-, -N(C(=O)L₁₃SR4)-, -N(C(=O)R₄), -N(L₁₃ OR₄)-, or -OC(=O)C(=O)O-; and A₇ is -C(=O)S- or -SC(=O)-.

In some embodiments, A₇ and A₈ are each independently -C(=O)O-, -OC(=O)-, -O-, -C(=O)N(R₄)-, or -N(R₄)C(=O)-.

In some embodiments, A₇ and A₈ are each independently -C(=O)O-, -OC(=O)-, -O-, -C(=O)N(H)-, or - N(H)C(=O)-.

In some embodiments, A₇ and A₈ are each independently -C(=O)O- or -OC(=O)-.

In some embodiments, A₇ is -C(=O)O-, -OC(=O)-, -O-, -C(=O)N(R₄)-, or -N(R₄)C(=O)-. In some embodiments, A₈ is -O-, -C(=O)N(R₄)-, or -N(R₄)C(=O)-.

In some embodiments, A₇ is -C(=O)O-, -OC(=O)-, -O-, -C(=O)N(R₄)-, or -N(R₄)C(=O)-. In some embodiments, A₈ is -O-, -C(=O)N(R₄)-, or -N(R₄)C(=O)-, and R₁ and R₂ are each independently - C(R₆)(OR₇)₂.

In some embodiments, A₇ and A₈ are each independently -C(=O)O-, -OC(=O)-, -O-, -C(=O)N(R₄)-, or -N(R₄)C(=O)-, and R₁ and R₂ are each independently -C(R₆)(OR₇)₂, -C(R₆)(SR₇)₂, or -C(R₆)(SR₇)(OR₇).

In some embodiments, A₇ and A₈ are each independently -C(=O)O-, -OC(=O)-, -O-, -C(=O)N(R₄)-, or -N(R₄)C(=O)-, and R₁ and R₂ are each independently -C(R₆)(OR₇)₂.

In some embodiments, A₇ and A₈ are each independently -C(=O)O-, -OC(=O)-, -O-, -C(=O)N(R₄)-, or -N(R₄)C(=O)-, and R₁ and R₂ are each independently -C(R₆)(SR₇)₂ or -C(R₆)(SR₇)(OR₇).

In some embodiments, A₇ and A₈ are each independently -C(=O)O- or -OC(=O)-, and R₁ and R₂ are each independently -C(R₆)(SR₇)₂ or -C(R₆)(SR₇)(OR₇).

In some embodiments, A₉ and A₁₀ are each independently -OC(=O)-, -C(=O)O-, or a bond.

In some embodiments, A₈ is -OC(=O)-.

In some embodiments, A₉ is -C(=O)O-.

In some embodiments, A₈ is a bond.

In some embodiments, A₁₀ is -OC(=O)-.

In some embodiments, A₁₀ is -C(=O)O-.

In some embodiments, A₁₀ is a bond.

In some embodiments, A₉ and A₁₀ are -OC(=O)-.

In some embodiments, A₉ and A₁₀ are -C(=O)O-.

In some embodiments, A₉ and A₁₀ are a bond.

In some embodiments, A₈ is -OC(=O)-, and A₁₀ is a bond.

In some embodiments, A₈ is -C(=O)O-, and A₁₀ is a bond.

In some embodiments, R₁ and R₂ are each independently C₁-C₂₄ alkyl, -C(R₆)(OL₁₄A₁₂R₇)₂, - C(R₆)(SL₁₄A₁₂R₇)₂, or -C(R₆)(SL₁₄A₁₂R₇)(OL₁₄A₁₂R₇).

In some embodiments, R₁ and R₂ are each independently C₁-C₂₄ alkyl.

In some embodiments, R₁ is a straight C₁-C₁₈ alkyl.

In some embodiments, R₁ is a straight C₁-C₁₅ alkyl.

In some embodiments, R₁ is a straight C₄-C₁₂ alkyl.

In some embodiments, R₁ is a straight C₇-C₁₀ alkyl, such as C₇-C₉ alkyl, C₇-C₉ alkyl, C₇ alkyl, or C₈ alkyl.

In some embodiments, R₁ is a substituted straight C₁-C₁₅ alkyl, or a substituted straight C₄-C₁₂ alkyl, or a substituted straight C₇-C₁₀ alkyl, or a substituted straight C₇-C₉ alkyl, or a substituted straight C₇-C₈ alkyl, or a substituted straight C₇ alkyl, or a substituted straight C₈ alkyl, e.g., substituted with -OR_{b}, -RₐOR_{b}, - OC(=O)R_{b}, -C(=O)OR_{b}, -C(=O)R_{b}, -RₐOC(=O)R_{b}, -RₐC(=O)OR_{b}, or -RₐC(=O)R_{b}, wherein each Rₐ is independently C₁-C₁₂ alkylene, and each R_{b} is independently H or C₁-C₁₂ alkyl.

In some embodiments, R₁ is a branched C₇-C₂₂ alkyl.

In some embodiments, R₁ is a branched C₉-C₂₀ alkyl.

In some embodiments, R₁ is a branched C₉-C₁₈ alkyl.

In some embodiments, R₁ is a branched C₉-C₁₇ alkyl, such as a branched C₉-C₁₃ alkyl, a branched C₁₇ alkyl, or a branched C₁₉ alkyl.

In some embodiments, R₁ is a substituted branched C₉-C₂₀ alkyl, or a substituted branched C₉-C₁₈ alkyl, or a substituted branched C₉-C₁₇ alkyl, or a substituted branched C₉-C₁₃ alkyl, or a substituted branched C₁₇ alkyl, or a substituted branched C₁₉ alkyl, e.g., substituted with -OR_{b}, -RₐOR_{b}, -OC(=O)R_{b}, -C(=O)OR_{b}, - C(=O)R_{b}, -RₐOC(=O)R_{b}, -RₐC(=O)OR_{b}, or -RₐC(=O)R_{b}, wherein each Rₐ is independently C₁-C₁₂ alkylene, and each R_{b} is independently H or C₁-C₁₂ alkyl.

In some embodiments, R₂ is a straight C₁-C₁₈ alkyl.

In some embodiments, R₂ is a straight C₁-C₁₅ alkyl.

In some embodiments, R₂ is a straight C₄-C₁₂ alkyl.

In some embodiments, R₂ is a straight C₇-C₁₀ alkyl, such as C₇-C₉ alkyl, C₇-C₈ alkyl, C₇ alkyl, or C₈ alkyl.

In some embodiments, R₂ is a substituted straight C₁-C₁₅ alkyl, or a substituted straight C₄-C₁₂ alkyl, or a substituted straight C₇-C₁₀ alkyl, or a substituted straight C₇-C₉ alkyl, or a substituted straight C₇-C₈ alkyl, or a substituted straight C₇ alkyl, or a substituted straight C₈ alkyl, e.g., substituted with -OR_{b}, -RₐOR_{b}, - OC(=O)R_{b}, -C(=O)OR_{b}, -C(=O)R_{b}, -RₐOC(=O)R_{b}, -RₐC(=O)OR_{b}, or -RₐC(=O)R_{b}, wherein each Rₐ is independently C₁-C₁₂ alkylene, and each R_{b} is independently H or C₁-C₁₂ alkyl.

In some embodiments, R₂ is a branched C₇-C₂₂ alkyl.

In some embodiments, R₂ is a branched C₉-C₂₀ alkyl.

In some embodiments, R₂ is a branched C₉-C₁₈ alkyl.

In some embodiments, R₂ is a branched C₉-C₁₇ alkyl, such as a branched C₉-C₁₃ alkyl, a branched C₁₇ alkyl, or a branched C₁₉ alkyl.

In some embodiments, R₂ is a substituted branched C₉-C₂₀ alkyl, or a substituted branched C₉-C₁₈ alkyl, or a substituted branched C₉-C₁₇ alkyl, or a substituted branched C₉-C₁₃ alkyl, or a substituted branched C₁₇ alkyl, or a substituted branched C₁₉ alkyl, e.g., substituted with -OR_{b}, -RₐOR_{b}, -OC(=O)R_{b}, -C(=O)OR_{b}, - C(=O)R_{b}, -RₐOC(=O)R_{b}, -RₐC(=O)OR_{b}, or -RₐC(=O)R_{b}, wherein each Rₐ is independently C₁-C₁₂ alkylene, and each R_{b} is independently H or C₁-C₁₂ alkyl.

In some embodiments, R₁ is a straight C₄-C₁₂ alkyl, and R₂ is a branched C₉-C₁₈ alkyl.

In some embodiments, R₁ is a straight C₇-C₁₀ alkyl, and R₂ is a branched C₉-C₁₇ alkyl.

In some embodiments, R₁ is a branched C₉-C₁₈ alkyl, and R₂ is a branched C₉-C₁₈ alkyl.

In some embodiments, R₁ is a branched C₉-C₁₇ alkyl, and R₂ is a branched C₉-C₁₇ alkyl.

In some embodiments, L₁₁ is a bond, and the branching of R₁ occurs at the α, β, or γ position of A₇, for example, the branching of R₁ occurs at the α position of A₇: 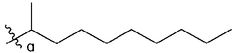 ; or the branching of R₁ occurs at the β position of A₇:

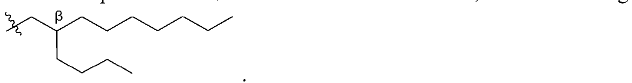

In some embodiments, L₁₂ is a bond, and the branching of R₂ occurs at the α, β, or γ position of A₈, for example, the branching of R₂ occurs at the α position of A₈: 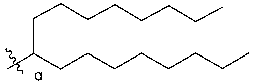 ; or the branching of R₂ occurs at the β position of A₈:

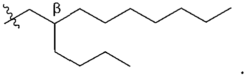

In some embodiments, R₁ or R₂ is one of the following structures: 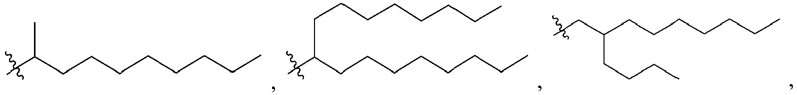 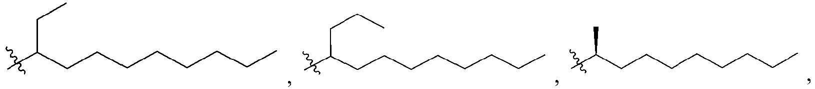 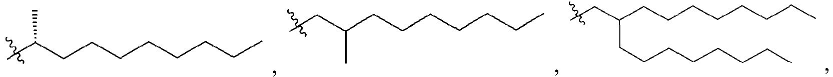 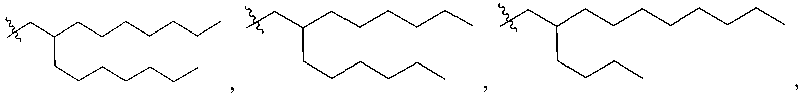 and

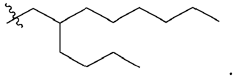

In some embodiments, R₁ and R₂ are each independently -C(R₆)(OL₁₄A₁₂R₇)₂, -C(R₆)(SL₁₄A₁₂R₇)₂, or - C(R₆)(SL₁₄A₁₂R₇)(OL₁₄A ₁₂R₇).

In some embodiments, R₁ and R₂ are each independently -C(R₆)(OR₇)₂, -C(R₆)(SR₇)₂, or - C(R₆)(SR₇)(OR₇).

In some embodiments, R₁ and R₂ are each independently -C(H)(OR₇)₂, -C(H)(SR₇)₂, or - C(H)(SR₇)(OR₇).

In some embodiments, R₁ is -C(R₆)(OR₇)₂.

In some embodiments, R₁ is C(H)(OR₇)₂.

In some embodiments, R₂ is -C(R₆)(OR₇)₂.

In some embodiments, R₂ is -C(H)(OR₇)₂.

In some embodiments, R₁ is -C(H)(SR₇)₂.

In some embodiments, R₁ is -C(H)(SR₇)(OR₇).

In some embodiments, R₂ is -C(H)(SR₇)₂.

In some embodiments, R₂ is -C(H)(SR₇)(OR₇).

In some embodiments, R₁ and R₂ are -C(H)(OR₇)₂.

In some embodiments, R₁ is -C(H)(OR₇)₂, -C(H)(SR₇)₂, or -C(H)(SR₇)(OR₇), and R₂ is -C(H)(SR₇)₂ or -C(H)(SR₇)(OR₇).

In some embodiments, R₁ and R₂ are -C(H)(SR₇)₂.

In some embodiments, R₁ and R₂ are -C(H)(SR₇)(OR₇).

In some embodiments, R₁ is -C(H)(OR₇)₂, and R₂ is -C(H)(SR₇)₂.

In some embodiments, R₁ is -C(H)(SR₇)₂, and R₂ is -C(H)(SR₇)₂.

In some embodiments, R₁ is -C(H)(SR₇)₂, and R₂ is -C(H)(SR₇)(OR₇).

In some embodiments, R₁ is -C(H)(OR₇)₂, and R₂ is -C(H)(SR₇)(OR₇).

In some embodiments, R₁ and R₂ are each independently C₁-C₂₄ alkyl or -C(R₆)(OR₇)₂.

In some embodiments, A₇ and A₈ are each independently -C(=O)O- or -OC(=O)-, and R₁ and R₂ are each independently C₁-C₂₄ alkyl or -C(R₆)(OR₇)₂.

In some embodiments, L₁₁ is C₁-C₅ alkylene, L₁₂ is C₁-C₅ alkylene, R₁ is -C(H)(OR₇)₂, R₂ is -C(H)(OR₇)₂, and R₇ is C₃-C₁₀ alkyl.

In some embodiments, A₂ is -C(=O)N(R₄)- or -N(R₄)C(=O)-, R₁ is a straight C₄-C₁₂ alkyl, and R₂ is a branched C₉-C₁₈ alkyl.

In some embodiments, A₂ is -C(=O)N(R₄)- or -N(R₄)C(=O)-, R₁ is a branched C₉-C₁₈ alkyl, and R₂ is a branched C₉-C₁₈ alkyl.

In some embodiments, A₂ is -C(=O)N(R₄)- or -N(R₄)C(=O)-, L₁₁ is C₁-C₅ alkylene, L₁₂ is C₁-C₅ alkylene, R₁ is -C(H)(OR₇)₂, and R₂ is -C(H)(OR₇)₂.

In some embodiments, A₇ and A₈ are each independently -C(=O)O-, -OC(=O)-, -OC(=O)O-, - C(=O)N(R₄)-, -N(R₄)C(=O)-, -N(C(=O)L₁₃OR₄)-, -N(C(=O)L₁₃SR₄)-, -N(C(=O)R₄), -N(L₁₃OR₄)-, or - OC(=O)C(=O)O-, and R₁ and R₂ are each independently C₁-C₂₄ alkyl.

In some embodiments, A₇ and A₈ are each independently -C(=O)O-, -OC(=O)-, -OC(=O)O-, - C(=O)N(R₄)-, -N(R₄)C(=O)-, -N(C(=O)L₁₃OR₄)-, -N(C(=O)L₁₃SR₄)-, -N(C(=O)R₄), -N(L₁₃OR₄)-, or - OC(=O)C(=O)O-, and R₁ and R₂ are each independently -C(R₆)(OL₁₄A₁₂R₇)₂.

In some embodiments, A₂ is -C(=O)N(R₄)- or -N(R₄)C(=O)-, A₇ is -C(=O)O- or -OC(=O)-, A₈ is - C(=O)O- or -OC(=O)-, R₁ is a straight C₄-C₁₂ alkyl, and R₂ is a branched C₉-C₁₈ alkyl.

In some embodiments, A₂ is -C(=O)N(R₄)- or -N(R₄)C(=O)-, A₇ is -C(=O)O- or -OC(=O)-, A₈ is - C(=O)O- or -OC(=O)-, R₁ is a branched C₉-C₁₈ alkyl, and R₂ is a branched C₉-C₁₈ alkyl.

In some embodiments, A₂ is -C(=O)N(R₄)- or -N(R₄)C(=O)-, A₇ is -C(=O)O- or -OC(=O)-, A₈ is - C(=O)O- or-OC(=O)-, L₁₁ is C₁-C₅ alkylene, L₁₂ is C₁-C₅ alkylene, R₁ is -C(H)(OR₇)₂, and R₂ is -C(H)(OR₇)₂.

In some embodiments, A₇ is -C(=O)O-, -OC(=O)-, -O-, -C(=O)N(R₄)-, or -N(R₄)C(=O)-, A₈ is - C(=O)O-, -OC(=O)-, -O-, -C(=O)N(R₄)-, or -N(R₄)C(=O)-, and R₁ and R₂ are -C(H)(OR₇)₂.

In some embodiments, A₇ is -C(=O)O-, -OC(=O)-, -O-, -C(=O)N(R₄)-, or -N(R₄)C(=O)-, A₈ is -O-, - C(=O)N(R₄)-, or -N(R₄)C(=O)-, and R₁ and R₂ are -C(H)(OR₇)₂.

In some embodiments, A₇ is -C(=O)O- or -OC(=O)-, A₈ is -C(=O)O- or -OC(=O)-, R₁ is -C(H)(OR₇)₂, -C(H)(SR₇)₂, or -C(H)(SR₇)(OR₇), and R₂ is -C(H)(SR₇)₂ or -C(H)(SR₇)(OR₇).

In some embodiments, A₇ is -SC(=O)O-, -OC(=S)O-, -C(=O)O-, or -OC(=O)S-, A₈ is -OC(=S)O-, - SC(=O)O-, or -OC(=O)S-, and R₁ and R₂ are branched C₉-C₁₈ alkyl.

In some embodiments, A₇ is -SC(=O)O-, -C(=O)O-, or -OC(=O)S-, A₈ is -SC(=O)O- or -OC(=O)S-, and R₁ and R₂ are branched C₉-C₁₈ alkyl.

In some embodiments, A₂ is -N(R₄)-, A₃ is -OC(O)-, -C(O)O-, -N((R₄))C(=O)-, -C(=O)N((R₄))-, - OC(O)O-, -N((R₄))C(=O)O-, or -OC(=O)N((R₄))-, A₇ and A₈ are each independently -C(=O)O- or -OC(=O)-, R₁ is -C(H)(OR₇)₂, and R₂ is -C(H)(OR₇)₂.

In some embodiments, A₂ is -N(R₄)-, A₃ is -OC(O)-, -C(O)O-, -N((R₄))C(=O)-, -C(=O)N((R₄))-, - OC(O)O-, -N((R₄))C(=O)O-, or -OC(=O)N((R₄))-, A₇ and A₈ are each independently -C(=O)O- or -OC(=O)-, A₉ and A₁₀ are each independently -C(=O)O- or -OC(=O)-, R₁ is -C(H)(OR₇)₂, and R₂ is -C(H)(OR₇)₂.

In some embodiments, each R₄ is independently H or C₁-C₃ alkyl.

In some embodiments, each R₄ is independently H or C₁-C₂ alkyl.

In some embodiments, each R₄ is independently H or methyl.

In some embodiments, each R₇ is independently C₁-C₁₂ alkyl, or C₂-C₁₂ alkenyl, or C₂-C₁₂ alkynyl, such as C₂-C₁₁ alkyl, C₇ alkenyl, or C₇ alkynyl.

In some embodiments, each R₇ is independently C₃-C₁₀ alkyl.

In some embodiments, each R₇ is independently C₅-C₇ alkyl, e.g., C₆-C₇ alkyl.

In some embodiments, each R₇ is independently C₁-C₁₂ alkyl substituted with methyl.

In some embodiments, L₁ is C₁-C₆ alkylene.

In some embodiments, L₁ is C₁-C₅ alkylene.

In some embodiments, L₁ is C₁-C₄ alkylene, e.g., C₁, C₂, or C₃ alkylene.

In some embodiments, L₁ is C₁-C₅ alkylene substituted with a substituent, such as C₁-C₄ alkylene substituted with a substituent, or, C₁, C₂, or C₃ alkylene substituted with a substituent, the substituent including, but not limited to, -OH, -SH, halogen, O, S, N, -CN, C₃-C₈ cyclohydrocarbyl, aryl, heterocyclic ring, -R_{b}, -RₐN(R_{b})₂, -RₐOR_{b}, -RₐSR_{b}, -RₐOC(=O)OR_{b}, -RₐOC(=O)SR_{b}, -RₐC(=O)N(Re)₂, -OC(=O)OR_{b}, - OC(=O)SR_{b}, -C(=O)N(R_{b})₂, -RₐOC(=O)R_{b}, -RₐC(=O)OR_{b}, -OC(=O)R_{b}, -C(=O)OR_{b}, -RₐSR_{b}, or 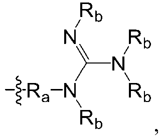 wherein each Rₐ is independently C₁-C₁₂ alkylene, and each R_{b} is independently H or C₁-C₁₂ alkyl.

In some embodiments, L₁ is C₁-C₅ alkylene substituted with hydroxyl, for example, L₁ is C₂-C₃ alkylene substituted with hydroxyl:
e.g., 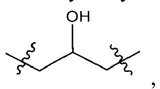 and chiral structures thereof 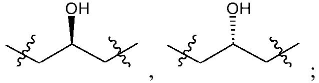
e.g., 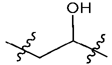 , and chiral structures thereof

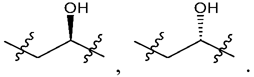

In some embodiments, L₂ is C₁-C₆ alkylene.

In some embodiments, L₂ is C₂-C₅ alkylene.

In some embodiments, L₂ is C₂-C₄ alkylene.

In some embodiments, L₂ is C₂-C₅ alkylene substituted with a substituent, e.g., C₂-C₃ alkylene substituted with a substituent, the substituent including C₁-C₄ alkyl, -OH, -RₐOC(=O)R_{b}, or -RₐC(=O)OR_{b}, wherein each Rₐ is independently C₁-C₁₂ alkylene, and each R_{b} is independently H or C₁-C₁₂ alkyl.

In some embodiments, L₂ is C₂-C₃ alkylene substituted with methyl, for example, L₂ is C₃ alkylene substituted with methyl, e.g.,

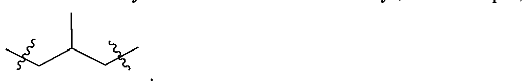

In some embodiments, L₃ is C₁-C₆ alkylene.

In some embodiments, L₃ is a bond.

In some embodiments, L₇ and L₈ are each independently C₁-C₁₄ alkylene.

In some embodiments, L₇ and L₈ are each independently C₁-C₁₀ alkylene.

In some embodiments, L₇ and L₈ are each independently C₄-C₈ alkylene, such as C₄-C₇ alkylene, C₅-C₇ alkylene, C₅ alkylene, or C₆ alkylene.

In some embodiments, L₇ and L₈ are each independently C₁-C₂ alkylene or a bond.

In some embodiments, L₉ and L₁₀ are each independently C₁-C₁₄ alkylene.

In some embodiments, L₉ and L₁₀ are each independently C₁-C₁₀ alkylene.

In some embodiments, L₉ and L₁₀ are each independently C₄-C₈ alkylene, such as C₄-C₇ alkylene, C₅-C₇ alkylene, C₅ alkylene, or C₆ alkylene.

In some embodiments, L₉ and L₁₀ are each independently C₄-C₅ alkylene or a bond.

In some embodiments, L₁₁ is C₁-C₅ alkylene.

In some embodiments, L₁₁ is C₂-C₄ alkylene, e.g., C₃ alkylene.

In some embodiments, L₁₁ is a bond.

In some embodiments, L₁₂ is C₁-C₅ alkylene.

In some embodiments, L₁₂ is C₂-C₄ alkylene, e.g., C₃ alkylene.

In some embodiments, L₁₂ is a bond.

In some embodiments, L₁₁ is a bond, and L₁₂ is a bond.

In some embodiments, L₁₁ is C₂-C₄ alkylene, and L₁₂ is C₂-C₄ alkylene.

In some embodiments, L₁₁ is a bond, and R₁ is a straight C₄-C₁₂ alkyl or a branched C₉-C₁₈ alkyl.

In some embodiments, L₁₂ is a bond, and R₂ is a straight C₄-C₁₂ alkyl or a branched C₉-C₁₈ alkyl.

In some embodiments, Y₁ is O, Y₂ is O, R₉ is -N(H)CH₃, L₁ is C₁-C₅ alkylene, A₂ is -C(=O)N(R₄)- or - N(R₄)C(=O)-, L₂ is C₂-C₅ alkylene, L₇ is C₁-C₁₀ alkylene, L₈ is C₁-C₁₀ alkylene, A₇ is -C(=O)O- or -OC(=O)-, A₈ is -C(=O)O- or -OC(=O)-, L₁₁ is a bond, L₁₂ is a bond, R₁ is a straight C₄-C₁₂ alkyl or a branched C₉-C₁₈ alkyl, and R₂ is a straight C₄-C₁₂ alkyl or a branched C₉-C₁₈ alkyl.

In some embodiments, Y₁ is O, Y₂ is O, R₉ is -N(H)CH₃, L₁ is C₁-C₅ alkylene, A₂ is -C(=O)N(R₄)- or - N(R₄)C(=O)-, L₂ is C₂-C₅ alkylene, L₇ is C₁-C₁₀ alkylene, L₈ is C₁-C₁₀ alkylene, A₇ is -C(=O)O- or -OC(=O)-, A₈ is -C(=O)O- or -OC(=O)-, L₁₁ is C₂-C₄ alkylene, L₁₂ is C₂-C₄ alkylene, R₁ is -C(H)(OR₇)₂, and R₂ is - C(H)(OR₇)₂.

In some embodiments, Y₁ is O, Y₂ is O, R₉ is -N(H)CH₃, L₁ is C₁-C₅ alkylene, L₇ is C₁-C₁₀ alkylene, L₈ is C₁-C₁₀ alkylene, A₇ is -C(=O)O-, -OC(=O)-, -O-, -C(=O)N(R₄)-, or -N(R₄)C(=O)-, A₈ is -C(=O)O-, - OC(=O)-, -O-, -C(=O)N(R₄)-, or -N(R₄)C(=O)-, L₁₁ is C₂-C₄ alkylene, L₁₂ is C₂-C₄ alkylene, and R₁ and R₂ are -C(H)(OR₇)₂.

In some embodiments, Y₁ is O, Y₂ is O, R₉ is -N(H)CH₃, L₁ is C₁-C₅ alkylene, L₇ is C₁-C₁₀ alkylene, L₈ is C₁-C₁₀ alkylene, A₇ is -C(=O)O- or -OC(=O)-, A₈ is -C(=O)O- or -OC(=O)-, L₁₁ is C₂-C₄ alkylene, L₁₂ is C₂-C₄ alkylene, R₁ is -C(H)(OR₇)₂, -C(H)(SR₇)₂, or -C(H)(SR₇)(OR₇), and R₂ is -C(H)(SR₇)₂, or - C(H)(SR₇)(OR₇).

In some embodiments, Y₁ is O, Y₂ is O, R₉ is -N(H)CH₃, L₁ is C₁-C₅ alkylene, L₇ is C₁-C₁₀ alkylene, L₈ is C₁-C₁₀ alkylene, A₇ is -SC(=O)O-, -OC(=S)O-, -C(=O)O-, -OC(=O)-, or -OC(=O)S-, A₈ is -SC(=O)O-, OC(=S)O-, or -OC(=O)S-, L₁₁ and L₁₂ are a bond, and R₁ and R₂ are branched C₉-C₁₈ alkyl.

In some embodiments, Y₁ is O, Y₂ is O, R₉ is -N(H)CH₃, L₁ is C₁-C₅ alkylene, L₇ is C₁-C₁₀ alkylene, L₈ is C₁-C₁₀ alkylene, A₇ is -SC(=O)O-, -C(=O)O-, -OC(=O)-, or -OC(=O)S-, A₈ is -SC(=O)O- or -OC(=O)S-, L₁₁ and L₁₂ are a bond, and R₁ and R₂ are branched C₉-C₁₈ alkyl.

In some embodiments, Y₁ is O, Y₂ is O, R₉ is -N(H)CH₃, L₁ is C₁-C₅ alkylene, each R₄ is independently H or C₁-C₂ alkyl, L₂ is C₂-C₅ alkylene, A₃ is -OC(=O)-, -C(=O)O-, -N(R₄)C(=O)-, -C(=O)N(R₄)-, -OC(=O)O-, -N(R₄)C(=O)O-, or -OC(=O)N(R₄)-, L₇ is C₁-C₁₀ alkylene, L₈ is C₁-C₁₀ alkylene, A₇ and A₈ are each independently -C(=O)O- or -OC(=O)-, L₁₁ and L₁₂ are a bond, R₁ is -C(H)(OR₇)₂, and R₂ is -C(H)(OR₇)₂.

In some embodiments, Y₁ is O, Y₂ is O, R₉ is -N(H)CH₃, L₁ is C₁-C₅ alkylene, each R₄ is independently H or C₁-C₂ alkyl, L₂ is C₂-C₅ alkylene, A₃ is -OC(=O)-, -C(=O)O-, -N(R₄)C(=O)-, -C(=O)N(R₄)-, -OC(=O)O-, -N(R₄)C(=O)O-, or -OC(=O)N(R₄)-, L₇ is C₁-C₂ alkylene or a bond, L₈ is C₁-C₂ alkylene or a bond, A₇ and A₈ are each independently -C(=O)O- or -OC(=O)-, L₉ is C₄-C₅ alkylene, L₁₀ is C₄-C₅ alkylene, A₉ and A₁₀ are each independently -C(=O)O- or -OC(=O)-, L₁₁ and L₁₂ are a bond, R₁ is -C(H)(OR₇)₂, and R₂ is - C(H)(OR₇)₂.

In some embodiments, in the amino lipid compound represented by formula (I-1), (I-3), (IV-1), (IV-3), (V-1), (V-3), (VI-1), or (VI-3), or a pharmaceutically acceptable salt thereof, or a stereoisomer thereof, R₁ and/or R₂ are/is -C(R₆)(SL₁₄A₁₂R₇)₂ or -C(R₆)(SL₁₄A₁₂R₇)(OL₁₄A₁₂R₇). In some embodiments, R₁ and R₂ may further be each independently -C(R₆)(C(=O)OL₁₄A₁₂R₇)₂, -C(R₆)(OC(=O)L₁₄A₁₂R₇)₂, - C(R₆)(C(=O)OL₁₄A₁₂R₇)R₇, or -C(R₆)(OC(=O)L₁₄A₁₂R₇)R₇.

In some embodiments, in the amino lipid compound represented by formula (I-1), (I-3), (IV-1), (IV-3), (V-1), (V-3), (VI-1), or (VI-3), or a pharmaceutically acceptable salt thereof, or a stereoisomer thereof, R₁ is C₁-C₂₄ hydrocarbyl, C₁-C₂₄ heterohydrocarbyl, A₁₁R₅, or -C(R₆)(OL₁₄A₁₂R₇)₂, and R₂ is -C(R₆)(SL₁₄A₁₂R₇)₂ or -C(R₆)(SL₁₄A₁₂R₇)(OL₁₄A₁₂R₇). In some embodiments, R₂ may also be -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, the present disclosure provides the amino lipid compound represented by formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein A₁ is -N(R₄)-, -C(=O)O-, -OC(=O)-, -OC(=O)O-, -C(=O)S-, -SC(=O)-, -C(=O)N(R₄)-, - N(R₄)C(=O)-, -OC(=O)N(R₄)-, -N(R₄)C(=O)O-, -N(R₄)C(=O)N(R₄)-, -N(C(=O)R₄), -OC(=O)C(=O)O-, - N(R₄)C(=O)C(=O)O-, -OC(=O)C(=O)N(R₄)-, -N(R₄)C(=O)C(=O)N(R₄)-, 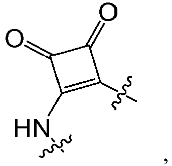 or 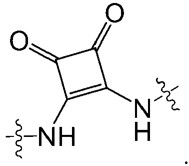 In some embodiments, A₁ is -N(R₄)-. In some embodiments, A₁ is -NH-.

In some embodiments, the present disclosure provides the amino lipid compound represented by formula (I-1), (I-2), (I-3), (I-4), (IV), (IV-1), (IV-2), (IV-3), (IV-4), (V), (V-1), (V-2), (V-3), or (V-4), or a pharmaceutically acceptable salt thereof, or a stereoisomer thereof, as described above, wherein A₂ is -N(R₄)-, -C(=O)O-, -OC(=O)-, -OC(=O)O-, -C(=O)S-, -SC(=O)-, -C(=O)N(R₄)-, -N(R₄)C(=O)-, -OC(=O)N(R₄)-, - N(R₄)C(=O)O-, -N(R₄)C(=O)N(R₄)-, -N(C(=O)R₄), -OC(=O)C(=O)O-, -N(R₄)C(=O)C(=O)O-, - OC(=O)C(=O)N(R₄)-, -N(R₄)C(=O)C(=O)N(R₄)-,

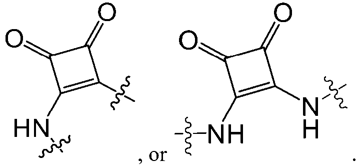

In some embodiments, A₂ is -N(R₄)-. In some embodiments, R₄ is H. In some embodiments, R₄ is methyl. In some embodiments, R₄ is C₂ alkyl, or C₃ or C₄ alkyl, or C₅ or C₆ alkyl.

In some embodiments, A₂ is -C(=O)N(R₄)-, -N(R₄)C(=O)-, -OC(=O)O-, -OC(=O)N(R₄)-, - N(R₄)C(=O)O-, -C(=O)O-, -OC(=O)-, -C(=O)S-, -SC(=O)-, -OC(=O)C(=O)O-, -N(R₄)C(=O)C(=O)O-, - OC(=O)C(=O)N(R₄)-, -N(R₄)C(=O)C(=O)N(R₄)-, 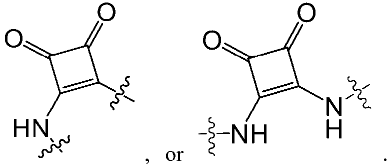 In some embodiments, R₄ is H.

In some embodiments, the present disclosure provides the amino lipid compound of formula (I), (IA), (I-1), (I-2), (I-3), (I-4), (IV), (IV-1), (IV-2), (IV-3), (IV-4), (V-1), (V-2), (V-3), (V-4), or (V-7), or a pharmaceutically acceptable salt thereof, or a stereoisomer thereof, as described above, wherein A₂ is - C(=O)N(R₄)- or -N(R₄)C(=O)-.

In some embodiments, A₂ is -C(=O)N(H)- or -N(H)C(=O)-.

In some embodiments, the present disclosure provides the amino lipid compound represented by formula (I-1), (I-2), (I-3), (I-4), (IV), (IV-1), (IV-2), (IV-3), or (IV-4), or a pharmaceutically acceptable salt thereof, or a stereoisomer thereof, as described above, wherein A₃ is -N(R₄)-, -C(=O)O-, -OC(=O)-, -OC(=O)O-, - C(=O)S-, -SC(=O)-, -C(=O)N(R₄)-, -N(R₄)C(=O)-, -OC(=O)N(R₄)-, -N(R₄)C(=O)O-, -N(R₄)C(=O)N(R₄)-, - N(C(=O)R₄), -OC(=O)C(=O)O-, -N(R₄)C(=O)C(=O)O-, -OC(=O)C(=O)N(R₄)-, -N(R₄)C(=O)C(=O)N(R₄)-,

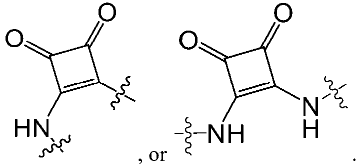

In some embodiments, A₃ is -N(R₄)-, -C(=O)N(R₄)-, -N(R₄)C(=O)-, -C(=O)O-, -OC(=O)-, 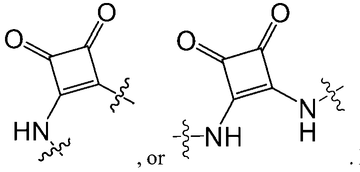 In some embodiments, R₄ is H. In some embodiments, R₄ is methyl. In some embodiments, R₄ is C₂ alkyl, or C₃ or C₄ alkyl, or C₅ or C₆ alkyl.

In some embodiments, the present disclosure provides the amino lipid compound represented by formula (I-1), (I-2), (I-3), or (I-4), or a pharmaceutically acceptable salt thereof, or a stereoisomer thereof, as described above, wherein A₄ is -N(R₄)-, -C(=O)O-, -OC(=O)-, -OC(=O)O-, -C(=O)S-, -SC(=O)-, -C(=O)N(R₄)-, - N(R₄)C(=O)-, -OC(=O)N(R₄)-, -N(R₄)C(=O)O-, -N(R₄)C(=O)N(R₄)-, -N(C(=O)R₄), -OC(=O)C(=O)O-, - N(R₄)C(=O)C(=O)O-, -OC(=O)C(=O)N(R₄)-, -N(R₄)C(=O)C(=O)N(R₄)-, 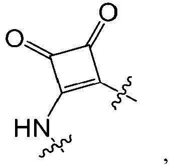 or

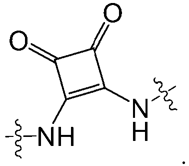

In some embodiments, A₄ is -N(R₄)-, -C(=O)N(R₄)-, -N(R₄)C(=O)-, 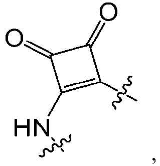 or 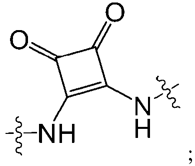 in some embodiments, R₄ is H. In some embodiments, R₄ is methyl. In some embodiments, R₄ is C₂ alkyl, or C₃ or C₄ alkyl, or C₅ or C₆ alkyl.

In some embodiments, the present disclosure provides the amino lipid compound represented by formula (I-1), (I-2), (I-3), or (I-4), or a pharmaceutically acceptable salt thereof, or a stereoisomer thereof, as described above, wherein A₄ is -N(R₄)-, -C(=O)O-, -OC(=O)-, -OC(=O)O-, -C(=O)S-, -SC(=O)-, -C(=O)N(R₄)-, - N(R₄)C(=O)-, -OC(=O)N(R₄)-, -N(R₄)C(=O)O-, -N(R₄)C(=O)N(R₄)-, -N(C(=O)R₄), -OC(=O)C(=O)O-, - N(R₄)C(=O)C(=O)O-, -OC(=O)C(=O)N(R₄)-, -N(R₄)C(=O)C(=O)N(R₄)-, 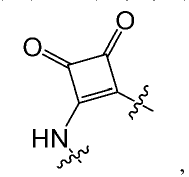 or

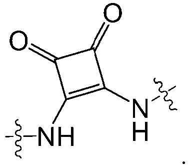

In some embodiments, the present disclosure provides the amino lipid compound represented by formula (I-1), (I-2), (I-3), or (I-4), or a pharmaceutically acceptable salt thereof, or a stereoisomer thereof, as described above, wherein A₅ is -N(R₄)-, -C(=O)N(R₄)-, -N(R₄)C(=O)-, 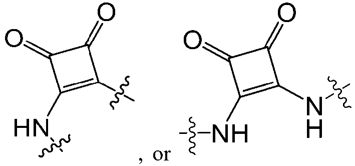 . In some embodiments, R₄ is H. In some embodiments, R₄ is methyl. In some embodiments, R₄ is C₂ alkyl, or C₃ or C₄ alkyl, or C₅ or C₆ alkyl.

In some embodiments, the present disclosure provides the amino lipid compound represented by formula (I-1), (I-2), (I-3), or (I-4), or a pharmaceutically acceptable salt thereof, or a stereoisomer thereof, as described above, wherein A₆ is -N(R₄)-, -C(=O)O-, -OC(=O)-, -OC(=O)O-, -C(=O)S-, -SC(=O)-, -C(=O)N(R₄)-, - N(R₄)C(=O)-, -OC(=O)N(R₄)-, -N(R₄)C(=O)O-, -N(R₄)C(=O)N(R₄)-, -N(C(=O)R₄), -OC(=O)C(=O)O-, - N(R₄)C(=O)C(=O)O-, -OC(=O)C(=O)N(R₄)-, -N(R₄)C(=O)C(=O)N(R₄)-, 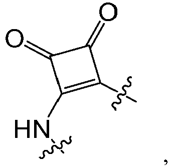 or

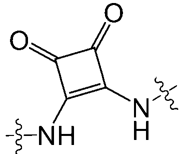

In some embodiments, A₆ is -N(R₄)-, -C(=O)N(R₄)-, -N(R₄)C(=O)-, 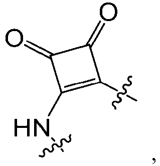 or 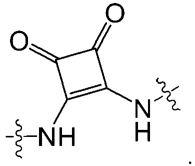 In some embodiments, R₄ is H. In some embodiments, R₄ is methyl. In some embodiments, R₄ is C₂ alkyl, or C₃ or C₄ alkyl, or C₅ or C₆ alkyl.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein L₁ is C₁-C₆ alkylene.

In some embodiments, L₁ is C₄, C₅, or C₆ alkylene.

In some embodiments, L₁ is C₁, C₂, C₃, or C₄ alkylene.

In some embodiments, L₁ is C₁, C₂, or C₃ alkylene. In some other embodiments, L₁ is C₄ alkylene.

In some embodiments, L₁ is a bond.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), or (V-4), or a pharmaceutically acceptable salt thereof, or a stereoisomer thereof, as described above, wherein L₂ is C₁-C₆ alkylene or a bond.

In some embodiments, L₂ is C₅ or C₆ alkylene.

In some embodiments, L₂ is C₁, C₂, C₃, or C₄ alkylene.

In some embodiments, L₂ is a bond.

In some embodiments, the present disclosure provides the amino lipid compound of formula (I-1), (I-2), (I-3), (I-4), (IV), (IV-1), (IV-2), (IV-3), or (IV-4), or a pharmaceutically acceptable salt thereof, or a stereoisomer thereof, as described above, wherein L₃ is C₁-C₆ alkylene or a bond.

In some embodiments, L₃ is C₄, C₅, or C₆ alkylene.

In some embodiments, L₃ is C₂ or C₃ alkylene.

In some embodiments, L₃ is C₁ alkylene or a bond.

In some embodiments, the present disclosure provides the amino lipid compound of formula (I-1), (I-2), (I-3), or (I-4), or a pharmaceutically acceptable salt thereof, or a stereoisomer thereof, as described above, wherein L₄ is a bond. In some other embodiments, L₄ is C₄, C₅, or C₆ alkylene. In some other embodiments, L₄ is C₂ or C₃ alkylene. In some other embodiments, L₄ is C₁ alkylene or a bond.

In some embodiments, the present disclosure provides the amino lipid compound of formula (I-1), (I-2), (I-3), or (I-4), or a pharmaceutically acceptable salt thereof, or a stereoisomer thereof, as described above, wherein L₅ is a bond. In some other embodiments, L₅ is C₄, C₅, or C₆ alkylene. In some other embodiments, L₅ is C₂ or C₃ alkylene. In some other embodiments, L₅ is C₁ alkylene or a bond.

In some embodiments, the present disclosure provides the amino lipid compound of formula (I-1), (I-2), (I-3), or (I-4), or a pharmaceutically acceptable salt thereof, or a stereoisomer thereof, as described above, wherein L₆ is a bond. In some other embodiments, L₆ is C₄, C₅, or C₆ alkylene. In some other embodiments, L₆ is C₂ or C₃ alkylene. In some other embodiments, L₆ is C₁ alkylene or a bond.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein L₁, L₂, L₃, L₄, L₅, or L₆ is selected from:

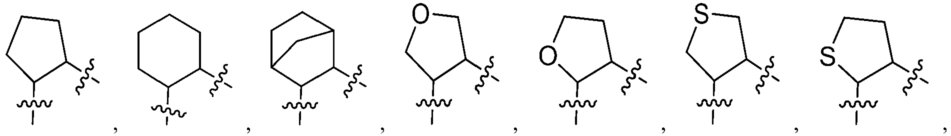

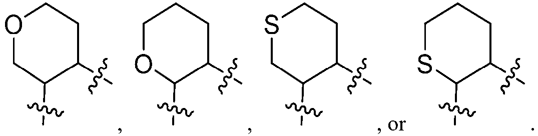

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein R₄ may be attached to a C atom on L₁, L₂, L₃, L₄, L₅, or L₆ to form a three-, four-, five-, six-, seven-, eight-, nine-, or ten-membered monocyclic or polycyclic N atom-containing heterocyclic ring.

In some embodiments, R₄ may be attached to a C atom on L₁, L₂, L₃, L₄, L₅, or L₆ to form a three-, four-, five-, or six-membered N atom-containing heterocyclic ring.

In some embodiments, one or more of -A₁-L₁-, -A₂-L₂-, -A₃-L₃-, -A₄-L₄-, -A₅-L₅-, or -A₆-L₆- is

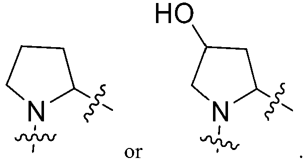

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein L₇ is C₁, C₂, C₃, C₄, C₅, C₆, C₇, C₈, C₉, C₁₀, C₁₁, C₁₂, C₁₃, or C₁₄ alkylene, or C₁, C₂, C₃, C₄, C₅, C₆, C₇, C₈, C₉, C₁₀, C₁₁, C₁₂, C₁₃, or C₁₄ heteroalkylene containing O, N, or S, or a bond. In some other embodiments, L₇ is C₄-C₁₁ alkylene. In some embodiments, L₇ is C₋C₉ alkylene. In some other embodiments, L₇ is C₇ or C₈ alkylene. In some embodiments, L₇ is a bond. In some embodiments, L₇ is C₁-C₄ alkylene.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein L₈ is C₁, C₂, C₃, C₄, C₅, C₆, C₇, C₈, C₉, C₁₀, C₁₁, C₁₂, C₁₃, or C₁₄ alkylene, or C₁, C₂, C₃, C₄, C₅, C₆, C₇, C₈, C₉, C₁₀, C₁₁, C₁₂, C₁₃, or C₁₄ heteroalkylene containing O, N, or S, or a bond. In some embodiments, L₈ is C₄-C₁₁ alkylene. In some embodiments, L₈ is C₆-C₉ alkylene. In some embodiments, L₈ is C₇ or C₈ alkylene. In some embodiments, L₈ is a bond. In some embodiments, L₈ is C₁-C₄ alkylene.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein A₇ and A₈ are each independently -C(=O)O-, -OC(=O)-, -OC(=O)O-, -C(=O)N(R₄)-, or - N(R₄)C(=O)-.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein L₉ is C₁, C₂, C₃, C₄, C₈, C₆, C₇, or C₈ alkylene, or a bond. In some embodiments, L₉ is C₃-C₆ alkylene. In some embodiments, L₉ is C₂ alkylene. In some embodiments, L₉ is C₃ alkylene. In some embodiments, L₉ is C₅ or C₆ alkylene. In some embodiments, L₉ is a bond.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein L₁₀ is C₁, C₂, C₃, C₄, C₅, C₆, C₇, or C₈ alkylene, or a bond. In some embodiments, L₁₀ is C₃-C₆ alkylene. In some embodiments, L₁₀ is C₂ alkylene. In some embodiments, L₁₀ is C₃ alkylene. In some embodiments, L₁₀ is C₅ or C₆ alkylene. In some embodiments, L₁₀ is a bond.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein A₉ and A₁₀ are each independently -C(=O)O-, -OC(=O)-, -OC(=O)O-, -C(=O)N(R₄)-, or - N(R₄)C(=O)-.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein L₁₁ is C₁, C₂, C₃, C₄, C₅, C₆, C₇, or C₈ alkylene, or a bond. In some embodiments, L ₁₁ is C₂ alkylene. In some embodiments, L₁₁ is C₃-C₆ alkylene. In some embodiments, L₁₁ is C₃ alkylene. In some embodiments, L₁₁ is C₅ or C₆ alkylene. In some embodiments, L₁₁ is a bond.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein L₁₂ is C₁, C₂, C₃, C₄, C₅, C₆, C₇, or C₈ alkylene, or a bond. In some embodiments, L₁₂ is C₂ alkylene. In some embodiments, L₁₂ is C₃-C₆ alkylene. In some embodiments, L₁₂ is C₃ alkylene. In some embodiments, L₁₂ is C₅ or C₆ alkylene. In some embodiments, L₁₂ is a bond.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein R₁ and R₂ are each independently a straight or branched C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl. In some embodiments, R₁ and R₂ are each independently a straight or branched C₈-C₂₀ alkyl. In some embodiments, R₁ and R₂ are each independently a straight or branched C₉-C₁₁ alkyl. In some embodiments, R₁ and R₂ are each independently a straight or branched C₁₅-C₁₉ alkyl.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein R₁ and R₂ are each independently -C(R₆)(OL₁₄A₁₂R₇)₂, -C(R₆)(SL₁₄A₁₂R₇)₂, or - C(R₆)(SL₁₄A₁₂R₇)(OL₁₄A₁₂R₇).

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (V-5), (V-6), (VI), (VI-1), (VI-2), (VI-3), (VI-4), or (VI-5), or a pharmaceutically acceptable salt thereof, or a stereoisomer thereof, as described above, wherein R₁ and R₂ are each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein R₁ and R₂ are each independently -C(R₆)(OR₇)₂, -C(R₆)(SR₇)₂, -C(R₆)(SR₇)(OR₇), - C(R₆)(C(=O)OR₇)₂, -C(R₆)(OC(=O)R₇)₂, -C(R₆)(C(=O)OR₇)R₇, or -C(R₆)(OC(=O)₂R₇)R₇.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (V-5), (V-5-1), (V-6), (VI), (VI-1), (VI-2), (VI-3), (VI-4), or (VI-5), or a pharmaceutically acceptable salt thereof, or a stereoisomer thereof, as described above, wherein each R₆ is independently C₁, C₂, C₃, C₄, C₈, or C₆ alkyl, or H. In some further embodiments, each R₆ is independently C₁-C₃ alkyl. In some embodiments, each R₆ is H.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (V-5), (V-5-1), (V-6), (VI), (VI-1), (VI-2), (VI-3), (VI-4), or (VI-5), or a pharmaceutically acceptable salt thereof, or a stereoisomer thereof, as described above, wherein each L₁₄ is independently C₁, C₂, C₃, C₄, C₅, C₆, C₇, or C₈ alkylene, or a bond. In some embodiments, each L₁₄ is independently C₁-C₄ alkylene, or a bond. In some embodiments, each L₁₄ is independently C₁-C₃ alkylene, or a bond.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (V-5), (V-5-1), (V-6), (VI), (VI-1), (VI-2), (VI-3), (VI-4), or (VI-5), or a pharmaceutically acceptable salt thereof, or a stereoisomer thereof, as described above, wherein each A₁₂ is independently -C(=O)O-, -OC(=O)-, -OC(=O)O-, - C(=O)N(R₄)-, or -N(R₄)C(=O)-.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein each R₇ is independently C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, or C₂₄ alkyl, alkenyl, or alkynyl, or H. In some embodiments, each R₇ is independently C₁-C₄ alkyl, alkenyl, or alkynyl, or H. In some embodiments, each R₇ is independently C₂ or C₃ alkyl, alkenyl, or alkynyl. In some embodiments, each R₇ is independently C₅-C₁₀ alkyl, alkenyl, or alkynyl. In some embodiments, each R₇ is independently C₁₁-C₂₁ alkyl, alkenyl, or alkynyl. In some embodiments, each R₇ is independently C₁₆-C₁₈ alkyl, alkenyl, or alkynyl. In some embodiments, each R₇ is independently selected from:

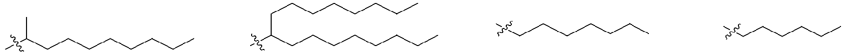

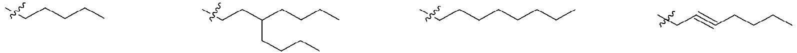

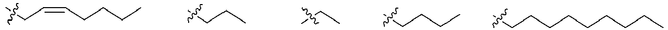

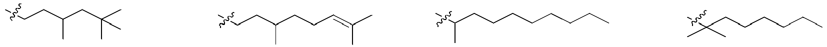

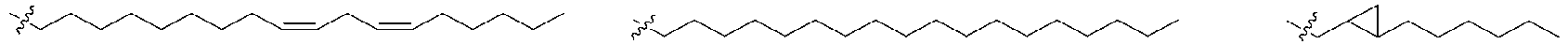

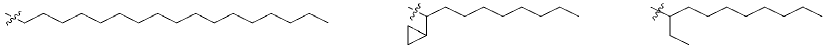

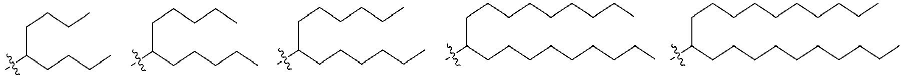

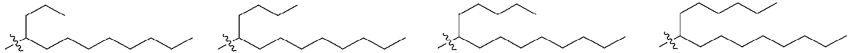

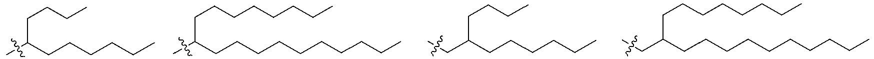

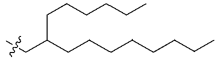

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (V-5), (V-5-1), (V-6), (VI), (VI-1), (VI-2), (VI-3), (VI-4), or (VI-5), or a pharmaceutically acceptable salt thereof, or a stereoisomer thereof, as described above, wherein R₁ and R₂ are each independently selected from:

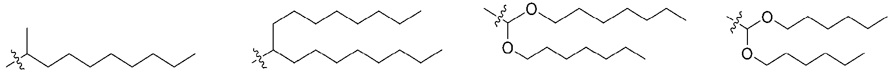

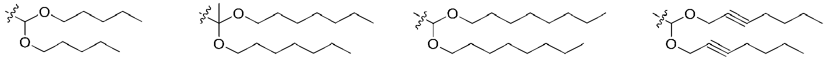

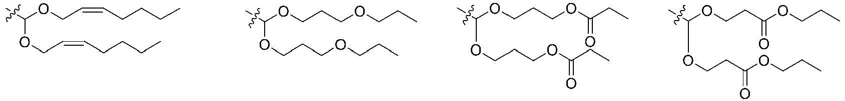

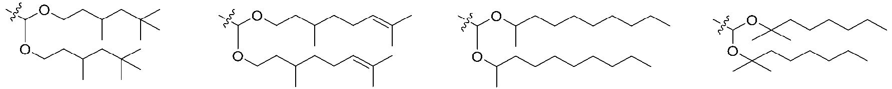

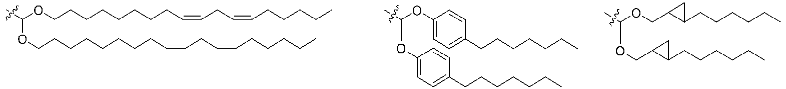

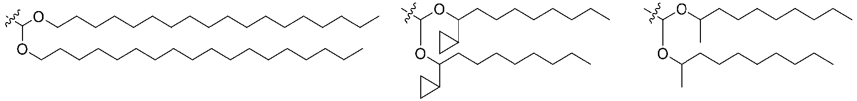

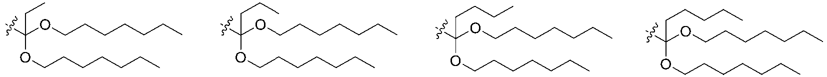

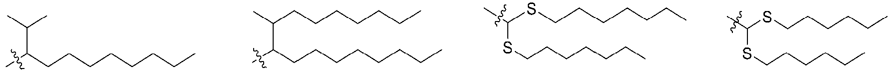

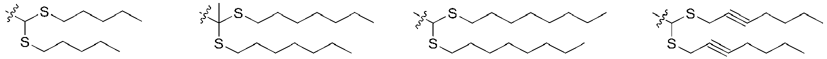

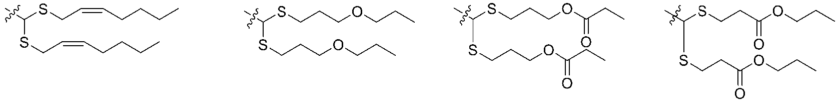

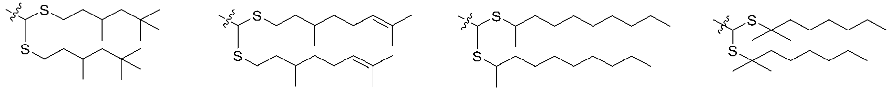

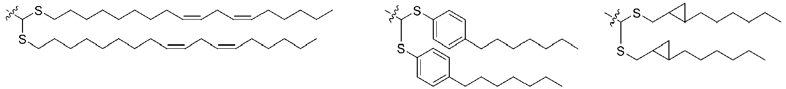

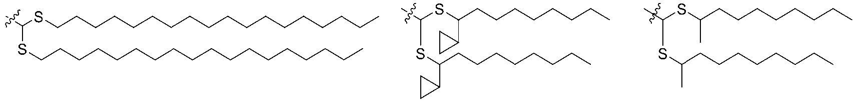

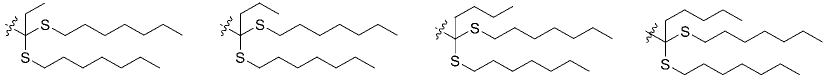

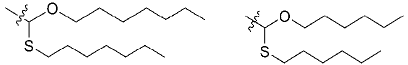

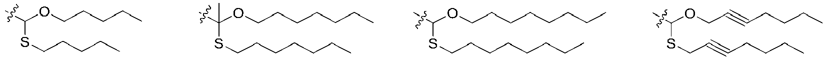

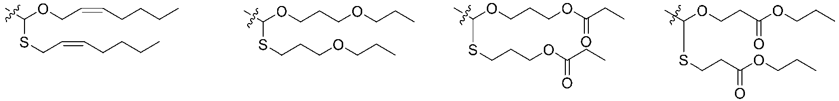

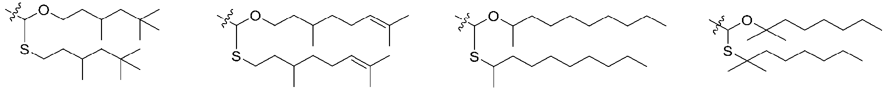

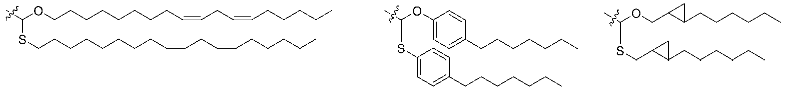

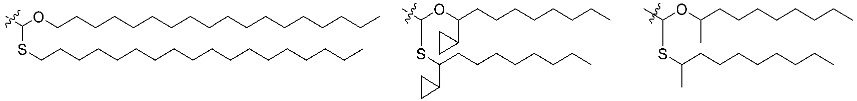

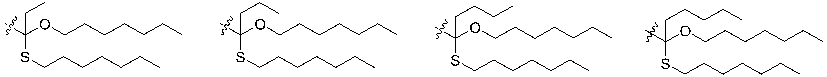

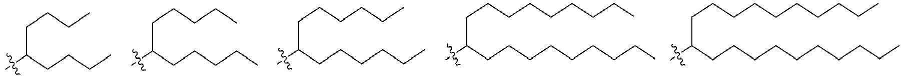

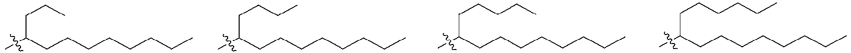

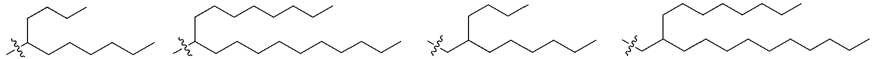

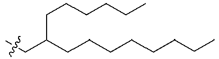

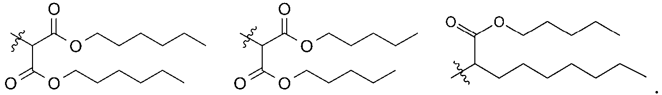

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein each R₁₁ is independently C₃₋₆ carbocyclic ring.

In some embodiments, each R₁₁ is independently C₃, C₄, C₈, or C₆ cycloalkyl, or C₃, C₄, C₅, or C₆ cycloalkenyl.

In some embodiments, each R₁₁ is independently C₃-C₆ cycloalkyl, e.g., cyclopropyl, cyclobutyl, cyclopentyl, or cyclohexyl.

In some embodiments, each R₁₁ is independently C₃₋₆ cycloalkyl optionally substituted with a substituent such as -OH, halogen, or C₁₋₆ alkyl, for example, R₁₁ is cyclohexyl substituted with -OH, e.g., 2-hydroxycyclohexyl.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein each R₁₁ is independently a heterocyclic ring.

In some embodiments, each R₁₁ is independently a five-, six-, seven-, or eight-membered heterocyclic ring.

In some embodiments, each R₁₁ is independently a five-, six-, seven-, or eight-membered heterocyclic ring containing N, O, or S.

In some embodiments, each R₁₁ is independently a five- or six-membered heterocyclic ring containing N, O, or S.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein each R₁₂ is independently C₁, C₂, C₃, C₄, C₅, or C₆ alkyl.

In some embodiments, each R ₁₂ is independently C₁-C₃ alkyl.

In some embodiments, each R₁₂ is independently C₄-C₆ alkyl.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein each R₁₂ is independently C₂, C₃, C₄, C₅, or C₆ alkenyl.

In some embodiments, each R ₁₂ is independently C₂ or C₃ alkenyl.

In some embodiments, each R₁₂ is independently C₄-C₆ alkenyl.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein each R₁₂ is independently C₃₋₆ carbocyclic ring.

In some embodiments, each R₁₂ is independently C₃, C₄, C₅, or C₆ cycloalkyl, or C₃, C₄, C₅, or C₆ cycloalkenyl.

In some embodiments, each R₁₂ is independently C₃-C₆ cycloalkyl, e.g., cyclopropyl, cyclobutyl, cyclopentyl, or cyclohexyl.

In some embodiments, each R₁₂ is independently C₃₋₆ cycloalkyl optionally substituted with a substituent such as -OH, halogen, or C₁₋₆ alkyl, for example, R₁₁ is cyclohexyl substituted with -OH, e.g., 2-hydroxycyclohexyl.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein each R₁₂ is independently a heterocyclic ring.

In some embodiments, each R₁₂ is independently a five-, six-, seven-, or eight-membered heterocyclic ring.

In some embodiments, each R₁₂ is independently a five-, six-, seven-, or eight-membered heterocyclic ring containing N, O, or S.

In some embodiments, each R₁₂ is independently a five- or six-membered heterocyclic ring containing N, O, or S.

In some embodiments, the present disclosure provides the amino lipid compound of formula (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein each R₁₂ is independently H, CN, NO₂, -OR, -S(O)₂R, or -S(O)₂N(R)₂.

In some embodiments, the present disclosure provides the amino lipid compound of formula (IA), (I-1), (I-2), (I-3), (I-4), (IV), (IV-1), (IV-2), (IV-3), (IV-4), (V), (V-1), (V-2), (V-3), (V-4), (VI), (VI-1), (VI-2), (VI-3), or (VI-4), or a pharmaceutically acceptable salt thereof, or a stereoisomer thereof, as described above, wherein -A₁-L₁-A₂-, -A₂-L₂-A₃-, -A₃-L₃-A₄-, -A₄-L₄-A₅-, or -A₅-L₅-A₆- is -K-Q-M- or -M-Q-K-, wherein M is -O-, -S-, -N(R₄)-, or a bond, K is -OC(=O)-, -C(=O)-, -N(R₄)C(=O)-, -SC(=O)-, or a bond, and Q is a group obtained from the loss of -OH from one carboxyl and of one -H from one amino of an amino acid, wherein the moiety of the amino acid from which -OH is lost is attached to M, and the amino acid is a compound containing an amino (-NH₂ or -NH-) and a carboxyl (-COOH) in the structure, including naturally or non-naturally occurring ones.

For example:
Q is a group obtained from the loss of -OH from an carboxyl and one H from an amino of α-alanine: 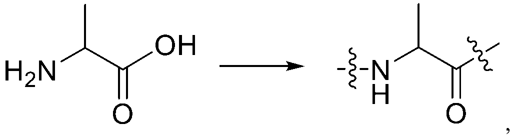 wherein the moiety of the α-alanine from which -OH is lost is attached to M: 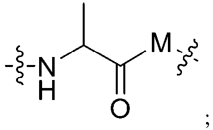 when M is -O-, -Q-M- is 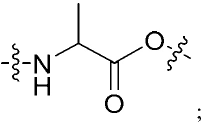 when M is -S-, -Q-M- is 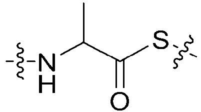 ; when M is -N(R₄)-, -Q-M- is 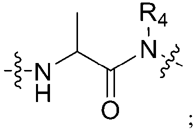 when M is a bond, -Q-M- is 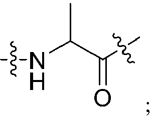 in the case where -Q-M- is 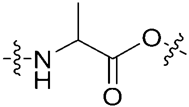 , when K is a bond, -K-Q-M- is 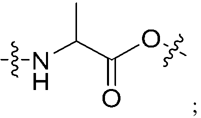 when K is -OC(=O)-, -K-Q-M- is 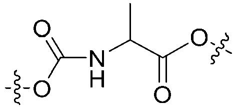 ; when K is -C(=O)-, -K-Q-M- is 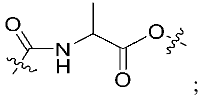 when K is -N(R₄)C(=O)-, -K-Q-M- is 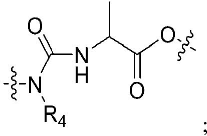 and when K is - SC(=O)-, -K-Q-M- is

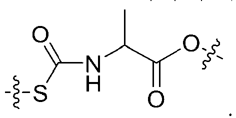

In some embodiments, in -K-Q-M- or -M-Q-K-, -NH- obtained from the loss of one -H from an amino of an amino acid may be further substituted with R₄. For example:
in 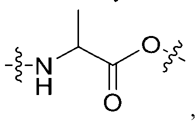 -NH- obtained from the loss of one -H from an amino of the α-alanine may be further substituted with R₄ into

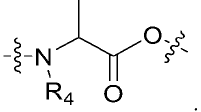

In some embodiments, the amino acid has a structure represented by formula (A) or (B):

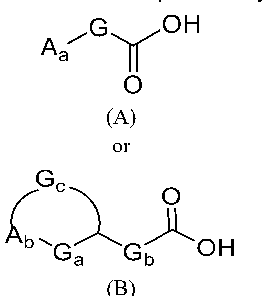

In formula (A):
Aₐ is -NH₂;
G is a straight C₁-C₆ hydrocarbylene optionally substituted with one or more substituents which include H, -OH, -SH, halogen, O, S, N, -CN, C₃-C₈ cyclohydrocarbyl, aryl, heterocyclic ring, -R_{b}, -RₐN(R_{b})₂, -RₐOR_{b}, -RₐSR_{b}, -RₐOC(=O)OR_{b}, -RₐOC(=O)SR_{b}, -RₐC(=O)N(R_{b})₂, -OC(=O)OR_{b}, -OC(=O)SR_{b}, -C(=O)N(R_{b})₂, - RₐOC(=O)R_{b}, -RₐC(=O)OR_{b}, -OC(=O)R_{b}, -C(=O)OR_{b}, -RₐSR_{b}, or

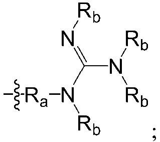

In formula (B):
A_{b} is -NH-;
Gₐ and G_{b} are each independently a straight C₁-C₅ hydrocarbylene or a bond, and the total number of the C atoms of Gₐ and G_{b} is at most 5;
G_{c} is C₁-C₆ hydrocarbylene optionally substituted with one or more substituents which include, but are not limited to, H, -OH, -SH, halogen, O, S, N, -CN, -R_{b}, -RₐN(R_{b})₂, -RₐOR_{b}, -RₐSR_{b}, or -RₐSR_{b};
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds.

In some embodiments, in the amino acid of formula (A), G is C₁, C₂, C₃, C₄, C₅, or C₆ alkyl, or C₂, C₃, C₄, C₈, or C₆ alkenyl, or C₃, C₄, C₅, or C₆ alkynyl. In some embodiments, G is C₁, C₂, C₃, C₄, C₅, or C₆ alkyl. In some other embodiments, G is C₁, C₂, or C₃ alkyl.

In some embodiments, in the amino acid of formula (A), when G is substituted with one or more of C₃-C₈ cyclohydrocarbyl, aryl, heterocyclic ring, -R_{b}, -RₐN(R_{b})₂, -RₐOR_{b}, -RₐSR_{b}, -RₐOC(=O)OR_{b}, - RₐOC(=O)SR_{b}, -RₐC(=O)N(R_{b})₂, -OC(=O)OR_{d}, -OC(=O)SR_{b}, -C(=O)N(R_{b})₂, -RₐOC(=O)R_{b,} -RₐC(=O)OR_{b}, -OC(=O)R_{b}, -C(=O)OR_{b}, -RₐSR_{b}, or 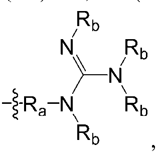 Rₐ and/or R_{b} may be attached to a C atom on G to form a monocyclic, bicyclic, or polycyclic structure. For example, G is substituted with n-propyl which is attached to a C atom on G to form a five-membered monocyclic structure, e.g., 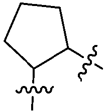 ; G is substituted with n-butyl which is attached to a C atom on G to form a six-membered monocyclic structure, e.g., 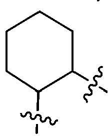 ; or G is substituted with cyclopentyl which is attached to a C atom on G to form a seven-membered bicyclic structure, e.g.,

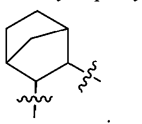

In some embodiments, in the amino acid of formula (A), G is optionally substituted with one or more substituents which include H, -OH, -(CH₂)₄-NH₂, -CH₂-CH(CH₃)₂, -(CH₂)₂-S-CH₃, -CH₂-(C=O)-NH₂, - (CH₂)₃-NH-C(=NH)-NH₂, -CH₂-OH, -CH(OH)-CH₃, -CH₃, -CH₂-SH, -CH₂-COOH, -(CH₂)₂-COOH, - (CH₂)₂-COOH, -CH₂-C₅H₆, 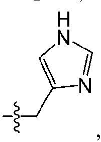 -CH(CH₃)-CH₂-CH₃, -CH(CH₃)₂, 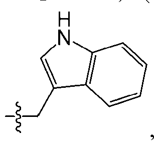 -CH₂-C₅H₅-OH, -CH₂-SeH, 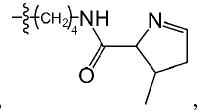 -CH₂-S-S-CH₂-CH(NH₂)-COOH, -(CH₂)₃-NH₂, or -(CH₂)₃-NH-C(=O)-NH₂.

In some embodiments, in the amino acid of formula (B), Gₐ and G_{b} are each independently C₁, C₂, C₃, C₄, or C₅ alkyl, or C₂, C₃, C₄, or C₅ alkenyl, or C₃, C₄, or C₅ alkynyl. In some embodiments, G is C₁, C₂, C₃, C₄, or C₅ alkyl. In some other embodiments, G is C₁, C₂, or C₃ alkyl.

In some embodiments, in the amino acid of formula (B), Gₐ and G_{b} are each independently a bond.

In some embodiments, in the amino acid of formula (B), G_{c} is C₁, C₂, C₃, C₄, C₅, or C₆ hydrocarbylene optionally substituted with one or more substituents. In some embodiments, G_{c} is C₁, C₂, C₃, C₄, C₅, or C₆ alkylene optionally substituted with one or more substituents. In some embodiments, G_{c} is C₂, C₃, C₄, C₅, or C₆ alkenylene with 1, 2, 3, or more double bonds, optionally substituted with one or more substituents. In some embodiments, G_{c} is C₂, C₃, C₄, C₅, or C₆ alkynylene alkenylene with 1, 2, 3, or more triple bonds, optionally substituted with one or more substituents.

In some embodiments, G_{c} is C₁, C₂, C₃, C₄, or C₅ alkylene optionally substituted with one or more substituents. In some embodiments, G_{c} is C₃, C₄, or C₅ alkylene optionally substituted with one or more substituents. In some embodiments, G_{c} is C₁, C₂, or C₃ alkylene optionally substituted with one or more substituents, and further is C₁ or C₂ alkylene optionally substituted with one or more substituents.

In some embodiments, in the amino acid of formula (B), G_{c} is substituted with one or more substituents which include H, -OH, -(CH₂)₄-NH₂, -CH₂-CH(CH₃)₂, -(CH₂)₂-S-CH₃, -CH₂-(C=O)-NH₂, -(CH₂)₃-NH-C(=NH)-NH₂, -CH₂-OH, -CH(OH)-CH₃, -CH₃, -CH₂-SH, -CH₂-COOH, -(CH₂)₂-COOH, -(CH₂)₂-COOH, - CH₂-C₅H₆, 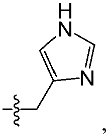 -CH(CH₃)-CH₂-CH₃, -CH(CH₃)₂, 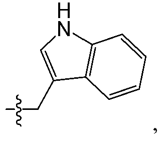 -CH₂-C₅H₅-OH, -CH₂-SeH, 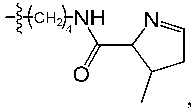 -CH₂-S-S-CH₂-CH(NH₂)-COOH, -(CH₂)₃-NH₂, or -(CH₂)₃-NH-C(=O)-NH₂.

In some embodiments, in the amino acid of formula (B), 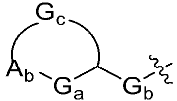 is selected from:

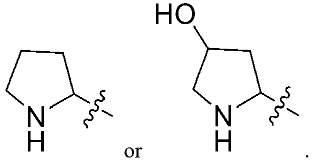

In some embodiments, suitable amino acids include, but are limited to, Glycine, Alanine, Valine, Leucine, Isoleucine, Phenylalanine, Tryptophan, Tyrosine, Aspartate, Histidine, Asparagine, Glutamate, Lysine, Glutamine, Methionine, Arginine, Serine, Threonine, Cysteine, Proline, Selenocysteine, Pyrrolysine, Cystine, Hydroxyproline, Ornithine, and citrulline.

In some embodiments, Q is selected from:

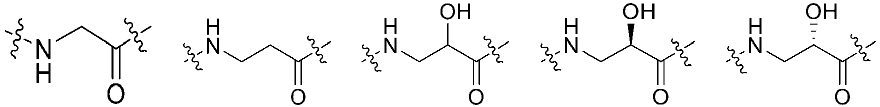

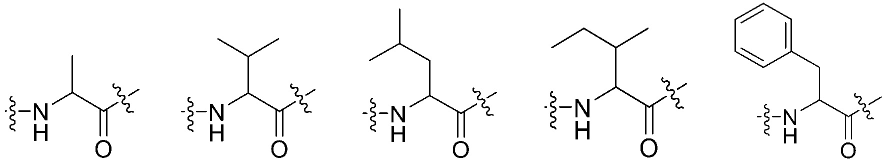

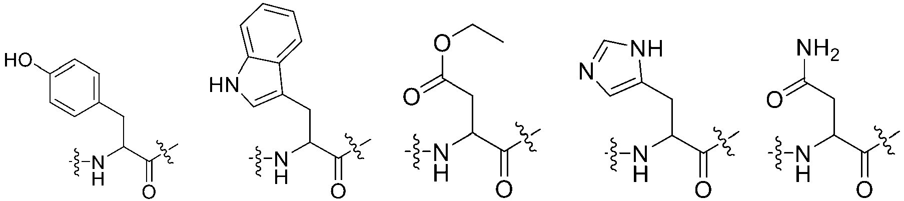

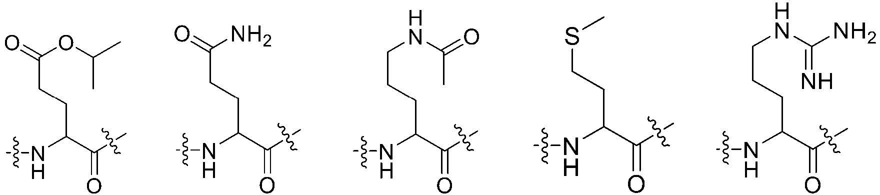

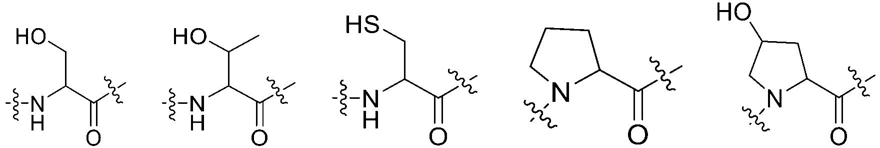

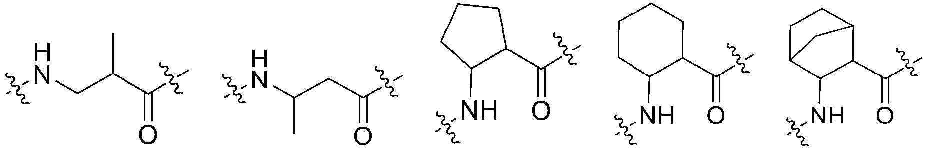

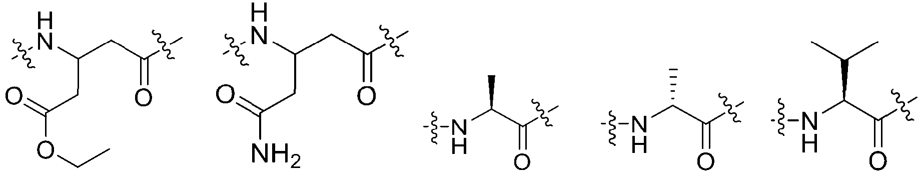

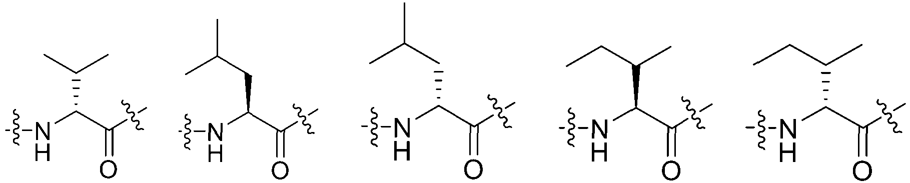

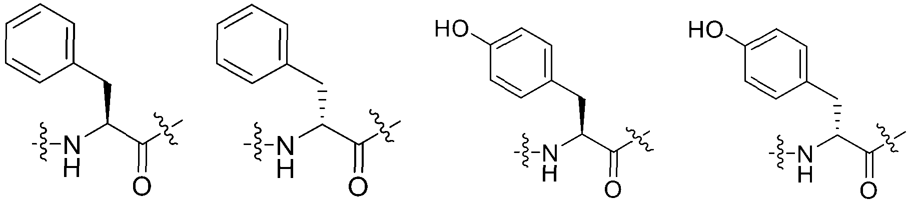

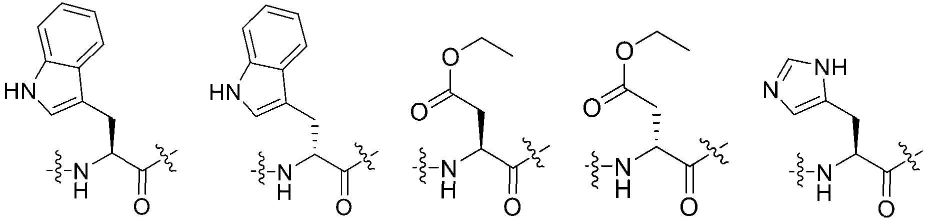

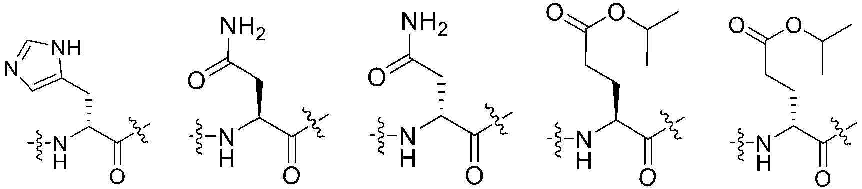

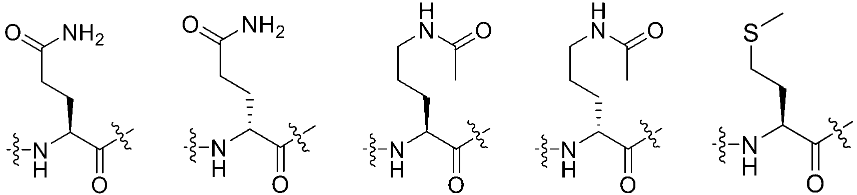

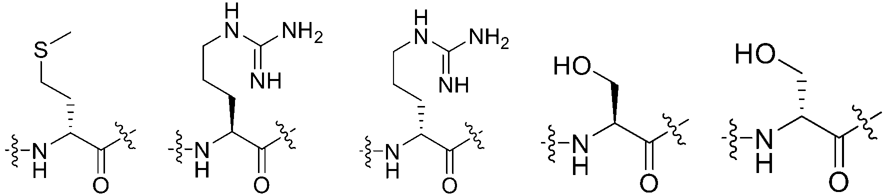

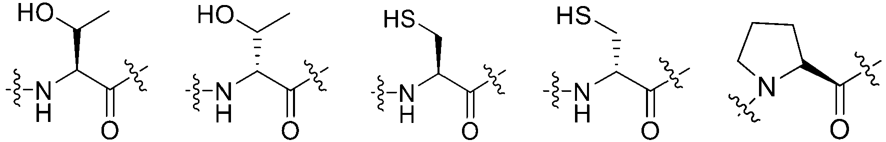

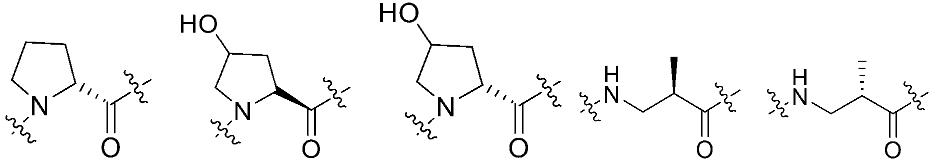

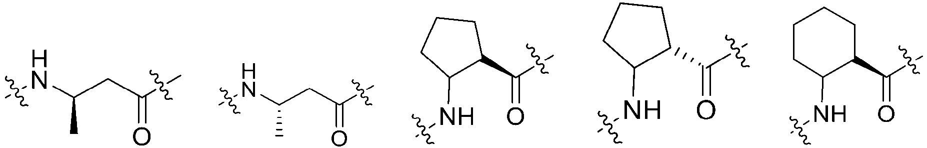

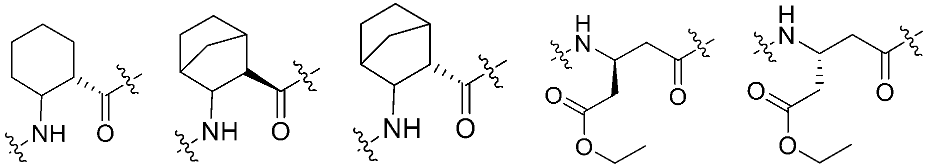

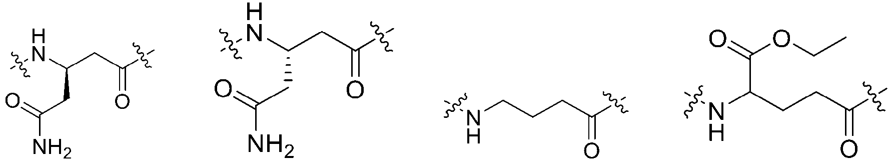

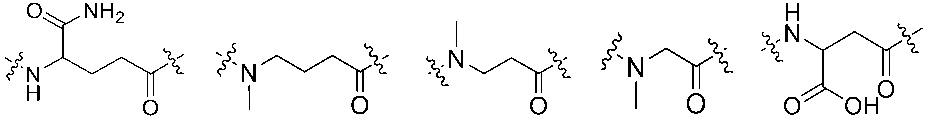

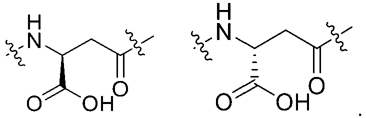

In some embodiments, the present disclosure provides the amino lipid compound of formula (I), (I-1), (I-2), (I-3), (I-4), (IV), (IV-1), (IV-2), (IV-3), (IV-4), (V), (V-1), (V-2), (V-3), (V-4), (V-5), (V-5-1), (V-6), (VI), (VI-1), (VI-2), (VI-3), (VI-4), (VI-5), (VII-1), (VII-1-1), (VII-2), (VII-2-1), (VII-3), or (VII-3-1), or a pharmaceutically acceptable salt thereof, or a stereoisomer thereof, as described above, wherein the hydrocarbyl, hydrocarbylene, alkyl, alkylene, alkenyl, alkenylene, alkynyl, alkynylene, heterohydrocarbyl, heterohydrocarbylene, carbocyclic ring, cyclohydrocarbyl, or heterocyclic ring is optionally substituted.

In some embodiments, the present disclosure provides the amino lipid compound of formula (I), (I-1), (I-2), (I-3), (I-4), (IV), (IV-1), (IV-2), (IV-3), (IV-4), (V), (V-1), (V-2), (V-3), (V-4), (V-5), (V-5-1), (V-6), (VI), (VI-1), (VI-2), (VI-3), (VI-4), (VI-5), (VII-1), (VII-1-1), (VII-2), (VII-2-1), (VII-3), or (VII-3-1), or a pharmaceutically acceptable salt thereof, or a stereoisomer thereof, as described above, wherein the hydrocarbyl, hydrocarbylene, alkyl, alkylene, alkenyl, alkenylene, alkynyl, alkynylene, heterohydrocarbyl, heterohydrocarbylene, carbocyclic ring, cyclohydrocarbyl, or heterocyclic ring is unsubstituted.

In some embodiments, the present disclosure provides the amino lipid compound of formula (I), (I-1), (I-2), (I-3), (I-4), (IV), (IV-1), (IV-2), (IV-3), (IV-4), (V), (V-1), (V-2), (V-3), (V-4), (V-5), (V-5-1), (V-6), (VI), (VI-1), (VI-2), (VI-3), (VI-4), (VI-5), (V-7), (V-7-1), (V-7-2), (V-7-3), (VI-6), (VI-6-1), (IV-5), or (IV-5-1), or a pharmaceutically acceptable salt thereof, or a stereoisomer thereof, as described above, wherein the hydrocarbyl, hydrocarbylene, alkyl, alkylene, alkenyl, alkenylene, alkynyl, alkynylene, heterohydrocarbyl, heterohydrocarbylene, carbocyclic ring, cyclohydrocarbyl, or heterocyclic ring is optionally substituted with one or more substituents which include, but are not limited to, hydroxyl, an ester group, hydrocarbyloxyl, hydrocarbyl, a carbocyclic ring, a heterocyclic ring, halogen, oxygen, sulfur, amino, and amido.

In some embodiments, the present disclosure provides the amino lipid compound of formula (I), (IA), (I-1), (I-2), (I-3), (I-4), (IV), (IV-1), (IV-2), (IV-3), (IV-4), (V), (V-1), (V-2), (V-3), (V-4), (V-5), (V-5-1), (V-5-2), (V-6), (V-6-1), (VI), (VI-1), (VI-2), (VI-3), (VI-4), (VI-5), (V-7), (V-7-1), (V-7-2), (V-7-3), (VI-6), (VI-6-1), (IV-5), or (IV-5-1), or a pharmaceutically acceptable salt thereof, or a stereoisomer thereof, as described above, wherein the amino lipid compound is selected from:

| Amino lipid compounds | Structural formulae |
|---|---|
| 1501 | 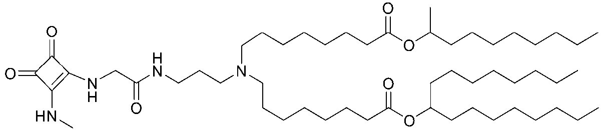 |
| 1502 | 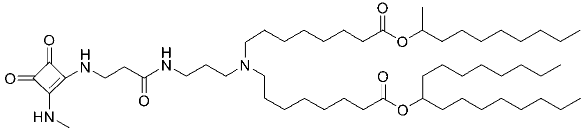 |
| 1503 | 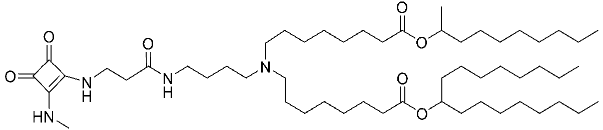 |
| 1504 | 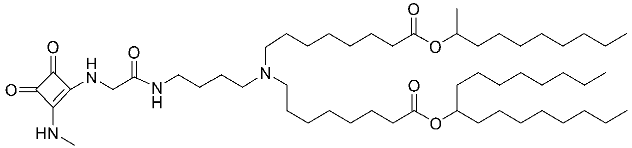 |
| 1505 | 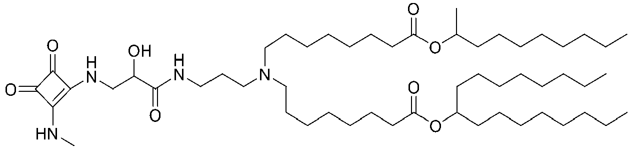 |
| 1506 | 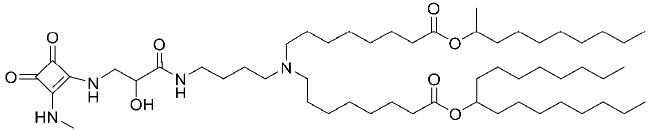 |
| 1507 | 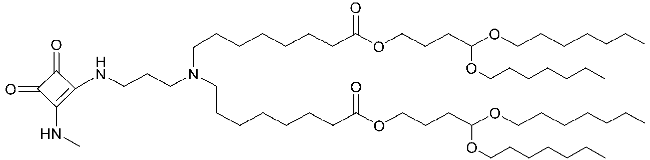 |
| 1508 | 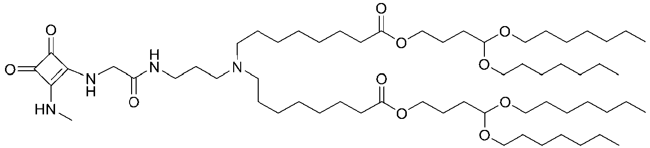 |
| 1509 | 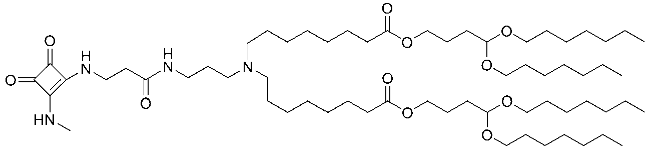 |
| 1510 | 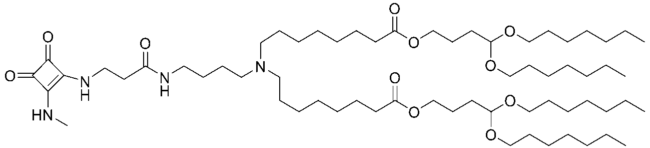 |
| 1511 | 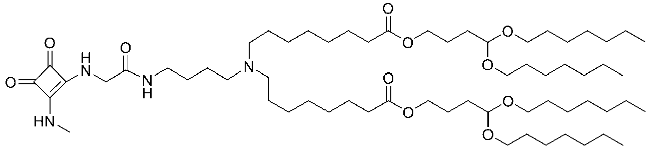 |
| 1512 | 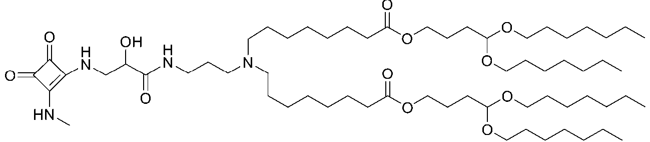 |
| 1513 | 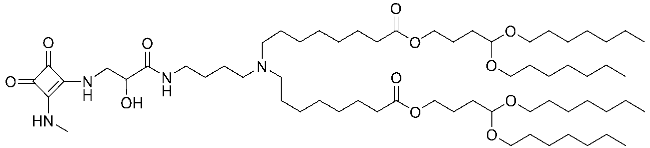 |
| 1523 | 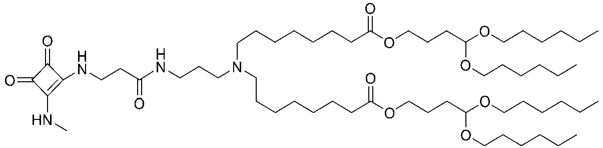 |
| 1524 | 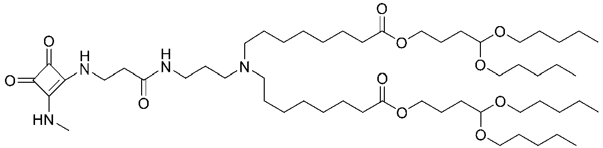 |
| 1525 | 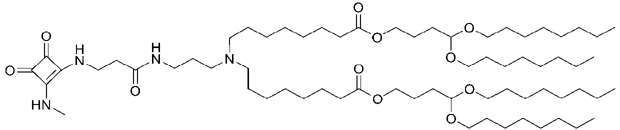 |
| 1526 | 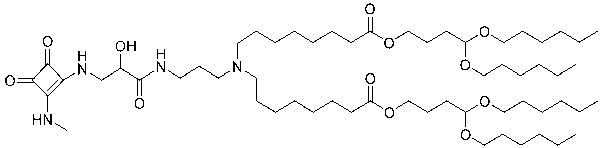 |
| 1527 | 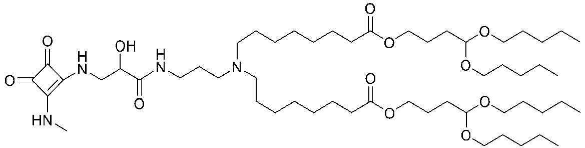 |
| 1528 | 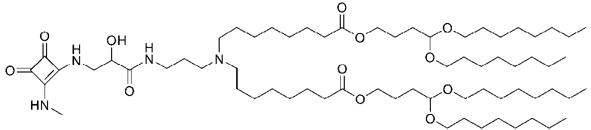 |
| 1529 | 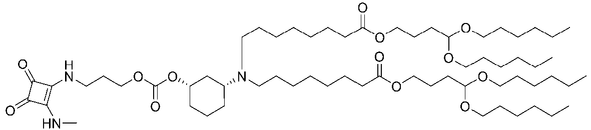 |
| 1530 | 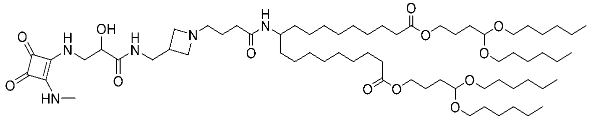 |
| 1531 | 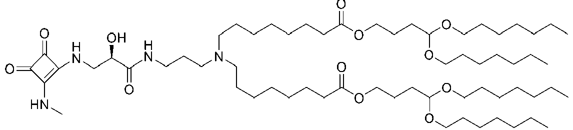 |
| 1532 | 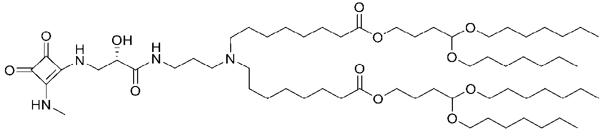 |
| 1533 | 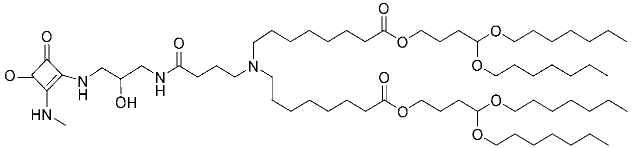 |
| 1534 | 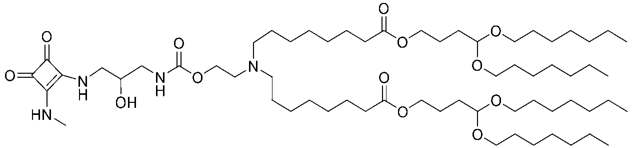 |
| 1535 | 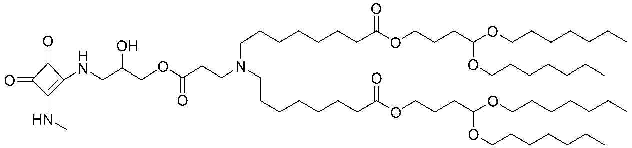 |
| 1536 | 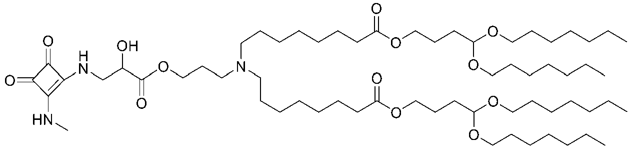 |
| 1537 | 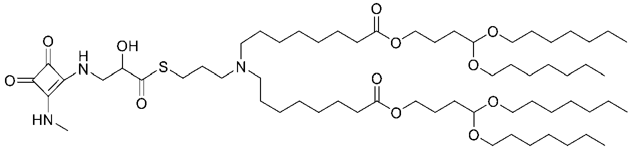 |
| 1538 | 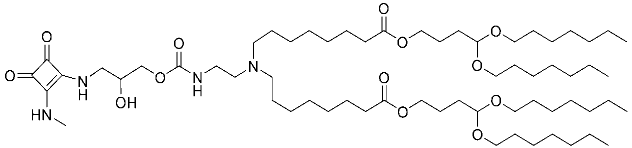 |
| 1539 | 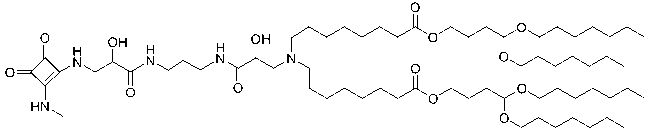 |
| 1540 | 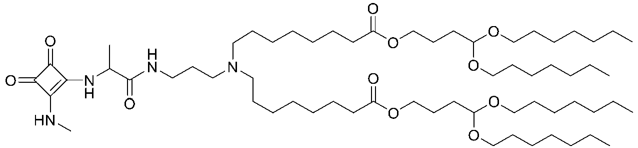 |
| 1541 | 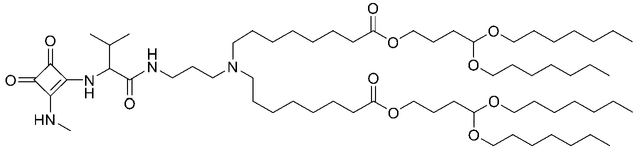 |
| 1542 | 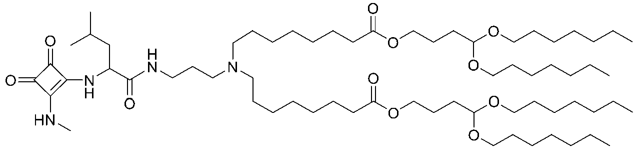 |
| 1543 | 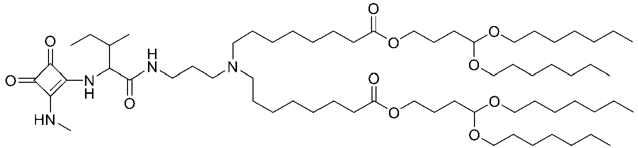 |
| 1544 | 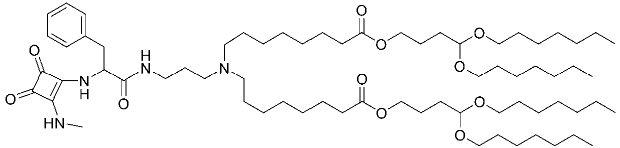 |
| 1545 | 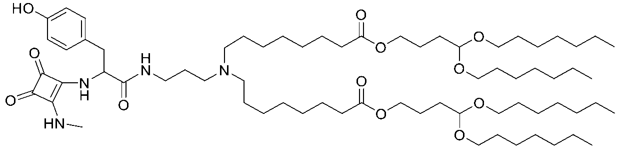 |
| 1546 | 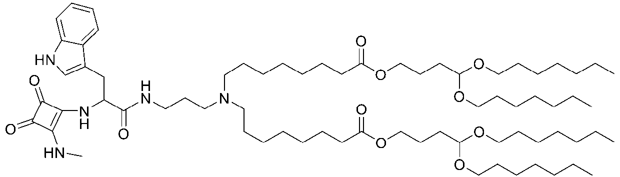 |
| 1547 | 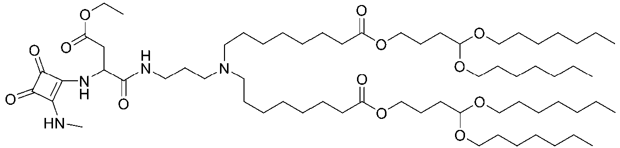 |
| 1548 | 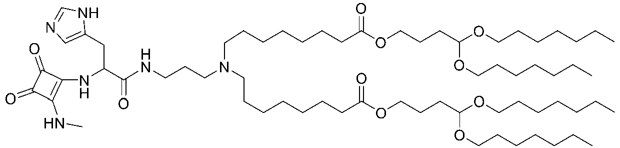 |
| 1549 | 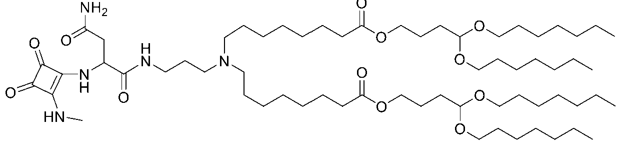 |
| 1550 | 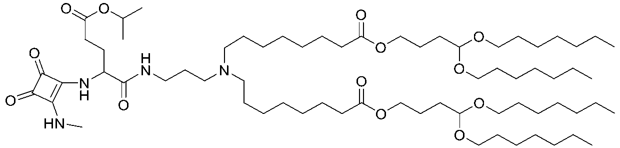 |
| 1551 | 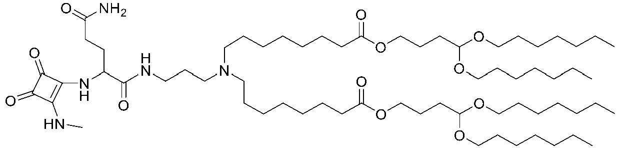 |
| 1552 | 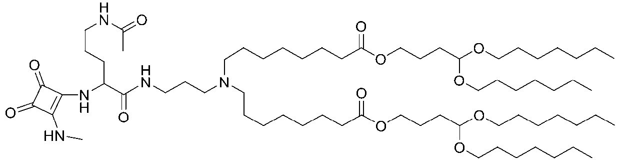 |
| 1553 | 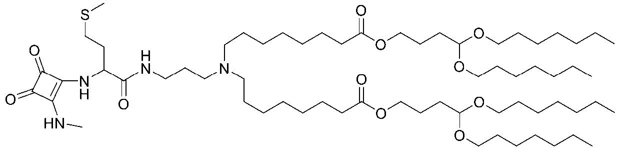 |
| 1554 | 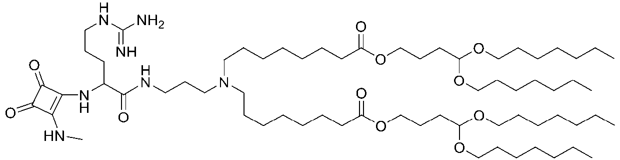 |
| 1555 | 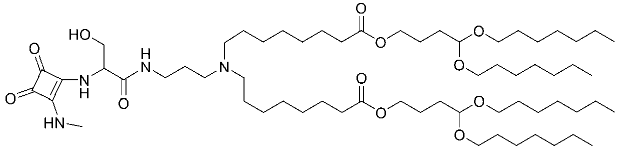 |
| 1556 | 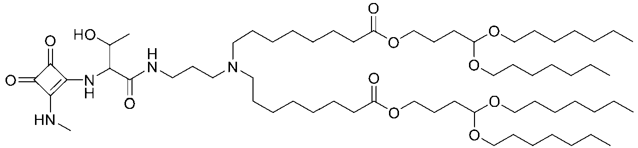 |
| 1557 | 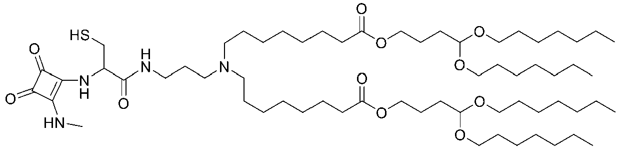 |
| 1558 | 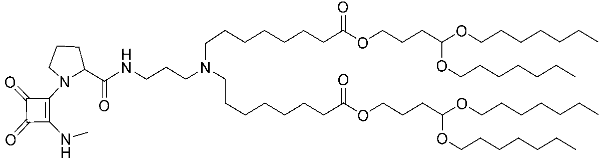 |
| 1559 | 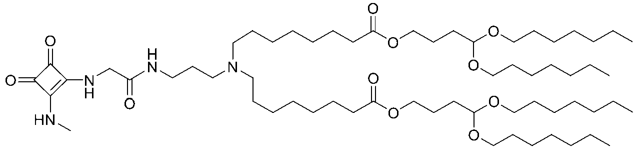 |
| 1560 | 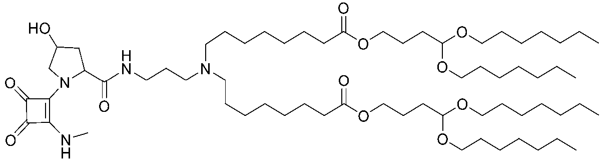 |
| 1561 | 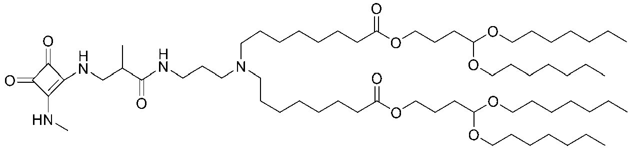 |
| 1562 | 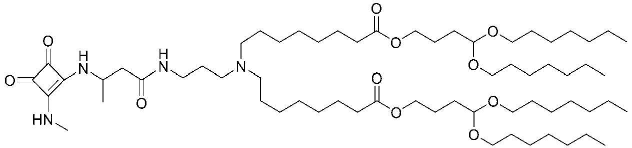 |
| 1563 | 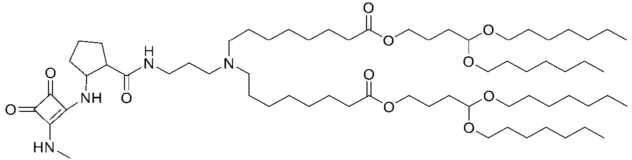 |
| 1564 | 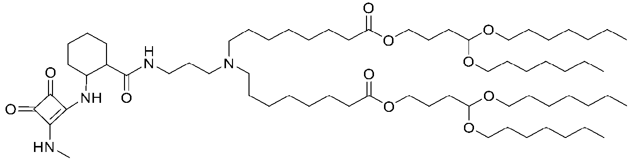 |
| 1565 | 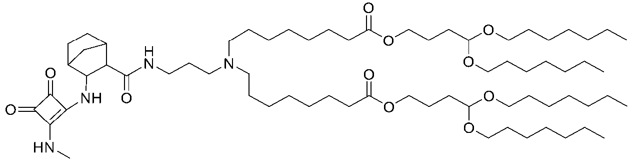 |
| 1566 | 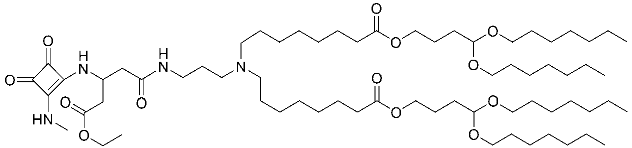 |
| 1567 | 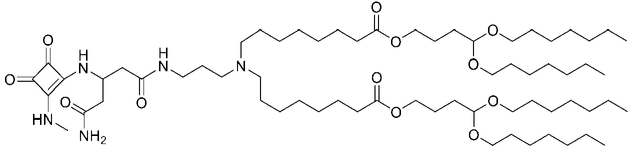 |
| 1568 | 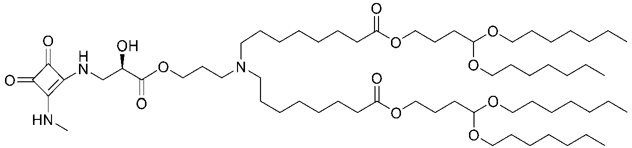 |
| 1569 | 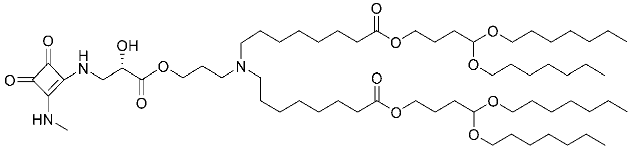 |
| 1570 | 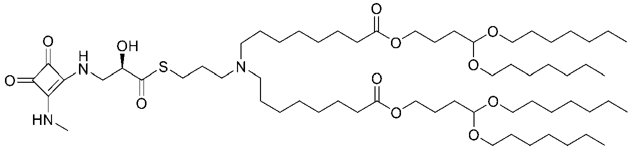 |
| 1571 | 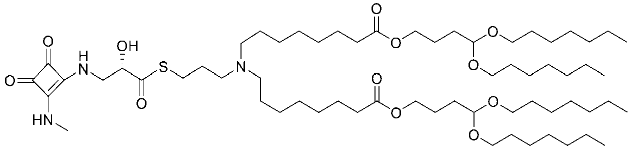 |
| 1572 | 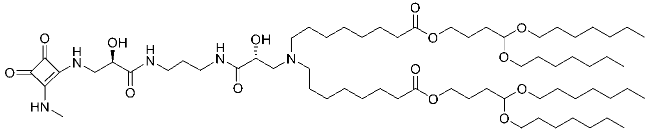 |
| 1573 | 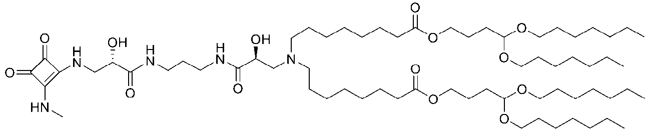 |
| 1574 | 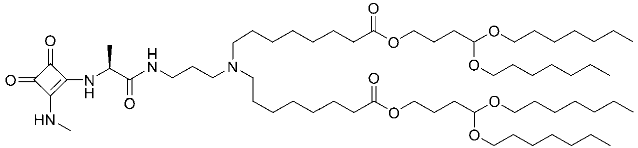 |
| 1575 | 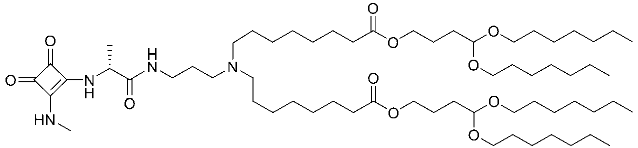 |
| 1576 | 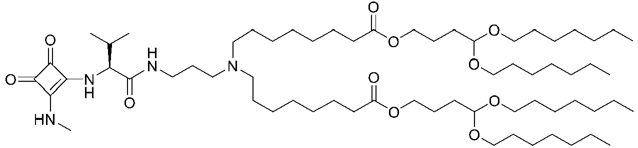 |
| 1577 | 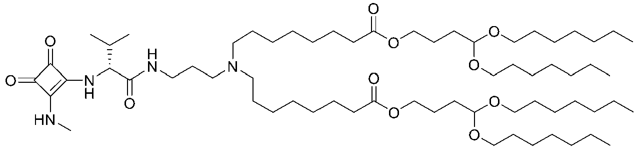 |
| 1578 | 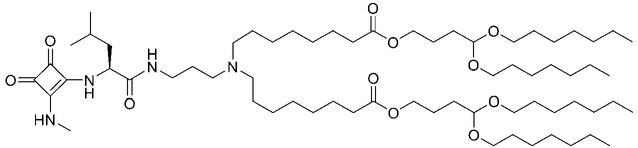 |
| 1579 | 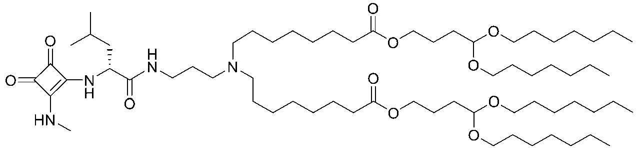 |
| 1580 | 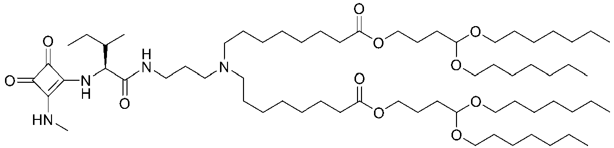 |
| 1581 | 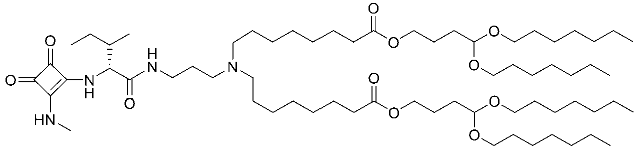 |
| 1582 | 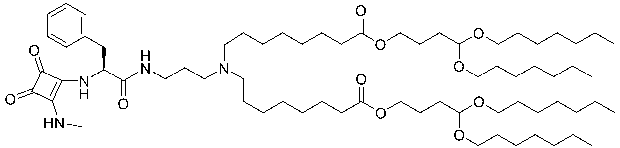 |
| 1583 | 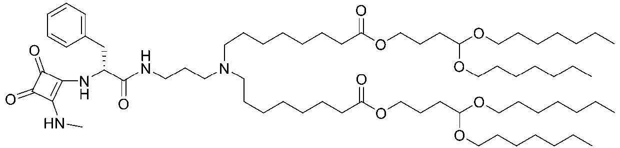 |
| 1584 | 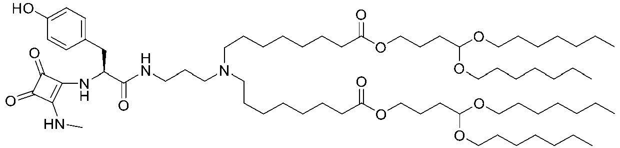 |
| 1585 | 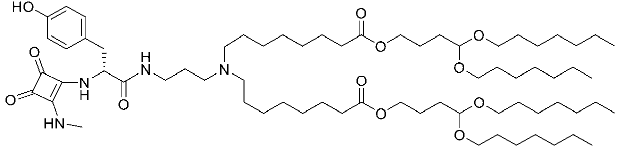 |
| 1586 | 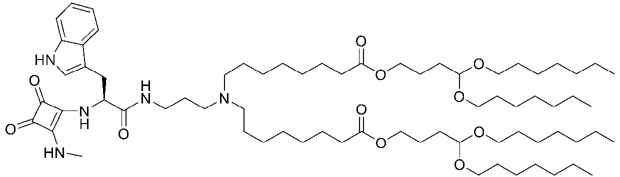 |
| 1587 | 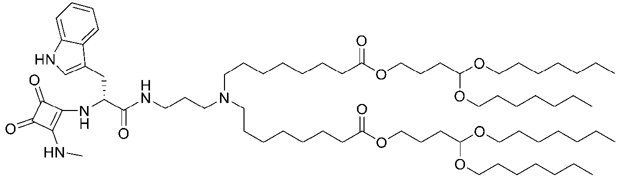 |
| 1588 | 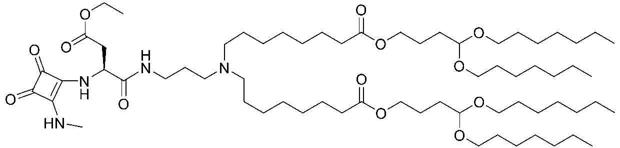 |
| 1589 | 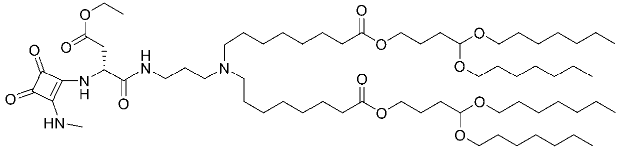 |
| 1590 | 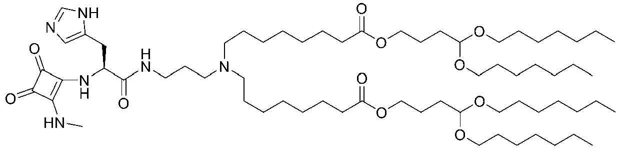 |
| 1591 | 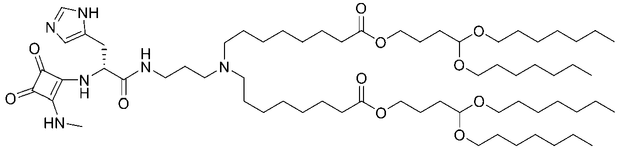 |
| 1592 | 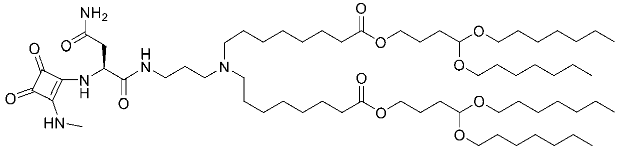 |
| 1593 | 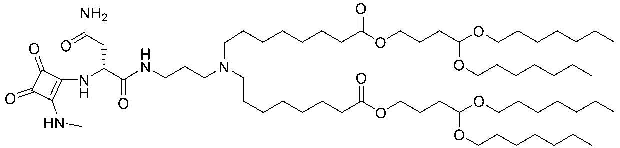 |
| 1594 | 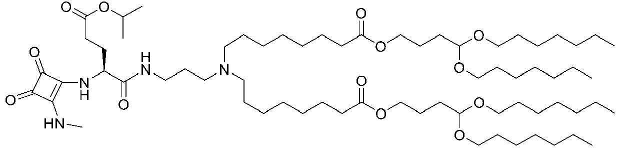 |
| 1595 | 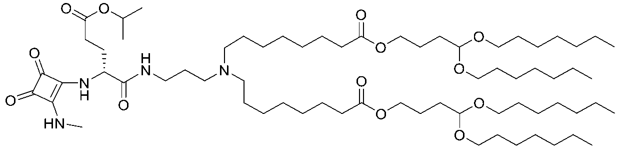 |
| 1596 | 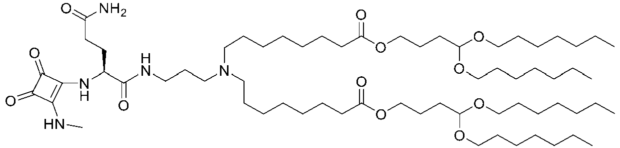 |
| 1597 | 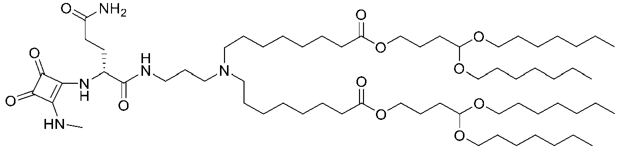 |
| 1598 | 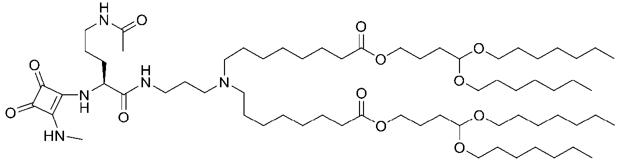 |
| 1599 | 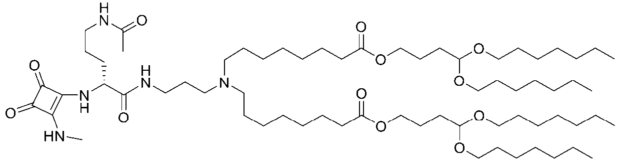 |
| 1600 | 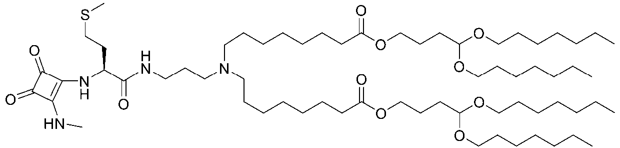 |
| 1601 | 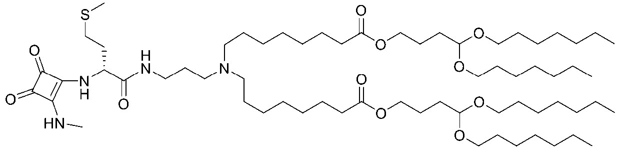 |
| 1602 | 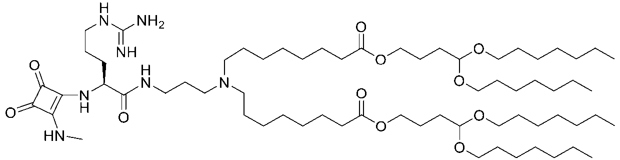 |
| 1603 | 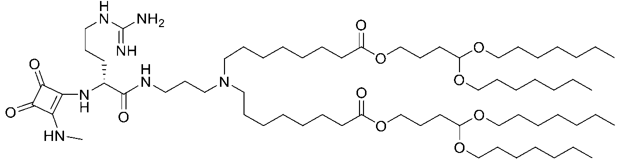 |
| 1604 | 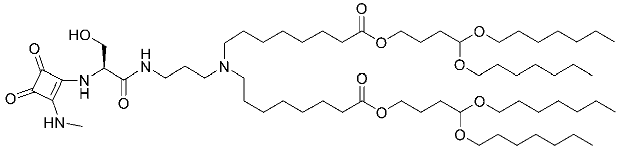 |
| 1605 | 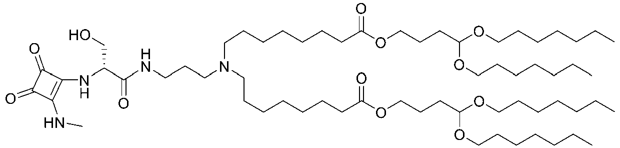 |
| 1606 | 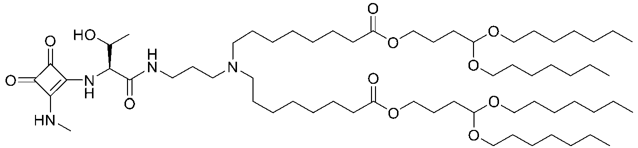 |
| 1607 | 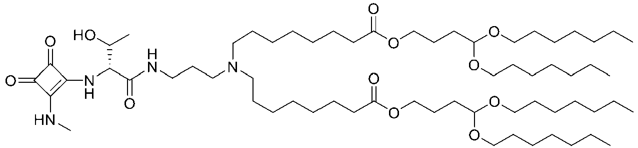 |
| 1608 | 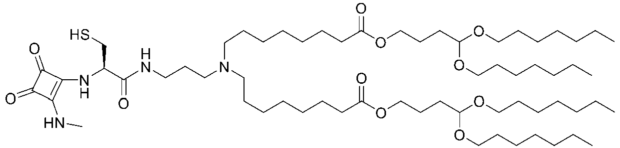 |
| 1609 | 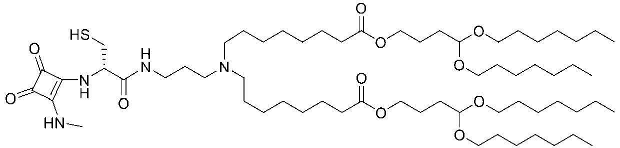 |
| 1610 | 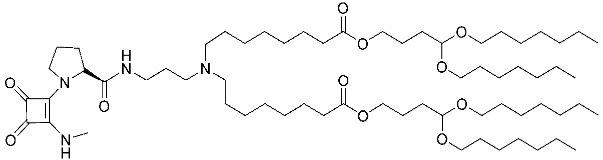 |
| 1611 | 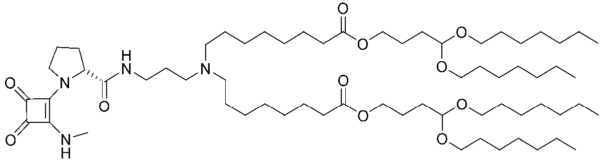 |
| 1612 | 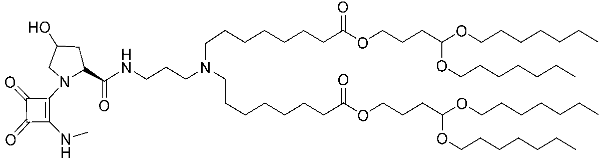 |
| 1613 | 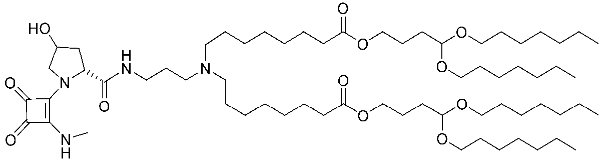 |
| 1614 | 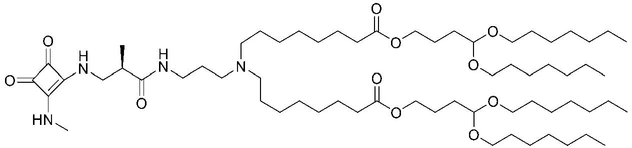 |
| 1615 | 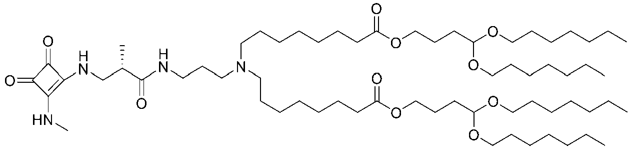 |
| 1616 | 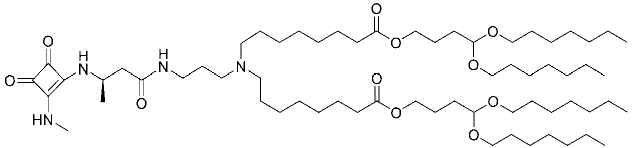 |
| 1617 | 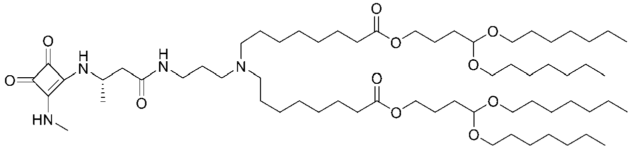 |
| 1618 | 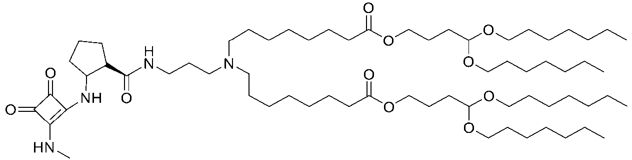 |
| 1619 | 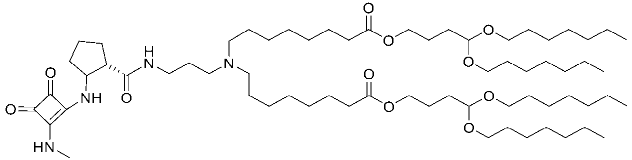 |
| 1620 | 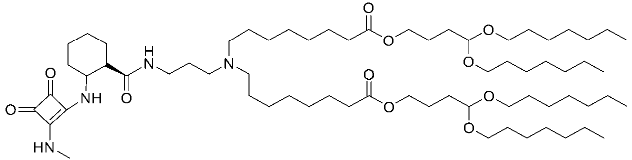 |
| 1621 | 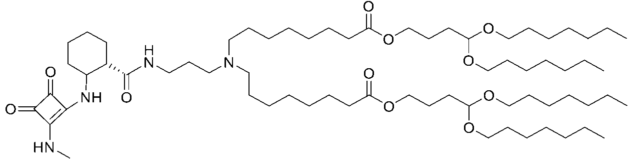 |
| 1622 | 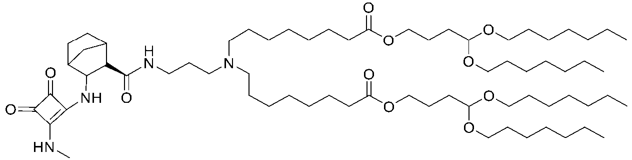 |
| 1623 | 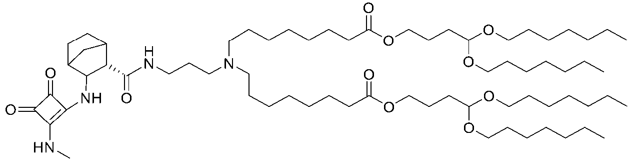 |
| 1624 | 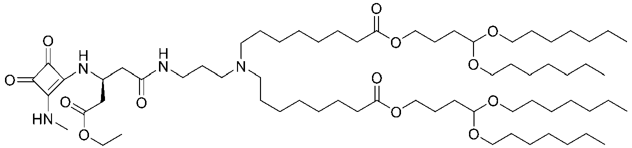 |
| 1625 | 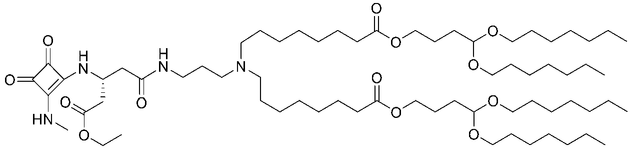 |
| 1626 | 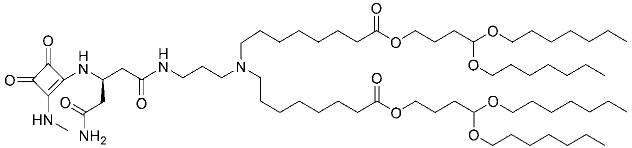 |
| 1627 | 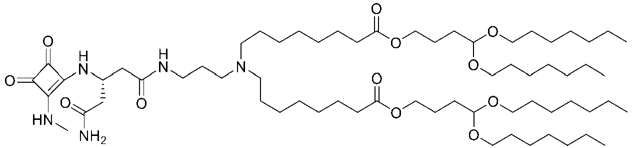 |
| 1628 | 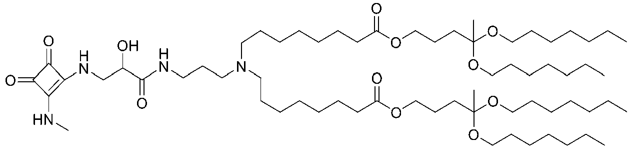 |
| 1629 | 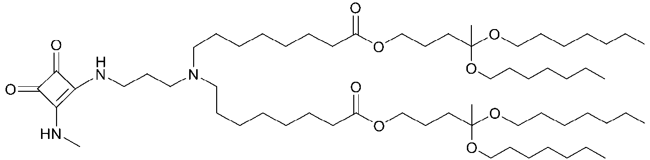 |
| 1630 | 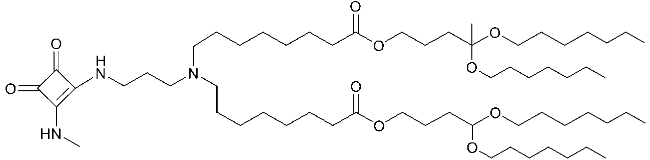 |
| 1631 | 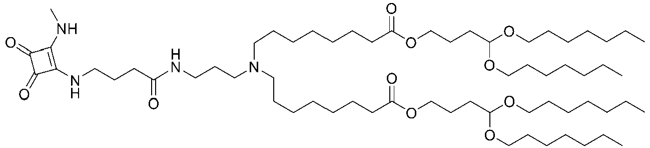 |
| 1632 | 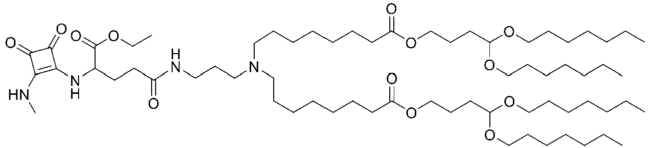 |
| 1633 | 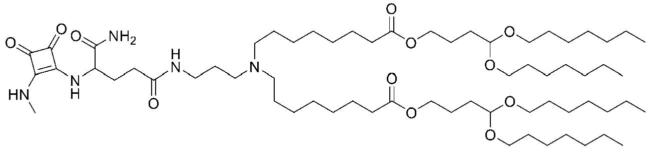 |
| 1634 | 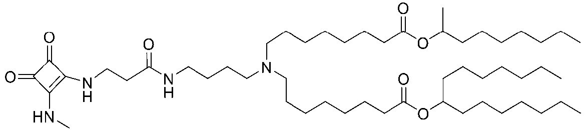 |
| 1635 | 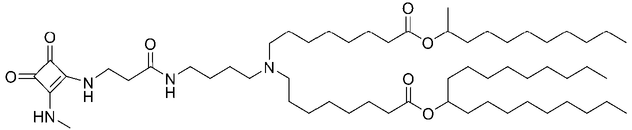 |
| 1636 | 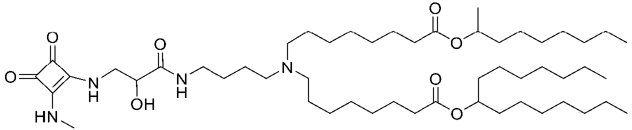 |
| 1637 | 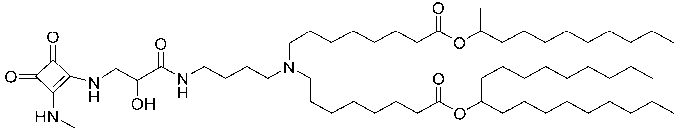 |
| 1638 | 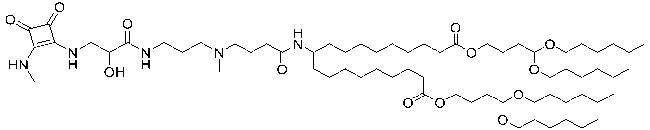 |
| 1639 | 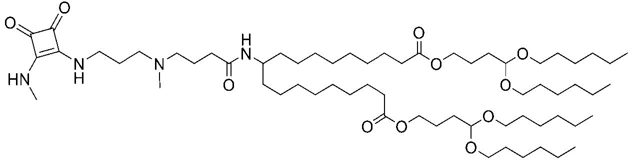 |
| 1640 | 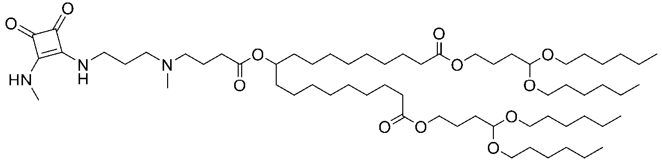 |
| 1641 | 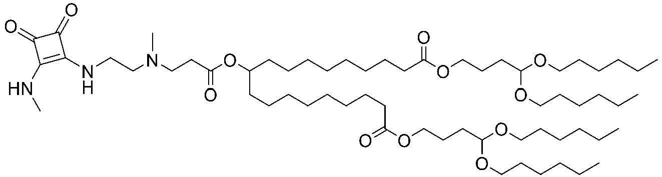 |
| 1642 | 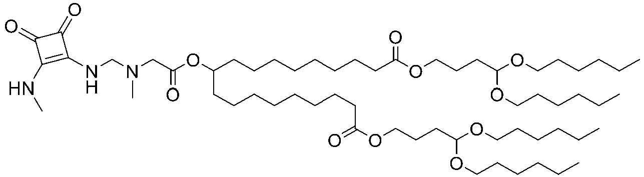 |
| 1643 | 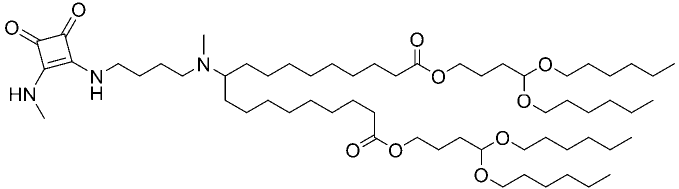 |
| 1644 | 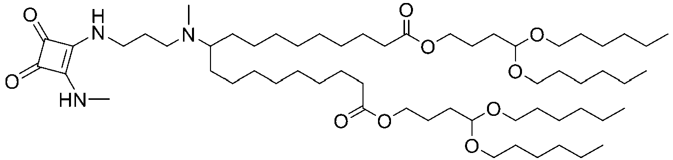 |
| 1645 | 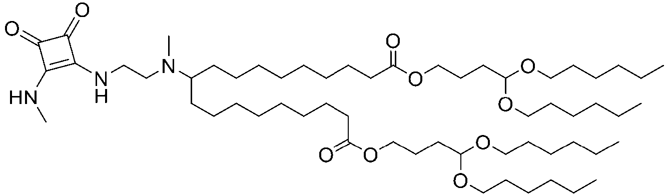 |
| 1646 | 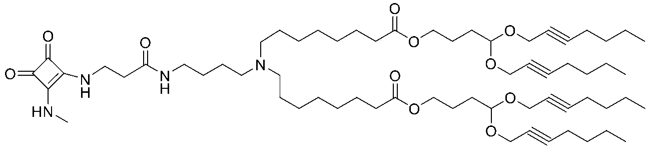 |
| 1647 | 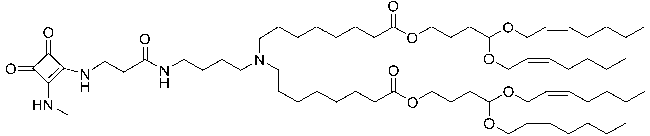 |
| 1648 | 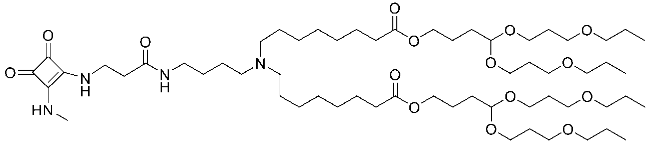 |
| 1649 | 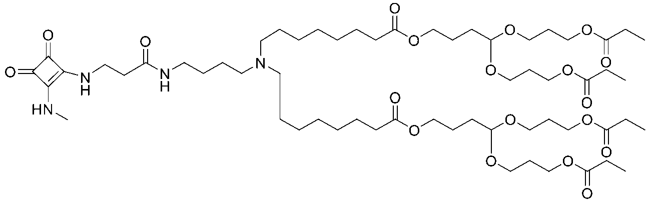 |
| 1650 | 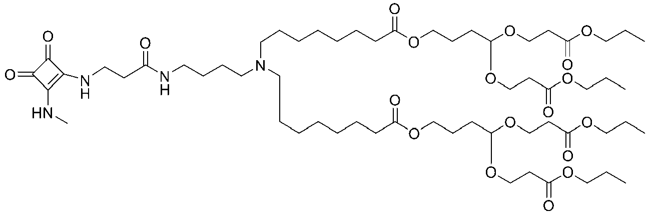 |
| 1651 | 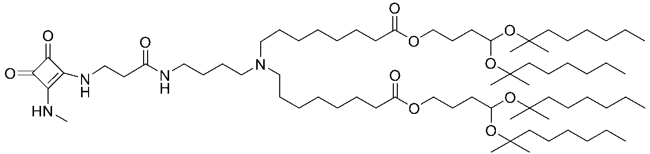 |
| 1652 | 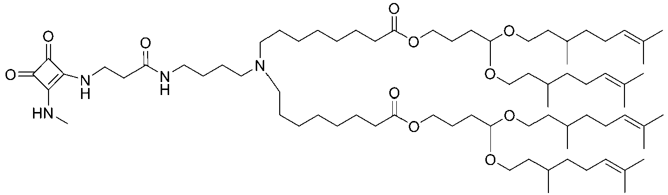 |
| 1653 | 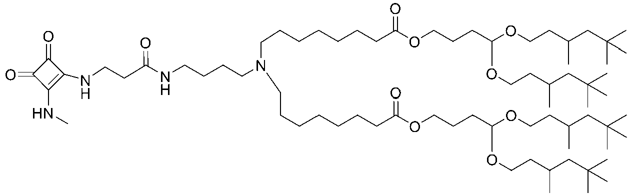 |
| 1654 | 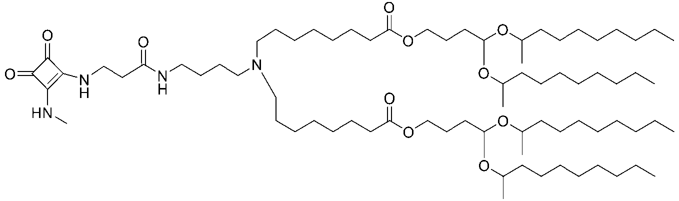 |
| 1655 | 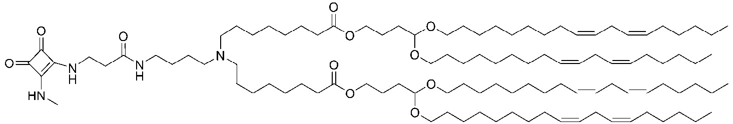 |
| 1656 | 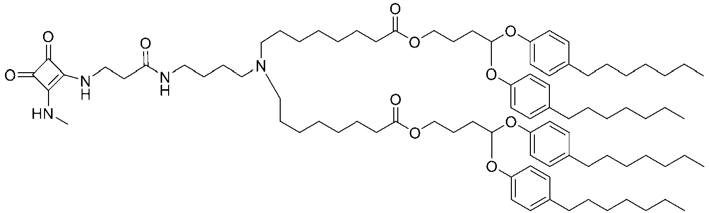 |
| 1657 | 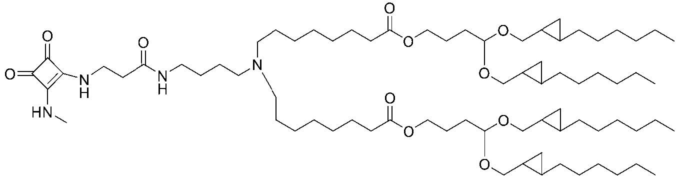 |
| 1658 | 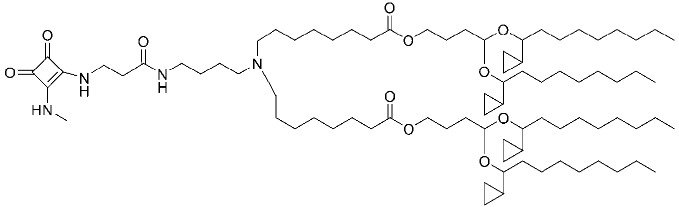 |
| 1659 | 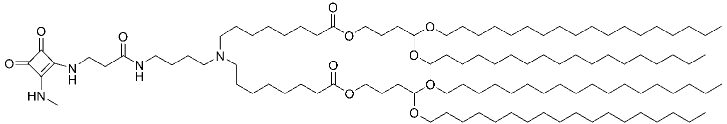 |
| 1660 | 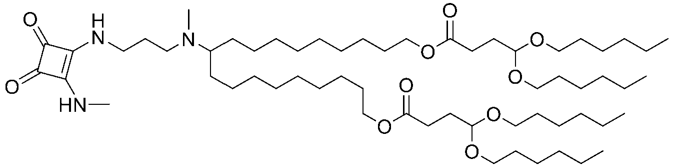 |
| 1661 | 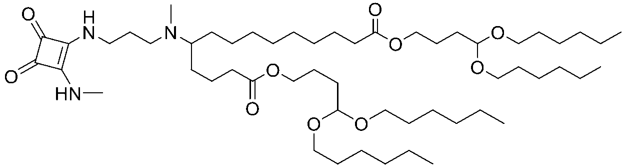 |
| 1662 | 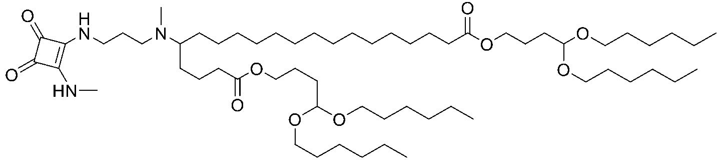 |
| 1663 | 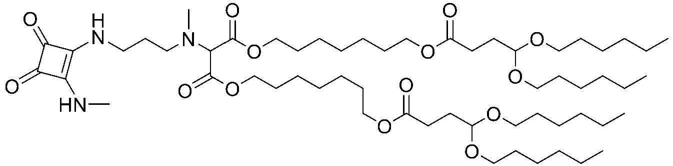 |
| 1664 | 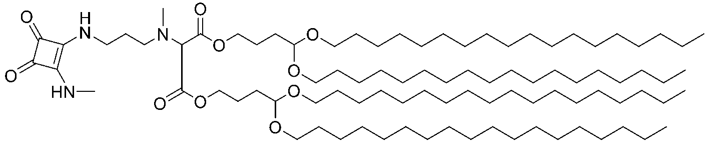 |
| 1665 | 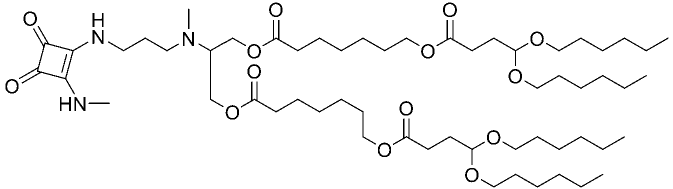 |
| 1666 | 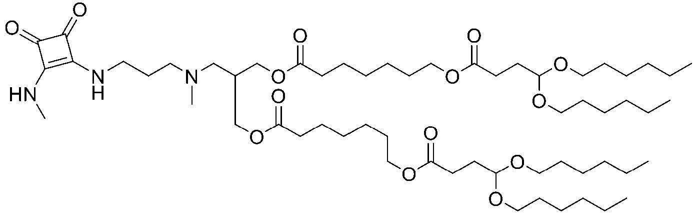 |
| 1667 | 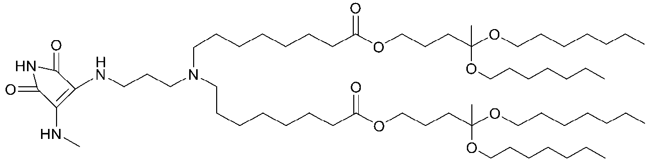 |
| 1668 | 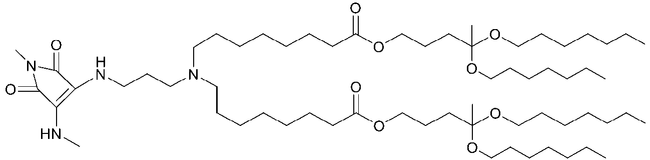 |
| 1669 | 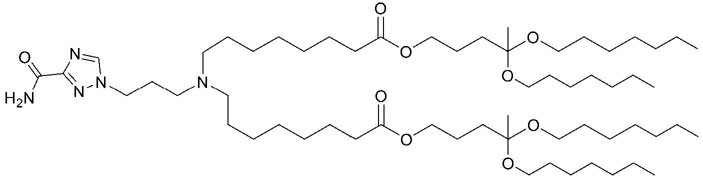 |
| 1670 | 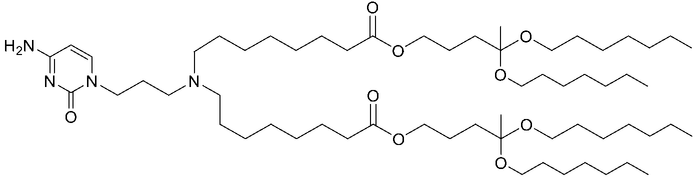 |
| 1671 | 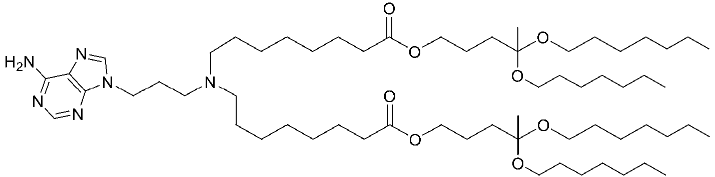 |
| 1672 | 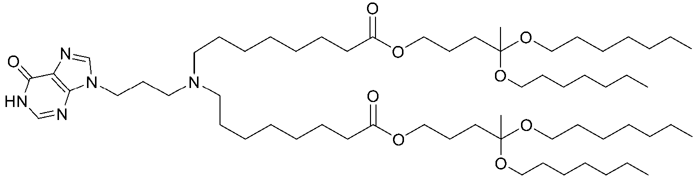 |
| 1673 | 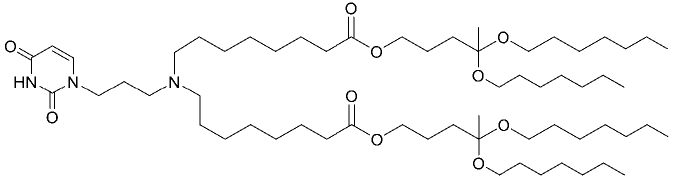 |
| 1674 | 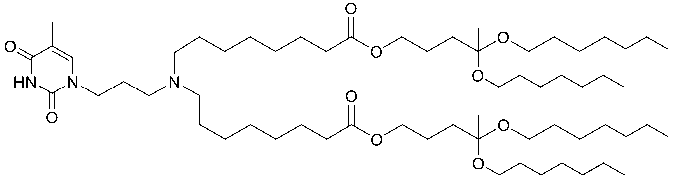 |
| 1675 | 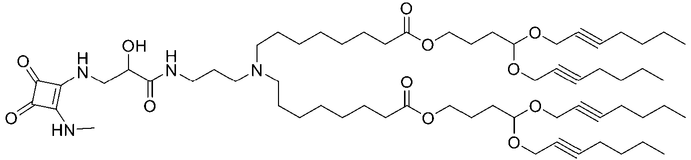 |
| 1676 | 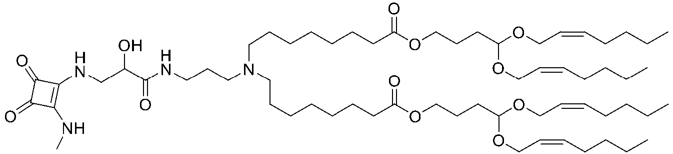 |
| 1677 | 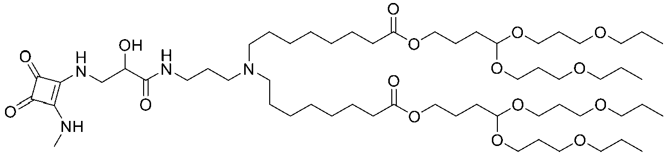 |
| 1678 | 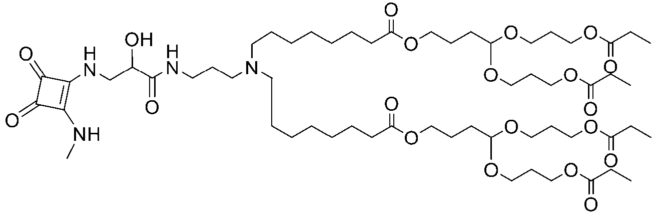 |
| 1679 | 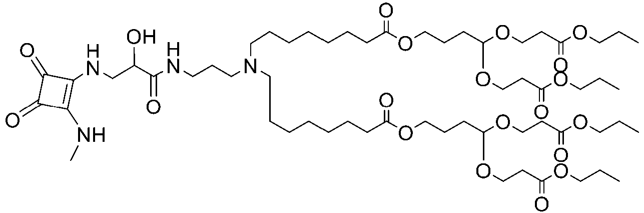 |
| 1680 | 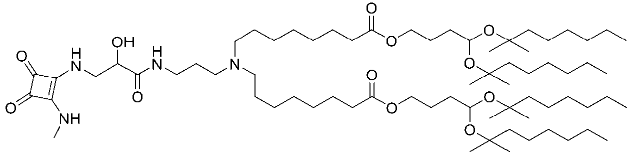 |
| 1681 | 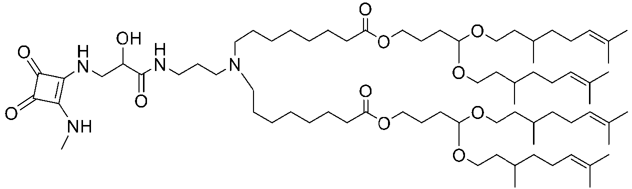 |
| 1682 | 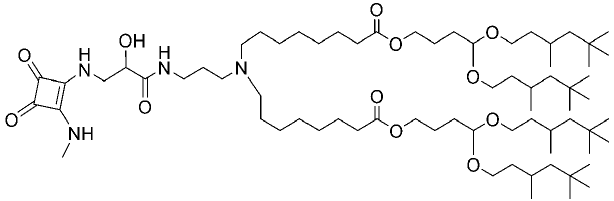 |
| 1683 | 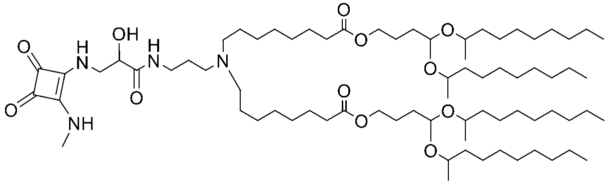 |
| 1684 | 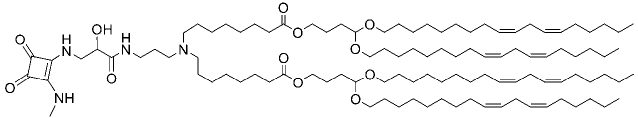 |
| 1685 | 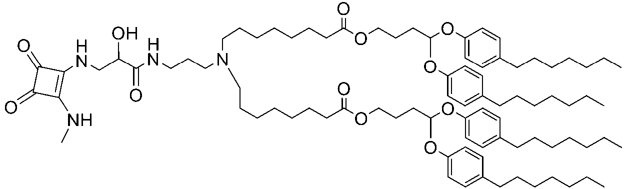 |
| 1686 | 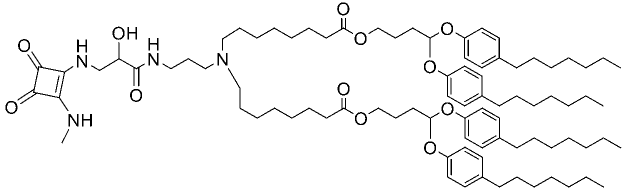 |
| 1687 | 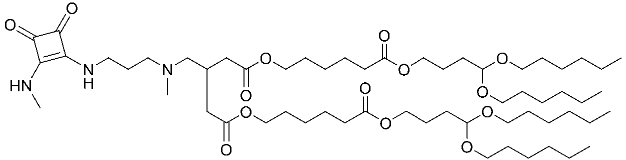 |
| 1688 | 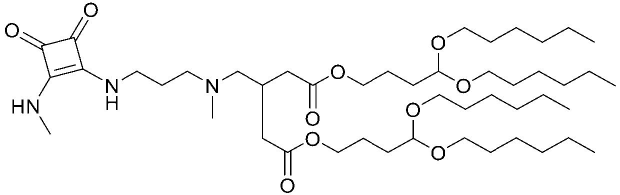 |
| 1689 | 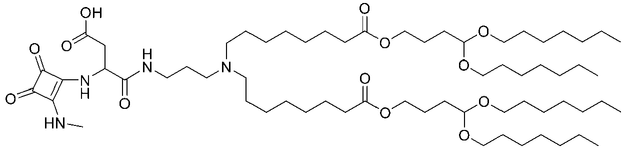 |
| 1690 | 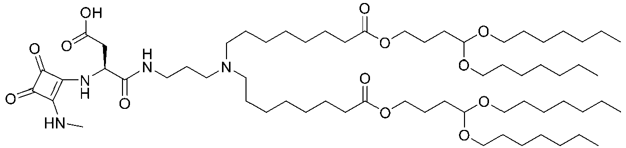 |
| 1691 | 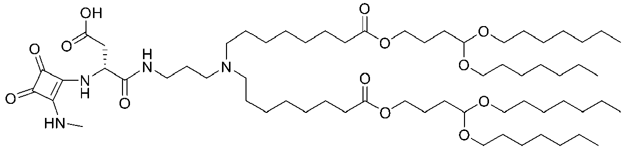 |
| 1692 | 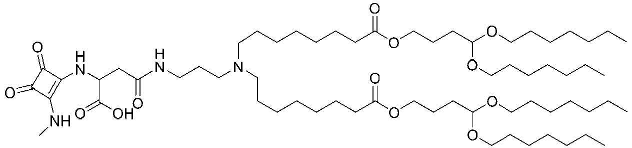 |
| 1693 | 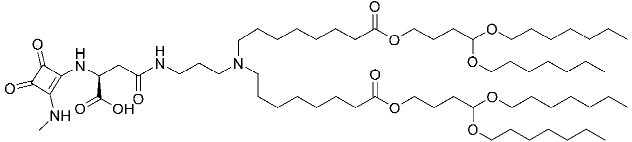 |
| 1694 | 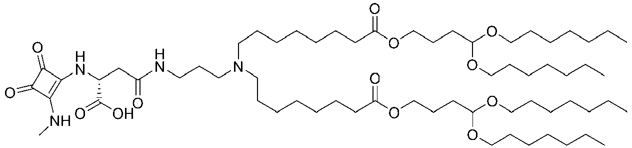 |
| 1695 | 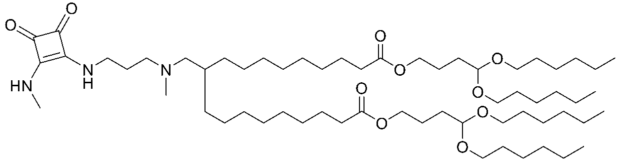 |
| 1696 | 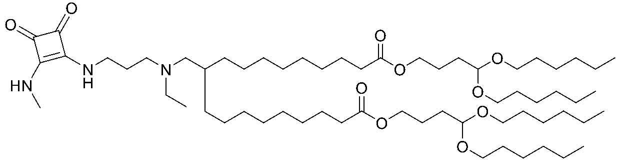 |
| 1697 | 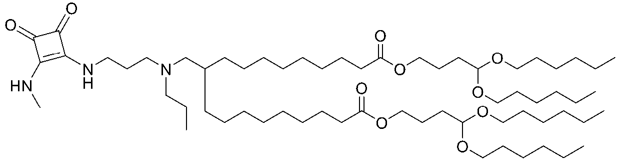 |
| 1698 | 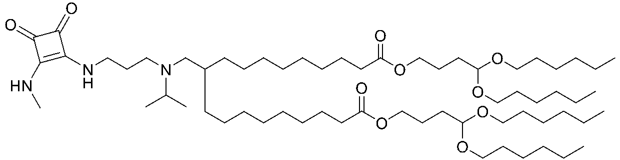 |
| 1699 | 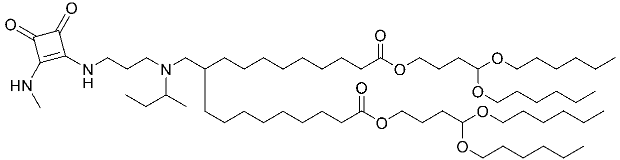 |
| 1700 | 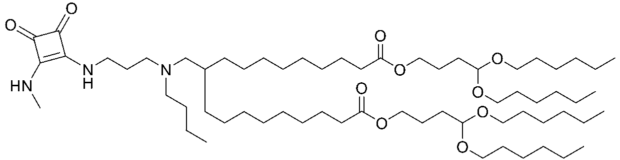 |
| 1701 | 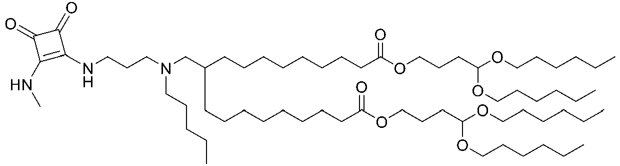 |
| 1702 | 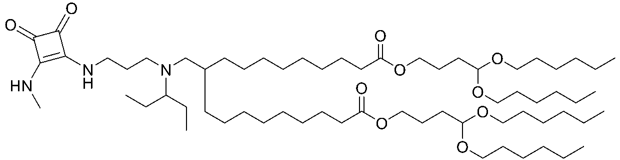 |
| 1703 | 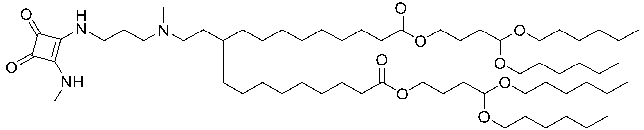 |
| 1704 | 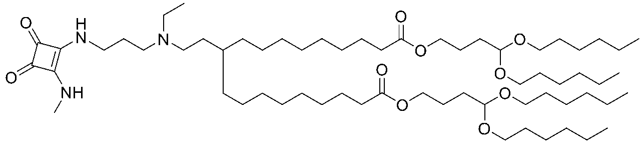 |
| 1705 | 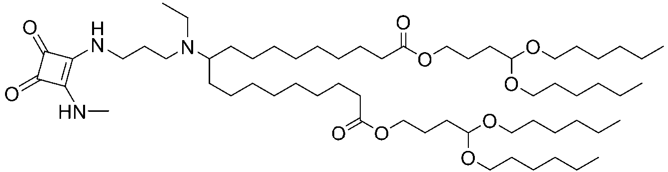 |
| 1706 | 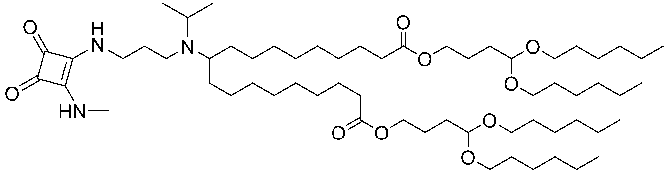 |
| 1707 | 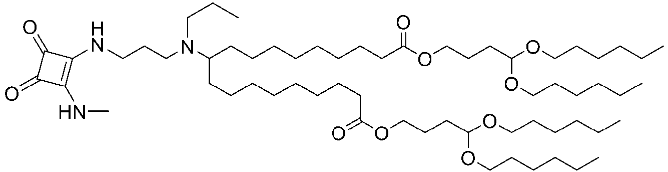 |
| 1708 | 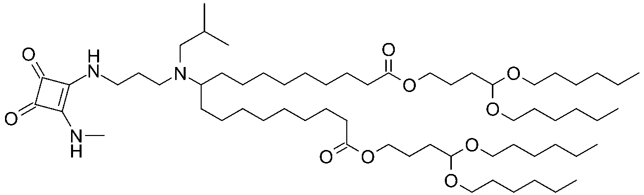 |
| 1709 | 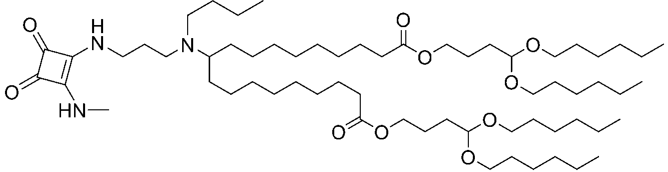 |
| 1710 | 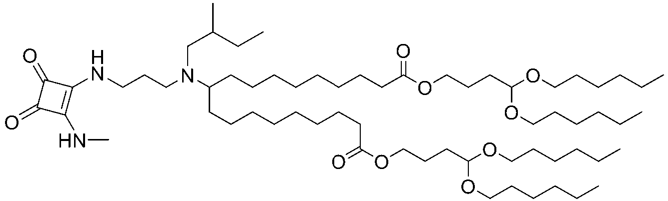 |
| 1711 | 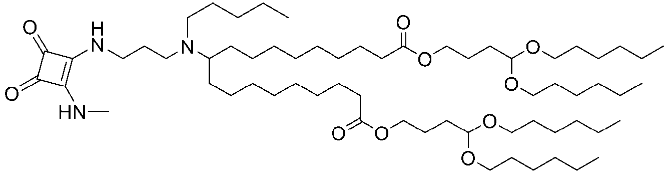 |
| 1712 | 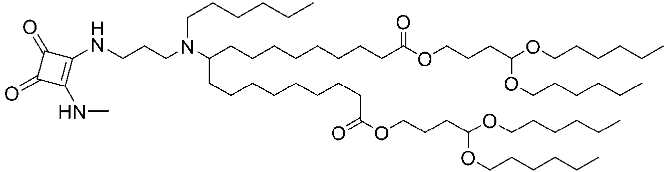 |
| 1713 | 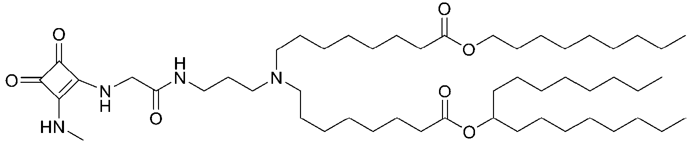 |
| 1714 | 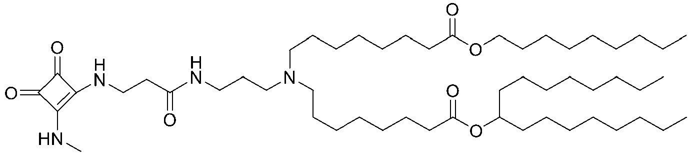 |
| 1715 | 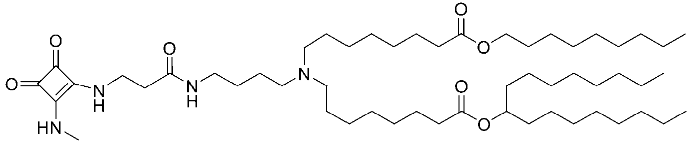 |
| 1716 | 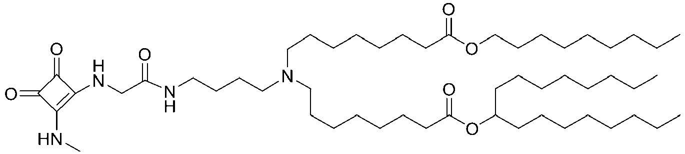 |
| 1717 | 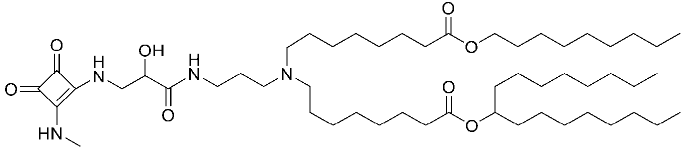 |
| 1718 | 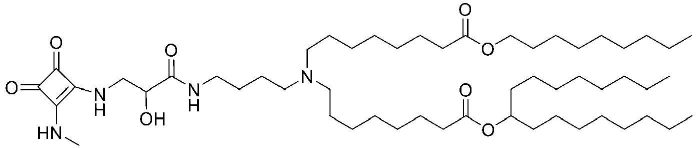 |
| 1719 | 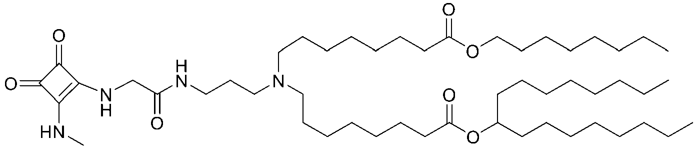 |
| 1720 | 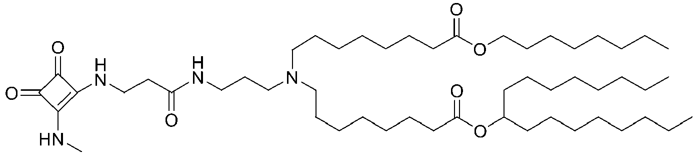 |
| 1721 | 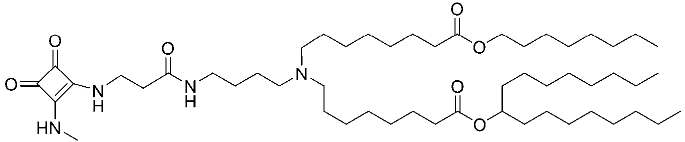 |
| 1722 | 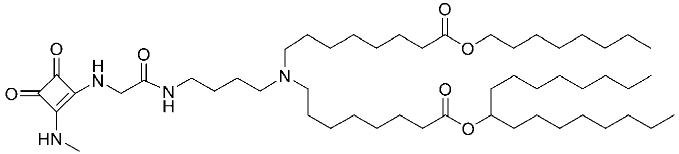 |
| 1723 | 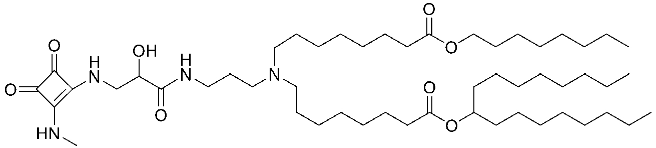 |
| 1724 | 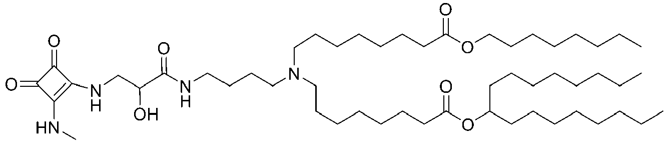 |
| 1725 | 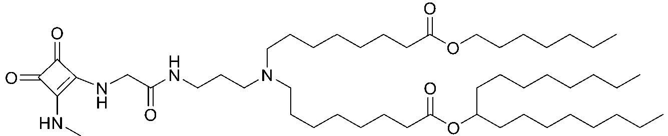 |
| 1726 | 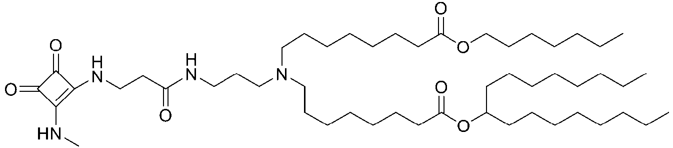 |
| 1727 | 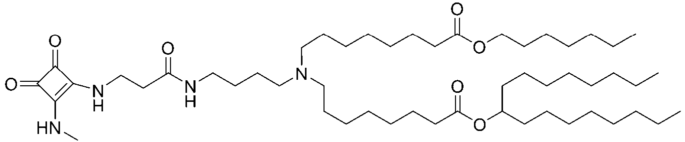 |
| 1728 | 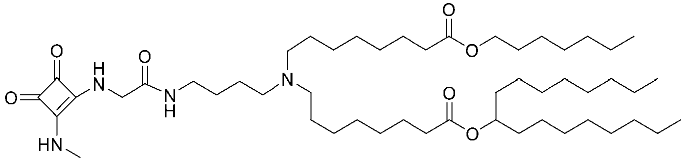 |
| 1729 | 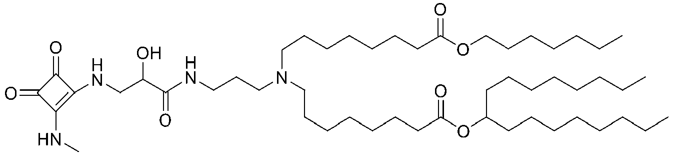 |
| 1730 | 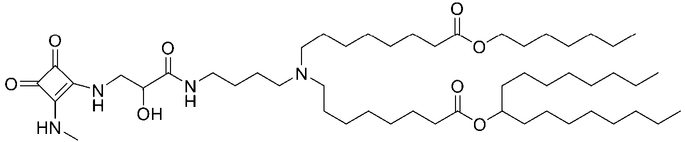 |
| 1731 | 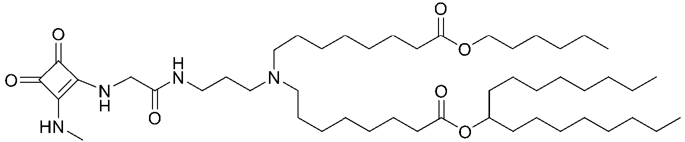 |
| 1732 | 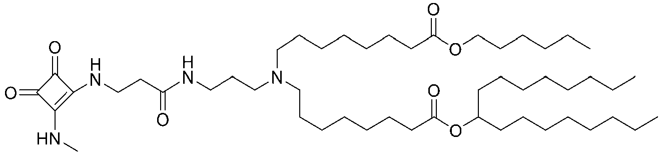 |
| 1733 | 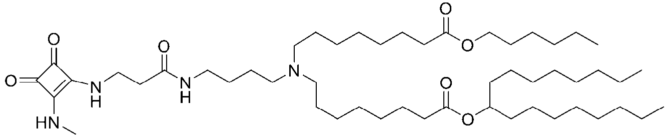 |
| 1734 | 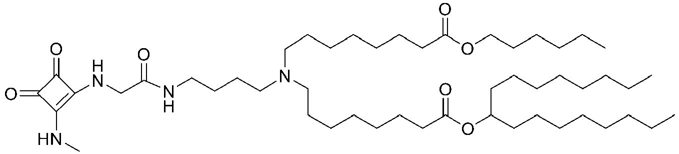 |
| 1735 | 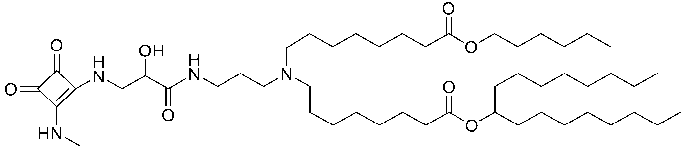 |
| 1736 | 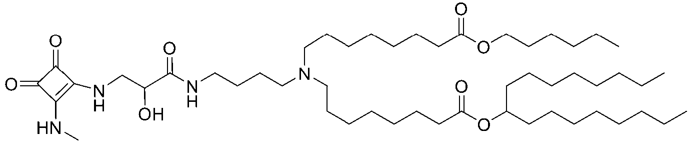 |
| 1737 | 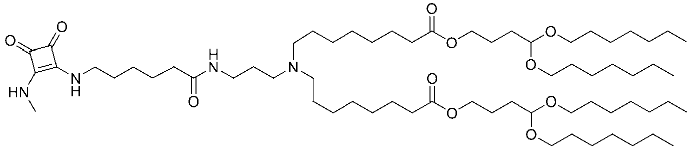 |
| 1738 | 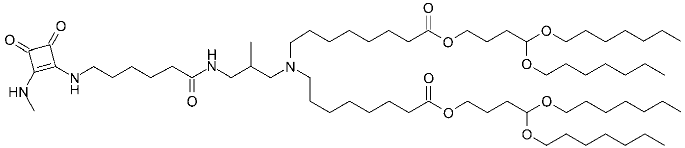 |
| 1739 | 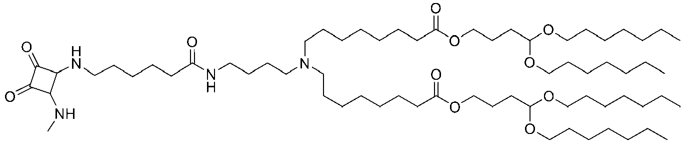 |
| 1740 | 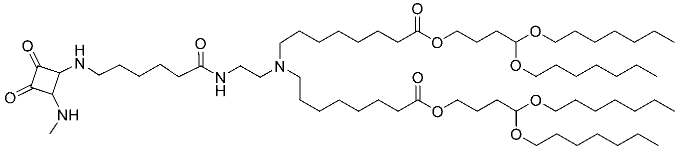 |
| 1741 | 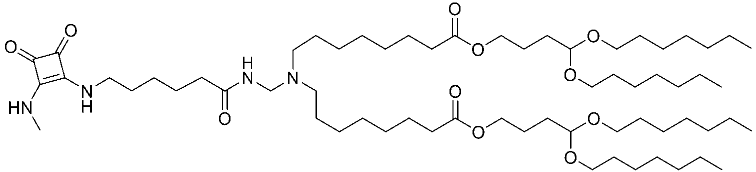 |
| 1742 | 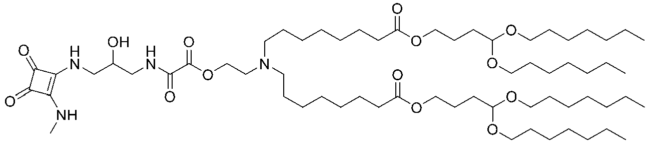 |
| 1743 | 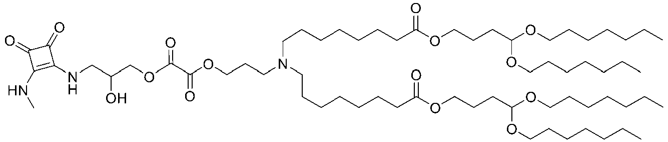 |
| 1744 | 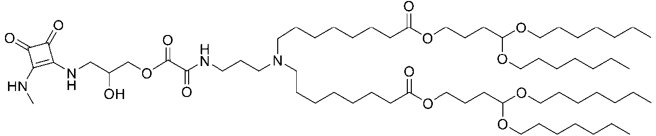 |
| 1745 | 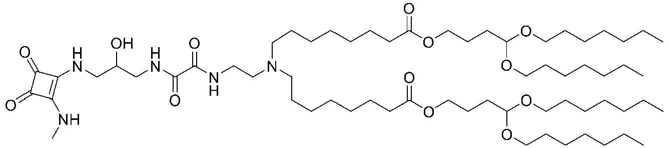 |
| 1746 | 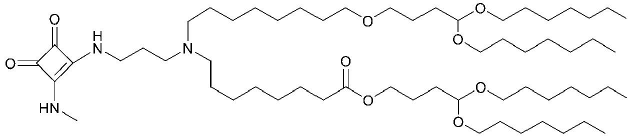 |
| 1747 | 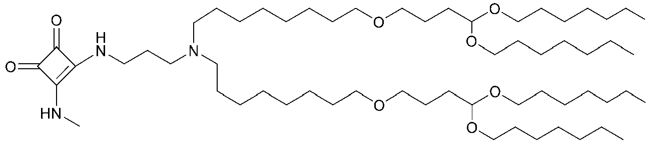 |
| 1748 | 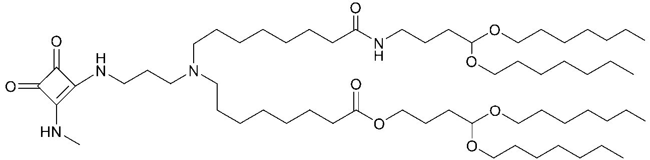 |
| 1749 | 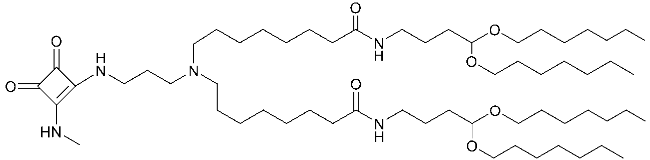 |
| 1750 | 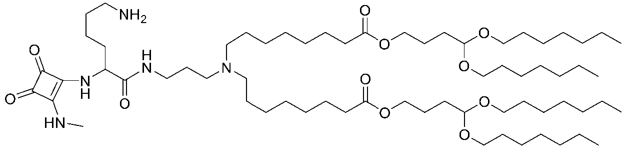 |
| 1751 | 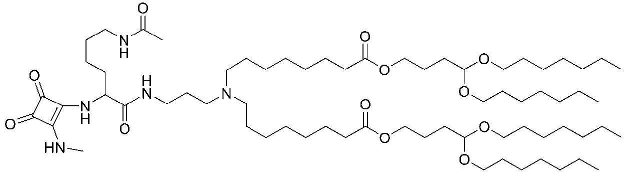 |
| 1752 | 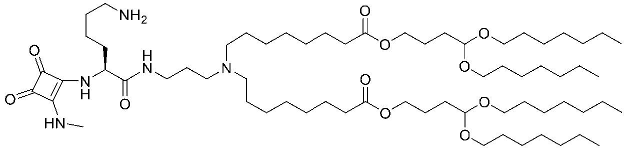 |
| 1753 | 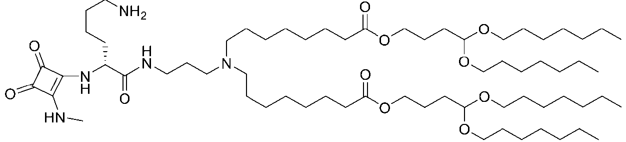 |
| 1754 | 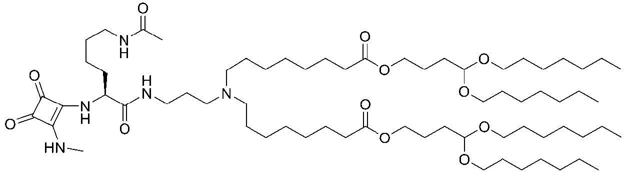 |
| 1755 | 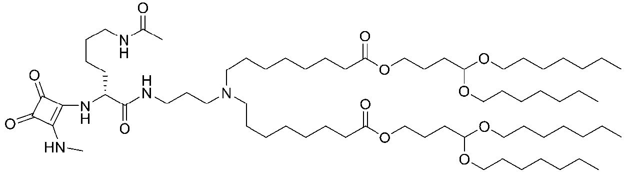 |
| 1756 | 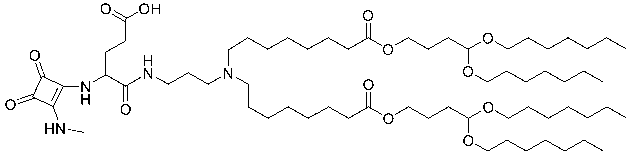 |
| 1757 | 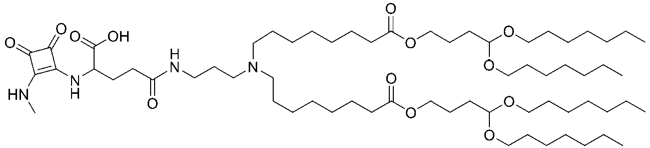 |
| 1758 | 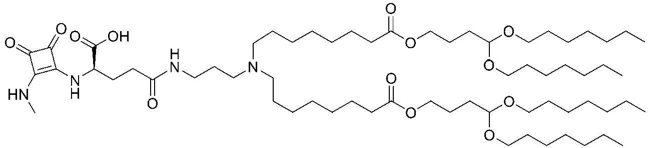 |
| 1759 | 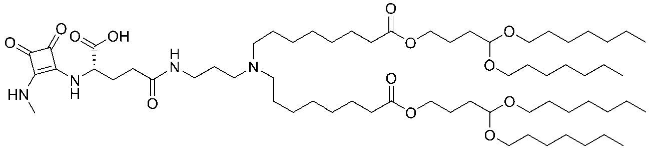 |
| 1760 | 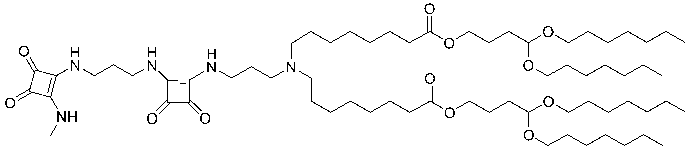 |
| 1761 | 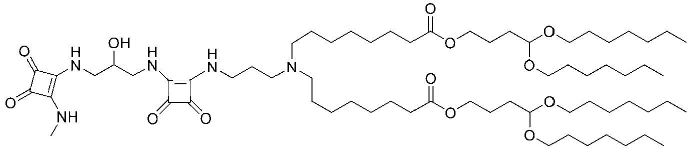 |
| 1762 | 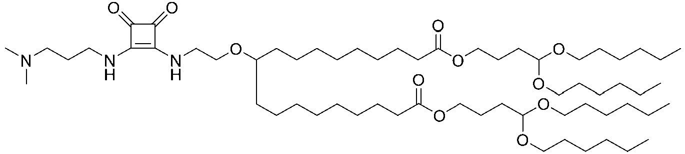 |
| 1763 | 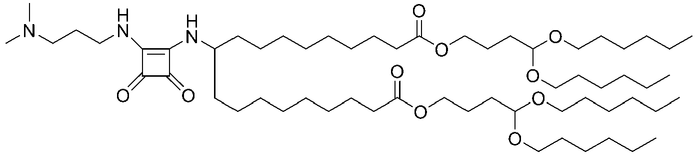 |
| 1764 | 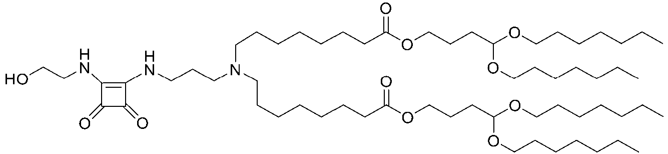 |
| 1765 | 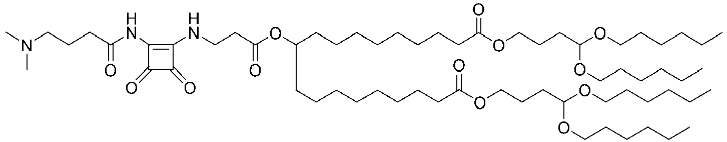 |
| 1766 | 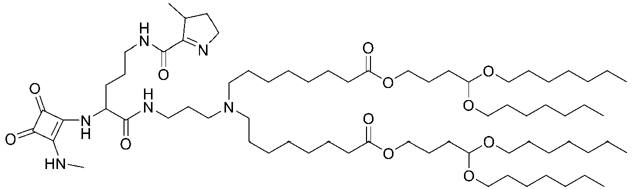 |
| 1767 | 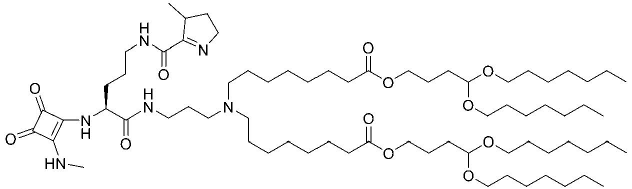 |
| 1768 | 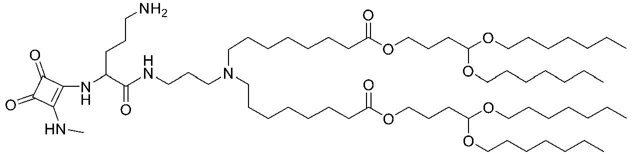 |
| 1769 | 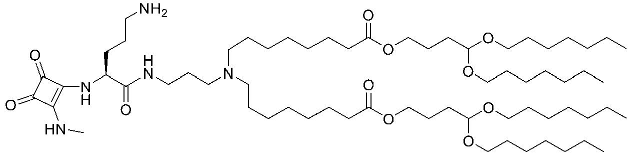 |
| 1770 | 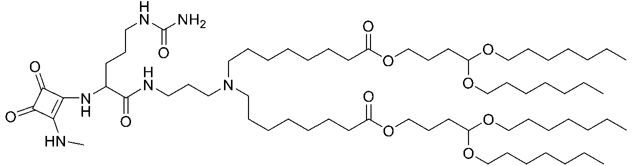 |
| 1771 | 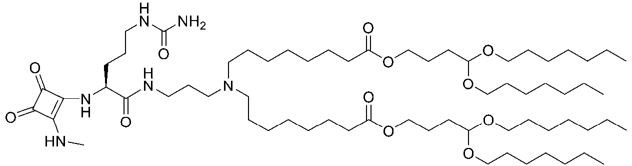 |
| 1772 | 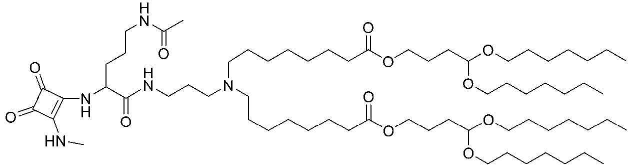 |
| 1773 | 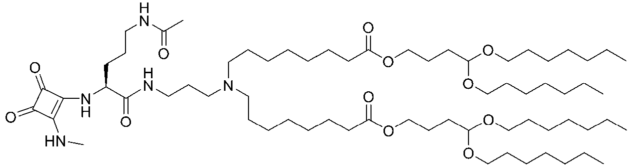 |
| 1774 | 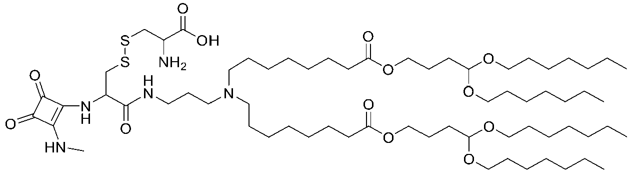 |
| 1775 | 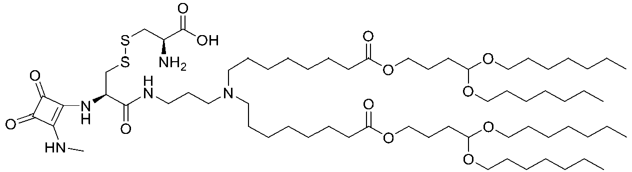 |
| 1776 | 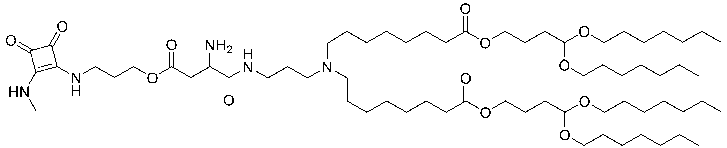 |
| 1777 | 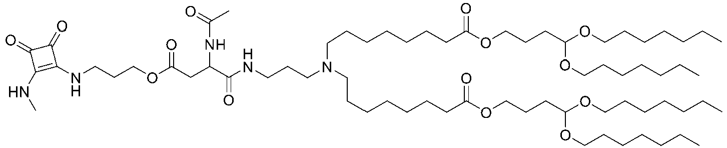 |
| 1778 | 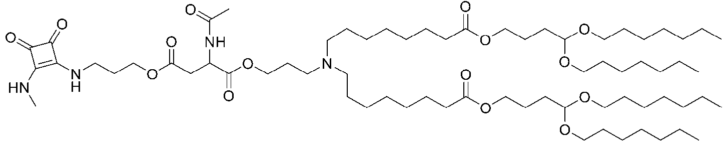 |
| 1779 | 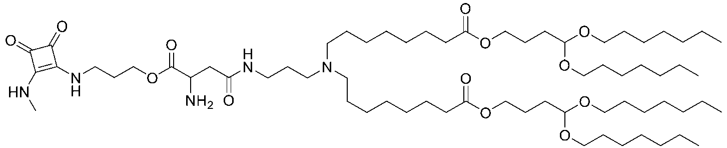 |
| 1780 | 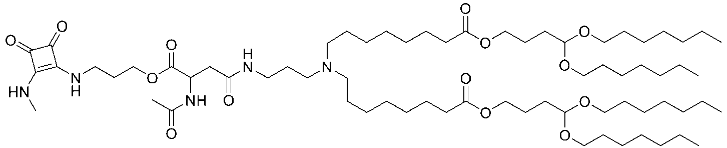 |
| 1781 | 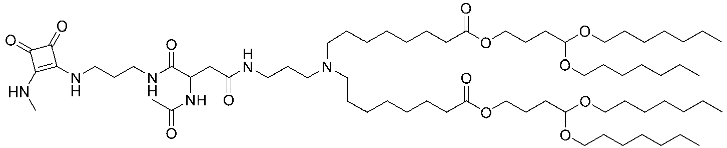 |
| 1782 | 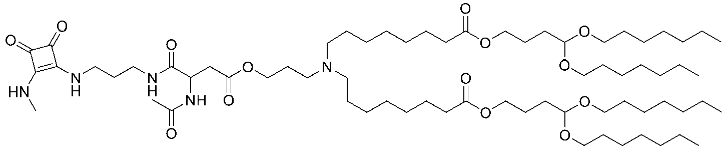 |
| 1783 | 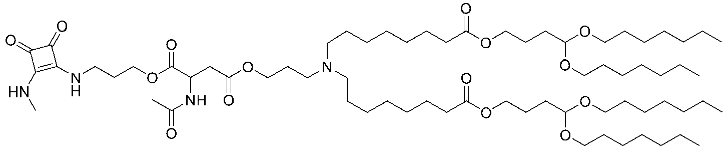 |
| 1784 | 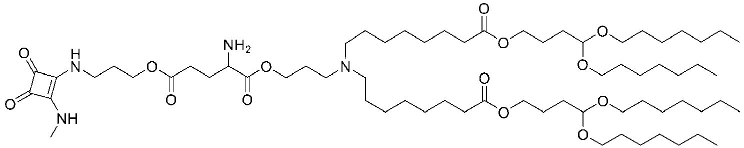 |
| 1785 | 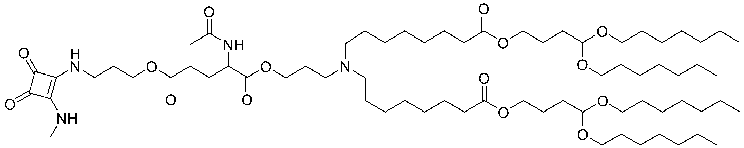 |
| 1786 | 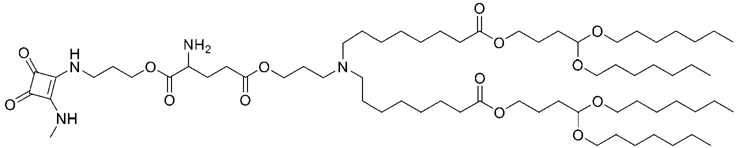 |
| 1787 | 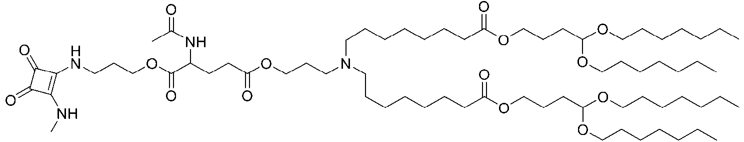 |
| 1788 | 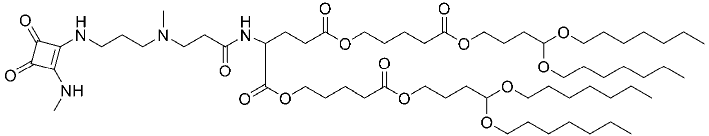 |
| 1789 | 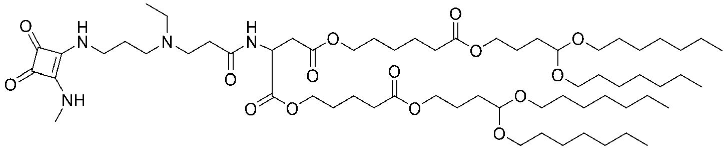 |
| 1790 | 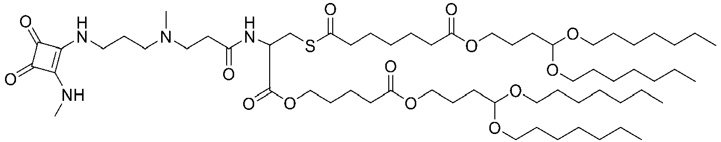 |
| 1791 | 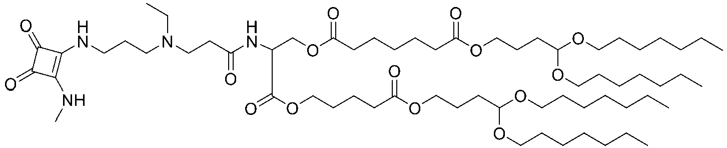 |
| 1792 | 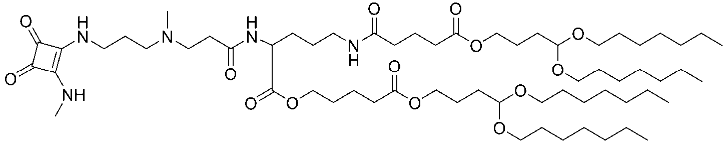 |
| 1793 | 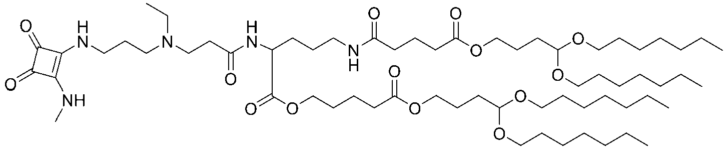 |
| 1794 | 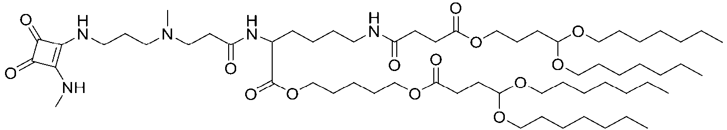 |
| 1795 | 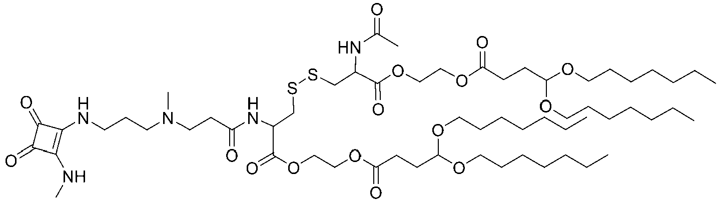 |
| 1796 | 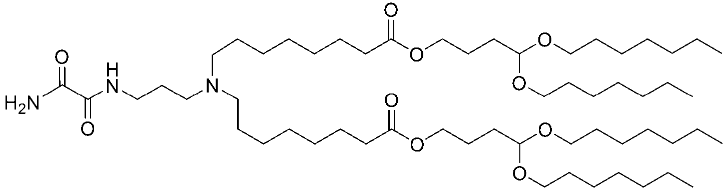 |
| 1797 | 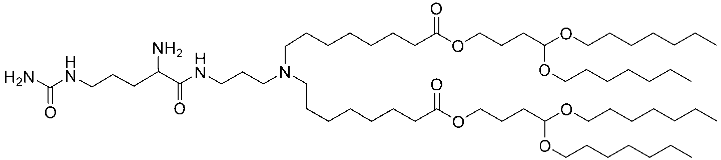 |
| 1798 | 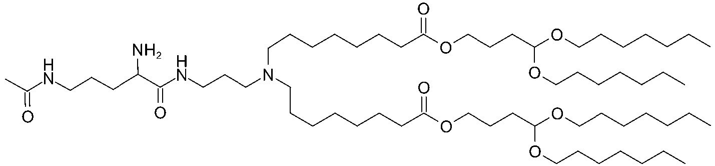 |
| 1799 | 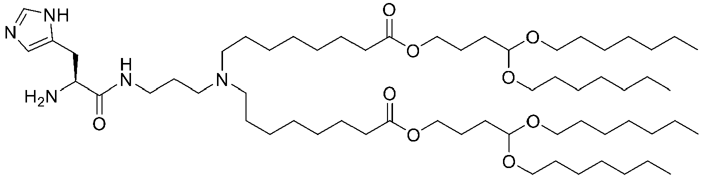 |
| 1800 | 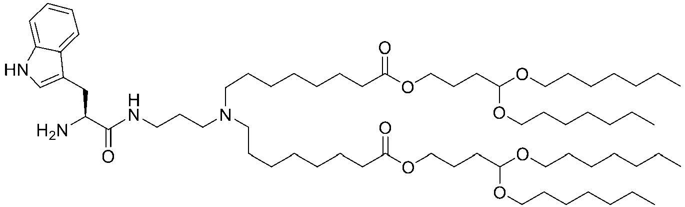 |
| 1801 | 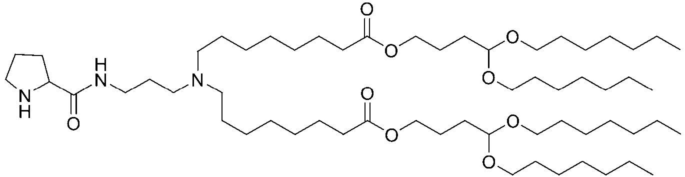 |
| 1802 | 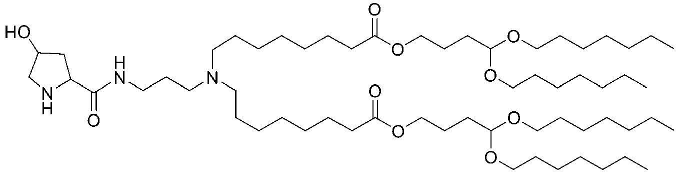 |
| 1803 | 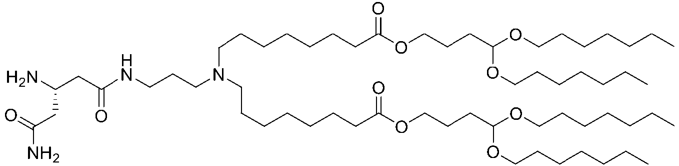 |
| 1804 | 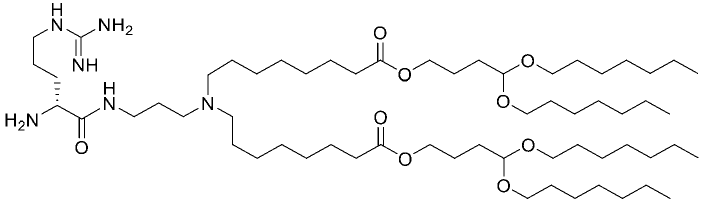 |
| 1805 | 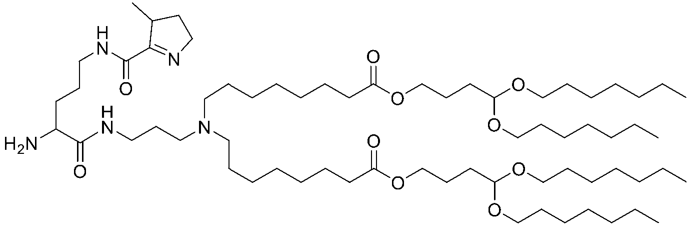 |
| 1806 | 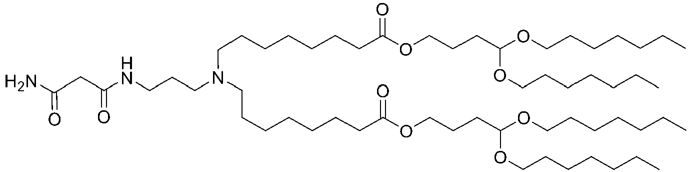 |
| 1807 | 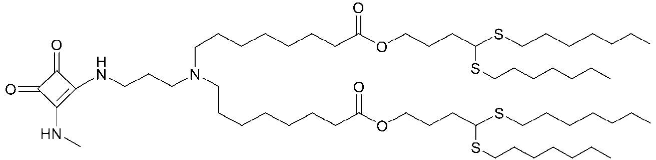 |
| 1808 | 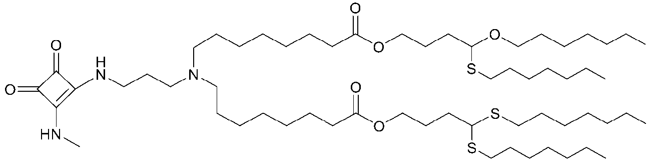 |
| 1809 | 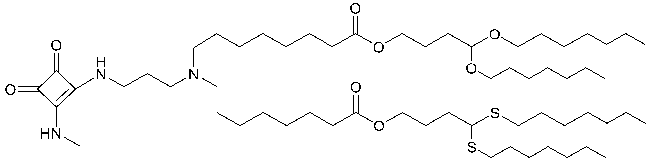 |
| 1810 | 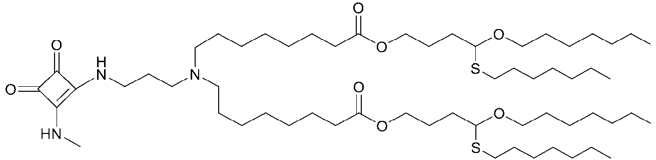 |
| 1811 | 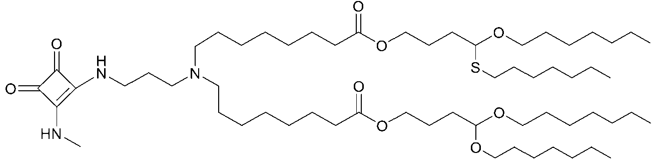 |
| 1812 | 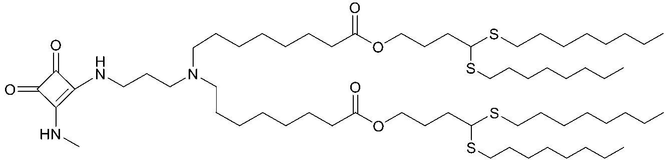 |
| 1813 | 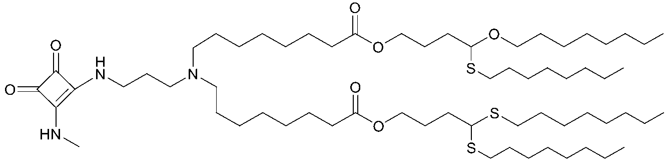 |
| 1814 | 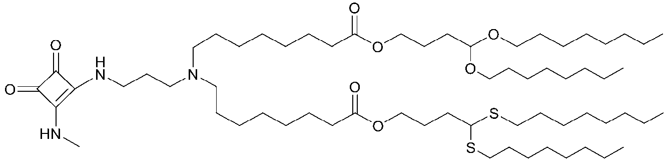 |
| 1815 | 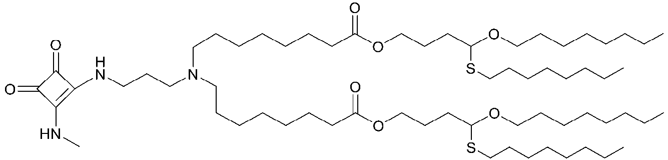 |
| 1816 | 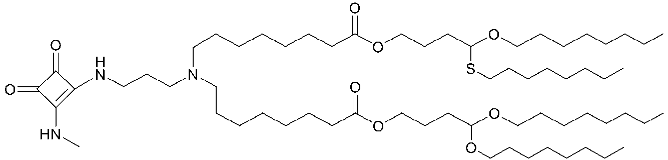 |
| 1817 | 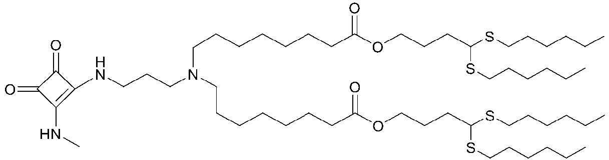 |
| 1818 | 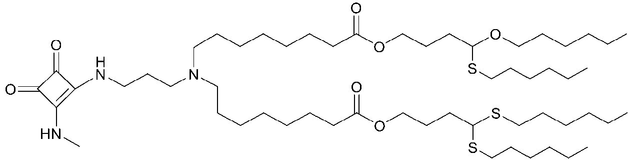 |
| 1819 | 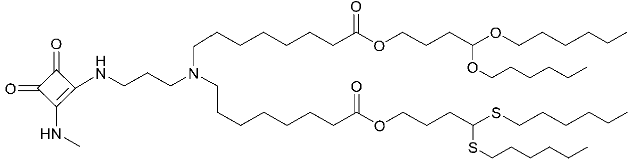 |
| 1820 | 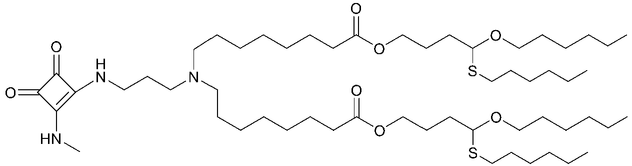 |
| 1821 | 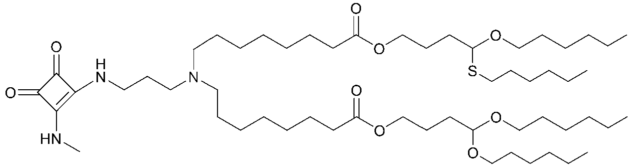 |
| 1822 | 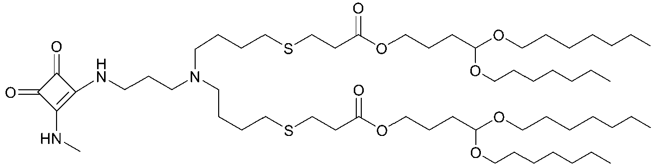 |
| 1823 | 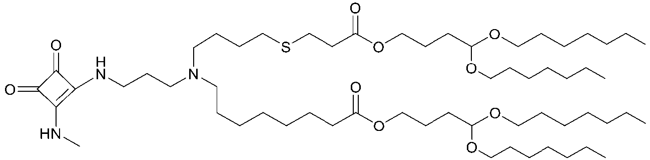 |
| 1824 | 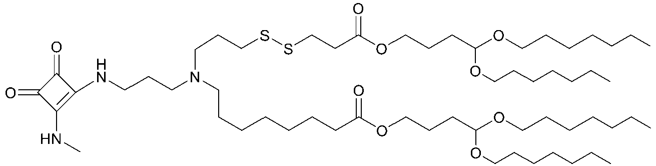 |
| 1825 | 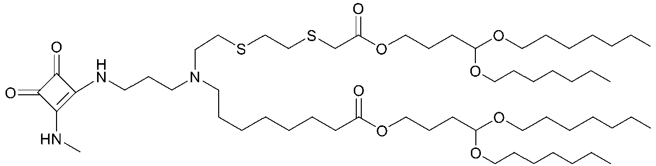 |
| 1826 | 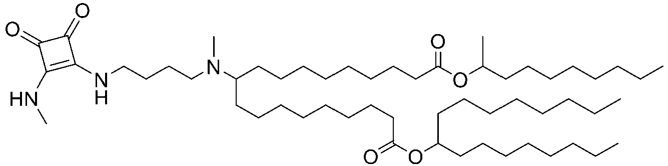 |
| 1827 | 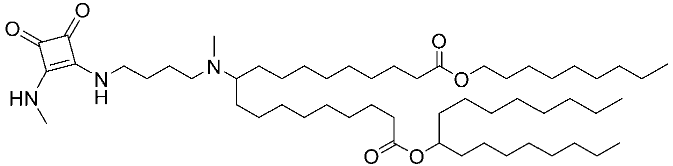 |
| 1828 | 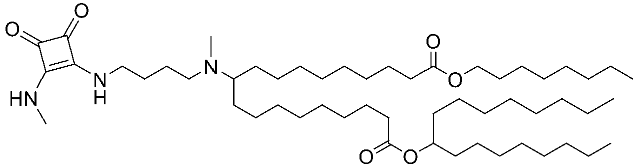 |
| 1829 | 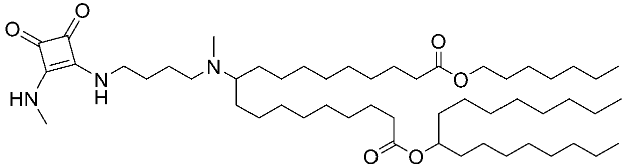 |
| 1830 | 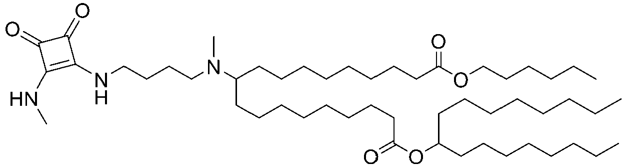 |
| 1831 | 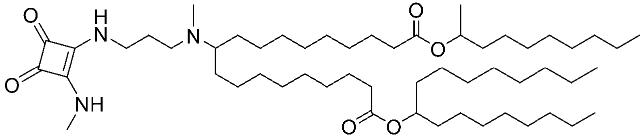 |
| 1832 | 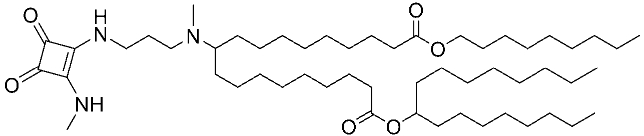 |
| 1833 | 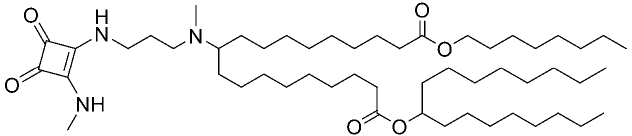 |
| 1834 | 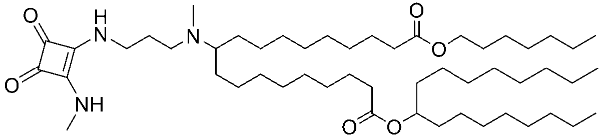 |
| 1835 | 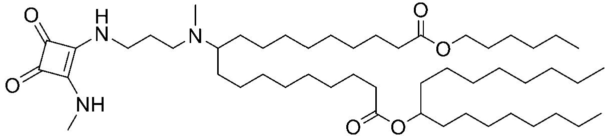 |
| 1836 | 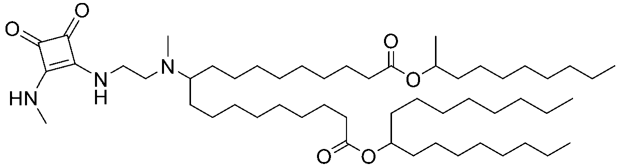 |
| 1837 | 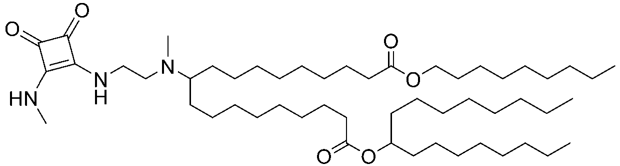 |
| 1838 | 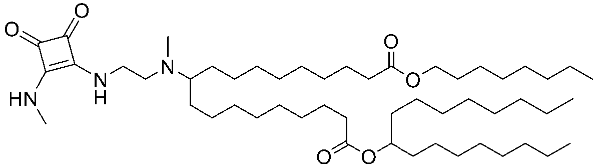 |
| 1839 | 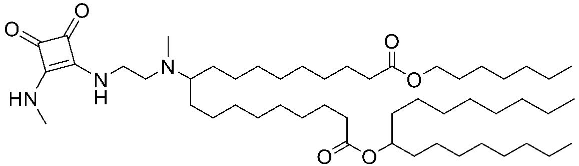 |
| 1840 | 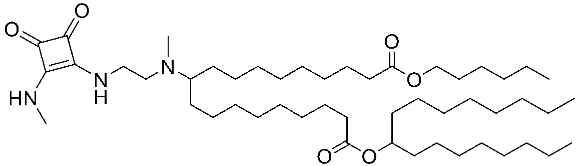 |
| 1841 | 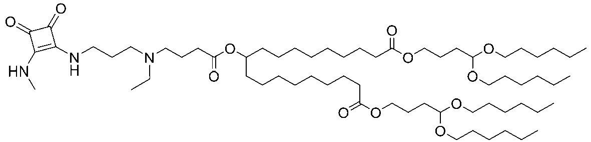 |
| 1842 | 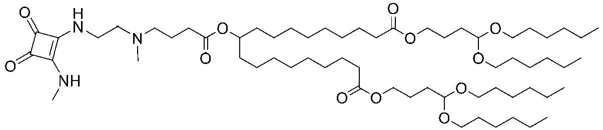 |
| 1843 | 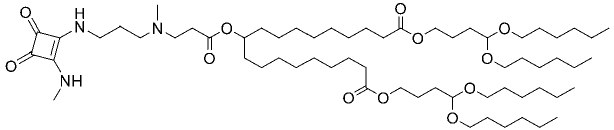 |
| 1844 | 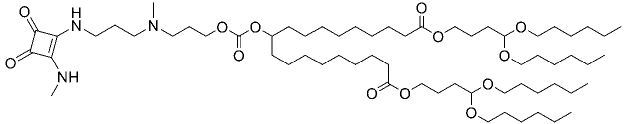 |
| 1845 | 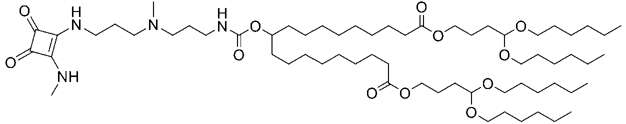 |
| 1846 | 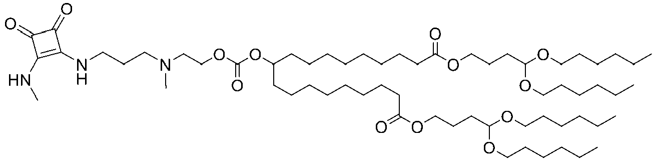 |
| 1847 | 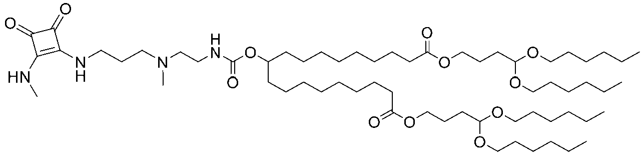 |
| 1848 | 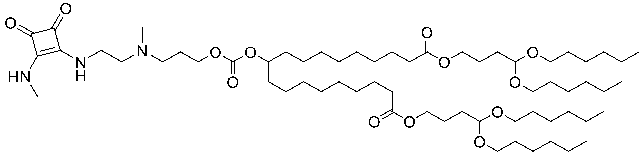 |
| 1849 | 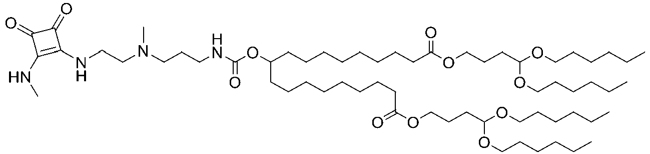 |
| 1850 | 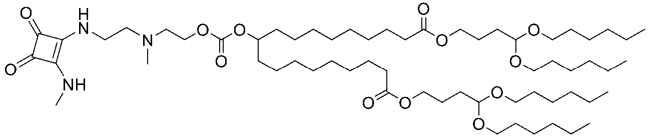 |
| 1851 | 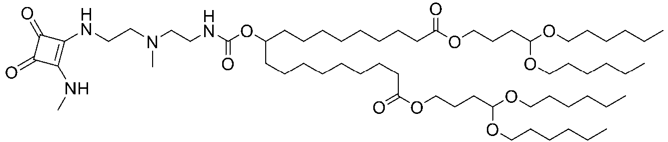 |
| 1852 | 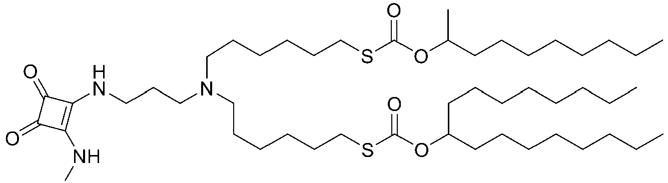 |
| 1853 | 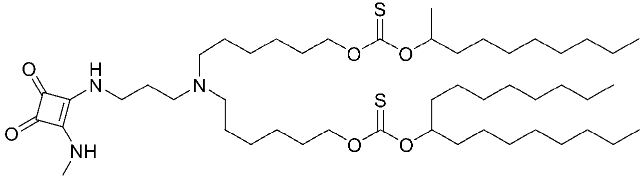 |
| 1854 | 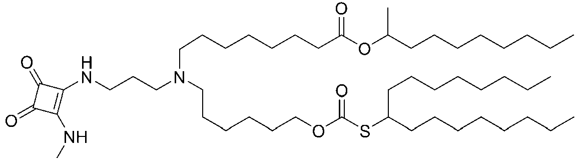 |
| 1855 | 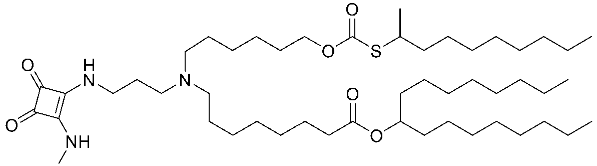 |
| 1856 | 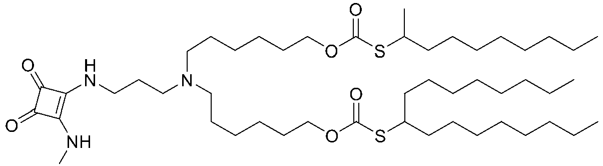 |
| 1857 | 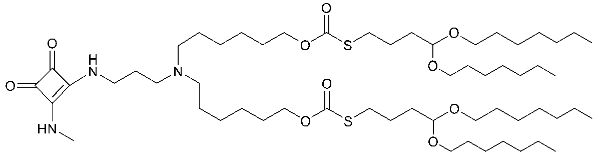 |
| 1858 | 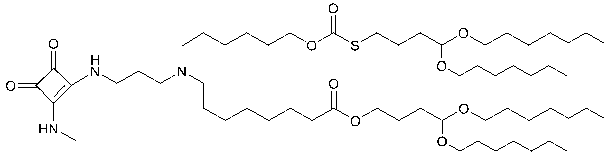 |
| 1859 | 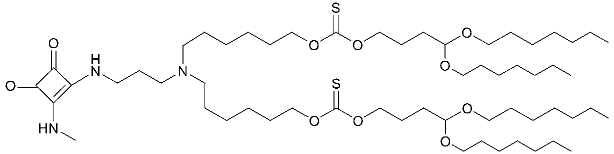 |
| 1860 | 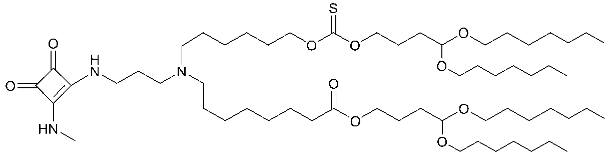 |
| 1861 | 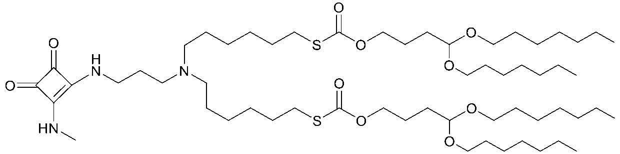 |
| 1862 | 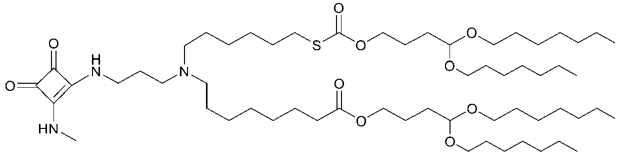 |
| 1863 | 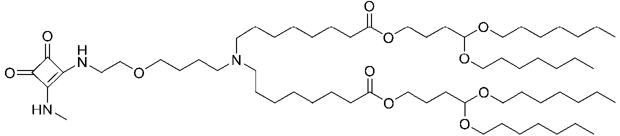 |
| 1864 | 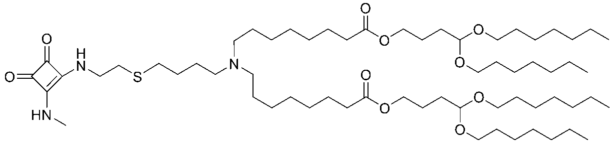 |
| 1865 | 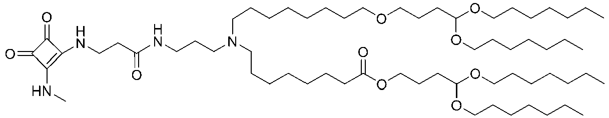 |
| 1866 | 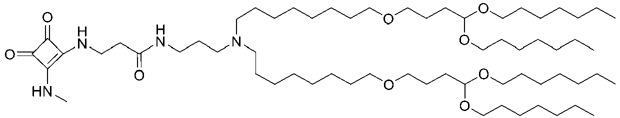 |
| 1867 | 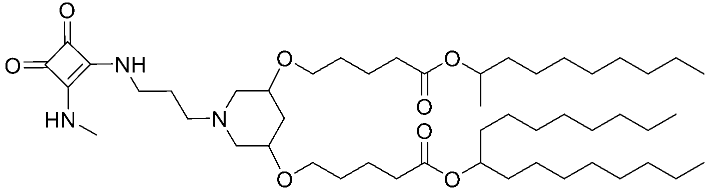 |
| 1868 | 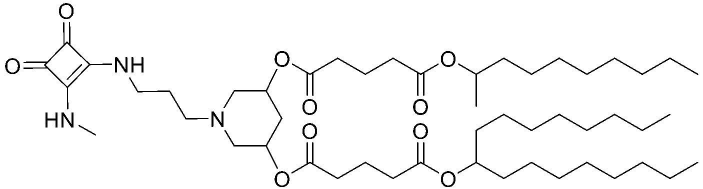 |
| 1869 | 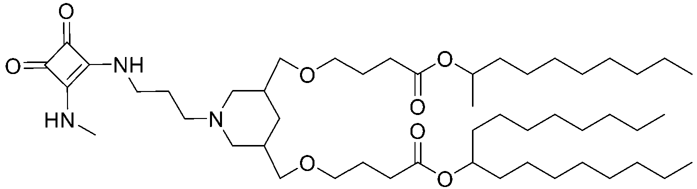 |
| 1870 | 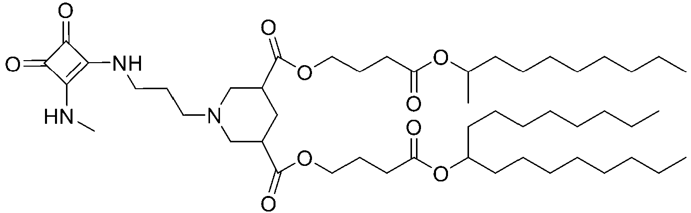 |
| 1871 | 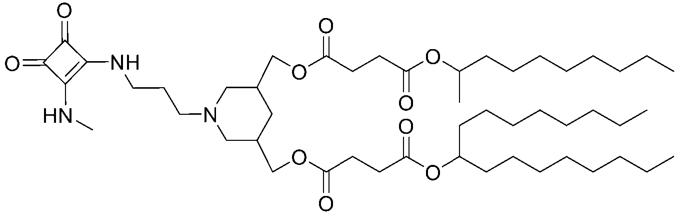 |
| 1872 | 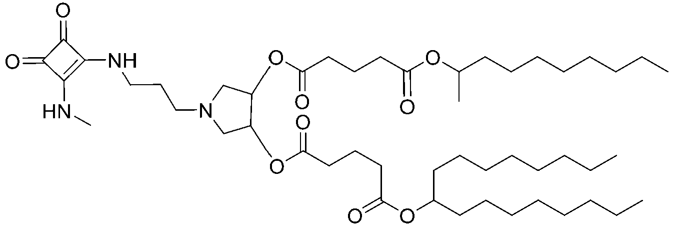 |
| 1873 | 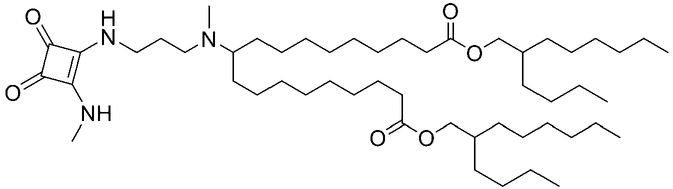 |
| 1874 | 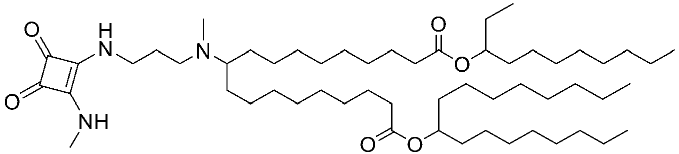 |
| 1875 | 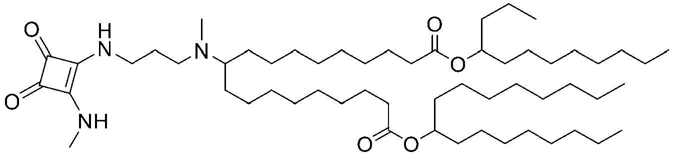 |
| 1876 | 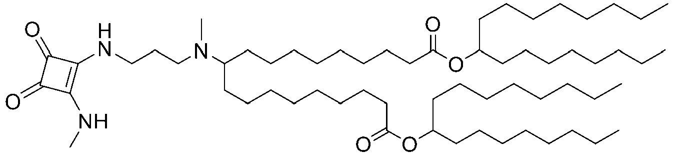 |
| 1877 | 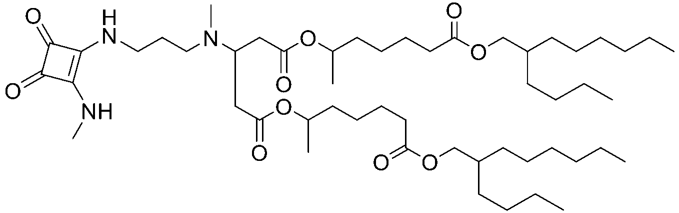 |
| 1878 | 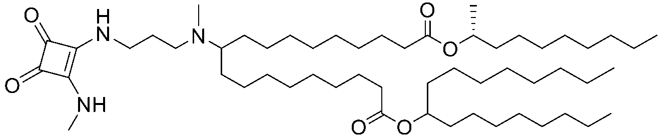 |
| 1879 | 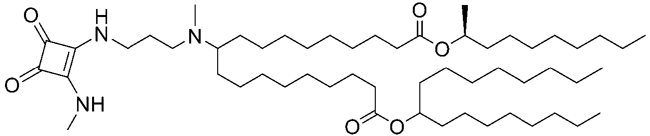 |
| 1880 | 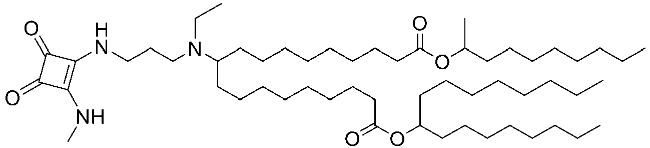 |
| 1881 | 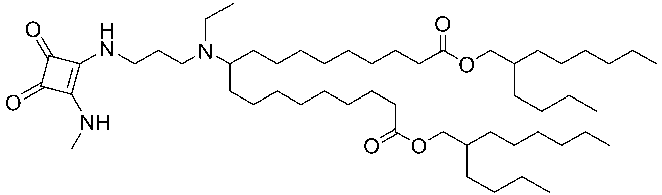 |
| 1882 | 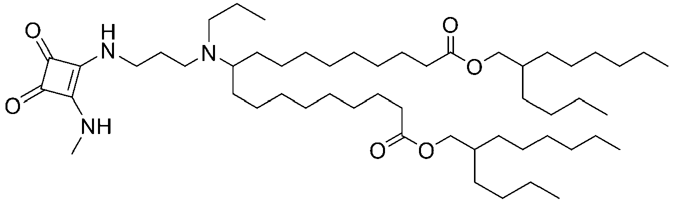 |
| 1883 | 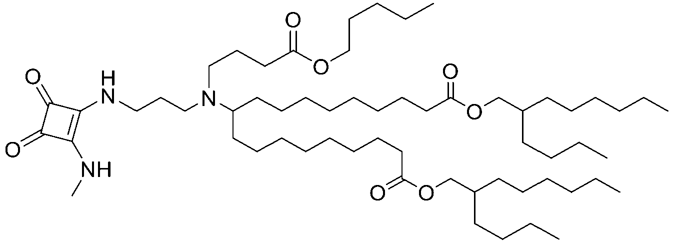 |
| 1884 | 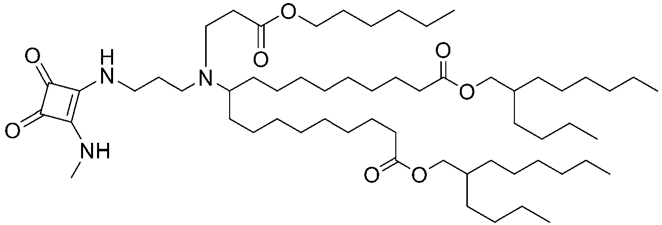 |
| 1885 | 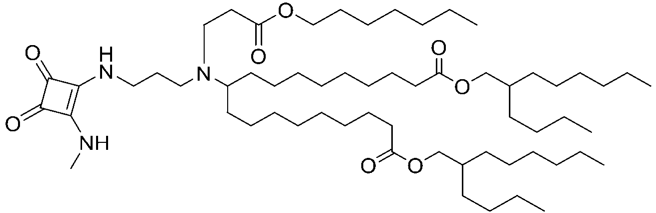 |
| 1886 | 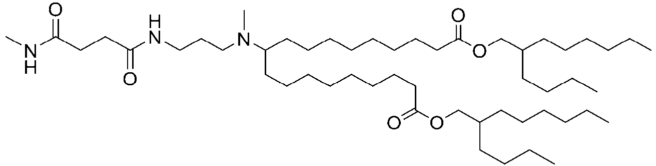 |
| 1887 | 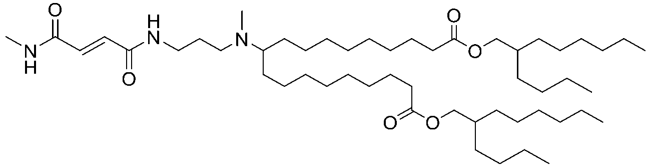 |
| 1888 | 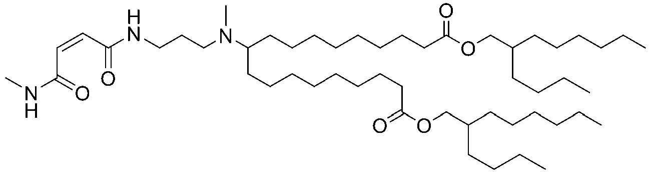 |
| 1889 | 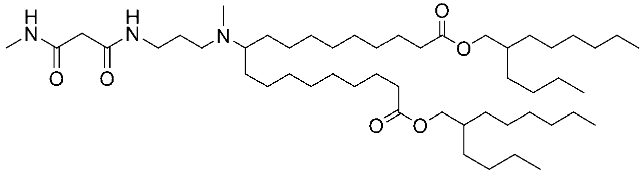 |
| 1890 | 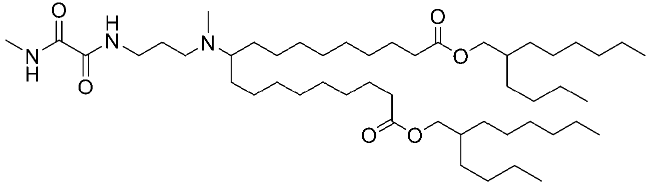 |
| 1891 | 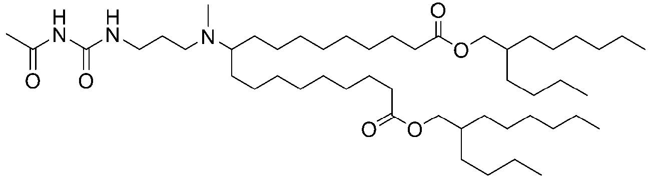 |
| 1892 | 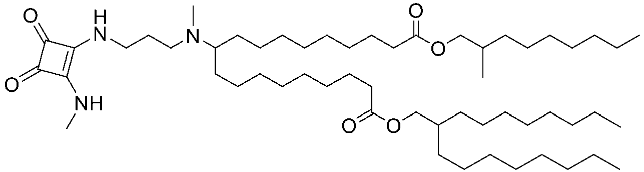 |
| 1893 | 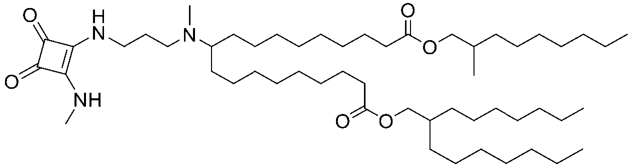 |
| 1894 | 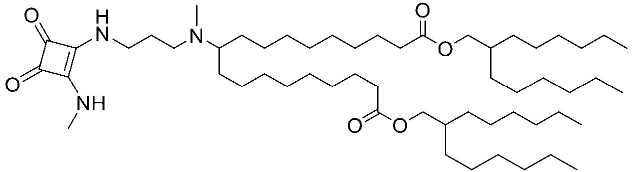 |
| 1895 | 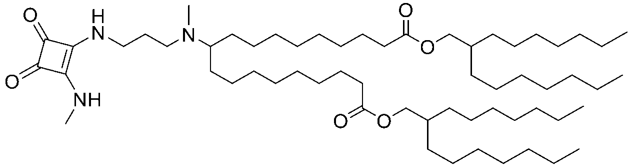 |
| 1896 | 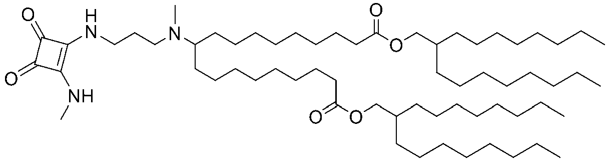 |
| 1897 | 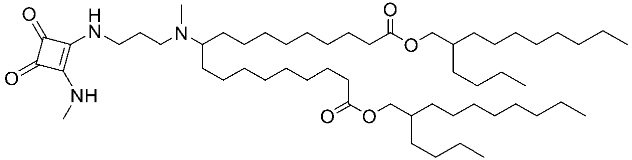 |
| 1898 | 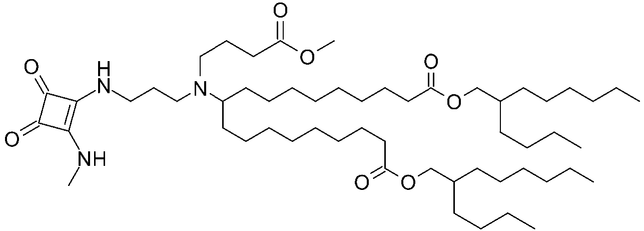 |
| 1899 | 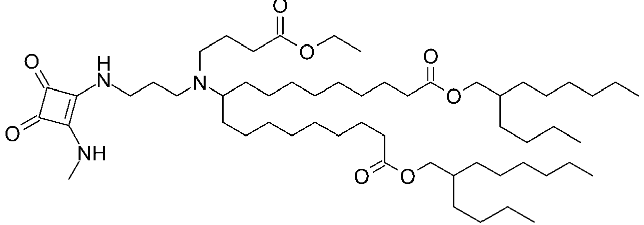 |
| 1900 | 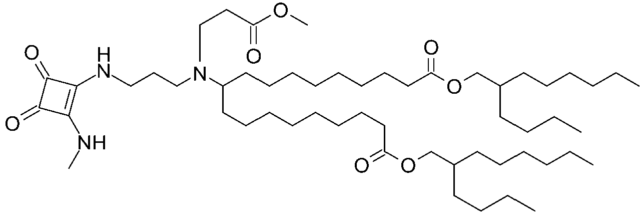 |
| 1901 | 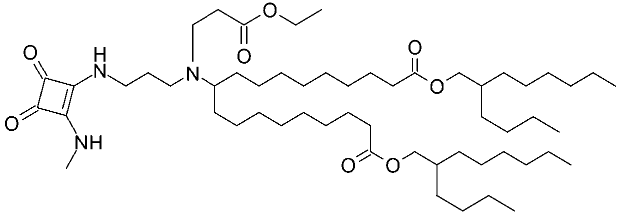 |
| 1902 | 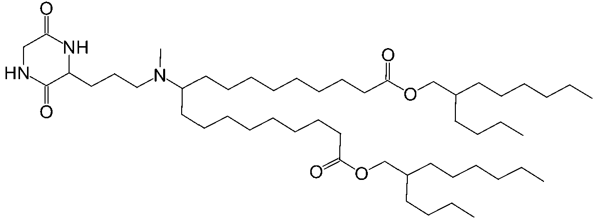 |
| 1903 | 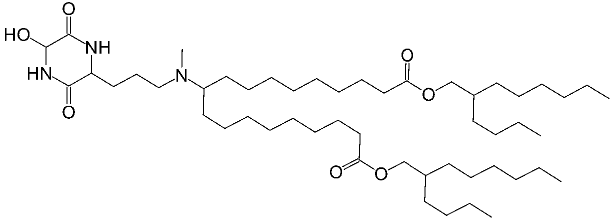 |
| 1904 | 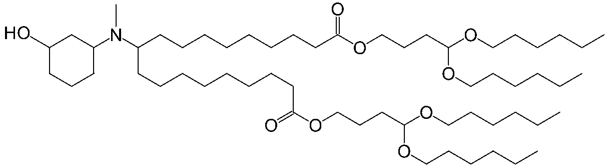 |
| 1905 | 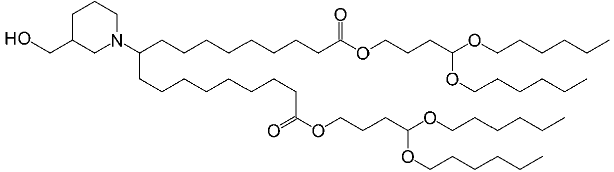 |
| 1906 | 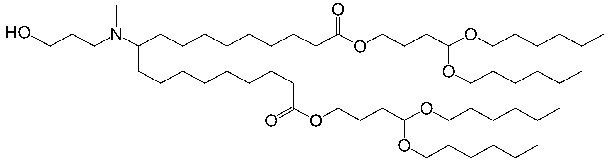 |
| 1907 | 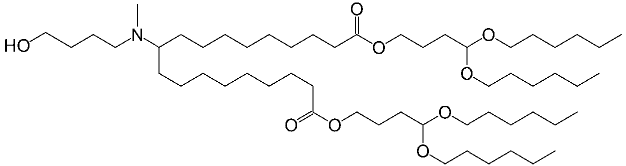 |
| 1908 | 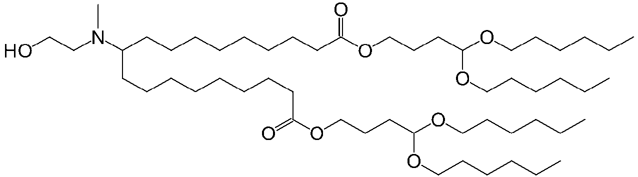 |
| 1909 | 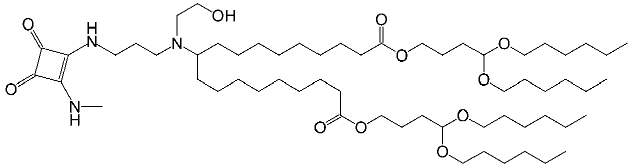 |
| 1910 | 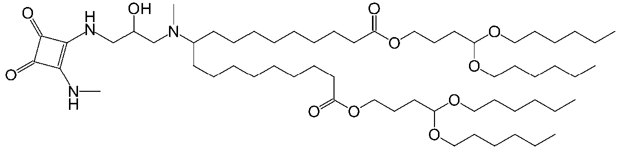 |
| 1911 | 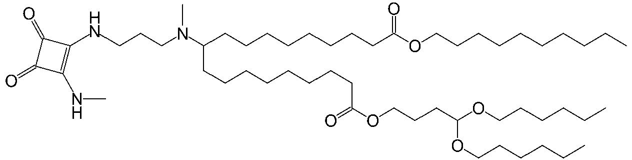 |
| 1912 | 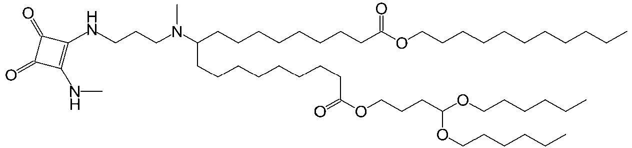 |
| 1913 | 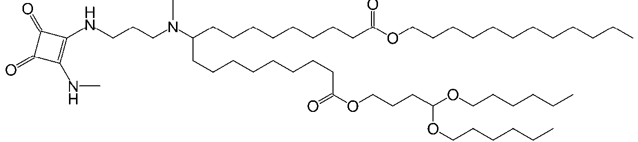 |
| 1914 | 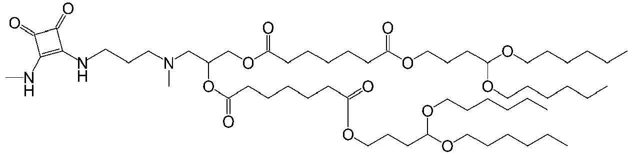 |
| 1915 | 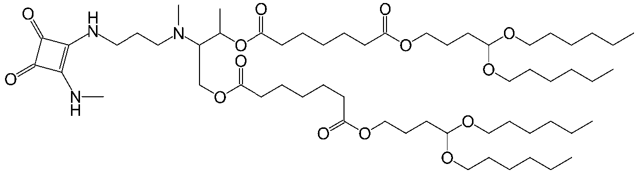 |
| 1916 | 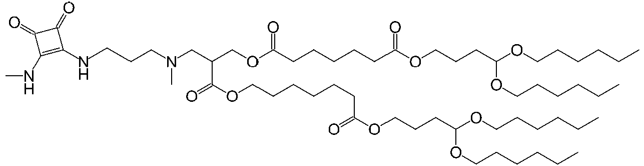 |
| 1917 | 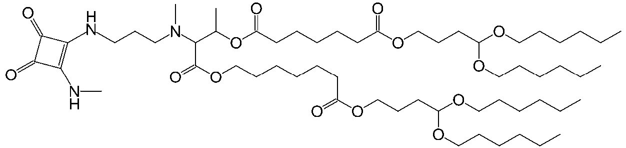 |
| 1918 | 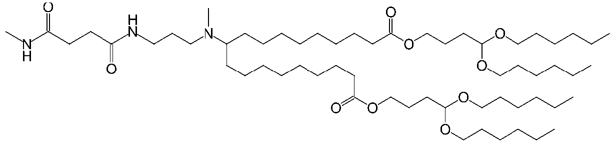 |
| 1919 | 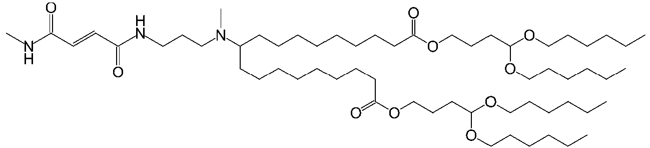 |
| 1920 | 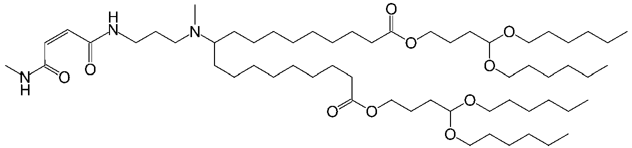 |
| 1921 | 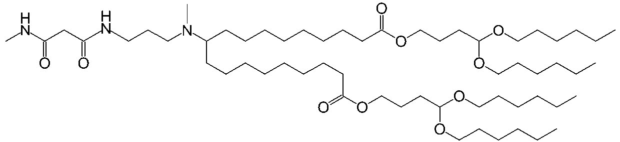 |
| 1922 | 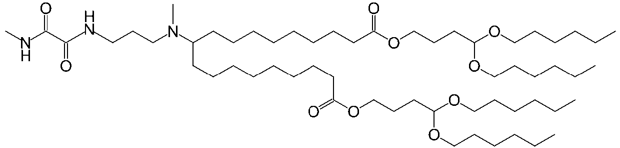 |
| 1923 | 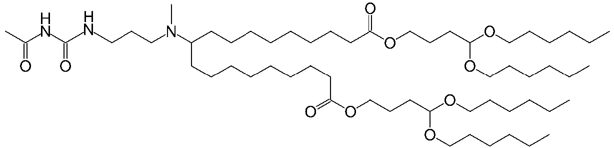 |
| 1924 | 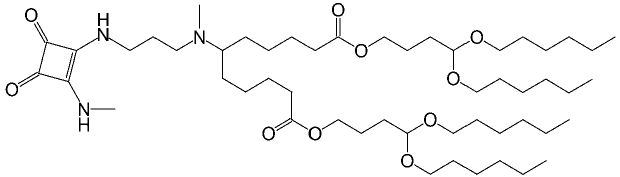 |
| 1925 | 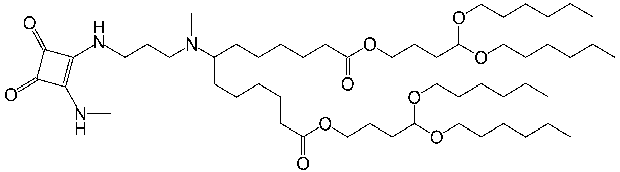 |
| 1926 | 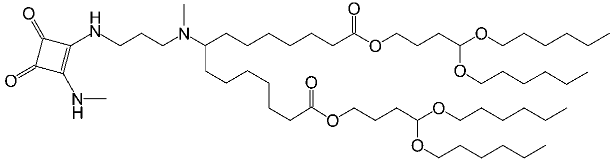 |
| 1927 | 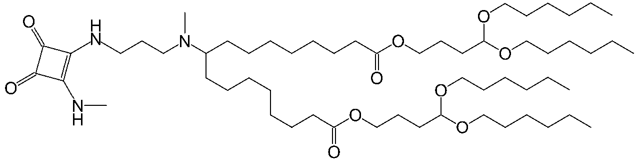 |
| 1928 | 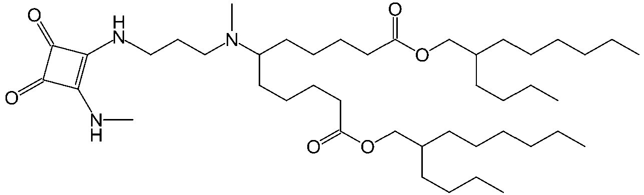 |
| 1929 | 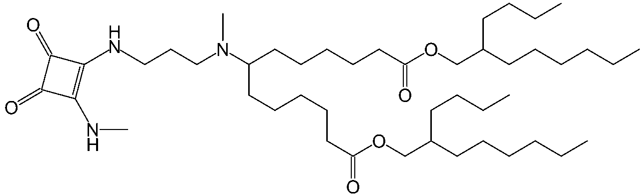 |
| 1930 | 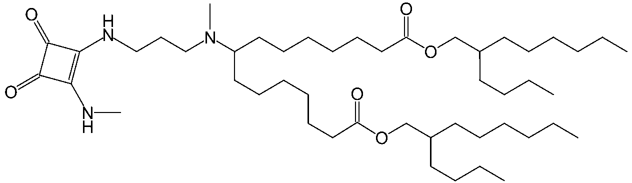 |
| 1931 | 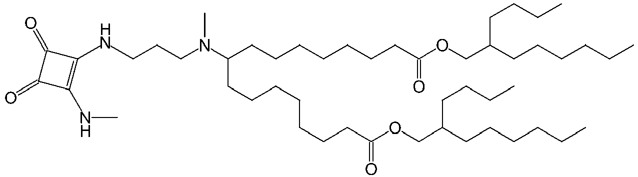 |
| 1932 | 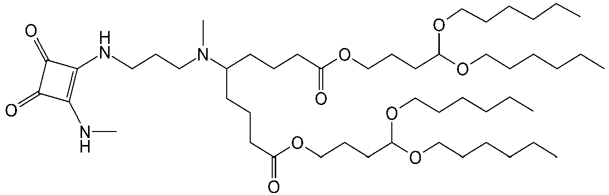 |
| 1933 | 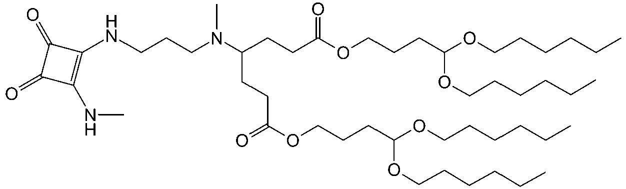 |
| 1934 | 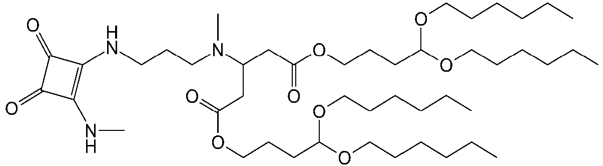 |
| 1935 | 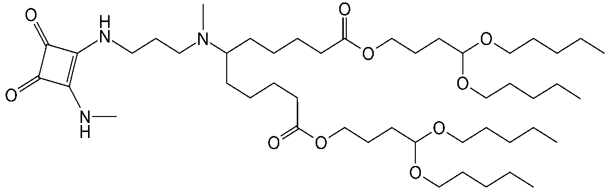 |
| 1936 | 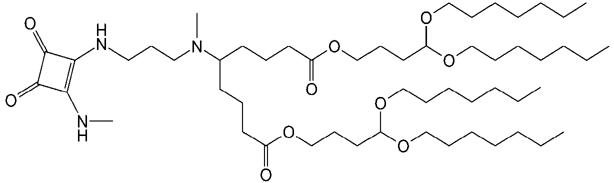 |
| 1937 | 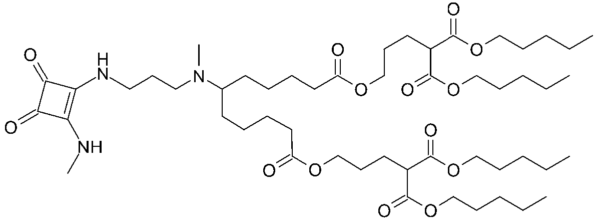 |
| 1938 | 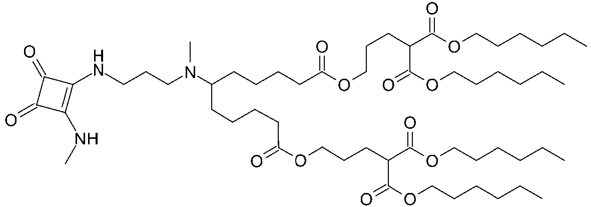 |
| 1939 | 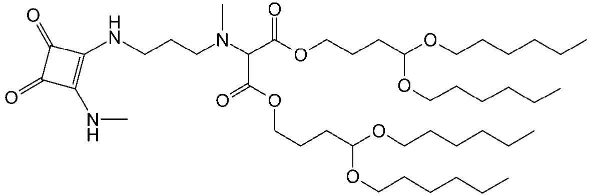 |
| 1940 | 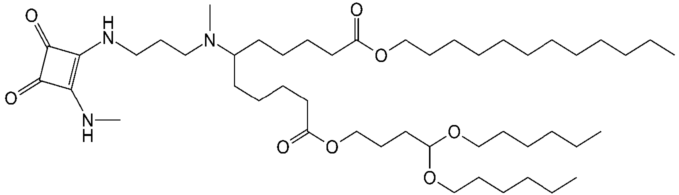 |
| 1941 | 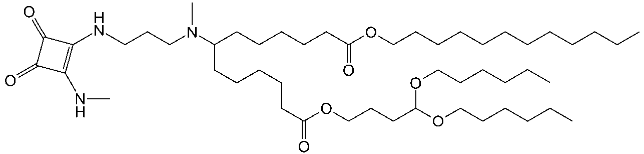 |
| 1942 | 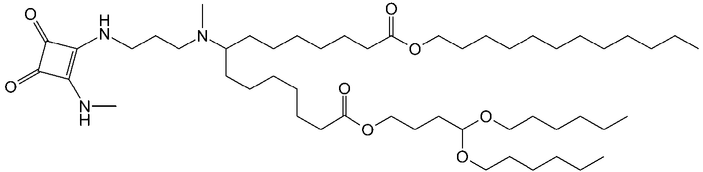 |
| 1943 | 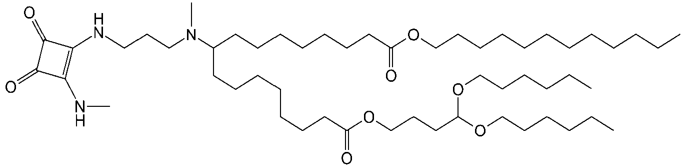 |
| 1944 | 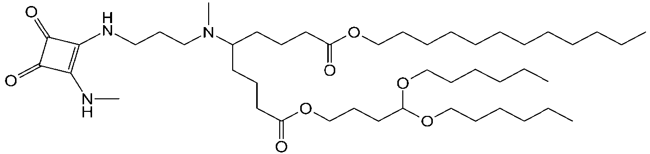 |
| 1945 | 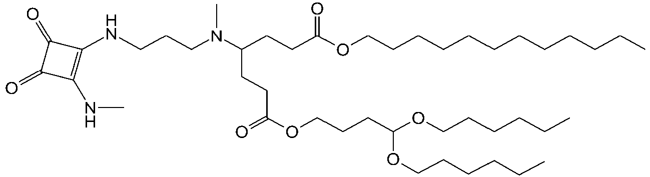 |
| 1946 | 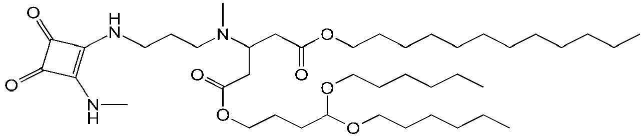 |
| 1947 | 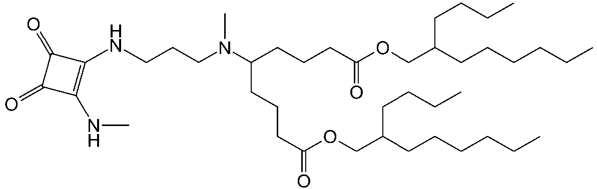 |
| 1948 | 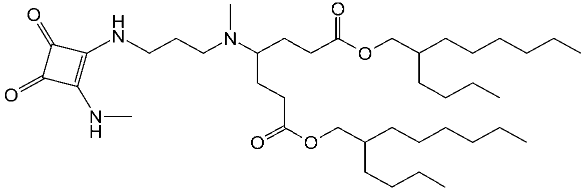 |
| 1949 | 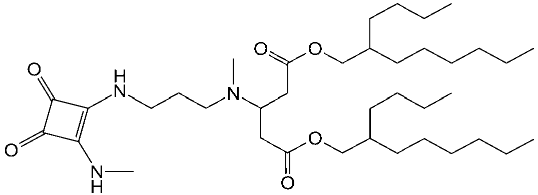 |
| 1950 | 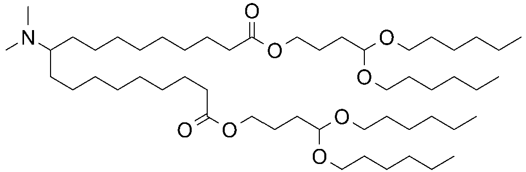 |
| 2047 | 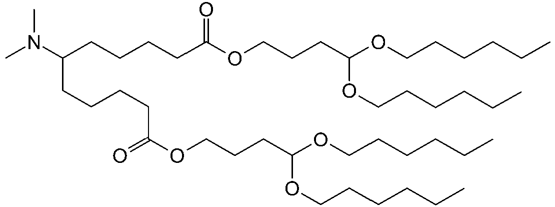 |
| 2048 | 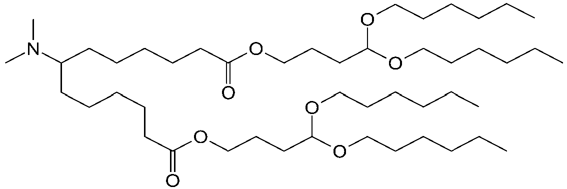 |
| 2049 | 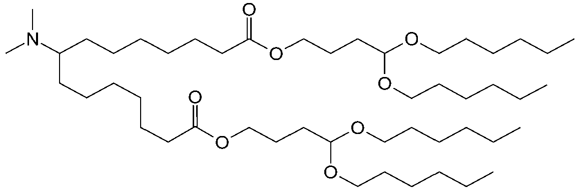 |
| 2050 | 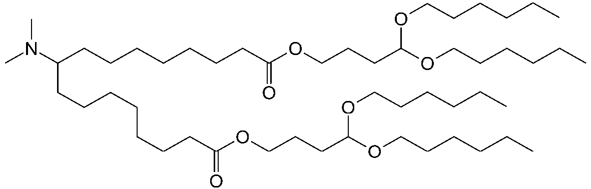 |
| 2058 | 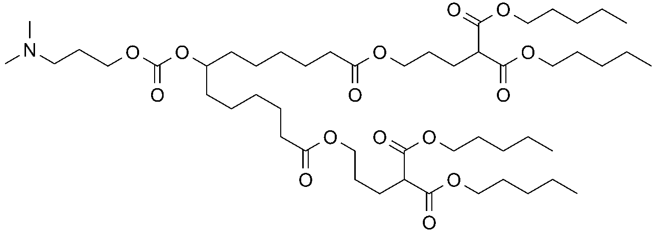 |
| 2059 | 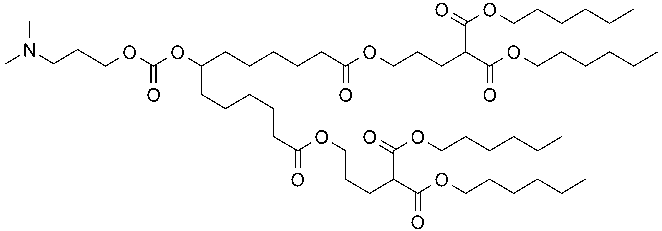 |
| 2060 | 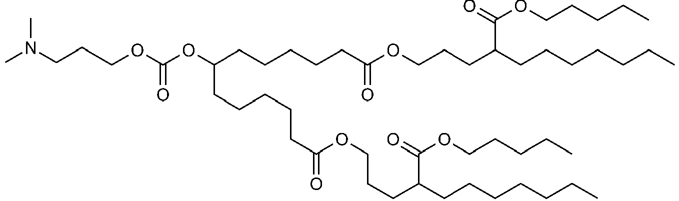 |

The amino lipid compounds of the present disclosure all have a hydrophobic characteristic due to the presence of long nonpolar residues and simultaneously a hydrophilic characteristic due to the amino group. Due to this amphiphilic characteristic, the amino lipid compounds of the present disclosure can be used to form a lipid nanoparticle, such as a lipid bilayer, a micelle, a liposome, and the like. In the context of the present disclosure, the term "lipid nanoparticle" means a nanometer-sized material produced by introducing an amino lipid compound into an aqueous solution. The particle is in particular a lipid nanoparticle, a lipid bilayer vesicle (a liposome), a multilayer vesicle or a micelle.

In some embodiments, the lipid nanoparticle is a liposome containing an amino lipid compound of the present disclosure. Within the scope of the present disclosure, a liposome is a microvesicle consisting of a bilayer of lipid amphipathic molecules encapsulating an aqueous compartment.

Liposome formation is not a spontaneous process. When a lipid is introduced into water, a lipid vesicle is firstly formed, thus forming a bilayer or a series of bilayers, each of which is separated by a water molecule. Liposomes can be formed by sonicating lipid vesicles in water.

Within the scope of the present disclosure, the term "lipid bilayer" means a thin film formed by two layers of lipid molecules. The term "micelle" means an aggregate of surfactant molecules dispersed in a liquid colloid. Typical micelles in an aqueous solution form aggregates with the hydrophilic head region upon contact with water, chelating the hydrophobic single tail region at the center of the micelle.

In one aspect, the present disclosure provides a use of the amino lipid compound of the present disclosure for the manufacture of a vehicle for an active ingredient. In some embodiments, the vehicle is in the form of a lipid nanoparticle, such as a lipid bilayer, micelle, liposome.

### Lipid nanoparticle

In another aspect, the present disclosure provides a lipid nanoparticle containing the amino lipid compound of the present disclosure.

In some embodiments, the lipid nanoparticle further contains one or more of a helper lipid, a structural lipid, and a PEG-lipid (polyethylene glycol-lipid).

In some further embodiments, the lipid nanoparticle further contains the helper lipid, the structural lipid, and the PEG-lipid.

In some embodiments, the lipid nanoparticle comprises the amino lipid compound in an amount (molar percent) of about 25.0% to 75.0%, such as about 25.0%-28.0%, 28.0%-32.0%, 32.0%-35.0%, 35.0%-40.0%, 40.0%-42.0%, 42.0%-45.0%, 45.0%-46.3%, 46.3%-48.0%, 48.0%-49.5%, 49.5%-50.0%, 50.0%-55.0%, 55.0%-60.0%, 60.0%-65.0% or 65.0%-75.0%, based on the total amount of the amino lipid compound, the helper lipid, the structural lipid, and the PEG-lipid.

In some embodiments, the lipid nanoparticle comprises the helper lipid in an amount (molar percent) of about 5.0% to 45.0%, such as about 5.0%-9.0%, 9.0%-9.4%, 9.4%-10.0%, 10.0%-10.5%, 10.5%-11.0%, 11.0%-15.0%, 15.0%-16.0%, 16.0%-18.0%, 18.0%-20.0%, 20.0%-25.0%, 25.0%-33.5%, 33.5%-37.0%, 37.0%-40.0%, 40.0%-42.0%, or 42.0%-45.0%, based on the total amount of the amino lipid compound, the helper lipid, the structural lipid, and the PEG-lipid.

In some embodiments, the lipid nanoparticle comprises the structural lipid in an amount (molar percent) of about 0.0% to 50.0%, such as about 0.0%-10.0%, 10.0%-15.5%, 15.5%-18.5%, 18.5%-22.5%, 22.5%-23.5%, 23.5%-28.5%, 28.5%-33.5%, 33.5%-35.0%, 35.0%-36.5%, 36.5%-38.0%, 38.0%-38.5%, 38.5%-39.0%, 39.0%-39.5%, 39.5%-40.5%, 40.5%-41.5%, 41.5%-42.5%, 42.5%-42.7%, 42.7%-43.0%, 43.0%-43.5%, 43.5%-45.0%, 45.0%-46.5%, 46.5%-48.5%, or 46.5%-50.0%, based on the total amount of the amino lipid compound, the helper lipid, the structural lipid, and the PEG-lipid.

In some embodiments, the lipid nanoparticle comprises the PEG-lipid in an amount (molar percent) of about 0.5% to 5.0%, such as about 0.5%-1.0%, 1.0%-1.5%, 1.5%-1.6%, 1.6%-2.0%, 2.0%-2.5%, 2.5%-3.0%, 3.0%-3.5%, 3.5%-4.0%, 4.0%-4.5%, or 4.5%-5.0%, based on the total amount of the amino lipid compound, the helper lipid, the structural lipid, and the PEG-lipid.

In some embodiments as described above, the helper lipid is a phospholipid. The phospholipid is generally semi-synthetic and may also be of natural origin or chemically modified. The phospholipid includes, but is not limited to, DSPC (distearoyl phosphatidylcholine), DOPE (dioleoyl phosphatidylethanolamine), DOPC (dioleoyl lecithin), DOPS (dioleoyl phosphatidylserine), DSPG (1,2-distearoyl-sn-glycero-3-phospho-(1'-rac-glycerol)), DPPG (dipalmitoyl phosphatidylglycerol), DPPC (dipalmitoyl phosphatidylcholine), DGTS (1,2-dipalmitoyl-sn-glycero-3-O-4'-(N,N,N-trimethyl) homoserine), lysophospholipid, and the like. In some embodiments, the helper lipid is one or more selected from the group consisting of DSPC, DOPE, DOPC, and DOPS. In some embodiments, the helper lipid is DSPC and/or DOPE.

In some embodiments, the structural lipid is a sterol, including but not limited to, cholesterol, cholesterol esters, steroid hormones, steroid vitamins, bile acid, cholesterin, ergosterol, β-sitosterol, oxidized cholesterol derivatives, and the like. In some embodiments, the structural lipid is at least one selected from cholesterol, cholesteryl esters, steroid hormones, steroid vitamins, and bile acid. In some embodiments, the structural lipid is cholesterol. In some embodiments, the structural lipid is high purity cholesterol, particularly injection grade high purity cholesterol, such as CHO-HP (manufactured by AVT).

As used herein, the term PEG-lipid (polyethylene glycol-lipid) is a conjugate of polyethylene glycol and a lipid structure. In some embodiments, the PEG-lipid is selected from PEG-DMG and PEG-distearoyl phosphatidylethanolamine (PEG-DSPE), preferably PEG-DMG. In some embodiments, the PEG-DMG is a polyethylene glycol (PEG) derivative of 1,2-dimyristoyl-sn-glycerol. In some embodiments, the PEG has an average molecular weight of about 2,000 to 5,000. In some embodiments, the PEG has an average molecular weight of about 2,000.

In some embodiments as described above, in the lipid nanoparticle, the molar ratio of the amino lipid compound of the present disclosure: helper lipid: structural lipid: PEG-lipid is about 45:10:42.5:2.5, or 45:11:41.5:2.5, or 42.0:10.5:45.0:2.5, or 42.0:16.0:39.5:2.5, or 40.0:16.0:41.5:2.5, or 40.0:18.0:39.5:2.5, or 35.0:16.0:46.5:2.5, or 35.0:25.0:36.5:3.5, or 28.0:33.5:35.0:3.5, or 32.0:37.0:40.5:0.5, or 35.0:40.0:22.5:2.5, or 40.0:42.0:15.5:2.5, or 40.0:20.0:38.5:1.5, or 45.0:15.0:38.5:1.5, or 55.0:5.0:38.5:1.5, or 60.0:5.0:33.5:1.5, or 45.0:20.0:33.5:1.5, or 50.0:20.0:28.5:1.5, or 55.0:20.0:23.5:1.5, or 60.0:20.0:18.5:1.5, or 40.0:15.0:43.5:1.5, or 50.0:15.0:33.5:1.5, or 55.0:15.0:28.5:1.5, or 60.0:15.0:23.5:1.5, or 40.0:10.0:48.5:1.5, or 45.0:10.0:43.5:1.5, or 55.0:10.0:33.5:1.5, or 40.0:5.0:53.5:1.5, or 45.0:5.0:48.5:1.5, or 50.0:5.0:43.5:1.5. In some such embodiments, the helper lipid is DOPE, and the structural lipid is CHO-HP.

In other embodiments as described above, in the lipid nanoparticle, the molar ratio of the amino lipid compound of the present disclosure: helper lipid: structural lipid: PEG-lipid is about 50.0:10.0:38.5:1.5, or 50.0:9.0:38.0:3.0, or 49.5:10.0:39.0:1.5, or 48.0:10.0:40.5:1.5, or 46.3:9.4:42.7:1.6, or 45.0:9.0:43.0:3.0, or 45.0:11.0:41.5:2.5, or 42.0:10.5:45.0:2.5, or 42.0:16.0:39.5:2.5, or 40.0:16.0:41.5:2.5, or 40.0:18.0:39.5:2.5, or 35.0:40.0:22.5:2.5, or 40.0:20.0:38.5:1.5, or 45.0:15.0:38.5:1.5, or 55.0:5.0:38.5:1.5, or 60.0:5.0:33.5:1.5, or 45.0:20.0:33.5:1.5, or 50.0:20.0:28.5:1.5, or 55.0:20.0:23.5:1.5, or 60.0:20.0:18.5:1.5, or 40.0:15.0:43.5:1.5, or 50.0:15.0:33.5:1.5, or 55.0:15.0:28.5:1.5, or 60.0:15.0:23.5:1.5, or 40.0:10.0:48.5:1.5, or 45.0:10.0:43.5:1.5, or 55.0:10.0:33.5:1.5, or 40.0:5.0:53.5:1.5, or 45.0:5.0:48.5:1.5, or 50.0:5.0:43.5:1.5. In some such embodiments, the helper lipid is DSPC, and the structural lipid is CHO-HP.

In some embodiments, the lipid nanoparticle has the amino lipid compound of the present disclosure, helper lipid, structural lipid, and PEG-lipid in molar percent (%) as shown in Nos. 1-24 in Table 1 below, based on the total amount of the amino lipid compound, the helper lipid, the structural lipid, and the PEG-lipid:

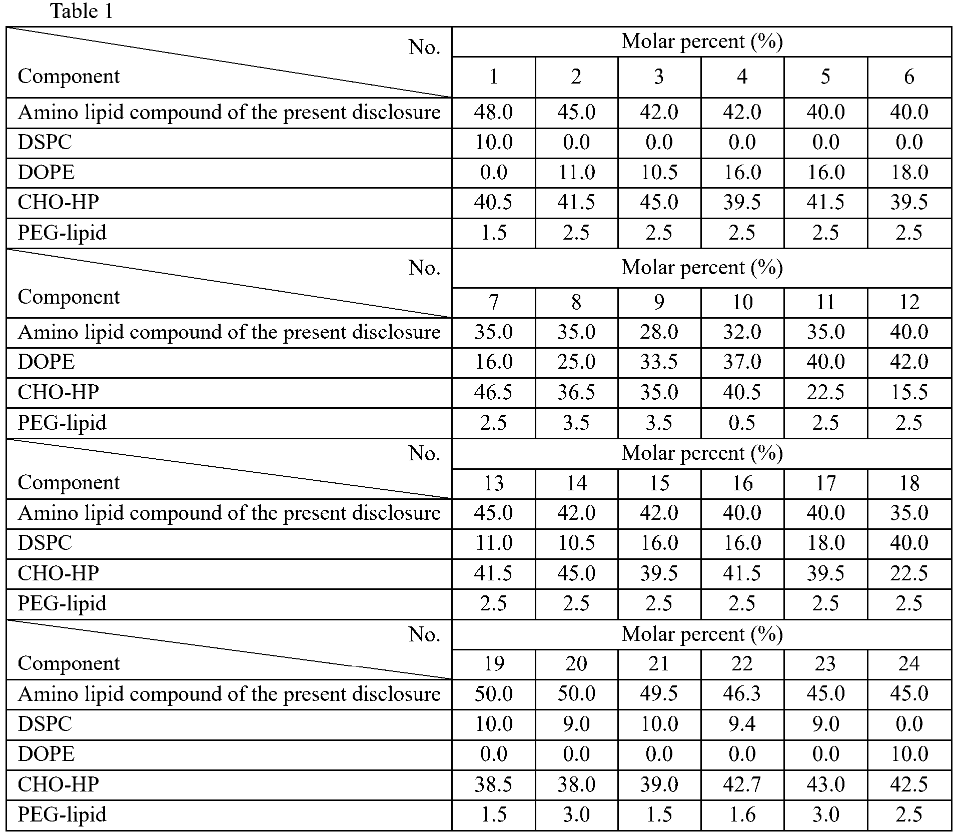

In some embodiments, the lipid nanoparticle has the amino lipid compound of the present disclosure, helper lipid, structural lipid, and PEG-lipid in molar percent (%) as shown in Nos. 25-42 in Table 2 below, based on the total amount of the amino lipid compound, the helper lipid, the structural lipid, and the PEG-lipid:

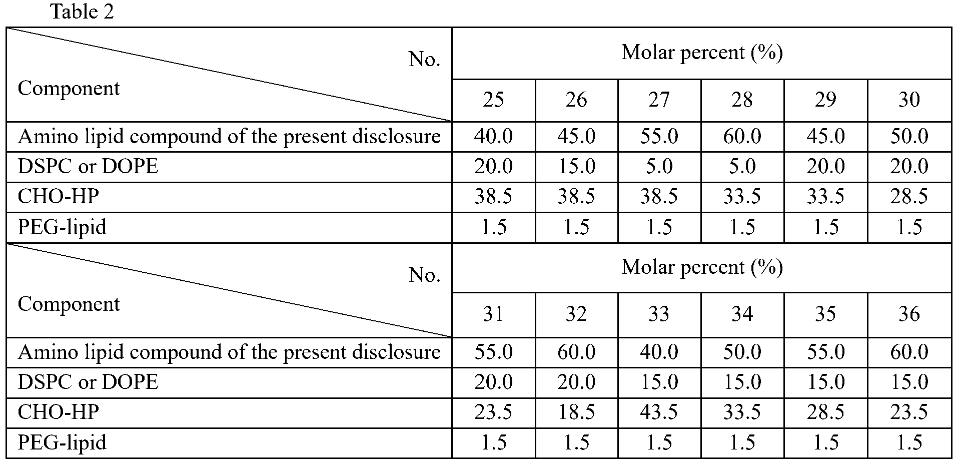

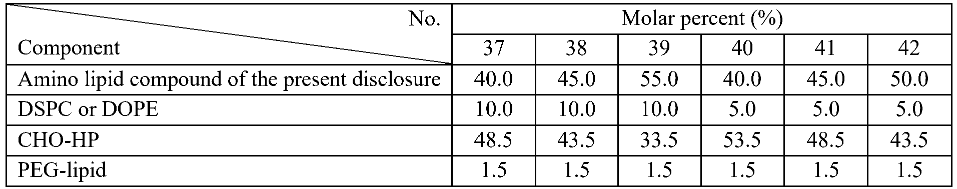

As described above, the lipid nanoparticle of the present disclosure may be used as a delivery vehicle for an active ingredient.

In some embodiments, the active ingredient includes a therapeutic and/or a prophylactic agent.

The term "therapeutic agent" or "prophylactic agent" refers to any agent that, when administered to a subject, has therapeutic, diagnostic, and/or prophylactic effects and/or elicits desired biological and/or pharmacological effects.

An "effective amount" or "therapeutically effective amount" refers to an amount of the amino lipid compound of the present invention or the lipid nanoparticle comprising the amino lipid compound of the present invention that is sufficient to effect treatment in a mammal (preferably a human), when administered to the mammal (preferably the human). The amount of the lipid nanoparticle of the present invention that constitutes a "therapeutically effective amount" will depend on the amino lipid compound, the condition and its severity, the mode of administration, and the age of the mammal to be treated, but may be routinely determined by one of ordinary skill in the art in light of their own knowledge and the present disclosure.

In some embodiments, the pharmaceutically active ingredient is a biologically active ingredient, which is a substance that has a biological effect when introduced into a cell or a host, for example, by stimulating an immune or inflammatory response, by exerting an enzymatic activity, by complementing a mutation, or the like. A biologically active ingredient includes, but is not limited to, a nucleic acid, a protein, a peptide, an antibody, a small molecule, and a mixture thereof.

In some embodiments, the biologically active ingredient is a nucleic acid.

In some embodiments, the biologically active ingredient is an antineoplastic agent, an antibiotic, an immunomodulator, an anti-inflammatory agent, an agent acting on the central nervous system, a polypeptide, a polypeptoid, or a mixture thereof.

A lipid nanoparticle may be referred to as "a lipid nanoparticle drug" when it encapsulates the active ingredient in its internal aqueous space.

In the context of the present disclosure, the term "cell" is a generic term and includes the culture of individual cells, tissues, organs, insect cells, avian cells, fish cells, amphibian cells, mammalian cells, primary cells, continuous cell lines, stem cells, and/or genetically engineered cells (e.g., recombinant cells expressing a heterologous polypeptide or protein). Recombinant cells include, for example, cells expressing heterologous polypeptides or proteins (such as growth factors or blood factors).

In some embodiments as described above, the lipid nanoparticle of the present disclosure further comprises a nucleic acid.

In some embodiments, the mass ratio of the amino lipid compound of the present disclosure to the nucleic acid in the lipid nanoparticle is about (5-30): 1, such as about (5-10): 1, (10-15): 1, (15-20): 1, (20-25): 1, or (25-30): 1. In some embodiments, the mass ratio of the amino lipid compound of the present disclosure to the nucleic acid in the lipid nanoparticle is about 10:1.

In some embodiments, the nucleic acid is selected from the group consisting of RNA, antisense oligonucleotide, and DNA.

In some embodiments, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA or a mixture thereof.

In some embodiments, the messenger RNA (mRNA) encodes a polypeptide and/or protein of interest. Any naturally or non-naturally occurring or otherwise modified polypeptide is included. In some embodiments, the polypeptide and/or protein encoded by the mRNA may have therapeutic and/or prophylactic effects when expressed in a cell.

In some embodiments, the RNA is an siRNA that is capable of selectively decreasing the expression of a gene of interest or down-regulating the expression of the gene. For example, an siRNA may be selected such that a gene associated with a particular disease, disorder, or condition is silenced upon administration of a lipid nanoparticle comprising the siRNA to a subject in need thereof. An siRNA may comprise a sequence complementary to an mRNA sequence encoding a gene or protein of interest. In some embodiments, the siRNA may be an immunomodulatory siRNA.

In certain embodiments, the RNA is sgRNA and/or cas9 mRNA. The sgRNA and/or cas9 mRNA may be used as a gene editing tool. For example, the sgRNA-Cas9 complex can affect mRNA translation of cellular genes.

In some embodiments, the RNA is an shRNA or a vector or plasmid encoding the same. The shRNA may be produced inside the target cell after delivery of an appropriate construct into the nucleus. Constructs and mechanisms associated with shRNA are well known in the relevant art.

In some embodiments, the DNA is a plasmid.

In some embodiments, the lipid nanoparticle is used to transfer nucleic acids. In some embodiments, the lipid nanoparticle may be used for, for example, gene therapy, gene vaccination, protein replacement therapy, antisense therapy, or therapy by interfering RNA.

### Pharmaceutical composition

In another aspect, the present invention provides a pharmaceutical composition comprising a lipid nanoparticle as described above and a pharmaceutically acceptable carrier, diluent, or excipient.

In some embodiments, the pharmaceutical composition further comprises a buffer solution. In some such embodiments, the buffer solution is selected from a phosphate buffer and a Tris buffer. In some such embodiments, the buffer solution is a phosphate buffer. In some embodiments, the buffer solution has a concentration of about 5 mmol/L to about 30 mmol/L. In some embodiments, the buffer solution has a concentration of about 10 mmol/L. In some embodiments, the buffer solution has a pH of about 6 to 8. In some embodiments, the buffer solution has a pH of about 7 to 8. In some embodiments, the buffer solution has a pH of about 7 to 7.5.

In some embodiments, the pharmaceutical composition further comprises a cryoprotectant. In some such embodiments, the cryoprotectant is selected from sucrose and trehalose. In some embodiments, the cryoprotectant is sucrose. In some embodiments, the cryoprotectant has a concentration of about 50 mg/ml to 100 mg/ml.

In some embodiments as described above, the pharmaceutical composition further comprises a cryoprotectant. In some such embodiments, the cryoprotectant is selected from sucrose and trehalose, preferably sucrose. In some embodiments, the cryoprotectant has a concentration of about 50 mg/ml to 100 mg/ml.

### Use

The lipid nanoparticle of the present disclosure has excellent properties of encapsulating biologically active ingredients. The lipid nanoparticle comprising biologically active ingredients can be used to deliver any of a variety of therapeutic agents into cells. The present disclosure includes use of the lipid nanoparticle as described above to deliver a biologically active ingredient into a cell. The present invention also provides a method of delivering a biologically active ingredient into a cell, tissue or organ, comprising contacting the lipid nanoparticle of the present disclosure comprising the biologically active ingredient with the cell, tissue or organ. This provides a subject with the possibility of new therapeutic treatment.

In some embodiments, the tissue or organ is selected from the group consisting of spleen, liver, kidney, lung, femur, ocular tissue, vascular endothelium in blood vessels, lymph, and tumor tissue.

In some embodiments, the cell is a mammalian cell. In some embodiments, the mammalian cell is in a mammal. As used herein, a subject may be any mammal. In some embodiments, the mammal is selected from the group consisting of mice, rats, pigs, cats, dogs, horses, goats, cattle, and monkeys, etc. In some embodiments, the subject is a human.

The present disclosure provides a method of producing a polypeptide and/or protein of interest in a mammalian cell, comprising contacting the cell with the lipid nanoparticle comprising mRNA encoding the polypeptide and/or protein of interest, upon contact of the cell with the lipid nanoparticle, the mRNA being able to be taken up into the cell and translated to produce the polypeptide and/or protein of interest.

In yet another aspect, the present disclosure provides a use of the amino lipid compound, lipid nanoparticle, or pharmaceutical composition of the present disclosure in the manufacture of a medicament. In some embodiments, the medicament is a nucleic acid drug. In some embodiments, the pharmaceutical composition is used for treatment and/or prevention of a disease.

In some embodiments, the disease is selected from the group consisting of rare diseases, infectious diseases, cancers, genetic diseases, autoimmune diseases, diabetes, neurodegenerative diseases, cardiovascular, renal vascular diseases, and metabolic diseases.

The medicaments are used for, for example, gene therapy, protein replacement therapy, antisense therapy, or therapy by interfering RNA, and gene vaccination.

In some embodiments, the cancers are selected from one or more of lung cancer, stomach cancer, liver cancer, esophageal cancer, colon cancer, pancreatic cancer, brain cancer, lymphoma, blood cancer, or prostate cancer. In some embodiments, the genetic disorders are selected from one or more of hemophilia, thalassemia, and Gaucher's disease.

In some embodiments, the gene vaccination is preferably used to treat and/or prevent cancer, allergy, toxicity, and pathogen infection. In some embodiments, the pathogen is selected from one or more of viruses, bacteria, or fungi.

The present disclosure provides a method of treating a disease or disorder in a mammal in need thereof, comprising administering to the mammal a therapeutically effective amount of the lipid nanoparticle as described above.

In some embodiments, the disease or disorder is selected from the group consisting of rare diseases, infectious diseases, cancers, genetic diseases, autoimmune diseases, diabetes, neurodegenerative diseases, cardiovascular, renal vascular diseases, and metabolic diseases.

In yet another aspect, the present disclosure provides a use of the amino lipid compound, lipid nanoparticle, or pharmaceutical composition of the disclosure in the manufacture of a medicament for nucleic acid transfer. In some embodiments, the nucleic acid is selected from the group consisting of RNA, antisense oligonucleotide, and DNA. In some embodiments, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA, or a mixture thereof. In some embodiments, the DNA is a plasmid.

### Preparation method:

In yet another aspect, the present disclosure further provides a general synthetic method for preparing an amino lipid compound of formula (III-1) of the present disclosure, for example, 1501-7, which can be prepared according to General reaction scheme 1-1 ("Method 1-1") or General reaction scheme 1-2 ("Method 1-2"), wherein A₁ is -N(R₄)-, and R₁ and R₂ are -C(R₆)(OL₁₄A₁₂R₇)₂.

### Method 1-1:

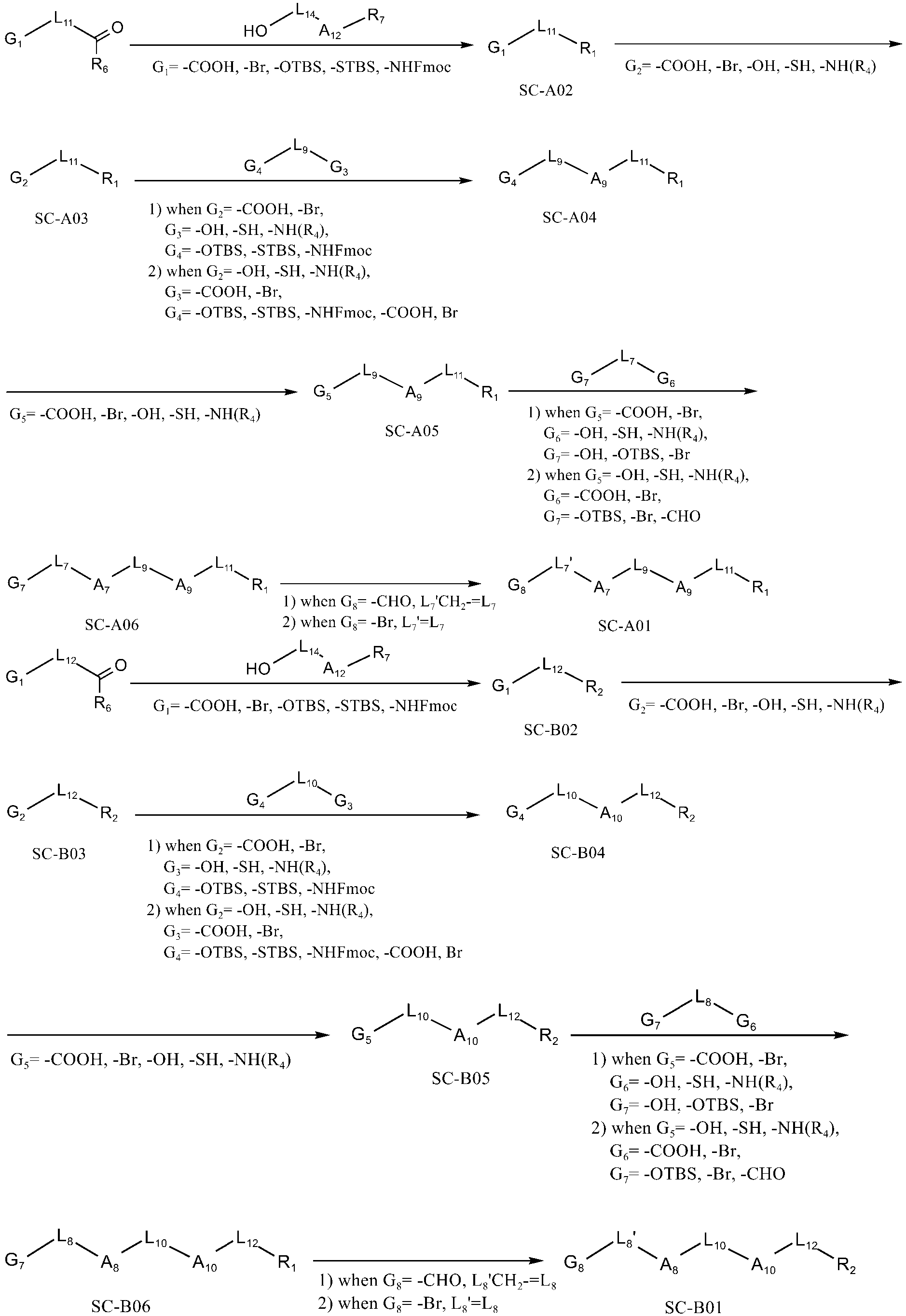

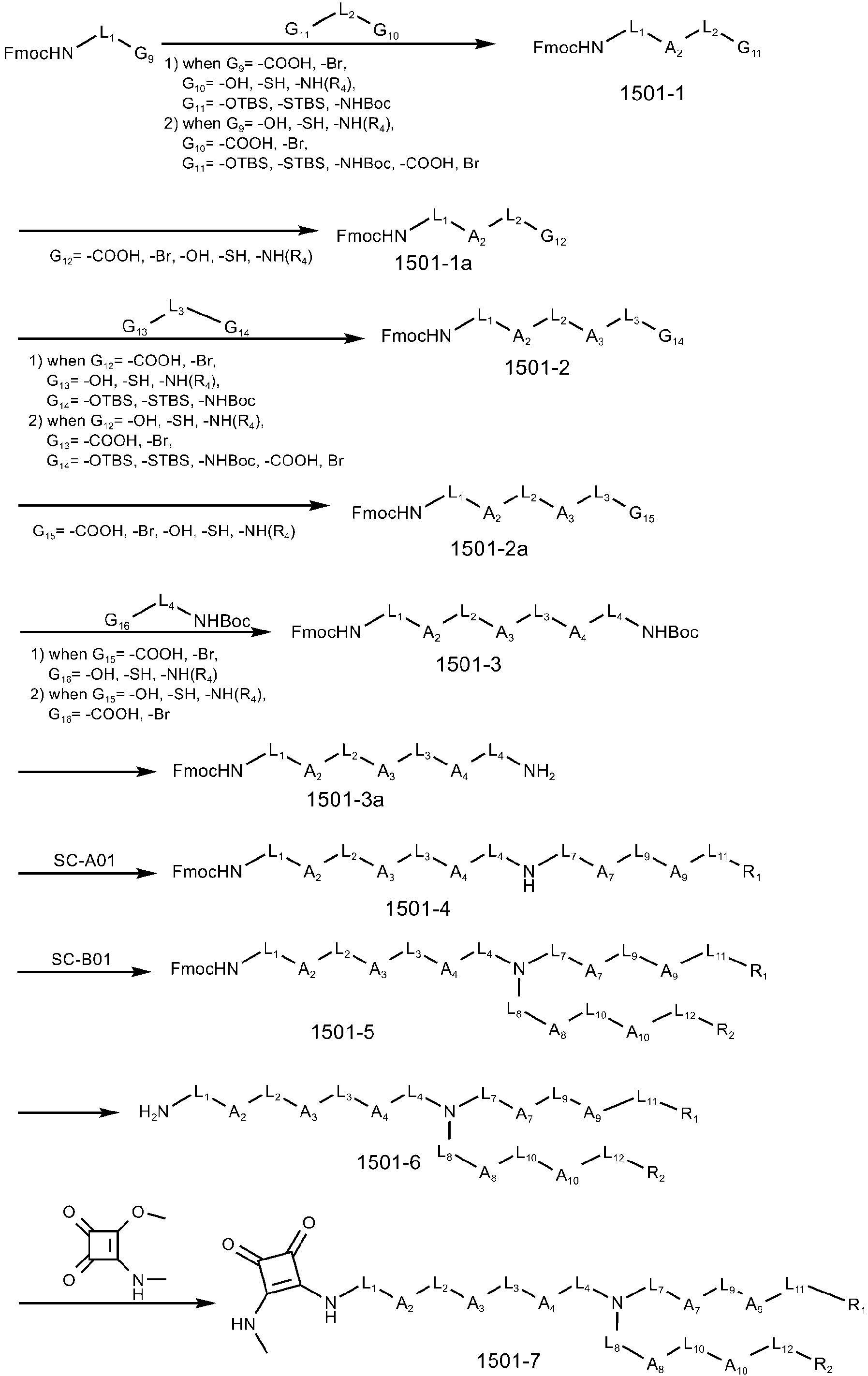

### Method 1-2:

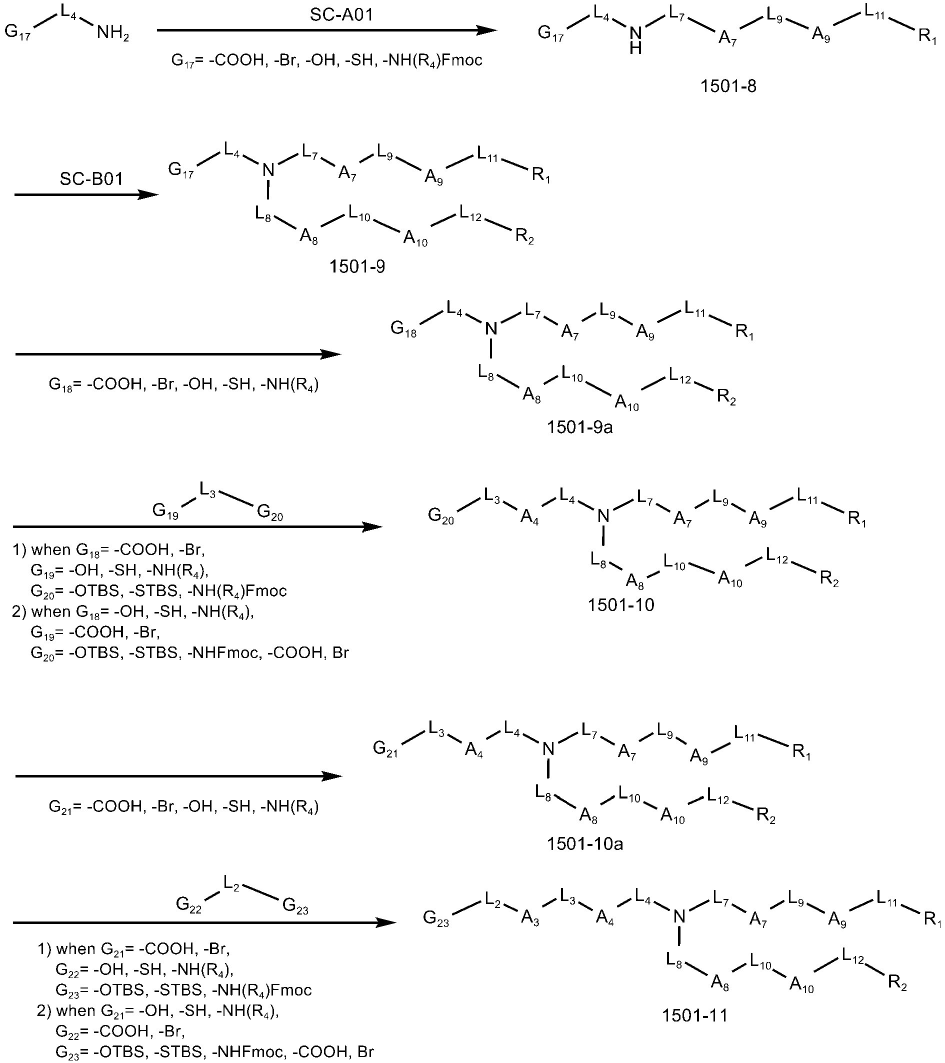

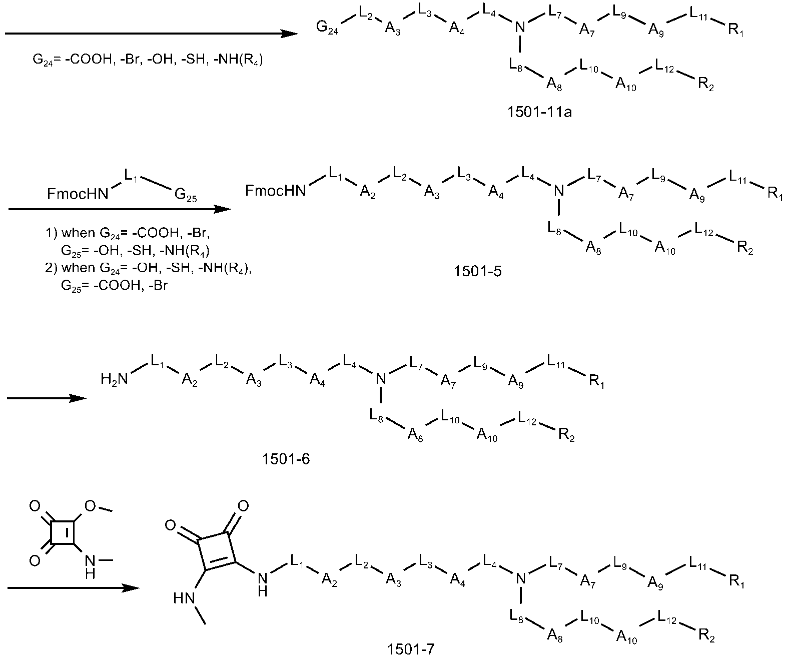

In yet another aspect, the present disclosure further provides a general synthetic method for preparing an amino lipid compound of formula (III-3) of the present disclosure, for example, 1501-16, which can be prepared according to General reaction scheme 2-1 ("Method 2-1") or General reaction scheme 2-2 ("Method 2-2"), wherein A₁ and A₄ are each independently -N(R₄)-, L₄ is a bond, and R₁ and R₂ are -C(R₆)(OL₁₄A₁₂R₇)₂.

### Method 2-1:

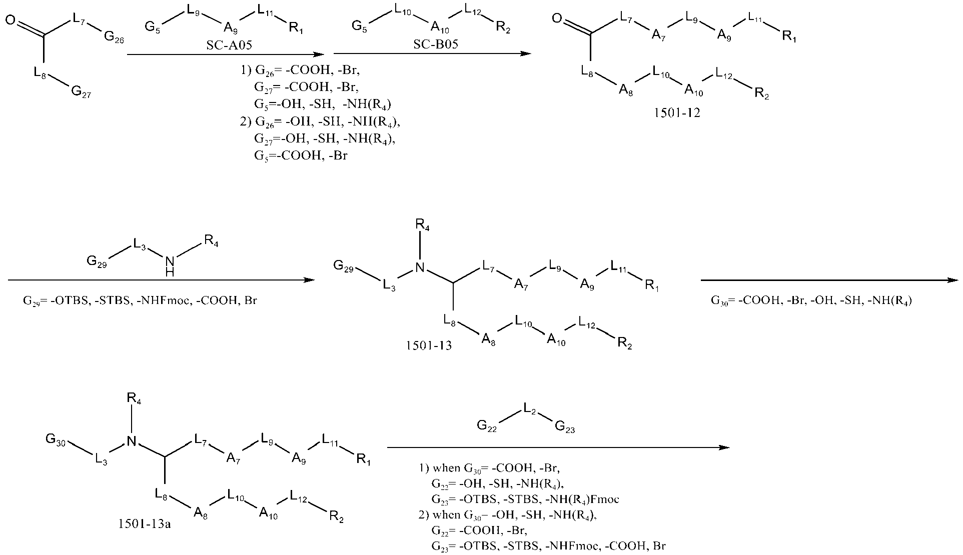

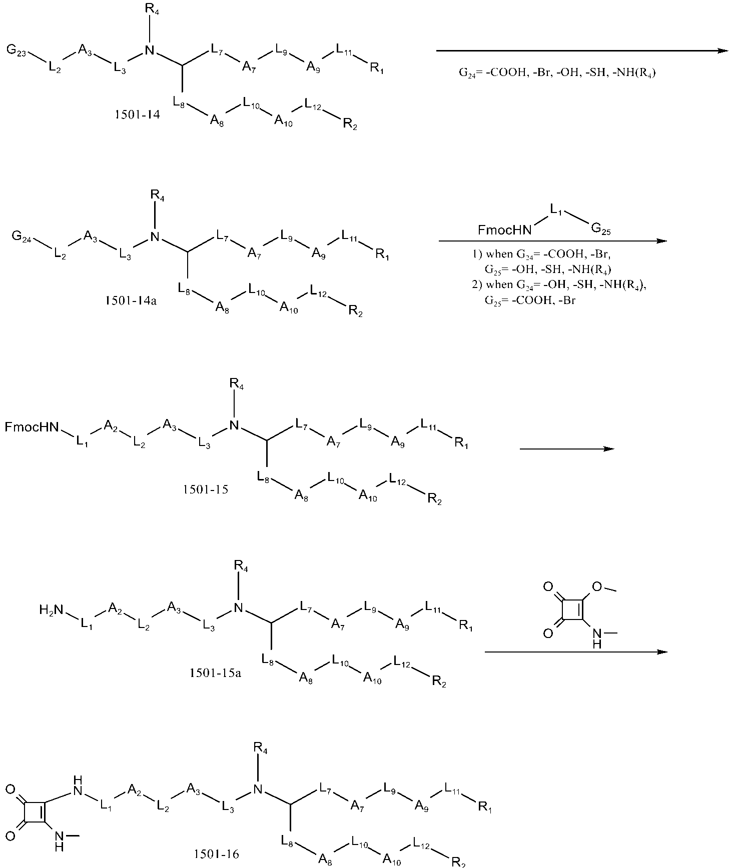

### Method 2-2:

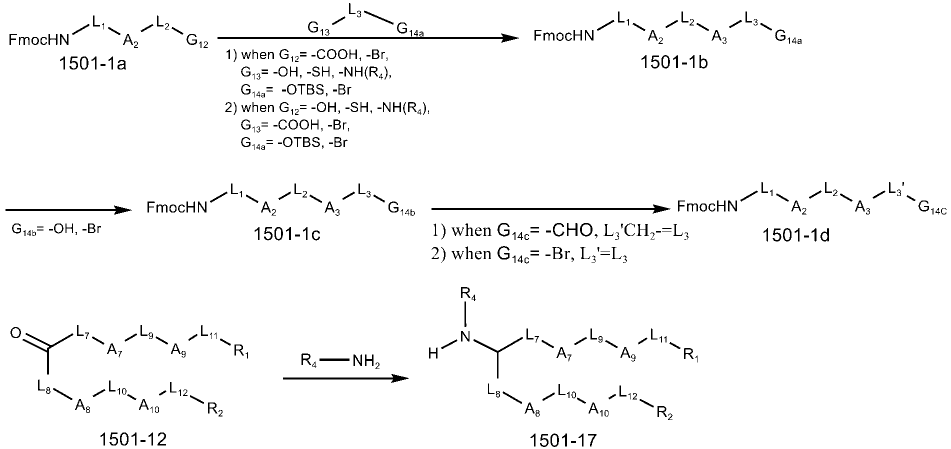

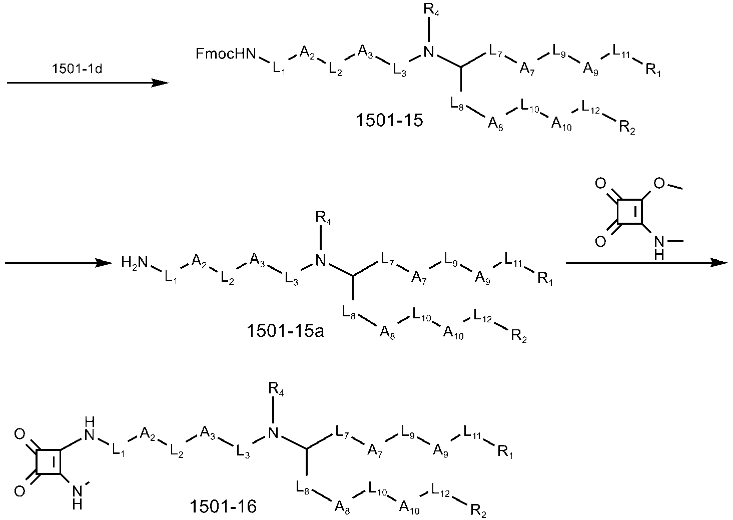

In yet another aspect, the present disclosure further provides a general synthetic method for preparing an amino lipid compound of formula (III-1) of the present disclosure, for example, 1501-20, which can be prepared according to General reaction scheme 3-1 ("Method 3-1") or General reaction scheme 3-2 ("Method 3-2"), wherein A₁ is -N(R₄)-, and R₁ and R₂ are each independently H, C₁-C₂₄ hydrocarbyl, C₁-C₂₄ heterohydrocarbyl, or A₁₁R₅.

### Method 3-1:

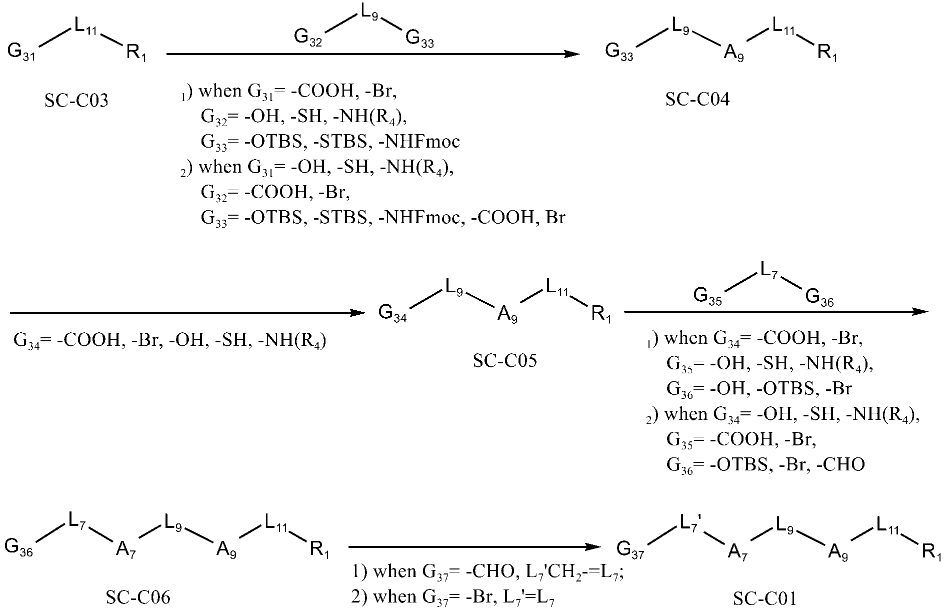

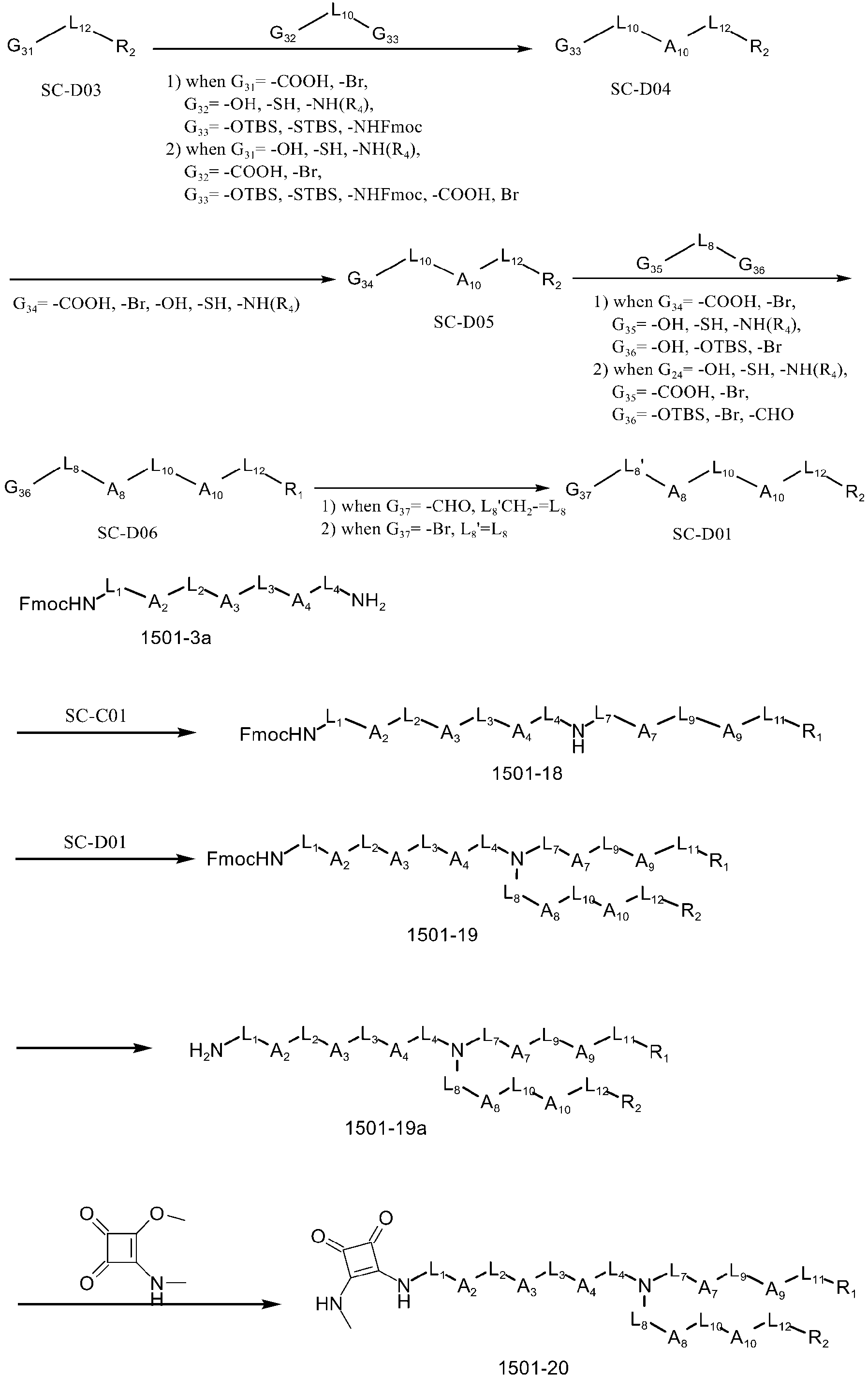

### Method 3-2:

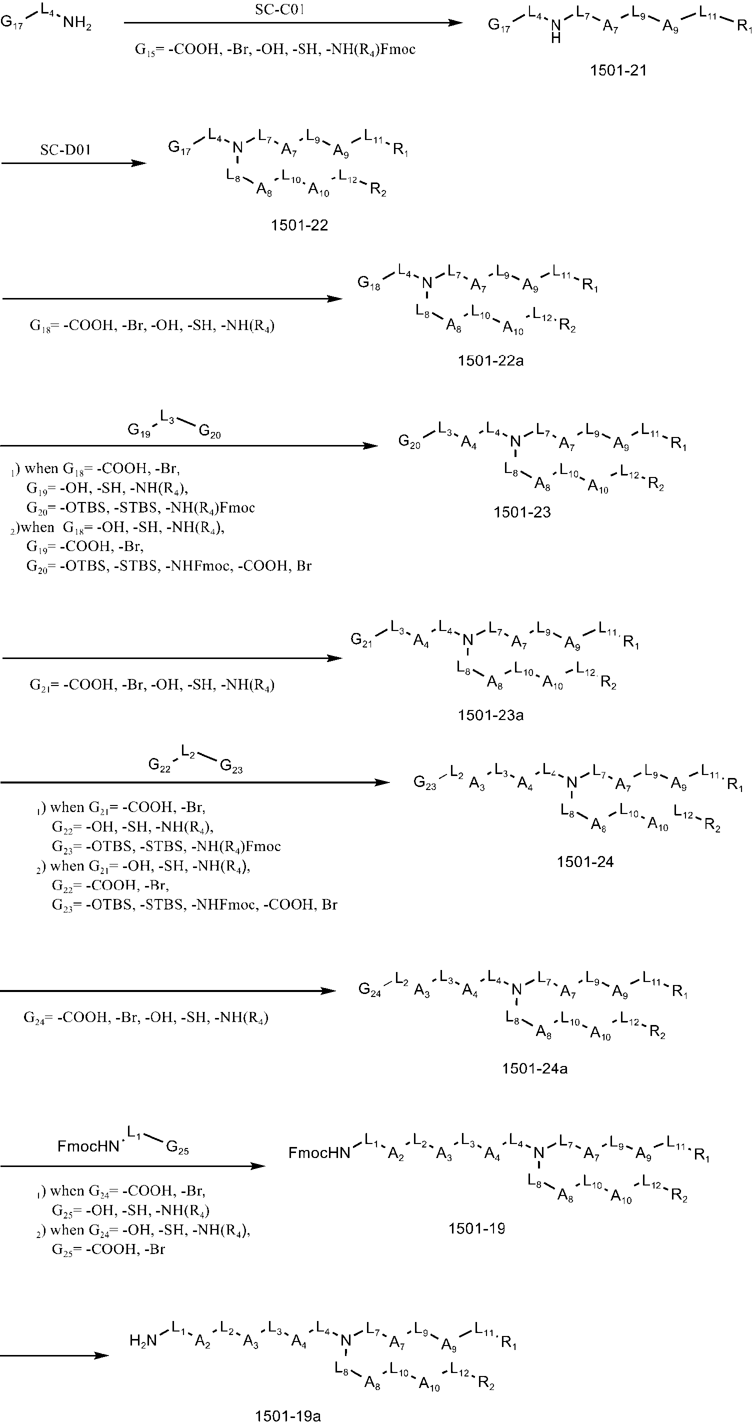

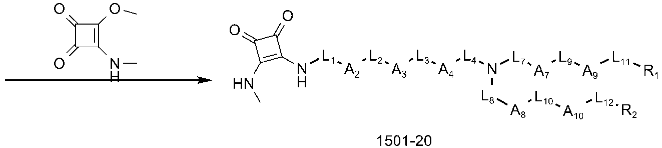

In yet another aspect, the present disclosure further provides a general synthetic method for preparing an amino lipid compound of formula (III-3) of the present disclosure, for example, 1501-29, which can be prepared according to General reaction scheme 4-1 ("Method 4-1") or General reaction scheme 4-2 ("Method 4-2"), wherein A₁ and A₄ are each independently -N(R₄)-, L₄ is a bond, and R₁ and R₂ are each independently H, C₁-C₂₄ hydrocarbyl, C₁-C₂₄ heterohydrocarbyl, or A₁₁R₅.

### Method 4-1:

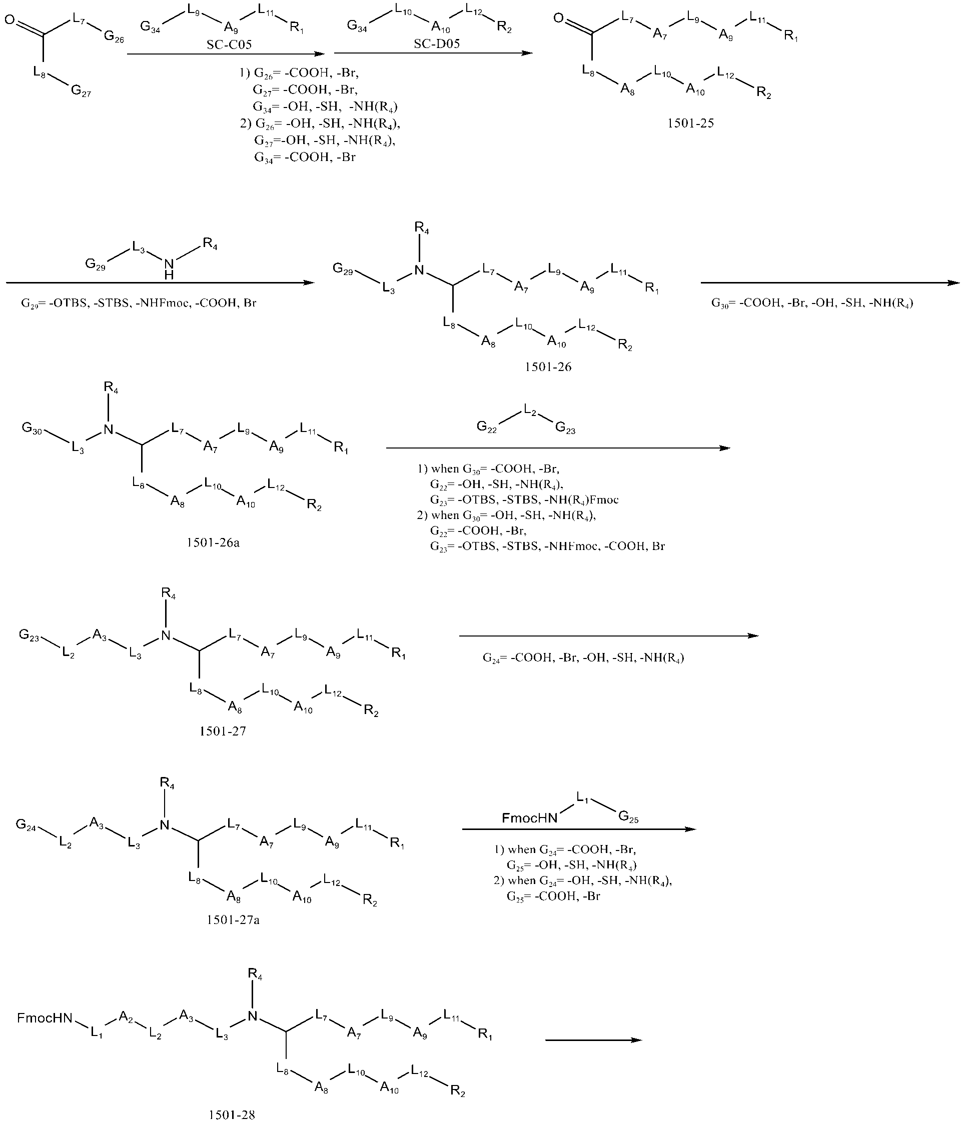

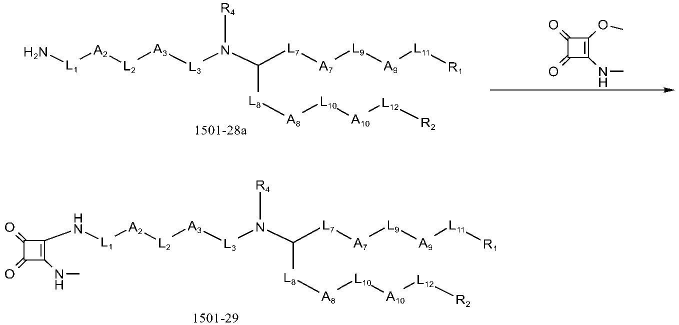

### Method 4-2:

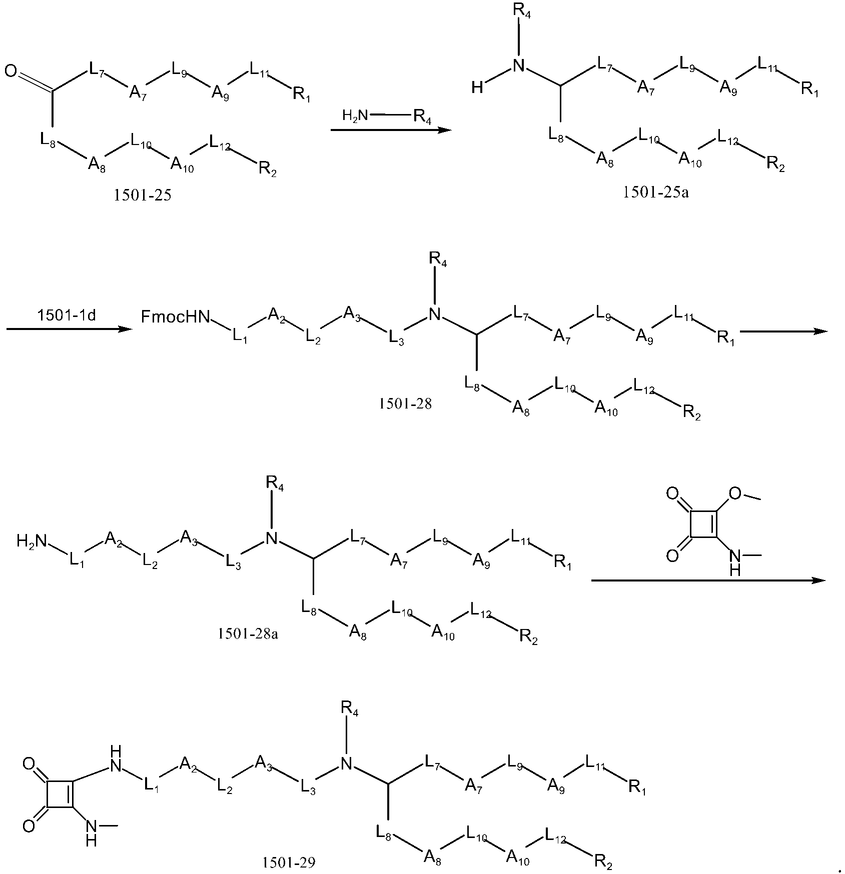

In yet another aspect, the present disclosure further provides a general synthetic method for preparing an amino lipid compound of formula (V-3) of the present disclosure, for example, 1501-33, which can be prepared according to General reaction scheme 5-1 ("Method 5-1"), wherein L₂, L₉, L₁₀, A₉, and A₁₀ are a bond, A₁ is -N(R₄)-, and R₁ and R₂ are -C(R₆)(OL₁₄A₁₂R₇)₂.

### Method 5-1:

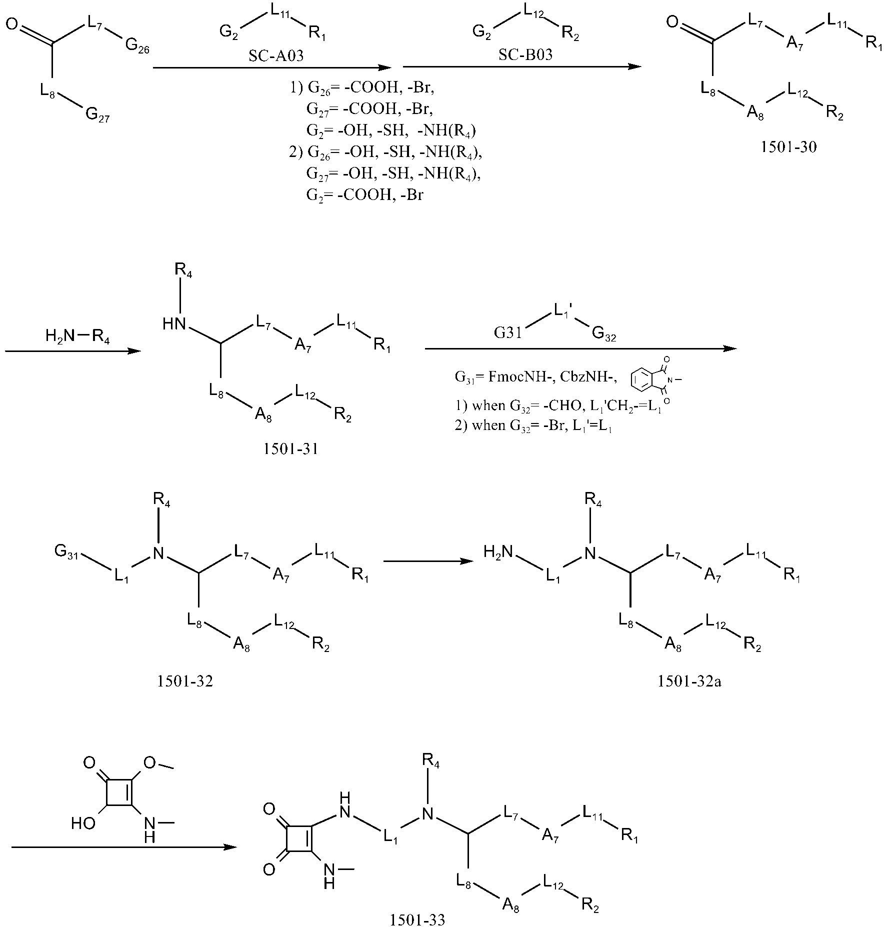

In yet another aspect, the present disclosure further provides a general synthetic method for preparing an amino lipid compound of formula (V-1) of the present disclosure, for example, 1501-37, which can be prepared according to General reaction scheme 6-1 ("Method 6-1"), wherein L₉, L₁₀, A₉, and A₁₀ are a bond, A₁ is -N(R₄)-, and R₁ and R₂ are each independently H, C₁-C₂₄ hydrocarbyl, C₁-C₂₄ heterohydrocarbyl, or A₁₁R₅.

### Method 6-1:

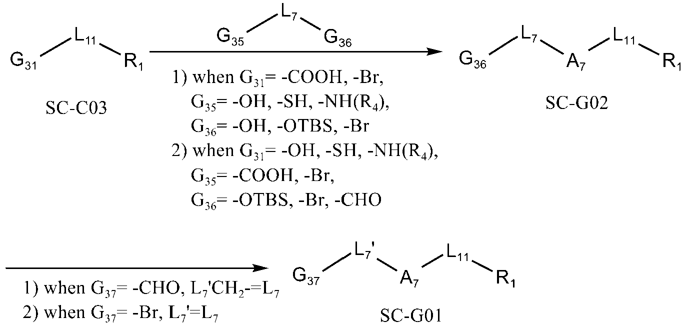

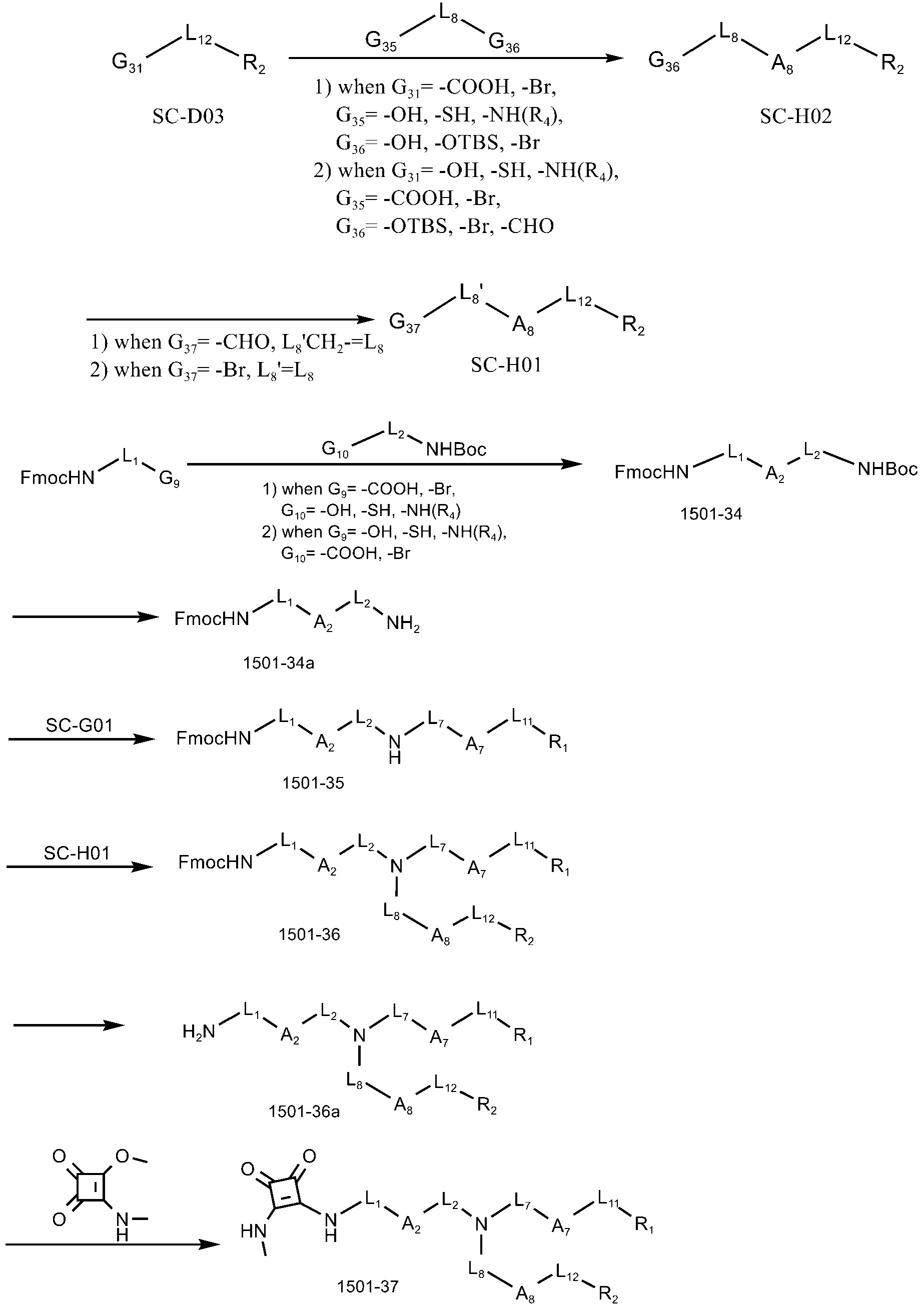

In yet another aspect, the present disclosure further provides a general synthetic method for preparing an amino lipid compound of formula (V-3) of the present disclosure, for example, 1501-41, which can be prepared according to General reaction scheme 7-1 ("Method 7-1"), wherein L₂, L₉, L₁₀, A₉, and A₁₀ are a bond, A₁ is -N(R₄)-, and R; and R₂ are each independently H, C₁-C₂₄ hydrocarbyl, C₁-C₂₄ heterohydrocarbyl, or A₁₁R₅.

### Method 7-1:

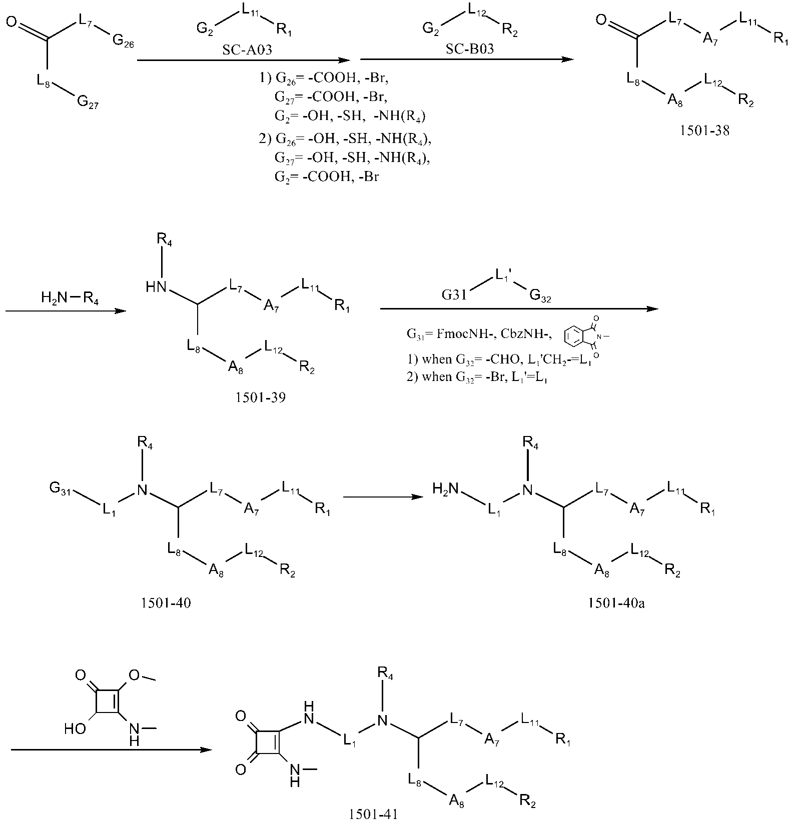

In yet another aspect, the present disclosure further provides a general synthetic method for preparing an amino lipid compound of formula (V-1) of the present disclosure, for example, 1501-44, which can be prepared according to General reaction scheme 8-1 ("Method 8-1"), wherein L₉, L₁₀, A₉, and A₁₀ are a bond, A₁ is -N(R₄)-, and R₁ and R₂ are -C(R₆)(OL₁₄A₁₂R₇)₂.

### Method 8-1:

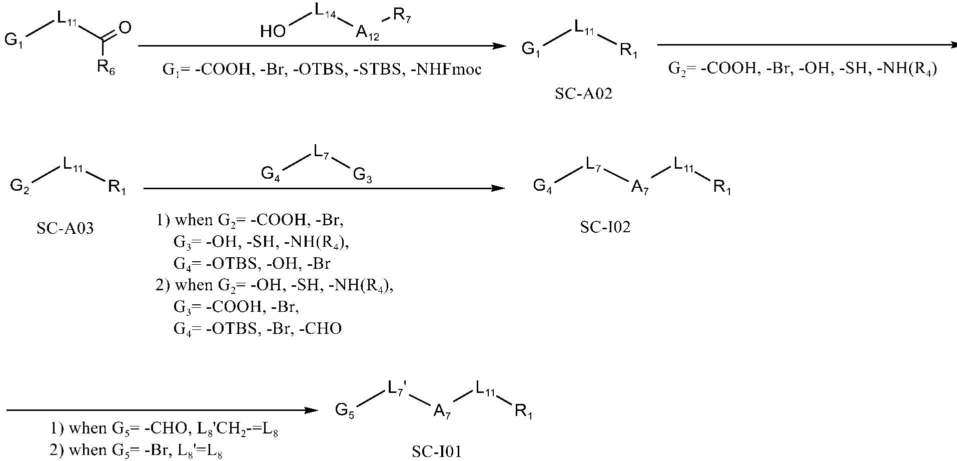

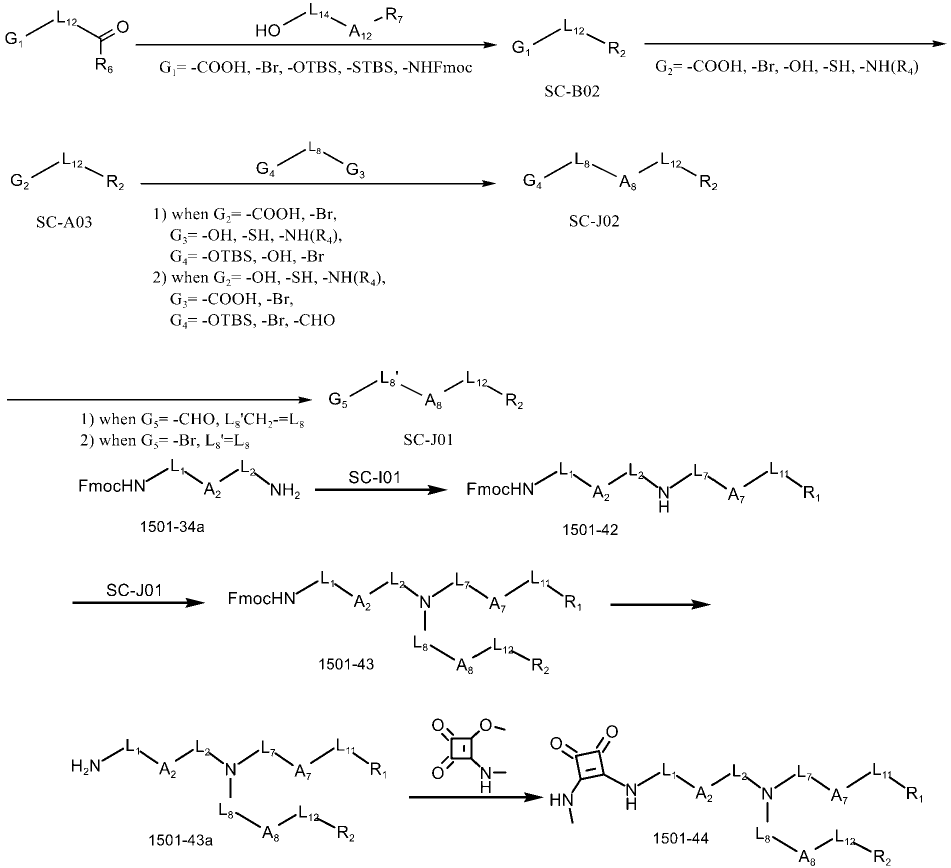

In yet another aspect, the lipid nanoparticle or the pharmaceutical composition of the present disclosure may be prepared according to methods known in the art. For example, the method may comprise the following steps:
(1) formulating: formulating a suitable aqueous phase; and formulating an organic phase comprising the amino lipid compound of the present disclosure and optionally a helper lipid, a structural lipid, and/or a PEG-lipid;
(2) encapsulation: mixing a suitable amount of the aqueous phase with the organic phase;
(3) dialysis: optionally dialyzing the mixture of step (2); and
(4) sterilization: optionally sterilizing the product of step (3), for example, by means of a sterilizing filter, such as a 0.22 µm microporous membrane.

In some embodiments, the lipid nanoparticle or the pharmaceutical composition of the present disclosure comprising a nucleic acid, particularly mRNA may be prepared by a method comprising the following steps:
(1) formulating: formulating an aqueous phase comprising the nucleic acid; and formulating an organic phase (e.g., an ethanol phase) comprising the amino lipid compound of the present disclosure and optionally a helper lipid, a structural lipid, and/or a PEG-lipid;
(2) encapsulation: mixing a suitable amount of the aqueous phase with the organic phase;
(3) dialysis: optionally dialyzing the mixture of step (2);
(4) sterilization: optionally sterilizing the product of step (3), for example, by means of a sterilizing filter, such as a 0.22 µm microporous membrane.

### Beneficial effect

The amino lipid compound of the present disclosure can form a vehicle, such as a lipid nanoparticle, with excellent bioactivity, can be used for delivering biologically active ingredients, especially water-insoluble drugs or active ingredients that are easily decomposed or degraded (such as nucleic acids), and improving its bioavailability, or immunological activity, or or transfection efficiency (for nucleic acids), or safety, or tissue and/or cell targeting or specificity.

In order to make the purposes, technical solutions, and advantages of the present invention clearer, the present invention is described below with reference to specific examples. The follow examples are merely illustrative of the present invention and are not intended to be limiting.

### Examples

The following examples are provided for purposes of illustration and not limitation.

The experimental methods for which specific conditions are not specified in the examples are usually under conventional conditions or conditions as recommended by the manufacturer of the raw material or commodity; and the reagents of unspecified origin are generally conventional reagents commercially available.

The abbreviations used in the examples have the following meanings:
- rt: Room temperature, 20-30°C;
- Pd/C: Palladium/carbon;
- EA: Ethyl acetate;
- DCM: Dichloromethane;
- TEA: Triethylamine;
- MPa: Megapascal;
- DMF: N,N-dimethylformamide;
- EDCI: 1-(3-Dimethylaminopropyl)-3-ethylcarbodiimide hydrochloride;
- DMAP: 4-Dimethylaminopyridine;
- Pyr: Pyridine;
- TEMPO: 2,2,6,6-Tetramethylpiperidinooxy;
- NaDCC: Sodium dichloroisocyanurate;
- TBSCl: *tert*-butyldimethylsilyl chloride;
- TFA: Trifluoroacetic acid;
- Imid: Imidazole;
- CPME: Cyclopentyl methyl ether;
- THF: Tetrahydrofuran;
- HOSu: N-hydroxysuccinimide;
- DCC: Dicyclohexylcarbodiimide;
- DCE: 1,2-Dichloroethane;
- Morpholine: Morpholine;
- H: Hour;
- Min: Minute;
- TBAF·3H₂O: Tetrabutylammonium fluoride trihydate;
- TBAF: Tetrabutylammonium fluoride;
- DEAD: Diethyl azodicarboxylate;
- PPh3: Triphenylphosphine;
- STAB: Sodium triacetoxyborohydride;
- MeOH: Methanol;
- EtOH: Ethanol;
- Formaldehyde: Formaldehyde.

### Example 1: Synthesis of Amino Lipid Compound 1503

### Step 1: Synthesis of 1500-A

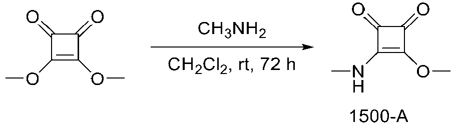

### Experimental procedure:

3,4-Dimethoxy-3-cyclobutene-1,2-dione (20.0 g, 140.7 mmol) and dichloromethane (1.0 L) were added to a 2 L single-necked flask, and methylamine (a 30% solution in methanol, 15.3 g, 148 mmol) was added dropwise with stirring at room temperature and was allowed to react at room temperature for 72 h after the dropwise addition was completed. Filtering was then performed, and the filter cake was washed once with water (20 mL) and once with dichloromethane (20 mL). The organic phase was separated from the filtrate, and the water phase was extracted twice with dichloromethane (100 mL). The organic phases were combined, and the solvent was removed from the organic phase by evaporation under reduced pressure at 40°C, obtaining a coarse product. The coarse product was purified by silica gel column chromatography and eluted with dichloromethane to obtain 16.74 g of a white solid product of 1500-A with a yield of 84.3%.

### Step 2: Synthesis of 1501-B

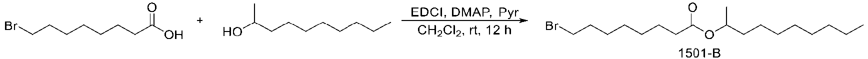

### Experimental procedure:

EDCI (36.3 g, 190.0 mmol), DMAP (1.54 g, 12.6 mmol), and dichloromethane (200 mL) were added to a 500 mL round-bottom flask, and cooled to -5°C. Pyridine (15.0 g, 190.0 mmol) was added dropwise with stirring, and stirred at -5°C for 10 min after the dropwise addition was completed. 8-Bromooctanoic acid (33.8 g, 151.6 mmol) and 2-decanol (20.0 g, 126.4 mmol) were added. The reaction solution was then warmed to room temperature and reacted for 12 h. 1.0 M aqueous hydrochloric acid solution (100 mL) was added dropwise to the reaction solution to quench, followed by extraction twice with dichloromethane (150 mL), and the organic phases were combined. The organic phase was then washed with saturated aqueous sodium chloride solution (200 mL), dried with anhydrous sodium sulfate, and filtered. The solvent was removed from the filtrate by evaporation under reduced pressure at 45°C, obtaining a coarse product of 1501-B. The coarse product was purified by silica gel column chromatography and eluted with n-hexane:ethyl acetate=20:1 to obtain 42.8 g of a colorless oily product of 1501-B with a yield of 93.2%.

### Step 3: Synthesis of 1501-C

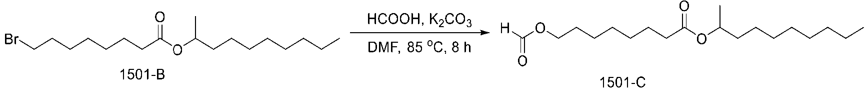

### Experimental procedure:

Potassium carbonate (12.2 g, 88.3 mmol) and DMF (200 mL) were added to a 500 mL round-bottom flask. Formic acid (8.67 g, 188.4 mmol) was added dropwise with stirring at room temperature, and stirred at room temperature for 1 h after the dropwise addition was completed. 1501-B (42.8 g, 117.7 mmol) was added. The reaction solution was heated to 85°C and reacted for 8 h. The reaction solution was added with water (1 L) and then extracted twice with ethyl acetate (300 mL), and the organic phases were combined. The organic phase was washed with saturated aqueous sodium bicarbonate solution (200 mL) and saturated aqueous sodium chloride solution (200 mL), dried with anhydrous sodium sulfate, and filtered. The solvent was removed from the filtrate by evaporation under reduced pressure at 45°C, obtaining a coarse product of 1501-C. The coarse product was purified by silica gel column chromatography and eluted with n-hexane:ethyl acetate=20:1 to obtain 29.91 g of a colorless oily product of 1501-C with a yield of 77.3%.

### Step 4: Synthesis of 1501-D

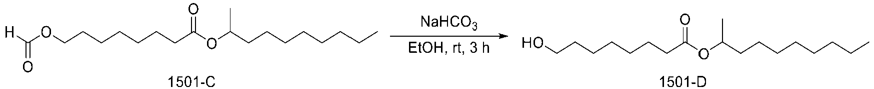

### Experimental procedure:

1501-C (29.9 g, 91.1 mmol) and absolute ethyl alcohol (180 mL) were added to a 500 mL round-bottom flask, added with sodium bicarbonate powder (3.06 g, 36.4 mmol) with stirring at room temperature, and stirred at room temperature for 3 h. Filtering was then performed, and the solvent was removed from the filtrate by evaporation under reduced pressure at 45°C. The residue was added with water (200 mL), and extracted twice with dichloromethane (200 mL), and the organic phases were combined. The organic phase was then washed with saturated aqueous sodium chloride solution (200 mL), dried with anhydrous sodium sulfate, and filtered. The solvent was removed from the filtrate by evaporation under reduced pressure at 45°C, obtaining a coarse product of 1501-D. The coarse product was purified by silica gel column chromatography and eluted with n-hexane:ethyl acetate = 10:1 to obtain 21.5 g of a colorless oily product of 1501-D with a yield of 78.6%.

### Step 5: Synthesis of 1501-E

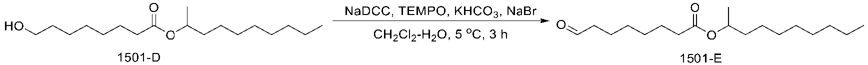

### Experimental procedure:

1501-D (21.5 g, 71.6 mmol) was added to a 500 mL single-necked flask, and then dichloromethane (215 mL), TEMPO (223 mg, 1.43 mmol), potassium bicarbonate (5.0 g, 50.1 mmol), and sodium bromide (294 mg, 2.86 mmol) were added. The reaction solution was cooled to 5°C, and then added dropwise with an aqueous solution of NaDCC (9.5 g, 42.9 mmol, dissolved in 85 mL water) using a constant-pressure funnel. After the dropwise addition was completed, the reaction was carried out at 5°C for 3 h. Filtering was then performed, and the filter cake was washed once with water (20 mL) and once with dichloromethane (20 mL). The organic phase was separated from the filtrate, and the water phase was extracted twice with dichloromethane (200 mL). The organic phases were combined, dried with anhydrous sodium sulfate, and filtered. The solvent was removed from the filtrate by evaporation under reduced pressure at 45°C, obtaining a coarse product of 1501-E. The coarse product was purified by silica gel column chromatography and eluted with dichloromethane to obtain 20.28 g of a colorless oily product of 1501-E with a yield of 94.9%.

### Step 6: Synthesis of 1501-F

With reference to the process of synthesizing 1501-B in Step 2, compound 1501-F was prepared according to the following reaction route, resulting in 25.8 g compound 1501-F with a yield of 83.0%.

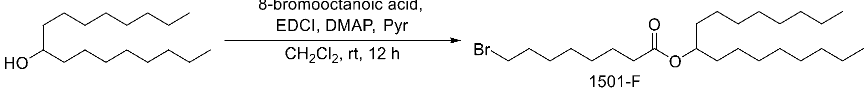

### Step 7: Synthesis of 1501-G

With reference to the process of synthesizing 1501-C in Step 3, compound 1501-G was prepared according to the following reaction route, resulting in 39.75 g compound 1501-G. The coarse product was directly used in the next step.

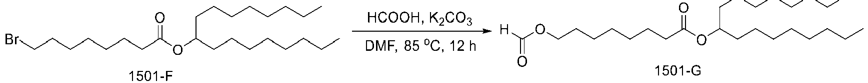

### Step 8: Synthesis of 1501-H

With reference to the process of synthesizing the 1501-D in Step 4, compound 1501-H was prepared according to the following reaction route, resulting in 29.3 g compound 1501-H. The yield of the two-step reaction was 80.5%.

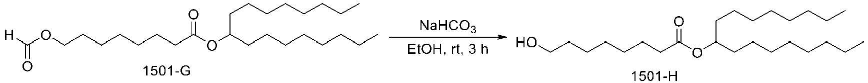

### Step 9: Synthesis of 1501-I

With reference to the process of synthesizing the 1501-E in Step 5, compound 1501-I was prepared according to the following reaction route, resulting in 22.35 g compound 1501-I with a yield of 92.6%.

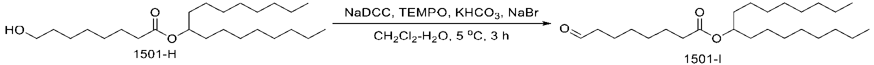

### Step 10: Synthesis of 1503-A

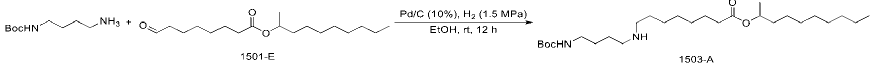

### Experimental procedure:

1501-E (21.3 g, 71.4 mmol), absolute ethyl alcohol (210 mL), N-Boc-1,4-butanediamine (16.1 g, 85.6 mmol) and Pd/C (10%, 2.1 g) were added to a 500 mL round-bottom flask, stirred homogeneously at room temperature, and then transferred to an autoclave. The autoclave was vacuumized and filled with hydrogen to 1.5 MPa to reacte at room temperature for 12 h. The autoclave was deflated to restore normal pressure. The autoclave was opened, and the reaction solution was filtered to remove Pd/C, and the solvent was removed by evaporation under reduced pressure at 45°C, obtaining a coarse product of 1503-A. The coarse product was purified by silica gel column chromatography and eluted with ethyl acetate:methanol = 20:1 to obtain 28.9 g of a colorless oily product of 1503-A with a yield of 86.3%.

### Step 11: Synthesis of 1503-B-Boc

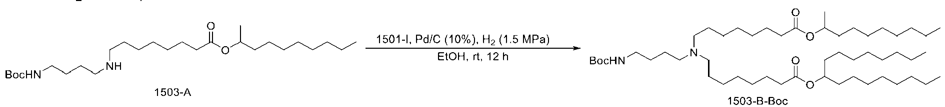

### Experimental procedure:

1501-I (17.3 g, 43.5 mmol), absolute ethyl alcohol (140 mL), 1503-A (13.6 g, 29.0 mmol) and Pd/C (10%, 1.4 g) were added to a 500 mL round-bottom flask, stirred homogeneously at room temperature, and then transferred to an autoclave. The autoclave was vacuumized and filled with hydrogen to 1.5 MPa to react at room temperature for 12 h. The autoclave was deflated to restore normal pressure. The autoclave was opened, and the reaction solution was filtered to remove Pd/C, and the solvent was removed by evaporation under reduced pressure at 45°C, obtaining a coarse product of 1503-B-Boc. The coarse product was purified by silica gel column chromatography and eluted with ethyl acetate:methanol = 20:1 to obtain 15.33 g of a colorless oily product of 1503-B-Boc with a yield of 62.3%.

### Step 12: Synthesis of 1503-B

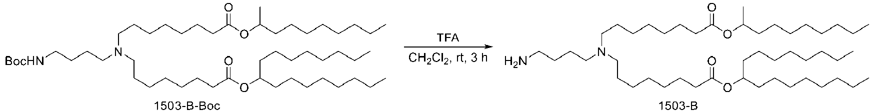

### Experimental procedure:

1503-B-Boc (5.7 g, 6.7 mmol) and dichloromethane (50 mL) were added to a 250 mL round-bottom flask, and added slowly with trifluoroacetic acid (17 mL) with stirring at room temperature to react at room temperature for 3 h. The solvent was removed by evaporation under reduced pressure. Ethyl acetate (50 mL) was added. The pH was adjusted to 8 with a saturated sodium bicarbonate solution. The organic phase was separated, and the water phase was extracted twice with ethyl acetate (100 mL). The organic phases were combined. The organic phase was then washed with saturated aqueous sodium chloride solution (100 mL), dried with anhydrous sodium sulfate, and filtered. The solvent was removed from the filtrate by evaporation under reduced pressure at 45°C, obtaining a coarse product of 1503-B. The coarse product was purified by silica gel column chromatography and eluted with ethyl acetate:methanol = 20:1 to obtain 4.75 g of a colorless oily product of 1503-B with a yield of 94.4%.

### Step 13: Synthesis of 1503-C-Boc

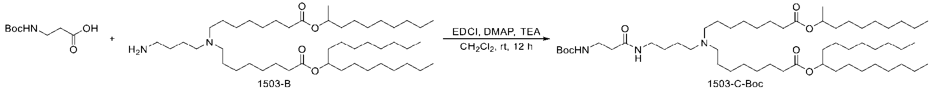

### Experimental procedure:

EDCI (575 mg, 3.0 mmol), DMAP (24 mg, 0.2 mmol), and dichloromethane (15 mL) were added to a 50 mL round-bottom flask, added with triethylamine (304 mg, 3.0 mmol) dropwise under stirring at room temperature, and stirred at room temperature for 10 min after the dropwise addition was completed. Boc-alanine (454 mg, 2.4 mmol) and 1503-B (1.5 g, 2.0 mmol) were added and reacted at room temperature for 12 h. The reaction solution was added with water (20 mL) to quench, and then extracted twice with dichloromethane (20 mL). The organic phases were combined. The organic phase was then washed with saturated aqueous sodium chloride solution (30 mL), dried with anhydrous sodium sulfate, and filtered. The solvent was removed from the filtrate by evaporation under reduced pressure at 45°C, obtaining 1.7 g of a coarse product of 1503-C-Boc. The coarse product was directly used in the reaction of the next step.

### Step 14: Synthesis of 1503-C

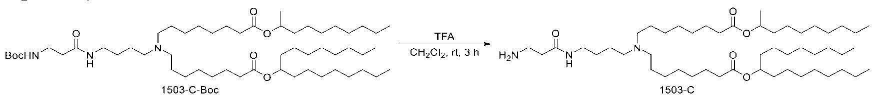

### Experimental procedure:

The coarse product of 1500-C-Boc (1.7 g, 1.84 mmol) and dichloromethane (17 mL) were added to a 50 mL round-bottom flask, slowly added with trifluoroacetic acid (6 mL) under stirring at room temperature, and reacted at room temperature for 3 h. The solvent was removed by evaporation under reduced pressure at 45°C. The residue was added with ethyl acetate (30 mL). The pH was adjusted to 8 with saturated sodium bicarbonate solution. The organic phase was separated, and the water phase was extracted twice with ethyl acetate (50 mL). The organic phases were combined. The organic phase was then washed with saturated aqueous sodium chloride solution (100 mL), dried with anhydrous sodium sulfate, and filtered. The solvent was removed from the filtrate by evaporation under reduced pressure at 45°C, obtaining a coarse product of 1503-C. The coarse product was purified by silica gel column chromatography and eluted with ethyl acetate:methanol = 20:1 to obtain 0.9 g of a colorless oily product of 1503-C. The yield of the two-step reaction was 55.0%.

### Step 15: Synthesis of Amino Lipid Compound 1503

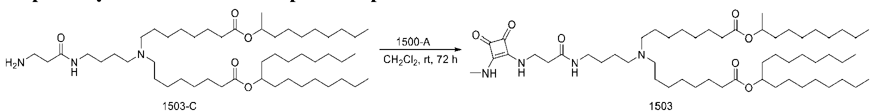

### Experimental procedure:

1503-C (900 mg, 1.1 mmol), dichloromethane (10 mL) and 1500-A (154 mg, 1.1 mmol) were added to a 25 mL round-bottom flask, and reacted at room temperature for 72 h. Water (20 mL) was added to the reaction solution to quench. The organic phase was separated. The water phase was extracted twice with dichloromethane (20 mL), and the organic phases were combined. The organic phase was then washed with saturated aqueous sodium chloride solution (30 mL), dried with anhydrous sodium sulfate, and filtered. The solvent was removed from the filtrate by evaporation under reduced pressure at 45°C, obtaining a coarse product of 1503. The coarse product was purified by silica gel column chromatography and eluted with ethyl acetate:methanol = 10:1 to obtain 370 mg of a colorless oily product of the amino lipid compound 1503 with a yield of 36.3% and a purity of 91.25%.

¹H NMR (600 MHz, CDCl₃) δ 8.16 (s, 1H), 8.06 (s, 1H), 7.64 (s, 1H), 4.96-4.87 (m, 2H), 3.91 (s, 2H), 3.39-3.29 (m, 5H), 3.22-3.03 (m, 6H), 2.67-2.58 (m, 2H), 2.32 (q, *J =* 7.3 Hz, 4H), 1.89-1.80 (m, 2H), 1.75 (s, 4H), 1.69-1.58 (m, 8H), 1.57-1.51 (m, 4H), 1.46-1.36 (m, 10H), 1.31 (dq, *J =* 13.8, 6.9 Hz, 38H), 1.23 (d, *J* = 6.2 Hz, 3H), 0.92 (t, *J* = 7.0 Hz, 9H).

LC-MS (ESI): Calculated for 931.8, Found (M+H): 932.3.

### Example 2: Synthesis of Amino Lipid Compound 1506

According to the method of Example 1, the amino lipid compound 1506 was prepared according to the following reaction route, resulting in 140 mg of the amino lipid compound 1506 with a yield of 16.4% and a purity of 90.47%.

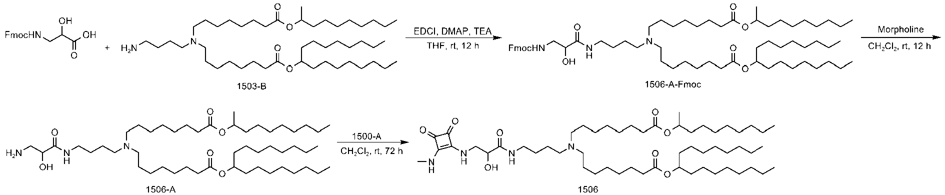

¹H NMR (600 MHz, CDCl₃) δ 7.65 (s, 1H), 7.63 (s, 1H), 7.38-7.31 (m, 1H), 4.91-4.82 (m, 2H), 4.28 (t, *J* = 3.8 Hz, 1H), 3.73-3.69 (m, 3H), 3.32 (s, 3H), 2.48-2.34 (m, 6H), 2.27 (q, *J* = 7.5 Hz, 4H), 1.64-1.53 (m, 5H), 1.53-1.36 (m, 15H), 1.34-1.20 (m, 48H), 1.19 (d, *J =* 6.2 Hz, 3H), 0.87 (t, *J =* 7.0 Hz, 9H).

LC-MS (ESI): Calculated for 947.8, Found (M+H): 948.2.

### Example 3: Synthesis of Amino Lipid Compound 1501

According to the method of Example 1, the amino lipid compound 1501 was prepared according to the following reaction route, resulting in 0.6 g of the amino lipid compound 1501 with a yield of 38.7% and a purity of 92.56%.

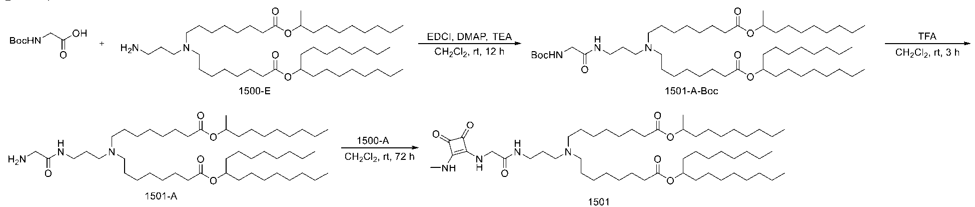

¹H NMR (600 MHz, CDCl₃) δ 8.34 (s, 1H), 7.50 (s, 1H), 7.27 (s, 1H), 4.92-4.82 (m, 2H), 4.30 (s, 2H), 3.33-3.27 (m, 5H), 2.51 (t, *J* = 6.1 Hz, 2H), 2.43-2.37 (m, 4H), 2.27 (q, *J* = 7.5 Hz, 4H), 2.02-1.82 (m, 2H), 1.70-1.63 (m, 2H), 1.63-1.54 (m, 4H), 1.56-1.45 (m, 4H), 1.45-1.38 (m, 4H), 1.34-1.22 (m, 48H), 1.19 (d, *J* = 6.2 Hz, 3H), 0.87 (t, *J* = 7.0 Hz, 9H).

LC-MS (ESI): Calculated for 903.8, Found (M+H): 904.2.

As for 1500-E, with reference to the process of synthesizing 1503-B in Example 1, the compound 1500-E was prepared according to the following route, resulting in 980 mg compound 1500-E with a yield of 74.0%.

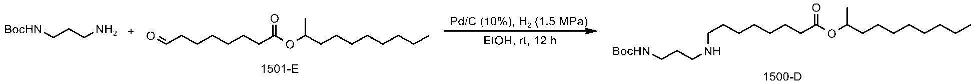

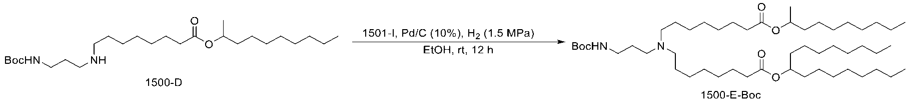

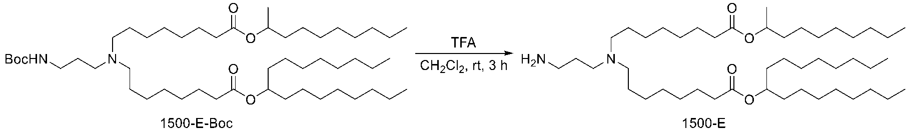

### Example 4: Synthesis of Amino Lipid Compound 1502

According to the method of Example 1, the amino lipid compound 1502 was prepared according to the following reaction route, resulting in 0.8 g of the amino lipid compound 1502 with a yield of 65.6% and a purity of 96.64%.

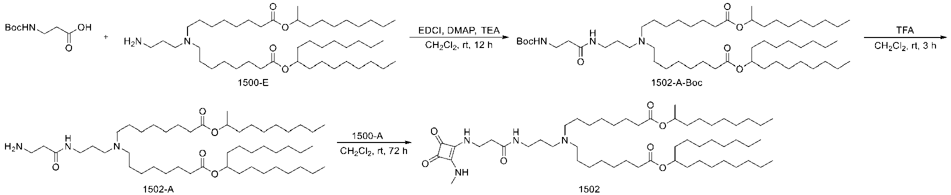

¹H NMR (600 MHz, CDCl₃) δ 4.97-4.86 (m, 2H), 3.90 (s, 2H), 3.39-3.28 (m, 5H), 2.84 (m, 2H), 2.70 (m, 4H), 2.59-2.49 (m, 2H), 2.31 (q, *J* = 7.5 Hz, 4H), 1.80 (s, 2H), 1.70-1.46 (m, 14H), 1.42-1.26 (m, 48H), 1.22 (t, *J* = 14.9 Hz, 3H), 0.92 (t, *J* = 7.0 Hz, 9H).

LC-MS (ESI): Calculated for 917.41, Found (M+H): 918.1.

### Example 5: Synthesis of Amino Lipid Compound 1504

According to the method of Example 1, the amino lipid compound 1504 was prepared according to the following reaction route, resulting in 210 mg of the amino lipid compound 1504 with a yield of 20.1% and a purity of 92.71%.

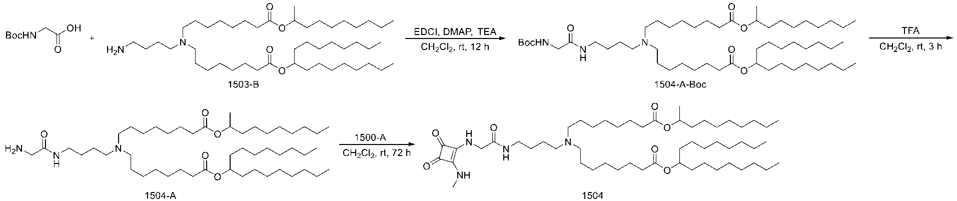

¹H NMR (600 MHz, CDCl₃) δ 4.97-4.85 (m, 2H), 4.33 (s, 2H), 3.36 (m, 3H), 3.27 (dd, *J* = 12.0, 6.1 Hz, 2H), 2.47 (dd, *J =* 14.6, 7.6 Hz, 6H), 2.32 (q, *J =* 7.6 Hz, 4H), 1.75-1.57 (m, 8H), 1.57-1.49 (m, 6H), 1.49-1.42 (m, 4H), 1.39-1.25 (m, 48H), 1.23 (d, *J=* 6.2 Hz, 3H), 0.92 (t, *J =* 6.9 Hz, 9H).

LC-MS (ESI): Calculated for 917.41, Found (M+H): 918.1.

### Example 6: Synthesis of Amino Lipid Compound 1505

According to the method of Example 1, the amino lipid compound 1505 was prepared according to the following reaction route, resulting in 980 mg of the amino lipid compound 1505 with a yield of 74.0% and a purity of 93.05%.

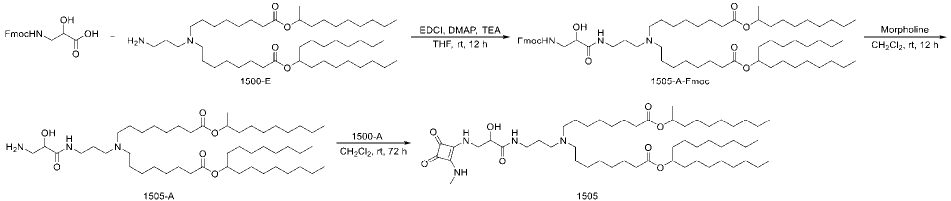

¹H NMR (600 MHz, CDCl₃) δ 7.99 (s, 2H), 7.76 (s, 1H), 4.91-4.83 (m, 2H), 4.28 (s, 1H), 3.74-3.70 (m, 3H), 3.30 (d, *J =* 2.7 Hz, 3H), 2.84 (s, 2H), 2.70 (s, 4H), 2.27 (q, *J =* 7.4 Hz, 4H), 1.89-1.73 (m, 2H), 1.54 (dd, *J =* 45.5, 19.5 Hz, 16H), 1.36-1.20 (m, 48H), 1.19 (d, *J =* 6.2 Hz, 3H), 0.87 (t, *J =* 6.9 Hz, 9H).

LC-MS (ESI): Calculated for 933.8, Found (M+H): 934.2.

### Example 7: Synthesis of Amino Lipid Compound 1512

### Step 1: Synthesis of 1512-AT

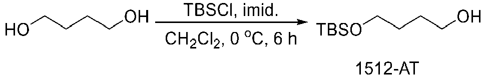

### Experimental procedure:

1,4-Butanediol (20.0 g, 222 mmol) was added to a 1 L single-necked flask, and then dichloromethane (660 mL) and imidazole (22.67 g, 333 mmol) were added. The reaction solution was cooled to 0°C, added with TBSCl (36.7 g, 244 mmol), and reacted at 0°C for 6 h. Saturated aqueous sodium bicarbonate solution (400 mL) was added. The organic phase was separated, and then extracted twice with dichloromethane (300 mL), and the organic phases were combined. The organic phase was then washed with saturated aqueous sodium chloride solution (300 mL), dried with anhydrous sodium sulfate, and filtered. The solvent was removed from the filtrate by evaporation under reduced pressure at 45°C, obtaining a coarse product of 1512-AT. The coarse product was purified by silica gel column chromatography and eluted with n-hexane:ethyl acetate = 5:1 to obtain 22.5 g of a colorless oily product of 1512-AT with a yield of 49.5%.

### Step 2: Synthesis of 1512-AQ

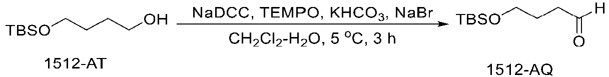

### Experimental procedure:

1512-AT (22.5 g, 110 mmol) was added to a 1 L single-necked flask, and then dichloromethane (300 mL), TEMPO (344 mg, 2.2 mmol), potassium bicarbonate (7.7 g, 77 mmol) and sodium bromide (453 mg, 4.4 mmol) were added. The reaction solution was cooled to 5°C, and then added dropwise with an aqueous solution of NaDCC (14.5 g, 66 mmol) using a constant-pressure funnel. After the dropwise addition was completed, the reaction was carried out at 5°C for 3 h, and filtered. The filter cake was washed once with water (50 mL) and once with dichloromethane (50 mL). The organic phase was separated from the filtrate. The water phase was extracted twice with dichloromethane (150 mL), and the organic phases were combined, dried with anhydrous sodium sulfate, and filtered. The solvent was removed from the filtrate by evaporation under reduced pressure at 45°C, obtaining a coarse product of 1512-AQ. The coarse product was purified by silica gel column chromatography and eluted with dichloromethane to obtain 11.4 g of a colorless oily product of 1512-AQ with a yield of 51.2%.

### Step 3: Synthesis of 1512-A

### Synthesis of 1512-AS

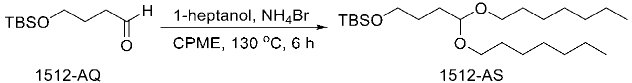

### Experimental procedure:

1512-AQ (10.0 g, 49.4 mmol) was added to a 250 mL single-necked flask, and then cyclopentyl methyl ether (80 mL), 1-heptanol (14.4 g, 123.5 mmol) and ammonium bromide (242 mg, 2.47 mmol) were added. A water separator device was mounted on the reaction flask, and the condenser was set to 10°C. The reaction solution was heated to 130°C for reflux to separate water, and reacted for 6 h. Filtering was then performed, and the solvent was removed from the filtrate by evaporation under reduced pressure at 45°C, obtaining a coarse product of 1512-AS. The coarse product was purified by silica gel column chromatography and eluted with n-hexane to obtain 9.55 g of a colorless oily product of 1512-AS with a yield of 46.4%.

### Synthesis of 1512-A

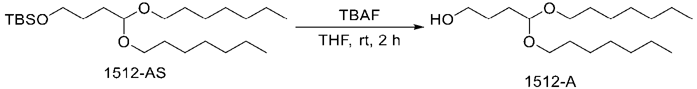

### Experimental procedure:

1512-AS (9.55 g, 22.9 mmol) was added to a 250 mL single-necked flask, and then added with tetrahydrofuran (110 mL) and TBAF (10.8 g, 34.4 mmol) to react at room temperature for 6 h. The reaction solution was added with saturated aqueous sodium bicarbonate solution (80 mL), then extracted twice with ethyl acetate (80 mL), and the organic phases were combined. The organic phase was then washed with saturated aqueous sodium chloride solution (100 mL), dried with anhydrous sodium sulfate, and filtered. The solvent was removed from the filtrate by evaporation under reduced pressure at 45°C, obtaining a coarse product of 1512-A. The coarse product was purified by silica gel column chromatography and eluted with n-hexane:ethyl acetate = 5:1 to obtain 6.05 g of a colorless oily product of 1512-A with a yield of 87.4%.

### Step 4: Synthesis of 1,8-octanedial

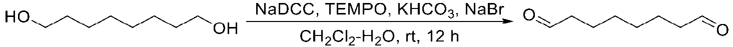

### Experimental procedure:

1,8-Octanediol (50.0 g, 342 mmol) was added to a 500 mL single-necked flask, and then dichloromethane (700 mL), TEMPO (2.67 g, 17.1 mmol), potassium bicarbonate (51.4 g, 513 mmol), and sodium bromide (2.8 g, 27.4 mmol) were added. The reaction solution was cooled to 5°C, then added dropwise with the aqueous solution of NaDCC (113.0 g, 513 mmol, dissolved in 500 mL water) using a constant-pressure funnel, and heated to room temperature to react for 12 h after the dropwise addition was completed. Filtering was then performed, and the filter cake was washed once with water (30 mL) and once with dichloromethane (30 mL). The organic phase was separated from the filtrate. The water phase was extracted twice with dichloromethane (500 mL). The organic phases were combined, dried with anhydrous sodium sulfate, and filtered. The solvent was removed from the filtrate by evaporation under reduced pressure at 45°C, obtaining a coarse product of 1,8-octanedial. The coarse product was purified by silica gel column chromatography and eluted with n-hexane:ethyl acetate = 2:1 to obtain 22.0 g 1,8-octanedial as a yellowish oil and 16.0 g 8-oxooctanoic acid as a white solid.

### Step 5: Synthesis of 8-oxooctanoic acid

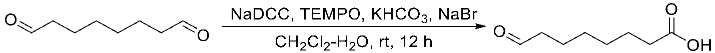

### Experimental procedure:

1,8-Octanedial (22.0 g, 154.7 mmol) was added to a 1 L single-necked flask, and then dichloromethane (300 mL), TEMPO (967 mg, 6.2 mmol), potassium bicarbonate (10.8 g, 108.3 mmol), and sodium bromide (1.27 g, 12.4 mmol) were added. The reaction solution was cooled to 5°C, then added dropwise with an aqueous solution of NaDCC (20.4 g, 92.8 mmol, dissolved in 200 mL water) using a constant-pressure funnel, and heated to room temperature to react for 12 h after the dropwise addition was completed. Filtering was then performed, and the filter cake was washed once with water (30 mL) and once with dichloromethane (30 mL). The organic phase was separated from the filtrate. The water phase was extracted twice with dichloromethane (300 mL). The organic phases were combined, dried with anhydrous sodium sulfate, and filtered. The solvent was removed from the filtrate by evaporation under reduced pressure at 45°C, obtaining a coarse product of 8-oxooctanoic acid. The coarse product was purified by silica gel column chromatography and eluted with n-hexane:ethyl acetate = 2:1 to obtain 8.52 g 8-oxooctanoic acid as a white solid. The yield of the two steps was 34.8%.

### Step 6: Synthesis of 1512-CB

### Synthesis of 1512-B

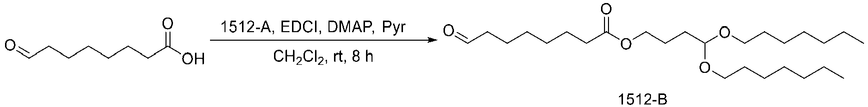

### Experimental procedure:

EDCI (6.75 g, 35.2 mmol), DMAP (800 mg, 7.04 mmol), and dichloromethane (70 mL) were added to a 500 mL round-bottom flask. The reaction solution was then cooled to -5°C, added dropwise with pyridine (2.8 g, 35.2 mmol) under stirring, and stirred at -5°C for 10 min after the dropwise addition was completed. 8-Oxooctanoic acid (3.7 g, 23.5 mmol) and 1512-A (7.1 g, 23.5 mmol) were added, and then warmed to room temperature to react for 8 h. Saturated aqueous sodium chloride solution (150 mL) was added for washing, and the organic phase was separated. The organic phase was dried with anhydrous sodium sulfate and filtered. The solvent was removed from the filtrate by evaporation under reduced pressure at 45°C, obtaining a coarse product of 1512-B. The coarse product was purified by silica gel column chromatography and eluted with n-hexane:ethyl acetate = 20:1 to obtain 5.53 g of a colorless oily product of 1512-B with a yield of 53.2%.

### Synthesis of 1512-CB

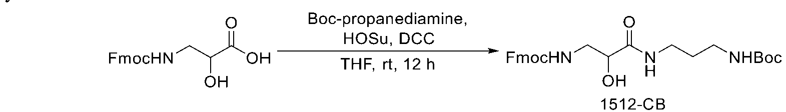

### Experimental procedure:

Fmoc-isoserine (10.0 g, 30.55 mmol) and tetrahydrofuran (70 mL) were added to a 250 mL round-bottom flask, added dropwise with HOSu (3.9 g, 33.6 mmol) and DCC (8.2 g, 39.7 mmol) under stirring at room temperature, and stirred at room temperature for 3 h, and then filtered, and the filtrate was retained for later use. Boc-propanediamine (5.3 g, 30.55 mmol), tetrahydrofuran (30 mL) and saturated sodium bicarbonate solution (20 mL) were added to another 250 mL round-bottom flask, and stirred until dissolved into a clear solution. The above-mentioned filtrate was then added to the reaction solution and reacted at room temperature for 12 h. The reaction solution was added with water (100 mL), and extracted twice with ethyl acetate (80 mL), and the organic phases were combined. The organic phase was then washed with saturated aqueous sodium chloride solution (150 mL), dried with anhydrous sodium sulfate, and filtered. The solvent was removed from the filtrate by evaporation under reduced pressure at 45°C, obtaining a coarse product of 1512-CB. The coarse product was purified by silica gel column chromatography and eluted with ethyl acetate:methanol = 10:1 to obtain 8.0 g 1512-CB as a white solid with a yield of 54.1%.

### Step 7: Synthesis of 1512-C

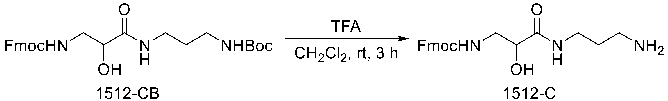

### Experimental procedure:

1512-CB (8.0 g, 16.5 mmol) and dichloromethane (80 mL) were added to a 250 mL round-bottom flask, added slowly with trifluoroacetic acid (25 mL) under stirring at room temperature, and reacted at room temperature for 3 h. The solvent was removed by evaporation under reduced pressure at 45°C, obtaining a coarse product of 1512-C. The coarse product was purified by silica gel column chromatography and eluted with ethyl acetate:methanol = 20:1 to obtain 4.7 g 1512-C as a white solid with a yield of 74.1%.

### Step 8: Synthesis of 1512-DF

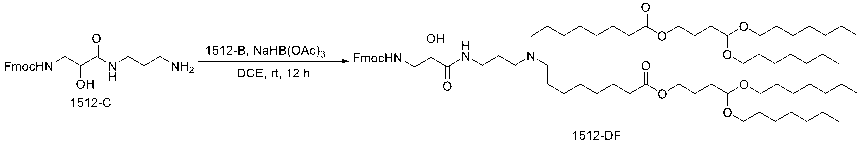

### Experimental procedure:

1512-C (4.7 g, 12.3 mmol), 1,2-dichloroethane (50 mL), and 1512-B (10.4 g, 24.5 mmol) were added to a 100 mL round-bottom flask, and stirred at room temperature for 0.5 h. NaHB(OAc)₃ (5.2 g, 24.5 mmol) was slowly added in batches to the reaction solution, and reacted at room temperature for 12 h. The solvent was removed by evaporation under reduced pressure at 45°C, obtaining a coarse product of 1512-DF. The coarse product was purified by silica gel column chromatography and eluted with ethyl acetate:methanol = 20:1 to obtain 4.45 g of a colorless oily product of 1512-DF with a yield of 29.2%.

### Step 9: Synthesis of 1512-D

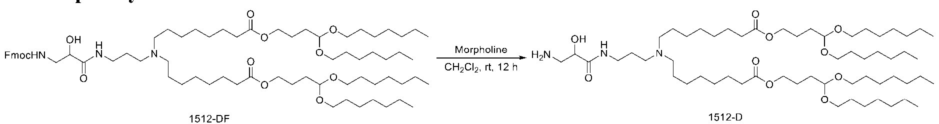

### Experimental procedure:

1512-DF (4.45 g, 3.6 mmol, 1.0 equivalent) and dichloromethane (50 mL) were added to a 250 mL round-bottom flask, added slowly with morpholine (25 mL) under stirring at room temperature, and reacted at room temperature for 12 h. The solvent was removed by evaporation under reduced pressure, and then ethyl acetate (100 mL) and water (100 mL) were added for extraction. The organic phase was separated. The water phase was extracted twice with ethyl acetate (100 mL), and the organic phases were combined. The organic phase was then washed with saturated aqueous sodium chloride solution (100 mL), dried with anhydrous sodium sulfate, and filtered. The solvent was removed from the filtrate by evaporation under reduced pressure at 45°C, thereby obtaining a coarse product of 1512-D. The coarse product was purified by column chromatography and eluted with ethyl acetate:methanol = 10:1 to obtain 2.47 g of a colorless oily product of 1512-D with a yield of 67.6%.

### Step 10: Synthesis of Amino Lipid Compound 1512

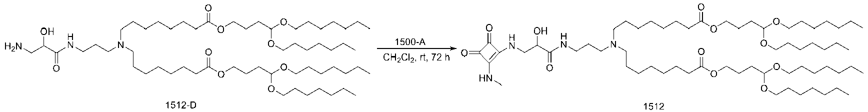

### Experimental procedure:

1512-D (2.47 g, 2.4 mmol), dichloromethane (50 mL), and 1500-E (344 mg, 2.4 mmol) were added to a 100 mL round-bottom flask, and reacted at room temperature for 72 h. 100 mL Water was added to the reaction solution to quench. The organic phase was separated. The water phase was extracted twice with dichloromethane (80 mL), and the organic phases were combined. The organic phase was then washed with saturated aqueous sodium chloride solution (100 mL), dried with anhydrous sodium sulfate, and filtered. The solvent was removed from the filtrate by evaporation under reduced pressure at 45°C, obtaining a coarse product of 1512. The coarse product was purified by silica gel column chromatography and eluted with ethyl acetate:methanol = 10:1 to obtain 1.0 g of white viscous product of the amino lipid compound 1512 with a yield of 37.1%.

¹H NMR (600 MHz, CDCl₃) δ 8.02 (s, 1H), 7.44 (s, 1H), 7.09 (s, 1H), 4.48 (t, *J =* 5.3 Hz, 2H), 4.08 (t, *J =* 6.1 Hz, 4H), 3.85 (s, 1H), 3.72 (dd, *J =* 14.0, 7.0 Hz, 3H), 3.57 (dt, *J* = 9.2, 6.7 Hz, 4H), 3.41 (dt, *J* = 9.3, 6.7 Hz, 4H), 3.30 (d, *J =* 4.9 Hz, 3H), 2.54-2.49 (m, 2H), 2.45 (d, *J =* 5.1 Hz, 2H), 2.40-2.35 (m, 4H), 2.30 (t, *J =* 7.5 Hz, 4H), 1.73-1.59 (m, 20H), 1.59-1.53 (m, 8H), 1.41 (dt, *J =* 14.7, 7.5 Hz, 4H), 1.37-1.25 (m, 38H), 1.24 (d, *J =* 7.0 Hz, 3H), 0.88 (t, *J =* 7.0 Hz, 9H).

LC-MS (ESI): Calculated for 1123.9, Found (M+H): 1124.4.

### Example 8: Synthesis of Amino Lipid Compound 1508

According to the method of Example 7, the amino lipid compound 1508 was prepared according to the following reaction route, resulting in 200 mg of the amino lipid compound 1508 with a yield of 30.0% and a purity of 91.70%.

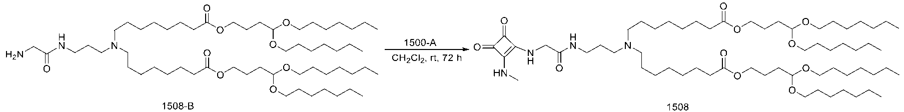

¹H NMR (600 MHz, CDCl₃) δ 8.32 (s, 1H), 7.44 (s, 1H), 7.20 (s, 1H), 4.47 (t, *J =* 5.0 Hz, 2H), 4.28 (s, 2H), 4.07 (t, *J* = 5.9 Hz, 4H), 3.72 (qd, *J* = 7.0, 1.6 Hz, 2H), 3.59-3.53 (m, 4H), 3.40 (dt, *J* = 7.4, 6.8 Hz, 4H), 3.30 (d, *J* = 4.0 Hz, 3H), 2.52 (t, *J* = 5.3 Hz, 2H), 2.44-2.37 (m, 4H), 2.29 (dd, *J =* 10.9, 4.2 Hz, 4H), 1.67 (t, *J = 10.1* Hz, 10H), 1.58 (ddd, *J=* 27.6, 13.8, 6.8 Hz, 12H), 1.45-1.38 (m, 4H), 1.37-1.22 (m, 44H), 0.88 (dd, *J* = 7.0, 6.3 Hz, 12H).

LC-MS (ESI): Calculated for 1093.9, Found(M+H): 1094.4.

### Example 9: Synthesis of Amino Lipid Compound 1509

According to the method of Example 7, the amino lipid compound 1509 was prepared according to the following reaction route, resulting in 100 mg of the amino lipid compound 1509 with a yield of 43.0% and a purity of 91.85%.

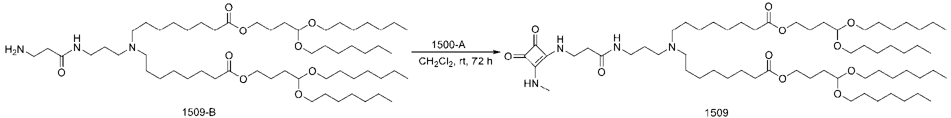

¹H NMR (600 MHz, CDCl₃) δ 7.97 (s, 1H), 7.44 (s, 1H), 7.09 (s, 1H), 4.48 (t, *J* = 5.3 Hz, 2H), 4.24 (t, *J* = 3.4 Hz, 2H), 4.08 (t, *J =* 6.1 Hz, 4H), 3.73 (dd, *J =* 14.0, 7.0 Hz, 2H), 3.57 (dt, *J =* 9.3, 6.7 Hz, 4H), 3.41 (dt, *J =* 9.3, 6.7 Hz, 4H), 3.33 (d, *J =* 3.9 Hz, 3H), 3.30-3.24 (m, 2H), 2.49 (s, 2H), 2.43-2.35 (m, 4H), 2.30 (t, *J =* 7.5 Hz, 4H), 1.73-1.59 (m, 20H), 1.59-1.53 (m, 8H), 1.41 (dd, *J =* 18.3, 11.1 Hz, 4H), 1.38-1.22 (m, 38H), 0.88 (t, *J =* 7.0 Hz, 12H).

LC-MS (ESI): Calculated for 1107.9, Found (M+H): 1108.5.

### Example 10: Synthesis of Amino Lipid Compound 1510

According to the method of Example 7, the amino lipid compound 1510 was prepared according to the following reaction route, resulting in 80 mg of the amino lipid compound 1510 with a yield of 19.0% and a purity of 96.95%.

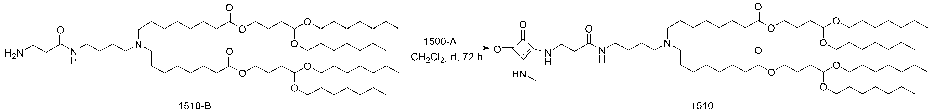

¹H NMR (600 MHz, CDCl₃) δ 7.44 (s, 1H), 7.20 (s, 1H), 7.09 (s, 1H), 4.48 (t, *J =* 5.3 Hz, 2H), 4.08 (t, *J* = 6.1 Hz, 4H), 3.72 (dd, *J* = 14.0, 7.0 Hz, 2H), 3.57 (dt, *J* = 9.2, 6.7 Hz, 4H), 3.41 (dt, *J* = 9.3, 6.7 Hz, 4H), 3.30 (d, *J =* 4.1 Hz, 3H), 3.23 (d, *J =* 5.7 Hz, 2H), 2.51 (d, *J =* 5.4 Hz, 2H), 2.41 (dd, *J =* 13.8, 6.8 Hz, 6H), 2.30 (t, *J =* 7.5 Hz, 4H), 1.79-1.64 (m, 16H), 1.64-1.45 (m, 14H), 1.41 (dt, *J =* 14.8, 7.5 Hz, 4H), 1.38-1.21 (m, 38H), 0.88 (t, *J =* 7.0 Hz, 12H).

LC-MS (ESI): Calculated for 1121.9, Found (M+H): 1122.5.

### Example 11: Synthesis of Amino Lipid Compound 1511

According to the method of Example 7, the amino lipid compound 1511 was prepared according to the following reaction route, resulting in 90 mg of the amino lipid compound 1511 with a yield of 20.3% and a purity of 95.93%.

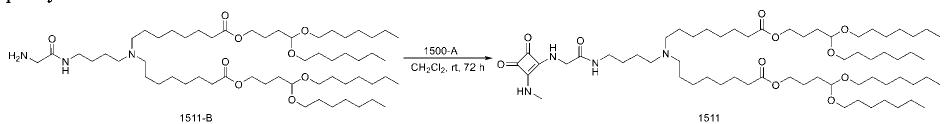

¹H NMR (600 MHz, CDCl₃) δ 8.62 (s, 1H), 7.44 (s, 1H), 7.09 (s, 1H), 4.48 (t, *J =* 5.3 Hz, 2H), 4.26 (s, 2H), 4.08 (t, *J =* 6.2 Hz, 4H), 3.57 (dt, *J =* 9.2, 6.7 Hz, 4H), 3.41 (dt, *J =* 9.2, 6.7 Hz, 4H), 3.31 (d, *J* = 2.4 Hz, 3H), 3.25 (d, *J* = 5.6 Hz, 2H), 2.54-2.44 (m, 6H), 2.30 (t, *J =* 7.5 Hz, 4H), 1.78-1.64 (m, 16H), 1.64-1.49 (m, 14H), 1.45 (t, *J* = 10.9 Hz, 4H), 1.38-1.22 (m, 38H), 0.88 (t, *J =* 7.0 Hz, 12H).

LC-MS (ESI): Calculated for 1107.9, Found (M+H): 1108.5.

### Example 12: Synthesis of Amino Lipid Compound 1507

According to the process of Step 10 of Example 7, the amino lipid compound 1507 was prepared according to the following reaction route, resulting in 100 mg of the amino lipid compound 1507 with a yield of 22.4% and a purity of 92.00%.

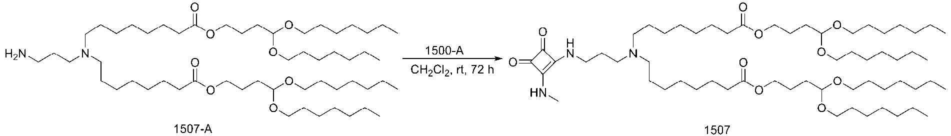

¹H NMR (600 MHz, CDCl₃) δ 4.48 (t, *J =* 5.2 Hz, 2H), 4.08 (t, *J =* 6.1 Hz, 4H), 3.72 (dd, *J =* 14.0, 7.0 Hz, 2H), 3.57 (dt, *J* = 9.2, 6.7 Hz, 4H), 3.41 (dt, *J =* 9.2, 6.7 Hz, 4H), 3.26 (d, *J =* 4.8 Hz, 3H), 2.58 (s, 2H), 2.48-2.41 (m, 4H), 2.30 (t, *J =* 7.5 Hz, 4H), 1.79-1.64 (m, 14H), 1.62 (dd, *J =* 14.5, 7.3 Hz, 4H), 1.59-1.53 (m, 8H), 1.43 (dt, *J =* 14.9, 7.5 Hz, 4H), 1.38-1.22 (m, 40H), 0.88 (t, *J =* 6.9 Hz, 12H).

LC-MS (ESI): Calculated for 1036.9, Found(M+H): 1037.3.

As for 1507-A, with reference to the procedures of Step 8 and Step 9 for 1512-D in Example 7, the compound 1507-A was prepared according to the following route, resulting in 930 mg compound 1507-A with a yield of 69.3%.

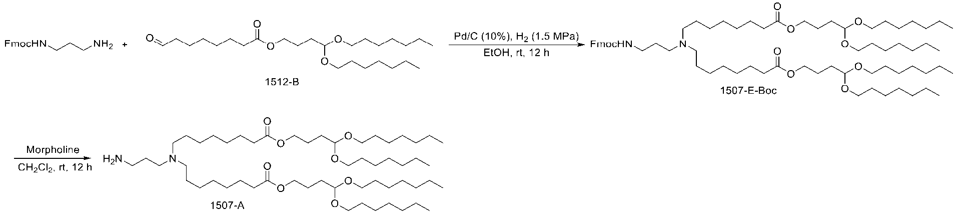

### Example 13: Synthesis of Amino Lipid Compound 1849

### Step 1: Synthesis of

### 4-TBS-BHB

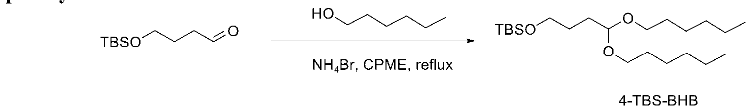

### Experimental procedure:

TBS-butyraldehyde (15.0 g, 74.12 mmol), CPME (75 mL), hexanol (18.93 g, 185.3 mmol), and ammonium bromide (0.36 g, 3.7 mmol) were added to a 250 mL single-necked round-bottom flask. A water separator device was mounted on the reaction flask, and the condenser was set to 10°C. The reaction solution was heated to 130°C for reflux to separate water, and reacted for 8 h. Filtering was then performed, and the filtrate was collected. The solvent was removed from the filtrate by evaporation under reduced pressure at 45°C, obtaining 36.0 g of a coarse product of 4-TBS-BHB. The coarse product was purified by silica gel column chromatography and eluted with ethyl acetate:n-heptane = 1: 80 to obtain 24.09 g 4-TBS-BHB with a yield of 83.6%.

### Step 2: Synthesis of 4-BHB

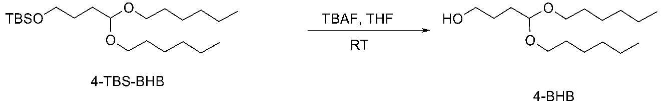

### Experimental procedure:

4-TBS-BHB (24.0 g, 61.74 mmol), tetrahydrofuran (120 mL), and TBAF·3H₂O (25.32 g, 80.27 mmol) were sequentially added to a 500 mL single-necked round-bottom flask, and stirred at room temperature overnight. The solvent was removed by evaporation under reduced pressure at 45°C. Water (200 mL) was added, followed by extraction twice with ethyl acetate (200 mL). The organic phases were separated and combined. The organic phase was then washed with saturated aqueous sodium chloride solution (100 mL), dried with anhydrous sodium sulfate, and filtered. The solvent was removed from the filtrate by evaporation under reduced pressure at 45°C, obtaining 17.2 g of a coarse product of 4-BHB. The coarse product was purified by silica gel column chromatography and eluted with ethyl acetate:n-heptane = 1:30 to obtain 15.1 g 4-BHB with a yield of 89.1 %.

### Step 3: Synthesis of DHN-T

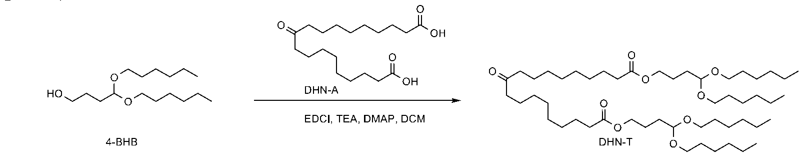

### Experimental procedure:

EDCI (11.12 g, 58 mmol), dichloromethane (80 mL), DMAP (566 mg, 4.64 mmol), and triethylamine (7.04 g, 69.6 mmol) were added to a 250 mL single-necked flask, stirred at room temperature, added with DHN-A (8.0 g, 23.2 mmol) and 4-BHB (11.46 g, 41.76 mmol), and reacted at room temperature overnight. Saturated aqueous ammonium chloride solution (100 mL) was added for quenching. The organic phase was separated. The water phase was extracted twice with dichloromethane (100 mL), and the organic phases were combined. The organic phase was then washed with saturated aqueous sodium chloride solution (100 mL). The solvent was removed by evaporation under reduced pressure at 45°C, obtaining 25.5 g of a coarse product of DHN-T. The coarse product was purified by silica gel column chromatography and eluted with ethyl acetate:n-heptane = 1: 10 to obtain 15.5 g DHN-T with a yield of 86.9%.

### Step 4: Synthesis of DHN-C

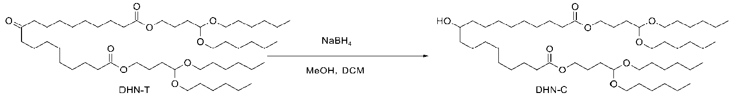

### Experimental procedure:

DHN-T (17.2 g, 20.16 mmol), dichloromethane (86 mL), and methanol (86 mL) were added to a 250 mL single-necked flask, stirred at 0°C, then added with sodium borohydride (0.8 g, 21.17 mmol), and reacted at 0°C for 2 h. Water (200 mL) was added at 0°C for quenching. The organic phase was separated. The water phase was extracted twice with dichloromethane (150 mL), and the organic phases were separated and mixed. The organic phase was washed with saturated aqueous sodium chloride solution (100 mL), dried with anhydrous sodium sulfate, and filtered. The solvent was removed from the filtrate by evaporation under reduced pressure at 45°C, obtaining 18.0 g of a coarse product of DHN-C. The coarse product was purified by silica gel column chromatography and eluted with ethyl acetate:n-heptane = 1:30 to obtain 16.5 g DHN-C with a yield of 95.6%.

### Step 5: Synthesis of 1849-A4

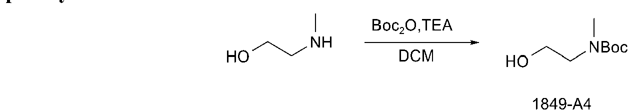

### Experimental procedure:

2-(Methylamino)ethanol (2.0 g, 26.6 mmol), triethylamine (4.04 g, 39.9 mmol), and dichloromethane (20 mL) were added to a 250 mL single-necked flask, stirred at 5°C for 15 min, then added with Boc anhydride (7.0 g, 31.9 mmol), stirring was continued at 5°C for 10 min, followed by stirring at room temperature overnight. The solvent was removed by evaporation under reduced pressure at 45°C, obtaining 4.7 g 1849-A4, which may be directly used in the reaction of the next step without purification.

### Step 6: Synthesis of 1849-A5

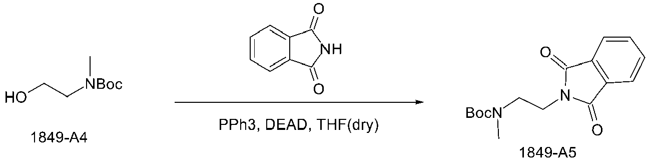

### Experimental procedure:

1849-A4 (4.7 g, 26.82 mmol), anhydrous THF (47 mL), PPh₃ (9.07 g, 34.58 mmol), and phthalimide (4.3 g, 29.26 mmol) were sequentially added to a 100 mL single-necked flask, stirred at 0°C for 15 min, and then added dropwise with DEAD (6.02 g, 34.58 mmol). After the dropwise addition was completed, the mixture was reacted at room temperature overnight. After the solvent was removed by evaporation under reduced pressure at 45°C, water (100 mL) was added, followd by extraction twice with ethyl acetate (100 mL). The organic phases were separated and combined. The organic phase was washed with saturated aqueous sodium chloride solution (100 mL), dried with anhydrous sodium sulfate, and filtered. The solvent was removed from the filtrate by evaporation under reduced pressure at 45°C, obtaining 18.0 g of a coarse product of 1849-A5. The coarse product was purified by silica gel column chromatography and eluted with ethyl acetate:n-heptane = 1:1 to obtain 7.28 g 1849-A5 with a yield of 89.2%.

### Step 7: Synthesis of 1849-A6

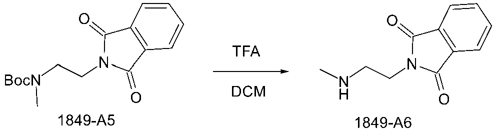

### Experimental procedure:

1849-A5 (7.28 g, 23.9 mmol) and dichloromethane (73 mL) were sequentially added to a 100 mL single-necked flask, stirred at room temperature until dissolved into a clear solution, then added dropwise with TFA (24 mL), and stirred at room temperature for 2 h. The solvent was removed by evaporation under reduced pressure at 45°C, and ethyl acetate (50 mL) was added. Saturated aqueous potassium bicarbonate solution (100 mL) was added for washing. The organic phase was separated. The water phase was extracted twice with ethyl acetate (50 mL), and the organic phases were combined. The organic phase was then washed with saturated aqueous sodium chloride solution (100 mL), dried with anhydrous sodium sulfate, and filtered. The solvent was removed from the filtrate by evaporation under reduced pressure at 45°C, obtaining 4.88 g of a coarse product of 1849-A6, which may be directly used in the reaction of the next step without purification.

### Step 8: Synthesis of 1849-A7

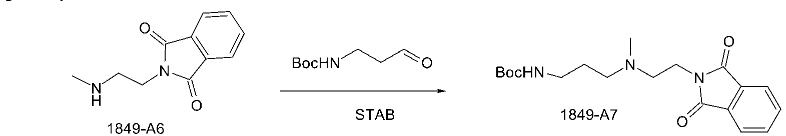

### Experimental procedure:

1849-A6 (4.88 g, 23.9 mmol), DCE (49 mL), and Boc aminopropanal (4.97 g, 28.68 mmol) were sequentially added to a 100 mL single-necked flask, stirred at room temperature for 30 min, then added with STAB (6.08 g, 28.68 mmol), and stirred at room temperature overnight. Saturated aqueous sodium bicarbonate solution (50 mL) was added to the reaction solution for washing. The organic phase was separated. The water phase was extracted twice with ethyl acetate (50 mL), and the organic phases were combined. The organic phase was washed with saturated aqueous sodium chloride solution (100 mL), dried with anhydrous sodium sulfate, and filtered. The solvent was removed from the filtrate by evaporation under reduced pressure at 45°C, obtaining 10.1 g of a coarse product of 1849-A7. The coarse product was purified by silica gel column chromatography and eluted with ethyl acetate:n-heptane = 1:1 to obtain 6.2 g 1849-A7 with a yield of 71.8%.

### Step 9: Synthesis of 1849-A8

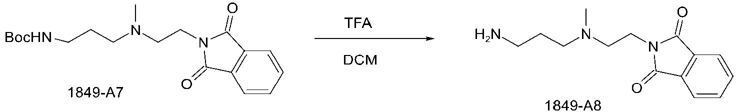

### Experimental procedure:

1849-A7 (6.2 g, 17.2 mmol) and dichloromethane (62 mL) were sequentially added to a 100 mL single-necked flask, stirred at room temperature until dissolved into a clear solution, then added dropwise with TFA (20 mL), and stirred at room temperature for 2 h. The solvent was removed by evaporation under reduced pressure at 45°C, and ethyl acetate (50 mL) was added. Saturated aqueous potassium bicarbonate solution (100 mL) was added for washing. The organic phase was separated. The water phase was extracted twice with ethyl acetate (50 mL), and the organic phases were combined. The organic phase was washed with saturated aqueous sodium chloride solution (100 mL), dried with anhydrous sodium sulfate, and filtered. The solvent was removed from the filtrate by evaporation under reduced pressure at 45°C, obtaining 4.5 g of a coarse product of 1849-A8, which may be directly used in the reaction of the next step without purification.

### Step 10: Synthesis of DHN-X

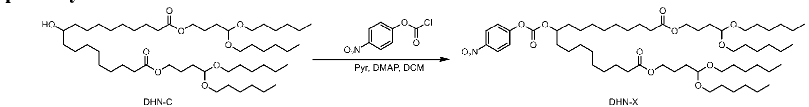

### Experimental procedure:

DHN-C (16.5 g, 19.2 mmol), dichloromethane (165 mL), pyridine (2.28 g, 28.8 mmol), and DMAP (234 mg, 1.92 mmol) were added to a 250 mL single-necked flask, stirred at room temperature, added with p-nitrophenyl chloroformate (5.8 g, 28.8 mmol), and reacted at room temperature for 2 h. Water (200 mL) was added for quenching. The organic phase was separated. The water phase was extracted twice with dichloromethane (150 mL), and the organic phases were combined. The organic phase was washed with 100 mL saturated aqueous sodium chloride solution, dried with anhydrous sodium sulfate, and filtered. The solvent was removed from the filtrate by evaporation under reduced pressure at 45°C. The residue was added with n-heptane (100 mL) for extraction, and filtered. The filter cake was washed with n-heptane (30 mL). The filtrate was collected. The solvent was removed from the filtrate by evaporation under reduced pressure at 45°C, obtaining 20.3 g of a coarse product of DHN-X. The coarse product was purified by silica gel column chromatography and eluted with ethyl acetate:n-heptane = 1:10 to obtain 16.7 g DHN-X with a yield of 85.1%.

### Step 11: Synthesis of 1849-A9

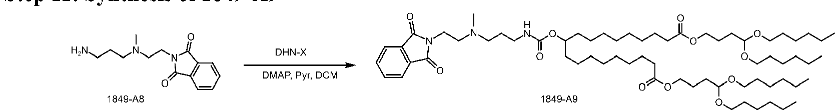

### Experimental procedure:

1849-A8 (1.0 g, 3.8 mmol), dichloromethane (10 mL), pyridine (454 mg, 5.7 mmol), and DHN-X (4.66 g, 4.56 mmol) were sequentially added to a 100 mL single-necked flask, stirred at room temperature, then added with DMAP (46 mg, 0.38 mmol), and stirred at room temperature for 48 h. Water (30 mL) was added for quenching. The organic phase was separated. The water phase was extracted twice with dichloromethane (15 mL), and the organic phases were combined. The organic phase was washed with saturated aqueous sodium chloride solution (20 mL), dried with anhydrous sodium sulfate, and filtered. The solvent was removed from the filtrate by evaporation under reduced pressure at 45°C, obtaining 5.8 g of a coarse product of 1849-A9. The coarse product was purified by silica gel column chromatography and eluted with ethyl acetate:n-heptane = 1:3 to obtain 2.6 g 1849-A9 with a yield of 59.8%.

### Step 12: Synthesis of 1849-A10

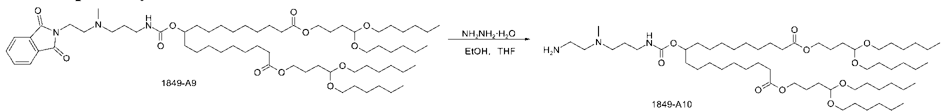

### Experimental procedure:

1849-A9 (2.6 g, 2.28 mmol), absolute ethyl alcohol (13 mL), and THF (13 mL) were sequentially added to a 100 mL single-necked flask, added dropwise with hydrazine hydrate (570 mg, 11.4 mmol), and warmed to 85°C for reflux for 2 h. Water (50 mL) was added, followd by extraction with ethyl acetate (100 mL). The organic phase was separated. The water phase was then extracted twice with ethyl acetate (50 mL), and the organic phases were combined. The organic phase was then washed with saturated aqueous sodium chloride solution (100 mL), dried with anhydrous sodium sulfate, and filtered. The solvent was removed from the filtrate by evaporation under reduced pressure at 45°C, obtaining 2.4 g of a coarse product of 1849-A10, which may be directly used in the reaction of the next step without purification.

### Step 13: Synthesis of Amino Lipid Compound 1849

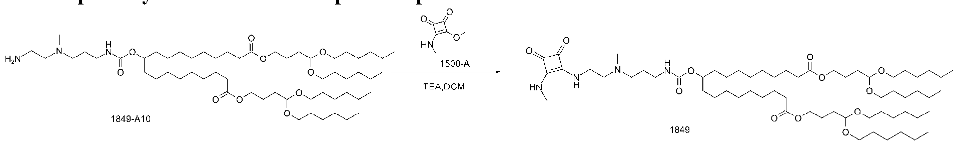

### Experimental procedure:

1849-A10 (1.22 g, 1.2 mmol), 1500-A (203 mg, 1.44 mmol), and dichloromethane (12 mL) were added to a 50 mL flask, added dropwise with triethylamine (121 mg, 1.2mmol) under stirring, and stirred at room temperature overnight. The reaction solution was sequentially washed with saturated aqueous potassium bicarbonate solution (20 mL) and saturated aqueous sodium chloride solution, then dried with anhydrous sodium sulfate, and filtered. The solvent was removed from the filtrate by evaporation under reduced pressure at 45°C. The residue was purified by silica gel column chromatography and eluted with ethyl acetate:methanol = 20:1, obtaining 400 mg of the amino lipid compound 1849 with a yield of 30.0 % and a purity of 90.5%.

¹H NMR (600 MHz, CDCl₃) δ 4.54 (dt, J = 12.2, 6.1 Hz, 1H), 4.49 (t, J = 5.2 Hz, 2H), 4.09 (t, J = 6.1 Hz, 4H), 3.77 (d, J = 5.0 Hz, 2H), 3.58 (dt, J = 9.2, 6.7 Hz, 4H), 3.42 (dt, J = 9.3, 6.7 Hz, 6H), 3.32 (d, J = 5.0 Hz, 3H), 2.55-2.48 (m, 2H), 2.47-2.40 (m, 2H), 2.30 (t, J = 7.5 Hz, 4H), 2.15 (s, 3H), 1.70 (ddd, J = 10.2, 5.8, 2.3 Hz, 10H), 1.63-1.54 (m, 12H), 1.51 (d, J = 4.4 Hz, 4H), 1.33 (tdd, J = 14.8, 11.8, 6.4 Hz, 44H), 0.90 (t, J = 6.9 Hz, 12H).

LC-MS (ESI): Calculated for 1123.65, Found (M+H): 1124.4.

### Example 14: Synthesis of Amino Lipid Compound 1845

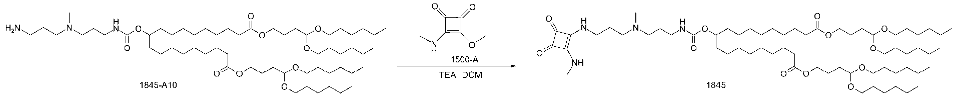

According to the general synthetic method, the amino lipid compound 1845 was synthesized according to the process of Step 13 of Example 13 with substituting compound 1845-A10 for 1849-A10, wherein 1845-A10 (1.92g, 1.86 mmol), 1500-A (316 mg, 2.24 mmol), and triethylamine (188 mg, 1.86 mmol) were used, resulting in 350 mg of the amino lipid compound 1845 with a yield of 16.5% and a purity of 97.24%.

¹H NMR (600 MHz, CDCl₃) δ 4.57-4.50 (m, 1H), 4.47 *(t, J =* 5.2 Hz, 2H), 4.07 *(t, J =* 6.1 Hz, 4H), 3.81 (s, 2H), 3.56 (dt, *J =* 9.2, 6.7 Hz, 4H), 3.40 (dt, *J* = 9.2, 6.7 Hz, 4H), 3.28 (dd, *J =* 13.5, 5.7 Hz, 5H), 2.45-2.38 (m, 2H), 2.37-2.30 (m, 2H), 2.28 (t, *J =* 7.5 Hz, 4H), 2.13 (s, 3H), 1.77 (d, *J =* 4.8 Hz, 4H), 1.71-1.64 (m, 8H), 1.61-1.49 (m, 16H), 1.40-1.17 (m, 44H), 0.88 (t, *J* = 6.9 Hz, 12H).

LC-MS (ESI): Calculated for 1137.68, Found (M+H): 1138.4.

### Example 15: Synthesis of Amino Lipid Compound 1847

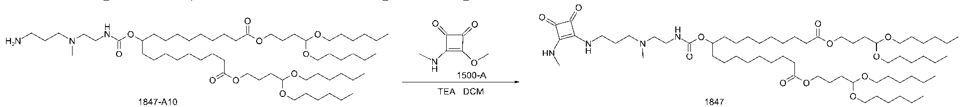

According to the general synthetic method, the amino lipid compound 1847 was synthesized according to the process of Step 13 of Example 13 with substituting compound 1847-A10 for 1849-A10, wherein 1847-A10 (650 mg, 0.64 mmol), 1500-A (109 mg, 0.77 mmol), and triethylamine (65 mg, 0.64 mmol) were used, resulting in 200 mg of the amino lipid compound 1847 with a yield of 27.8% and a purity of 94.46%.

¹H NMR (600 MHz, CDCl₃) δ 4.49 (s, 1H), 4.42 (t, *J =* 5.3 Hz, 2H), 4.02 (t, *J =* 6.2 Hz, 4H), 3.68 (s, 2H), 3.51 (dt, *J =* 9.3, 6.7 Hz, 4H), 3.35 (dt, *J =* 9.3, 6.7 Hz, 4H), 3.26 (d, *J* = 5.0 Hz, 5H), 2.46 (t, *J* = 5.9 Hz, 2H), 2.42-2.38 (m, 2H), 2.23 (t, *J =* 7.6 Hz, 4H), 2.16 (d, *J =* 4.5 Hz, 3H), 1.74-1.69 (m, 2H), 1.67-1.58 (m, 8H), 1.55-1.48 (m, 12H), 1.43 (d, *J* = 2.7 Hz, 4H), 1.25 (ttd, *J =* 15.5, 14.4, 7.1 Hz, 44H), 0.83 (t, *J =* 7.0 Hz, 12H).

LC-MS (ESI): Calculated for 1123.65, Found (M+H): 1124.4.

### Example 16: Synthesis of Amino Lipid Compound 1844

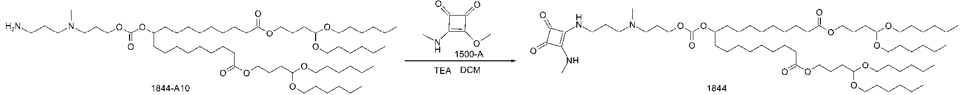

According to the general synthetic method, the amino lipid compound 1844 was synthesized according to the process of Step 13 of Example 13 with substituting compound 1844-A10 for 1849-A10, wherein 1844-A10 (1.5g, 1.46 mmol), 1500-A (317 mg, 2.3 mmol), and triethylamine (151 mg, 1.5 mmol) were used, resulting in 802 mg of the amino lipid compound 1844 with a yield of 48.2% and a purity of 95.67%.

¹H NMR (600 MHz, CDCl₃) δ 4.68-4.56 (m, 1H), 4.47 *(t, J =* 5.2 Hz, 2H), 4.26-4.18 (m, 2H), 4.11-4.02 (m, 4H), 3.69 (s, 2H), 3.59-3.51 (m, 4H), 3.43-3.36 (m, 4H), 3.29 (d, *J =* 4.9 Hz, 3H), 2.50-2.43 (m, 2H), 2.43-2.36 (m, 2H), 2.27 (t, *J* = 7.6 Hz, 4H), 2.18 (s, 3H), 1.96-1.73 (m, 4H), 1.74-1.63 (m, 8H), 1.63-1.48 (m, 16H), 1.41-1.19 (m, 44H), 0.95-0.80 (m, 12H).

LC-MS (ESI): Calculated for 1138.66, Found(M+H): 1139.5.

### Example 17: Synthesis of Amino Lipid Compound 1846

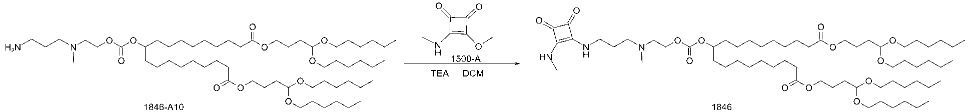

According to the general synthetic method, the amino lipid compound 1846 was synthesized according to the process of Step 13 of Example 13 with substituting compound 1846-A10 for 1849-A10, wherein 1846-A10 (1.3 g, 1.3 mmol), 1500-A (268 mg, 1.9 mmol), and triethylamine (131.5 mg, 1.3 mmol) were used, resulting in 515 mg of the amino lipid compound 1846 with a yield of 35.2% and a purity of 96.35%.

¹H NMR (600 MHz, CDCl₃) δ 4.67-4.57 (m, 1H), 4.45 (t, *J =* 5.2 Hz, 2H), 4.24 (t, *J =* 5.0 Hz, 2H), 4.04 (t, *J* = 6.1 Hz, 4H), 3.85-3.60 (m, 2H), 3.59-3.49 (m, 4H), 3.43-3.33 (m, 4H), 3.28 (t, *J =* 12.2 Hz, 3H), 2.63 (t, *J =* 5.1 Hz, 2H), 2.52 (t, *J* = 5.7 Hz, 2H), 2.31-2.18 (m, 7H), 1.80-1.70 (m, 2H), 1.71-1.59 (m, 8H), 1.61-1.46 (m, 16H), 1.38-1.17 (m, 44H), 0.89-0.81 (m, 12H).

LC-MS (ESI): Calculated for 1124.64, Found(M+H): 1125.5.

### Example 18: Synthesis of Amino Lipid Compound 1848

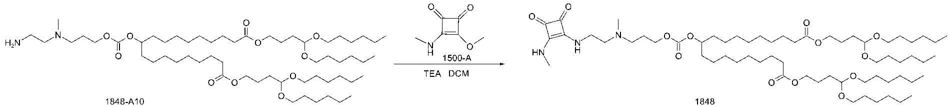

According to the general synthetic method, the amino lipid compound 1846 was synthesized according to the process of Step 13 of Example 13 with substituting compound 1848-A10 for 1849-A10, wherein 1848-A10 (900 mg, 0.89 mmol), 1500-A (187 mg, 1.33 mmol), and triethylamine (90 mg, 0.89 mmol) were used, resulting in 300 mg of the amino lipid compound 1848 with a yield of 30.0% and a purity of 94.80%.

¹H NMR (600 MHz, CDCl₃) δ 4.60-4.53 (m, 1H), 4.47 (t, *J =* 5.2 Hz, 2H), 4.34 (t, *J =* 5.6 Hz, 2H), 4.06 (t, *J =* 6.1 Hz, 4H), 3.73 (d, *J* = 4.9 Hz, 2H), 3.55 (dt, *J =* 9.2, 6.7 Hz, 4H), 3.39 (dt, *J* = 9.2, 6.7 Hz, 4H), 3.31 (d, *J* = 5.0 Hz, 3H), 2.55-2.49 (m, 2H), 2.46 (t, *J =* 6.1 Hz, 2H), 2.27 (t, *J =* 7.6 Hz, 4H), 2.16 (s, 3H), 1.84 (dt, *J =* 11.8, 5.8 Hz, 2H), 1.73-1.63 (m, 8H), 1.61-1.49 (m, 16H), 1.34-1.23 (m, 44H), 0.91-0.83 (m, 12H).

LC-MS (ESI): Calculated for 1124.64, Found(M+H): 1125.5.

### Example 19: Synthesis of Amino Lipid Compound 1883

### Step 1: Synthesis of 1883-A

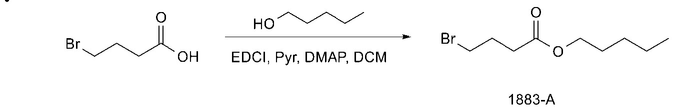

### Experimental procedure:

EDCI (15.18 g, 79.2 mmol), DMAP (806 mg, 6.6 mmol), pyridine (6.26 g, 79.2 mmol), and dichloromethane (110 mL) were added to a 250 mL single-necked flask, added with 4-bromo-butyric acid (11.0 g, 65.87 mmol) and n-pentanol (5.8 g, 66 mmol) under stirring at room temperature, and reacted at room temperature overnight. Water (100 mL) was added for quenching. The organic phase was separated. The water phase was extracted twice with dichloromethane (100 mL), and the organic phases were combined. The organic phase was washed with saturated aqueous sodium chloride solution (100 mL). The solvent was removed by evaporation under reduced pressure at 45°C, obtaining 19.5 g of a coarse product of 1883-A. The coarse product was purified by silica gel column chromatography and eluted with ethyl acetate:n-heptane = 1:10 to obtain 14.9 g 1883-A with a yield of 95.34%.

### Step 2: Synthesis of 1883-B

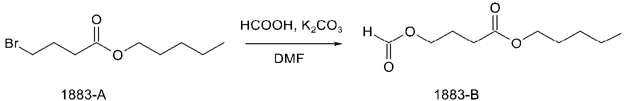

### Experimental procedure:

K₂CO₃ (6.5 g, 47 mmol) and DMF (150 mL) were sequentially added to a 500 mL single-necked round-bottom flask, then added dropwise with HCOOH (4.6 g, 100.3 mmol), stirred for 1 h, then added dropwise with 1883-A (12.9 g, 54.6 mmol), heated to 85°C, and stirred for 5 h. The reaction solution was added with water (200 mL), and extracted twice with ethyl acetate (200 mL). The organic phases were separated and combined. The organic phase was then washed with saturated aqueous sodium bicarbonate solution (100 mL), dried with anhydrous sodium sulfate, and filtered. The solvent was removed from the filtrate by rotary evaporation under reduced pressure at 45°C, obtaining 13.5 g of a coarse product of 1883-B, which may be directly used in the reaction of the next step without purification.

### Step 3: Synthesis of 1883-C

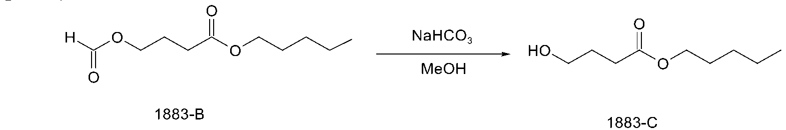

### Experimental procedure:

1883-B (13.5 g, 66.7 mmol) and methanol (135 mL) were added to a 500 mL round-bottom flask, then added with sodium bicarbonate powder (2.24 g, 26.7 mmol) with stirring at room temperature, and stirred at room temperature for 3 h. The reaction solution was filtered, evaporated under reduced pressure at 45°C to remove the solvent, and added wiht water (200 mL), followed by extraction twice with dichloromethane (200 mL). The organic phases were combined. The organic phase was washed with saturated aqueous sodium chloride solution (200 mL), dried with anhydrous sodium sulfate, and filtered. The solvent was removed from the filtrate by evaporation under reduced pressure at 45°C, obtaining a coarse product. The coarse product was purified by silica gel column chromatography and eluted with n-hexane:ethyl acetate = 10:1 to obtain 11.0 g 1883-C with a yield of 94.7%.

### Step 4: Synthesis of 1883-D

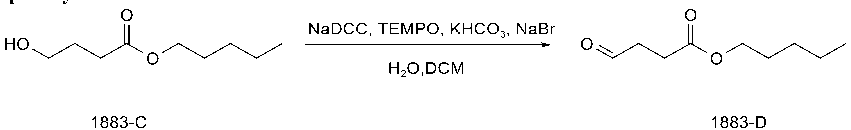

### Experimental procedure:

1883-C (11.0 g, 63.13 mmol), dichloromethane (30 mL), TEMPO (197 mg, 1.26 mmol), KHCO₃ (4.4 g, 44.19 mmol), and NaBr (259 mg, 2.52 mmol) were added to a 250 mL single-necked flask, stirred at 5°C for 15 min, then added dropwise with an aqueous solution of NaDCC (8.33 g, 37.87 mmol, dissolved in 70 mL water), and reacted at 5°C for 2 h. The organic phase was separated from the reaction solution. Dichloromethane (150 mL) was added to the water phase for extraction twice, and the organic phases were combined. The organic phase was washed with saturated aqueous sodium chloride solution (100 mL), dried with anhydrous sodium sulfate, and filtered. The solvent was removed from the filtrate by evaporation under reduced pressure at 45°C, obtaining 11.6 g of a coarse product of 1883-D. The coarse product was purified by silica gel column chromatography and eluted with ethyl acetate:n-heptane = 1:10 to obtain 9.24 g 1883-D with a yield of 85.0%.

### Step 5: Synthesis of DEN-T

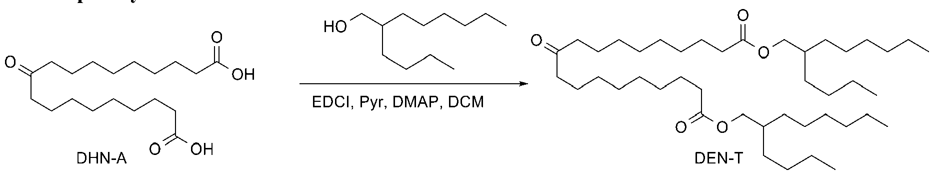

### Experimental procedure:

EDCI (11.12 g, 58 mmol), DMAP (566 mg, 4.64 mmol), pyridine (5.51 g, 69.6 mmol), and dichloromethane (95 mL) were added to a 250 mL single-necked flask, stirred at room temperature, then added with DHN-A (8g, 23.36 mmol) and 2-butyl-octanol (9.5 g, 51 mmol), and reacted at room temperature overnight. Saturated aqueous ammonium chloride solution (100 mL) was added for quenching. The organic phase was separated. The water phase was extracted twice with dichloromethane (100 mL), and the organic phases were combined. The organic phase was washed with saturated aqueous sodium chloride solution (100 mL). The solvent was removed by evaporation under reduced pressure at 45°C, obtaining 18.2 g of a coarse product of DEN-T. The coarse product was purified by silica gel column chromatography and eluted with ethyl acetate:n-heptane = 1:10 to obtain 13.8 g DEN-T with a yield of 86.9%.

### Step 6: Synthesis of 1883-A1

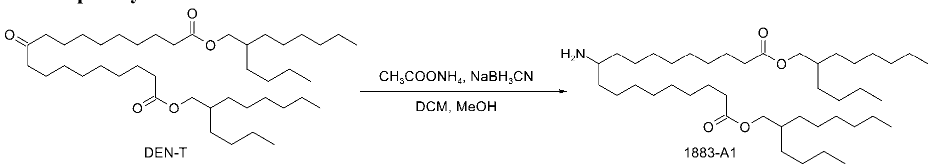

### Experimental procedure:

DEN-T (15.0 g, 22.1 mmol), dichloromethane (75 mL), methanol (75 mL), and ammonium acetate (15.0 g, 194 mmol) were added to a 500 mL single-necked round-bottom flask, added with sodium cyanoborohydride (1.38 g) under stirring, and stirred at room temperature overnight. Filtering was performed, and water (150 mL) was added to the filtrate for washing. The organic phase was separated. The water phase was further extracted once with dichloromethane (150 mL), and the organic phases were combined. The organic phase was washed with saturated aqueous sodium bicarbonate solution (150 mL). The solvent was removed by evaporation under reduced pressure at 45°C, obtaining 17.0 g of a coarse product of 1883-A1. The coarse product was purified by silica gel column chromatography and eluted with ethyl acetate:methanol = 20:1 to obtain 7.8 g 1883-A1 with a yield of 51.9%.

### Step 7: Synthesis of 1883-A2

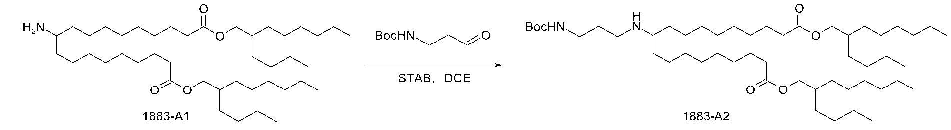

### Experimental procedure:

1883-A1 (7.8 g, 11.47 mmol), Boc-aminopropanal (2.65 g, 15.28 mmol), and 1,2-dichloroethane (26 mL) were added to a 250 mL single-necked round-bottom flask, stirred at room temperature for 30 min, then added with sodium triacetoxyborohydride (3.23 g, 15.28 mmol), and stirred at room temperature overnight. Water (80 mL) was added to the reaction solution for washing. The organic phase was separated. Dichloromethane (100 mL) was added to the water phase for extraction, and the organic phases were combined. The organic phase was washed with saturated aqueous sodium bicarbonate solution (100 mL). The solvent was removed by evaporation under reduced pressure at 45°C, obtaining 9.5 g of a coarse product of 1883-A2. The coarse product was purified by silica gel column chromatography and eluted with ethyl acetate:methanol = 20:1 to obtain 4.3 g 1883-A2 with a yield of 44.79%.

### Step 8: Synthesis of 1883-A3

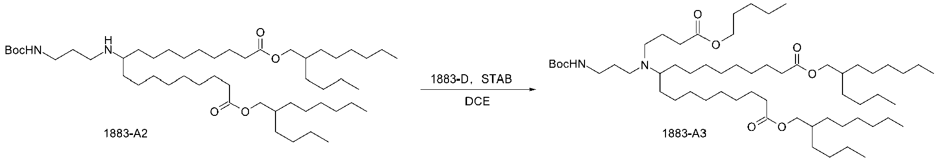

### Experimental procedure:

1883-A2 (4.3 g, 5.1 mmol), 1883-D (1.3 g, 7.55 mmol), and 1,2-dichloroethane (43 mL) were added to a 250 mL single-necked round-bottom flask, stirred at room temperature for 0.5 h, then added with sodium triacetoxyborohydride (1.62 g, 7.65 mmol), and stirred at room temperature overnight. Water (80 mL) was added to the reaction solution for washing. The organic phase was separated. Dichloromethane (100 mL) was added to the water phase for extraction, and the organic phases were combined. The organic phase was washed with saturated aqueous sodium bicarbonate solution (100 mL). The solvent was then removed by evaporation under reduced pressure at 45°C, obtaining a coarse product of 1883-A3. The coarse product was purified by silica gel column chromatography and eluted with ethyl acetate:n-hexane = 1:1 to obtain 2.28 g 1883-A3 with a yield of 44.7%.

### Step 9: Synthesis of 1883-A4

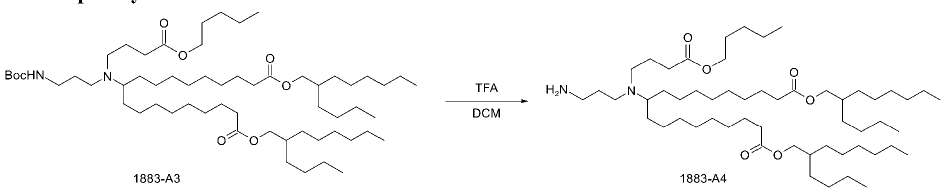

### Experimental procedure:

1883-A3 (2.28 g, 2.3 mmol) and dichloromethane (22 mL) were added to a 100 mL single-necked flask, stirred at room temperature until dissolved into a clear solution, then added dropwise with TFA (7 mL), and stirred at room temperature for 2 h. The solvent was removed by evaporation under reduced pressure at 45°C, and ethyl acetate (50 mL) was added, followed by addition of saturated aqueous potassium bicarbonate solution (100 mL) for washing. The organic phase was separated. The water phase was extracted twice with ethyl acetate (50 mL), and the organic phases were combined, washed with saturated aqueous sodium chloride solution (100 mL), dried with anhydrous sodium sulfate, and filtered. The solvent was removed from the filtrate by evaporation under reduced pressure at 45°C, obtaining 1.1 g of a coarse product of 1883-A4, which may be directly used in the reaction of the next step without purification.

### Step 10: Synthesis of Amino Lipid Compound 1883

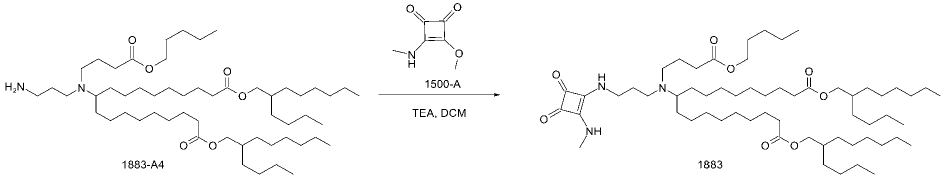

### Experimental procedure:

1883-A4 (1.1 g, 1.23 mmol), 1500-A (210 mg, 1.48 mmol), and dichloromethane (12 mL) were added to a 50 mL flask, stirred, added dropwise with triethylamine (149 mg, 1.48 mmol), and placed at room temperature overnight. The reaction solution was washed with saturated aqueous potassium bicarbonate solution (50 mL), and washed with saturated aqueous sodium chloride solution (50 mL), then dried with anhydrous sodium sulfate, and filtered. The solvent was removed from the filtrate by evaporation under reduced pressure at 45°C, obtaining a coarse product. The coarse product was purified by silica gel column chromatography and eluted with ethyl acetate:methanol= 20:1 to obtain 560 mg of the amino lipid compound 1883 with a yield of 45.4% and a purity of 94.63%.

¹H NMR (600 MHz, CDCl₃) δ 4.07 (t, *J =* 6.8 Hz, 2H), 3.97 (d, *J =* 5.8 Hz, 4H), 3.68 (d, *J =* 3.8 Hz, 2H), 3.32 (d, *J =* 5.1 Hz, 3H), 2.50 (t, *J =* 6.2 Hz, 2H), 2.42-2.33 (m, 5H), 2.30 (t, *J =* 7.5 Hz, 4H), 1.81 (s, 4H), 1.76-1.69 (m, 4H), 1.69-1.57 (m, 8H), 1.41-1.22 (m, 60H), 0.90 (dt, *J =* 13.2, 7.0 Hz, 15H).

LC-MS (ESI): Calculated for 1002.56, Found (M+H): 1003.4.

### Example 20: Synthesis of Amino Lipid Compound 1884

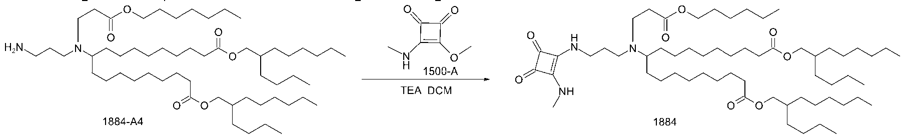

According to the general synthetic method, the amino lipid compound 1834 was synthesized according to the process of Step 10 of Example 19 with substituting 1884-A4 for 1883-A4, wherein 1884-A4 (320 mg, 0.35 mmol), triethylamine (43 mg, 0.43 mmol), and 1500-A (60 mg, 0.43 mmol) were used, resulting in 170 mg of the amino lipid compound 1884 with a yield of 48.5% and a purity of 95.68%.

¹H NMR (600 MHz, CDCl₃) δ4.07 (t, *J =* 6.8 Hz, 2H), 4.00 (d, *J =* 5.8 Hz, 4H), 3.77 (s, 2H), 3.36 (d, *J* = 5.1 Hz, 2H), 2.80-2.72 (m, 2H), 2.58 (t, *J =* 5.6 Hz, 2H), 2.54-2.48 (m, 2H), 2.45-2.38 (m, 1H), 2.34 (t, *J* = 7.5 Hz, 4H), 1.77 (m, 2H), 1.70-1.60 (m, 8H), 1.45-1.16 (m, 64H), 0.93 (dt, *J=* 13.0, 7.0 Hz, 15H).

LC-MS (ESI): Calculated for 1002.56, Found(M+H): 1003.3.

### Example 21: Synthesis of Amino Lipid Compound 1885

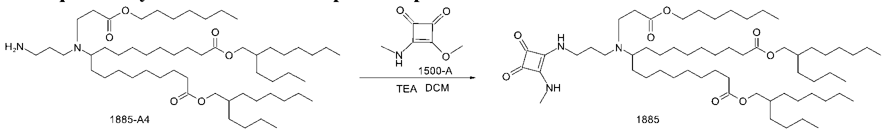

According to the general synthetic method, the amino lipid compound 1885 was synthesized according to the process of Step 10 of Example 19 with substituting 1885-A4 for 1883-A4, wherein 1885-A4 (323 mg, 0.35 mmol), triethylamine (43 mg, 0.43 mmol), and 1500-A (60 mg, 0.43 mmol) were used, resulting in 180 mg of the amino lipid compound 1885 with a yield of 50.6% and a purity of 96.96%.

¹H NMR (600 MHz, CDCl₃) δ 4.03 (t, J = 6.8 Hz, 2H), 3.96 (d, J = 5.8 Hz, 4H), 3.32 (d, J = 5.1 Hz, 3H), 2.76-2.70 (m, 2H), 2.54 (t, J = 5.6 Hz, 2H), 2.51-2.45 (m, 2H), 2.40-2.34 (m, 1H), 2.29 (t, J = 7.5 Hz, 4H), 1.73 (dt, J = 11.4, 5.9 Hz, 2H), 1.65-1.57 (m, 10H), 1.42-1.18 (m, 64H), 0.89 (dt, J = 13.0, 7.0 Hz, 15H).

LC-MS (ESI): Calculated for 1016.59, Found(M+H): 1016.3.

### Example 22: Synthesis of Amino Lipid Compound 1898

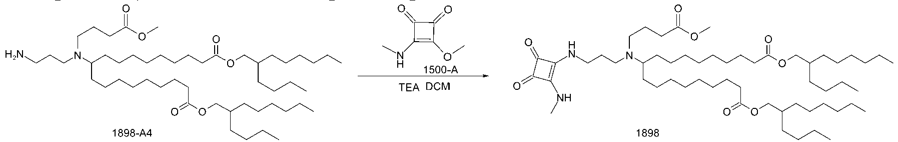

According to the general synthetic method, the amino lipid compound 1898 was synthesized according to the process of Step 10 of Example 19 with substituting 1898-A4 for 1883-A4, wherein 1898-A4 (1.0 g, 1.19 mmol), triethylamine (143 mg, 1.42 mmol), and 1500-A (200 mg, 1.42 mmol) were used, resulting in 637 mg of the amino lipid compound 1898 with a yield of 56.6% and a purity of 97.99%.

¹H NMR (600 MHz, CDCl₃) δ 4.00 (d, *J =* 5.8 Hz, 4H), 3.71 (d, *J =* 7.8 Hz, 5H), 3.36 (d, *J =* 5.1 Hz, 3H), 2.51 (t, *J =* 6.5 Hz, 2H), 2.43-2.37 (m, 5H), 2.33 (t, *J =* 7.5 Hz, 4H), 1.79-1.71 (m, 4H), 1.68-1.62 (m, 6H), 1.36-1.25 (m, 56H), 0.91 (t, *J =* 6.6 Hz, 12H).

LC-MS (ESI): Calculated for 946.45, Found(M+H): 947.2.

### Example 23: Synthesis of Amino Lipid Compound 1899

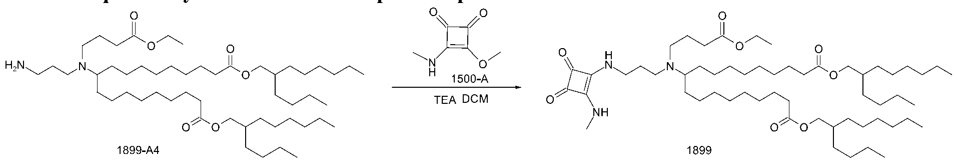

According to the general synthetic method, the amino lipid compound 1899 was synthesized according to the process of Step 10 of Example 19 with substituting 1899-A4 for 1883-A4, wherein 1899-A4 (1.0 g, 1.17 mmol), triethylamine (141 mg, 1.4 mmol), and 1500-A (198 mg, 1.4 mmol) were used, resulting in 598 mg of the amino lipid compound 1899 with a yield of 53.0% and a purity of 98.93%.

¹H NMR (600 MHz, CDCl₃) δ 4.12 (q, *J =* 7.1 Hz, 2H), 3.95 (d, *J =* 5.8 Hz, 4H), 3.67 (s, 2H), 3.31 (d, *J =* 5.1 Hz, 3H), 2.47 (d, *J* = 12.9 Hz, 2H), 2.37-2.33 (m, 4H), 2.29 (t, *J =* 7.5 Hz, 4H), 1.91 (s, 2H), 1.70 (dd, *J =* 16.0, 9.6 Hz, 4H), 1.63-1.56 (m, 6H), 1.37-1.12 (m, 58H), 0.87 (t, *J =* 6.6 Hz, 12H).

LC-MS (ESI): Calculated for 960.48, Found(M+H): 961.2.

### Example 24: Synthesis of Amino Lipid Compound 1644

### Step 1: Synthesis of 3-PBAL

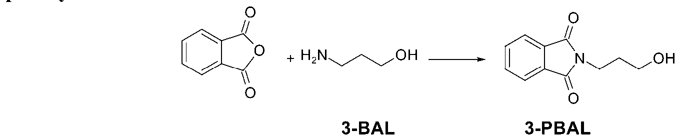

### Experimental procedure:

Phthalic anhydride (10 g, 67.5 mmol), toluene (100 ml), and 3-BAL (6.83 g, 81.0 mmol) were added to a 250 mL single-necked flask which was connected to a water separator, subjected to reflux to separate water at 140°C, and reacted for 3 h. After the reaction stopped, the reaction solution was cooled to the room temperature, then washed with water (50 mL). Phases were separated. The organic phase was collected. The solvent was removed from the organic phase by evaporation under reduced pressure at 45°C, obtaining 13.6 g of a white solid with a yield of 98.2%.

### Step 2: Synthesis of 1644-B

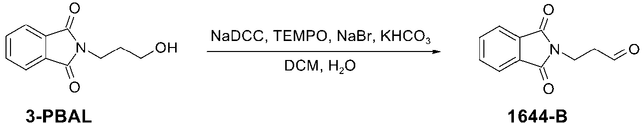

### Experimental procedure:

3-PBAL (13.6 g, 66.3 mmol), dichloromethane (85 ml), TEMPO (0.52 g, 3.3 mmol), sodium bromide (0.68 g, 6.6 mmol), and potassium bicarbonate (5.3 g, 53.0 mmol) were added to a 250 mL single-necked flask, and placed in a 5°C low temperature tank. After the internal temperature was reduced to 5°C, an aqueous solution of NaDCC (70 ml water, 9.5 g NaDCC, 43.1 mmol) was added dropwise to the reaction, and reacted at 5°C for 5 h after the dropwise addition was completed. Filtering was then performed. The filter cake was subjected to drip washing with dichloromethane (20 ml). The filtrate was collected and subjected to phases separation. The organic phase was collected. The water phase was extracted once with dichloromethane (50 ml). The organic phases were combined. The solvent was removed from the organic phase by evaporation under reduced pressure at 45°C, obtaining 13.4 g of a coarse product. The coarse product was purified by silica gel column chromatography and eluted with dichloromethane to obtain 11.8 g 1644-B with a yield of 87.6%.

### Step 3: Synthesis of 1644-C

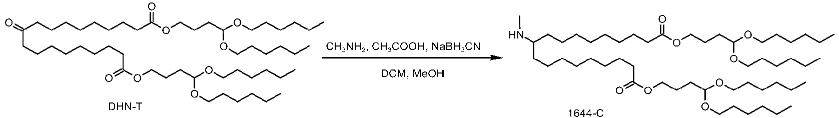

### Experimental procedure:

A 2M solution of methylamine in methanol (47 mL, 93.36 mmol) was added to a 250 mL single-necked flask, slowly added dropwise with acetic acid (5.6 g, 93.36 mmol), stirred at room temperature for 30 min, then sequentially added with DHN-T (10.0 g, 11.69 mmol), dichloromethane (50 mL), and sodium cyanoborohydride (0.81 g, 12.84 mmol), and reacted at room temperature for 14 h. The solvent was removed by evaporation under reduced pressure at 45°C. Water (150 mL) was added, followed by extraction twice with dichloromethane (150 mL), and the organic phases were combined. The organic phase was washed with saturated aqueous sodium chloride solution (100 mL), dried with anhydrous sodium sulfate, and filtered. The solvent was removed from the filtrate by evaporation under reduced pressure at 45°C, obtaining 11.4 g of a coarse product of 1644-C. The coarse product was purified by silica gel column chromatography and eluted with dichloromethane:methanol = 10:1 to obtain 9.01 g 1644-C with a yield of 88.6%.

### Step 4: Synthesis of 1644-D

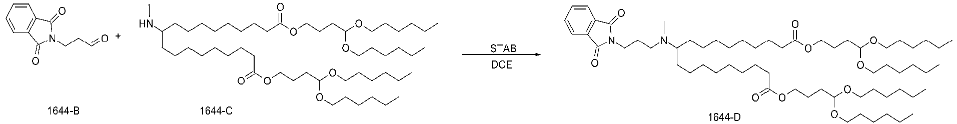

### Experimental procedure:

1644-C (9.0 g, 10.34 mmol), 1644-B (3.15 g, 15.51 mmol), and DCE (90.0 mL) were added to a 250 mL single-necked flask, added with sodium triacetoxyborohydride (2.31 g, 10.34 mmol), and reacted at room temperature for 3 h. Water (150 mL) was added, followed by extraction twice with dichloromethane (150 mL), and the organic phases were combined. The organic phase was washed with saturated aqueous sodium chloride solution (100 mL), dried with anhydrous sodium sulfate, and filtered. The solvent was removed from the filtrate by evaporation under reduced pressure at 45°C, thereby obtaining 12.8 g of a coarse product of 1644-D. The coarse product was purified by silica gel column chromatography and eluted with ethyl acetate and n-heptane = 1:1 to obtain 6.97 g 1644-D with a yield of 63.7%.

### Step 5: Synthesis of 1644-H

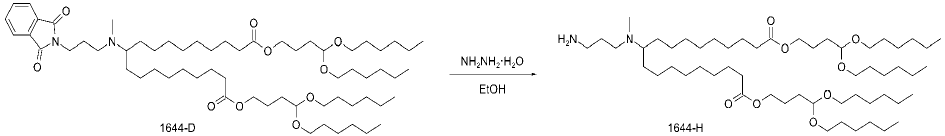

### Experimental procedure:

1644-D (6.0 g, 5.67 mmol), EtOH (30 mL), and hydrazine hydrate (2.89 g, 57.9 mmol) were sequentially added to a 100 mL single-necked flask, warmed to 90°C, and reacted for 4 h. The solvent was removed by evaporation under reduced pressure at 45°C. The residue was added with 5% aqueous potassium **bicarbonate** solution (50 mL) and ethyl acetate (50 mL), and stirred for 10 min. Phases were then separated. The organic phase was collected. The water phase was extracted with ethyl acetate (50 mL) again, and the organic phases were combined. The organic phase was washed with saturated aqueous sodium chloride solution (50 mL), and then evaporated under reduced pressure at 45°C to remove the solvent, obtaining 6.0 g of a coarse product of 1644-H. The coarse product was purified by silica gel column chromatography and eluted with ethyl acetate:methanol = 5:1 to obtain 3.59 g 1644-H with a yield of 68.2%.

### Step 6: Synthesis of Amino Lipid Compound 1664

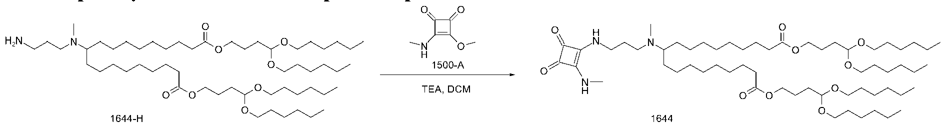

### Experimental procedure:

1644-H (3.5 g, 3.77 mmol), dichloromethane (20 mL), triethylamine (0.57 g, 5.66 mmol), and 1500-A (0.79 g, 5.66 mmol) were sequentially added to a 100 mL single-necked flask, and reacted at room temperature overnight. The solvent was removed by evaporation under reduced pressure at 45°C. 5% Aqueous potassium bicarbonate solution (50 mL) and ethyl acetate (50 mL) were added, and stirred for 10 min. Phases were then separated. The organic phase was collected. The water phase was extracted with ethyl acetate (50 mL) again, and the organic phases were combined. The organic phase was washed with saturated aqueous sodium chloride solution (50 mL). The solvent was removed by evaporation under reduced pressure at 45°C, obtaining 4.2 g of a coarse product of the amino lipid compound 1644. The coarse product was purified by silica gel column chromatography and eluted with ethyl acetate:n-heptane = 1:1 to obtain 1.6 g of the amino lipid compound 1644 with a yield of 40.92% and a purity of 94.6%.

¹H NMR (600 MHz, CDCl₃) δ 4.48 (t, *J* = 5.3 Hz, 2H), 4.08 (t, *J =* 6.2 Hz, 4H), 3.66-3.54 (m, 6H), 3.41 (dt, *J =* 9.3, 6.7 Hz, 4H), 3.27 (d, *J* = 4.7 Hz, 3H), 2.54 (s, 2H), 2.37 (s, 1H), 2.29 (t, *J =* 7.5 Hz, 4H), 2.19 (s, 3H), 1.77-1.54 (m, 22H), 1.46-1.22 (m, 48H), 0.89 (t, *J =* 6.9 Hz, 12H).

LC-MS (ESI): Calculated for 1036.58, Found (M+H): 1037.2.

### Example 25: Synthesis of Amino Lipid Compound 1643

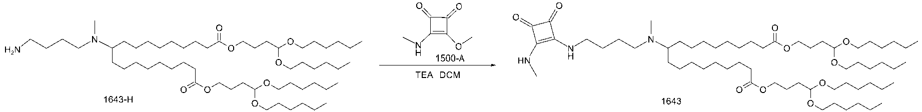

According to the general synthetic method, the amino lipid compound 1643 was synthesized according to the process of Step 6 of Example 24 with substituting 1643-H for 1644-H, wherein 1643-H (440 mg, 0.467 mmol), triethylamine (47 mg, 0.467 mmol), and 1500-A (99 mg, 0.701 mmol) were used, resulting in 250 mg of the amino lipid compound 1643 with a yield of 50.9% and a purity of 96.88%.

¹H NMR (600 MHz, CDCl₃) δ 4.48 (t, *J =* 5.3 Hz, 2H), 4.08 (t, *J =* 6.1 Hz, 4H), 3.57 (m, 6H), 3.41 (dt, *J =* 9.3, 6.7 Hz, 4H), 3.31 (d, *J* = 4.9 Hz,3H), 2.44-2.25 (m, 7H), 2.15 (s, 3H), 1.74-1.49 (m, 24H), 1.44-1.16 (m, 48H), 0.89 (t, *J =* 6.9 Hz, 12H).

LC-MS (ESI): Calculated for 1050.6, Found(M+H): 1051.2.

### Example 26: Synthesis of Amino Lipid Compound 1645

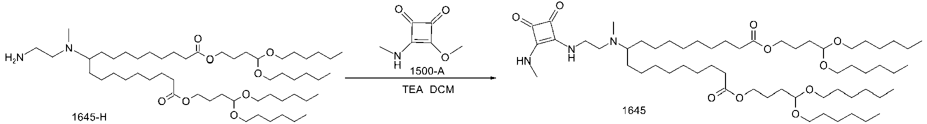

According to the general synthetic method, the amino lipid compound 1645 was synthesized according to the process of Step 6 of Example 24 with substituting 1645-H for 1644-H, wherein 1645-H (790 mg, 0.865 mmol), triethylamine (130 mg, 1.3 mmol), and 1500-A (183 mg, 1.3 mmol) were used, resulting in 520 mg of the amino lipid compound 1645 with a yield of 58.8% and a purity of 90.56%.

¹H NMR (600 MHz, CDC13) δ 4.52 (t, *J =* 5.2 Hz, 2H), 4.11 (t, *J =* 6.1 Hz, 4H), 3.75-3.57 (m, 6H), 3.44 (dt, *J* = 9.3, 6.7 Hz, 4H), 3.30 (d, *J =* 4.9 Hz, 3H), 2.68 (t, *J =* 5.5 Hz, 2H), 2.34 (dt, *J =* 15.1, 6.9 Hz, 5H), 2.22 (s, 3H), 1.91-1.55 (m, 20H), 1.36 (m, 48H), 0.92 (t, *J =* 6.9 Hz, 12H).

LC-MS (ESI): Calculated for 1022.5, Found (M+H): 1023.3.

### Example 27: Synthesis of Amino Lipid Compound 1831

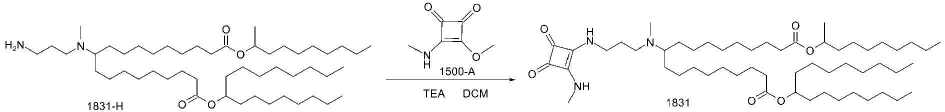

According to the general synthetic method, the amino lipid compound 1831 was synthesized according to the process of Step 6 of Example 24 with substituting 1831-H for 1644-H, wherein 1831-H (1.9 g, 2.4 mmol), triethylamine (364 mg, 3.6 mmol), and 1500-A (508 mg, 3.6 mmol) were used, resulting in 1.456 g of the amino lipid compound 1831 with a yield of 67.3% and a purity of 96.61 %.

¹H NMR (600 MHz, CDCl₃) δ 4.95-4.86 (m, 2H), 3.70-3.55 (m, 2H), 3.31 (d, *J* = 4.5 Hz, 3H), 2.59 (s, 2H), 2.42 (dd, *J =* 12.8, 5.5 Hz, 1H), 2.30 (dd, *J =* 15.2, 7.7 Hz, 4H), 2.24 (s, 3H), 1.82-1.76 (m, 2H), 1.70-1.42 (m, 13H), 1.38-1.14 (m, 60H), 0.91 (t, *J=* 7.0 Hz, 9H).

LC-MS (ESI): Calculated for 902.44, Found (M+H): 903.1.

### Example 28: Synthesis of Amino Lipid Compound 1832

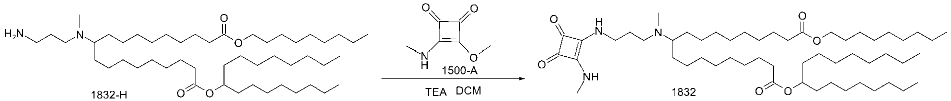

According to the general synthetic method, the amino lipid compound 1832 was synthesized according to the process of Step 6 of Example 24 with substituting 1832-H for 1644-H, wherein 1832-H (402 mg, 0.515 mmol), triethylamine (78 mg, 0.775 mmol), and 1500-A (110 mg, 0.775 mmol) were used, resulting in 170 mg of the amino lipid compound 1832 with a yield of 37.1% and a purity of 93.72%.

¹H NMR (600 MHz, CDCl₃) δ 4.89-4.82 (m, 1H), 4.05 (t, *J =* 6.8 Hz, 2H), 3.82-3.49 (m, 2H), 3.27 (d, *J =* 4.4 Hz, 3H), 2.52 (t, *J =* 5.6 Hz, 2H), 2.36 (dd, *J =* 12.0, 5.9 Hz, 1H), 2.28 (dd, *J =* 14.5, 7.2 Hz, 4H), 2.17 (s, 3H), 1.74 (dd, *J =* 12.0, 6.0 Hz, 4H), 1.64-1.57 (m, 6H), 1.50 (d, *J =* 6.0 Hz, 4H), 1.44-1.37 (m, 2H), 1.32-1.22 (m, 56H), 0.87 (m, 9H).

LC-MS (ESI): Calculated for 888.42, Found (M+H): 889.3.

### Example 29: Synthesis of Amino Lipid Compound 1833

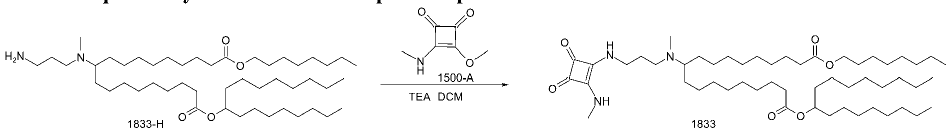

According to the general synthetic method, the amino lipid compound 1833 was synthesized according to the process of Step 6 of Example 24 with substituting 1833-H for 1644-H, wherein 1833-H (1.02 g, 1.33 mmol), triethylamine (202 mg, 2.0 mmol), and 1500-A (282 mg, 2.0 mmol) were used, resulting in 320 mg of the amino lipid compound 1833 with a yield of 27.5% and a purity of 98.25%.

¹H NMR (600 MHz, CDCl₃) δ 4.88-4.82 (m, 1H), 4.05 (t, *J =* 6.8 Hz, 2H), 3.76-3.45 (m, 2H), 3.27 (d, *J* = 4.4 Hz, 3H), 2.52 (t, *J* = 5.6 Hz, 2H), 2.37 (dd, *J* = 12.2, 6.0 Hz, 1H), 2.28 (m, 4H), 2.18 (s, 3H), 1.73 (dt, *J =* 12.5, 6.3 Hz, 4H), 1.64-1.57 (m, 6H), 1.50 (d, *J =* 6.0 Hz, 4H), 1.41 (d, *J =* 5.9 Hz, 2H), 1.33-1.22 (m, 54H), 0.87 (m, 9H).

LC-MS (ESI): Calculated for 874.39, Found(M+H): 875.3.

### Example 30: Synthesis of Amino Lipid Compound 1834

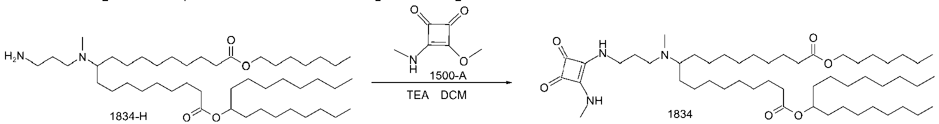

According to the general synthetic method, the amino lipid compound 1834 was synthesized according to the process of Step 6 of Example 24 with substituting 1834-H for 1644-H, wherein 1834-H (1.6 g, 2.13 mmol), triethylamine (320 mg, 3.18 mmol), and 1500-A (449 mg, 3.18 mmol) were used, resulting in 400 mg of the amino lipid compound 1834 with a yield of 21.8% and a purity of 96.63%.

¹H NMR (600 MHz, CDCl₃) δ 4.92-4.79 (m, 1H), 4.06 (t, *J =* 6.7 Hz, 2H), 3.65 (m, 2H), 3.29 (d, *J =* 4.4 Hz, 3H), 2.60 (t, *J =* 5.6 Hz, 2H), 2.46-2.34 (m, 1H), 2.33-2.24 (m, 4H), 2.24-2.15 (s, 3H), 1.83-1.70 (dt, *J* = 12.5, 6.3 Hz, 4H), 1.64-1.60 (m, 6H), 1.50 (m, 4H), 1.40 (m, 2H), 1.37-1.12 (m, 52H), 0.90-0.84 (m, 9H).

LC-MS (ESI): Calculated for 860.36, Found(M+H): 861.0.

### Example 31: Synthesis of Amino Lipid Compound 1835

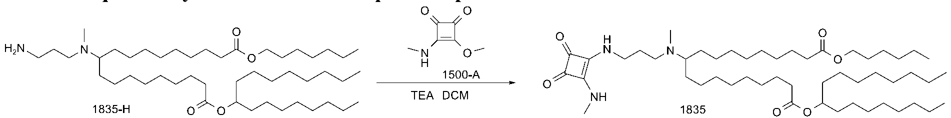

According to the general synthetic method, the amino lipid compound 1835 was synthesized according to the process of Step 6 of Example 24 with substituting 1835-H for 1644-H, wherein 1835-H (1.3 g, 1.73 mmol), triethylamine (263 mg, 2.6 mmol), and 1500-A (367 mg, 2.6 mmol) were used, resulting in 0.98 g of the amino lipid compound 1835 with a yield of 67.0% and a purity of 97.69%.

¹H NMR (600 MHz, CDCl₃) δ 4.92-4.79 (m, 1H), 4.06 (t, *J=* 6.7 Hz, 2H), 3.65 (m, 2H), 3.29 (d, *J* = 4.4 Hz, 3H), 2.60 (t, *J =* 5.6 Hz, 2H), 2.46-2.34 (m, 1H), 2.33-2.24 (m, 4H), 2.24-2.15 (s, 3H), 1.83-1.70 (dt, *J =* 12.5, 6.3 Hz, 4H), 1.64-1.59 (m, 6H), 1.50 (m, 4H), 1.40 (m, 2H), 1.37-1.12 (m, 50H), 0.90-0.84 (m, 9H).

LC-MS (ESI): Calculated for 846.34, Found(M+H): 847.0.

### Example 32: Synthesis of Amino Lipid Compound 1873

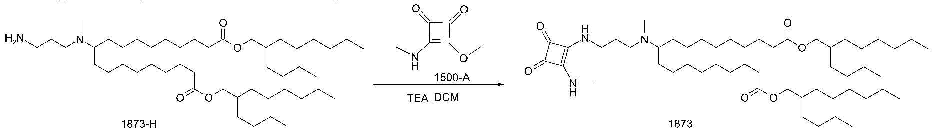

According to the general synthetic method, the amino lipid compound 1873 was synthesized according to the process of Step 6 of Example 24 with substituting 1873-H for 1644-H, wherein 1873-H (1.2 g, 1.6 mmol), triethylamine (243 mg, 2.4 mmol), and 1500-H (338 mg, 2.4 mmol) were used, resulting in 820 mg of the amino lipid compound 1873 with a yield of 59.7% and a purity of 93.46%.

¹H NMR (600 MHz, CDCl₃) δ 4.00 (d, *J =* 5.8 Hz, 4H), 3.65 (s, 2H), 3.31 (d, *J* = 3.9 Hz, 3H), 2.56 (t, *J* = 5.6 Hz, 2H), 2.40 (dd, *J =* 12.1, 5.8 Hz, 1H), 2.34 (t, *J =* 7.5 Hz, 4H), 2.22 (s, 3H), 1.77 (s, 2H), 1.65 (dd, *J=* 14.3, 7.2 Hz, 6H), 1.51-1.23 (m, 56H), 0.92 (m,12H).

LC-MS (ESI): Calculated for 860.36, Found(M+H): 861.2.

### Example 33: Synthesis of Amino Lipid Compound 1874

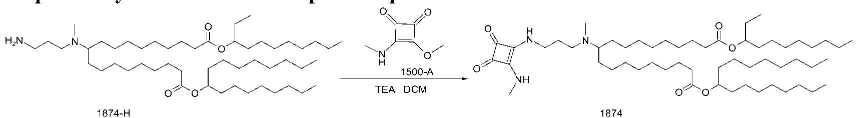

According to the general synthetic method, the amino lipid compound 1874 was synthesized according to the process of Step 6 of Example 24 with substituting 1874-H for 1644-H, wherein 1874-H (1.2 g, 1.49 mmol), triethylamine (294 mg, 2.91 mmol), and 1500-A (0.41 mg, 2.91 mmol) were used, resulting in 950 mg of the amino lipid compound 1874 with a yield of 69.6% and a purity of 92.46%.

¹H NMR (600 MHz, CDCl₃) δ 4.91-4.80 (m, 2H), 3.85-3.44 (m, 2H), 3.29 (d, *J=* 4.5 Hz, 3H), 2.50 (t, *J* = 6.0 Hz, 2H), 2.35 (dd, *J* = 12.3, 6.1 Hz, 1H), 2.28 (t, *J* = 7.5 Hz, 4H), 2.17 (s, 3H), 1.74 (p, *J* = 6.3 Hz, 2H), 1.65-1.57 (m, 4H), 1.51 (s, 2H), 1.40 (dd, *J=* 13.7, 6.0 Hz, 2H), 1.28 (dd, *J* = 21.0, 7.9 Hz, 66H), 0.93 (m, 12H).

LC-MS (ESI): Calculated for 916.47, Found(M+H): 917.1.

### Example 34: Synthesis of Amino Lipid Compound 1875

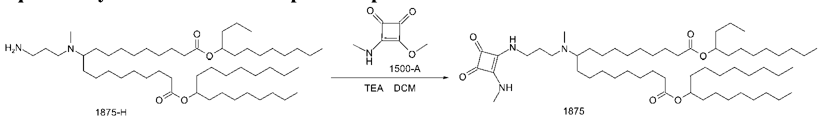

According to the general synthetic method, the amino lipid compound 1875 was synthesized according to the process of Step 6 of Example 24 with substituting 1875-H for 1644-H, wherein 1875-H (1.2 g, 1.46 mmol), triethylamine (217 mg, 2.15 mmol), and 1500-A (303 mg, 2.15 mmol) were used, resulting in 693 mg of the amino lipid compound 1875 with a yield of 51.0% and a purity of 93.66%.

¹H NMR (600 MHz, CDCl₃) δ 4.91-4.80 (m, 2H), 3.85-3.44 (m, 2H), 3.29 (d, *J =* 4.5 Hz, 3H), 2.50 (t, *J* = 6.0 Hz, 2H), 2.35 (dd, *J =* 12.3, 6.1 Hz, 1H), 2.28 (t, *J* = 7.5 Hz, 4H), 2.17 (s, 3H), 1.74 (p, *J* = 6.3 Hz, 2H), 1.65-1.57 (m, 4H), 1.51 (s, 2H), 1.40 (dd, *J =* 13.7, 6.0 Hz, 2H), 1.28 (dd, *J =* 21.0, 7.9 Hz, 68H), 0.88 (t, *J* = 7.0 Hz, 12H).

LC-MS (ESI): Calculated for 930.50, Found(M+H): 931.1.

### Example 35: Synthesis of Amino Lipid Compound 1876

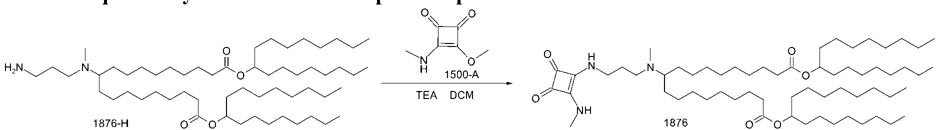

According to the general synthetic method, the amino lipid compound 1876 was synthesized according to the process of Step 6 of Example 24 with substituting 1876-H for 1644-H, wherein 1876-H (1.75 g, 1.96 mmol), triethylamine (297 mg, 2.94 mmol), and 1500-A (415 mg, 2.94 mmol) were used, resulting in 1.17 g of the amino lipid compound 1876 with a yield of 59.7% and a purity of 93.46%.

¹H NMR (600 MHz, CDCl₃) δ 4.91-4.80 (m, 2H), 3.85-3.44 (m, 2H), 3.29 (d, *J=* 4.5 Hz, 3H), 2.50 (t, *J* = 6.0 Hz, 2H), 2.35 (dd, *J* = 12.3, 6.1 Hz, 1H), 2.28 (t, *J* = 7.5 Hz, 4H), 2.17 (s, 3H), 1.74 (p, *J* = 6.3 Hz, 2H), 1.65-1.57 (m, 4H), 1.51 (s, 4H), 1.40 (dd, *J =* 13.7, 6.0 Hz, 2H), 1.28 (dd, *J* = 21.0, 7.9 Hz, 74H), 0.88 (t, *J* = 7.0 Hz, 12H).

LC-MS (ESI): Calculated for 1000.63, Found(M+H): 1001.2.

### Example 36: Synthesis of Amino Lipid Compound 1880

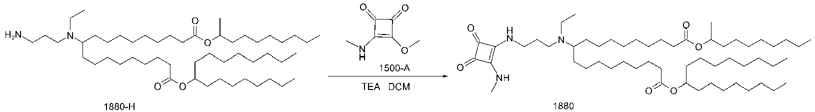

According to the general synthetic method, the amino lipid compound 1880 was synthesized according to the process of Step 6 of Example 24 with substituting 1880-H for 1644-H, wherein 1880-H (1.2 g, 1.49 mmol), triethylamine (242 mg, 2.4 mmol), and 1500-A (338 mg, 2.4 mmol) were used, resulting in 811 mg of the amino lipid compound 1880 with a yield of 59.4% and a purity of 93.46%.

¹H NMR (600 MHz, CDCl₃) δ 4.92-4.81 (m, 2H), 3.68 (s, 2H), 3.29 (d, *J* = 4.6 Hz, 3H), 2.50 (t, *J =* 6.3 Hz, 2H), 2.48-2.36 (m, 3H), 2.28 (q, *J* = 7.7 Hz, 4H), 1.84-1.69 (m, 4H), 1.63-1.57 (m, 5H), 1.52-1.48 (m, 4H), 1.27 (d, *J* = 21.6 Hz, 64H), 1.00 (t, *J=* 7.1 Hz, 3H), 0.88 (t, *J =* 7.1 Hz, 9H).

LC-MS (ESI): Calculated for 916.47, Found(M+H): 917.1.

### Example 37: Synthesis of Amino Lipid Compound 1881

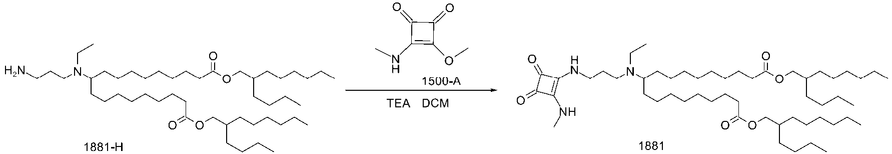

According to the general synthetic method, the amino lipid compound 1881 was synthesized according to the process of Step 6 of Example 24 with substituting 1881-H for 1644-H, wherein 1881-H (2.0 g, 2.61 mmol), triethylamine (172 mg, 1.7 mmol), and 1500-A (240 mg, 1.7 mmol) were used, resulting in 1141 mg of the amino lipid compound 1881 with a yield of 50.0% and a purity of 97.3%.

¹H NMR (600 MHz, CDCl₃) δ 4.00 (d, *J =* 5.8 Hz, 4H), 3.72 (s, 2H), 3.34 (d*, J =* 4.6 Hz, 3H), 2.56-2.40 (m, 5H), 2.34 (t, *J* = 7.5 Hz, 4H), 1.85-1.71 (m, 5H), 1.64 (dd, *J* = 14.4, 7.3 Hz, 6H), 1.44-1.16 (m, 53H), 1.03 (t, *J =* 7.1 Hz, 3H), 0.92 (m, 12H).

LC-MS (ESI): Calculated for 874.39, Found (M+H): 875.2.

### Example 38: Synthesis of Amino Lipid Compound 1882

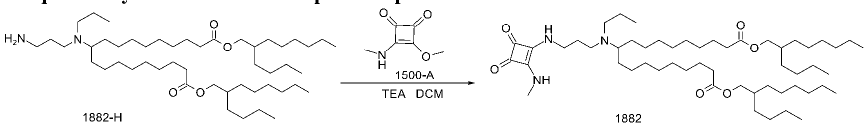

According to the general synthetic method, the amino lipid compound 1882 was synthesized according to the process of Step 6 of Example 24 with substituting 1882-H for 1644-H, wherein 1882-H (1.3 g, 1.67 mmol), triethylamine (252 mg, 2.5 mmol), and 1500-A (0.35 g, 2.48 mmol) were used, resulting in 741 mg of the amino lipid compound 1882 with a yield of 49.9% and a purity of 96.3%.

¹H NMR (600 MHz, CDCl₃) δ 4.00 *(d, J=* 5.8 Hz, 4H), 3.72 (s, 2H), 3.34 (d, *J =* 4.9 Hz, 3H), 2.51 (t, *J* = 6.4 Hz, 2H), 2.42-2.30 (m, 7H), 1.84-1.71 (m, 5H), 1.64 (dd, *J* = 14.4, 7.3 Hz, 6H), 1.46-1.18 (m, 55H), 0.96-0.87 (m, 15H).

LC-MS (ESI): Calculated for 888.42, Found (M+H): 889.1.

### Example 39: Synthesis of Amino Lipid Compound 1909

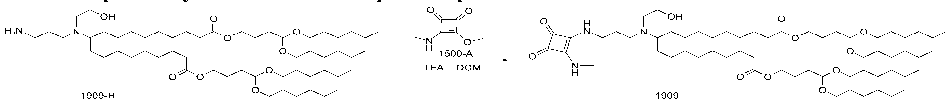

According to the general synthetic method, the amino lipid compound 1909 was synthesized according to the process of Step 6 of Example 24 with substituting 1909-H for 1644-H, wherein 1909-H (1.5 g, 1.57 mmol), triethylamine (0.24 g, 2.35 mmol), and 1500-A (0.33 g, 2.34 mmol) were used, resulting in 760 mg of the amino lipid compound 1909 with a yield of 45.5% and a purity of 95.4%.

¹H NMR (600 MHz, CDCl₃) δ 4.52 (t, *J =* 5.2 Hz, 2H), 4.11 (t, *J =* 6.1 Hz, 4H), 3.79-3.72 (m, 4H), 3.63-3.56 (m, 4H), 3.44 (m, 4H), 3.29 (d, *J* = 5.1 Hz, 3H), 2.66 (m, 4H), 2.50-2.44 (m, 1H), 2.32 (t, *J* = 7.6 Hz, 4H), 1.76-1.67 (m, 6H), 1.66-1.54 (m, 16H), 1.40-1.24 (m, 48H), 0.92 (t, *J =* 6.9 Hz, 12H).

LC-MS (ESI): Calculated for 1066.60, Found (M+H): 1067.2.

### Example 40: Synthesis of Amino Lipid Compound 1912

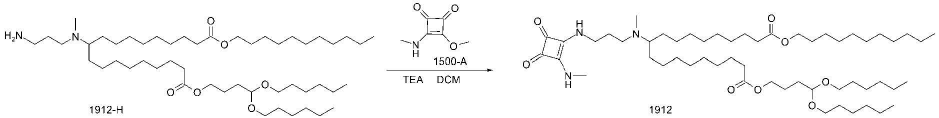

According to the general synthetic method, the amino lipid compound 1912 was synthesized according to the process of Step 6 of Example 24 with substituting 1912-H for 1644-H, wherein 1912-H (1.0 g, 1.21 mmol), triethylamine (0.18 g, 1.82 mmol), and 1500-A (0.26 g, 1.82 mmol) were used, resulting in 523 mg of the amino lipid compound 1912 with a yield of 46.3% and a purity of 93.8%.

¹H NMR (600 MHz, CDCl₃) δ 4.48 (t, *J =* 5.2 Hz, 1H), 4.06 (dt, *J* = 13.6, 6.4 Hz, 4H), 3.60-3.53 (dt, *J* = 9.2, 6.7 Hz, 2H), 3.44-3.37 (dt, *J* = 9.3, 6.7 Hz, 2H), 3.28 (d, *J =* 4.1 Hz, 3H), 2.52 (m, 2H), 2.38 (m, 1H), 2.29 (t, *J* = 7.5 Hz, 4H), 2.18 (s, 3H), 1.79-1.73 (m, 2H), 1.73-1.64 (m, 4H), 1.58 (m, 10H), 1.45-1.19 (m, 54H), 0.93-0.84 (m, 9H).

LC-MS (ESI): Calculated for 934.44, Found (M+H): 935.2.

### Example 41: Synthesis of Amino Lipid Compound 1913

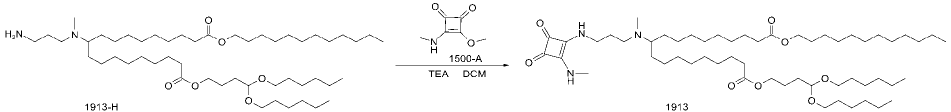

According to the general synthetic method, the amino lipid compound 1913 was synthesized according to the process of Step 6 of Example 24 with substituting 1913-H for 1644-H, wherein 1913-H (1.5 g, 1.79 mmol), triethylamine (0.27 g, 2.68 mmol), and 1500-A (0.38 g, 2.68 mmol) were used, resulting in 903 mg of the amino lipid compound 1913 with a yield of 53.3% and a purity of 95.9%.

¹H NMR (600 MHz, CDCl₃) δ 4.48 (t, *J =* 5.2 Hz, 1H), 4.06 (dt, *J =* 13.6, 6.4 Hz, 4H), 3.57 (dt, *J* = 9.2, 6.7 Hz, 2H), 3.41 (dt, *J* = 9.3, 6.7 Hz, 2H), 3.28 (d, *J* = 4.0 Hz, 3H), 2.50 (m, 2H), 2.39-2.32 (m, 1H), 2.29 (t, *J* = 7.5 Hz, 4H), 2.18 (s, 3H), 1.79-1.71 (m, 2H), 1.71-1.65 (m, 4H), 1.65-1.53 (m, 10H), 1.46-1.22 (m, 56H), 0.93-0.85 (m, 9H).

LC-MS (ESI): Calculated for 948.47, Found (M+H): 949.3.

### Example 42: Synthesis of Amino Lipid Compound 1924

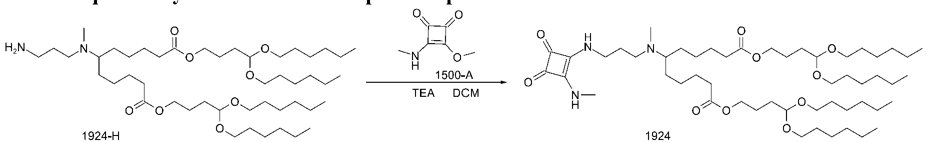

According to the general synthetic method, the amino lipid compound 1924 was synthesized according to the process of Step 6 of Example 24 with substituting 1924-H for 1644-H, wherein 1924-H (1.7 g, 2.09 mmol), triethylamine (0.32 g, 3.13 mmol), and 1500-A (0.44 g, 3.13 mmol) were used, resulting in 880 mg of the amino lipid compound 1924 with a yield of 45.7% and a purity of 95.9%.

¹H NMR (600 MHz, CDCl₃) δ 4.48 (t, *J =* 5.3 Hz, 2H), 4.07 *(t, J =* 6.2 Hz, 4H), 3.73-3.61 (m, 2H), 3.57 (dt, *J =* 9.2, 6.7 Hz, 4H), 3.41 (dt, *J* = 9.3, 6.7 Hz, 4H), 3.31 (d, *J* = 5.0 Hz, 3H), 2.49 (t, *J* = 6.2 Hz, 2H), 2.42-2.36 (m, 1H), 2.34-2.29 (m, 4H), 2.13 (s, 3H), 1.79-1.74 (m, 2H), 1.74-1.64 (m, 6H), 1.64-1.51 (m, 12H), 1.49-1.40 (m, 2H), 1.39-1.15 (m, 32H), 0.89 (t, *J* = 6.9 Hz, 12H).

LC-MS (ESI): Calculated for 924.36, Found (M+H): 925.2.

### Example 43: Synthesis of Amino Lipid Compound 1925

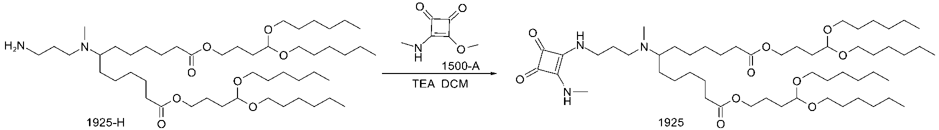

According to the general synthetic method, the amino lipid compound 1925 was synthesized according to the process of Step 6 of Example 24 with substituting 1925-H for 1644-H, wherein 1925-H (1.2 g, 1.42 mmol), triethylamine (0.22 g, 2.13 mmol), and 1500-A (0.30 g, 2.13 mmol) were used, resulting in 600 mg of the amino lipid compound 1925 with a yield of 44.3% and a purity of 93.8%.

¹H NMR (600 MHz, CDCl₃) δ 4.48 (t, *J* = 5.3 Hz, 2H), 4.08 *(t, J =* 6.2 Hz, 4H), 3.74-3.60 (m, 2H), 3.57 (dt, *J =* 9.3, 6.7 Hz, 4H), 3.45-3.37 (m, 4H), 3.28 (d, *J* = 4.8 Hz, 3H), 2.55 (t, *J =* 5.5 Hz, 2H), 2.43-2.36 (m, 1H), 2.34-2.28 (m, 4H), 2.19 (s, 3H), 1.65-1.53 (m, 22H), 1.51-1.41 (m, 2H), 1.41-1.22 (m, 34H), 0.89 (t, *J* = 7.0 Hz, 12H).

LC-MS (ESI): Calculated for 952.41, Found (M+H): 953.2.

### Example 44: Synthesis of Amino Lipid Compound 1926

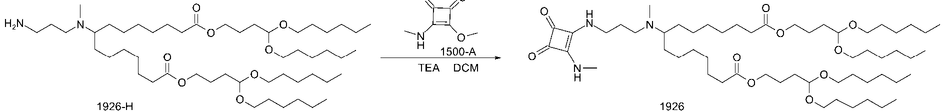

According to the general synthetic method, the amino lipid compound 1926 was synthesized according to the process of Step 6 of Example 24 with substituting 1926-H for 1644-H, wherein 1926-H (0.8 g, 0.92 mmol), triethylamine (0.14 g, 1.38 mmol), and 1500-A (0.19 g, 1.38 mmol) were used, resulting in 355 mg of the amino lipid compound 1926 with a yield of 39.5% and a purity of 95.6%.

¹H NMR (600 MHz, CDCl₃) δ 4.48 (t, *J =* 5.2 Hz, 2H), 4.08 (t, *J =* 6.2 Hz, 4H), 3.71-3.60 (m, 2H), 3.57 (dt, *J =* 9.2, 6.7 Hz, 4H), 3.41 (dt, *J =* 9.3, 6.7 Hz, 4H), 3.28 (d, *J* = 4.6 Hz, 3H), 2.53 (m, 2H), 2.38 (m, 1H), 2.30 (t, *J* = 7.5 Hz, 4H), 2.18 (s, 3H), 1.79-1.73 (m, 2H), 1.73-1.65 (m, 8H), 1.59 (m, 12H), 1.43 (m, 2H), 1.40-1.22 (m, 38H), 0.89 (t, *J* = 6.9 Hz, 12H).

LC-MS (ESI): Calculated for 980.47, Found (M+H): 981.2.

### Example 45: Synthesis of Amino Lipid Compound 1927

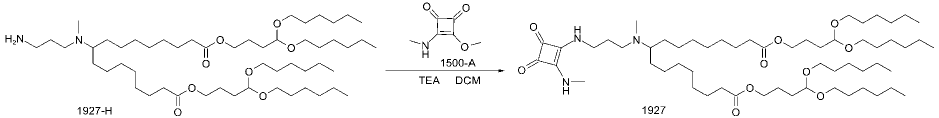

According to the general synthetic method, the amino lipid compound 1927 was synthesized according to the process of Step 6 of Example 24 with substituting 1927-H for 1644-H, wherein 1927-H (1.5 g, 1.67 mmol), triethylamine (0.25 g, 2.5 mmol), and 1500-A (0.35 g, 2.5 mmol) were used, resulting in 920 mg of the amino lipid compound 1927 with a yield of 54.7% and a purity of 94.7%.

¹H NMR (600 MHz, CDCl₃) δ 4.46 (t, *J =* 5.3 Hz, 2H), 4.06 (t, *J =* 6.2 Hz, 4H), 3.75-3.59 (m, 2H), 3.59-3.50 (dt, *J =* 9.2, 6.7 Hz, 4H), 3.46-3.34 (dt, *J =* 9.3, 6.7 Hz, 4H), 3.26 (d, *J =* 4.3 Hz, 3H), 2.56 (m, 2H), 2.41 (m, 1H), 2.27 (t, *J =* 7.6 Hz, 4H), 2.21 (s, 3H), 1.83-1.72 (m, 2H), 1.73-1.62 (m, 8H), 1.56 (m, 12H), 1.47-1.37 (m, 2H), 1.37-1.17 (m, 42H), 0.87 (t, *J =* 7.0 Hz, 12H).

LC-MS (ESI): Calculated for 1008.52, Found(M+H): 1009.2.

### Example 46: Synthesis of Amino Lipid Compound 1928

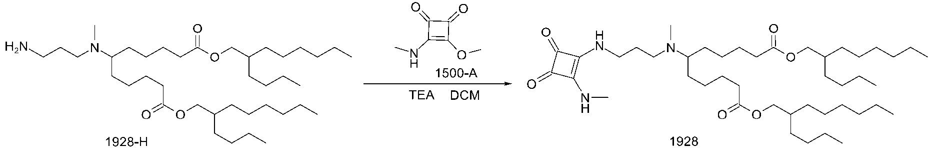

According to the general synthetic method, the amino lipid compound 1928 was synthesized according to the process of Step 6 of Example 24 with substituting 1928-H for 1644-H, wherein 1928-H (2.3 g, 3.6 mmol), triethylamine (0.55 g, 5.4 mmol), and 1500-A (0.76 g, 5.4 mmol) were used, resulting in 1.38 g of the amino lipid compound 1928 with a yield of 51.4% and a purity of 96.1%.

¹H NMR (600 MHz, CDCl₃) δ 4.00 (d, *J* = 5.8 Hz, 4H), 3.64 (m, 2H), 3.34 (d, *J* = 5.0 Hz, 3H), 2.53 (t, *J =* 6.1 Hz, 2H), 2.42 (p, *J* = 6.6 Hz, 1H), 2.37 (td, *J =* 7.3, 1.7 Hz, 4H), 2.16 (s, 3H), 1.80 (m, 4H), 1.69-1.62 (m, 4H), 1.54-1.45 (m, 2H), 1.40-1.24 (m, 38H), 0.92 (m, 12H).

LC-MS (ESI): Calculated for 748.15, Found(M+H): 749.1.

### Example 47: Synthesis of Amino Lipid Compound 1929

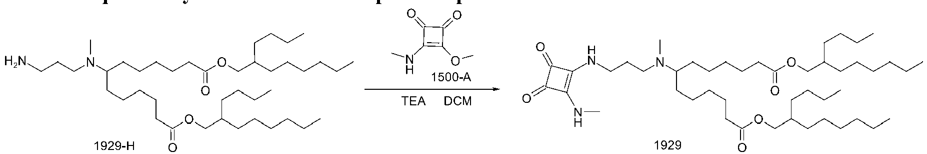

According to the general synthetic method, the amino lipid compound 1929 was synthesized according to the process of Step 6 of Example 24 with substituting 1929-H for 1644-H, wherein 1929-H (0.8 g, 1.2 mmol), triethylamine (0.18 g, 1.8 mmol), and 1500-A (0.25 g, 1.8 mmol) were used, resulting in 536 mg of the amino lipid compound 1929 with a yield of 57.6% and a purity of 94.8%.

¹H NMR (600 MHz, CDCl₃) δ 4.00 (d, *J* = 5.8 Hz, 4H), 3.69 (m, 2H), 3.33 (d, *J* = 5.0 Hz, 3H), 2.54 (t, *J* = 6.1 Hz, 2H), 2.40 (m, 1H), 2.37 (td, *J* = 7.3, 1.7 Hz, 4H), 2.20 (s, 3H), 1.80 (m, 4H), 1.68-1.63 (m, 4H), 1.53-1.41 (m, 2H), 1.41-1.26 (m, 42H), 0.92 (m, 12H).

LC-MS (ESI): Calculated for 776.20, Found(M+H): 773.1.

### Example 48: Synthesis of Amino Lipid Compound 1930

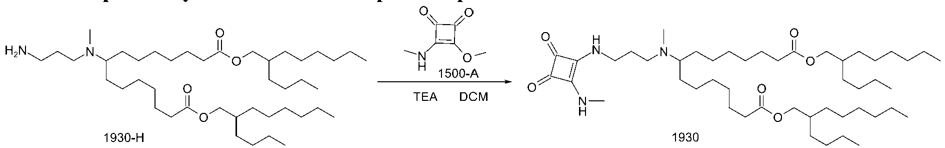

According to the general synthetic method, the amino lipid compound 1930 was synthesized according to the process of Step 6 of Example 24 with substituting 1930-H for 1644-H, wherein 1930-H (1.3 g, 1.87 mmol), triethylamine (0.28 g, 2.8 mmol), and 1500-A (0.4 g, 2.8 mmol) were used, resulting in 882 mg of the amino lipid compound 1930 with a yield of 58.7% and a purity of 97.1%.

¹H NMR (400 MHz, CDCl₃) δ 3.97 (d, *J* = 5.8 Hz, 4H), 3.71-3.57 (m, 2H), 3.28 (d, *J* = 4.4 Hz, 3H), 2.57-2.47 (m, 2H), 2.39-2.27 (m, 5H), 2.17 (s, 3H), 1.85 (m, 4H), 1.61 (m, 4H), 1.42 (m, 2H), 1.36-1.21 (m, 46H), 0.95-0.83 (m, 12H).

LC-MS (ESI): Calculated for 804.25, Found(M+H): 805.1.

### Example 49: Synthesis of Amino Lipid Compound 1931

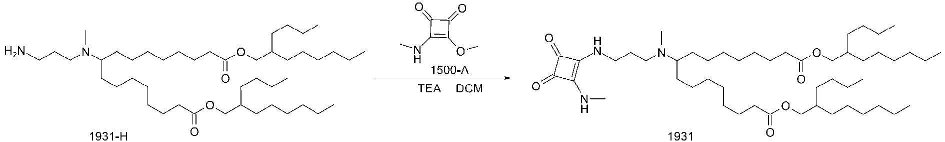

According to the general synthetic method, the amino lipid compound 1931 was synthesized according to the process of Step 6 of Example 24 with substituting 1931-H for 1644-H, wherein 1931-H (0.9 g, 1.24 mmol), triethylamine (0.19 g, 1.87 mmol), and 1500-A (0.26 g, 1.87 mmol) were used, resulting in 646 mg of the amino lipid compound 1931 with a yield of 62.4% and a purity of 95.8%.

¹H NMR (400 MHz, CDCl₃) δ 3.97 (d, *J* = 5.8 Hz, 4H), 3.63 (m, 2H), 3.28 (d, *J* = 3.9 Hz, 3H), 2.55 (t, *J =* 5.9 Hz, 2H), 2.43-2.35 (m, 1H), 2.30 (t, *J* = 7.5 Hz, 4H), 2.20 (s, 3H), 1.80-1.72 (m, 2H), 1.68-1.57 (m, 6H), 1.50-1.38 (m, 2H), 1.26 (m, 50H), 0.89 (m, 12H).

LC-MS (ESI): Calculated for 832.31, Found(M+H): 833.2.

### Example 50: Synthesis of Amino Lipid Compound 1936

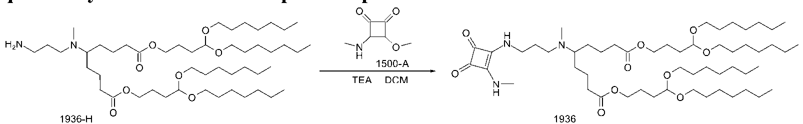

According to the general synthetic method, the amino lipid compound 1936 was synthesized according to the process of Step 6 of Example 24 with substituting 1936-H for 1644-H, wherein 1936-H (1.4 g, 1.66 mmol), triethylamine (0.25 g, 2.49 mmol), and 1500-A (0.35 g, 2.49 mmol) were used, resulting in 899 mg of the amino lipid compound 1936 with a yield of 56.8% and a purity of 94.7%.

¹H NMR (400 MHz, CDCl₃) δ 4.48 (t, *J=* 5.2 Hz, 2H), 4.13-4.03 (m, 4H), 3.74 (m, 2H), 3.57 (dt, *J=* 9.2, 6.7 Hz, 4H), 3.41 (dt, *J=* 9.3, 6.7 Hz, 4H), 3.31 (d, *J=* 5.0 Hz, 3H), 2.50 (t, *J=* 5.9 Hz, 2H), 2.42-2.30 (m, 5H), 2.15 (s, 3H), 1.69 (m, 12H), 1.60-1.51 (m, 10H), 1.39-1.16 (m, 36H), 0.88 *(t, J=* 6.9 Hz, 12H).

LC-MS (ESI): Calculated for 952.41, Found (M+H): 953.2.

**Example 51: Synthesis of Amino Lipid Compound 1640**

### Step 1: Synthesis of 1640-A0

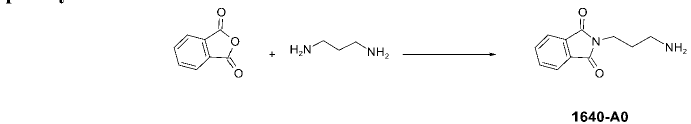

### Experimental procedure:

Phthalic anhydride (10 g, 67.5 mmol), toluene (100 ml), and 1,3-propanediamine (10 g, 135 mmol) were added to a 250 mL single-necked flask which was connected to a water separator, and was subjected to reflux in an oil bath at 140°C to react for 3 h. After the reaction stopped, the internal temperature was reduced to room temperature, and then water (100 mL) was added for washing. Phases were separated. The organic phase was collected. The solvent was removed from the organic phase by evaporation under reduced pressure at 45°C, obtaining 11.6 g 1640-A0.

### Step 2: Synthesis of 1640-A5

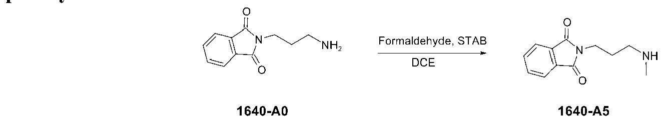

### Experimental procedure:

1640-A0 (11.6 g, 56.8 mmol), 1,2-dichloroethane (120 ml), and 37% aqueous formaldehyde solution (3.2 g, 39.76 mmol) were added to a 250 mL single-necked flask, then added with sodium triacetoxyborohydride (14.4 g, 68.16 mmol), and reacted at room temperature for 3 h. 10% Aqueous potassium bicarbonate solution (60 ml) was added, and stirred, and then phases were separated. The organic phase was collected. The water phase was extracted once with dichloromethane (60 ml), and the organic phases were combined. The solvent was removed from the organic phase by evaporation under reduced pressure at 45°C, obtaining 12.3 g of a coarse product of 1640-A5. The coarse product was purified by silica gel column chromatography and eluted with ethyl acetate:methanol = 20:1 to obtain 7.04 g 1640-A5 with a yield of 56.8%.

### Step 3: Synthesis of 1640-A2

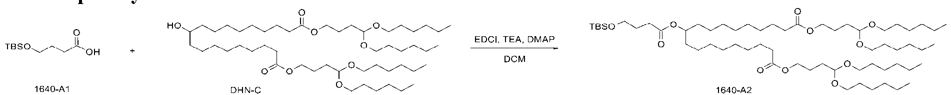

### Experimental procedure:

EDCI (1.68 g, 8.75 mmol), dichloromethane (50 ml), triethylamine (885 mg, 8.75 mmol), and DMAP (214 mg, 1.75 mmol) were added to a 100 mL single-necked flask, stirred for 10 min, then added with DHN-C (5.0 g, 5.83 mmol) and 1640-A1 (1.53 g, 7.0 mmol), and reacted for 15 h. Water (100 mL) was added, and stirred for 10 min. Phases were separated. The organic phase was collected. The water phase was extracted once with dichloromethane (50 mL), and the organic phases were combined. The solvent was removed from the organic phase by evaporation under reduced pressure at 45°C, obtaining 7.1 g of a coarse product of 1640-A2. The coarse product was purified by silica gel column chromatography and eluted with ethyl acetate and n-heptane = 1:30 to obtain 4.6 g 1640-A2 with a yield of 74.6%.

### Step 4: Synthesis of 1640-A3

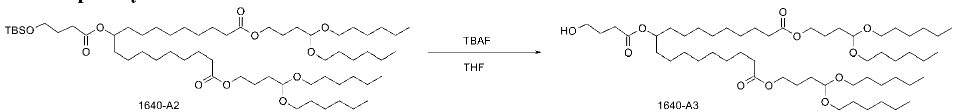

### Experimental procedure:

1640-A2 (4.6 g, 4.35 mmol), THF (46 mL), and TBAF·3H₂O (1.51 g, 4.79 mmol) were added to a 100 mL single-necked flask, and reacted at room temperature for 15 h. THF was removed by evaporation under reduced pressure at 45°C, obtaining a coarse product. The coarse product was purified by silica gel column chromatography and eluted with ethyl acetate:n-heptane = 1:20 to obtain 3.87 g 1640-A3 with a yield of 94.4%.

### Step 5: Synthesis of 1640-A4

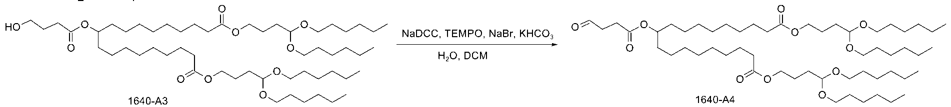

### Experimental procedure:

1640-A3 (3.8 g, 4.03 mmol), dichloromethane (24 mL), TEMPO (31 mg, 0.2 mmol), NaBr (41 mg, 0.4 mmol), and KHCO₃ (0.32 g, 3.2 mmol) were added to a 100 mL single-necked flask, cooled to below 5°C, and slowly added dropwise with an aqueous solution of NaDCC (576 mg NaDCC, 2.62 mmol, 19 ml water), and reacted at 5°C for 6 h after the dropwise addition was completed. Filtering was performed, and the filtrate was subjected to phases separation. The organic phase was collected. The water phase was extracted once with dichloromethane (30 mL), and the organic phases were combined. The solvent was removed from the organic phase by evaporation under reduced pressure at 45°C, obtaining 4.1 g of a coarse product of 1640-A4. The coarse product was purified by silica gel column chromatography and eluted with ethyl acetate:n-heptane = 1:30 to obtain 3.51 g 1640-A4 with a yield of 92.5%.

### Step 6: Synthesis of 1640-A6

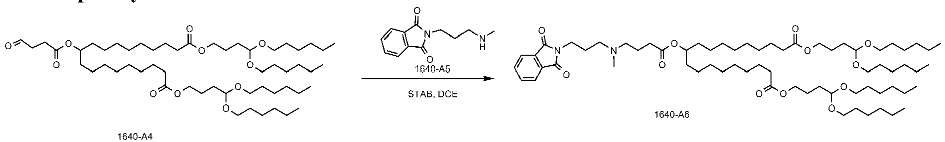

### Experimental procedure:

1640-A4 (3.7 g, 3.93 mmol), DCE (37 mL), and 1640-A5 (857 mg, 3.93 mmol) were added to a 100 mL single-necked flask, and finally added with STAB (1.0 g, 4.72 mmol), and reacted at room temperature for 3 h. Water (30 mL) was added to the reaction solution, and stirred for 5 min. Phases were separated. The organic phase was collected. The water phase was extracted once with dichloromethane (50 mL), and the organic phases were combined. The solvent was removed from the organic phase by rotary evaporation under reduced pressure at 45°C, obtaining 5.1 g of a coarse product of 1640-A6. The coarse product was purified by silica gel column chromatography and eluted with ethyl acetate:n-heptane = 1:1 to obtain 3.27 g 1640-A6 with a yield of 72.8%.

### Step 7: Synthesis of 1640-A7

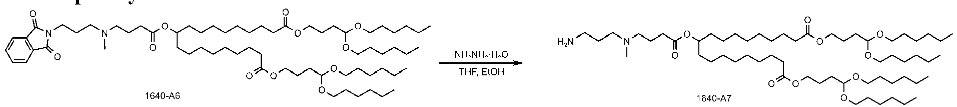

### Experimental procedure:

1640-A6 (3.27 g, 2.86 mmol), ethanol (17 mL), and THF (17 mL) were sequentially added to a 100 mL single-necked flask, added dropwise with hydrazine hydrate (715 mg, 14.3 mmol), heated to 85°C, and subjected to reflux and stirring for 2 h. After the reaction solution was cooled to the room temperature, water (50 mL) was added, followed by extraction twice with ethyl acetate (50 mL), and the organic phases were combined. The solvent was removed from the organic phase by evaporation under reduced pressure at 45°C, obtaining 3 g of a coarse product of 1640-A7. The coarse product was purified by silica gel column chromatography and eluted with ethyl acetate:methanol = 20:1 to obtain 2.77 g 1640-A7 with a yield of 95.5%.

### Step 8: Synthesis of Amino Lipid Compound 1640

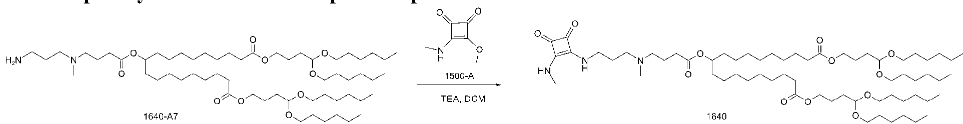

### Experimental procedure:

1640-A7 (2.7 g, 2.66 mmol), dichloromethane (27 mL), 1500-A (0.56 g, 4.0 mmol), and triethylamine (404 mg, 4.0 mmol) were added to a 100 mL single-necked flask, and reacted at room temperature for 15 h. The reaction solution was washed with saturated aqueous potassium bicarbonate solution (30 mL) and washed with saturated aqueous sodium chloride solution (30 mL), then dried with anhydrous sodium sulfate, and filtered. The solvent was removed from the filtrate by evaporation under reduced pressure at 45°C. The residue was purified by silica gel column chromatography and eluted with ethyl acetate:methanol = 20:1, obtaining 1.34 g of the amino lipid compound 1640 with a yield of 44.8 % and a purity of 94.6%.

¹H NMR (600 MHz, CDCl₃) δ 4.88-4.76 (m, 1H), 4.51 (t, *J* = 5.0 Hz, 2H), 4.11 *(t, J =* 5.9 Hz, 4H), 3.76 (s, 2H), 3.60 (dd, *J* = 15.8, 6.8 Hz, 4H), 3.43 (dt, *J* = 19.4, 9.6 Hz, 4H), 3.34 (d, *J=* 4.9 Hz, 3H), 2.49 (t, *J* = 5.8 Hz, 2H), 2.43 (t, *J* = 6.3 Hz, 2H), 2.38 (dd, *J* = 19.3, 12.9 Hz, 2H), 2.32 (t, *J* = 7.5 Hz, 4H), 2.19 (s, 3H), 1.90-1.78 (m, 4H), 1.71 (s, 8H), 1.68-1.53 (m, 16H), 1.45-1.24 (m, 44H), 0.92 (t, *J=* 6.8 Hz, 12H).

LC-MS (ESI): Calculated for 1122.66, ^{Found} (M+H): 1123.4.

### Example 52: Synthesis of Amino Lipid Compound 1720

### Step 1: Synthesis of 1720-E

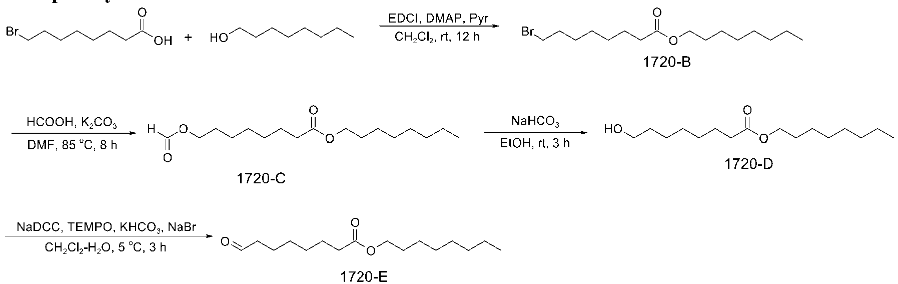

With reference to the synthesis processes of Step 2 to Step 5 of Example 1, 1720-B was obtained from the esterification of 8-bromooctanoic acid and 1-octanol; 1720-C was obtained from esterfying 1720-B with formic acid; 1720-C was hydrolyzed to obtain 1720-D; and 1720-D was oxidized to obtain 1720-E. 1720-D (10 g, 36.71 mmol), dichloromethane (60 ml), TEMPO (287 mg, 1.84 mmol), NaBr (378 mg, 3.67 mmol), and KHCO₃ (2.94 g, 29.37 mmol) were used, resulting in 9.17 g 1720-E with a yield of 92.4%.

### Step 2: Synthesis of 1720-A1

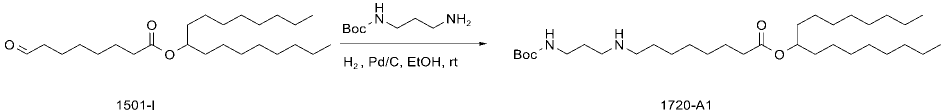

### Experimental procedure:

A 300 mL autoclave was added with ethanol (100 mL), 1501-I (10.0 g, 25.21 mmol), Boc-propanediamine (5.27 g, 30.25 mmol), and 10% Pd/C (1.0 g, 10%w.t.), vacuumized, then filled with nitrogen for displacement for three times, subsequently filled with hydrogen for displacement for three times, and finally filled with hydrogen to 2.0 MPa. The reaction was performed at room temperature for 12 h. Filtering was then performed, and the filtrate was collected. The solvent was removed by evaporation under reduced pressure at 45°C, obtaining 15.5 g of a coarse product of 1720-A1. The coarse product was purified by silica gel column chromatography and eluted with ethyl acetate:n-heptane = 1:5 to obtain 11.89 g 1720-A1 with a yield of 85.0%.

### Step 3: Synthesis of 1720-A2

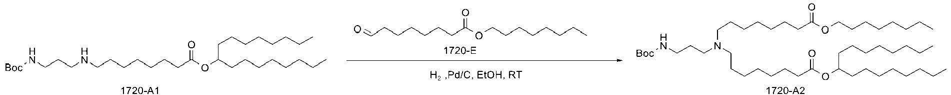

### Experimental procedure:

A 300 mL autoclave was added sequentially with 1720-A1 (11.89 g, 21.42 mmol), 1720-E (6.95 g, 25.70 mmol), absolute ethyl alcohol (60 ml), and 10% Pd/C (1.19 g, 10%w.t.), vacuumized, then filled with nitrogen for displacement for three times, subsequently filled with hydrogen for displacement for three times, and finally filled with hydrogen to 2.0 MPa. The reaction was performed at room temperature for 12 h. Filtering was then performed, and the filtrate was collected. The solvent was removed by evaporation under reduced pressure at 45°C, obtaining 19.0 g of a coarse product of 1720-A2. The coarse product was purified by silica gel column chromatography and eluted with ethyl acetate:n-heptane = 70:30 to obtain 12.13 g 1720-A2 with a yield of 70.0%.

### Step 4: Synthesis of 1720-A3

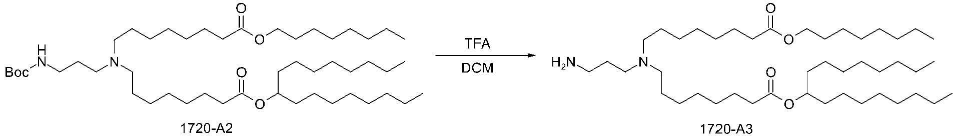

### Experimental procedure:

1720-A2 (12.13 g, 14.99 mmol) and dichloromethane solution of 20% TFA (120 mL) were added to a 250 mL single-necked flask, and reacted at room temperature with stirring for 4 h. The solvent was removed by evaporation under reduced pressure at 45°C, and then dichloromethane (30 mL) was added. Rotary evaporation under reduced pressure was continued to bring away the remaining TFA, which was repeated for three times, obtaining 10.8 g of a coarse product of 1720-A3. The coarse product was purified by silica gel column chromatography and eluted with ethyl acetate:methanol = 90:10 to obtain 9.88 g 1720-A3 with a yield of 92.9%.

### Step 5: Synthesis of 1720-A4

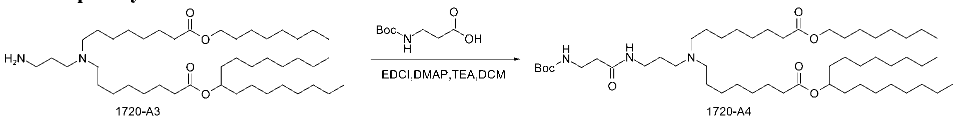

### Experimental procedure:

EDCI (4.0 g, 20.91 mmol), dichloromethane (100 mL), DMAP (169 mg, 1.39 mmol), and triethylamine (2.11 g, 20.91 mmol) were added to a 250 mL single-necked flask, stirred homogeneously, then added with 1720-A3 (9.88 g, 13.93 mmol) and Boc-alanine (3.17 g, 16.75 mmol), and reacted at room temperature with stirring for 12 h. Water (100 mL) was added. Phases were separated. The water phase was extracted twice with dichloromethane (100 mL), and the organic phases were combined. The organic phase was washed with saturated brine (100 mL), then dried with anhydrous sodium sulfate, and filtered. The solvent was removed from the filtrate by evaporation under reduced pressure at 45°C, obtaining 14.6 g of a coarse product of 1720-A4. The coarse product was purified by silica gel column chromatography and eluted with ethyl acetate:n-heptane = 80:20 to obtain 9.11 g 1720-A4 with a yield of 74.3%.

### Step 6: Synthesis of 1720-A5

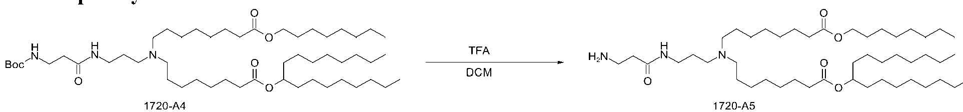

### Experimental procedure:

1720-A4 (9.11 g, 10.35 mmol) and dichloromethane solution of 20% TFA (91 mL) were added to a 250 mL single-necked flask, and reacted at room temperature with stirring for 4 h. The solvent was removed by evaporation under reduced pressure at 45°C, and then dichloromethane (30 mL) was added. Rotary evaporation under reduced pressure was continued to bring away the remaining TFA, which was repeated for three times, obtaining 9.3 g of a coarse product of 1720-A5. The coarse product was purified by silica gel column chromatography and eluted with ethyl acetate:methanol = 90:10 to obtain 7.40 g 1720-A5 with a yield of 91.6%.

### Step 7: Synthesis of Amino Lipid Compound 1720

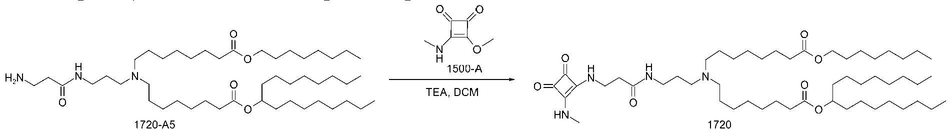

### Experimental procedure:

1720-A5 (3.9 g, 5.0 mmol), dichloromethane (39 mL), triethylamine (607 mg, 6.0 mmol), and 1500-A (846 mg, 6.0 mmol) were sequentially added to a 100 mL single-necked flask, and reacted at room temperature overnight. The solvent was removed by evaporation under reduced pressure at 45°C, obtaining 4.6 g of a coarse product of 1720. The coarse product was purified by silica gel column chromatography and eluted with ethyl acetate and methanol = 90:10 to obtain 1.97 g of the amino lipid compound 1720 with a yield of 44.5% and a purity of 96.6%.

¹H NMR (600 MHz, CDCl₃) 5.01-4.73 (m, 1H), 4.04 (t, *J* = 6.8 Hz, 2H), 3.33-3.24 (m, 5H), 2.54-2.45 (m, 4H), 2.41-2.33 (m, 4H), 2.28 (q, *J* = 7.6 Hz, 4H), 1.96 (s, 2H), 1.67-1.56 (m, 8H), 1.50 (m, 4H), 1.44-1.35 (m, 4H), 1.36-1.20 (m, 46H), 0.91-0.84 (m, 9H).

LC-MS (ESI): Calculated for 889.36, Found (M+H): 890.2.

### Example 53: Synthesis of Amino Lipid Compound 1523

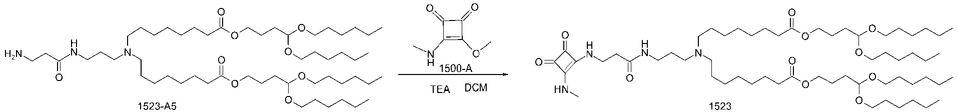

According to the general synthetic method, the amino lipid compound 1523 was synthesized according to the process of Step 7 of Example 52 with substituting 1523-A5 for 1720-A5, wherein 1523-A5 (4.0 g, 4.24 mmol), triethylamine (514 mg, 5.09 mmol), and 1500-A (718 mg, 5.09 mmol) were used, resulting in 1.97 g of the amino lipid compound 1523 with a yield of 44.1% and a purity of 95.29%.

¹H NMR (600 MHz, CDCl₃) δ 4.51 (t, *J* = 5.2 Hz, 2H), 4.12 (t, *J =* 6.1 Hz, 4H), 3.90 (s, 2H), 3.85-3.81 (m, 2H), 3.75 (s, 2H), 3.61 (dt, *J* = 9.2, 6.7 Hz, 4H), 3.45 (dt, *J* = 9.2, 6.7 Hz, 4H), 3.41 (d, *J* = 5.0 Hz, 2H), 3.38-3.34 (m, 2H), 3.33 (d, *J* = 4.8 Hz, 3H), 2.56 (t, *J=* 5.8 Hz, 2H), 2.34 (t, *J=* 7.5 Hz, 4H), 1.74 (m, 14H), 1.62 (m, 12H), 1.55 (m, 4H), 1.42-1.33 (m, 32H), 0.94 (m, 12H).

LC-MS (ESI): Calculated for 1051.55, Found (M+H): 1052.4.

### Example 54: Synthesis of Amino Lipid Compound 1524

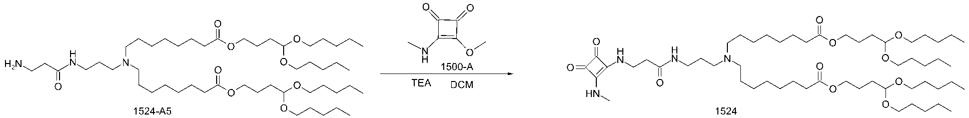

According to the general synthetic method, the amino lipid compound 1524 was synthesized according to the process of Step 7 of Example 52 with substituting 1524-A5 for 1720-A5, wherein 1524-A5 (2.0 g, 2.26 mmol), triethylamine (273 mg, 2.71 mmol), and 1500-A (382 mg, 2.71 mmol) were used, resulting in 879 mg of the amino lipid compound 1524 with a yield of 39.1% and a purity of 95.04%.

¹H NMR (600 MHz, CDCl₃) δ 4.52 (t, *J =* 5.2 Hz, 2H), 4.12 (t, *J =* 6.1 Hz, 4H), 3.90 (s, 2H), 3.85-3.81 (m, 2H), 3.75 (s, 2H), 3.61 (dt, *J* = 9.2, 6.7 Hz, 4H), 3.45 (dt, *J* = 9.2, 6.7 Hz, 4H), 3.41 (d, *J* = 5.0 Hz, 2H), 3.38-3.34 (m, 2H), 3.33 (d, *J* = 4.8 Hz, 3H), 2.56 (t, *J=* 5.8 Hz, 2H), 2.34 (t, *J =* 7.5 Hz, 4H), 1.74 (m, 14H), 1.62 (m, 12H), 1.55 (m, 4H), 1.42-1.33 (m, 24H), 0.94 (m, 12H).

LC-MS (ESI): Calculated for 995.44, Found (M+H): 996.3.

### Example 55: Synthesis of Amino Lipid Compound 1525

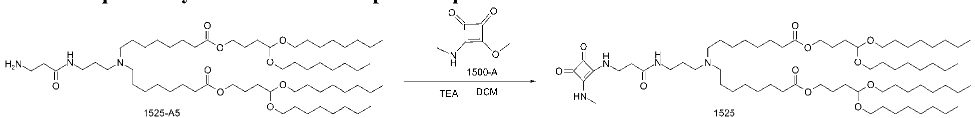

According to the general synthetic method, the amino lipid compound 1525 was synthesized according to the process of Step 7 of Example 52 with substituting 1525-A5 for 1720-A5, wherein 1525-A5 (2.0 g, 1.90 mmol), triethylamine (230 mg, 2.28 mmol), and 1500-A (321 mg, 2.28 mmol) were used, resulting in 888 mg of the amino lipid compound 1525 with a yield of 40.2% and a purity of 95.64%.

¹H NMR (600 MHz, CDCl₃) δ 4.52 (t, *J* = 5.2 Hz, 2H), 4.12 (t, *J* = 6.2 Hz, 4H), 3.89 (s, 2H), 3.76 (q, *J* = 7.0 Hz, 2H), 3.60 (dt, *J =* 9.2, 6.7 Hz, 4H), 3.45 (dt, *J =* 9.2, 6.7 Hz, 4H), 3.34 (d, *J* = 3.9 Hz, 4H), 3.38-3.34 (m, 2H), 3.33 (d, *J =* 4.8 Hz, 3H), 2.56 (t, *J =* 5.8 Hz, 2H), 2.34 (t, *J =* 7.5 Hz, 4H), 1.74 (m, 14H), 1.63 (m, 12H), 1.54(m, 4H), 1.43-1.33 (m, 48H), 0.92 (t, *J=* 7.0 Hz, 12H).

LC-MS (ESI): Calculated for 1163.76, Found (M+H): 1164.6.

### Example 56: Synthesis of Amino Lipid Compound 1526

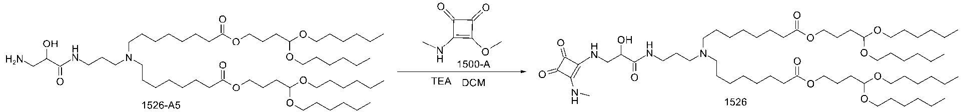

According to the general synthetic method, the amino lipid compound 1526 was synthesized according to the process of Step 7 of Example 52 with substituting 1526-A5 for 1720-A5, wherein 1526-A5 (2.0 g, 2.09 mmol), triethylamine (260 mg, 2.58 mmol), and 1500-A (364 mg, 2.58 mmol) were used, resulting in 939 mg of the amino lipid compound 1526 with a yield of 42.1% and a purity of 91.23%.

¹H NMR (600 MHz, CDCl₃) δ 4.48 (t, *J* = 5.3 Hz, 2H), 4.08 (t, *J* = 6.1 Hz, 4H), 3.57 (dt, *J =* 9.2, 6.7 Hz, 2H), 3.41 (dt, *J =* 9.3, 6.7 Hz, 4H), 3.31 (d, *J* = 12.9 Hz, 3H), 2.49 (t, *J* = 6.4 Hz, 2H), 2.47-2.35 (m, 4H), 2.29 (t, *J =* 7.5 Hz, 4H), 2.13 (m, 5H), 1.76-1.64 (m, 8H), 1.64-1.51 (m, 12H), 1.49-1.39 (m, 8H), 1.40-1.19 (m, 36H), 0.96-0.84 (m, 12H).

LC-MS (ESI): Calculated for 1067.55, Found (M+H): 1068.3.

### Example 57: Synthesis of Amino Lipid Compound 1527

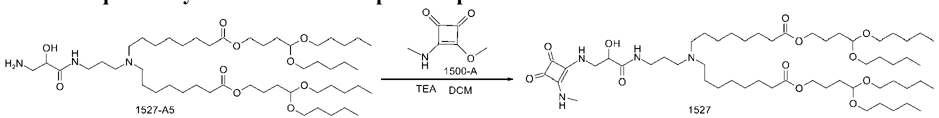

According to the general synthetic method, the amino lipid compound 1527 was synthesized according to the process of Step 7 of Example 52 with substituting 1527-A5 for 1720-A5, wherein 1527-A5 (2.0 g, 2.22 mmol), triethylamine (278 mg, 2.76 mmol), and 1500-A (389 mg, 2.76 mmol) were used, resulting in 932 mg of the amino lipid compound 1527 with a yield of 41.5% and a purity of 98.18%.

¹H NMR (600 MHz, CDCl₃) δ 4.46 (t, *J* = 5.3 Hz, 2H), 4.09 (t, *J* = 6.1 Hz, 4H), 3.58 (dt, *J=* 9.2, 6.7 Hz, 2H), 3.41 (dt, *J =* 9.3, 6.7 Hz, 4H), 3.31 (d, *J* = 12.9 Hz, 3H), 2.49 (t, *J* = 6.4 Hz, 2H), 2.48-2.35 (m, 4H), 2.31 (t, *J=* 7.5 Hz, 4H), 2.14 (m, 5H), 1.77-1.64 (m, 8H), 1.65-1.52 (m, 12H), 1.49-1.39 (m, 8H), 1.40-1.19 (m, 28H), 0.93-0.82 (m, 12H).

LC-MS (ESI): Calculated for 1011.44, Found (M+H): 1012.3.

### Example 58: Synthesis of Amino Lipid Compound 1528

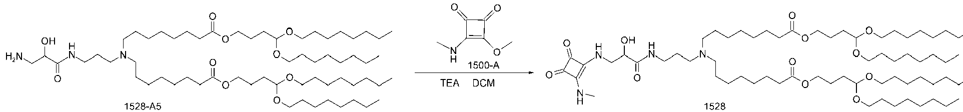

According to the general synthetic method, the amino lipid compound 1528 was synthesized according to the process of Step 7 of Example 52 with substituting 1528-A5 for 1720-A5, wherein 1528-A5 (2.0 g, 1.87 mmol), triethylamine (232 mg, 2.31 mmol), and 1500-A (326 mg, 2.31 mmol) were used, resulting in 89.7 mg of the amino lipid compound 1528 with a yield of 40.7% and a purity of 95.09%.

¹H NMR (600 MHz, CDCl₃) δ 4.48 (t, *J* = 5.3 Hz, 2H), 4.08 (t, *J* = 6.1 Hz, 4H), 3.57 (dt, *J=* 9.2, 6.7 Hz, 2H), 3.41 (dt, *J=* 9.3, 6.7 Hz, 4H), 3.31 (d, *J* = 12.9 Hz, 3H), 2.49 (t, *J* = 6.4 Hz, 2H), 2.47-2.35 (m, 4H), 2.29 (t, *J =* 7.5 Hz, 4H), 2.13 (s, 5H), 1.76-1.64 (m, 10H), 1.64-1.51 (m, 12H), 1.49-1.39 (m, 8H), 1.40-1.19 (m, 50H), 0.96-0.84 (m, 12H).

LC-MS (ESI): Calculated for 1179.76, Found (M+H): 1180.5.

### Example 59: Synthesis of Amino Lipid Compound 1531

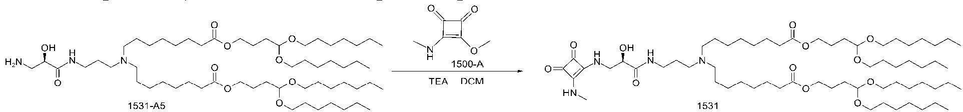

According to the general synthetic method, the amino lipid compound 1531 was synthesized according to the process of Step 7 of Example 52 with substituting 1531-A5 for 1720-A5, wherein 1531-A5 (2.0 g, 1.97 mmol), triethylamine (239 mg, 2.36 mmol), and 1500-A (333 mg, 2.36 mmol) were used, resulting in 914 mg of the amino lipid compound 1531 with a yield of 41.3% and a purity of 99.19%.

¹H NMR (600 MHz, CDCl₃) δ 4.48 (t, *J=* 5.2 Hz, 2H), 4.24 (s, 1H), 4.07 *(t, J=* 6.1 Hz, 4H), 3.87 (d, *J* = 62.2 Hz, 2H), 3.56 (m, *J* = 9.2, 6.7 Hz, 4H), 3.40 (m, *J* = 9.2, 6.7 Hz, 4H), 3.32 (s, 5H), 2.46 (t, *J* = 6.5 Hz, 2H), 2.42-2.33 (m, 4H), 2.29 (t, *J* = 7.5 Hz, 4H), 1.81-1.63 (m, 20H), 1.57 (m, 10H), 1.47-1.05 (m, 40H), 0.88 (t, *J=* 6.9 Hz, 12H).

LC-MS (ESI): Calculated for 1123.65, Found (M+H): 1124.3.

### Example 60: Synthesis of Amino Lipid Compound 1532

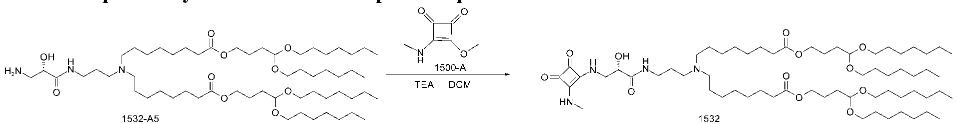

According to the general synthetic method, the amino lipid compound 1532 was synthesized according to the process of Step 7 of Example 52 with substituting 1532-A5 for 1720-A5, wherein 1532-A5 (2.0 g, 1.97 mmol), triethylamine (239 mg, 2.36 mmol), and 1500-A (333 mg, 2.36 mmol) were used, resulting in 929 mg of the amino lipid compound 1532 with a yield of 42.0% and a purity of 98.18%.

¹H NMR (600 MHz, CDCl₃) δ 4.48 (t, *J=* 5.2 Hz, 2H), 4.24 (s, 1H), 4.07 *(t, J=* 6.1 Hz, 4H), 3.87 (d, *J* = 62.2 Hz, 2H), 3.56 (m, *J =* 9.2, 6.7 Hz, 4H), 3.40 (m, *J =* 9.2, 6.7 Hz, 4H), 3.32 (s, 5H), 2.46 *(t, J=* 6.5 Hz, 2H), 2.42-2.33 (m, 4H), 2.29 (t, *J* = 7.5 Hz, 4H), 1.81-1.63 (m, 20H), 1.57 (m, 10H), 1.47-1.05 (m, 40H), 0.88 (t, *J=* 6.9 Hz, 12H).

LC-MS (ESI): Calculated for 1123.65, Found (M+H): 1124.3.

### Example 61: Synthesis of Amino Lipid Compound 1721

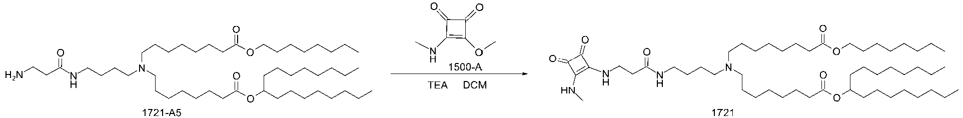

According to the general synthetic method, the amino lipid compound 1721 was synthesized according to the process of Step 7 of Example 52 with substituting 1721-A5 for 1720-A5, wherein 1721-A5 (2.0 g, 2.56 mmol), triethylamine (310 mg, 3.08 mmol), and 1500-A (434 mg, 3.08 mmol) were used, resulting in 1.14 g of the amino lipid compound 1721 with a yield of 49.3% and a purity of 91.98%.

¹H NMR (600 MHz, CDCl₃) δ 4.89 (m, 1H), 4.09 (t, *J* = 6.8 Hz, 2H), 3.92 (m, 2H), 3.34 (d, *J* = 3.8 Hz, 3H), 3.26 (d, *J=* 5.6 Hz, 2H), 2.60 (dd, *J=* 12.4, 6.9 Hz, 8H), 2.36-2.29 (m, 4H), 1.71-1.62 (m, 6H), 1.53 (m, 12H), 1.41-1.22 (m, 46H), 0.92 (m, 9H).

LC-MS (ESI): Calculated for 903.39, Found (M+H): 904.1.

### Example 62: Synthesis of Amino Lipid Compound 1726

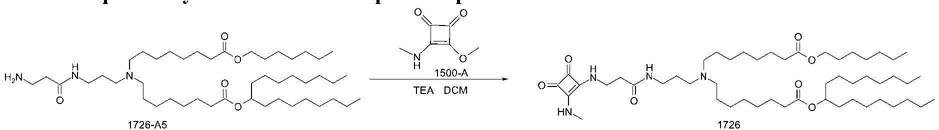

According to the general synthetic method, the amino lipid compound 1726 was synthesized according to the process of Step 7 of Example 52 with substituting 1726-A5 for 1720-A5, wherein 1726-A5 (2.0 g, 2.52 mmol), triethylamine (316 mg, 3.13 mmol), and 1500-A (441 mg, 3.13 mmol) were used, resulting in 1.02 g of the amino lipid compound 1726 with a yield of 46.3% and a purity of 97.92%.

¹H NMR (600 MHz, CDCl₃) δ 4.93-4.85 (m, 1H), 4.09 (t, *J* = 6.8 Hz, 2H), 3.92 (m, 2H), 3.41-3.21 (m, 5H), 2.54 (t, *J* = 5.5 Hz, 4H), 2.44-2.37 (m, 4H), 2.33 (q, *J=* 7.6 Hz, 4H), 1.71-1.61 (m, 8H), 1.53 (t, *J* = 13.9 Hz, 4H), 1.44 (dt, *J* = 14.9, 7.5 Hz, 4H), 1.40-1.23 (m, 44H), 0.92 (m, 9H).

LC-MS (ESI): Calculated for 875.33, Found (M+H): 876.1.

### Example 63: Synthesis of Amino Lipid Compound 1727

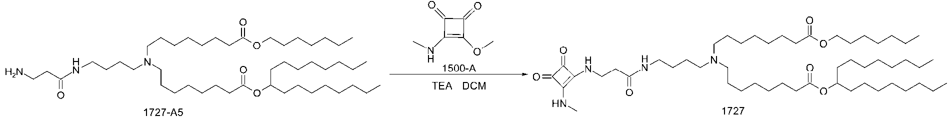

According to the general synthetic method, the amino lipid compound 1727 was synthesized according to the process of Step 7 of Example 52 with substituting 1727-A5 for 1720-A5, wherein 1727-A5 (2.0 g, 2.56 mmol), triethylamine (316 mg, 3.13 mmol), and 1500-A (441 mg, 3.13 mmol) were used, resulting in 882 mg of the amino lipid compound 1727 with a yield of 38.7% and a purity of 91.70%.

¹H NMR (600 MHz, CDCl₃) δ 5.05-4.79 (m, 1H), 4.05 (t, *J* = 6.8 Hz, 2H), 3.84 (m, 2H), 3.30 (d, *J* = 3.8 Hz, 3H), 3.28-3.13 (m, 2H), 2.64-2.47 (m, 2H), 2.38 (dd, *J* = 14.8, 7.8 Hz, 6H), 2.28 (q, *J=* 7.6 Hz, 4H), 1.66-1.56 (m, 6H), 1.56-1.45 (m, 8H), 1.43-1.17 (m, 48H), 0.94-0.81 (m, 9H).

LC-MS (ESI): Calculated for 889.36, Found (M+H): 890.3.

### Example 64: Synthesis of Amino Lipid Compound 1732

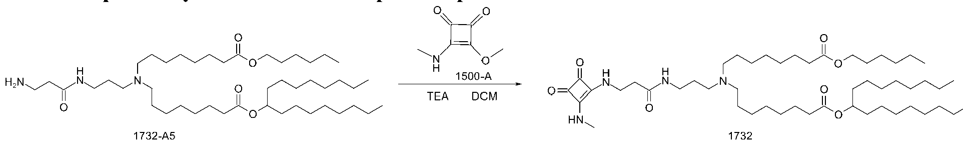

According to the general synthetic method, the amino lipid compound 1732 was synthesized according to the process of Step 7 of Example 52 with substituting 1732-A5 for 1720-A5, wherein 1732-A5 (2.0 g, 2.66 mmol), triethylamine (328 mg, 3.25 mmol), and 1500-A (458 mg, 3.25 mmol) were used, resulting in 900 mg of the amino lipid compound 1732 with a yield of 39.3% and a purity of 99.57%.

¹H NMR (600 MHz, CDCl₃) δ 4.93-4.86 (m, 1H), 4.09 (t, *J* = 6.8 Hz, 2H), 3.89 (m, 2H), 3.42-3.23 (m, 5H), 2.60-2.48 (m, 4H), 2.47-2.37 (m, 4H), 2.32 (m, 4H), 2.14 (s, 2H), 1.79-1.19 (m, 56H), 0.96-0.86 (m, 9H).

LC-MS (ESI): Calculated for 861.31, Found (M+H): 862.1.

### Example 65: Synthesis of Amino Lipid Compound 1733

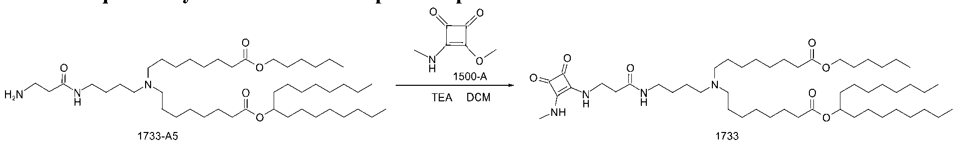

According to the general synthetic method, the amino lipid compound 1733 was synthesized according to the process of Step 7 of Example 52 with substituting 1733-A5 for 1720-A5, wherein 1733-A5 (2.0 g, 2.61 mmol), triethylamine (316 mg, 3.13 mmol), and 1500-A (441 mg, 3.13 mmol) were used, resulting in 913 mg of the amino lipid compound 1733 with a yield of 40.0% and a purity of 94.98%.

¹H NMR (600 MHz, CDCl₃) δ 4.93-4.84 (m, 1H), 4.09 (t, *J* = 6.8 Hz, 2H), 3.89 (m, 2H), 3.52 (s, 6H), 3.34 (s, 3H), 3.25 (q, *J* = 6.1 Hz, 2H), 2.59 (t, *J* = 5.7 Hz, 2H), 2.34-2.29 (m, 4H), 1.69-1.59 (m, 6H), 1.59-1.49 (m, 8H), 1.49-1.41 (m, 4H), 1.40-1.23 (m, 42H), 0.92 (m, 9H).

LC-MS (ESI): Calculated for 875.33, Found (M+H): 876.3.

### Example 66: Synthesis of Amino Lipid Compound 1746

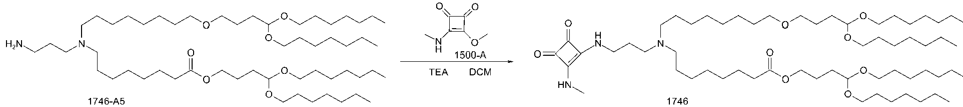

According to the general synthetic method, the amino lipid compound 1746 was synthesized according to the process of Step 7 of Example 52 with substituting 1746-A5 for 1720-A5, wherein 1746-A5 (2.0 g, 2.19 mmol), triethylamine (261 mg, 2.58 mmol), and 1500-A (364 mg, 2.58 mmol) were used, resulting in 996 mg of the amino lipid compound 1746 with a yield of 44.5% and a purity of 96.65%.

¹H NMR (600 MHz, CDCl₃) δ 4.52 (t, *J* = 5.4 Hz, 2H), 4.12 (t, *J* = 6.2 Hz, 2H), 3.60 (dtd, *J* = 7.8, 6.6, 1.0 Hz, 4H), 3.44 (dt, *J=* 15.1, 6.6 Hz, 8H), 3.30 (d, *J =* 4.4 Hz, 3H), 2.61 (s, 2H), 2.52-2.43 (m, 4H), 2.33 (t, *J =* 7.5 Hz, 2H), 2.01-1.54 (m, 26H), 1.51-1.28 (m, 48H), 0.92 (m, 12H).

LC-MS (ESI): Calculated for 1022.59, Found (M+H): 1023.4.

### Example 67: Synthesis of Amino Lipid Compound 1747

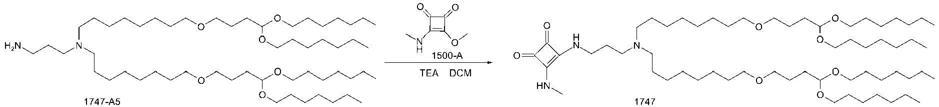

According to the general synthetic method, the amino lipid compound 1747 was synthesized according to the process of Step 7 of Example 52 with substituting 1747-A5 for 1720-A5, wherein 1747-A5 (2.0 g, 2.22 mmol), triethylamine (269 mg, 2.67 mmol), and 1500-A (376 mg, 2.67 mmol) were used, resulting in 967 mg of the amino lipid compound 1747 with a yield of 43.2% and a purity of 97.72%.

¹H NMR (600 MHz, CDCl₃) δ 4.47 (t, *J* = 5.4 Hz, 2H), 3.56 (dt, *J* = 9.2, 6.7 Hz, 4H), 3.40 (dt, *J* = 14.9, 6.5 Hz, 12H), 3.25 (d, *J = 4.2* Hz, 3H), 2.57-2.52 (m, 2H), 2.45-2.37 (m, 4H), 1.74 (dd, *J* = 11.9, 5.9 Hz, 4H), 1.68-1.60 (m, 8H), 1.59-1.51 (m, 12H), 1.44-1.21 (m, 52H), 0.88 (t, *J =* 7.0 Hz, 12H).

LC-MS (ESI): Calculated for 1008.61, Found (M+H): 1009.4.

### Example 68: Synthesis of Amino Lipid Compound 1748

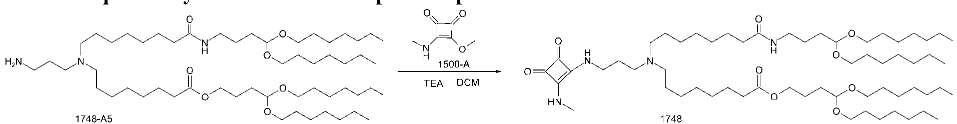

According to the general synthetic method, the amino lipid compound 1748 was synthesized according to the process of Step 7 of Example 52 with substituting 1748-A5 for 1720-A5, wherein 1748-A5 (2.0 g, 2.16 mmol), triethylamine (270 mg, 2.68 mmol), and 1500-A (378 mg, 2.68 mmol) were used, resulting in 941 mg of the amino lipid compound 1748 with a yield of 42.1% and a purity of 99.31 %.

¹H NMR (600 MHz, CDCl₃) δ 4.47 (dt, *J* = 8.6, 5.3 Hz, 2H), 4.07 (t, *J* = 6.2 Hz, 2H), 3.66 (bs, 2H), 3.60-3.52 (m, 4H), 3.40 (dt, *J* = 9.2, 6.7 Hz, 4H), 3.28 (d, *J* = 4.9 Hz, 3H), 3.23 (dd, *J* = 12.5, 6.5 Hz, 2H), 2.47 *(t, J =* 6.1 Hz, 2H), 2.36 (m, 4H), 2.28 (t, *J =* 7.6 Hz, 2H), 2.17 (t, *J =* 7.5 Hz, 2H), 1.85 (bs, 2H), 1.77-1.51 (m, 22H), 1.42-1.21 (m, 47H), 0.88 (t, *J =* 6.9 Hz, 12H).

LC-MS (ESI): Calculated for 1035.59, Found (M+H): 1036.2.

### Example 69: Synthesis of Amino Lipid Compound 1749

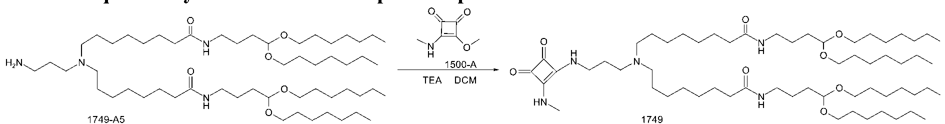

According to the general synthetic method, the amino lipid compound 1749 was synthesized according to the process of Step 7 of Example 52 with substituting 1749-A5 for 1720-A5, wherein 1749-A5 (2.0 g, 2.16 mmol), triethylamine (270 mg, 2.68 mmol), and 1500-A (378 mg, 2.68 mmol) were used, resulting in 922 mg of the amino lipid compound 1749 with a yield of 41.2% and a purity of 97.19%.

¹H NMR (600 MHz, CDCl₃) δ 4.46 (t, *J* = 5.3 Hz, 2H), 3.66 (bs, 2H), 3.56 (dt, *J* = 9.2, 6.7 Hz, 4H), 3.40 (dt, *J =* 9.2, 6.7 Hz, 4H), 3.29 (d, *J* = 5.0 Hz, 3H), 3.24 (dd, *J* = 12.6, 6.6 Hz, 4H), 2.44 (t, *J* = 6.0 Hz, 2H), 2.33 (t, *J* = 7.0 Hz, 4H), 2.16 (t, *J* = 7.6 Hz, 4H), 1.83 (bs, 2H), 1.76-1.68 (m, 2H), 1.68-1.50 (m, 20H), 1.42-1.19 (m, 48H), 0.88 (t, *J* = 7.0 Hz, 12H).

LC-MS (ESI): Calculated for 1034.61, Found (M+H): 1035.2.

### Example 70: Synthesis of Amino Lipid Compound 1807

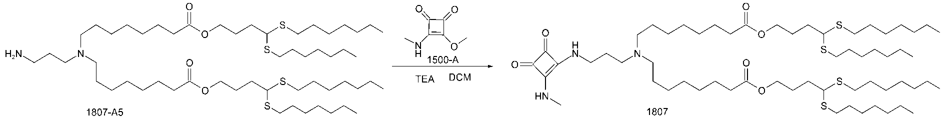

According to the general synthetic method, the amino lipid compound 1807 was synthesized according to the process of Step 7 of Example 52 with substituting 1807-A5 for 1720-A5, wherein 1807-A5 (2.0 g, 2.02 mmol), triethylamine (247 mg, 2.45 mmol), and 1500-A (345 mg, 2.45 mmol) were used, resulting in 966 mg of the amino lipid compound 1807 with a yield of 43.5% and a purity of 92.36%.

¹H NMR (600 MHz, CDCl₃) δ 4.08 (t, *J* = 6.0 Hz, 4H), 3.76 (t, *J* = 6.5 Hz, 2H), 3.65 (s, 2H), 3.27 (s, 3H), 2.72-2.61 (m, 4H), 2.62-2.50 (m, 6H), 2.47-2.36 (m, 4H), 2.30 (t, *J* = 7.5 Hz, 4H), 1.94-1.81 (m, 8H), 1.82-1.71 (m, 2H), 1.67-1.52 (m, 10H), 1.48-1.20 (m, 50H). 0.88 (t, *J =* 7.0 Hz, 12H).

LC-MS (ESI): Calculated for 1100.82, Found (M+H): 1101.7.

### Example 71: Synthesis of Amino Lipid Compound 1854

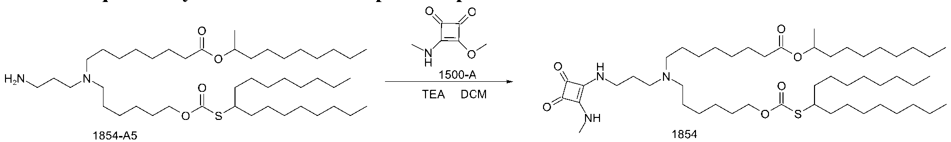

According to the general synthetic method, the amino lipid compound 1854 was synthesized according to the process of Step 7 of Example 52 with substituting 1854-A5 for 1720-A5, wherein 1854-A5 (2.0 g, 2.65 mmol), triethylamine (321 mg, 3.18 mmol), and 1500-A (448 mg, 3.18 mmol) were used, resulting in 998 mg of the amino lipid compound 1854 with a yield of 43.7% and a purity of 94.13%.

¹H NMR (600 MHz, CDCl₃) δ 4.92-4.84 (m, 1H), 4.18 (t, *J =* 6.7 Hz, 2H), 3.65 (bs, 2H), 3.36-3.19 (m, 4H), 2.53 (t, *J =* 6.1 Hz, 2H), 2.41 (m, 4H), 2.27 (t, *J =* 7.5 Hz, 2H), 1.87 (bs, 2H), 1.79-1.70 (m, 2H), 1.69-1.51 (m, 10H), 1.50-1.21 (m, 48H), 1.19 (d, *J =* 6.3 Hz, 3H), 0.87 (t, *J =* 7.0 Hz, 9H).

LC-MS (ESI): Calculated for 864.37, Found (M+H): 865.0.

### Example 72: Synthesis of Amino Lipid Comnound 1855

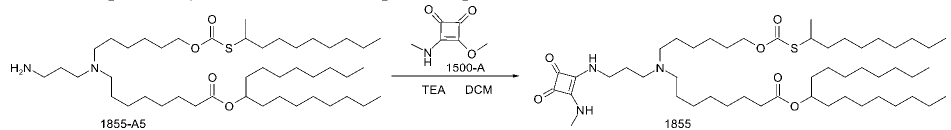

According to the general synthetic method, the amino lipid compound 1855 was synthesized according to the process of Step 7 of Example 52 with substituting 1855-A5 for 1720-A5, wherein 1855-A5 (2.0 g, 2.65 mmol), triethylamine (321 mg, 3.18 mmol), and 1500-A (448 mg, 3.18 mmol) were used, resulting in 923 mg of the amino lipid compound 1855 with a yield of 40.3% and a purity of 91.09%.

¹H NMR (600 MHz, CDCl₃) δ 4.90-4.79 (m, 1H), 4.24-4.14 (m, 2H), 3.65 (bs, 2H), 3.38 (m, 1H), 3.27 (d, *J =* 4.3 Hz, 3H), 2.54 (t, *J =* 6.0 Hz, 2H), 2.46-2.36 (m, 4H), 2.28 (t, *J* = 7.5 Hz, 2H), 1.85 (bs, 2H), 1.79-1.71 (m, 2H), 1.69-1.46 (m, 10H), 1.46-1.22 (m, 51H), 0.87 (t, *J* = 7.0 Hz, 9H) .

LC-MS (ESI): Calculated for 864.37, Found (M+H): 865.0.

### Example 73: Synthesis of Amino Lipid Compound 1856

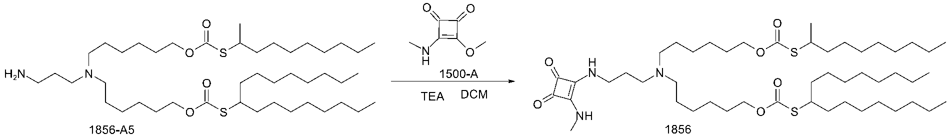

According to the general synthetic method, the amino lipid compound 1856 was synthesized according to the process of Step 7 of Example 52 with substituting 1856-A5 for 1720-A5, wherein 1856-A5 (2.0 g, 2.59 mmol), triethylamine (314 mg, 3.11 mmol), and 1500-A (438 mg, 3.11 mmol) were used, resulting in 948 mg of the amino lipid compound 1856 with a yield of 41.5% and a purity of 92.09%.

¹H NMR (600 MHz, CDCl₃) δ 4.20-4.18 (m, 4H), 3.64 (bs, 2H), 3.41-3.36 (m, 1H), 3.35-3.29 (m, 1H), 3.27 (d, J = 4.4 Hz, 3H), 2.55 (t, J = 5.8 Hz, 2H), 2.48-2.35 (m, 4H), 1.80-1.49 (m, 18H), 1.48-1.18 (m, 45H), 0.88 (t, J = 7.0 Hz, 9H).

LC-MS (ESI): Calculated for 882.4, Found (M+H): 863.0.

**Example 74: Synthesis of Amino Lipid Compound 1950**

### Step 1: Synthesis of 1950-A1

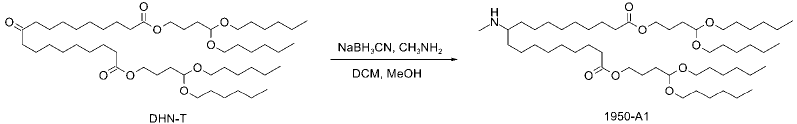

### Experimental procedure:

A solution of methylamine in methanol (5.71 g, 60.8 mmol) was added to a 250 mL single-necked flask, cooled to below 5°C, and slowly added dropwise with a solution of acetic acid in methanol (3.65 g acetic acid, 33 mL methanol). After the dropwise addition was completed, stirring was continued at room temperature (25°C) for 30 min, followed by addition of a solution of DHN-T in dichloromethane (6.5 g DHN-T, 7.60 mmol, 33 mL dichloromethane) and a final addition of sodium cyanoborohydride (0.48 g, 7.60 mmol), and reaction was carried out overnight. After the reaction stopped, water (80 mL) and dichloromethane (50 mL) were added for extraction twice, and the organic phases were combined. The organic phase was washed with saturated sodium chloride solution (80 mL). The solvent was removed by evaporation under reduced pressure at 45°C, obtaining 7.1 g of a coarse product of 1950-A1. The coarse product was purified by silica gel column chromatography and eluted with ethyl acetate:n-heptane = 1:1 to obtain 5.09 g 1950-A1 with a yield of 76.9%.

### Step 2: Synthesis of Amino Lipid Compound 1950

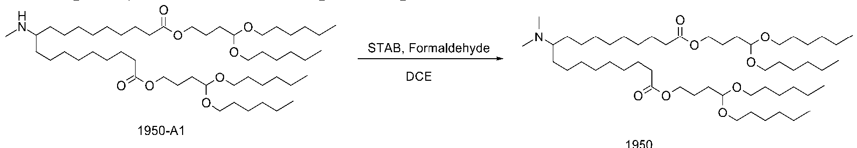

### Experimental procedure:

1950-A1 (2.0 g, 2.30 mmol), 1,2-dichloroethane (20 mL), aqueous formaldehyde solution (0.56 g), and sodium triacetoxyborohydride (1.46 g, 6.90 mmol) were sequentially added to a 250 mL round-bottom flask, and reacted at room temperature for 2 h. Water (50 mL) was added, and stirred. Phases were separated. The organic phase was collected. The water phase was extracted with dichloromethane (50 mL), and the organic phases were combined. After the organic phase was washed with saturated sodium chloride solution (80 mL), the solvent was removed by evaporation under reduced pressure at 45°C, obtaining 2.2 g of a coarse product of 1950. The coarse product was purified by silica gel column chromatography and eluted with ethyl acetate:n-heptane = 1:1 to obtain 1.3 g of the amino lipid compound 1950 with a yield of 63.91% and a purity of 94.66%.

¹H NMR (600 MHz, CDCl₃) δ 4.48 (t, *J* = 5.1 Hz, 2H), 4.08 (t, *J* = 6.0 Hz, 4H), 3.57 (dt, *J =* 9.1, 6.7 Hz, 4H), 3.41 (dt, *J =* 9.1, 6.7 Hz, 4H), 2.29 (t, *J* = 7.6 Hz, 4H), 2.25 (m, 1H), 2.22 (s, 6H), 1.73-1.65 (m, 8H), 1.58 (m, 12H), 1.38-1.24 (m, 48H), 0.89 (t, *J =* 6.9 Hz, 12H).

LC-MS (ESI): Calculated for 884.42, Found (M+H): 885.1.

### Example 75: Synthesis of Amino Lipid Compound 2047

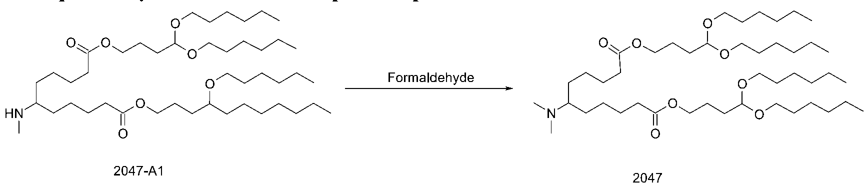

According to the general synthetic method, the amino lipid compound 2047 was synthesized according to the process of Step 2 of Example 74 with substituting 2047-A1 for 1950-A1, wherein 2047-A1 (1.5 g, 1.98 mmol) and 37% aqueous formaldehyde solution (0.48 g, 5.95 mmol) were used, resulting in 0.41 g of the amino lipid compound 2047 with a yield of 26.82% and a purity of 95.65%.

¹H NMR (600 MHz, CDCl₃) δ 4.48 (t, *J* = 5.3 Hz, 2H), 4.08 (t, *J* = 6.2 Hz, 4H), 3.57 (dt, *J* = 9.3, 6.7 Hz, 4H), 3.41 (dt, *J* = 9.3, 6.7 Hz, 4H), 2.32-2.28 (m, 4H), 2.28-2.23 (m, 1H), 2.20 (s, 6H), 1.74-1.65 (m, 12H), 1.63-1.50 (m, 12H), 1.37-1.24 (m, 28H), 0.88 *(t, J=* 6.9 Hz, 12H).

LC-MS (ESI): Calculated for 772.21, Found (M+H): 772.8.

### Example 76: Synthesis of Amino Lipid Compound 2048

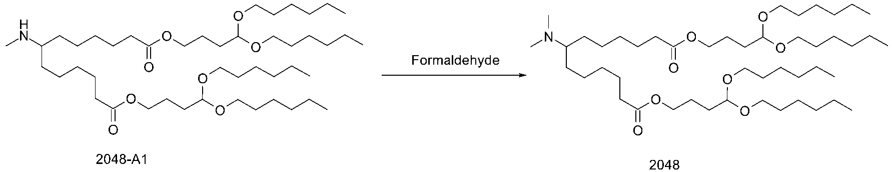

According to the general synthetic method, the amino lipid compound 2048 was synthesized according to the process of Step 2 of Example 74 with substituting 2048-A1 for 1950-A1, wherein 2048-A1 (1.5 g, 1.91 mmol) and 37% aqueous formaldehyde solution (0.46 g, 5.72 mmol) were used, resulting in 679 mg of the amino lipid compound 2048 with a yield of 44.42% and a purity of 91.99%.

¹H NMR (600 MHz, CDCl₃) δ 4.48 (t, *J* = 5.2 Hz, 2H), 4.08 (t, *J* = 6.1 Hz, 4H), 3.57 (dt, *J* = 9.3, 6.7 Hz, 4H), 3.41 (dt, *J =* 9.3, 6.7 Hz, 4H), 2.29 (t, *J* = 7.6 Hz, 4H), 2.23 (m, 1H), 2.20 (s, 6H), 1.73-1.60 (m, 16H), 1.59-1.53 (m, 8H), 1.35-1.24 (m, 32H), 0.89 (t, *J* = 6.9 Hz, 12H).

LC-MS (ESI): Calculated for 800.26, Found (M+H): 800.9

### Example 77: Synthesis of Amino Lipid Compound 2049

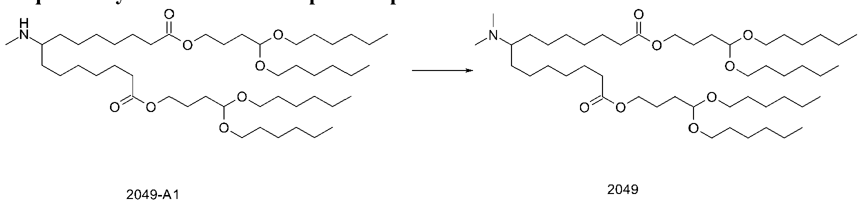

According to the general synthetic method, the amino lipid compound 2049 was synthesized according to the process of Step 2 of Example 74 with substituting 2049-A1 for 1950-A1, wherein 2049-A1 (1.4 g, 1.72 mmol) and 37% aqueous formaldehyde solution (0.425 g, 5.16 mmol) were used, resulting in 761 mg of the amino lipid compound 2049 with a yield of 53.42% and a purity of 91.95%.

¹H NMR (600 MHz, CDCl₃) δ 4.48 (t, J = 5.2 Hz, 2H), 4.07 (t, J = 6.1 Hz, 4H), 3.56 (dt, J = 9.2, 6.7 Hz, 4H), 3.40 (dt, J = 9.2, 6.7 Hz, 4H), 2.28 (t, J = 7.5 Hz, 11H), 1.79-1.64 (m, 6H), 1.59-1.51 (m, 14H), 1.40-1.24 (m, 40H), 0.88 (t, J = 6.9 Hz,12H).

LC-MS (ESI): Calculated for 828.31, Found (M+H): 829.0.

### Example 78: Synthesis of Amino Lipid Compound 2050

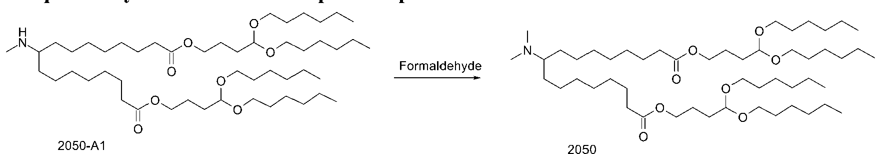

According to the general synthetic method, the amino lipid compound 2050 was synthesized according to the process of Step 2 of Example 74 with substituting 2050-A1 for 1950-A1, wherein 2050-A1 (2.0 g, 2.37 mmol) and 37% aqueous formaldehyde solution (0.58 g, 7.12 mmol) were used, resulting in 1150 mg of the amino lipid compound 2050 with a yield of 56.66% and a purity of 90.22%.

¹H NMR (600 MHz, CDCl₃) δ 4.48 (t, *J* = 5.3 Hz, 2H), 4.08 (t, *J* = 6.2 Hz, 4H), 3.57 (dt, *J* = 9.3, 6.7 Hz, 4H), 3.41 (dt, *J =* 9.3, 6.7 Hz, 4H), 2.29 (t, *J* = 7.6 Hz, 4H), 2.23 (m, 1H), 2.20 (s, 6H), 1.73-1.65 (m, 8H), 1.64-1.53 (m, 12H), 1.36-1.25 (m, 44H), 0.89 (t, *J* = 7.0 Hz, 12H).

LC-MS (ESI): Calculated for 856.37, Found (M+H): 857.0.

### Experimental Example 1: Preparation of Lipid Nanoparticle Encapsulating Luciferase mRNA (Fluc mRNA)

(1) Formulating:
   A specified amount of Fluc mRNA stock solution, 0.2 M sodium acetate buffer, and DEPC water were added to a container, and mixed well to obtain a water phase;
   The amino lipid compound of the present disclosure, a helper lipid (DSPC), a structural lipid (cholesterol), and a PEG-lipid were separately dissolved in absolute ethanol to prepare respective solutions at concentrations of 20 mg/mL, 10 mg/mL, 20 mg/mL, and 25 mg/mL, respectively. The above four solutions were pipetted at a molar ratio of Lipid: DSPC: CHO-HP: M-DMG-2000 of 48:10:40.5:1.5, and mixed well to prepare an alcohol phase.
(2) Encapsulation: The water phase and the alcohol phase were aspirate into water phase and alcoholic phase syringes at a flow rate of water phase: alcohol phase = 9 mL/min: 3 mL/min by using a microfluidic preparation instrument (MPE-L2), and encapsulation was carried out at a flow rate of water phase: alcohol phase = 12 mL/min: 4 mL/min to obtain mRNA-encapsulating lipid nanoparticle (mRNA-LNP).
(3) Dialysis: The product of step (2) was loaded in a dialysis bag and placed in a Tris Buffer-8% (m/V) sucrose solution for replacement to remove ingredients such as residual ethanol, unassembled lipids. Dialysis was conducted for 2 h with magnetic stirring at room temperature and under protection from light (dialysate was replaced every 1 hour).
(4) The product of step (3) was sterilized by passing through a 0.22 µm microporous membrane, and then packaged.

A variety of lipid nanoparticle formulations (LNP formulations) encapsulating Fluc mRNA were prepared, wherein the amino lipid compounds contained in the variety of LNP formulations were different from each other, and each LNP formulation had a Fluc mRNA concentration of 0.2 µg/µL, a mass ratio of Fluc mRNA to Lipid of 1:10, a particle size of 80 nm-130 nm, and an encapsulation efficiency of 85% or higher.

### Experimental Example 2: Performance Evaluation of in vivo Delivery of Lipid Nanoparticle

Animal preparation: Female BALB/c mice of 6-8 weeks old were selected and raised in an SPF grade breeding room. Animal testing was conducted in strict accordance with the guidelines of national health institutions and animal ethics requirements.

*In vivo* Delivery: prior to injection of the test LNP formulation, the LNP formulation was gently and repeatedly inverted to thoroughly mix the sample of the formulation. A corresponding amount of the LNP formulation was aspirated with a 1 mL insulin syringe, and the LNP formulation was injected by tail vein injection (IV), with 3 mice in duplicate per formulation. Each mouse was injected with the corresponding LNP formulation encapsulating the luciferase mRNA (Fluc mRNA) prepared in Experimental Example 1 at an injection volume of 70 µL, 50 µL or 40 µL.

6 hours after injection of LNP formulations, mice were injected with 200 µL D-Luciferin luciferase developing substrate (Catalog No.122799; Manufacturer: Perkin Elmer). After the substrate was injected, the mice were anesthetized with isoflurane inhalation, and the injection time of luciferase developing substrate was recorded. 10 minutes after the substrate injection, the animals were placed in supine position, and the signal distribution and expression intensity of luciferase in the body and various organs of the animals were observed with In Vivo Imaging System (IVIS).

The intensity of fluorescence expression induced by the lipid nanoparticle encapsulating luciferase mRNA (Fluc mRNA) with a representative amino lipid compound is shown in Table 3, with the LNP formulation containing 030 or 1500 as a control LNP formulation. The preparation method of the control LNP formulation was the same as that of the LNP formulations in Experimental Example 1, with the difference lying in that the amino lipid compounds of the present disclosure were replaced with 030 or 1500 in the preparation of the control LNP formulation. In Table 3, the unit of average photon number is p/s/cm²/sr.

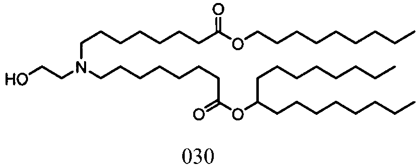

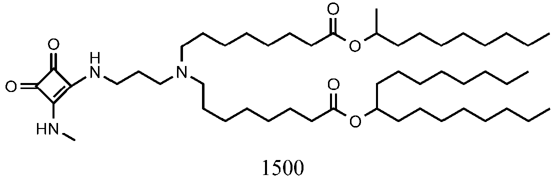

**Table 3**

| Amino lipid compounds | Volume injected (µL) | Average number of photons (Total *in-vivo* flux) | Average number of photons (Liver) | Average number of photons (Spleen) |
|---|---|---|---|---|
| 1501 | 70 | 1.04E+07 | 2.12E+06 | 3.22E+06 |
| 1502 | 70 | 4.55E+07 | 8.70E+06 | 1.16E+07 |
| 1503 | 70 | 1.93E+08 | 3.11E+07 | 1.04E+07 |
| 1504 | 70 | 2.17E+07 | 4.47E+06 | 8.59E+06 |
| 1512 | 70 | 1.82E+08 | 2.69E+07 | 1.20E+07 |
| 1525 | 70 | 3.22E+07 | 7.33E+06 | 4.06E+06 |
| 1526 | 70 | 3.83E+07 | 7.95E+06 | 3.25E+07 |
| 1532 | 70 | 3.96E+07 | 1.23E+07 | 4.21E+06 |
| 1640 | 70 | 8.16E+07 | 2.40E+07 | 6.15E+06 |
| 1643 | 70 | 4.20E+08 | 1.17E+08 | 1.90E+07 |
| 1644 | 70 | 5.82E+08 | 9.97E+07 | 7.42E+06 |
| 1720 | 70 | 1.61E+07 | 3.23E+06 | 2.06E+06 |
| 1721 | 70 | 1.69E+07 | 3.48E+06 | 1.75E+06 |
| 1726 | 70 | 1.83E+07 | 3.81E+06 | 5.12E+06 |
| 1727 | 70 | 1.04E+07 | 2.48E+06 | 1.15E+06 |
| 1746 | 70 | 6.99E+08 | 1.34E+08 | 5.37E+06 |
| 1747 | 70 | 5.83E+08 | 1.93E+08 | 3.56E+06 |
| 1748 | 70 | 2.09E+07 | 6.51E+06 | 6.89E+06 |
| 1749 | 70 | 1.04E+07 | 2.53E+06 | 6.42E+05 |
| 1807 | 70 | 9.77E+07 | 1.94E+07 | 5.70E+06 |
| 1831 | 70 | 1.77E+08 | 4.13E+07 | 2.33E+06 |
| 1832 | 70 | 1.39E+07 | 6.89E+06 | 7.87E+05 |
| 1833 | 70 | 3.88E+07 | 1.42E+07 | 1.50E+06 |
| 1834 | 70 | 6.48E+07 | 2.49E+07 | 9.77E+05 |
| 1843 | 70 | 9.00E+07 | 2.02E+07 | 3.47E+06 |
| 1845 | 70 | 1.27E+07 | 5.57E+06 | 4.88E+06 |
| 1847 | 70 | 1.91E+07 | 5.53E+06 | 1.27E+06 |
| 1849 | 70 | 1.30E+07 | 3.64E+06 | 1.60E+06 |
| 1854 | 70 | 4.55E+08 | 9.43E+07 | 1.75E+07 |
| 1855 | 70 | 2.12E+08 | 3.14E+06 | 4.70E+05 |
| 1856 | 70 | 2.31E+08 | 4.49E+07 | 7.20E+06 |
| 1873 | 70 | 3.18E+08 | 9.82E+07 | 4.50E+06 |
| 1844 | 50 | 5.13E+07 | 1.38E+07 | 6.36E+06 |
| 1874 | 50 | 2.42E+08 | 7.03E+07 | 6.51E+06 |
| 1875 | 50 | 6.80E+07 | 2.50E+07 | 9.22E+05 |
| 1880 | 50 | 1.91E+07 | 6.10E+06 | 6.52E+05 |
| 1881 | 50 | 2.38E+07 | 5.43E+06 | 1.57E+06 |
| 1928 | 50 | 4.10E+07 | 7.71E+06 | 3.10E+06 |
| 1929 | 50 | 1.04E+08 | 1.73E+07 | 3.10E+06 |
| 1930 | 50 | 2.02E+08 | 3.57E+07 | 6.67E+06 |
| 1931 | 50 | 1.58E+08 | 3.31E+07 | 2.68E+06 |
| 1950 | 50 | 1.22E+08 | 2.64E+07 | 1.96E+05 |
| 2047 | 50 | 9.03E+07 | 1.96E+07 | 1.06E+06 |
| 2048 | 50 | 4.13E+08 | 8.55E+07 | 4.62E+06 |
| 2050 | 50 | 3.00E+08 | 6.30E+07 | 2.74E+06 |
| 1912 | 40 | 7.52E+07 | 2.08E+07 | 1.06E+07 |
| 1913 | 40 | 5.27E+07 | 1.68E+07 | 6.07E+06 |
| 1924 | 40 | 1.29E+08 | 4.84E+07 | 1.79E+07 |
| 1925 | 40 | 8.98E+07 | 3.05E+07 | 7.38E+06 |
| 1926 | 40 | 5.72E+07 | 1.46E+07 | 4.36E+06 |
| 1927 | 40 | 7.59E+07 | 2.37E+07 | 7.00E+06 |
| 2060 | 70 | 4.67E+07 | 5.61E+06 | 3.50E+05 |
| 1500 | 70 | 1.48E+07 | 2.17E+06 | 7.30E+06 |
| 030 | 70 | 4.89E+08 | 1.29E+08 | 2.73E+07 |
| 030 | 50 | 1.21E+08 | 3.27E+07 | 6.08E+06 |
| 030 | 40 | 4.33E+07 | 1.20E+07 | 4.93E+06 |

### Experimental Example 3: Evaluation of in vivo Safety of Lipid Nanoparticle

A variety of lipid nanoparticle formulations (LNP formulations) encapsulating human erythropoietin mRNA (hEPO mRNA) were prepared according to the method as described in Experimental Example 1, with replacing the luciferase mRNA (Fluc mRNA) with human erythropoietin mRNA (hEPO mRNA), with a concentration of human erythropoietin mRNA (hEPO mRNA) of 0.5 µg/µL, a mass ratio of hEPO mRNA to Lipid of 1:10, a particle size of 90 nm-130 nm, and an encapsulation efficiency of above 90% or higher for each LNP formulation.

Animal preparation: Female SD rats of 6-8 weeks old were selected and raised in an SPF grade breeding room. Animal testing was conducted in strict accordance with the guidelines of national health institutions and animal ethics requirements.

*In vivo* Delivery: Prior to injection of the test LNP formulations, the LNP formulations were gently and repeatedly inverted to thoroughly mix the formulation samples. A corresponding amount of the formulation samples were aspirated with a 2 ml syringe, and the LNP formulations were injected by tail vein injection (IV), with 4 rats per formulation. Each rat was injected with (1200 µL, 3mpk) of the human erythropoietin mRNA (hEPO mRNA)-encapsulating LNP formulation. Tris buffer was used as a blank control.

Serum acquisition: Blood samples of rats were collected 12 h after injection, placed in tubes without anticoagulants, and naturally coagulated at room temperature for 30 min-60 min, and then centrifuged at a speed of 3500 rpm for 10 min to obtain the supernatant, which was the serum.

The detection of alanine transaminase was carried out according to instructions of the kit (Nanjing Jiancheng Bioengineering Institute, Catalog. No. C009-2-1), and a standard curve was made using the standard provided in the kit. D-PBS was used in the experiment, which was purchased from Sangon Biotech (Shanghai) Co., Ltd., Catalog No. E607009-0600.

**The method of detection of enzyme activity of alanine aminotransferase (ALT) in serum of the mice is as follows:**

### Preparation of ALT standard curve:

(1) Enzymatic reaction: 0, 2, 4, 6, 8 and 10 µL of 2 µmol/mL sodium pyruvate standard solution were sequentially added to 5 µL of 0.1 mol/L phosphate buffer, and the volume was supplemented to 25 µL with a ALT matrix solution (alanine aminotransferase matrix solution), and repeatedly aspirating and spitting with a pipette for mixing well;
(2) Addition reaction: 20 µL 2,4-dinitrophenylhydrazine solution was added to all reaction wells in (1), mixed by aspirating and spitting, and then placed in an incubator at 37°C to react for 20 min;
(3) Developing: 200 µL of 0.4 mol/L NaOH solution was added to all reaction wells in (2) to stop the reaction, mixed by aspirating and spitting, and incubated at room temperature for 15 min. The OD value of each well was measured at 510 nm in a microplate reader; and
(4) Data processing of standard curve: The corresponding absolute OD value for each well was obtained by subtracting the OD value for the well with 0 µL sodium pyruvate from the measured OD value for each well, the corresponding ALT Karmen units being 0, 28, 57, 97, 150 and 200, respectively. The standard curve was obtain by taking the absolute OD value as the abscissa and the corresponding Karmen unit as the ordinate.

### Detection of ALT enzyme activity in serum samples:

(1) Reagent preparation: an ALT matrix solution was placed in an incubator at 37°C for preheating;
(2) Enzymatic reaction: 5 µL diluted serum was aspirated and added to a 96-well plate, then 20 µL matrix solution was added to the corresponding sample well and mixed by repeatedly aspirating and spitting to avoid bubbling, and then placed in an incubator at 37°C for 30 min;
(3) Addition reaction: 20 µL 2,4-dinitrophenylhydrazine was added to all reaction wells in (2), mixed by aspirating and spitting, and then reacted in an incubator at 37°C for 20 min;
(4) Developing: 200 µL of 0.4 mol/L NaOH solution was added to all reaction wells in (3) to stop the reaction, mixed by aspirating and spitting, and then incubated at room temperature for 15 min. The OD value for each well was measured at a wavelength of 510 nm in a microplate reader; and
(5) Calculation of ALT enzyme activity in serum: The absolute OD value of the corresponding sample well was obtained by subtracting the OD value for the control well (sample well without biochemical reaction) from the obtained OD value of the sample well, and was substituted into the standard curve formula to calculate the ALT enzyme activity (Karmen unit) for the corresponding serum sample. The Karman unit was converted into an activity unit (1 Karmen unit = 0.482 U/L).

**The method of detection of enzyme activity of aspartate aminotransferase (AST) in serum of the mice is as follows:**

### Preparation of AST standard curve:

(1) Enzymatic reaction: 0, 2, 4, 6, and 8 µL of 2 µmol/mL sodium pyruvate standard solution were sequentially added to 5 µL of 0.1 mol/L phosphate buffer, and the volume was supplemented to 25 µL with a AST matrix solution (aspartate aminotransferase matrix solution), and repeatedly aspirating and spitting with a pipette for mixing well;
(2) Addition reaction: 20 µL 2,4-dinitrophenylhydrazine solution was added to all reaction wells in (1), mixed by aspirating and spitting, and then placed in an incubator at 37°C to react for 20 min;
(3) Developing: 200 µL of 0.4 mol/L NaOH solution was added to all reaction wells in (2) to stop the reaction, mixed by aspirating and spitting, and incubated at room temperature for 15 min. The OD value of each well was measured at 510 nm in a microplate reader; and
(4) Data processing of standard curve: The corresponding absolute OD value for each well was obtained by subtracting the OD value for the well with 0 µL sodium pyruvate from the measured OD value for each well, the corresponding AST Karmen units being 0, 24, 61, 114 and 190, respectively. The standard curve was obtain by taking the absolute OD value as the abscissa and the corresponding Karmen unit as the ordinate.

### Detection of AST enzyme activity in serum samples:

(1) Reagent preparation: an AST matrix solution was placed in an incubator at 37°C for preheating;
(2) Enzymatic reaction: 5 µL diluted serum was aspirated and added to a 96-well plate, then 20 µL matrix solution was added to the corresponding sample well and mixed by repeatedly aspirating and spitting to avoid bubbling, and then placed in an incubator at 37°C for 30 min;
(3) Addition reaction: 20 µL 2,4-dinitrophenylhydrazine was added to all reaction wells in (2), mixed by aspirating and spitting, and then reacted in an incubator at 37°C for 20 min;
(4) Developing: 200 µL of 0.4 mol/L NaOH solution was added to all reaction wells in (3) to stop the reaction, mixed by aspirating and spitting, and then incubated at room temperature for 15 min. The OD value for each well was measured at a wavelength of 510 nm in a microplate reader; and
(5) Calculation of AST enzyme activity in serum: The absolute OD value of the corresponding sample well was obtained by subtracting the OD value for the control well (sample well without biochemical reaction) from the obtained OD value of the sample well, and was substituted into the standard curve formula to calculate the AST enzyme activity (Karmen unit) for the corresponding serum sample. The Karman unit was converted into an activity unit (1 Karmen unit = 0.482 U/L).

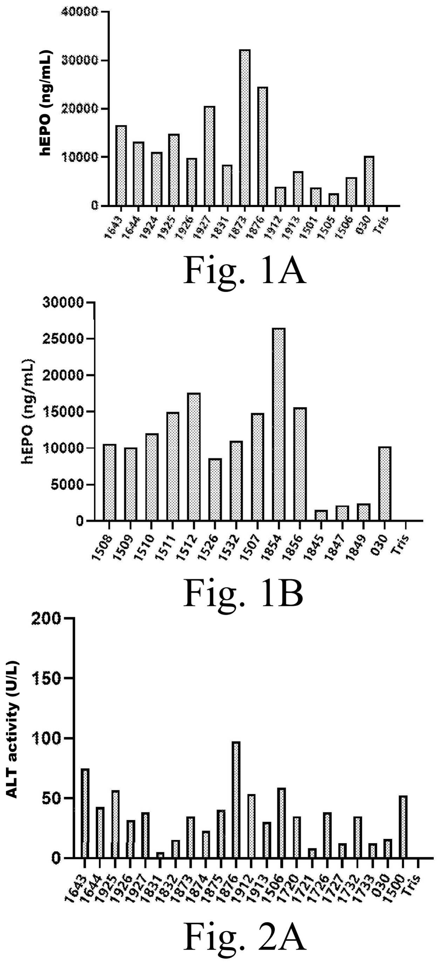

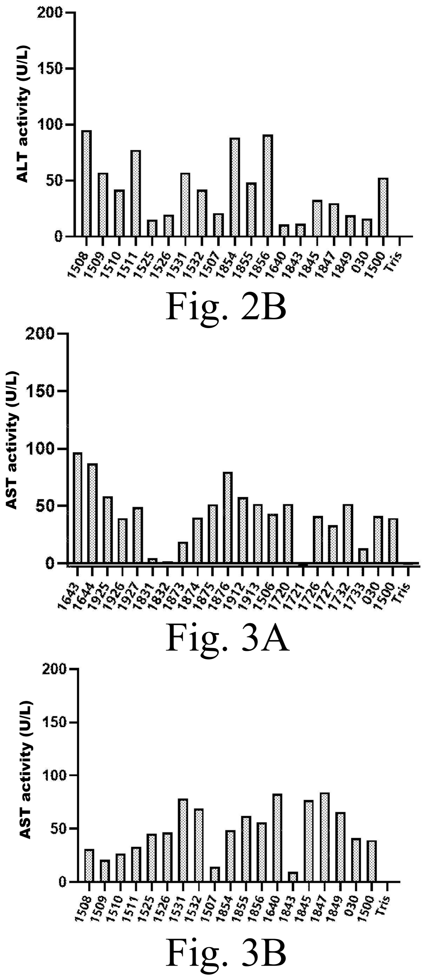

The *in vivo* delivery and safety evaluation results of the lipid nanoparticles encapsulating human erythropoietin (hEPO) mRNA with the representative amino lipid compounds are shown in FIG. 1A to FIG. 3B, with Tris, 1500 and 030 as controls. "Tris" refers to the control group injected with Tris-sucrose buffer solution only.

As can be seen from FIG. 1A to FIG. 3B, the lipid nanoparticles containing the representative amino lipid compounds exhibit lower liver toxicity.

### Experimental Example 4: Evaluation of the delivery efficiency of lipid nanoparticles to immune cell populations in spleen

According to the method described in Experimental Example 1, eGFP mRNA encapsulating LNPs were prepared. C57BL/6 mice (3 mice per group) were each intravenously injected with 100µL (containing 20 µg mRNA, 1mpk) of the eGFP mRNA encapsulating LNPs. After 12 hours, the mice were dissected, and the spleens were collected to prepare single-cell suspensions. After blocking with Fc blocking antibodies and surface staining with fluorescent-labeled antibodies, flow cytometry was conducted to analyze the expression levels of eGFP in various immune cell populations to confirm the delivery efficiency of lipid nanoparticles comprising different amino lipid compounds to immune cell populations in spleen.

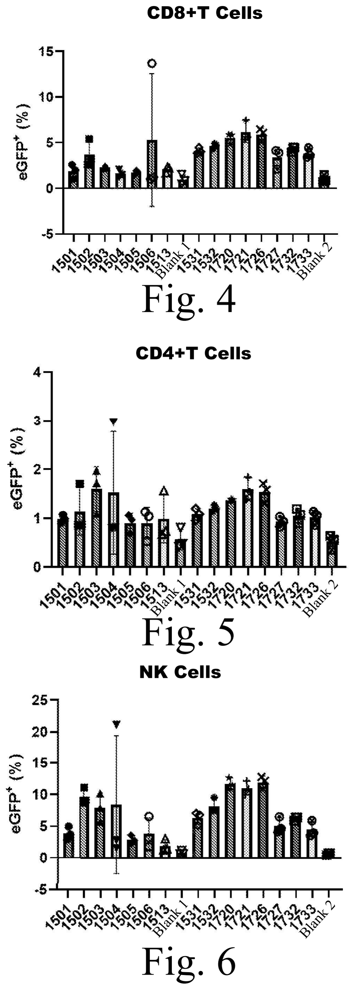

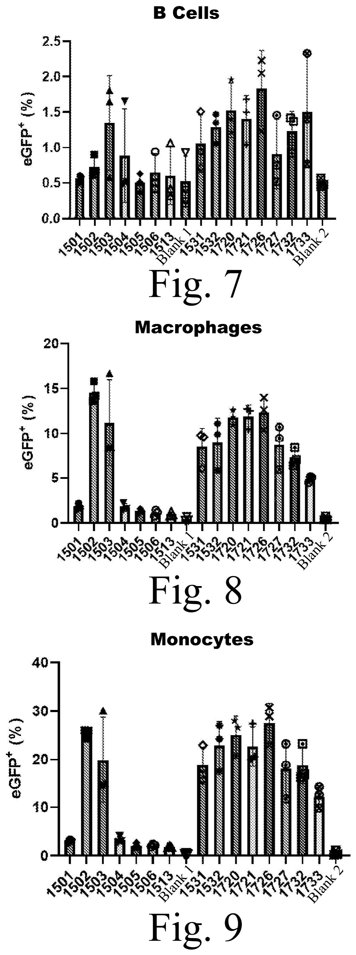

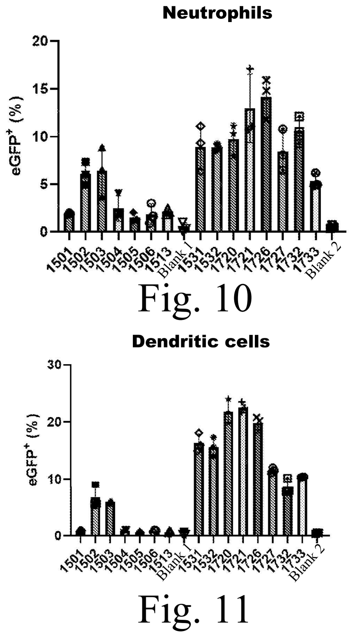

The test results are shown in FIG. 4 to FIG. 11. "Blank 1" and "Blank 2" in FIG. 4 and FIG. 11 both refer to the blank LNP groups injected with 100 µL LNP containing no eGFP mRNA, wherein the amino lipid compound in the blank LNP groups was ALC-0315.

As can be seen from FIG. 6 to FIG. 7, the lipid nanoparticles comprising different amino lipid compounds exhibited differences in the delivery to immune cells, in which the lipid nanoparticles containing representative amino lipid compounds had a delivery preference to immune cells.

In addition to those described in this disclosure, various modifications to the present invention will be apparent to those skilled in the art from the foregoing description. Such modifications are also intended to fall within the scope of the appended claims.

## Claims

1. An amino lipid compound having a structure of formula (IA): 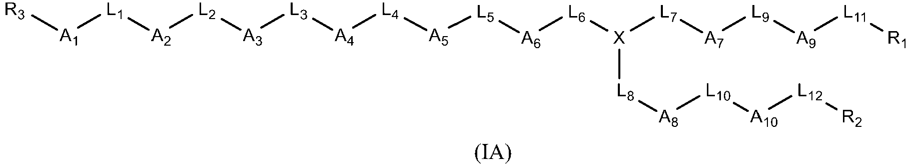 or a pharmaceutically acceptable salt thereof, or a stereoisomer thereof,
wherein:
X is C, N, or CR₁₃;
L₁, L₂, L₃, L₄, L₅, and L₆ are each independently C₁-C₆ hydrocarbylene, C₃-C₈ carbocyclic ring, heterocyclic ring, C₁-C₆ heterohydrocarbylene, or a bond;
L₇, L₈, L₉, L₁₀, L₁₁, and L₁₂ are each independently C₁-C₁₈ hydrocarbylene, C₁-C₁₈ heterohydrocarbylene, or a bond;
A₁, A₂, A₃, A₄, A₅, A₆, A₇, A₈, A₉, and A₁₀ are each independently -N(R₄)-, -C(=O)O-, -OC(=O)-, - OC(=O)O-, -O-, -C(=O)-, -S-, -C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, -N(R₄)C(=O)O-, -N(R₄)C(=O)N(R₄)-, -SC(=O)N(R₄)-, -N(R₄)C(=O)S-, - N(C(=O)L₁₃OR₄)-, -N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, -OP(=O)(OH)O-, -OP(=O)(H)O-, -OS(=O)₂NH-, -NHS(=O)₂O-, -OC(=O)C(=O)O-, - N(R₄)C(=O)C(=O)O-, -OC(=O)C(=O)N(R₄)-, -N(R₄)C(=O)C(=O)N(R₄)-, 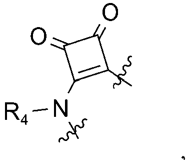 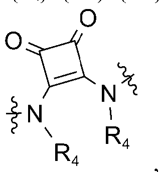 or a bond;
each L₁₃ is independently C₁-C₈ hydrocarbylene, or C₁-C₈ heterohydrocarbylene, or a bond;
R₁ and R₂ are each independently H, C₁-C₂₄ hydrocarbyl, C₁-C₂₄ heterohydrocarbyl, A₁₁R₅, - C(R₆)(OL₁₄A₁₂R₇)₂, -C(R₆)(SL₁₄A₁₂R₇)₂, or -C(R₆)(SL₁₄A₁₂R₇)(OL₁₄A₁₂R₇);
each A₁₁ is independently -C(=O)O-, -OC(=O)-, -OC(=O)O-, -O-, -C(=O)-, -S-, -C(=O)S-, -SC(=O)-, - C(=O)N(R₄)-, -N(R₄)C(=O)-, -OC(=O)N(R₄)-, -N(R₄)C(=O)O-, -N(R₄)C(=O)N(R₄)-, -OC(=O)S-, - SC(=O)O-, -SC(=O)S-, -SC(=O)N(R₄)-, -N(R₄)C(=O)S-, -N(C(=O)L₁₃OR₄)-, -N(C(=O)L₁₃SR₄)-, - N(C(=O)R₄), -N(L₁₃OR₄)-, -N(L₁₃SR₄)-, -N(R₄)-, -OS(=O)O-, -OS(=O)₂O-, -OP(=O)O-, -OP(=O)(OH)O-, - OP(=O)(H)O-, -OS(=O)₂NH-, -NHS(=O)₂O-, or a benzene ring;
each A₁₂ is independently -C(=O)O-, -OC(=O)-, -OC(=O)O-, -O-, -C(=O)-, -S-, -C(=O)S-, -SC(=O)-, - C(=O)N(R₄)-, -N(R₄)C(=O)-, -OC(=O)N(R₄)-, -N(R₄)C(=O)O-, -N(R₄)C(=O)N(R₄)-, -OC(=O)S-, - SC(=O)O-, -SC(=O)S-, -SC(=O)N(R₄)-, -N(R₄)C(=O)S-, -N(C(=O)L₁₃OR₄)-, -N(C(=O)L₁₃SR₄)-, - N(C(=O)R₄), -N(L₁₃OR₄)-, -N(L₁₃SR₄)-, -N(R₄)-, -OS(=O)O-, -OS(=O)₂O-, -OP(=O)O-, -OP(=O)(OH)O-, - OP(=O)(H)O-, -OS(=O)₂NH-, -NHS(=O)₂O-, a benzene ring, or a bond;
each R₄ is independently H, C₁-C₆ hydrocarbyl, or C₁-C₆ heterohydrocarbyl;
each R₅ is independently H, C₁-C₂₄ hydrocarbyl, or C₁-C₂₄ heterohydrocarbyl;
each R₆ is independently H, C₁-C₆ hydrocarbyl, or C₁-C₆ heterohydrocarbyl;
each L₁₄ is independently C₁-C₁₈ hydrocarbylene, C₁-C₁₈ heterohydrocarbylene, or a bond;
each R₇ is independently C₁-C₂₄ hydrocarbyl or C₁-C₂₄ heterohydrocarbyl;
R₃ is -L₁₅-Z;
L₁₅ is C₁-C₁₂ hydrocarbylene, C₁-C₁₂ heterohydrocarbylene, or a bond;
Z is H, a carbocyclic ring, a heterocyclic ring, -CN, -OR₈, -OL₁₆N(R₈)₂, -C(=O)OR₈, -C(=O)R₈, - C(=O)SR₈, -OC(=O)R₈, -OC(=O)OR₈, -OL₁₆OR₈, -N(R₈)₂, -C(=O)N(R₈)₂, -C(=S)N(R₈)₂, -S(=O)₂R₈, - S(=O)₂N(R₈)₂, -OC(=O)N(R₈)₂, -C(=NRs)N(R₈)₂, -C(=NR₈)R₈, -C(=O)N(R₈)OR₈, -CH(R₈)N(R₈)C(=O)OR₈, -C(R₈)₃, -N(R₈)C(=O)R₈, -N(R₈)C(=O)OR₈, -N(R₈)S(=O)₂R₈, -N(R₈)C(=O)N(R₈)₂, -N(Rs)C(=S)N(R₈)₂, - N(R₈)C(=NR₈)N(R₈)₂, -N(R₈)C(=CHR₈)N(R₈)₂, -N(OR₈)C(=O)R₈, -N(OR₈)S(=O)₂R₈, -N(OR₈)C(=O)OR₈, -N(OR₈)C(=O)N(R₈)₂, -N(OR₈)C(=S)N(R₈)₂, -N(OR₈)C(NR₈)N(R₈)₂, -N(OR₈)C(=CHR₈)N(R₈)₂, - OP(=O)(OR₈)₂, -P(=O)(OR₈)₂, -C(=N-CN)N(R₈)₂, -C(=N-O-CH₃)N(R₈)₂, -C(=N-SO₂-NH₂)N(R₈)₂, - C(=CH-NO₂)N(R₈)₂, -C(=O)OR₁₁, -N(R₈)R₁₁, -N(R₈)S(=O)₂R₁₁, -N(R₈)C(=NR₁₂)N(R₈)₂, - N(R₈)C(=CHR₁₂)N(R₈)₂, -N(OR₈)C(=NR₁₂)N(R₈)₂, -N(OR₈)C(=CHR₁₂)N(R₈)₂, -C(=NR₁₂)N(R₈)₂, - C(=NR₁₂)R₈, or -C(R₈)N(R₈)₂C(=O)OR₈;
each R₈ is independently hydrogen, or C₁-C₁₂ alkyl, or C₂-C₁₂ alkenyl with 1, 2, 3, or more double bonds, or C₂-C₁₂ alkynyl with 1, 2, 3, or more triple bonds, or C₁-C₁₂ heteroalkyl, or C₂-C₁₂ heteroalkenyl with 1, 2, 3, or more double bonds, or C₂-C₁₂ heteroalkynyl with 1, 2, 3, or more triple bonds;
each R₁₁ is independently C₃₋₆ carbocyclic ring or heterocyclic ring;
each R₁₂ is independently H, CN, NO₂, C₁₋₆ alkyl, -OR, -S(O)₂R, -S(O)₂N(R)₂, C₂₋₆ alkenyl, C₃₋₆ carbocyclic ring or heterocyclic ring;
each L₁₆ is independently C₁-C₁₈ hydrocarbylene, C₁-C₁₈ heterohydrocarbylene, or a bond; and
R₁₃ is H, C₁-C₆ hydrocarbyl, or C₁-C₆ heterohydrocarbyl containing O, N, or S.

2. The amino lipid compound according to claim 1, or a pharmaceutically acceptable salt thereof, or a stereoisomer thereof, wherein R₁ and R₂ are further each independently -C(R₆)(C(=O)OL₁₄A₁₂R₇)₂, - C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

3. The amino lipid compound according to claim 1 or 2, or a pharmaceutically acceptable salt thereof, or a stereoisomer thereof, wherein Z is 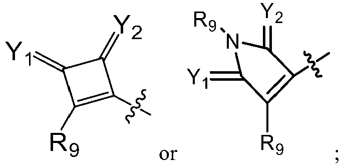
wherein Y₁ and Y₂ are each independently O or S;
each R₉ is independently H, halogen, -R_{b}, -N(R_{b})₂, -CN, -N₃, -C(=O)OR_{b}, -OC(=O)R_{b}, -OR_{b}, -SR_{b}, -S(-O)R_{b}, -S(=O)OR_{b}, -S(=O)₂OR_{b}, -N(R_{b})₂, -N(R_{b})S(=O)₂R_{b}, -NHRₐN(R_{b})₂, -NHRₐORₐN(R_{b})₂, -NHRₐOR_{b}, or -N(RₐOR_{b})₂;
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds; and
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds.

4. The amino lipid compound according to any one of claims 1 to 3, or a pharmaceutically acceptable salt thereof, or a stereoisomer thereof, wherein Z is

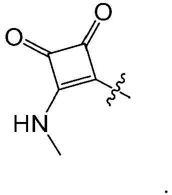

5. The amino lipid compound according to any one of claims 1 to 4, or a pharmaceutically acceptable salt thereof, or a stereoisomer thereof, wherein A₂ is -N(R₄)-, -C(=O)O-, -OC(=O)-, -OC(=O)O-, -O-, -C(=O)-, -S-, -C(=O)S-, -SC(=O)-, -C(=O)N(R₄)-, -N(R₄)C(=O)-, -OC(=O)S-, -SC(=O)O-, -SC(=O)S-, - OC(=O)N(R₄)-, -N(R₄)C(=O)O-, -N(R₄)C(=O)N(R₄)-, -SC(=O)N(R₄)-, -N(R₄)C(=O)S-, -N(C(=O)L₁₃OR₄)-, -N(C(=O)L₁₃SR₄)-, -N(C(=O)R₄), -N(L₁₃OR₄)-, -N(L₁₃SR₄)-, -OS(=O)O-, -OS(=O)₂O-, -OP(=O)O-, - OP(=O)(OH)O-, -OP(=O)(H)O-, -OS(=O)₂NH-, -NHS(=O)₂O-, -OC(=O)C(=O)O-, -N(R₄)C(=O)C(=O)O-, -OC(=O)C(=O)N(R₄)-, -N(R₄)C(=O)C(=O)N(R₄)-,

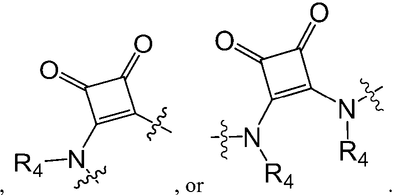

6. The amino lipid compound according to any one of claims 1 to 5, or a pharmaceutically acceptable salt thereof, or a stereoisomer thereof, wherein L₁₅ is a bond.

7. The amino lipid compound according to any one of claims 1 to 6, or a pharmaceutically acceptable salt thereof, or a stereoisomer thereof, wherein A₁ is -N(R₄)-.

8. The amino lipid compound according to any one of claims 1 to 7, or a pharmaceutically acceptable salt thereof, or a stereoisomer thereof, wherein A₁ is -NH-.

9. The amino lipid compound according to any one of claims 1 to 8, or a pharmaceutically acceptable salt thereof, or a stereoisomer thereof, wherein the amino lipid compound has a structure represented by formula (V-2):

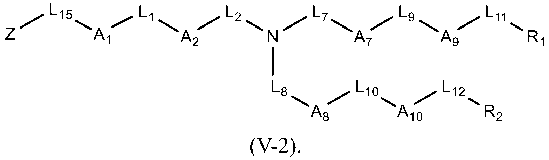

10. The amino lipid compound according to any one of claims 1 to 3, or a pharmaceutically acceptable salt thereof, or a stereoisomer thereof, wherein the amino lipid compound has a structure represented by formula (V-5): 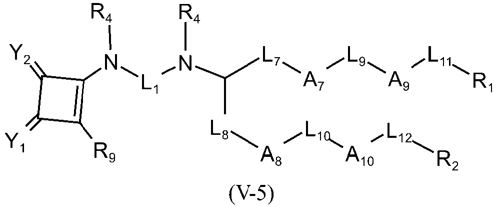 wherein R₁ and R₂ are each independently C₁-C₂₄ hydrocarbyl, -C(R₆)(OL₁₄A₁₂R₇)₂, - C(R₆)(SL₁₄A₁₂R₇)₂, -C(R₆)(SL₁₄A₁₂R₇)(OL₁₄Aₗ₁R₇), -C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

11. The amino lipid compound according to claim 10, or a pharmaceutically acceptable salt thereof, or a stereoisomer thereof, wherein the amino lipid compound has a structure represented by formula (V-5-1):

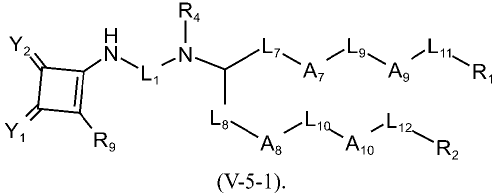

12. The amino lipid compound according to claim 10 or 11, or a pharmaceutically acceptable salt thereof, or a stereoisomer thereof, wherein:
Y₁ and Y₂ are each independently O or S;
R₉ is H, halogen, -R_{b}, -N(R_{b})₂, -CN, -N₃, -C(=O)OR_{b}, -OC(=O)R_{b}, -OR_{b}, -SR_{b}, -S(-O)R_{b}, -S(=O)OR_{b}, - S(=O)₂OR_{b}, -N(R_{b})S(=O)₂R_{b}, -NHRₐN(R_{b})₂, -NHRₐORₐN(R_{b})₂, -NHRₐOR_{b}, or -N(RₐOR_{b})₂;
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds;
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds;
L₁ is C₁-C₆ hydrocarbylene, C₃-C₈ carbocyclic ring, or C₁-C₆ heterohydrocarbylene;
L₇ and L₈ are each independently C₁-C₁₀ hydrocarbylene;
L₁₁ and L₁₂ are each independently C₁-C₅ hydrocarbylene or a bond;
A₇ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 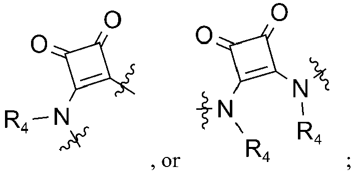
A₈ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 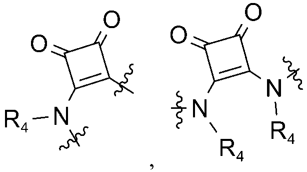 or a bond;
R₁ and R₂ are each independently C₁-C₂₄ alkyl, -C(R₆)(OR₇)₂, -C(R₆)(SR₇)₂, or -C(R₆)(SR₇)(OR₇);
each R₇ is independently C₁-C₁₂ hydrocarbyl; and
L₉, L₁₀, A₉, and A₁₀ are a bond.

13. The amino lipid compound according to any one of claims 1 to 12, or a pharmaceutically acceptable salt thereof, or a stereoisomer thereof, wherein L₉, L₁₀, Ag, and A₁₀ are a bond, A₇ and A₈ are each independently -C(=O)O- or -OC(=O)-, and R₁ and R₂ are -C(H)(OR₇)₂.

14. The amino lipid compound according to any one of claims 1 to 12, or a pharmaceutically acceptable salt thereof, or a stereoisomer thereof, wherein L₉, L₁₀, L₁₁, and L₁₂ are a bond, A₇ is -C(=O)O- or -OC(=O)-, A₈ is -C(=O)O- or -OC(=O)-, A₉ and A₁₀ are a bond, and R₁ and R₂ are each independently C₁-C₂₄ alkyl.

15. The amino lipid compound according to any one of claims 1 to 3, or a pharmaceutically acceptable salt thereof, or a stereoisomer thereof, wherein the amino lipid compound has a structure represented by formula (V-6): 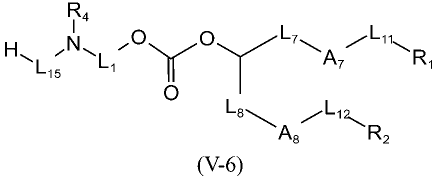 wherein R₁ and R₂ are each independently -C(R₆)(SL₁₄A₁₂R₇)₂, -C(R₆)(SL₁₄A₁₂R₇)(OL₁₄A₁₂R₇), - C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or - C(R₆)(OC(=O)L₁₄A₁₂R₇)R₇.

16. The amino lipid compound according to claim 15, or a pharmaceutically acceptable salt thereof, or a stereoisomer thereof, wherein:
L₁ is C₁-C₆ hydrocarbylene or C₁-C₆ heterohydrocarbylene;
L₁₅ is C₁-C₂ hydrocarbylene;
L₇ and L₈ are each independently C₁-C₁₀ hydrocarbylene;
L₁₁ and L₁₂ are each independently C₁-C₅ hydrocarbylene;
A₇ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 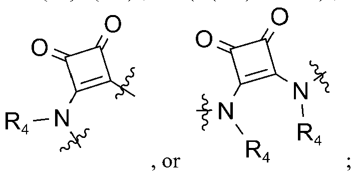
A₈ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 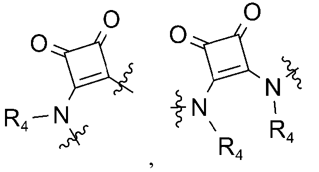 or a bond;
R₁ and R₂ are each independently -C(R₆)(C(=O)OR₇)₂, -C(R₆)(OC(=O)R₇)₂, -C(R₆)(C(=O)OR₇)R₇, or - C(R₆)(OC(=O)R₇)R₇; and
each R₇ is independently C₁-C₁₂ hydrocarbyl.

17. The amino lipid compound according to any one of claims 1 to 3, or a pharmaceutically acceptable salt thereof, or a stereoisomer thereof, wherein the amino lipid compound has a structure represented by formula (V-7) : 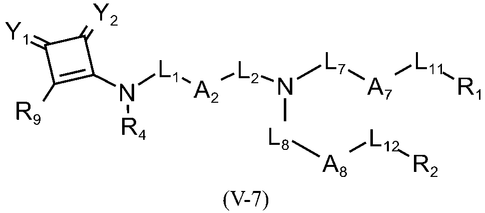 wherein:
Y₁ and Y₂ are each independently O or S;
each R₉ is independently H, halogen, -R_{b}, -N(R_{b})₂, -CN, -N₃, -C(=O)OR_{b}, -OC(=O)R_{b}, -OR_{b}, -SR_{b}, -S(-O)R_{b}, -S(=O)OR_{b}, -S(=O)₂OR_{b}, -N(R_{b})S(=O)₂R_{b}, -NHRₐN(R_{b})₂, -NHRₐORₐN(R_{b})₂, -NHRₐOR_{b}, or - N(RₐOR_{b})₂;
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds;
each R_{b} is independently H, C₁-C₁₂ alkyl, C₂-C₁₂ alkenyl, C₂-C₁₂ alkynyl, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds;
L₁ and L₂ are each independently C₁-C₆ hydrocarbylene, C₃-C₈ carbocyclic ring, or C₁-C₆ heterohydrocarbylene;
L₇ and L₈ are each independently C₁-C₁₀ hydrocarbylene;
L₁₁ and L₁₂ are each independently C₁-C₅ hydrocarbylene or a bond;
A₂ and A₇ are each independently -N(R₄)-, -C(=O)O-, -OC(=O)-, -OC(=O)O-, -O-, -C(=O)-, -S-, - C(=O)S-, -SC(=O)-, -C(=O)N(R₄)-, -N(R₄)C(=O)-, -OC(=O)S-, -SC(=O)O-, -SC(=O)S-, -OC(=O)N(R₄)-, - N(R₄)C(=O)O-, -N(R₄)C(=O)N(R₄)-, -SC(=O)N(R₄)-, -N(R₄)C(=O)S-, -N(C(=O)L₁₃OR₄)-, - N(C(=O)L₁₃SR₄)-, -N(C(=O)R₄), -N(Li₃OR₄)-, -N(L₁₃SR₄)-, -OS(=O)O-, -OS(=O)₂O-, -OP(=O)O-, - OP(=O)(OH)O-, -OP(=O)(H)O-, -OS(=O)₂NH-, -NHS(=O)₂O-, -OC(=O)C(=O)O-, -N(R₄)C(=O)C(=O)O-, -OC(=O)C(=O)N(R₄)-, -N(R₄)C(=O)C(=O)N(R₄)-, 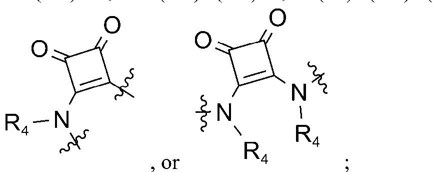
R₁ and R₂ are each independently C₁-C₂₄ hydrocarbyl, -C(R₆)(OL₁₄A₁₂R₇)₂, -C(R₆)(SL₁₄A₁₂R₇)₂, - C(R₆)(SL₁₄A₁₂R₇)(OL₁₄A₁₂R₇), -C(R₆)(C(=O)OL₁₄A₁₂R₇)₂, -C(R₆)(OC(=O)L₁₄A₁₂R₇)₂, - C(R₆)(C(=O)OL₁₄A₁₂R₇)R₇, or -C(R₆)(OC(=O)L₁₄A₁₂R₇)R₇; and
each R₇ is independently C₁-C₁₂ hydrocarbyl.

18. The amino lipid compound according to claim 17, or a pharmaceutically acceptable salt thereof, or a stereoisomer thereof, wherein the amino lipid compound has a structure represented by formula (V-7-1) or (V-7-2): 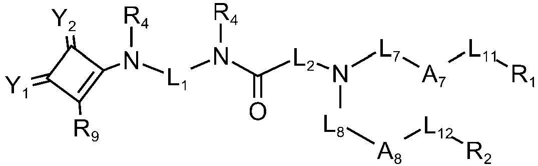 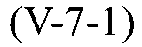 or 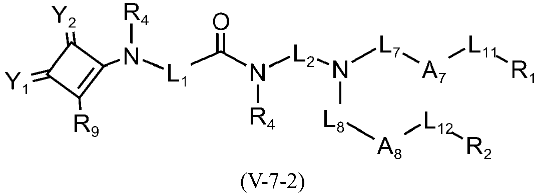 wherein:
A₇ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 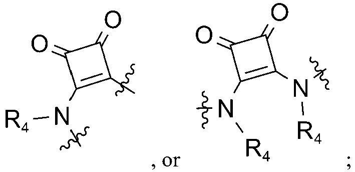
A₈ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 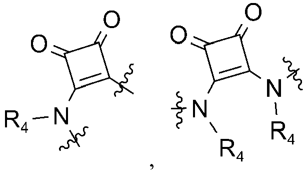 or a bond; and
R₁ and R₂ are each independently C₁-C₂₄ alkyl, -C(R₆)(OR₇)₂, -C(R₆)(SR₇)₂, or -C(R₆)(SR₇)(OR₇).

19. The amino lipid compound according to any one of claims 1 to 3, or a pharmaceutically acceptable salt thereof, or a stereoisomer thereof, wherein the amino lipid compound has a structure represented by formula (VI-5): 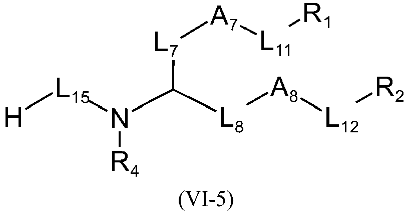 wherein R₁ and R₂ are each independently C₁-C₂₄ hydrocarbyl, -C(R₆)(OL₁₄A₁₂R₇)₂, - C(R₆)(SL₁₄A₁₂R₇)₂, -C(R₆)(SL₁₄A₁₂R₇)(OL₁₄A₁₂R₇), -C(R₆)(C(=O)OL₁₄A₁₂R₇)₂, -C(R₆)(OC(=O)L₁₄A₁₂R₇)₂, -C(R₆)(C(=O)OL₁₄A₁₂R₇)R₇, or -C(R₆)(OC(=O)L₁₄A₁₂R₇)R₇.

20. The amino lipid compound according to claim 19, or a pharmaceutically acceptable salt thereof, or a stereoisomer thereof, wherein:
L₁₅ is C₁-C₂ hydrocarbylene;
R₄ is C₁-C₆ hydrocarbyl or C₁-C₆ heterohydrocarbyl;
L₇ and L₈ are each independently C₁-C₁₀ hydrocarbylene;
L₁₁ and L₁₂ are each independently C₁-C₅ hydrocarbylene;
A₇ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 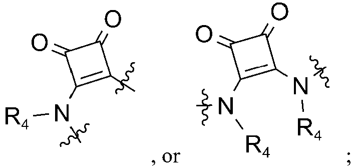
A₈ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 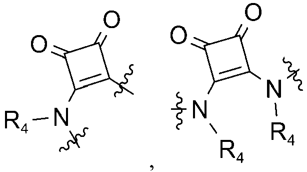 or a bond;
R₁ and R₂ are each independently -C(R₆)(OR₇)₂, -C(R₆)(SR₇)₂, or -C(R₆)(SR₇)(OR₇); and
each R₇ is independently C₁-C₁₂ hydrocarbyl.

21. The amino lipid compound according to any one of claims 1 to 8, 19, and 20, or a pharmaceutically acceptable salt thereof, or a stereoisomer thereof, wherein A₇ and A₈ are -C(=O)O-, L₇ and L₈ are each independently C₄-C₈ alkylene, and R₁ and R₂ are -C(H)(OR₇)₂.

22. The amino lipid compound according to any one of claims 1 to 8, 19, and 21, or a pharmaceutically acceptable salt thereof, or a stereoisomer thereof, wherein L₁₅ is C₁ alkylene.

23. The amino lipid compound according to any one of claims 1 to 3, or a pharmaceutically acceptable salt thereof, or a stereoisomer thereof, wherein the amino lipid compound has a structure represented by formula (VI-6): 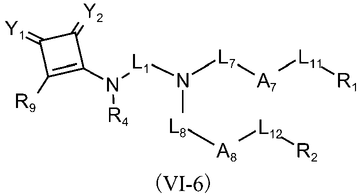 wherein:
Y₁ and Y₂ are each independently O or S;
each R₉ is independently H, halogen, -R_{b}, -N(R_{b})₂, -CN, -N₃, -C(=O)OR_{b}, -OC(=O)R_{b}, -OR_{b}, -SR_{b}, -S(-O)R_{b}, -S(=O)OR_{b}, -S(=O)₂OR_{b}, -N(R_{b})S(=O)₂R_{b}, -NHRₐN(R_{b})₂, -NHRₐORₐN(R_{b})₂, -NHRₐOR_{b}, or - N(RₐOR_{b})₂;
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds;
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds;
L₁ is C₁-C₆ hydrocarbylene, C₃-C₈ carbocyclic ring, or C₁-C₆ heterohydrocarbylene;
L₇ and L₈ are each independently C₁-C₁₀ hydrocarbylene;
L₁₁ and L₁₂ are each independently C₁-C₅ hydrocarbylene or a bond; and
A₇, As, R₁, and R₂ are one selected from the following:
(1) R₁ and R₂ are each independently -C(R₆)(OR₇)₂, -C(R₆)(SR₇)₂, or -C(R₆)(SR₇)(OR₇); and
A₇ and A₈ are each independently -C(=O)O-, -OC(=O)-, -OC(=O)O-, -OC(=O)C(=O)O-, -O-, - SC(=O)O-, -OC(=O)S-, -C(=O)N(R₄)-, or -N(R₄)C(=O)-;
(2) R₁ and R₂ are each independently C₁-C₂₄ hydrocarbyl; A₇ is -OC(=O)S- or -SC(=O)O-; A₈ is - C(=O)O-, -OC(=O)-, -OC(=O)O-, -O-, -SC(=O)O-, -OC(=O)S-, -C(=O)N(R₄)-, -N(R₄)C(=O)-, or - OC(=O)C(=O)O-; and
(3) R₁ and R₂ are each independently C₁-C₂₄ hydrocarbyl; and A₇ and A₈ are each -OC(=S)O-.

24. The amino lipid compound according to any one of claims 1 to 3, or a pharmaceutically acceptable salt thereof, or a stereoisomer thereof, wherein the amino lipid compound has a structure represented by formula (IV-5): 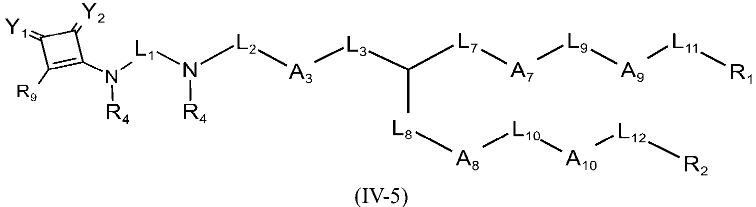 wherein:
Y₁ and Y₂ are each independently O or S;
each R₉ is independently H, halogen, -R_{b}, -N(R_{b})₂, -CN, -N₃, -C(=O)OR_{b}, -OC(=O)R_{b}, -OR_{b}, -SR_{b}, -S(-O)R_{b}, -S(=O)OR_{b}, -S(=O)₂OR_{b}, -N(R_{b})S(=O)₂R_{b}, -NHRₐN(R_{b})₂, -NHRₐORₐN(R_{b})₂, -NHRₐOR_{b}, or - N(RₐOR_{b})₂;
each Rₐ is independently C₁-C₁₂ alkylene, C₂-C₁₂ alkenylene, C₂-C₁₂ alkynylene, C₃-C₈ cyclohydrocarbyl, C₃-C₈ heterocyclyl, C₂-C₁₂ alkenylene with 1, 2, 3, or more double bonds, C₂-C₁₂ alkynylene with 1, 2, 3, or more triple bonds, C₁-C₁₂ heteroalkylene containing O, N, or S, C₂-C₁₂ heteroalkenylene containing O, N, or S with 1, 2, 3, or more double bonds, or C₂-C₁₂ heteroalkynylene containing O, N, or S with 1, 2, 3, or more triple bonds;
each R_{b} is independently H, C₁-C₁₂ alkyl, C₂-C₁₂ alkenylene, C₂-C₁₂ alkynylene, C₃-C₈ cyclohydrocarbyl, C₃-C₈ heterocyclyl, C₂-C₁₂ alkenyl with 1, 2, 3, or more double bonds, C₂-C₁₂ alkynyl with 1, 2, 3, or more triple bonds, C₁-C₁₂ heteroalkyl containing O, N, or S, C₂-C₁₂ heteroalkenyl containing O, N, or S with 1, 2, 3, or more double bonds, or C₂-C₁₂ heteroalkynyl containing O, N, or S with 1, 2, 3, or more triple bonds;
L₁ and L₂ are each independently C₁-C₆ hydrocarbylene;
L₃ is C₁-C₆ hydrocarbylene or a bond;
L₇ and L₈ are each independently C₁-C₁₀ hydrocarbylene or a bond;
L₁₁ and L₁₂ are each independently C₁-C₅ hydrocarbylene or a bond;
A₃ is -C(=O)O-, -OC(=O)-, -OC(=O)O-, -C(=O)N(R₄)-, -N(R₄)C(=O)-, -OC(=O)N(R₄)-, - N(R₄)C(=O)O-, -N(C(=O)L₁₃OR₄)-, -N(C(=O)L₁₃SR₄)-, -N(C(=O)R₄), -N(L₁₃OR₄)-, -OC(=O)C(=O)O-, - N(R₄)C(=O)C(=O)O-, -OC(=O)C(=O)N(R₄)-, -N(R₄)C(=O)C(=O)N(R₄)-, 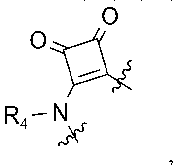 or 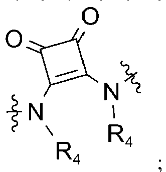 A₇ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 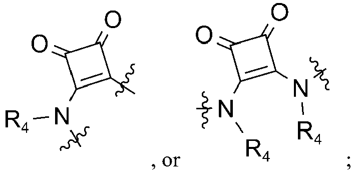
A₈ is -C(=O)O-, -OC(=O)-, -OC(=O)O-, -C(=O)N(R₄)-, -N(R₄)C(=O)-, -N(C(=O)L₁₃OR₄)-, - N(C(=O)L₁₃SR₄)-, -N(C(=O)R₄), -N(L₁₃OR₄)-, -OC(=O)C(=O)O-, 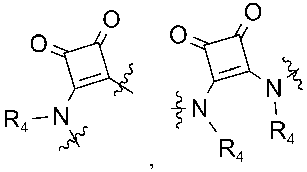 or a bond; and
R₁ and R₂ are each independently C₁-C₂₄ alkyl, -C(R₆)(OR₇)₂, -C(R₆)(SR₇)₂, or -C(R₆)(SR₇)(OR₇).

25. The amino lipid compound according to claim 24, or a pharmaceutically acceptable salt thereof, or a stereoisomer thereof, wherein the amino lipid compound has a structure represented by formula (IV-5-1): 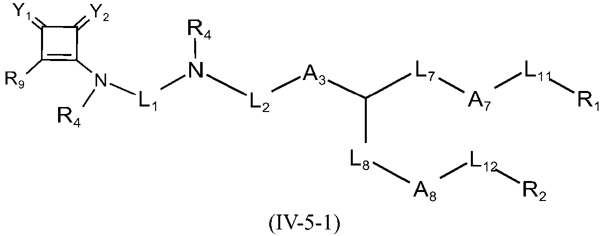 wherein R₁ and R₂ are each independently -C(R₆)(OR₇)₂.

26. The amino lipid compound according to any one of claims 1 to 11, 15, 17, and 19, or a pharmaceutically acceptable salt thereof, or a stereoisomer thereof, wherein A₇ and A₈ are each independently -C(=O)O-, -OC(=O)-, -OC(=O)O-, -C(=O)N(R₄)-, -N(R₄)C(=O)-, -N(C(=O)L₁₃OR₄)-, -N(C(=O)L₁₃SR₄)-, - N(C(=O)R₄), -N(L₁₃OR₄)-, -OC(=O)C(=O)O-,

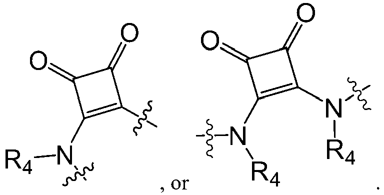

27. The amino lipid compound according to any one of claims 1 to 12, 15 to 20, and 24 to 26, or a pharmaceutically acceptable salt thereof, or a stereoisomer thereof, wherein A₇ and A₈ are each independently -C(=O)O-, -OC(=O)-, -OC(=O)O-, -C(=O)N(R₄)-, -N(R₄)C(=O)-, or -OC(=O)C(=O)O-.

28. The amino lipid compound according to claim 27, or a pharmaceutically acceptable salt thereof, or a stereoisomer thereof, wherein A₇ and A₈ are each independently -C(=O)O- or -OC(=O)-.

29. The amino lipid compound according to claim 28, or a pharmaceutically acceptable salt thereof, or a stereoisomer thereof, wherein A₇ and A₈ are -C(=O)O-.

30. The amino lipid compound according to any one of claims 12, 18, 23, and 24, or a pharmaceutically acceptable salt thereof, or a stereoisomer thereof, wherein A₇ and A₈ are each independently -C(=O)O- or - OC(=O)-, L₁₁ is a bond, L₁₂ is a bond, and R₁ and R₂ are each independently a straight C₄-C₁₂ alkyl or a branched C₉-C₁₈ alkyl.

31. The amino lipid compound according to any one of claims 12, 18, 20, and 23 to 25, or a pharmaceutically acceptable salt thereof, or a stereoisomer thereof, wherein A₇ and A₈ are each independently -C(=O)O- or -OC(=O)-, and R₁ and R₂ are each independently -C(H)(OR₇)₂.

32. The amino lipid compound according to any one of claims 1, 3 to 12, 17 to 19, 23, 24, and 26 to 29, or a pharmaceutically acceptable salt thereof, or a stereoisomer thereof, wherein R₁ and R₂ are each independently C₁-C₂₄ alkyl.

33. The amino lipid compound according to claim 30 or 32, or a pharmaceutically acceptable salt thereof, or a stereoisomer thereof, wherein R₁ is a straight C₄-C₁₂ alkyl, and R₂ is a branched C₉-C₁₈ alkyl.

34. The amino lipid compound according to claim 30 or 32, or a pharmaceutically acceptable salt thereof, or a stereoisomer thereof, wherein R₁ is a straight C₇-C₁₀ alkyl, and R₂ is a branched C₉-C₁₇ alkyl.

35. The amino lipid compound according to claim 30 or 32, or a pharmaceutically acceptable salt thereof, or a stereoisomer thereof, wherein R₁ is a branched C₉-C₁₈ alkyl, and R₂ is a branched C₉-C₁₈ alkyl.

36. The amino lipid compound according to claim 30 or 32, or a pharmaceutically acceptable salt thereof, or a stereoisomer thereof, wherein R₁ is a branched C₉-C₁₇ alkyl, and R₂ is a branched C₉-C₁₇ alkyl.

37. The amino lipid compound according to any one of claims 1, 3 to 12, and 17 to 29, or a pharmaceutically acceptable salt thereof, or a stereoisomer thereof, wherein R₁ and R₂ are each independently -C(H)(OR₇)₂.

38. The amino lipid compound according to claim 31 or 37, or a pharmaceutically acceptable salt thereof, or a stereoisomer thereof, wherein each R₇ is independently C₃-C₁₀ alkyl.

39. The amino lipid compound according to claim 31 or 37, or a pharmaceutically acceptable salt thereof, or a stereoisomer thereof, wherein each R₇ is independently a straight C₅-C₇ alkyl.

40. The amino lipid compound according to any one of claims 1 to 39, or a pharmaceutically acceptable salt thereof, or a stereoisomer thereof, wherein Y₁ and Y₂ are O.

41. The amino lipid compound according to any one of claims 1 to 40, or a pharmaceutically acceptable salt thereof, or a stereoisomer thereof, wherein R₉ is -N(R_{b})₂.

42. The amino lipid compound according to any one of claims 1 to 41, or a pharmaceutically acceptable salt thereof, or a stereoisomer thereof, wherein each R_{b} is independently H, C₁-C₆ alkyl, or C₂-C₃ alkenyl.

43. The amino lipid compound according to any one of claims 1 to 42, or a pharmaceutically acceptable salt thereof, or a stereoisomer thereof, wherein R₉ is -N(H)CH₃.

44. The amino lipid compound according to any one of claims 1 to 18 and 23 to 43, or a pharmaceutically acceptable salt thereof, or a stereoisomer thereof, wherein L₁ is C₁-C₅ alkylene.

45. The amino lipid compound according to any one of claims 1 to 16 and 26 to 43, or a pharmaceutically acceptable salt thereof, or a stereoisomer thereof, wherein L₁ is C₂-C₅ alkylene.

46. The amino lipid compound according to claim 44 or 45, or a pharmaceutically acceptable salt thereof, or a stereoisomer thereof, wherein L₁ is C₁-C₅ alkylene substituted with a substituent, or C₂-C₅ alkylene substituted with a substituent.

47. The amino lipid compound according to any one of claims 1 to 3, 17 and 18, and 24 to 46, or a pharmaceutically acceptable salt thereof, or a stereoisomer thereof, wherein L₂ is C₂-C₄ alkylene.

48. The amino lipid compound according to any one of claims 1 to 47, or a pharmaceutically acceptable salt thereof, or a stereoisomer thereof, wherein L₇ and L₈ are each independently C₄-C₈ alkylene.

49. The amino lipid compound according to any one of claims 1 to 48, or a pharmaceutically acceptable salt thereof, or a stereoisomer thereof, wherein L₁₁ is C₂-C₄ alkylene or a bond.

50. The amino lipid compound according to any one of claims 1 to 49, or a pharmaceutically acceptable salt thereof, or a stereoisomer thereof, wherein L₁₂ is C₂-C₄ alkylene or a bond.

51. The amino lipid compound according to any one of claims 1 to 50, or a pharmaceutically acceptable salt thereof, or a stereoisomer thereof, wherein each R₄ is independently H or C₁-C₂ alkyl.

52. The amino lipid compound according to any one of claims 1 to 51, having one of the following structures:
| Amino lipid compounds | Structural formulae |
|---|---|
| 1501 | 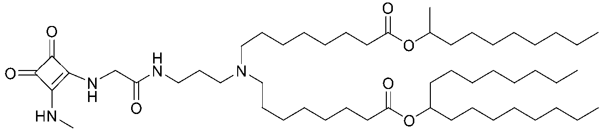 |
| 1502 | 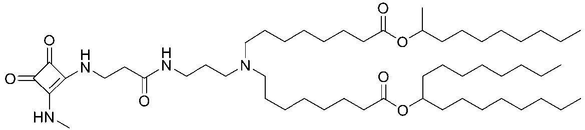 |
| 1503 | 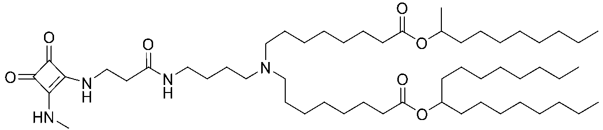 |
| 1504 | 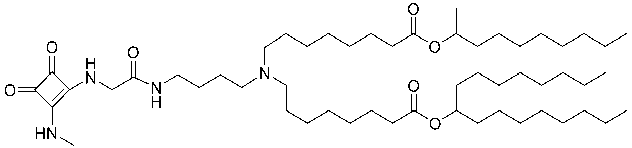 |
| 1505 | 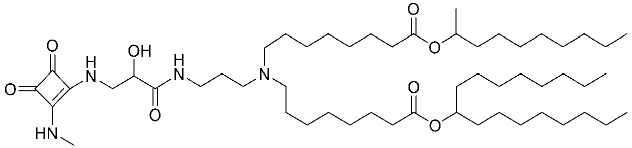 |
| 1506 | 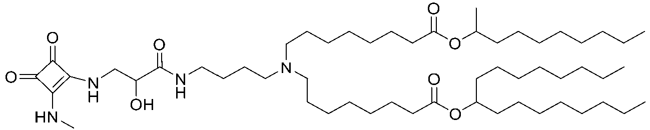 |
| 1507 | 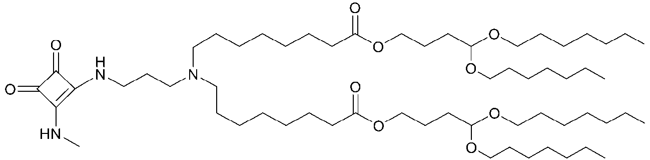 |
| 1508 | 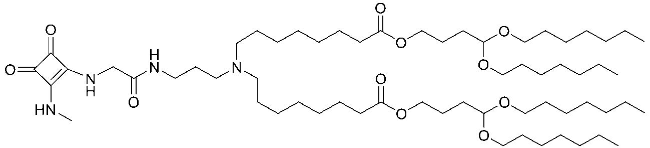 |
| 1509 | 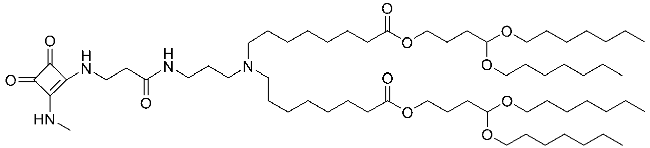 |
| 1510 | 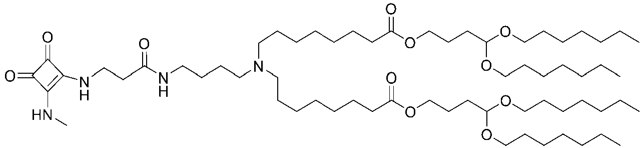 |
| 1511 | 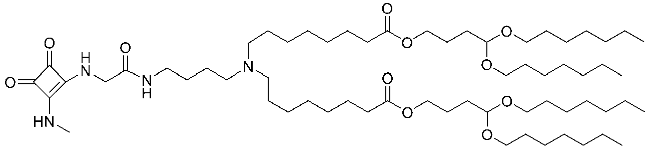 |
| 1512 | 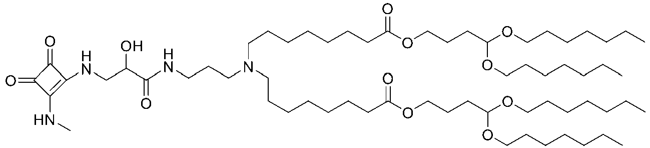 |
| 1513 | 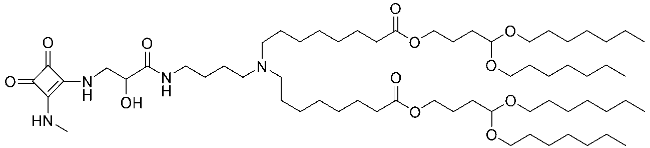 |
| 1525 | 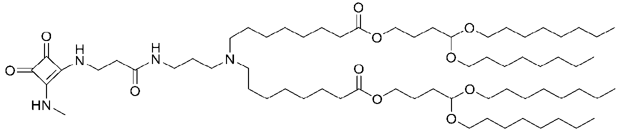 |
| 1526 | 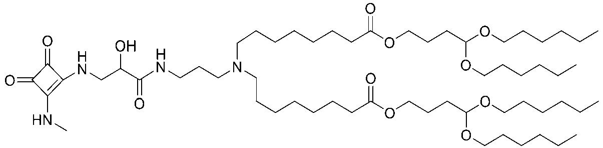 |
| 1531 | 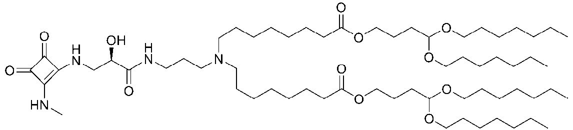 |
| 1532 | 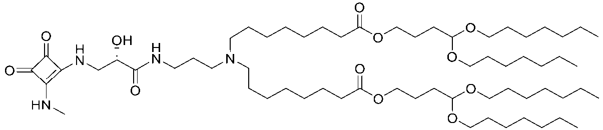 |
| 1640 | 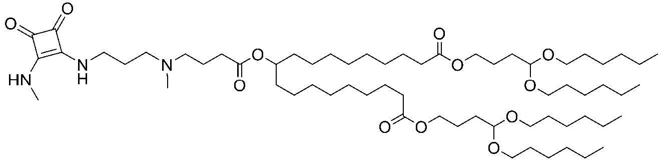 |
| 1643 | 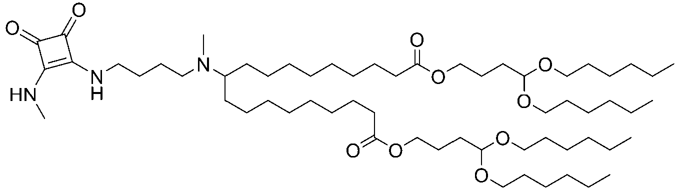 |
| 1644 | 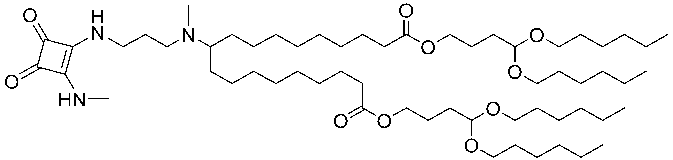 |
| 1720 | 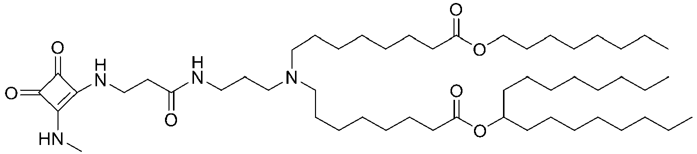 |
| 1721 | 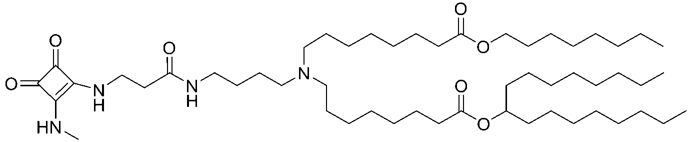 |
| 1726 | 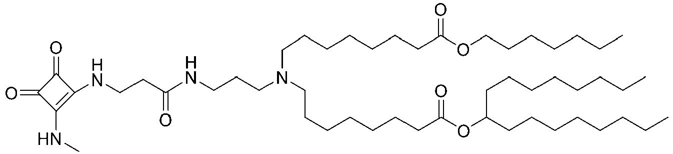 |
| 1727 | 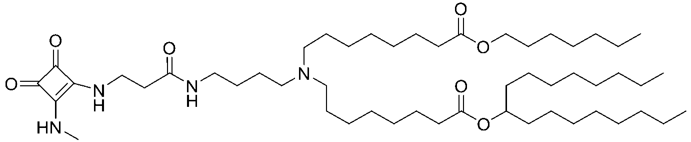 |
| 1732 | 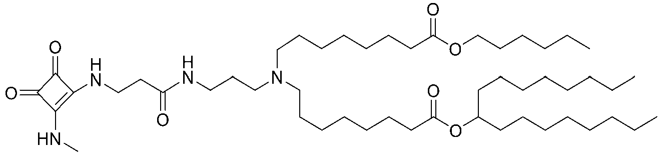 |
| 1733 | 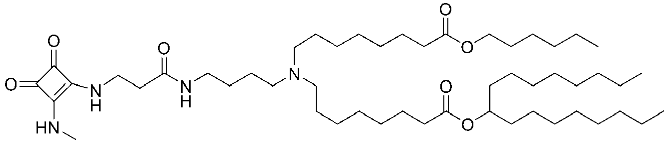 |
| 1746 | 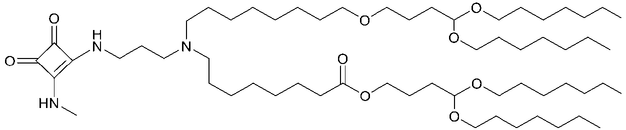 |
| 1747 | 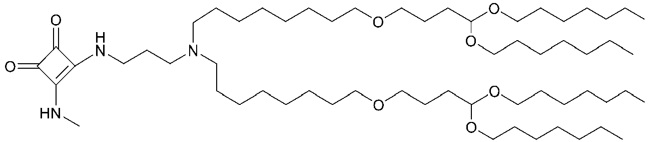 |
| 1748 | 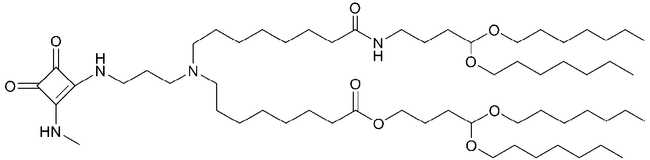 |
| 1749 | 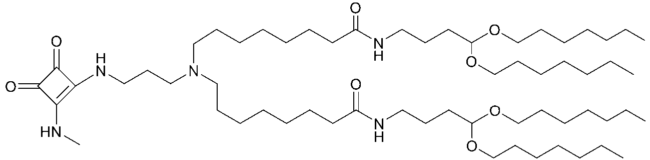 |
| 1807 | 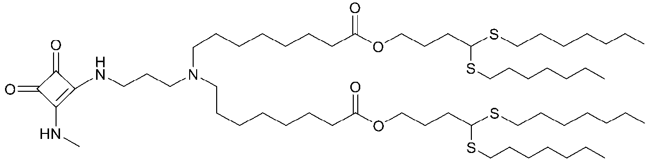 |
| 1831 | 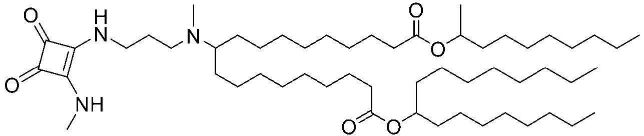 |
| 1832 | 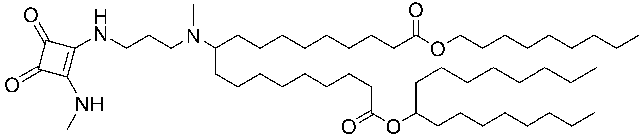 |
| 1833 | 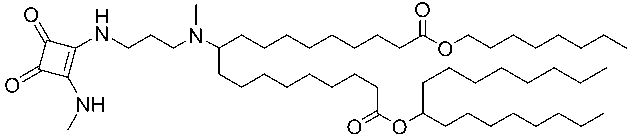 |
| 1834 | 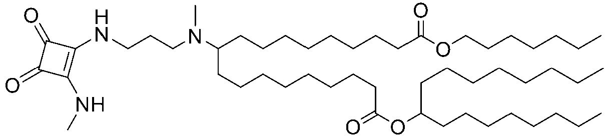 |
| 1843 | 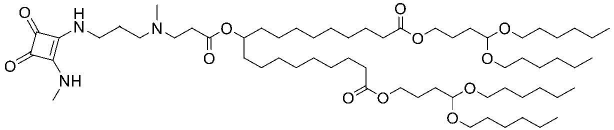 |
| 1844 | 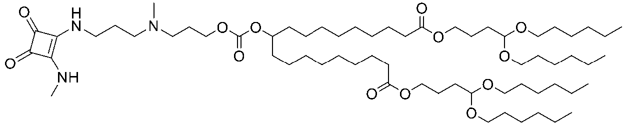 |
| 1845 | 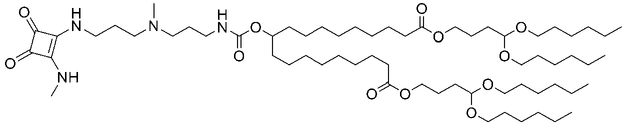 |
| 1847 | 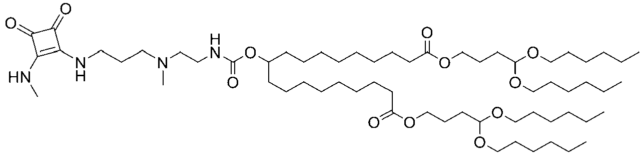 |
| 1849 | 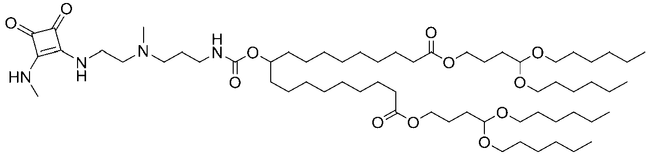 |
| 1854 | 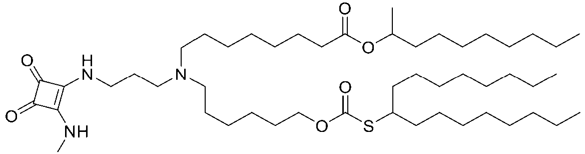 |
| 1855 | 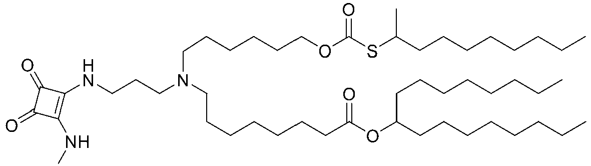 |
| 1856 | 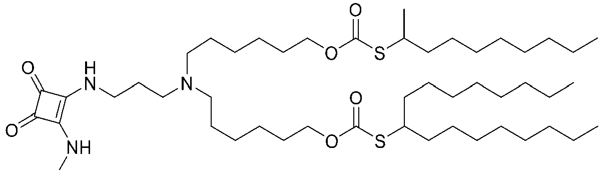 |
| 1873 | 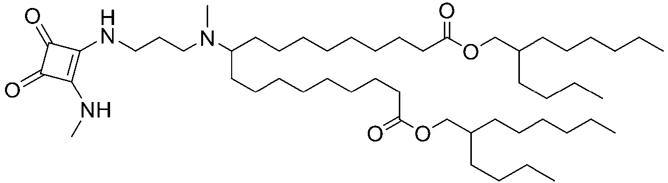 |
| 1874 | 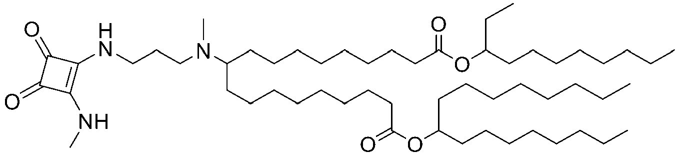 |
| 1875 | 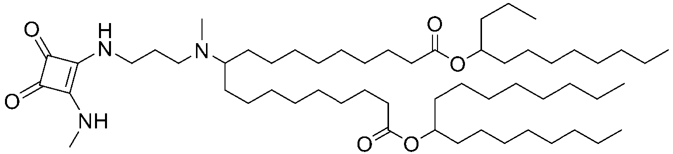 |
| 1876 | 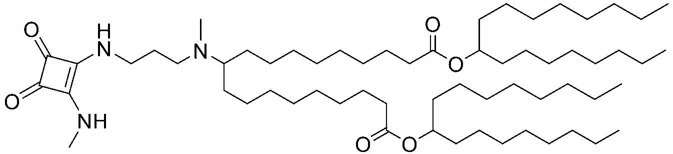 |
| 1880 | 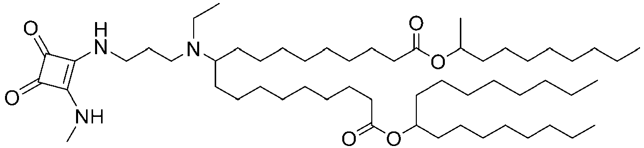 |
| 1881 | 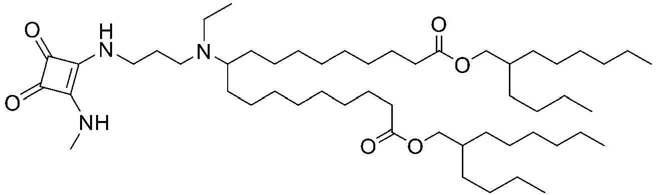 |
| 1912 | 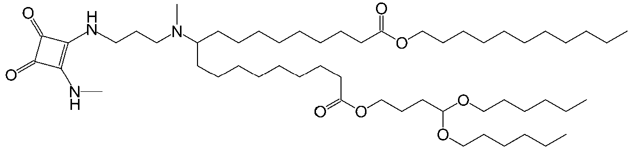 |
| 1913 | 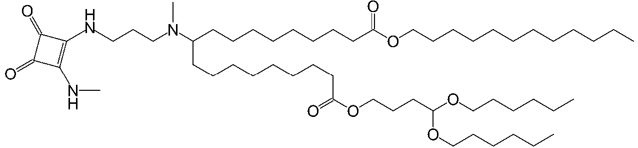 |
| 1924 | 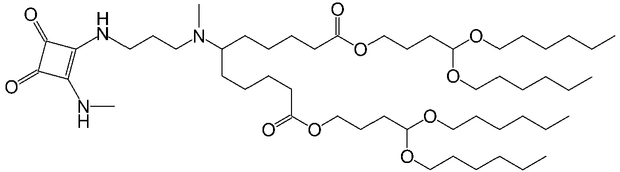 |
| 1925 | 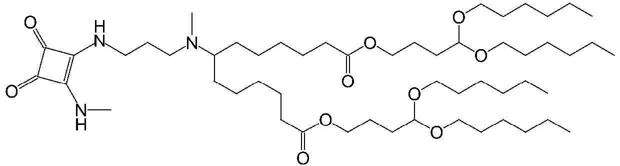 |
| 1926 | 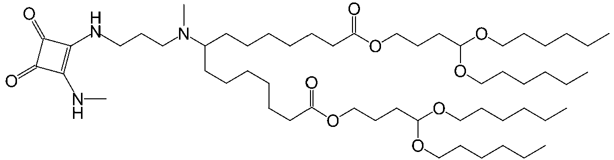 |
| 1927 | 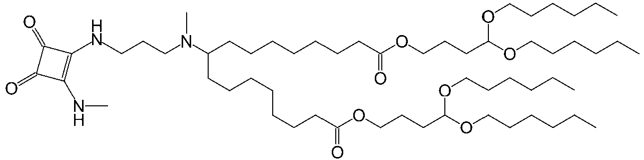 |
| 1928 | 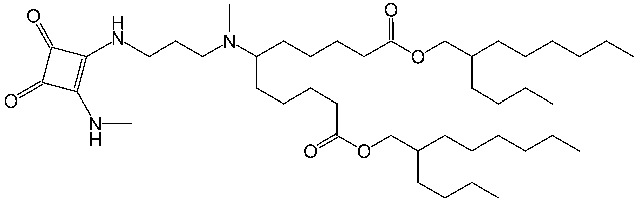 |
| 1929 | 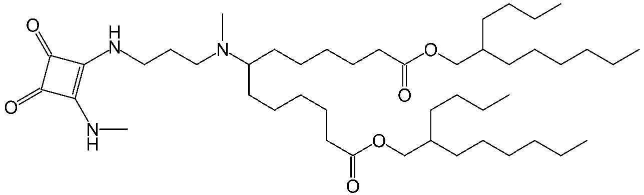 |
| 1930 | 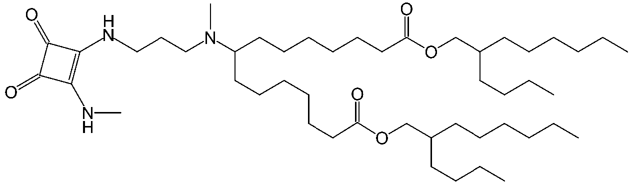 |
| 1931 | 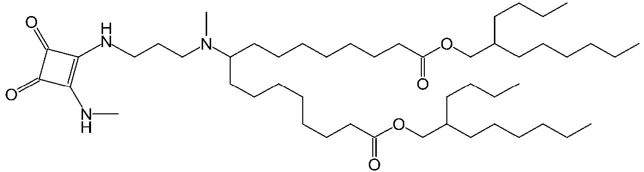 |
| 1950 | 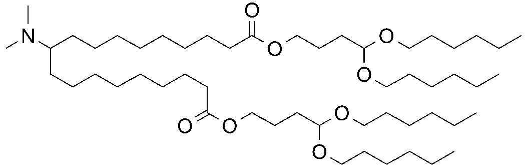 |
| 2047 | 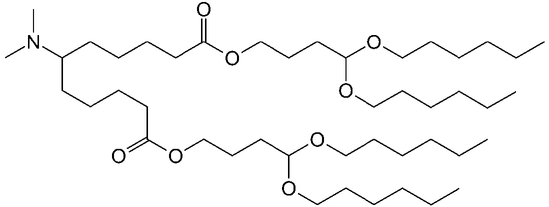 |
| 2048 | 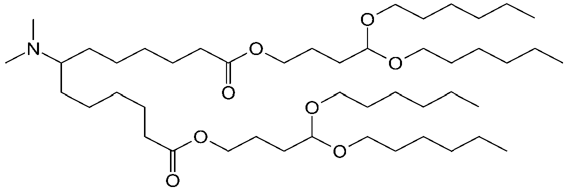 |
| 2050 | 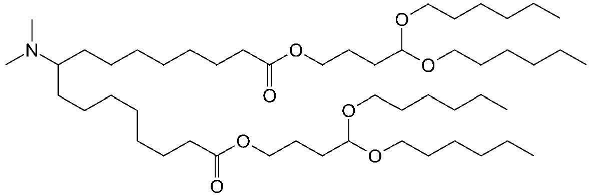 |
| 2058 | 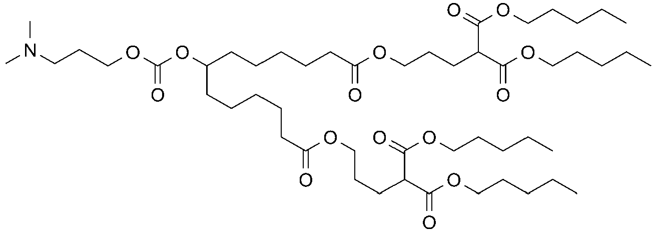 |
| 2059 | 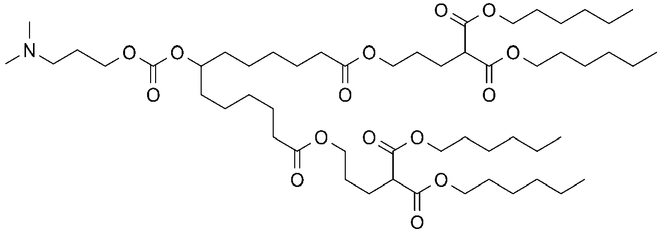 |
| 2060 | 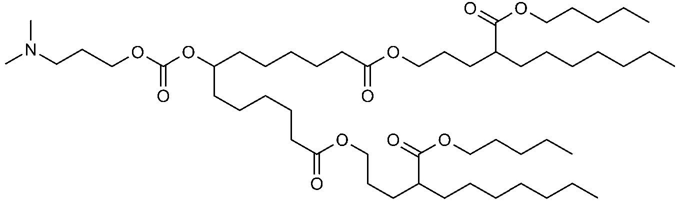 |

53. A lipid nanoparticle comprising the amino lipid compound of any one of claims 1 to 52.

54. A pharmaceutical composition comprising the amino lipid compound of any one of claims 1 to 52 or the lipid nanoparticle of claim 53, and a pharmaceutically acceptable carrier, diluent, or excipient.

55. Use of the amino lipid compound of any one of claims 1 to 52 in the manufacture of a vehicle for an active ingredient.

56. Use of the amino lipid compound of any one of claims 1 to 52, the lipid nanoparticle of claim 53, or the pharmaceutical composition of claim 54 in the manufacture of a medicament.

57. Use of the amino lipid compound of any one of claims 1 to 52, the lipid nanoparticle of claim 53, or the pharmaceutical composition of claim 54 in the manufacture of a medicament for nucleic acid transfer.
